# EUROPEAN PATENT APPLICATION

(11) **EP 4 548 918 A2**
(43) Date of publication of application: **07.05.2025**
(21) Application number: 24222489.7
(22) Date of filing: 08.01.2021
(51) Int. Cl.: A61K 31/437

(54) **TRICYCLIC COMPOUNDS AS INHIBITORS OF KRAS**

(30) Priority: 10.01.2020 US 202062959536 P; 15.07.2020 US 202063052274 P
(62) Divisional of application: 21705672.0
(71) Applicant: Incyte Corporation, Wilmington, DE 19803 (US)
(72) Inventor: WANG, Xiaozhao, Wilmington (US); ZHU, Wenyu, Wilmington (US); YANG, Jeffrey, Wilmington (US); HE, Chunhong, Wilmington (US); ZHANG, Fenglei, Wilmington (US); HAN, Heeoon, Wilmington (US); ZHAO, Le, Wilmington (US); LI, Yong, Wilmington (US); GAN, Pei, Wilmington (US); QI, Chao, Wilmington (US); LAW, Chunyin, Marshall, Wilmington (US); SOKOLSKY, Alexander, Wilmington (US); LI, Zhenwu, Wilmington (US); HOANG, Gia, Wilmington (US); CARLSEN, Peter, Wilmington (US); POLAM, Padmaja, Wilmington (US); WU, Liangxing, Wilmington (US); YAO, Wenqing, Wilmington (US)
(74) Representative: Boult Wade Tennant LLP

(57) **Abstract**

Disclosed are compounds of Formula I, methods of using the compounds for inhibiting KRAS activity and pharmaceutical compositions comprising such compounds. The compounds are useful in treating, preventing or ameliorating diseases or disorders associated with KRAS activity such as cancer.

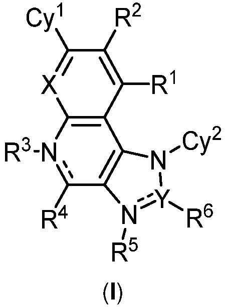

## Description

### RELATED APPLICATIONS

This application claims priority to U.S. Provisional Application No. 62/959,536 filed on January 10, 2020 and U.S. Provisional Application No. 63/052,274 filed on July 15, 2020. The contents of each application are hereby incorporated by reference in their entireties.

### FIELD OF THE INVENTION

The disclosure provides compounds as well as their compositions and methods of use. The compounds modulate KRAS activity and are useful in the treatment of various diseases including cancer.

### BACKGROUND OF THE INVENTION

Ras proteins are part of the family of small GTPases that are activated by growth factors and various extracellular stimuli. The Ras family regulates intracellular signaling pathways responsible for growth, migration, survival and differentiation of cells. Activation of RAS proteins at the cell membrane results in the binding of key effectors and initiation of a cascade of intracellular signaling pathways within the cell, including the RAF and PI3K kinase pathways. Somatic mutations in RAS may result in uncontrolled cell growth and malignant transformation while the activation of RAS proteins is tightly regulated in normal cells (Simanshu, D. et al. Cell 170.1 (2017):17-33).

The Ras family is comprised of three members: KRAS, NRAS and HRAS. RAS mutant cancers account for about 25% of human cancers. KRAS is the most frequently mutated isoform accounting for 85% of all RAS mutations whereas NRAS and HRAS are found mutated in 12% and 3% of all Ras mutant cancers respectively (Simanshu, D. et al. Cell 170.1 (2017):17-33). KRAS mutations are prevalent amongst the top three most deadly cancer types: pancreatic (97%), colorectal (44%), and lung (30%) (Cox, A.D. et al. Nat Rev Drug Discov (2014) 13:828-51). The majority of RAS mutations occur at amino acid residue 12, 13, and 61. The frequency of specific mutations varies between RAS gene isoforms and while G12 and Q61 mutations are predominant in KRAS and NRAS respectively, G12, G13 and Q61 mutations are most frequent in HRAS. Furthermore, the spectrum of mutations in a RAS isoform differs between cancer types. For example, KRAS G12D mutations predominate in pancreatic cancers (51%), followed by colorectal adenocarcinomas (45%) and lung cancers (17%) while KRAS G12 V mutations are associated with pancreatic cancers (30%), followed by colorectal adenocarcinomas (27%) and lung adenocarcinomas (23%) (Cox, A.D. et al. Nat Rev Drug Discov (2014) 13:828-51). In contrast, KRAS G12C mutations predominate in non-small cell lung cancer (NSCLC) comprising 11-16% of lung adenocarcinomas, and 2-5% of pancreatic and colorectal adenocarcinomas (Cox, A.D. et al. Nat. Rev. Drug Discov. (2014) 13:828-51). Genomic studies across hundreds of cancer cell lines have demonstrated that cancer cells harboring KRAS mutations are highly dependent on KRAS function for cell growth and survival (McDonald, R. et al. Cell 170 (2017): 577-592). The role of mutant KRAS as an oncogenic driver is further supported by extensive in vivo experimental evidence showing mutant KRAS is required for early tumour onset and maintenance in animal models (Cox, A.D. et al. Nat Rev Drug Discov (2014) 13:828-51).

Taken together, these findings suggest that KRAS mutations play a critical role in human cancers; development of inhibitors targeting mutant KRAS may therefore be useful in the clinical treatment of diseases that are characterized by a KRAS mutation.

### SUMMARY

The present disclosure provides, *inter alia*, a compound of Formula I: 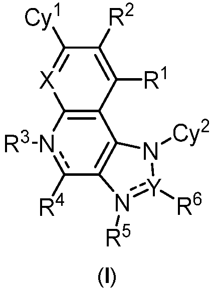 or a pharmaceutically acceptable salt thereof, wherein constituent variables are defined herein.

The present disclosure further provides a pharmaceutical composition comprising a compound of the disclosure, or a pharmaceutically acceptable salt thereof, and at least one pharmaceutically acceptable carrier or excipient.

The present disclosure further provides methods of inhibiting KRAS activity, which comprises administering to an individual a compound of the disclosure, or a pharmaceutically acceptable salt thereof. The present disclosure also provides uses of the compounds described herein in the manufacture of a medicament for use in therapy. The present disclosure also provides the compounds described herein for use in therapy.

The present disclosure further provides methods of treating a disease or disorder in a patient comprising administering to the patient a therapeutically effective amount of a compound of the disclosure, or a pharmaceutically acceptable salt thereof.

### DETAILED DESCRIPTION

### Compounds

In an aspect, provided herein is a compound of Formula I: 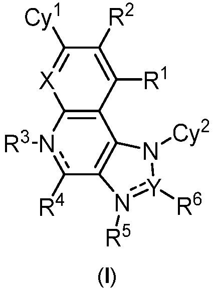 or a pharmaceutically acceptable salt thereof, wherein:
each --- represents a single bond or a double bond;
X is N or CR⁷;
Y is N or C;
R¹ is selected from H, D, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, CN, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, NR^{c1}S(O)₂NR^{c1}R^{d1}, S(O)R^{b1}, S(O)NR^{c1}R^{d1}, S(O)₂R^{b1}, S(O)₂NR^{c1}R^{d1}, and BR^{h1}Rⁱ¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R^{g};
R² is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, C(=NR^{e2})R^{b2}, C(=NOR^{a2})R^{b2}, C(=NR^{e2})NR^{c2}R^{d2}, NR^{c2}C(=NR^{e2})NR^{c2}R^{d2}, NR^{c2}C(=NR^{e2})R^{b2}, NR^{c2}S(O)R^{b2}, NR^{c2}S(O)₂R^{b2}, NR^{c2}S(O)₂NR^{c2}R^{d2}, S(O)R^{b2}, S(O)NR^{c2}R^{d2}, S(O)₂R^{b2}, S(O)₂NR^{c2}R^{d2}, and BR^{h2}Rⁱ²; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²²;
Cy¹ is selected from C₃₋₁₀ cycloalkyl, 4-10 membered heterocycloalkyl, C₆₋₁₀ aryl and 5-10 membered heteroaryl; wherein the 4-10 membered heterocycloalkyl and 5-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 5-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 5-10 membered heteroaryl are each optionally substituted with 1, 2, 3 or 4 substituents independently selected from R¹⁰;
when R³N---CR⁴ is a single bond, then R⁴ is selected from =O and =S; and
R³ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R³N---CR⁴ is a double bond, then R³ is absent; and
R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, NR^{c3}C(O)NR^{c3}R ^{d3}, C(=NR^{e3})R^{b3}, C(=NOR^{a3})R^{b3}, C(=NR^{e3})NR^{c3}R^{d3}, NR^{c3}C(=NR^{e3})NR^{c3}R^{d3}, NR^{c3}C(=NR^{e3})R^{b3}, NR^{c3}S(O)R^{b3}, NR^{c3}S(O)₂R^{b3}, NR^{c3}S(O)₂NR^{c3}R^{d3}, S(O)R^{b3}, S(O)NR^{c3}R^{d3}, S(O)₂R^{b3}, S(O)₂NR^{c3}R^{d3}, and BR^{h3}Rⁱ³; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R⁵N---YR⁶ is a single bond and Y is C, then YR⁶ is selected from C=O and C=S; and
R⁵ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁵⁰;
when R⁵N---YR⁶ is a double bond and Y is N, then R⁵ and R⁶ are absent;
when R⁵N---YR⁶ is a double bond and Y is C, then R⁵ is absent; and
R⁶ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a6}, SR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, C(=NR^{e6})R^{b6}, C(=NOR^{a6})R^{b6}, C(=NR^{e6})NR^{c6}R^{d6}, NR^{c6}C(=NR^{e6})NR^{c6}R^{d6}, NR^{c6}C(=NR^{e6})R^{b6}, NR^{c6}S(O)R^{b6}, NR^{c6}S(O)₂R^{b6}, NR^{c6}S(O)₂NR^{c6}R^{d6}, S(O)R^{b6}, S(O)NR^{c6}R^{d6}, S(O)₂R^{b6}, S(O)₂NR^{c6}R^{d6}, and BR^{h6}Rⁱ⁶; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶⁰;
R⁷ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a7}, SR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7}, C(=NR^{e7})R^{b7}, C(=NOR^{a7})R^{b7}, C(=NR^{e7})NR^{c7}R^{d7}, NR^{c7}C(=NR^{e7})NR^{c7}R^{d7}, NR^{c7}C(=NR^{e7})R^{b7}, NR^{c7}S(O)R^{b7}, NR^{c7}S(O)₂R^{b7}, NR^{c7}S(O)₂NR^{c7}R^{d7}, S(O)R^{b7}, S(O)NR^{c7}R^{d7}, S(O)₂R^{b7}, S(O)₂NR^{c7}R^{d7}, and BR^{h7}Rⁱ⁷; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷⁰;
Cy² is selected from C₃₋₁₀ cycloalkyl, 4-14 membered heterocycloalkyl, C₆₋₁₀ aryl and 5-10 membered heteroaryl; wherein the 4-14 membered heterocycloalkyl and 5-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 5-10 membered heteroaryl and 4-14 membered heterocycloalkyl is optionally substituted by oxo to form a carbonyl group; and wherein the C₃₋₁₀ cycloalkyl, 4-14 membered heterocycloalkyl, C₆₋₁₀ aryl and 5-10 membered heteroaryl are each optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a10}, SR^{a10}, C(O)R^{b10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, NR^{c10}C(O)NR^{c10}R^{d10}, C(=NR^{e10})R^{b10}, C(=NOR^{a10})R^{b10}, C(=NR^{e10})NR^{c10}R^{d10}, NR^{c10}C(=NR^{e10})NR^{c10}R^{d10}, NR^{c10}S(O)R^{b10}, NR^{c10}S(O)₂R^{b10}, NR^{c10}S(O)₂NR^{c10}R^{d10}, S(O)R^{b10}, S(O)NR^{c10}R^{d10}, S(O)₂R^{b10}, S(O)₂NR^{c10}R^{d10}, and BR^{h10}Rⁱ¹⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R¹¹;
each R¹¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a11}, SR^{a11}, C(O)R^{b11}, C(O)NR^{c11}R^{d11}, C(O)OR^{a11}, OC(O)R^{b11}, OC(O)NR^{c11}R^{d11}, NR^{c11}R^{d11}, NR^{c11}C(O)R^{b11}, NR^{c11}C(O)OR^{a11}, NR^{c11}C(O)NR^{c11}R^{d11}, NR^{c11}S(O)R^{b11}, NR^{c11}S(O)₂R^{b11}, NR^{c11}S(O)₂NR^{c11}R^{d11}, S(O)R^{b11}, S(O)NR^{c11}R^{d11}, S(O)₂R^{b11}, S(O)₂NR^{c11}R^{d11}, and BR^{h11}Rⁱ¹¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R¹²;
each R¹² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a12}, SR^{a12}, C(O)R^{b12}, C(O)NR^{c12}R^{d12}, C(O)OR^{a12}, OC(O)R^{b12}, OC(O)NR^{c12}R^{d12}, NR^{c12}R^{d12}, NR^{c12}C(O)R^{b12}, NR^{c12}C(O)OR^{a12}, NR^{c12}C(O)NR^{c12}R^{d12}, NR^{c12}S(O)R^{b12}, NR^{c12}S(O)₂R^{b12}, NR^{c12}S(O)NR^{c12}R^{d12}, S(O)R^{b12}, S(O)NR^{c12}R^{d12}, S(O)₂R^{b12}, S(O)₂NR^{c12}R^{d12}, and BR^{h12}Rⁱ¹²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R²⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a20}, SR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)^{Rb20}, NR^{c20}C(O)OR^{a20}, NR^{c20}C(O)NR^{c20}R^{d20}, C(=NR^{e20})R^{b20}, C(=NOR^{a20})R^{b20}, C(=NR^{e20})NR^{c20}R^{d20}, NR^{c20}C(=NR^{e20})NR^{c20}R^{d20}, NR^{c20}S(O)R^{b20}, NR^{c20}S(O)₂R^{b20}, NR^{c20}S(O)₂NR^{c20}R^{d20}, S(O)R^{b20}, S(O)NR^{c20}R^{d20}, S(O)₂R^{b20}, S(O)₂NR^{c20}R^{d20}, and BR^{h20}Rⁱ²⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²¹;
each R²¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a21}, SR^{a21}, C(O)R^{b21}, C(O)NR^{c21}R^{d21}, C(O)OR^{a21}, OC(O)R^{b21}, OC(O)NR^{c21}R^{d21}, NR^{c21}R^{d21}, NR^{c21}C(O)R^{b21}, NR^{c21}C(O)OR^{a21}, NR^{c21}C(O)NR^{c21}R^{d21}, NR^{c21}S(O)R^{b21}, NR^{c21}S(O)₂R^{b21}, NR^{c21}S(O)₂NR^{c21}R^{d21}, S(O)R^{b21}, S(O)^{NRc21}R^{d21}, S(O)₂R^{b21}, S(O)₂NR^{c21}R^{d21}, and BR^{h21}Rⁱ²¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R^{g};
each R²² is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a22}, SR^{a22}, C(O)R^{b22}, C(O)NR^{c22}R^{d22}, C(O)OR^{a22}, OC(O)R^{b22}, OC(O)NR^{c22}R^{d22}, NR^{c22}R^{d22}, NR^{c22}C(O)R^{b22}, NR^{c22}C(O)OR^{a22}, NR^{c22}C(O)NR^{c22}R^{d22}, NR^{c22}S(O)R^{b22}, NR^{c22}S(O)₂R^{b22}, NR^{c22}S(O)₂NR^{c22}R^{d22}, S(O)R^{b22}, S(O)NR^{c22}R^{d22}, S(O)₂R^{b22}, S(O)₂NR^{c22}R^{d22}, and BR^{h22}Rⁱ²²; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²³;
each R²³ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a23}, SR^{a23}, C(O)R^{b23}, C(O)NR^{c23}R^{d23}, C(O)OR^{a23}, OC(O)R^{b23}, OC(O)NR^{c23}R^{d23}, NR^{c23}R^{d23}, NR^{c23}C(O)R^{b23}, NR^{c23}C(O)OR^{a23}, NR^{c23}C(O)NR^{c23}R^{d23}, NR^{c23}S(O)R^{b23}, NR^{c23}S(O)₂R^{b23}, NR^{c23}S(O)₂NR^{c23}R^{d23}, S(O)R^{b23}, S(O)NR^{c23}R^{d23}, S(O)₂R^{b23}, S(O)₂NR^{c23}R^{d23}, and BR^{h23}Rⁱ²³; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²⁴;
each R²⁴ is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a24}, SR^{a24}, C(O)R^{b24}, C(O)NR^{c24}R^{d24}, C(O)OR^{a24}, OC(O)R^{b24}, OC(O)NR^{c24}R^{d24}, NR^{c24}R^{d24}, NR^{c24}C(O)R^{b24}, NR^{c24}C(O)OR^{a24}, NR^{c24}C(O)NR^{c24}R^{d24}, NR^{c24}S^{b24}, NR^{c24}S(O)₂R^{b24}, NR^{c24}S(O)₂NR^{c24}R^{d24}, S(O)R^{b24}, S(O)NR^{c24}R^{d24}, S(O)₂R^{b24}, S(O)₂NR^{c24}R^{d24}, and BR^{h24}Rⁱ²⁴; wherein said C₁₋₆ alkyl, C₂₋₆ alkenyl, C₂₋₆ alkynyl, C₃₋₆ cycloalkyl, phenyl, 5-6 membered heteroaryl and 4-7 membered heterocycloalkyl, are each optionally substituted with 1, 2, 3, or 4 substituents independently selected from R^{g};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a30}, SR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, NR^{c30}C(O)NR^{c30}R^{d30}, NR^{c30}S(O)R^{b30}, NR^{c30}S(O)₂R^{b30}, NR^{c30}S(O)₂NR^{c30}R^{d30}, S(O)R^{b30}, S(O)NR^{c30}R^{d30}, S(O)₂R^{b30}, S(O)₂NR^{c30}R^{d30}, and BR^{h30}Rⁱ³⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a31}, SR^{a31}, C(O)R^{b31}, C(O)NR^{c31}R^{d31}, C(O)OR^{a31}, OC(O)R^{b31}, OC(O)NR^{c31}R^{d31}, NR^{c31}R^{d31}, NR^{c31}C(O)R^{b31}, NR^{c31}C(O)OR^{a31}, NR^{c31}C(O)NR^{c31}R^{d31}, NR^{e31}S(O)R^{b31}, NR^{c31}S(O)₂R^{b31}, NR^{c31}S(O)₂NR^{c31}R^{d31}, S(O)R^{b31}, S(O)NR^{c31}R^{d31}, S(O)₂R^{b31}, S(O)₂NR^{c31}R^{d31}, and BR^{h31}Rⁱ³¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³²;
each R³² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a32}, SR^{a32}, C(O)R^{b32}, C(O)NR^{c32}R^{d32}, C(O)OR^{a32}, OC(O)R^{b32}, OC(O)NR^{c32}R^{d32}, NR^{c32}R^{d32}, NR^{c32}C(O)R^{b32}, NR^{c32}C(O)OR^{a32}, NR^{c32}C(O)NR^{c32}R^{d32}, NR^{c32}S(O)R^{b32}, NR^{c32}S(O)₂R^{b32}, NR^{c32}S(O)₂NR^{c32}R^{d32}, S(O)R^{b32}, S(O)NR^{c32}R^{d32}, S(O)₂R^{b32}, S(O)₂NR^{c32}R^{d32}, and BR^{h32}Rⁱ³²; wherein said C₁₋₆ alkyl, C₂₋₆ alkenyl, C₂₋₆ alkynyl, C₃₋₆ cycloalkyl, phenyl, 5-6 membered heteroaryl and 4-7 membered heterocycloalkyl, are each optionally substituted with 1, 2, 3, or 4 substituents independently selected from R^{g};
each R⁵⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a50}, SR^{a50}, C(O)R^{b50}, C(O)NR^{c50}R^{d50}, C(O)OR^{a50}, OC(O)R^{b50}, OC(O)NR^{c50}R^{d50}, NR^{c50}R^{d50}, NR^{c50}C(O)R^{b50}, NR^{c50}C(O)OR^{a50}, NR^{c50}C(O)NR^{c50}R^{d50}, NR^{c50}S(O)R^{b50}, NR^{c50}S(O)₂R^{b50}, NR^{c50}S(O)₂NR^{c50}R^{d50}, S(O)R^{b50}, S(O)NR^{c50}R^{d50}, S(O)₂R^{b50}, S(O)₂NR^{c50}R^{d50}, and BR^{h50}Rⁱ⁵⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁵¹;
each R⁵¹ is independently selected from C₁₋₆ alkyl, C₂₋₆ alkenyl, C₂₋₆ alkynyl, C₁₋₆ haloalkyl, C₃₋₆ cycloalkyl, C₆₋₁₀ aryl, 5-6 membered heteroaryl, 4-7 membered heterocycloalkyl, halo, D, CN, OR^{a51}, SR^{a51}, C(O)R^{b51}, C(O)NR^{c51}R^{d51}, C(O)OR^{a51}, OC(O)R^{b51}, OC(O)NR^{c51}R^{d51}, NR^{c51}R^{d51}, NR^{c51}C(O)R^{b51}, NR^{c51}C(O)OR^{a51}, NR^{c51}C(O)NR^{c51}R^{d51}, NR^{c51}S(O)R^{b51}, NR^{c51}S(O)₂R^{b51}, NR^{c51}S(O)₂NR^{c51}R^{d51}, S(O)R^{b51}, S(O)NR^{c51}R^{d51}, S(O)₂R^{b51}, S(O)₂NR^{c51}R^{d51}, and BR^{h51}Rⁱ⁵¹; wherein said C₁₋₆ alkyl, C₂₋₆ alkenyl, C₂₋₆ alkynyl, C₃₋₆ cycloalkyl, C₆₋₁₀ aryl, 5-6 membered heteroaryl and 4-7 membered heterocycloalkyl, are each optionally substituted with 1, 2, 3, or 4 substituents independently selected from R⁵²;
each R⁵² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a52}, SR^{a52}, C(O)R^{b52}, C(O)NR^{c52}R^{d52}, C(O)OR^{a52}, OC(O)R^{b52}, OC(O)NR^{c52}R^{d52}, NR^{c52}R^{d52}, NR^{c52}C(O)R^{b52}, NR^{c52}C(O)OR^{a52}, NR^{c52}C(O)NR^{c52}R^{d52}, NR^{c52}S(O)R^{b52}, NR^{c52}S(O)₂R^{b52}, NR^{c52}S(O)₂NR^{c52}R^{d52}, S(O)R^{b52}, S(O)NR^{c52}R^{d52}, S(O)₂R^{b52}, S(O)₂NR^{c52}R^{d52}, and BR^{h52}Rⁱ⁵²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R⁶⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a60}, SR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, NR^{c60}C(O)NR^{c60}R^{d60}, NR^{c60}S(O)R^{b60}, NR^{c60}S(O)₂R^{b60}, NR^{c60}S(O)₂NR^{c60}R^{d60}, S(O)R^{b60}, S(O)NR^{c60}R^{d60}, S(O)₂R^{b60}, S(O)₂NR^{c60}R^{d60}, and BR^{h60}Rⁱ⁶⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶¹;
each R⁶¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a61}, SR^{a61}, C(O)R^{b61}, C(O)NR^{c61}R^{d61}, C(O)OR^{a61}, OC(O)R^{b61}, OC(O)NR^{c61}R^{d61}, NR^{c61}R^{d61}, NR^{c61}C(O)R^{b61}, NR^{c61}C(O)OR^{a61}, NR^{c61}C(O)NR^{c61}R^{d61} , NR^{c61}S(O)R^{b61}, NR^{c61}S(O)₂R^{b61}, NR^{c61}S(O)₂NR^{c61}R^{d61}, S(O)R^{b61}, S(O)NR^{c61}R^{d61}, S(O)₂R^{b61}, S(O)₂NR^{c61}R^{d61}, and BR^{h61}Rⁱ⁶¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶²;
each R⁶² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, ORa62, SR^{a62}, C(O)R^{b62}, C(O)NR^{c62}R^{d62}, C(O)OR^{a62} , OC(O)R^{b62}, OC(O)NR^{c62}R^{d62}, NR^{c62}R^{d62}, NR^{c62}C(O)R^{b62}, NR^{c62}C(O)OR^{a62}, NR^{c62}C(O)NR^{c62}R^{d62}, NR^{c62}S(O)R^{b62}, NR^{c62}S(O)₂R^{b62}, NR^{c62}S(O)₂NR^{c62}R^{d62}, S(O)R^{b62}, S(O)NR^{c62}R^{d62}, S(O)₂R^{b62}, S(O)₂NR^{c62}R^{d62}, and BR^{h62}Rⁱ⁶²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R⁷⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a70}, SRa⁷⁰, C(O)R^{b70}, C(O)NR^{c70}R^{d70}, C(O)OR^{a70}, OC(O)R^{b70}, OC(O)NR^{c70}R^{d70}, NR^{c70}R^{d70}, NR^{c70}C(O)R^{b70}, NR^{c70}C(O)OR^{a70}, NR^{c70}C(O)NR^{c70}R^{d70}, NR^{c70}S(O)R^{b70}, NR^{c70}S(O)₂R^{b70}, NR^{c70}S(O)₂NR^{c70}R^{d70}, S(O)R^{b70}, S(O)NR^{c70}R^{d70}, S(O)₂R^{b70}, S(O)₂NR^{c70}R^{d70}, and BR^{h70}Rⁱ⁷⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷¹;
each R⁷¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a71}, SR^{a71}, C(O)R^{b71}, C(O)NR^{c71}R^{d71}, C(O)OR^{a71}, OC(O)R^{b71}, OC(O)NR^{c71}R^{d71}, NR^{c71}R^{d71}, NR^{c71}C(O)R^{b71}, NR^{c71}C(O)OR^{a71}, NR^{c71}C(O)NR^{c71}R^{d71}, NR^{c71}S(O)R^{b71}, NR^{c71}S(O)₂R^{b71}, NR^{c71}S(O)₂NR^{c71}R^{d71}, S(O)R^{b71}, S(O)NR^{c71}R^{d71}, S(O)₂R^{b71}, S(O)₂NR^{c71}R^{d71}, and BR^{h71}Rⁱ⁷¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷²;
each R⁷² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a72}, SR^{a72}, C(O)R^{b72}, C(O)NR^{c72}R^{d72}, C(O)OR^{a72}, OC(O)R^{b72}, OC(O)NR^{c72}R^{d72}, NR^{c72}R^{d72}, NR^{c72}C(O)R^{b72}, NR^{c72}C(O)OR^{a72}, NR^{c72}C(O)NR^{c72}R^{d72}, NR^{c72}S(O)R^{b72}, NR^{c72}S(O)₂R^{b72}, NR^{c}72S(O)₂NR^{c72}R^{d72}, S(O)R^{b72}, S(O)NR^{c72}R^{d72}, S(O)₂R^{b72}, S(O)₂NR^{c72}R^{d72}, and BR^{h72}Rⁱ⁷²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R^{a1}, R^{b1}, R^{c1}, and R^{d1} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R^{g};
or any R^{c1} and R^{d1} attached to the same N atom, together with the N atom to which they are attached, form a 4-, 5-, 6- or 7-membered heterocycloalkyl group optionally substituted with 1, 2 or 3 substituents independently selected from R^{g};
each R^{h1} and Rⁱ¹ is independently selected from OH, C₁₋₆ alkoxy, and C₁₋₆ haloalkoxy; or any R^{h1} and Rⁱ¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a2}, R^{b2}, R^{c2} and R^{d2} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²²;
or any R^{c2} and R^{d2} attached to the same N atom, together with the N atom to which they are attached, form a 4-, 5-, 6- or 7-membered heterocycloalkyl group optionally substituted with 1, 2, 3, or 4 substituents independently selected from R²²;
each R^{e2} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h2} and Rⁱ² is independently selected from OH, C₁₋₆ alkoxy, and C₁₋₆ haloalkoxy; or any R^{h2} and Rⁱ² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{e3} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h3} and Rⁱ³ is independently selected from OH, C₁₋₆ alkoxy, and C₁₋₆ haloalkoxy; or any R^{h3} and Rⁱ³ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{j3} is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶⁰;
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R⁶⁰;
each R^{e6} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h6} and Rⁱ⁶ is independently selected from OH, C₁₋₆ alkoxy, and C₁₋₆ haloalkoxy; or any R^{h6} and Rⁱ⁶ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a7}, R^{b7}, R^{c7} and R^{d7} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷⁰;
or any R^{c7} and R^{d7} attached to the same N atom, together with the N atom to which they are attached, form a 4-, 5-, 6- or 7-membered heterocycloalkyl group optionally substituted with 1, 2, 3, or 4 substituents independently selected from R⁷⁰;
each R^{e7} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h7} and Rⁱ⁷ is independently selected from OH, C₁₋₆ alkoxy, and C₁₋₆ haloalkoxy; or any R^{h7} and Rⁱ⁷ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R¹¹;
or any R^{c10} and R^{d10} attached to the same N atom, together with the N atom to which they are attached, form a 4-, 5-, 6- or 7-membered heterocycloalkyl group optionally substituted with 1, 2, 3, or 4 substituents independently selected from R¹¹;
each R^{e10} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h10} and Rⁱ¹⁰ is independently selected from OH, C₁₋₆ alkoxy, and C₁₋₆ haloalkoxy; or any R^{h10} and Rⁱ¹⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a11}, R^{b11}, R^{c11} and R^{d11}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R¹²;
or any R^{c11} and R^{d11} attached to the same N atom, together with the N atom to which they are attached, form a 4-, 5-, 6- or 7-membered heterocycloalkyl group optionally substituted with 1, 2 or 3 substituents independently selected from R¹²;
each R^{h11} and Rⁱ¹¹ is independently selected from OH, C₁₋₆ alkoxy, and C₁₋₆ haloalkoxy; or any R^{h11} and Rⁱ¹¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a12}, R^{b12}, R^{c12} and R^{d12}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
each R^{h12} and Rⁱ¹² is independently selected from OH, C₁₋₆ alkoxy, and C₁₋₆ haloalkoxy; or any R^{h12} and Rⁱ¹² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²¹;
or any R^{c20} and R^{d20} attached to the same N atom, together with the N atom to which they are attached, form a 4-, 5-, 6- or 7-membered heterocycloalkyl group optionally substituted with 1, 2, 3, or 4 substituents independently selected from R²¹;
each R^{e20} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h20} and Rⁱ²⁰ is independently selected from OH, C₁₋₆ alkoxy, and C₁₋₆ haloalkoxy; or any R^{h20} and Rⁱ²⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a21}, R^{b21}, R^{c21} and R^{d21}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R^{g};
or any R^{c21} and R^{d21} attached to the same N atom, together with the N atom to which they are attached, form a 4-, 5-, 6- or 7-membered heterocycloalkyl group optionally substituted with 1, 2 or 3 substituents independently selected from R^{g};
each R^{h21} and Rⁱ²¹ is independently selected from OH, C₁₋₆ alkoxy, and C₁₋₆ haloalkoxy; or any R^{h21} and Rⁱ²¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a22}, R^{b22}, R^{c22} and R^{d22} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²³;
or any R^{c22} and R^{d22} attached to the same N atom, together with the N atom to which they are attached, form a 4-, 5-, 6- or 7-membered heterocycloalkyl group optionally substituted with 1, 2, 3, or 4 substituents independently selected from R²³;
each R^{h22} and Rⁱ²² is independently selected from OH, C₁₋₆ alkoxy, and C₁₋₆ haloalkoxy; or any R^{h22} and Rⁱ²² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a23}, R^{b23}, R^{c23} and R^{d23}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R²⁴;
or any R^{c23} and R^{d23} attached to the same N atom, together with the N atom to which they are attached, form a 4-, 5-, 6- or 7-membered heterocycloalkyl group optionally substituted with 1, 2 or 3 substituents independently selected from R²⁴;
each R^{h23} and Rⁱ²³ is independently selected from OH, C₁₋₆ alkoxy, and C₁₋₆ haloalkoxy; or any R^{h23} and Rⁱ²³ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a24}, R^{b24}, R^{c24} and R^{d24}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
each R^{h24} and Rⁱ²⁴ is independently selected from OH, C₁₋₆ alkoxy, and C₁₋₆ haloalkoxy; or any R^{h24} and Rⁱ²⁴ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{h30} and Rⁱ³⁰ is independently selected from OH, C₁₋₆ alkoxy, and C₁₋₆ haloalkoxy; or any R^{h30} and Rⁱ³⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a31}, R^{b31}, R^{c31} and R^{d31}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R³²;
or any R^{c31} and R^{d31} attached to the same N atom, together with the N atom to which they are attached, form a 4-, 5-, 6- or 7-membered heterocycloalkyl group optionally substituted with 1, 2 or 3 substituents independently selected from R³²;
each R^{h31} and Rⁱ³¹ is independently selected from OH, C₁₋₆ alkoxy, and C₁₋₆ haloalkoxy; or any R^{h31} and Rⁱ³¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a32}, R^{b32}, R^{c32} and R^{d32}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c32} and R^{d32} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h32} and Rⁱ³² is independently selected from OH, C₁₋₆ alkoxy, and C₁₋₆ haloalkoxy; or any R^{h32} and Rⁱ³² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a50,} R^{b50}, R^{c50} and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁵¹;
or any R^{c50} and R^{d50} attached to the same N atom, together with the N atom to which they are attached, form a 4-, 5-, 6- or 7-membered heterocycloalkyl group optionally substituted with 1, 2 or 3 substituents independently selected from R⁵¹;
each R^{h50} and Rⁱ⁵⁰ is independently selected from OH, C₁₋₆ alkoxy, and C₁₋₆ haloalkoxy; or any R^{h50} and Rⁱ⁵⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a51}, R^{b51}, R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁵²;
or any R^{c51} and R^{d51} attached to the same N atom, together with the N atom to which they are attached, form a 4-, 5-, 6- or 7-membered heterocycloalkyl group optionally substituted with 1, 2, 3, or 4 substituents independently selected from R⁵²;
each R^{h51} and Rⁱ⁵¹ is independently selected from OH, C₁₋₆ alkoxy, and C₁₋₆ haloalkoxy; or any R^{h51} and Rⁱ⁵¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a52}, R^{b52}, R^{c52} and R^{d52}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c52} and R^{d52} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h52} and Rⁱ⁵² is independently selected from OH, C₁₋₆ alkoxy, and C₁₋₆ haloalkoxy; or any R^{h52} and Rⁱ⁵² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a60}, R^{b60}, R^{c60} and R^{d60} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶¹;
or any R^{c60} and R^{d60} attached to the same N atom, together with the N atom to which they are attached, form a 4-, 5-, 6- or 7-membered heterocycloalkyl group optionally substituted with 1, 2, 3, or 4 substituents independently selected from R⁶¹;
each R^{h60} and Rⁱ⁶⁰ is independently selected from OH, C₁₋₆ alkoxy, and C₁₋₆ haloalkoxy; or any R^{h60} and Rⁱ⁶⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a61}, R^{b61}, R^{c61} and R^{d61}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁶²;
or any R^{c61} and R^{d61} attached to the same N atom, together with the N atom to which they are attached, form a 4-, 5-, 6- or 7-membered heterocycloalkyl group optionally substituted with 1, 2 or 3 substituents independently selected from R⁶²;
each R^{h61} and Rⁱ⁶¹ is independently selected from OH, C₁₋₆ alkoxy, and C₁₋₆ haloalkoxy; or any R^{h61} and Rⁱ⁶¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a62}, R^{b62}, R^{c62} and R^{d62}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c62} and R^{d62} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h62} and Rⁱ⁶² is independently selected from OH, C₁₋₆ alkoxy, and C₁₋₆ haloalkoxy; or any R^{h62} and Rⁱ⁶² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a70}, R^{b70}, R^{c70} and R^{d70} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷¹;
or any R^{c70} and R^{d70} attached to the same N atom, together with the N atom to which they are attached, form a 4-, 5-, 6- or 7-membered heterocycloalkyl group optionally substituted with 1, 2, 3, or 4 substituents independently selected from R⁷¹;
each R^{h70} and Rⁱ⁷⁰ is independently selected from OH, C₁₋₆ alkoxy, and C₁₋₆ haloalkoxy; or any R^{h70} and Rⁱ⁷⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a71}, R^{b71}, R^{c71} and R^{d71}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁷²;
or any R^{c71} and R^{d71} attached to the same N atom, together with the N atom to which they are attached, form a 4-, 5-, 6- or 7-membered heterocycloalkyl group optionally substituted with 1, 2 or 3 substituents independently selected from R⁷²;
each R^{h71} and Rⁱ⁷¹ is independently selected from OH, C₁₋₆ alkoxy, and C₁₋₆ haloalkoxy; or any R^{h71} and Rⁱ⁷¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a72}, R^{b72}, R^{c72} and R^{d72}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c72} and R^{d72} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h72} and Rⁱ⁷² is independently selected from OH, C₁₋₆ alkoxy, and C₁₋₆ haloalkoxy; or any R^{h72} and Rⁱ⁷² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl; and
each R^{g} is independently selected from D, OH, NO₂, CN, halo, C₁₋₆ alkyl, C₂₋₆ alkenyl, C₂₋₆ alkynyl, C₁₋₆ haloalkyl, C₃₋₆ cycloalkyl, C₃₋₆ cycloalkyl-C₁₋₂ alkylene, C₁₋₆ alkoxy, C₁₋₆ haloalkoxy, C₁₋₃ alkoxy-C₁₋₃ alkyl, C₁₋₃ alkoxy-C₁₋₃ alkoxy, HO-C₁₋₃ alkoxy, HO-C₁₋₃ alkyl, cyano-C₁₋₃ alkyl, H₂N-C₁₋₃ alkyl, amino, C₁₋₆ alkylamino, di(C₁₋₆ alkyl)amino, thio, C₁₋₆ alkylthio, C₁₋₆ alkylsulfinyl, C₁₋₆ alkylsulfonyl, carbamyl, C₁₋₆ alkylcarbamyl, di(C₁₋₆ alkyl)carbamyl, carboxy, C₁₋₆ alkylcarbonyl, C₁₋₆ alkoxycarbonyl, C₁₋₆ alkylcarbonylamino, C₁₋₆ alkoxycarbonylamino, C₁₋₆ alkylcarbonyloxy, aminocarbonyloxy, C₁₋₆ alkylaminocarbonyloxy, di(C₁₋₆ alkyl)aminocarbonyloxy, C₁₋₆ alkylsulfonylamino, aminosulfonyl, C₁₋₆ alkylaminosulfonyl, di(C₁₋₆ alkyl)aminosulfonyl, aminosulfonylamino, C₁₋₆ alkylaminosulfonylamino, di(C₁₋₆ alkyl)aminosulfonylamino, aminocarbonylamino, C₁₋₆ alkylaminocarbonylamino, and di(C₁₋₆ alkyl)aminocarbonylamino;
provided that, Cy¹ is other than 3,5-dimethylisoxazol-4-yl, 3,5-dimethyl-1H-pyrazol-4-yl or 4-(1-oxo-2-propen-1-yl)phenyl.

In an embodiment,
each - - - represents a single bond or a double bond;
X is N or CR⁷
Y is N or C;
R¹ is selected from H, D, C₁₋₆ alkyl, C₁₋₆ haloalkyl, halo, CN, OR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)OR^{a1}, S(O)₂R^{b1}, and S(O)₂NR^{c1}R^{d1}; wherein said C₁₋₆ alkyl, is optionally substituted with 1, 2, 3, or 4 substituents independently selected from R^{g};
R² is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a2}, SR^{a2}, C(O)R^{b2}, C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)₂R^{b2}, NR^{c2}S(O)₂NR^{c2}R^{d2}, S(O)₂R^{b2}, and S(O)₂NR^{c2}R^{d2}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, 3 or 4 substituents independently selected from R¹⁰;
when R³N---CR⁴ is a single bond, then R⁴ is =O; and
R³ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R³N---CR⁴ is a double bond, then R³ is absent; and
R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, NR^{c3}C(O)NR^{c3}R^{d3}, NR^{c3}S(O)₂R^{b3}, NR^{c3}S(O)₂NR^{c3}R^{d3}, S(O)₂R^{b3}, and S(O)₂NR^{c3}R^{d3}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R⁵N---YR⁶ is a single bond and Y is C, then YR⁶ is C=O; and
R⁵ is selected from H, C₁₋₆ alkyl, C₁₋₆ haloalkyl, C₃₋₁₀ cycloalkyl, and 4-10 membered heterocycloalkyl; wherein said C₁₋₆ alkyl, C₃₋₁₀ cycloalkyl, and 4-10 membered heterocycloalkyl, are each optionally substituted with 1, 2, 3, or 4 substituents independently selected from R⁵⁰;
when R⁵N---YR⁶ is a double bond and Y is N, then R⁵ and R⁶ are absent;
when R⁵N---YR⁶ is a double bond and Y is C, then R⁵ is absent; and
R⁶ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, S(O)₂R^{b6}, and S(O)₂NR^{c6}R^{d6}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶⁰;
R⁷ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl, halo, D, CN, OR^{a7}, SR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7}, NR^{c7}S(O)₂R^{b7}, NR^{c7}S(O)₂NR^{c7}R^{d7}, S(O)₂R^{b7}, and S(O)₂NR^{c7}R^{d7}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁷⁰;
Cy² is selected from 4-14 membered heterocycloalkyl, each has at least one ring-forming carbon atom and 1, 2, 3, or 4 ring-forming heteroatoms independently selected from N, O, and S; wherein the N and S are optionally oxidized; wherein a ring-forming carbon atom of 4-14 membered heterocycloalkyl is optionally substituted by oxo to form a carbonyl group; and wherein the 4-14 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, halo, D, CN, OR^{a10}, SR^{a10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, NR^{c10}C(O)NR^{c10}R^{d10}, NR^{c10}S(O)₂R^{b10}, NR^{c10}S(O)₂NR^{c10}R^{d10}, S(O)₂R^{b10}, and S(O)₂NR^{c10}R^{d10}; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R¹¹;
each R¹¹ is independently selected from C₁₋₆ alkyl, C₁₋₆ haloalkyl, halo, D, CN, OR^{a11}, C(O)R^{b11}, C(O)NR^{c11}R^{d11}, C(O)OR^{a11}, OC(O)R^{b11}, OC(O)NR^{c11}R^{d11}, NR^{c11}R^{d11}, NR^{c11}C(O)R^{b11}, NR^{c11}C(O)OR^{a11}, S(O)₂R^{b11}, and S(O)₂NR^{c11}R^{d11};
each R²⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, halo, D, CN, OR^{a20}, SR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, NR^{c20}C(O)NR^{c20}R^{d20}, NR^{c20}S(O)₂R^{b20}, NR^{c20}S(O)₂NR^{c20}R^{d20}, S(O)2R^{b20}, and S(O)₂NR^{c20}R^{d20}; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R²¹;
each R²¹ is independently selected from C₁₋₆ alkyl, C₂₋₆ alkenyl, C₂₋₆ alkynyl, C₁₋₆ haloalkyl, halo, D, CN, OR^{a21}, C(O)R^{b21}, C(O)NR^{c21}R^{d21}, C(O)OR^{a21}, OC(O)R^{b21}, OC(O)NR^{c21}R^{d21}, NR^{c21}R^{d21}, NR^{c21}C(O)R^{b21}, NR^{c21}C(O)OR^{a21}, S(O)₂R^{b21}, and S(O)₂NR^{c21}R^{d21}; wherein said C₁₋₆ alkyl, C₂₋₆ alkenyl, and C₂₋₆ alkynyl, are each optionally substituted with 1, 2, 3, or 4 substituents independently selected from R^{g};
each R²² is independently selected from C₁₋₆ alkyl, C₁₋₆ haloalkyl, halo, D, CN, OR^{a22}, C(O)R^{b22}, C(O)NR^{c22}R^{d22}, C(O)OR^{a22}, OC(O)R^{b22}, OC(O)NR^{c22}R^{d22}, NR^{c22}R^{d22}, NR^{c22}C(O)R^{b22}, NR^{c22}C(O)OR^{a22}, S(O)₂R^{b22}, and S(O)₂NR^{c22}R^{d22};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a30}, SR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, S(O)₂R^{b30}, and S(O)₂NR^{c30}R^{d30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl, halo, D, CN, OR^{a31}, C(O)R^{b31}, C(O)NR^{c31}R^{d31}, C(O)OR^{a31}, OC(O)R^{b31}, OC(O)NR^{c31}R^{d31}, NR^{c31}R^{d31}, NR^{c31}C(O)R^{b31}, NR^{c31}C(O)OR^{a31}, S(O)₂R^{b31}, and S(O)₂NR^{c31}R^{d31}; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R³²;
each R³² is independently selected from C₁₋₆ alkyl, C₁₋₆ haloalkyl, halo, D, CN, OR^{a32}, C(O)R^{b32}, C(O)NR^{c32}R^{d32}, C(O)OR^{a32}, OC(O)R^{b32}, OC(O)NR^{c31}R^{d32}, NR^{c31}R^{d32}, NR^{c32}C(O)R^{b32}, NR^{c32}C(O)OR^{a32}, S(O)₂R^{b32}, and S(O)₂NR^{c32}R^{d32};
each R⁵⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a50}, C(O)R^{b50}, C(O)NR^{c50}R^{d50}, C(O)OR^{a50}, OC(O)R^{b50}, OC(O)NR^{c50}R^{d50}, NR^{c50}R^{d50}, NR^{c50}C(O)R^{b50}, NR^{c50}C(O)OR^{a50}, S(O)₂R^{b50}, and S(O)₂NR^{c50}R^{d50}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁵¹;
each R⁵¹ is independently selected from C₁₋₆ alkyl, C₁₋₆ haloalkyl, halo, D, CN, OR^{a51}, C(O)R^{b51}, C(O)NR^{c51}R^{d51}, C(O)OR^{a51}, OC(O)R^{b51}, OC(O)NR^{c51}R^{d51}, NR^{c51}R^{d51}, NR^{c51}C(O)R^{b51}, NR^{c51}C(O)OR^{a51}, S(O)₂R^{b51}, and S(O)₂NR^{c51}R^{d51}; wherein said C₁₋₆ alkyl, are each optionally substituted with 1, 2, 3, or 4 substituents independently selected from R⁵²;
each R⁵² is independently selected from C₁₋₆ alkyl, C₁₋₆ haloalkyl, halo, D, CN, OR^{a52}, C(O)R^{b52}, C(O)NR^{c52}R^{d52}, C(O)OR^{a52}, OC(O)R^{b52}, OC(O)NR^{c52}R^{d52}, NR^{c52}R^{d52}, NR^{c52}C(O)R^{b52}, NR^{c52}C(O)OR^{a52}, S(O)₂R^{b52}, and S(O)₂NR^{c52}R^{d52};
each R⁶⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a60}, SR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, S(O)₂R^{b60}, and S(O)₂NR^{c60}R^{d60}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁶¹;
each R⁶¹ is independently selected from C₁₋₆ alkyl, C₁₋₆ haloalkyl, halo, D, CN, OR^{a61}, C(O)R^{b61}, C(O)NR^{c61}R^{d61}, C(O)OR^{a61}, OC(O)R^{b61}, OC(O)NR^{c61}R^{d61}, NR^{c61}R^{d61}, NR^{c61}C(O)R^{b61}, NR^{c61}C(O)OR^{a61}, S(O)₂R^{b61}, and S(O)₂NR^{c61}R^{d61};
each R⁷⁰ is independently selected from C₁₋₆ alkyl, C₁₋₆ haloalkyl, halo, D, CN, OR^{a70}, C(O)R^{b70}, C(O)NR^{c70}R^{d70}, C(O)OR^{a70}, OC(O)R^{b70}, OC(O)NR^{c70}R^{d70}, NR^{c70}R^{d70}, NR^{c70}C(O)R^{b70}, NR^{c70}C(O)OR^{a70}, S(O)₂R^{b70}, and S(O)₂NR^{c70}R^{d70};
each R^{a1}, R^{b1}, R^{c1}, and R^{d1} is independently selected from H, C₁₋₆ alkyl, C₁₋₆ haloalkyl; wherein said C₁₋₆ alkyl, are each optionally substituted with 1, 2, 3, or 4 substituents independently selected from R^{g};
each R^{a2}, R^{b2}, R^{c2} and R^{d2} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²²;
or any R^{c2} and R^{d2} attached to the same N atom, together with the N atom to which they are attached, form a 4-, 5-, 6- or 7-membered heterocycloalkyl group optionally substituted with 1, 2, 3, or 4 substituents independently selected from R²²;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{j3} is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶⁰;
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R⁶⁰;
each R^{a7}, R^{b7}, R^{c7} and R^{d7} is independently selected from H, C₁₋₆ alkyl, and C₁₋₆ haloalkyl; wherein said C₁₋₆ alkyl, are each optionally substituted with 1, 2, 3, or 4 substituents independently selected from R⁷⁰;
each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl; wherein said C₁₋₆ alkyl, C₂₋₆ alkenyl, C₂₋₆ alkynyl, C₃₋₁₀ cycloalkyl, and 4-10 membered heterocycloalkyl are each optionally substituted with 1, 2, 3, or 4 substituents independently selected from R¹¹;
or any R^{c10} and R^{d10} attached to the same N atom, together with the N atom to which they are attached, form a 4-, 5-, 6- or 7-membered heterocycloalkyl group optionally substituted with 1, 2, 3, or 4 substituents independently selected from R¹¹;
each R^{a11}, R^{b11}, R^{c11} and R^{d11}, is independently selected from H, C₁₋₆ alkyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R²¹;
or any R^{c20} and R^{d20} attached to the same N atom, together with the N atom to which they are attached, form a 4-, 5-, 6- or 7-membered heterocycloalkyl group optionally substituted with 1, 2, 3, or 4 substituents independently selected from R²¹;
each R^{a21}, R^{b21}, R^{c21} and R^{d21}, is independently selected from H, C₁₋₆ alkyl, C₂₋₆ alkenyl, C₂₋₆ alkynyl, C₁₋₆ haloalkyl; wherein said C₁₋₆ alkyl C₂₋₆ alkenyl, and C₂₋₆ alkynyl, are each optionally substituted with 1, 2, 3, or 4 substituents independently selected from R^{g};
each R^{a22}, R^{b22}, R^{c22} and R^{d22} is independently selected from H, C₁₋₆ alkyl, and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{a31}, R^{b31}, R^{c31} and R^{d31}, is independently selected from H, C₁₋₆ alkyl, C₂₋₆ alkenyl, C₂₋₆ alkynyl, C₁₋₆ haloalkyl, C₃₋₆ cycloalkyl, and 4-7 membered heterocycloalkyl; wherein said C₁₋₆ alkyl C₂₋₆ alkenyl, C₂₋₆ alkynyl, C₃₋₆ cycloalkyl, and 4-7 membered heterocycloalkyl are each optionally substituted with 1, 2, 3, or 4 substituents independently selected from R³²;
or any R^{c31} and R^{d31} attached to the same N atom, together with the N atom to which they are attached, form a 4-, 5-, 6- or 7-membered heterocycloalkyl group optionally substituted with 1, 2 or 3 substituents independently selected from R³²;
each R^{a32}, R^{b32}, R^{c32} and R^{d32}, is independently selected from H, C₁₋₆ alkyl, and C₁₋₆ haloalkyl;
each R^{a50}, R^{b50}, R^{c50}, and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁵¹;
or any R^{c50} and R^{d50} attached to the same N atom, together with the N atom to which they are attached, form a 4-, 5-, 6- or 7-membered heterocycloalkyl group optionally substituted with 1, 2 or 3 substituents independently selected from R⁵¹;
each R^{a51}, R^{b51}, R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁵²;
each R^{a52}, R^{b52}, R^{c52} and R^{d52}, is independently selected from H, C₁₋₆ alkyl, and C₁₋₆ haloalkyl;
each R^{a60}, R^{b60}, R^{c60} and R^{d60} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶¹;
or any R^{c60} and R^{d60} attached to the same N atom, together with the N atom to which they are attached, form a 4-, 5-, 6- or 7-membered heterocycloalkyl group optionally substituted with 1, 2, 3, or 4 substituents independently selected from R⁶¹;
each R^{a61}, R^{b61}, R^{c61} and R^{d61}, is independently selected from H, C₁₋₆ alkyl, and C₁₋₆ haloalkyl;
each R^{a70}, R^{b70}, R^{c70} and R^{d70} is independently selected from H, C₁₋₆ alkyl, and C₁₋₆ haloalkyl; and
each R^{g} is independently selected from D, OH, CN, halo, C₁₋₆ alkyl, C₂₋₆ alkenyl, C₂₋₆ alkynyl, C₁₋₆ haloalkyl, C₃₋₆ cycloalkyl, C₃₋₆ cycloalkyl-C₁₋₂ alkylene, C₁₋₆ alkoxy, C₁₋₆ haloalkoxy, C₁₋₃ alkoxy-C₁₋₃ alkyl, C₁₋₃ alkoxy-C₁₋₃ alkoxy, HO-C₁₋₃ alkoxy, HO-C₁₋₃ alkyl, cyano-C₁₋₃ alkyl, H₂N-C₁₋₃ alkyl, amino, C₁₋₆ alkylamino, di(C₁₋₆ alkyl)amino, C₁₋₆ alkylthio, C₁₋₆ alkylsulfonyl, C₁₋₆ alkylcarbamyl, di(C₁₋₆ alkyl)carbamyl, carboxy, C₁₋₆ alkylcarbonyl, C₁₋₆ alkoxycarbonyl, C₁₋₆ alkylcarbonylamino, C₁₋₆ alkoxycarbonylamino, C₁₋₆ alkylcarbonyloxy, and di(C₁₋₆ alkyl)aminosulfonyl.

In another embodiment,
each --- represents a single bond or a double bond;
X is N or CR⁷;
Y is N or C;
R¹ is selected from H, D, C₁₋₆ alkyl, C₁₋₆ haloalkyl, halo, and CN;
R² is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a2}, and NR^{c2}R^{d2};
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, 3 or 4 substituents independently selected from R¹⁰;
when R³N---CR⁴ is a single bond, then R⁴ is =O; and
R³ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R³N---CR⁴ is a double bond, then R³ is absent; and
R⁴ is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, and S(O)₂R^{b3}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R⁵N---YR⁶ is a single bond and Y is C, then YR⁶ is C=O; and
R⁵ is selected from H, C₁₋₆ alkyl, and C₁₋₆ haloalkyl; wherein said C₁₋₆ alkyl, are each optionally substituted with 1, 2, 3, or 4 substituents independently selected from R⁵⁰;
when R⁵N---YR⁶ is a double bond and Y is N, then R⁵ and R⁶ are absent;
when R⁵N---YR⁶ is a double bond and Y is C, then R⁵ is absent; and
R⁶ is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, and S(O)₂R^{b6}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁶⁰;
R⁷ is selected from H, C₁₋₆ alkyl, C₁₋₆ haloalkyl, halo, D, CN, OR^{a7}, and NR^{c7}R^{d7};
Cy² is selected from 4-10 membered heterocycloalkyl, each has at least one ring-forming carbon atom and 1, 2, 3, or 4 ring-forming heteroatoms independently selected from N, O, and S; wherein a ring-forming carbon atom of 4-10 membered heterocycloalkyl is optionally substituted by oxo to form a carbonyl group; and wherein the 4-10 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, halo, D, CN, OR^{a10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, and S(O)₂R^{b10};
each R²⁰ is independently selected from C₁₋₆ alkyl, C₂₋₆ alkenyl, C₂₋₆ alkynyl, C₁₋₆ haloalkyl, halo, D, CN, OR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, and S(O)₂R^{b20}; wherein said C₁₋₆ alkyl, C₂₋₆ alkenyl, and C₂₋₆ alkynyl, are each optionally substituted with 1, 2, 3, or 4 substituents independently selected from R²¹;
each R²¹ is independently selected from C₁₋₆ alkyl, C₁₋₆ haloalkyl, halo, D, CN, OR^{a21}, and NR^{c21}R^{d21};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c03}C(O)R^{b30}, NR^{c30}C(O)OR^{a30}, and S(O)₂R^{b30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³¹;
each R³¹ is independently selected from C₁₋₆ alkyl, C₁₋₆ haloalkyl, halo, D, CN, OR^{a31}, and NR^{c31}R^{d31};
each R⁵⁰ is independently selected from C₁₋₆ alkyl, C₁₋₆ haloalkyl, C₃₋₁₀ cycloalkyl, 4-10 membered heterocycloalkyl, C₆₋₁₀aryl, 5-10 membered heteroaryl, halo, D, CN, OR^{a50}, and NR^{c52}R^{d52}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁵¹;
each R⁵¹ is independently selected from C₁₋₆ alkyl, C₁₋₆ haloalkyl, halo, D, CN, OR^{a51}, and NR^{c51}R^{d51};
each R⁶⁰ is independently selected from C₁₋₆ alkyl, C₁₋₆ haloalkyl, C₃₋₁₀ cycloalkyl, 4-10 membered heterocycloalkyl, C₆₋₁₀aryl, 5-10 membered heteroaryl, halo, D, CN, OR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, and S(O)₂R^{b60};
each R^{a2}, R^{c2} and R^{d2} is independently selected from H, C₁₋₆ alkyl, C₁₋₆ haloalkyl, C₃₋₁₀ cycloalkyl, 4-10 membered heterocycloalkyl, C₆₋₁₀aryl and 5-10 membered heteroaryl;
or any R^{c2} and R^{d2} attached to the same N atom, together with the N atom to which they are attached, form a 4-, 5-, 6- or 7-membered heterocycloalkyl group;
each R^{a3}, R^{b3}, R^{c3} and R^{d3} is independently selected from H, C₁₋₆ alkyl, C₁₋₆ haloalkyl, C₃₋₁₀ cycloalkyl, 4-10 membered heterocycloalkyl, C₆₋₁₀aryl and 5-10 membered heteroaryl; wherein said C₁₋₆ alkyl, C₃₋₁₀ cycloalkyl, 4-10 membered heterocycloalkyl, C₆₋₁₀aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{j3} is independently selected from C₁₋₆ alkyl, C₁₋₆ haloalkyl, C₃₋₁₀ cycloalkyl, 4-10 membered heterocycloalkyl, C₆₋₁₀aryl and 5-10 membered heteroaryl; wherein said C₁₋₆ alkyl, C₃₋₁₀ cycloalkyl, 4-10 membered heterocycloalkyl, C₆₋₁₀aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₁₋₆ haloalkyl, C₃₋₁₀ cycloalkyl, 4-10 membered heterocycloalkyl, C₆₋₁₀aryl and 5-10 membered heteroaryl; wherein said C₁₋₆ alkyl, C₃₋₁₀ cycloalkyl, 4-10 membered heterocycloalkyl, C₆₋₁₀aryl and 5-10 membered heteroaryl, are each optionally substituted with 1, 2, 3, or 4 substituents independently selected from R⁶⁰;
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R⁶⁰;
each R^{a7}, R^{c7} and R^{d7} is independently selected from H, C₁₋₆ alkyl, and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, and C₁₋₆haloalkyl; wherein said C₁₋₆ alkyl, C₂₋₆ alkenyl, and C₂₋₆ alkynyl, are each optionally substituted with 1, 2, 3, or 4 substituents independently selected from R²¹;
each R^{a21}, R^{c21} and R^{d21}, is independently selected from H, C₁₋₆ alkyl, and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{a31}, R^{c31} and R^{d31}, is independently selected from H, C₁₋₆ alkyl, and C₁₋₆ haloalkyl;
each R^{a50}, R^{c52} and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁵¹;
or any R^{c50} and R^{d50} attached to the same N atom, together with the N atom to which they are attached, form a 4-, 5-, or 6-membered heterocycloalkyl group optionally substituted with 1, 2 or 3 substituents independently selected from R⁵¹;
each R^{a51}, R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; and
each R^{a60}, R^{b60}, R^{c60} and R^{d60} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl;
or any R^{c60} and R^{d60} attached to the same N atom, together with the N atom to which they are attached, form a 4-, 5-, 6- or 7-membered heterocycloalkyl group.

In yet another embodiment,
each - - - represents a single bond or a double bond;
X is N or CR⁷;
Y is N or C;
R¹ is selected from H, D, C₁₋₃ alkyl, C₁₋₃ haloalkyl, halo, and CN;
R² is selected from H, C₁₋₃ alkyl, C₁₋₃ haloalkyl, phenyl, 5-6 membered heteroaryl, halo, D, and CN;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2 or 3 substituents independently selected from R¹⁰;
when R³N- - -CR⁴ is a single bond, then R⁴ is =O; and
R³ is selected from H, C₁₋₃ alkyl, C₁₋₃ haloalkyl, phenyl, and 5-6 membered heteroaryl; wherein said C₁₋₃ alkyl, phenyl, and 5-6 membered heteroaryl, are each optionally substituted with 1 or 2 substituents independently selected from R³⁰;
when R³N- - -CR⁴ is a double bond, then R³ is absent; and
R⁴ is selected from H, C₁₋₃ alkyl, C₁₋₃ haloalkyl, C₃₋₆ cycloalkyl, 4-6 membered heterocycloalkyl, C₆₋₁₀ aryl, 5-6 membered heteroaryl, halo, D, CN, OR^{a3}, and NR^{c3}R^{j3}; wherein said C₁₋₃ alkyl, C₃₋₆ cycloalkyl, 4-6 membered heterocycloalkyl, C₆₋₁₀ aryl, and 5-6 membered heteroaryl, are each optionally substituted with 1 or 2 substituents independently selected from R³⁰;
when R⁵N- - -YR⁶ is a single bond and Y is C, then YR⁶ is C=O; and
R⁵ is selected from H, C₁₋₃ alkyl, and C₁₋₃ haloalkyl; wherein said C₁₋₃ alkyl, is optionally substituted with 1 or 2 substituents independently selected from R⁵⁰;
when R⁵N- - -YR⁶ is a double bond and Y is N, then R⁵ and R⁶ are absent;
when R⁵N- - -YR⁶ is a double bond and Y is C, then R⁵ is absent; and
R⁶ is selected from H, C₁₋₃ alkyl, C₁₋₃ haloalkyl, C₃₋₆ cycloalkyl, 4-6 membered heterocycloalkyl, phenyl, 5-6 membered heteroaryl, halo, D, CN, OR^{a6}, and NR^{c6}R^{d6}; wherein said C₁₋₃ alkyl, C₃₋₆ cycloalkyl, 4-6 membered heterocycloalkyl, phenyl, and 5-6 membered heteroaryl, are each optionally substituted with 1 or 2 substituents independently selected from R⁶⁰;
R⁷ is selected from H, C₁₋₃ alkyl, C₁₋₃ haloalkyl, halo, D, and CN;
Cy² is selected from 4-6 membered heterocycloalkyl, each has at least one ring-forming carbon atom and 1, 2, 3, or 4 ring-forming heteroatoms independently selected from N, O, and S; wherein a ring-forming carbon atom of 4-6 membered heterocycloalkyl is optionally substituted by oxo to form a carbonyl group; and wherein the 4-6 membered heterocycloalkyl, is optionally substituted with 1 or 2 substituents independently selected from R²⁰;
each R¹⁰ is independently selected from C₁₋₃ alkyl, C₁₋₃ haloalkyl, halo, D, CN, OR^{a10}, and NR^{c10}R^{d10};
each R²⁰ is independently selected from C₁₋₃ alkyl, C₂₋₃ alkenyl, C₂₋₃ alkynyl, C₁₋₃ haloalkyl, halo, D, CN, OR^{a20}, C(O)R^{b20}, C(O)NR^{c20}R^{d20}, C(O)OR^{a20}, and NR^{c20}R^{d20}; wherein said C₁₋₃ alkyl, C₂₋₃ alkenyl, and C₂₋₃ alkynyl, are each optionally substituted with 1 or 2 substituents independently selected from R²¹;
each R²¹ is independently selected from C₁₋₃ alkyl, C₁₋₃ haloalkyl, halo, D, and CN;
each R³⁰ is independently selected from C₁₋₃ alkyl, C₁₋₃ haloalkyl, C₃₋₆ cycloalkyl, 4-6 membered heterocycloalkyl, phenyl, 5-6 membered heteroaryl, halo, D, CN, OR^{a30}, and NR^{c30}R^{d30}; wherein said C₁₋₃ alkyl, C₃₋₆ cycloalkyl, 4-6 membered heterocycloalkyl, phenyl, and 5-6 membered heteroaryl, are each optionally substituted with 1 or 2 substituents independently selected from R³¹;
each R³¹ is independently selected from C₁₋₃ alkyl, C₁₋₃ haloalkyl, halo, D, and CN;
each R⁵⁰ is independently selected from C₁₋₃ alkyl, C₁₋₃ haloalkyl, C₃₋₆ cycloalkyl, 4-6 membered heterocycloalkyl, halo, D, and CN; wherein said C₁₋₃ alkyl, C₃₋₆ cycloalkyl, and 4-6 membered heterocycloalkyl, are each optionally substituted with 1 or 2 substituents independently selected from R⁵¹;
each R⁵¹ is independently selected from C₁₋₃ alkyl, C₁₋₃ haloalkyl, halo, D, and CN;
each R⁶⁰ is independently selected from C₁₋₃ alkyl, C₁₋₃ haloalkyl, C₃₋₆ cycloalkyl, 4-6 membered heterocycloalkyl, halo, D, CN, OR^{a60}, and NR^{c60}R^{d60};
each R^{a3} and R^{c3} is independently selected from H, C₁₋₃ alkyl, and C₁₋₃ haloalkyl; wherein said C₁₋₃ alkyl, is optionally substituted with 1 or 2 substituents independently selected from R³⁰;
each R^{j3} is independently selected from C₁₋₃ alkyl, and C₁₋₃ haloalkyl; wherein said C₁₋₃ alkyl, is optionally substituted with 1 or 2 substituents independently selected from R³⁰;
each R^{a6}, R^{c6}, and R^{d6} is independently selected from H, C₁₋₃ alkyl, C₁₋₃ haloalkyl; wherein said C₁₋₃ alkyl, is optionally substituted with 1 or 2 substituents independently selected from R⁶⁰;
each R^{a10}, R^{b10}, R^{c10} and R^{d10} is independently selected from H, C₁₋₃ alkyl, and C₁₋₃ haloalkyl;
each R^{a20}, R^{b20}, R^{c20} and R^{d20} is independently selected from H, C₁₋₃ alkyl, C₂₋₃ alkenyl, C₂₋₃ alkynyl, and C₁₋₃ haloalkyl; wherein said C₁₋₃ alkyl, C₂₋₃ alkenyl, and C₂₋₃ alkynyl, are each optionally substituted with 1 or 2 substituents independently selected from R²¹;
each R^{a30}, R^{c30} and R^{d30} is independently selected from H, C₁₋₃ alkyl, and C₁₋₃ haloalkyl; wherein said C₁₋₃ alkyl, are each optionally substituted with 1 or 2 substituents independently selected from R³¹; and
each R^{a60}, R^{c60} and R^{d60} is independently selected from H, C₁₋₃ alkyl, C₁₋₃haloalkyl.

In yet another embodiment,
each - - - represents a single bond or a double bond;
X is N or CR⁷;
Y is N or C;
R¹ is H;
R² is selected from H, C₁₋₆ alkyl, 5-10 membered heteroaryl, halo, and CN;
Cy¹ is selected from C₃₋₁₀ cycloalkyl, 4-10 membered heterocycloalkyl, C₆₋₁₀ aryl and 6-10 membered heteroaryl; each optionally substituted with 1, 2, 3 or 4 substituents independently selected from R¹⁰;
when R³N- - -CR⁴ is a single bond, then R⁴ is =O; and
R³ is selected from C₁₋₆ alkyl, C₆₋₁₀ aryl, and 5-10 membered heteroaryl; each optionally substituted with 1, 2, 3, or 4 substituents independently selected from R³⁰;
when R³N- - -CR⁴ is a double bond, then R³ is absent; and
R⁴ is selected from H, 4-10 membered heterocycloalkyl, C₆₋₁₀ aryl, and OR^{a3}; wherein said C₁₋₆ alkyl, 4-10 membered heterocycloalkyl, and C₆₋₁₀ aryl, are each optionally substituted with 1, 2, 3, or 4 substituents independently selected from R³⁰;
when R⁵N- - -YR⁶ is a single bond and Y is C, then YR⁶ is C=O; and
R⁵ is selected from H, and C₁₋₆ alkyl; wherein said C₁₋₆ alkyl, is optionally substituted with 1, 2, 3, or 4 substituents independently selected from R⁵⁰;
when R⁵N- - -YR⁶ is a double bond and Y is N, then R⁵ and R⁶ are absent;
when R⁵N- - -YR⁶ is a double bond and Y is C, then R⁵ is absent; and
R⁶ is selected from H, C₁₋₆ alkyl, 4-10 membered heterocycloalkyl, and 5-10 membered heteroaryl, wherein said C₁₋₆ alkyl, 4-10 membered heterocycloalkyl, and 5-10 membered heteroaryl, are each optionally substituted with 1, 2, 3, or 4 substituents independently selected from R⁶⁰;
R⁷ is halo;
Cy² is selected from 4-10 membered heterocycloalkyl, optionally substituted with 1, 2, 3 or 4 substituents independently selected from R²⁰;
each R¹⁰ is independently selected from C₁₋₆ alkyl, halo, CN, and OR^{a10};
each R²⁰ is independently selected from C₁₋₆ alkyl, CN, OR^{a20}, C(O)R^{b20}, and C(O)NR^{c20}R^{d20}; wherein said C₁₋₆ alkyl, is optionally substituted with 1, 2, 3, or 4 substituents independently selected from R²¹;
each R²¹ is CN;
each R³⁰ is independently selected from C₁₋₆ alkyl, 4-10 membered heterocycloalkyl, 5-10 membered heteroaryl, halo, OR^{a30}, and NR^{c30}R^{d30}; wherein said C₁₋₆ alkyl, 4-10 membered heterocycloalkyl, and 5-10 membered heteroaryl, are each optionally substituted with 1, 2, 3, or 4 substituents independently selected from R³¹;
each R³¹ is C₁₋₆ alkyl;
each R⁵⁰ is 4-10 membered heterocycloalkyl, optionally substituted with 1, 2, 3, or 4 substituents independently selected from R⁵¹;
each R⁵¹ is C₁₋₆ alkyl;
each R⁶⁰ is independently selected from C₁₋₆ alkyl, 4-10 membered heterocycloalkyl, OR^{a60}, and NR^{c60}R^{d60};
each R^{a3}, is C₁₋₆ alkyl, optionally substituted with 1, 2, 3, or 4 substituents independently selected from R³⁰;
each R^{a10}, is independently selected from H, and C₁₋₆ alkyl;
each R^{a20}, R^{b20}, R^{c20} and R^{d20} is independently selected from H, C₁₋₆ alkyl, and C₂₋₆ alkenyl; wherein said C₁₋₆ alkyl, and C₂₋₆ alkenyl, are each optionally substituted with 1, 2, 3, or 4 substituents independently selected from R²¹;
each R^{a30}, R^{c30} and R^{d30} is independently selected from H, and C₁₋₆ alkyl; and each R^{a60}, R^{c60} and R^{d60} is independently selected from H, and C₁₋₆ alkyl.

In another aspect, provided herein is a compound of Formula I, or a pharmaceutically acceptable salt thereof, wherein:
each - - - independently represents a single bond or a double bond;
X is N or CR⁷;
Y is N or C;
R¹ and R² are each independently selected from H, D, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-6 membered heteroaryl, halo, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}(O)R^{b}, NR^{c}C(O)OR^{a}, NR^{c}C(O)NR^{c}R^{d}, NR^{c}S(O)R^{b}, NR^{c}S(O)₂R^{b}, NR^{c}S(O)₂NR^{c}R^{d}, S(O)R^{b}, S(O)NR^{c}R^{d}, S(O)₂R^{b}, S(O)₂NR^{c}R^{d}, and BR^{h}Rⁱ; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-6 membered heteroaryl, are each optionally substituted with 1, 2, 3, or 4 substituents independently selected from R^{g};
Cy¹ is selected from C₃₋₁₀ cycloalkyl, 4-10 membered heterocycloalkyl, C₆₋₁₀ aryl and 5-10 membered heteroaryl; wherein the 4-10 membered heterocycloalkyl and 5-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 5-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 5-10 membered heteroaryl are each optionally substituted with 1, 2, 3 or 4 substituents independently selected from R¹⁰;
when R³N- - -CR⁴ is a single bond, then R⁴ is selected from =O and =S; and
R³ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R³N- - -CR⁴ is a double bond, then R³ is absent; and
R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{d3}, NR^{c3}C(O)R^{b3}, NR^{c3}C(O)OR^{a3}, NR^{c3}C(O)NR^{c3}R^{d3}, C(=NR^{e3})R^{b3}, C(=NOR^{a3})R^{b3}, C(=NR^{e3})NR^{c3}R^{d3}, NR^{c3}C(=NR^{e3})NR^{c3}R^{d3}, NR^{c3}C(=NR^{e3})R^{b3}, NR^{c3}S(O)R^{b3}, NR^{c3}S(O)₂R^{b3}, NR^{c3}S(O)₂NR^{c3}R^{d3}, S(O)R^{b3}, S(O)NR^{c3}R^{d3}, S(O)₂R^{b3}, S(O)₂NR^{c3}R^{d3}, and BR^{h3}Rⁱ³; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R⁵N- - -YR⁶ is a single bond and Y is C, then YR⁶ is selected from C=O and C=S; and
R⁵ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁵⁰;
when R⁵N- - -YR⁶ is a double bond and Y is N, then R⁵ and R⁶ are absent;
when R⁵N- - -YR⁶ is a double bond and Y is C, then R⁵ is absent; and
R⁶ is selected from H, D, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, CN, OR^{a6}, SR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, NR^{c6}S(O)R^{b6}, NR^{c6}S(O)₂R^{b6}, NR^{c6}S(O)₂NR^{c6}R^{d6}, S(O)R^{b6}, S(O)NR^{c6}R^{d6}, S(O)₂R^{b6}, S(O)₂NR^{c6}R^{d6} and BR^{h6}Rⁱ⁶; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R^{g};
R⁷ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a7}, SR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7}, C(=NR^{e7})R^{b7}, C(=NOR^{a7})R^{b7}, C(=NR^{e7})NR^{c7}R^{d7}, NR^{c7}C(=NR^{e7})NR^{c7}R^{d7}, NR^{c7}C(=NR^{e7})R^{b7}, NR^{c7}S(O)R^{b7} , NR^{c7}S(O)₂R^{b7}, NR^{c7}S(O)₂NR^{c7}R^{d7}, S(O)R^{b7}, S(O)NR^{c7}R^{d7}, S(O)₂R^{b7}, S(O)₂NR^{c7}R^{d7}, and BR^{h7}Rⁱ⁷; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷⁰;
Cy² is selected from C₃₋₁₀ cycloalkyl, 4-14 membered heterocycloalkyl, C₆₋₁₀ aryl and 5-10 membered heteroaryl; wherein the 4-10 membered heterocycloalkyl and 5-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 5-10 membered heteroaryl and 4-14 membered heterocycloalkyl is optionally substituted by oxo to form a carbonyl group; and wherein the C₃₋₁₀ cycloalkyl, 4-14 membered heterocycloalkyl, C₆₋₁₀ aryl and 5-10 membered heteroaryl are each optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a10}, SR^{a10}, C(O)R^{b10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, NR^{c10}C(O)NR^{c10}R^{d10}, C(=NR^{e10})R^{b10}, C(=NOR^{a10})R^{b10}, C(=NR^{e10})NR^{c10}R^{d10}, NR^{c10}C(=NR^{e10})NR^{c10}R^{d10}, NR^{c10}S(O)R^{b10}, NR^{c10}S(O)₂R^{b10}, NR^{c10}S(O)₂NR^{c10}R^{d10}, S(O)R^{b10}, S(O)NR^{c10}R^{d10}, S(O)₂R^{b10}, S(O)₂NR^{c10}R^{d10}, and BR^{h10}Rⁱ¹⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R¹¹;
each R¹¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a11}, SR^{a11}, C(O)R^{b11}, C(O)NR^{c11}R^{d11}, C(O)OR^{a11}, OC(O)R^{b11}, OC(O)NR^{c11}R^{d11}, NR^{c11}R^{d11}, NR^{c11}C(O)R^{b11}, NR^{c11}C(O)OR^{a11}, NR^{c11}C(O)NR^{c11}R^{d11}, NR^{c11}S(O)R^{b11}, NR^{c11}S(O)₂R^{b11}, NR^{c11}S(O)₂NR^{c11}R^{d11}, S(O)R^{b11}, S(O)NR^{c11}R^{d11}, S(O)₂R^{b11}, S(O)₂NR^{c11}R^{d11}, and BR^{h11}Rⁱ¹¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R¹²;
each R¹² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a12}, SR^{a12}, C(O)R^{b12}, C(O)NR^{c12}R^{d12}, C(O)OR^{a12}, OC(O)R^{b12}, OC(O)NR^{e12}R^{d12}, NR^{c12}R^{d12}, NR^{c12}C(O)R^{b12}, NR^{c12}C(O)OR^{a12}, NR^{c12}C(O)NR^{c12}R^{d12}, NR^{c12}S(O)R^{b12}, NR^{c12}S(O)₂R^{b12}, NR^{c12}S(O)₂NR^{c12}R^{d12}, S(O)R^{b12}, S(O)NR^{c12}R^{d12}, S(O)₂R^{b12}, S(O)₂NR^{c12}R^{d12}, and BR^{h12}Rⁱ¹²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R²⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a20}, SR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, NR^{c20}C(O)NR^{c20}R^{d20}, C(=NR^{e20})R^{b20}, C(=NOR^{a20})R^{b20}, C(=NR^{e20})NR^{c20}R^{d20}, NR^{c20}C(=NR^{e20})NR^{c20}R^{d20}, NR^{c20}S(O)R^{b20}, NR^{c20}S(O)₂R^{b20}, NR^{c20}S(O)₂NR^{c20}R^{d20}, S(O)R^{b20}, S(O)NR^{c20}R^{d20}, S(O)₂R^{b20}, S(O)₂NR^{c20}R^{d20}, and BR^{h20}Rⁱ²⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²¹;
each R²¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a21}, SR^{a21}, C(O)R^{b21}, C(O)NR^{c21}R^{d21}, C(O)OR^{a21}, OC(O)R^{b21}, OC(O)NR^{c21}R^{d21}, NR^{c21}R^{d21}, NR^{c21}C(O)R^{b21}, NR^{c21}C(O)OR^{a21}, NR^{c21}C(O)NR^{c21}R^{d21}, NR^{c21}S(O)R^{b21}, NR^{c21}S(O)₂R^{b21}, NR^{c21}S(O)₂NR^{c21}R^{d21}, S(O)R^{b21}, S(O)NR^{c21}R^{d21}, S(O)₂R^{b21}, S(O)₂NR^{c21}R^{d21}, and BR^{h21}Rⁱ²¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R^{g};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a30}, SR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, NR^{c30}C(O)NR^{c30}R^{d30}, NR^{c30}S(O)R^{b30}, NR^{c30}S(O)₂R^{b30}, NR^{c30}S(O)₂NR^{c30}R^{d30}, S(O)R^{b30}, S(O)NR^{c30}R^{d30}, S(O)₂R^{b30}, S(O)₂NR^{c30}R^{d30}, and BR^{h30}Rⁱ³⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a31}, SR^{a31}, C(O)R^{b31}, C(O)NR^{c31}R^{d31}, C(O)OR^{a31}, OC(O)R^{b31}, OC(O)NR^{c31}R^{d31}, NR^{c31}R^{d31}, NR^{c31}C(O)R^{b31}, NR^{c31}C(O)OR^{a31}, NR^{c31}C(O)NR^{c31}R^{d31}, NR^{c31}S(O)R^{b31}, NR^{c31}S(O)₂R^{b31}, NR^{c31}S(O)₂NR^{c31}R^{d31}, S(O)R^{b31}, S(O)NR^{c31}R^{d31}, S(O)₂R^{b31}, S(O)₂NR^{c31}R^{d31}, and BR^{h31}Rⁱ³¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³²;
each R³² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a32}, SR^{a32}, C(O)R^{b32}, C(O)NR^{c32}R^{d32}, C(O)OR^{a32}, OC(O)R^{b32}, OC(O)NR^{c32}R^{d32}, NR^{c32}R^{d32}, NR^{c32}C(O)R^{b32}, NR^{c32}C(O)OR^{a32}, NR^{c32}C(O)NR^{c32}R^{d32}, NR^{c32}S(O)R^{b32}, NR^{c32}S(O)₂R^{b32}, NR^{c32}S(O)₂NR^{c32}R^{d32}, S(O)R^{b32}, S(O)NR^{c32}R^{d32}, S(O)₂R^{b32}, S(O)₂NR^{c32}R^{d32}, and BR^{h32}Rⁱ³²; wherein said C₁₋₆ alkyl, C₂₋₆ alkenyl, C₂₋₆ alkynyl, C₃₋₆ cycloalkyl, phenyl, 5-6 membered heteroaryl and 4-7 membered heterocycloalkyl, are each optionally substituted with 1, 2, 3, or 4 substituents independently selected from R^{g};
each R⁵⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a50}, SR^{a50}, C(O)R^{b50}, C(O)NRc⁵⁰R^{d50}, C(O)OR^{a50}, OC(O)R^{b50}, OC(O)NR^{c50}R^{d50}**,** NR^{c50}R^{d50}, NR^{c50}C(O)R^{b50}, NR^{c50}C(O)OR^{a50}, NR^{c50}C(O)NR^{c50}R^{d50}, NR^{c50}S(O)R^{b50}, NR^{c50}S(O)₂R^{b50}, NR^{c50}S(O)₂NR^{c50}R^{d50}, S(O)R^{b50}, S(O)NR^{c50}R^{d50}, S(O)₂R^{b50}, S(O)₂NR^{c50}R^{d50}, and BR^{h50}Rⁱ⁵⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁵¹;
each R⁵¹ is independently selected from C₁₋₆ alkyl, C₂₋₆ alkenyl, C₂₋₆ alkynyl, C₁₋₆ haloalkyl, C₃₋₆ cycloalkyl, C₆₋₁₀ aryl, 5-6 membered heteroaryl, 4-7 membered heterocycloalkyl, halo, D, CN, OR^{a51}, SR^{a51}, C(O)R^{b51}, C(O)NR^{c51}R^{d51}, C(O)OR^{a51}, OC(O)R^{b51}, OC(O)NR^{c51}R^{d51}, NR^{c51}R⁵¹, NR^{c51}C(O)R^{b51}, NR^{c51}C(O)OR^{a51}, NR^{c51}C(O)NR^{c51}R^{d51}, NR^{c51}S(O)R^{b51}, NR^{c51}S(O)₂R^{b51}, NR^{c51}S(O)₂NR^{c51}R^{d51}, S(O)R^{b51}, S(O)NR^{c51}R^{d51}, S(O)₂R^{b51}, S(O)₂NR^{c51}R^{d51}, and BR^{h51}Rⁱ⁵¹; wherein said C₁₋₆ alkyl, C₂₋₆ alkenyl, C₂₋₆ alkynyl, C₃₋₆ cycloalkyl, C₆₋₁₀ aryl, 5-6 membered heteroaryl and 4-7 membered heterocycloalkyl, are each optionally substituted with 1, 2, 3, or 4 substituents independently selected from R⁵²;
each R⁵² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a52}, SR^{a52}, C(O)R^{b52}, C(O)NR^{c52}R^{d52}, C(O)OR^{a52}, OC(O)R^{b52}, OC(O)NR^{c52}R^{d52}, NR^{c52}R^{d52}, NR^{c52}C(O)R^{b52}, NR^{c52}C(O)OR^{a52}, NR^{c52}C(O)NR^{c52}R^{d52}, NR^{c52}S(O)R^{b52}, NR^{c52}S(O)₂R^{b52}, NR^{c52}S(O)₂NR^{c52}R^{d52}, S(O)R^{b52}, S(O)NR^{c52}R^{d52}, S(O)₂R^{b52}, S(O)₂NR^{c52}R^{d52}, and BR^{h52}Rⁱ⁵²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R⁷⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a70}, SR^{a70}, C(O)R^{b70}, C(O)NR^{c70}R^{d70}, C(O)OR^{a70}, OC(O)R^{b70}, OC(O)NR^{c70}R^{d70}, NR^{c70}R^{d70}, NR^{c70}C(O)R^{b70}, NR^{c70}C(O)OR^{a70}, NR^{c70}C(O)NR^{c70}OR^{d70}, NR^{c70}S(O)R^{b70}, NR^{c70}S(O)₂R^{b70}, NR^{c70}S(O)₂NR^{c70}R^{d70}, S(O)R^{b70}, S(O)NR^{c70}R^{d70}, S(O)₂R^{b70}, S(O)₂NR^{c70}R^{d70}, and BR^{h70}Rⁱ⁷⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷¹;
each R⁷¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a71}, SR^{a71}, C(O)R^{b71}, C(O)NR^{c71}R^{d71}, C(O)OR^{a71}, OC(O)R^{b71}, OC(O)NR^{c71}R^{d71}, NR^{c71}R^{d71}, NR^{c71}C(O)R^{b71}, NR^{c71}C(O)OR^{a71}, NR^{c71}C(O)NR^{c71}R^{d71}, NR^{c71}S(O)R^{b71}, NR^{c71}S(O)₂R^{b71}, NR^{c71}S(O)₂NR^{c71}R^{d71}, S(O)R^{b71}, S(O)NR^{c71}R^{d71}, S(O)₂R^{b71}, S(O)₂NR^{c71}R^{d71}, and BR^{h71}Rⁱ⁷¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷²;
each R⁷² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a72}, SR^{a72}, C(O)R^{b72}, C(O)NR^{c72}R^{d72}, C(O)OR^{a72}, OC(O)R^{b72}, OC(O)NR^{c72}R^{d72}, NR^{c72}R^{d72}, NR^{c72}C(O)R^{b72}, NR^{c72}C(O)OR^{a72}, NR^{c72}C(O)NR^{c72}R^{d72}, NR^{c72}S(O)R^{b72}, NR^{c72}S(O)₂R^{b72}, NR^{c72}S(O)₂NR^{c72}R^{d72}, S(O)R^{b72}, S(O)NR^{c72}R^{d72}, S(O)₂R^{b72}, S(O)₂NR^{c72}R^{d72}, and BR^{h72}Rⁱ⁷²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R^{a}, R^{b}, R^{c}, and R^{d} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R^{g};
or any R^{c} and R^{d} attached to the same N atom, together with the N atom to which they are attached, form a 4-, 5-, 6- or 7-membered heterocycloalkyl group optionally substituted with 1, 2 or 3 substituents independently selected from R^{g};
each R^{h} and Rⁱ is independently selected from OH, C₁₋₆ alkoxy, and C₁₋₆ haloalkoxy; or any R^{h} and Rⁱ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{e3} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h3} and Rⁱ³ is independently selected from OH, C₁₋₆ alkoxy, and C₁₋₆ haloalkoxy; or any R^{h3} and Rⁱ³ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a6}, R^{b6}, R^{c6}, and R^{d6} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R^{g};
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R^{g};
each R^{h6} and Rⁱ⁶ is independently selected from OH, C₁₋₆ alkoxy, and C₁₋₆ haloalkoxy; or any R^{h6} and Rⁱ⁶ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a7}, R^{b7}, R^{c7} and R^{d7} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷⁰;
or any R^{c7} and R^{d7} attached to the same N atom, together with the N atom to which they are attached, form a 4-, 5-, 6- or 7-membered heterocycloalkyl group optionally substituted with 1, 2, 3, or 4 substituents independently selected from R⁷⁰;
each R^{e7} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h7} and Rⁱ⁷ is independently selected from OH, C₁₋₆ alkoxy, and C₁₋₆ haloalkoxy; or any R^{h7} and Rⁱ⁷ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R¹¹;
or any R^{c10} and R^{d10} attached to the same N atom, together with the N atom to which they are attached, form a 4-, 5-, 6- or 7-membered heterocycloalkyl group optionally substituted with 1, 2, 3, or 4 substituents independently selected from R¹¹;
each R^{e10} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h10} and Rⁱ¹⁰ is independently selected from OH, C₁₋₆ alkoxy, and C₁₋₆ haloalkoxy; or any R^{h10} and Rⁱ¹⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a11}, R^{b11}, R^{c11} and R^{d11}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R¹²;
or any R^{c11} and R^{d11} attached to the same N atom, together with the N atom to which they are attached, form a 4-, 5-, 6- or 7-membered heterocycloalkyl group optionally substituted with 1, 2 or 3 substituents independently selected from R¹²;
each R^{h11} and Rⁱ¹¹ is independently selected from OH, C₁₋₆ alkoxy, and C₁₋₆ haloalkoxy; or any R^{h11} and Rⁱ¹¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a12}, R^{b12}, R^{c12} and R^{d12}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
each R^{h12} and Rⁱ¹² is independently selected from OH, C₁₋₆ alkoxy, and C₁₋₆ haloalkoxy; or any R^{h12} and Rⁱ¹² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²¹;
or any R^{c20} and R^{d20} attached to the same N atom, together with the N atom to which they are attached, form a 4-, 5-, 6- or 7-membered heterocycloalkyl group optionally substituted with 1, 2, 3, or 4 substituents independently selected from R²¹;
each R^{e20} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h20} and Rⁱ²⁰ is independently selected from OH, C₁₋₆ alkoxy, and C₁₋₆ haloalkoxy; or any R^{h20} and Rⁱ²⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a21}, R^{b21}, R^{c21} and R^{d21}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R^{g};
or any R^{c21} and R^{d21} attached to the same N atom, together with the N atom to which they are attached, form a 4-, 5-, 6- or 7-membered heterocycloalkyl group optionally substituted with 1, 2 or 3 substituents independently selected from R^{g};
each R^{h21} and Rⁱ²¹ is independently selected from OH, C₁₋₆ alkoxy, and C₁₋₆ haloalkoxy; or any R^{h21} and Rⁱ²¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{h30} and Rⁱ³⁰ is independently selected from OH, C₁₋₆ alkoxy, and C₁₋₆ haloalkoxy; or any R^{h30} and Rⁱ³⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a31}, R^{b31}, R^{c31} and R^{d31}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R³²;
or any R^{c31} and R^{d31} attached to the same N atom, together with the N atom to which they are attached, form a 4-, 5-, 6- or 7-membered heterocycloalkyl group optionally substituted with 1, 2 or 3 substituents independently selected from R³²;
each R^{h31} and Rⁱ³¹ is independently selected from OH, C₁₋₆ alkoxy, and C₁₋₆ haloalkoxy; or any R^{h31} and Rⁱ³¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a32}, R^{b32}, R^{c32} and R^{d32}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c32} and R^{d32} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h32} and Rⁱ³² is independently selected from OH, C₁₋₆ alkoxy, and C₁₋₆ haloalkoxy; or any R^{h32} and Rⁱ³² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a50}, R^{b50}, R^{c50} and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl are each optionally substituted with 1, 2, 3, or 4 substituents independently selected from R⁵¹;
or any R^{c50} and R^{d50} attached to the same N atom, together with the N atom to which they are attached, form a 4-, 5-, 6- or 7-membered heterocycloalkyl group optionally substituted with 1, 2 or 3 substituents independently selected from R⁵¹;
each R^{h50} and Rⁱ⁵⁰ is independently selected from OH, C₁₋₆ alkoxy, and C₁₋₆ haloalkoxy; or any R^{h50} and Rⁱ⁵⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a51}, R^{b51}, R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁵²;
or any R^{c51} and R^{d51} attached to the same N atom, together with the N atom to which they are attached, form a 4-, 5-, 6- or 7-membered heterocycloalkyl group optionally substituted with 1, 2, 3, or 4 substituents independently selected from R⁵²;
each R^{h51} and Rⁱ⁵¹ is independently selected from OH, C₁₋₆ alkoxy, and C₁₋₆ haloalkoxy; or any R^{h51} and Rⁱ⁵¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a52}, R^{b52}, R^{c52} and R^{d52}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c52} and R^{d52} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h52} and Rⁱ⁵² is independently selected from OH, C₁₋₆ alkoxy, and C₁₋₆ haloalkoxy; or any R^{h52} and Rⁱ⁵² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a70}, R^{b70}, R^{c70} and R^{d70} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷¹;
or any R^{c70} and R^{d70} attached to the same N atom, together with the N atom to which they are attached, form a 4-, 5-, 6- or 7-membered heterocycloalkyl group optionally substituted with 1, 2, 3, or 4 substituents independently selected from R⁷¹;
each R^{h70} and Rⁱ⁷⁰ is independently selected from OH, C₁₋₆ alkoxy, and C₁₋₆ haloalkoxy; or any R^{h70} and Rⁱ⁷⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a71}, R^{b71}, R^{c71} and R^{d71}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁷²;
**or any** R^{c71} **and** R^{d71} attached to the same N atom, together with the N atom to which they are attached, form a 4-, 5-, 6- or 7-membered heterocycloalkyl group optionally substituted with 1, 2 or 3 substituents independently selected from R⁷²;
each R^{h71} and Rⁱ⁷¹ is independently selected from OH, C₁₋₆ alkoxy, and C₁₋₆ haloalkoxy; or any R^{h71} and Rⁱ⁷¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a72}, R^{b72}, R^{c72} and R^{d72}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c72} and R^{d72} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h72} and Rⁱ⁷² is independently selected from OH, C₁₋₆ alkoxy, and C₁₋₆ haloalkoxy; or any R^{h72} and Rⁱ⁷² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl; and
each R^{g} is independently selected from D, OH, NO₂, CN, halo, C₁₋₆ alkyl, C₂₋₆ alkenyl, C₂₋₆ alkynyl, C₁₋₆ haloalkyl, C₃₋₆ cycloalkyl, C₃₋₆ cycloalkyl-C₁₋₂ alkylene, C₁₋₆ alkoxy, C₁₋₆ haloalkoxy, C₁₋₃ alkoxy-C₁₋₃ alkyl, C₁₋₃ alkoxy-C₁₋₃ alkoxy, HO-C₁₋₃ alkoxy, HO-C₁₋₃ alkyl, cyano-C₁₋₃ alkyl, H₂N-C₁₋₃ alkyl, amino, C₁₋₆ alkylamino, di(C₁₋₆ alkyl)amino, thio, C₁₋₆ alkylthio, C₁₋₆ alkylsulfinyl, C₁₋₆ alkylsulfonyl, carbamyl, C₁₋₆ alkylcarbamyl, di(C₁₋₆ alkyl)carbamyl, carboxy, C₁₋₆ alkylcarbonyl, C₁₋₆ alkoxycarbonyl, C₁₋₆ alkylcarbonylamino, C₁₋₆ alkoxycarbonylamino, C₁₋₆ alkylcarbonyloxy, aminocarbonyloxy, C₁₋₆ alkylaminocarbonyloxy, di(C₁₋₆ alkyl)aminocarbonyloxy, C₁₋₆ alkylsulfonylamino, aminosulfonyl, C₁₋₆ alkylaminosulfonyl, di(C₁₋₆ alkyl)aminosulfonyl, aminosulfonylamino, C₁₋₆ alkylaminosulfonylamino, di(C₁₋₆ alkyl)aminosulfonylamino, aminocarbonylamino, C₁₋₆ alkylaminocarbonylamino, and di(C₁₋₆ alkyl)aminocarbonylamino;
provided that, Cy¹ is other than 3,5-dimethylisoxazol-4-yl or 3,5-dimethyl-1H-pyrazol-4-yl.

In an embodiment,
each - - - independently represents a single bond or a double bond;
X is N or CR⁷;
Y is N or C;
R¹ and R² are each independently selected from H, D, C₁₋₆ alkyl, C₂₋₆ alkenyl, C₂₋₆ alkynyl, C₁₋₆ haloalkyl, halo, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{C}C(O)OR^{a}, NR^{c}C(O)NR^{c}R^{d}, NR^{C}S(O)₂R^{b}, S(O)₂R^{b}, and S(O)₂NR^{c}R^{d}; wherein said C₁₋₆ alkyl, C₂₋₆ alkenyl, and C₂₋₆ alkynyl, are each optionally substituted with 1, 2, 3, or 4 substituents independently selected from R^{g};
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, 3 or 4 substituents independently selected from R¹⁰;
when R³N- - -CR⁴ is a single bond, then R⁴ is selected from =O and =S; and
R³ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R³N- - -CR⁴ is a double bond, then R³ is absent; and
R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{d3}, NR^{c3}C(O)R^{b3}, NR^{c3}C(O)OR^{a3}, NR^{c3}C(O)NR^{c3}R^{d3}, NR^{c3}S(O)₂R^{b3}, S(O)₂R^{b3}, and S(O)₂NR^{c3}R^{d3}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R⁵N- - -YR⁶ is a single bond and Y is C, then YR⁶ is selected from C=O and C=S; and
R⁵ is selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R⁵⁰;
when R⁵N- - -YR⁶ is a double bond and Y is N, then R⁵ and R⁶ are absent;
when R⁵N- - -YR⁶ is a double bond and Y is C, then R⁵ is absent; and
R⁶ is selected from H, D, C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, CN, OR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, NR^{c6}S(O)₂R^{b6}, S(O)₂R^{b6}, and S(O)₂NR^{c6}R^{d6}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R^{g};
R⁷ is selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl, halo, D, CN, OR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7} NR^{c7}S(O)₂R^{b7}, S(O)₂R^{b7}, and S(O)₂NR^{c7}R^{d7}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R⁷⁰;
Cy² is 4-14 membered heterocycloalkyl; wherein the 4-14 membered heterocycloalkyl has at least one ring-forming carbon atom and 1, 2, 3, or 4 ring-forming heteroatoms independently selected from N, O, and S; wherein the N and S are optionally oxidized; wherein a ring-forming carbon atom of 4-14 membered heterocycloalkyl is optionally substituted by oxo to form a carbonyl group; and wherein the 4-14 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, OR^{a10}, C(O)R^{b10}, C(O)NR^{c19}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, NR^{c10}C(O)NR^{c10}R^{d10}, NR^{c10}S(O)₂R^{b10}, S(O)₂R^{b10}, and S(O)₂NR^{c10}R^{d10}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R¹¹;
each R¹¹ is independently selected from C₁₋₆ alkyl, C₁₋₆ haloalkyl, halo, D, CN, OR^{a11}, C(O)R^{b11}, C(O)NR^{c11}R^{d11}, C(O)OR^{a11}, OC(O)R^{b11}, OC(O)NR^{c11}R^{d11}, NR^{c11}R^{d11}, NR^{c11}C(O)R^{b11}, NR^{c11}C(O)OR^{a11}, S(O)₂R^{b11}, and S(O)₂NR^{c11}R^{d11};
each R²⁰ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, OR^{a20}, C(0)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, NR^{c20}C(O)NR^{c20}R^{d20}, NR^{c20}S(O)₂R^{b20}, S(O)₂R^{b20}, and S(O)₂NR^{c20}R^{d20}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R²¹;
each R²¹ is independently selected from C₁₋₆ alkyl, C₂₋₆ alkenyl, C₂₋₆ alkynyl, C₁₋₆ haloalkyl, halo, D, CN, OR^{a21}, C(O)R^{b21}, C(O)NR^{c21}R^{d21}, C(O)OR^{a21}, OC(O)R^{b21}, OC(O)NR^{c21}R^{d21}, NR^{c21}R^{d21}, NR^{c21}C(O)R^{b21}, NR^{c21}C(O)OR^{a21}, S(O)₂R^{b21}, and S(O)₂NR^{c21}R^{d21};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, NR^{c30}C(O)NR^{c30}R^{d30}, NR^{c30}S(O)₂R^{b30}, S(O)₂R^{b30}, and S(O)₂NR^{c30}R^{d30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, OR^{a31}, C(O)R^{b31}, C(O)NR^{c31}R^{d31}, C(O)OR^{a31}, OC(O)R^{b31}, OC(O)NR^{c31}R^{d31}, NR^{c31}R^{d31}, NR^{c31}C(O)R^{b31}, NR^{c31}C(O)OR^{a31}, NR^{c31}C(O)NR^{c31}R^{d31}, NR^{c31}S(O)₂R^{b31}, S(O)₂R^{b31}, and S(O)₂NR^{c31}R^{d31}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R³²;
each R³² is independently selected from C₁₋₆ alkyl, C₂₋₆ alkenyl, C₂₋₆ alkynyl, C₁₋₆ haloalkyl, halo, D, CN, OR^{a32}, C(O)R^{b32}, C(O)NR^{c32}R^{d32}, C(O)OR^{a32}, OC(O)R^{b32}, OC(O)NR^{c32}R^{d32}, NR^{c32}R^{d32}, NR^{c32}C(O)R^{b32}, NR^{c32}C(O)OR^{a32}, S(O)₂R^{b32}, and S(O)₂NR^{c32}R^{d32};
each R⁵⁰ is independently selected from C₁₋₆ alkyl, C₂₋₆ alkenyl, C₂₋₆ alkynyl, C₁₋₆ haloalkyl, halo, D, CN, OR^{a50}, C(O)R^{b50}, C(O)NR^{c50}R^{d50}, C(O)OR^{a50}, OC(O)R^{b50}, OC(O)NR^{c50}R^{d50}, NR^{c50}R^{d50}, NR^{c50}C(O)R^{b50}, NR^{c50}C(O)OR^{a50}, S(O)₂R^{b50}, and S(O)₂NR^{c50}R^{d50};
each R⁷⁰ is independently selected from C₁₋₆ alkyl, C₂₋₆ alkenyl, C₂₋₆ alkynyl, C₁₋₆ haloalkyl, halo, D, CN, NO₂, OR^{a70}, C(O)R^{b70}, C(O)NR^{c70}R^{d70}, C(O)OR^{a70}, OC(O)R^{b70}, OC(O)NR^{c70}R^{d70}, NR^{c70}R^{d70}, NR^{c70}C(O)R^{b70}, NR^{c70}C(O)OR^{a70}, S(O)₂R^{b70}, and S(O)₂NR^{c70}R^{d70};
each R^{a}, R^{b}, R^{c}, and R^{d} is independently selected from H, C₁₋₆ alkyl, C₂₋₆ alkenyl, C₂₋₆ alkynyl, and C₁₋₆ haloalkyl; wherein said C₁₋₆ alkyl, C₂₋₆ alkenyl, and C₂₋₆ alkynyl, are each optionally substituted with 1, 2, 3, or 4 substituents independently selected from R^{g};
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R^{g};
or any R^{c6} and R^{d6} attached to the same N atom, together with the N atom to which they are attached, form a 4-, 5-, or 6-membered heterocycloalkyl group optionally substituted with 1, 2 or 3 substituents independently selected from R^{g};
each R^{a7}, R^{b7}, R^{c7} and R^{d7} is independently selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R⁷⁰;
or any R^{c7} and R^{d7} attached to the same N atom, together with the N atom to which they are attached, form a 4-, 5-, or 6-membered heterocycloalkyl group optionally substituted with 1, 2, 3, or 4 substituents independently selected from R⁷⁰;
each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R¹¹;
or any R^{c10} and R^{d10} attached to the same N atom, together with the N atom to which they are attached, form a 4-, 5-, or 6-membered heterocycloalkyl group optionally substituted with 1, 2, 3, or 4 substituents independently selected from R¹¹;
each R^{a11}, R^{b11}, R^{c11} and R^{d11}, is independently selected from H, C₁₋₆ alkyl, C₂₋₆ alkenyl, C₂₋₆ alkynyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R²¹;
or any R^{c20} and R^{d20} attached to the same N atom, together with the N atom to which they are attached, form a 4-, 5-, or 6-membered heterocycloalkyl group optionally substituted with 1, 2, 3, or 4 substituents independently selected from R²¹;
each R^{a21}, R^{b21}, R^{c21} and R^{d21}, is independently selected from H, C₁₋₆ alkyl, C₂₋₆ alkenyl, C₂₋₆ alkynyl, and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{a31}, R^{b31}, R^{c31} and R^{d31}, is independently selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl C₂₋₆ alkenyl, C₂₋₆ alkynyl, C₃₋₆ cycloalkyl, and 4-6 membered heterocycloalkyl are each optionally substituted with 1, 2, 3, or 4 substituents independently selected from R³²;
or any R^{c31} and R^{d31} attached to the same N atom, together with the N atom to which they are attached, form a 4-, 5-, or 6-membered heterocycloalkyl group optionally substituted with 1, 2 or 3 substituents independently selected from R³²;
each R^{a32}, R^{b32}, R^{c32} and R^{d32}, is independently selected from H, C₁₋₆ alkyl, C₂₋₆ alkenyl, C₂₋₆ alkynyl and C₁₋₆ haloalkyl;
each R^{a50}, R^{b50}, R^{c50} and R^{d50}, is independently selected from H, C₁₋₆ alkyl, C₂₋₆ alkenyl, C₂₋₆ alkynyl, and C₁₋₆ haloalkyl;
each R^{a70}, R^{b70}, R^{c70} and R^{d70} is independently selected from H, C₁₋₆ alkyl, C₂₋₆ alkenyl, C₂₋₆ alkynyl, and C₁₋₆ haloalkyl; and
each R^{g} is independently selected from D, OH, CN, halo, C₁₋₆ alkyl, C₂₋₆ alkenyl, C₂₋₆ alkynyl, C₁₋₆ haloalkyl, C₃₋₆ cycloalkyl, C₁₋₆ alkoxy, C₁₋₆ haloalkoxy, C₁₋₃ alkoxy-C₁₋₃ alkyl, C₁₋₃ HO-C₁₋₃ alkyl, cyano-C₁₋₃ alkyl, H₂N-C₁₋₃ alkyl, amino, C₁₋₆ alkylamino, di(C₁₋₆ alkyl)amino, C₁₋₆ alkylsulfonyl, C₁₋₆ alkylcarbamyl, di(C₁₋₆ alkyl)carbamyl, carboxy, C₁₋₆ alkylcarbonyl, C₁₋₆ alkoxycarbonyl, C₁₋₆ alkylcarbonylamino, C₁₋₆ alkoxycarbonylamino, C₁₋₆ alkylcarbonyloxy, C₁₋₆ alkylaminocarbonyloxy, di(C₁₋₆ alkyl)aminocarbonyloxy, C₁₋₆ alkylaminocarbonylamino, and di(C₁₋₆ alkyl)aminocarbonylamino.

In another embodiment,
each - - - independently represents a single bond or a double bond;
X is N or CR⁷;
Y is N or C;
R¹ and R² are each independently H or halo;
Cy¹ is C₆₋₁₀ aryl or 6-10 membered heteroaryl, both of which are optionally substituted with 1, 2, 3 or 4 substituents independently selected from R¹⁰;
when R³N- - -CR⁴ is a single bond, then R⁴ is =O;
when R³N- - -CR⁴ is a double bond, then R³ is absent;
R³ is selected from H, C₁₋₆ alkyl, C₃₋₁₀ cycloalkyl, 4-10 membered heterocycloalkyl, C₆₋₁₀ aryl, and 5-10 membered heteroaryl;
R⁴ is selected from H, 4-10 membered heterocycloalkyl, C₆₋₁₀ aryl, and OR^{a3}; wherein heterocycloalkyl and aryl are each optionally substituted with 1, 2, 3, or 4 substituents independently selected from R³⁰;
when R⁵N- - -YR⁶ is a single bond and Y is C, then YR⁶ is C=O, and R⁵ is H;
when R⁵N- - -YR⁶ is a double bond and Y is N, then R⁵ and R⁶ are absent;
when R⁵N- - -YR⁶ is a double bond and Y is C, then R⁵ is absent, and R⁶ is H;
R⁷ is H or halo;
Cy² is 4-10 membered heterocycloalkyl optionally substituted with one or two substituents independently selected from R²⁰;
each R¹⁰ is independently selected from C₁₋₆ alkyl, halo, and OR^{a10};
R²⁰ is selected from C₁₋₆ alkyl, C(O)R^{b20}, C(O)OR^{a20}, and OC(O)R^{b20};
each R³⁰ is independently selected from 4-10 membered heterocycloalkyl, 5-10 membered heteroaryl, halo, OR^{a30}, NR^{c30}R^{d30}, wherein heterocycloalkyl and heteroaryl are each optionally substituted with 1, 2, 3, or 4 substituents independently selected from R³¹;
R³¹ is C₁₋₆ alkyl;
R^{a3} is C₁₋₆ alkyl optionally substituted with 1, 2, 3, or 4 substituents independently selected from R³⁰;
R^{a10} is H;
each R^{a20} and R^{b20} is independently selected from H, C₁₋₆ alkyl, and C₂₋₆ alkenyl; and
R^{a30}, R^{c30} and R^{d30} are each independently H or C₁₋₆ alkyl.

In another aspect of Formula I,
each - - - independently represents a single bond or a double bond;
X is N, CH, or C-halo;
Y is N or C;
R¹ and R² are each independently H or halo;
Cy¹ is C₆₋₁₀ aryl or 6-10 membered heteroaryl, both of which are optionally substituted with 1, 2, 3 or 4 substituents independently selected from C₁₋₆ alkyl, halo, and OH;
alternatively, Cy¹ is C₆₋₁₀ aryl or 6-10 membered heteroaryl, both of which are optionally fused to C₄₋₁₀ cycloalkyl or 4-10 membered heterocycloalkyl;
when R³N- - -CR⁴ is a single bond, then R⁴ is =O;
when R³N- - -CR⁴ is a double bond, then R³ is absent;
R³ is selected from H, C₁₋₆ alkyl, C₃₋₁₀ cycloalkyl, 4-10 membered heterocycloalkyl, C₆₋₁₀ aryl, and 5-10 membered heteroaryl;
R⁴ is selected from H, 4-10 membered heterocycloalkyl, C₆₋₁₀ aryl, and OC₁₋₆ alkyl; wherein alkyl, heterocycloalkyl and aryl are each optionally substituted with a group selected from 4-10 membered heterocycloalkyl, 5-10 membered heteroaryl, halo, OH, OC₁₋₆ alkyl, NH₂, NH(C₁₋₆ alkyl), and N(C₁₋₆ alkyl)₂;
when R⁵N- - -YR⁶ is a single bond and Y is C, then YR⁶ is C=O, and R⁵ is H;
when R⁵N- - -YR⁶ is a double bond and Y is N, then R⁵ and R⁶ are absent;
when R⁵N- - -YR⁶ is a double bond and Y is C, then R⁵ is absent, and R⁶ is H; and
Cy² is 4-10 membered heterocycloalkyl optionally substituted with one or two substituents independently selected from C₁₋₆ alkyl, C(O)H, C(O)C₁₋₆ alkyl, C(O)C₂₋₆ alkenyl, C(O)OH, C(O)OC₁₋₆ alkyl, C(O)OC₂₋₆ alkenyl, OC(O)H, OC(O)C₁₋₆ alkyl, and OC(O)C₂₋₆ alkenyl; wherein alkyl is optionally substituted with CN.

In an embodiment, X is CR⁷. In another embodiment, Y is N. In yet another embodiment, Y is C and R⁶ is =O. In still another embodiment, R² is halo. In an embodiment, R⁷ is halo. In another embodiment, both R² and R⁷ are halo. In yet another embodiment, Cy¹ is C₆₋₁₀ aryl or 6-10 membered heteroaryl, wherein aryl and heteroaryl are each optionally substituted with 1, 2, 3 or 4 substituents independently selected from R¹⁰. In still another embodiment, R⁴ is =O. In an embodiment, R¹ is H.

In another embodiment, the compound of Formula I is a compound of Formula II: 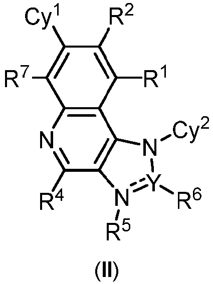 or a pharmaceutically acceptable salt thereof.

In yet another embodiment of Formula I and Formula II,
- - - independently represents a single bond or a double bond;
Y is N or C;
R¹ and R² are each independently selected from H, D, C₁₋₆ alkyl, C₂₋₆ alkenyl, C₂₋₆ alkynyl, C₁₋₆ haloalkyl, halo, CN, OR^{a}, and NR^{c}R^{d};
Cy¹ is selected from C₆₋₁₀ aryl and 6-10 membered heteroaryl; wherein the 6-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 6-10 membered heteroaryl is optionally substituted by oxo to form a carbonyl group; and wherein the C₆₋₁₀ aryl and 6-10 membered heteroaryl are each optionally substituted with 1, 2, 3 or 4 substituents independently selected from R¹⁰;
R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a3}, C(O)R^{b3}, C(O)NR^{c3}R^{d3}, C(O)OR^{a3}, OC(O)R^{b3}, OC(O)NR^{c3}R^{d3}, NR^{c3}R^{d3}, NR^{c3}C(O)R^{b3}, NR^{c3}C(O)OR^{a3}, and S(O)₂R^{b3}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R⁵N- - -YR⁶ is a single bond and Y is C, then YR⁶ is selected from C=O and C=S; and
R⁵ is selected from H, C₁₋₆ alkyl, C₂₋₆ alkenyl, C₂₋₆ alkynyl, and C₁₋₆ haloalkyl;
when R⁵N- - -YR⁶ is a double bond and Y is N, then R⁵ and R⁶ are absent;
when R⁵N- - -YR⁶ is a double bond and Y is C, then R⁵ is absent; and
R⁶ is selected from H, D, C₁₋₆ alkyl, C₂₋₆ alkenyl, C₂₋₆ alkynyl, C₁₋₆ haloalkyl, halo, CN, OR^{a6}, and NR^{c6}R^{d6};
R⁷ is selected from H, C₁₋₆ alkyl, C₂₋₆ alkenyl, C₂₋₆ alkynyl, C₁₋₆ haloalkyl, halo, D, CN, OR^{a7}, and NR^{c7}R^{d7};
Cy² is 4-10 membered heterocycloalkyl; wherein the 4-10 membered heterocycloalkyl has at least one ring-forming carbon atom and 1, 2, 3, or 4 ring-forming heteroatoms independently selected from N, O, and S; wherein the N and S are optionally oxidized; wherein a ring-forming carbon atom of 4-10 membered heterocycloalkyl is optionally substituted by oxo to form a carbonyl group; and wherein the 4-10 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, halo, D, CN, OR^{a10}, C(O)R^{b10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, and S(O)₂R^{b10};
each R²⁰ is independently selected from C₁₋₆ alkyl, C₂₋₆ alkenyl, C₂₋₆ alkynyl, C₁₋₆ haloalkyl, halo, D, CN, OR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, and S(O)₂R^{b20};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, and S(O)₂R^{b30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, halo, D, CN, OR^{a31}, and NR^{c31}R^{d31};
each R^{a}, R^{c}, and R^{d} is independently selected from H, C₁₋₆ alkyl, C₂₋₆ alkenyl, C₂₋₆ alkynyl, and C₁₋₆ haloalkyl;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{c6}, and R^{d6} is independently selected from H, C₁₋₆ alkyl, C₂₋₆ alkenyl, C₂₋₆ alkynyl, and C₁₋₆ haloalkyl;
each R^{a7}, R^{c7} and R^{d7} is independently selected from H, C₁₋₆ alkyl, C₂₋₆ alkenyl, C₂₋₆ alkynyl, and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹; and each R^{a31}, R^{c31} and R^{d31}, is independently selected from H, C₁₋₆ alkyl, C₂₋₆ alkenyl, C₂₋₆ alkynyl, and C₁₋₆ haloalkyl.

In another embodiment,
each - - - independently represents a single bond or a double bond;
Y is N or C;
R¹ and R² are each independently H or halo;
Cy¹ is C₆₋₁₀ aryl or 6-10 membered heteroaryl, both of which are optionally substituted with 1, 2, 3 or 4 substituents independently selected from R¹⁰;
R⁴ is selected from H, 4-10 membered heterocycloalkyl, C₆₋₁₀ aryl, and OR^{a3}; wherein heterocycloalkyl and aryl are each optionally substituted with 1, 2, 3, or 4 substituents independently selected from R³⁰;
when R⁵N- - -YR⁶ is a single bond and Y is C, then YR⁶ is C=O, and R⁵ is H;
when R⁵N- - -YR⁶ is a double bond and Y is N, then R⁵ and R⁶ are absent;
when R⁵N- - -YR⁶ is a double bond and Y is C, then R⁵ is absent, and R⁶ is H;
R⁷ is H or halo;
Cy² is 4-10 membered heterocycloalkyl optionally substituted with one or two substituents independently selected from R²⁰;
each R¹⁰ is independently selected from C₁₋₆ alkyl, halo, and OR^{a10};
R²⁰ is C(O)R^{b20};
each R³⁰ is independently selected from 4-10 membered heterocycloalkyl, 5-10 membered heteroaryl, halo, OR^{a30}, NR^{c30}R^{d30}, wherein heterocycloalkyl and heteroaryl are each optionally substituted with 1, 2, 3, or 4 substituents independently selected from R³¹;
R³¹ is C₁₋₆ alkyl;
R^{a3} is C₁₋₆ alkyl optionally substituted with 1, 2, 3, or 4 substituents independently selected from R³⁰;
R^{a10} is H;
R^{b20} is H, C₁₋₆ alkyl, or C₂₋₆ alkenyl; and
R^{a30}, R^{c30} and R^{d30} are each independently H or C₁₋₆ alkyl.

In yet another embodiment,
- - - represents a single bond or a double bond;
Y is N or C;
R¹ and R² are each independently H or halo;
Cy¹ is C₆₋₁₀ aryl or 6-10 membered heteroaryl, both of which are optionally substituted with 1 or 2 substituents independently selected from R¹⁰;
R⁴ is selected from H, 4-10 membered heterocycloalkyl, C₆₋₁₀ aryl, and OR^{a3}; wherein heterocycloalkyl and aryl are each optionally substituted with 1 or 2 substituents independently selected from R³⁰;
when R⁵N- - -YR⁶ is a single bond and Y is C, then YR⁶ is C=O, and R⁵ is H;
when R⁵N- - -YR⁶ is a double bond and Y is N, then R⁵ and R⁶ are absent;
when R⁵N- - -YR⁶ is a double bond and Y is C, then R⁵ is absent, and R⁶ is H;
R⁷ is H or halo;
Cy² is 4-6 membered heterocycloalkyl optionally substituted with one or two substituents independently selected from R²⁰;
each R¹⁰ is independently selected from C₁₋₆ alkyl, halo, and OR^{a10};
R²⁰ is selected from C(O)R^{b20}, C(O)OR^{a20}, and OC(O)R^{b20};
each R³⁰ is independently selected from halo, OR^{a30}, NR^{c30}R^{d30};
R^{a3} is C₁₋₆ alkyl optionally substituted with 1, 2, 3, or 4 substituents independently selected from R³⁰;
R^{a10} is H;
each R^{a20} and R^{b20} is independently selected from H, C₁₋₆ alkyl, and C₂₋₆ alkenyl; and
R^{a30}, R^{c30} and R^{d30} are each independently H or C₁₋₆ alkyl.

In an embodiment, the compound of Formula I is a compound of Formula la: 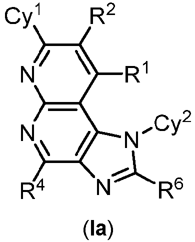 or a pharmaceutically acceptable salt thereof.

In another embodiment, the compound of Formula I is a compound of Formula Ib: 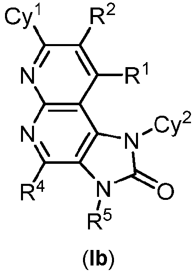 or a pharmaceutically acceptable salt thereof.

In yet another embodiment, the compound of Formula I is a compound of Formula Ic: 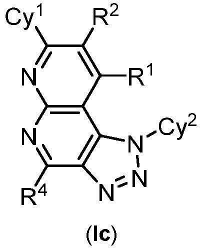 or a pharmaceutically acceptable salt thereof.

In still another embodiment, the compound of Formula I is a compound of Formula Id: 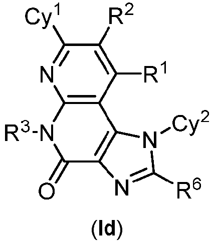 or a pharmaceutically acceptable salt thereof.

In an embodiment, the compound of Formula I is a compound of Formula le: 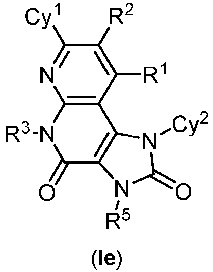 or a pharmaceutically acceptable salt thereof.

In another embodiment, the compound of Formula I is a compound of Formula If: 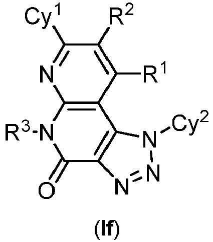 or a pharmaceutically acceptable salt thereof.

In yet another embodiment, the compound of Formula I is a compound of Formula Ig: 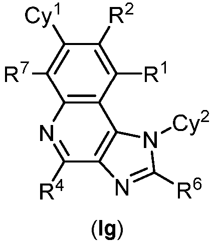 or a pharmaceutically acceptable salt thereof.

In still another embodiment, the compound of Formula I is a compound of Formula Ih: 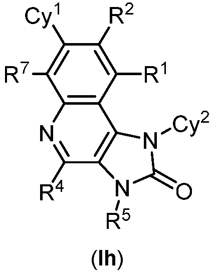 or a pharmaceutically acceptable salt thereof.

In an embodiment, the compound of Formula I is a compound of Formula li: 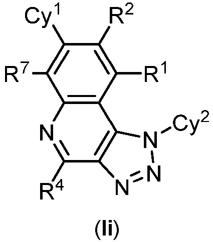 or a pharmaceutically acceptable salt thereof.

In another embodiment, the compound of Formula I is a compound of Formula Ij: 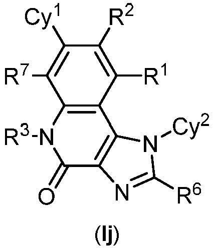 or a pharmaceutically acceptable salt thereof.

In yet another embodiment, the compound of Formula I is a compound of Formula Ik: 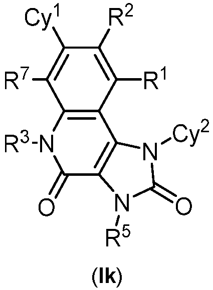 or a pharmaceutically acceptable salt thereof.

In still another embodiment, the compound of Formula I is a compound of Formula Im: 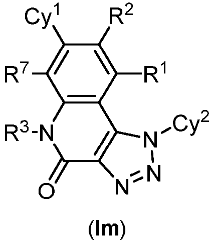 or a pharmaceutically acceptable salt thereof.

In an embodiment, the compound of Formula II is a compound of Formula IIa: 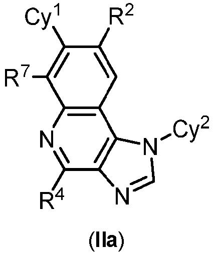 or a pharmaceutically acceptable salt thereof.

In another embodiment, the compound of Formula II is a compound of Formula Ilb: 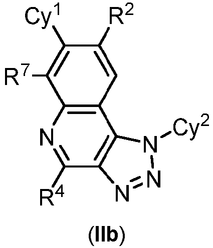 or a pharmaceutically acceptable salt thereof.

In yet another embodiment, the compound of Formula II is a compound of Formula IIc: 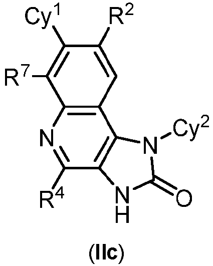 or a pharmaceutically acceptable salt thereof.

In an embodiment of a compound of Formula I, or pharmaceutically acceptable salt thereof,
each - - - represents a single bond or a double bond;
X is N or CR⁷;
Y is N or C;
R¹ is selected from H, D, C₁₋₆ alkyl, C₁₋₆ haloalkyl, halo, and CN;
R² is selected from H, C₁₋₆ alkyl, C₁₋₆ haloalkyl, halo, D, CN, OR^{a2}, and NR^{c2}R^{d2}; wherein said C₁₋₆ alkyl, is optionally substituted with 1 or 2, substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, 3 or 4 substituents independently selected from R¹⁰;
when R³N- - -CR⁴ is a single bond, then R⁴ is =O; and
R³ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R³N- - -CR⁴ is a double bond, then R³ is absent; and
R⁴ is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, and S(O)₂R^{b3}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R⁵N- - -YR⁶ is a single bond and Y is C, then YR⁶ is C=O; and
R⁵ is selected from H, C₁₋₆ alkyl, and C₁₋₆ haloalkyl; wherein said C₁₋₆ alkyl, are each optionally substituted with 1, 2, 3, or 4 substituents independently selected from R⁵⁰;
when R⁵N- - -YR⁶ is a double bond and Y is N, then R⁵ and R⁶ are absent;
when R⁵N- - -YR⁶ is a double bond and Y is C, then R⁵ is absent; and
R⁶ is selected from H, C₁₋₆ alkyl, and C₁₋₆ haloalkyl;
R⁷ is selected from H, C₁₋₆ alkyl, C₁₋₆ haloalkyl, halo, D, CN, OR^{a7}, and NR^{c7}R^{d7};
Cy² is selected from 4-10 membered heterocycloalkyl, each has at least one ring-forming carbon atom and 1, 2, 3, or 4 ring-forming heteroatoms independently selected from N, O, and S; wherein a ring-forming carbon atom of 4-10 membered heterocycloalkyl is optionally substituted by oxo to form a carbonyl group; and wherein the 4-10 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, halo, D, CN, OR^{a10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, and S(O)₂R^{b10};
each R²⁰ is independently selected from C₁₋₆ alkyl, C₂₋₆ alkenyl, C₂₋₆ alkynyl, C₁₋₆ haloalkyl, halo, D, CN, OR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, and S(O)₂R^{b20}; wherein said C₁₋₆ alkyl, C₂₋₆ alkenyl, and C₂₋₆ alkynyl, are each optionally substituted with 1, 2, 3, or 4 substituents independently selected from R²¹;
each R²¹ is independently selected from C₁₋₆ alkyl, C₁₋₆ haloalkyl, halo, D, CN, OR^{a21}, and NR^{c21}R^{d21};
each R²² is independently selected from C₁₋₆ alkyl, C₁₋₆ haloalkyl, halo, D, and CN;
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, and S(O)₂R^{b30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³¹;
each R³¹ is independently selected from C₁₋₆ alkyl, C₁₋₆ haloalkyl, halo, D, CN, OR^{a31}, and NR^{c31}R^{d31};
each R⁵⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a50}, and NR^{c50}R^{d50}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁵¹;
each R⁵¹ is independently selected from C₁₋₆ alkyl, C₁₋₆ haloalkyl, halo, D, CN, OR^{a51}, and NR^{c51}R^{d51};
each R^{a2}, R^{c2} and R^{d2} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl;
or any R^{c2} and R^{d2} attached to the same N atom, together with the N atom to which they are attached, form a 4-, 5-, 6- or 7-membered heterocycloalkyl group;
each R^{a3}, R^{b3}, R^{c3} and R^{d3} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{j3} is independently selected from C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a7}, R^{c7} and R^{d7} is independently selected from H, C₁₋₆ alkyl, and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, and C₁₋₆ haloalkyl; wherein said C₁₋₆ alkyl, C₂₋₆ alkenyl, and C₂₋₆ alkynyl, are each optionally substituted with 1, 2, 3, or 4 substituents independently selected from R²¹;
each R^{a21}, R^{c21} and R^{d21}, is independently selected from H, C₁₋₆ alkyl, and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{a31}, R^{c31} and R^{d31}, is independently selected from H, C₁₋₆ alkyl, and C₁₋₆ haloalkyl;
each R^{a50}, R^{c50} and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁵¹;
or any R^{c50} and R^{d50} attached to the same N atom, together with the N atom to which they are attached, form a 4-, 5-, or 6-membered heterocycloalkyl group optionally substituted with 1, 2 or 3 substituents independently selected from R⁵¹; and
each R^{a51}, R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl.

In another embodiment of a compound of Formula I, or pharmaceutically acceptable salt thereof,
each --- represents a single bond or a double bond;
X is N or CR⁷;
Y is N or C;
R¹ is selected from H, D, C₁₋₃ alkyl, C₁₋₃ haloalkyl, halo, and CN;
R² is selected from H, C₁₋₃ alkyl, C₁₋₃ haloalkyl, halo, D, and CN; wherein said C₁₋₃ alkyl, is optionally substituted with 1 or 2, substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, or 3 ring-forming heteroatoms independently selected from N and O; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2 or 3 substituents independently selected from R¹⁰;
when R³N---CR⁴ is a single bond, then R⁴ is =O; and
R³ is selected from H, C₁₋₃ alkyl, C₁₋₃ haloalkyl, phenyl, and 5-6 membered heteroaryl; wherein said C₁₋₃ alkyl, phenyl, and 5-6 membered heteroaryl, are each optionally substituted with 1 or 2 substituents independently selected from R³⁰;
when R³N---CR⁴ is a double bond, then R³ is absent; and
R⁴ is selected from H, C₁₋₃ alkyl, C₁₋₃ haloalkyl, C₃₋₆ cycloalkyl, 4-6 membered heterocycloalkyl, C₆₋₁₀ aryl, 5-6 membered heteroaryl, halo, D, CN, OR^{a3}, and NR^{c3}R^{j3}; wherein said C₁₋₃ alkyl, C₃₋₆ cycloalkyl, 4-6 membered heterocycloalkyl, C₆₋₁₀ aryl, and 5-6 membered heteroaryl, are each optionally substituted with 1 or 2 substituents independently selected from R³⁰;
when R⁵N---YR⁶ is a single bond and Y is C, then YR⁶ is C=O; and
R⁵ is selected from H, C₁₋₃ alkyl, and C₁₋₃ haloalkyl; wherein said C₁₋₃ alkyl, is optionally substituted with 1 or 2 substituents independently selected from R⁵⁰;
when R⁵N---YR⁶ is a double bond and Y is N, then R⁵ and R⁶ are absent;
when R⁵N---YR⁶ is a double bond and Y is C, then R⁵ is absent; and R⁶ is selected from H, C₁₋₃ alkyl, and C₁₋₃ haloalkyl;
R⁷ is selected from H, C₁₋₃ alkyl, C₁₋₃ haloalkyl, halo, D, and CN;
Cy² is selected from 4-6 membered heterocycloalkyl, each has at least one ring-forming carbon atom and 1, 2, 3, or 4 ring-forming heteroatoms independently selected from N, O, and S; wherein a ring-forming carbon atom of 4-6 membered heterocycloalkyl is optionally substituted by oxo to form a carbonyl group; and wherein the 4-6 membered heterocycloalkyl, is optionally substituted with 1 or 2 substituents independently selected from R²²;
each R¹⁰ is independently selected from C₁₋₃ alkyl, C₁₋₃ haloalkyl, C₃₋₅ cycloalkyl, halo, D, CN, OR^{a10}, and NR^{c10}R^{d10};
each R²⁰ is independently selected from C₁₋₃ alkyl, C₂₋₃ alkenyl, C₂₋₃ alkynyl, C₁₋₃ haloalkyl, halo, D, CN, OR^{a20}, C(O)R^{b20}, C(O)NR^{c20}R^{d20}, C(O)OR^{a20}, and NR^{c20}R^{d20}; wherein said C₁₋₃ alkyl, C₂₋₃ alkenyl, and C₂₋₃ alkynyl, are each optionally substituted with 1 or 2 substituents independently selected from R²¹;
each R²¹ is independently selected from C₁₋₃ alkyl, C₁₋₃ haloalkyl, halo, D, CN OR^{a21}, and NR^{c21}R^{d21};
each R²² is independently selected from C₁₋₆ alkyl, C₁₋₆ haloalkyl, halo, D, and CN;
each R³⁰ is independently selected from C₁₋₃ alkyl, C₁₋₃ haloalkyl, C₃₋₆ cycloalkyl, 4-6 membered heterocycloalkyl, phenyl, 5-6 membered heteroaryl, halo, D, CN, OR^{a30}, and NR^{c30}R^{d30}; wherein said C₁₋₃ alkyl, C₃₋₆ cycloalkyl, 4-6 membered heterocycloalkyl, phenyl, and 5-6 membered heteroaryl, are each optionally substituted with 1 or 2 substituents independently selected from R³¹;
each R³¹ is independently selected from C₁₋₃ alkyl, C₁₋₃ haloalkyl, halo, D, and CN;
each R⁵⁰ is independently selected from C₁₋₃ alkyl, C₁₋₃ haloalkyl, C₃₋₆ cycloalkyl, 4-6 membered heterocycloalkyl, halo, D, and CN; wherein said C₁₋₃ alkyl, C₃₋₆ cycloalkyl, and 4-6 membered heterocycloalkyl, are each optionally substituted with 1 or 2 substituents independently selected from R⁵¹;
each R⁵¹ is independently selected from C₁₋₃ alkyl, C₁₋₃ haloalkyl, halo, D, and CN;
each R^{a3} and R^{c3} is independently selected from H, C₁₋₃ alkyl, and C₁₋₃ haloalkyl; wherein said C₁₋₃ alkyl, is optionally substituted with 1 or 2 substituents independently selected from R³⁰;
each R^{j3} is independently selected from C₁₋₃ alkyl, and C₁₋₃ haloalkyl; wherein said C₁₋₃ alkyl, is optionally substituted with 1 or 2 substituents independently selected from R³⁰;
each R^{a10}, R^{b10}, R^{c10} and R^{d10} is independently selected from H, C₁₋₃ alkyl, and C₁₋₃ haloalkyl;
each R^{a20}, R^{b20}, R^{c20} and R^{d20} is independently selected from H, C₁₋₃ alkyl, C₂₋₃ alkenyl, C₂₋₃ alkynyl, and C₁₋₃ haloalkyl; wherein said C₁₋₃ alkyl, C₂₋₃ alkenyl, and C₂₋₃ alkynyl, are each optionally substituted with 1 or 2 substituents independently selected from R²¹;
each R^{a21}, R^{c21} and R^{d21}, is independently selected from H, C₁₋₆ alkyl, and C₁₋₆ haloalkyl; and
each R^{a30}, R^{c30} and R^{d30} is independently selected from H, C₁₋₃ alkyl, and C₁₋₃ haloalkyl; wherein said C₁₋₃ alkyl, are each optionally substituted with 1 or 2 substituents independently selected from R³¹.

In yet another embodiment of a compound of Formula I, or pharmaceutically acceptable salt thereof,
each --- represents a single bond or a double bond;
X is N or CR⁷;
Y is N or C;
R¹ is H;
R² is selected from H, C₁₋₆ alkyl, C₁₋₆ haloalkyl, halo, and CN; wherein said C₁₋₆ alkyl, is optionally substituted with 1 or 2, substituents independently selected from R²²;
Cy¹ is selected from C₃₋₁₀ cycloalkyl, 4-10 membered heterocycloalkyl, C₆₋₁₀ aryl and 6-10 membered heteroaryl; each optionally substituted with 1, 2, 3 or 4 substituents independently selected from R¹⁰;
when R³N---CR⁴ is a single bond, then R⁴ is =O; and
R³ is selected from C₁₋₆ alkyl, C₆₋₁₀ aryl, and 5-10 membered heteroaryl; each optionally substituted with 1, 2, 3, or 4 substituents independently selected from R³⁰;
when R³N---CR⁴ is a double bond, then R³ is absent; and
R⁴ is selected from H, 4-10 membered heterocycloalkyl, C₆₋₁₀ aryl, 5-10 membered heteroaryl, and OR^{a3}; wherein said C₁₋₆ alkyl, 4-10 membered heterocycloalkyl, and C₆₋₁₀ aryl, are each optionally substituted with 1, 2, 3, or 4 substituents independently selected from R³⁰;
when R⁵N---YR⁶ is a single bond and Y is C, then YR⁶ is C=O; and
R⁵ is selected from H, and C₁₋₆ alkyl; wherein said C₁₋₆ alkyl, is optionally substituted with 1, 2, 3, or 4 substituents independently selected from R⁵⁰;
when R⁵N---YR⁶ is a double bond and Y is N, then R⁵ and R⁶ are absent;
when R⁵N---YR⁶ is a double bond and Y is C, then R⁵ is absent; and R⁶ is selected from H;
R⁷ is selected from H and halo;
Cy² is selected from 4-10 membered heterocycloalkyl, optionally substituted with 1, 2, 3 or 4 substituents independently selected from R²⁰;
each R¹⁰ is independently selected from C₁₋₆ alkyl, C₁₋₆ haloalkyl, C₃₋₁₀ cycloalkyl, halo, CN, and OR^{a10};
each R²² is independently selected from C₁₋₆ alkyl, CN, OR^{a20}, C(O)R^{b20}, and C(O)NR^{c20}R^{d20}; wherein said C₁₋₆ alkyl, is optionally substituted with 1, 2, 3, or 4 substituents independently selected from R²¹;
each R²¹ is independently selected from C₁₋₆ haloalkyl, halo, CN, OR^{a21}, and NR^{c21}R^{d21};
each R²² is CN;
each R³⁰ is independently selected from C₁₋₆ alkyl, 4-10 membered heterocycloalkyl, 5-10 membered heteroaryl, halo, OR^{a30}, and NR^{c30}R^{d30}; wherein said C₁₋₆ alkyl, 4-10 membered heterocycloalkyl, and 5-10 membered heteroaryl, are each optionally substituted with 1, 2, 3, or 4 substituents independently selected from R³¹;
each R³¹ is C₁₋₆ alkyl;
each R⁵⁰ is 4-10 membered heterocycloalkyl, optionally substituted with 1, 2, 3, or 4 substituents independently selected from R⁵¹;
each R⁵¹ is C₁₋₆ alkyl;
each R^{a3}, is C₁₋₆ alkyl, optionally substituted with 1, 2, 3, or 4 substituents independently selected from R³⁰;
each R^{a10}, is independently selected from H, and C₁₋₆ alkyl;
each R^{a20}, R^{b20}, R^{c20} and R^{d20} is independently selected from H, C₁₋₆ alkyl, C₂₋₆ alkenyl and C₂₋₆ alkynyl; wherein said C₁₋₆ alkyl, C₂₋₆ alkenyl, and C₂₋₆ alkynyl are each optionally substituted with 1, 2, 3, or 4 substituents independently selected from R²¹;
each R^{a21}, R^{c21} and R^{d21}, is independently selected from H and C₁₋₆ alkyl; and
each R^{a30}, R^{c30} and R^{d30} is independently selected from H, and C₁₋₆ alkyl.

In still another embodiment of a compound of Formula I, or a pharmaceutically acceptable salt thereof,
each --- represents a single bond or a double bond;
X is N or CR⁷;
Y is N or ;
R¹ is selected from H, D, C₁₋₆ alkyl, C₁₋₆ haloalkyl, halo, and CN;
R² is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a2}, and NR^{c2}R^{d2}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, 3 or 4 substituents independently selected from R¹⁰;
when R³N---CR⁴ is a single bond, then R⁴ is =O; and
R³ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R³N---CR⁴ is a double bond, then R³ is absent; and
R⁴ is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, and S(O)₂R^{b3}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R⁵N---YR⁶ is a single bond and Y is C, then YR⁶ is C=O; and
R⁵ is selected from H, C₁₋₆ alkyl, and C₁₋₆ haloalkyl; wherein said C₁₋₆ alkyl, are each optionally substituted with 1, 2, 3, or 4 substituents independently selected from R⁵⁰;
when R⁵N---YR⁶ is a double bond and Y is N, then R⁵ and R⁶ are absent;
when R⁵N---YR⁶ is a double bond and Y is C, then R⁵ is absent; and
R⁶ is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, and S(O)₂R^{b6}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁶⁰;
R⁷ is selected from H, C₁₋₆ alkyl, C₁₋₆ haloalkyl, halo, D, CN, OR^{a7}, and NR^{c7}R^{d7}; wherein said C₁₋₆ alkyl is optionally substituted with 1, 2, 3, or 4 substituents independently selected from R⁷⁰;
Cy² is selected from 4-10 membered heterocycloalkyl, each has at least one ring-forming carbon atom and 1, 2, 3, or 4 ring-forming heteroatoms independently selected from N, O, and S; wherein a ring-forming carbon atom of 4-10 membered heterocycloalkyl is optionally substituted by oxo to form a carbonyl group; and wherein the 4-10 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, halo, D, CN, OR^{a10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, and S(O)₂R^{b10};
each R²⁰ is independently selected from C₁₋₆ alkyl, C₂₋₆ alkenyl, C₂₋₆ alkynyl, C₁₋₆ haloalkyl, halo, D, CN, OR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, and S(O)₂R^{b20}; wherein said C₁₋₆ alkyl, C₂₋₆ alkenyl, and C₂₋₆ alkynyl, are each optionally substituted with 1, 2, 3, or 4 substituents independently selected from R²¹;
each R²¹ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl halo, D, CN, OR^{a21}, and NR^{c21}R^{d21};
each R²² is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl halo, D, CN, OR^{a22}, and NR^{c22}R^{d22}; wherein said C₁₋₆ alkyl C₃₋₁₀ cycloalkyl, 4-10 membered heterocycloalkyl, C₆₋₁₀ aryl, and 5-10 membered heteroaryl are each optionally substituted with 1, 2, 3, or 4 substituents independently selected from R²³;
each R²³ is independently selected from C₁₋₆ alkyl, C₁₋₆ haloalkyl, halo, D, CN, OR^{a23}, and NR^{c23}R^{d23};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, and S(O)₂R^{b30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³¹;
each R³¹ is independently selected from C₁₋₆ alkyl, C₁₋₆ haloalkyl, halo, D, CN, OR^{a31}, and NR^{c31}R^{d31};
each R⁵⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a50}, and NR^{c50}R^{d50};
each R⁶⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, and S(O)₂R^{b60}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁶¹;
each R⁶¹ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a61}, and NR^{c61}R^{d61};
each R⁷⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a70}, and NR^{c70}R^{d70};
each R^{a2}, R^{c2} and R^{d2} is independently selected from H, C₁₋₆ alkyl, C₁₋₆ haloalkyl, C₃₋₁₀ cycloalkyl, 4-10 membered heterocycloalkyl, C₆₋₁₀aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²²;
or any R^{c2} and R^{d2} attached to the same N atom, together with the N atom to which they are attached, form a 4-, 5-, 6- or 7-membered heterocycloalkyl group optionally substituted with 1, 2 or 3 substituents independently selected from R²²;
each R^{a3}, R^{b3}, R^{c3} and R^{d3} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
**each** R^{j3} is independently selected from C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶⁰;
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R⁶⁰;
each R^{a7}, R^{c7} and R^{d7} is independently selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷⁰;
or any R^{c7} and R^{d7} attached to the same N atom, together with the N atom to which they are attached, form a 4-, 5-, 6- or 7-membered heterocycloalkyl group optionally substituted with 1, 2, 3, or 4 substituents independently selected from R⁷⁰;
each R^{a10}, R^{b10}, R^{c10} and R^{d10} is independently selected from H, C₁₋₆ alkyl, C₂₋₆ alkenyl, C₂₋₆ alkynyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, and C₁₋₆ haloalkyl; wherein said C₁₋₆ alkyl, C₂₋₆ alkenyl, and C₂₋₆ alkynyl, are each optionally substituted with 1, 2, 3, or 4 substituents independently selected from R²¹;
each R^{a21}, R^{c21} and R^{d21}, is independently selected from H, C₁₋₆ alkyl, and C₁₋₆ haloalkyl;
each R^{a22}, R^{c22} and R^{d22}, is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²³;
**or any** R^{c22} and R^{d22} attached to the same N atom, together with the N atom to which they are attached, form a 4-, 5-, 6- or 7-membered heterocycloalkyl group optionally substituted with 1, 2, 3, or 4 substituents independently selected from R²³;
each R^{a23}, R^{c23} and R^{d23}, is independently selected from H, C₁₋₆ alkyl, and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{a31}, R^{c31} and R^{d31}, is independently selected from H, C₁₋₆ alkyl, and C₁₋₆ haloalkyl;
each R^{a50}, R^{c50} and R^{d50}, is independently selected from H, C₁₋₆ alkyl, and C₁₋₆ haloalkyl;
each R^{a60}, R^{b60}, R^{c60} and R^{d60} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶¹;
or any R^{c60} and R^{d60} attached to the same N atom, together with the N atom to which they are attached, form a 4-, 5-, 6- or 7-membered heterocycloalkyl group optionally substituted with 1, 2, 3, or 4 substituents independently selected from R⁶¹;
each R^{a61}, R^{c61} and R^{d61}, is independently selected from H, C₁₋₆ alkyl, and C₁₋₆ haloalkyl; and
each R^{a70}, R^{c70} and R^{d70}, is independently selected from H, C₁₋₆ alkyl, and C₁₋₆ haloalkyl.

In another embodiment of Formula II:
--- represents a single bond or a double bond;
Y is N or C;
R¹ is selected from H, D, C₁₋₆ alkyl, C₂₋₆ alkenyl, C₂₋₆ alkynyl, C₁₋₆ haloalkyl, halo, CN, OR^{a1}, and NR^{c1}R^{d1};
R² is selected from H, D, C₁₋₆ alkyl, C₂₋₆ alkenyl, C₂₋₆ alkynyl, C₁₋₆ haloalkyl, halo, CN, OR^{a2}, and NR^{c2}R^{d2};
Cy¹ is selected from C₆₋₁₀ aryl and 6-10 membered heteroaryl; wherein the 6-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 6-10 membered heteroaryl is optionally substituted by oxo to form a carbonyl group; and wherein the C₆₋₁₀ aryl and 6-10 membered heteroaryl are each optionally substituted with 1, 2, 3 or 4 substituents independently selected from R¹⁰;
R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, and S(O)₂R^{b3}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R⁵N---YR⁶ is a single bond and Y is C, then YR⁶ is selected from C=O and C=S; and
R⁵ is selected from H, C₁₋₆ alkyl, C₂₋₆ alkenyl, C₂₋₆ alkynyl, and C₁₋₆ haloalkyl;
when R⁵N---YR⁶ is a double bond and Y is N, then R⁵ and R⁶ are absent;
when R⁵N---YR⁶ is a double bond and Y is C, then R⁵ is absent; and
R⁶ is selected from H, D, C₁₋₆ alkyl, C₂₋₆ alkenyl, C₂₋₆ alkynyl, C₁₋₆ haloalkyl, halo, CN, OR^{a6}, and NR^{c6}R^{d6};
R⁷ is selected from H, C₁₋₆ alkyl, C₂₋₆ alkenyl, C₂₋₆ alkynyl, C₁₋₆ haloalkyl, halo, D, CN, OR^{a7}, and NR^{c7}R^{d7};
Cy² is 4-10 membered heterocycloalkyl; wherein the 4-10 membered heterocycloalkyl has at least one ring-forming carbon atom and 1, 2, 3, or 4 ring-forming heteroatoms independently selected from N, O, and S; wherein the N and S are optionally oxidized; wherein a ring-forming carbon atom of 4-10 membered heterocycloalkyl is optionally substituted by oxo to form a carbonyl group; and wherein the 4-10 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, halo, D, CN, OR^{a10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, and S(O)₂R^{b10};
each R²⁰ is independently selected from C₁₋₆ alkyl, C₂₋₆ alkenyl, C₂₋₆ alkynyl, C₁₋₆ haloalkyl, halo, D, CN, OR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, and S(O)₂R^{b20};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, and S(O)₂R^{b30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, halo, D, CN, OR^{a31}, and NR^{c31}R^{d31};
each R^{a1}, R^{c1}, and R^{d1} is independently selected from H, C₁₋₆ alkyl, C₂₋₆ alkenyl, C₂₋₆ alkynyl, and C₁₋₆ haloalkyl;
each R^{a2}, R^{c2}, and R^{d2} is independently selected from H, C₁₋₆ alkyl, C₂₋₆ alkenyl, C₂₋₆ alkynyl, and C₁₋₆ haloalkyl;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{c6}, and R^{d6} is independently selected from H, C₁₋₆ alkyl, C₂₋₆ alkenyl, C₂₋₆ alkynyl, and C₁₋₆ haloalkyl;
each R^{a7}, R^{c7} and R^{d7} is independently selected from H, C₁₋₆ alkyl, C₂₋₆ alkenyl, C₂₋₆ alkynyl, and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹; and
each R^{a31}, R^{c31} and R^{d31}, is independently selected from H, C₁₋₆ alkyl, C₂₋₆ alkenyl, C₂₋₆ alkynyl, and C₁₋₆ haloalkyl.

Provided below are additional embodiments of the formulae provided herein.

In an embodiment, R¹ is selected from H, D, C₁₋₆ alkyl, C₂₋₆ alkenyl, C₂₋₆ alkynyl, C₁₋₆ haloalkyl, halo, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)OR^{a}, NR^{c}C(O)NR^{c}R^{d}, NR^{c}S(O)₂R^{b}, S(O)₂R^{b}, and S(O)₂NR^{c}R^{d}; wherein said C₁₋₆ alkyl, C₂₋₆ alkenyl, and C₂₋₆ alkynyl, are each optionally substituted with 1 or 2 substituents independently selected from R⁹.

In another embodiment, R¹ is selected from H, D, C₁₋₆ alkyl, C₂₋₆ alkenyl, C₂₋₆ alkynyl, C₁₋₆ haloalkyl, halo, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)OR^{a}, NR^{c}C(O)NR^{c}R^{d}, NR^{c}S(O)₂R^{b}, S(O)₂R^{b}, and S(O)₂NR^{c}R^{d.}

In an embodiment, R¹ is selected from H, D, C₁₋₆ alkyl, C₂₋₆ alkenyl, C₂₋₆ alkynyl, C₁₋₆ haloalkyl, halo, CN, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)₂R^{b1}, S(O)₂R^{b1}, and S(O)₂NR^{c1}R^{d1}; wherein said C₁₋₆ alkyl, C₂₋₆ alkenyl, and C₂₋₆ alkynyl, are each optionally substituted with 1 or 2 substituents independently selected from R^{g}.

In another embodiment, R¹ is selected from H, D, C₁₋₆ alkyl, C₂₋₆ alkenyl, C₂₋₆ alkynyl, C₁₋₆ haloalkyl, halo, CN, OR^{a1}, SR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)₂R^{b1}, S(O)₂R^{b1}, and S(O)₂NR^{c1}R^{d1}.

In yet another embodiment, R¹ is selected from H, D, C₁₋₆ alkyl, C₂₋₆ alkenyl, C₂₋₆ alkynyl, C₁₋₆ haloalkyl, halo, and CN. In still another embodiment, R¹ is selected from H, D, and C₁₋₃ alkyl. In an embodiment, R¹ is H.

In another embodiment, R² is selected from H, D, C₁₋₆ alkyl, C₂₋₆ alkenyl, C₂₋₆ alkynyl, C₁₋₆ haloalkyl, halo, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)OR^{a}, NR^{c}C(O)NR^{c}R^{d}, NR^{c}S(O)₂R^{b}, S(O)₂R^{b}, and S(O)₂NR^{c}R^{d}; wherein said C₁₋₆ alkyl, C₂₋₆ alkenyl, and C₂₋₆ alkynyl, are each optionally substituted with 1 or 2 substituents independently selected from R^{g}.

In another embodiment R² is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a2}, and NR^{c2}R^{d2}. In another embodiment R² is selected from H, C₁₋₃ alkyl, C₁₋₃ haloalkyl, phenyl, 5-6 membered heteroaryl, halo, D, and CN. In another embodiment R² is selected from H, C₁₋₃ alkyl, 5-6 membered heteroaryl, halo, and CN.

In yet another embodiment, R² is selected from H, D, C₁₋₆ alkyl, C₂₋₆ alkenyl, C₂₋₆ alkynyl, C₁₋₆ haloalkyl, halo, CN, OR^{a}, SR^{a}, C(O)R^{b}, C(O)NR^{c}R^{d}, C(O)OR^{a}, OC(O)R^{b}, OC(O)NR^{c}R^{d}, NR^{c}R^{d}, NR^{c}C(O)R^{b}, NR^{c}C(O)OR^{a}, NR^{c}C(O)NR^{c}R^{d}, NR^{c}S(O)₂R^{b}, S(O)₂R^{b}, and S(O)₂NR^{c}R^{d.}

In still another embodiment, R² is selected from H, D, C₁₋₆ alkyl, C₁₋₆ haloalkyl, halo, and CN. In another embodiment, R² is selected from C₁₋₆ alkyl, C₁₋₆ haloalkyl, and halo. In an embodiment, R² is halo. In another embodiment, R² is chloro.

In an embodiment, R² is selected from H, C₁₋₆ alkyl, C₁₋₆ haloalkyl, halo, and CN; wherein said C₁₋₆ alkyl is optionally substituted with 1 or 2 substituents independently selected from R²².

In yet another embodiment, Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, or 3 substituents independently selected from R¹⁰.

In still another embodiment, Cy¹ is selected from C₆₋₁₀ aryl and 6-10 membered heteroaryl; wherein the 6-10 membered heteroaryl each has at least one ring-forming carbon atom and 1, 2, or 3 ring-forming heteroatoms independently selected from N, O, and S; wherein the N and S are optionally oxidized; wherein a ring-forming carbon atom of 6-10 membered heteroaryl is optionally substituted by oxo to form a carbonyl group; and wherein the C₆₋₁₀ aryl and 6-10 membered heteroaryl are each optionally substituted with 1, 2, or 3 substituents independently selected from R¹⁰.

In an embodiment, Cy¹ is selected from phenyl, naphthalenyl, quinolinyl, isoquinolinyl, indazolyl, chromanyl, 2,3-dihydro-1H-indenyl, 2,3-dihydrobenzo[b][1,4]dioxinyl, and pyridinyl; wherein the phenyl, naphthalenyl, quinolinyl, isoquinolinyl, indazolyl, chromanyl, 2,3-dihydro-1H-indenyl, 2,3-dihydrobenzo[b][1,4]dioxinyl, and pyridinyl are each optionally substituted with 1, 2, or 3 substituents independently selected from R¹⁰. In an embodiment, Cy¹ is selected from phenyl, naphthalenyl and indazolyl; wherein the phenyl, naphthalenyl, quinolinyl, isoquinolinyl, indazolyl, chromanyl, 2,3-dihydro-1H-indenyl, and 2,3-dihydrobenzo[b][1,4]dioxinyl are each optionally substituted with 1, 2, or 3 substituents independently selected from R¹⁰. In another embodiment, R¹⁰ is halo or OH.

In yet another embodiment, Cy¹ is selected from 3-hydroxy-naphthalen-1-yl, 2-fluoro-6-hydroxyphenyl, 2-fluoro-3-hydroxyphenyl, and 5-methyl-1H-indazol-4-yl.

In another embodiment, Cy¹ is C₆₋₁₀ aryl or 6-10 membered heteroaryl, both of which are optionally substituted with one or two R¹⁰. In yet another embodiment, Cy¹ is C₆₋₁₀ aryl optionally substituted with one or two R¹⁰. In still another embodiment, R¹⁰ is halo. In an embodiment, Cy¹ is 6-10 membered heteroaryl optionally substituted with one or two R¹⁰. In another embodiment, R¹⁰ is selected from C₁₋₆ alkyl, halo, and OH. In yet another embodiment, Cy¹ is 5-10 membered heteroaryl provided that Cy¹ is other than 3,5-dimethylisoxazol-4-yl or 3,5-dimethyl-1H-pyrazol-4-yl.

In an embodiment, X is N. In another embodiment, X is CR⁷.

In yet another embodiment, R⁷ is selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl, halo, D, CN, OR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7} NR^{c7}S(O)₂R^{b7}, S(O)₂R^{b7}, and S(O)₂NR^{c7}R^{d7}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R⁷⁰.

In still another embodiment, R⁷ is selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl, halo, D, CN, OR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7} NR^{c7}S(O)₂R^{b7}, S(O)₂R^{b7}, and S(O)₂NR^{c7}R^{d7}.

In an embodiment, R⁷ is selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl, halo, D, CN, OR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7} NR^{c7}S(O)₂R^{b7}, S(O)₂R^{b7}, and S(O)₂NR^{c7}R^{d7}.

In another embodiment, R⁷ is selected from H, D, C₁₋₆ alkyl, C₁₋₆ haloalkyl, halo, and CN. In an embodiment, R⁷ is selected from H and halo. In yet another embodiment, R⁷ is halo. In still another embodiment, R⁷ is fluoro.

In an embodiment, when R³N- - -CR⁴ is a single bond, then R⁴ is =O. In another embodiment, when R³N- - -CR⁴ is a single bond, then R⁴ is =S. In yet another embodiment, when R³N- - -CR⁴ is a double bond, then R³ is absent.

In still another embodiment, R³ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl, wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, or 3 substituents independently selected from R³⁰.

In an embodiment, R³ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl. In another embodiment R³ is selected from H, C₁₋₆ alkyl, C₁₋₆ haloalkyl, phenyl, and 5-6 membered heteroaryl; wherein said C₁₋₆ alkyl, phenyl, and 5-6 membered heteroaryl, are each optionally substituted with 1 or 2 substituents independently selected from R³⁰. In another embodiment R³ is selected from C₁₋₃ alkyl, phenyl, and 5-6 membered heteroaryl; wherein said C₁₋₃ alkyl, phenyl, and 5-6 membered heteroaryl, are each optionally substituted with 1 or 2 substituents independently selected from R³⁰.

In another embodiment, R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{d3}, NR^{c3}C(O)R^{b3}, NR^{c3}C(O)OR^{a3}, NR^{c3}C(O)NR^{c3}R ^{d3}, NR^{c3}S(O)₂R^{b3}, S(O)₂R^{b3}, and S(O)₂NR^{c3}R^{d3}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰.

In yet another embodiment, R⁴ is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a3}, C(O)R^{b3}, C(O)NR^{c3}R ^{d3}, C(O)OR^{a3}, OC(O)R^{b3}, OC(O)NR^{c3}R^{d3}, NR^{c3}R^{d3}, NR^{c3}C(O)R^{b3}, and S(O)₂R^{b3}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰.

In still another embodiment, R⁴ is selected from H, C₁₋₆ alkyl, C₁₋₆ haloalkyl, 4-10 membered heterocycloalkyl, C₆₋₁₀ aryl, 5-10 membered heteroaryl, halo, D, CN, OR^{a3}, and NR^{c3}R^{d3}; wherein said C₁₋₆ alkyl, 4-10 membered heterocycloalkyl, C₆₋₁₀ aryl, and 5-10 membered heteroaryl, are each optionally substituted with 1, 2, or 3 substituents independently selected from R³⁰.

In another embodiment, R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, NR^{c3}C(O)NR^{c3}R^{d3}, NR^{c3}S(O)₂R^{b3}, S(O)₂R^{b3}, and S(O)₂NR^{c3}R^{d3}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰.

In yet another embodiment, R⁴ is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3} , and S(O)₂R^{b3}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰.

In still another embodiment, R⁴ is selected from H, C₁₋₆ alkyl, C₁₋₆ haloalkyl, 4-10 membered heterocycloalkyl, C₆₋₁₀ aryl, 5-10 membered heteroaryl, halo, D, CN, OR^{a3}, and NR^{c3}R^{j3}; wherein said C₁₋₆ alkyl, 4-10 membered heterocycloalkyl, C₆₋₁₀ aryl, and 5-10 membered heteroaryl, are each optionally substituted with 1, 2, or 3 substituents independently selected from R³⁰.

In an embodiment, R⁴ is selected from H, 4-10 membered heterocycloalkyl, C₆₋₁₀ aryl, 5-10 membered heteroaryl, and OR^{a3}; wherein said 4-10 membered heterocycloalkyl, C₆₋₁₀ aryl, and 5-10 membered heteroaryl are each optionally substituted with 1 or 2 substituents independently selected from R³⁰.

In an embodiment, R⁴ is selected from H, 4-10 membered heterocycloalkyl, C₆₋₁₀ aryl, and OR^{a3}; wherein said 4-10 membered heterocycloalkyl, and C₆₋₁₀ aryl, are each optionally substituted with 1 or 2 substituents independently selected from R³⁰.In another embodiment, R⁴ is selected from H, 1-(methylpyrrolidin-2-yl)methoxy, 1-(methyl-1H-pyrazol-5-yl)methoxy, 3-(dimethylamino)azetidin-1-yl, 3-hydroxynaphthalen-1-yl, and 2-fluoro-6-hydroxyphenyl.

In an embodiment, R⁴ is selected from 3-(dimethylamino)-azetidin-1-yl, 3-(dimethylamino)-3-methylazetidin-1-yl, ((S)-1-methylpyrrolidin-2-yl)methoxy, ((S)-1-(dimethylamino)propan-2-yl)oxy, and 2-methyl-1H-imidazol-1-yl.

In an embodiment, R⁴ is selected from H, 4-10 membered heterocycloalkyl, C₆₋₁₀ aryl, and OR^{a3}, wherein heterocycloalkyl and aryl are each optionally substituted with one or two R³⁰. In another embodiment, R⁴ is C₆₋₁₀ aryl optionally substituted with one or two R³⁰. In yet another embodiment, R³⁰ is independently OH or halo. In another embodiment, R⁴ is 4-10 membered heterocycloalkyl optionally substituted with one or two R³⁰.

In still another embodiment,
R⁴ is OR^{a3};
R^{a3} is C₁₋₆ alkyl substituted with R³⁰; and
R³⁰ is 4-6 membered heterocycloalkyl or 5-10 membered heteroaryl, both of which are optionally substituted with C₁₋₃ alkyl.

In an embodiment, R³⁰ is pyrrolidine optionally substituted with C₁₋₃ alkyl. In another embodiment, R³⁰ is pyrazole optionally substituted with C₁₋₃ alkyl.

In yet another embodiment, R⁴ is azetidine optionally substituted with NR^{c30}R^{d30}. In still another embodiment, R^{c30} and R^{d30} are each C₁₋₃ alkyl.

In yet another embodiment, Y is N. In still another embodiment, Y is C. In another embodiment, when R⁵N- - -YR⁶ is a single bond, Y is C, and YR⁶ is C=O. In yet another embodiment, when R⁵N- - -YR⁶ is a single bond, Y is C, and YR⁶ is C=S. In still another embodiment, when R⁵N- - -YR⁶ is a single bond, Y is N, and YR⁶ is NH. In another embodiment, when R⁵N- - -YR⁶ is a double bond, Y is N, and R⁵ and R⁶ are absent. In another embodiment when R⁵N- - -YR⁶ is a double bond and Y is C, then R⁵ is absent.

In an embodiment, R⁵ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl, wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, or 3 substituents independently selected from R⁵⁰.

In another embodiment, R⁵ is selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, or 3 substituents independently selected from R⁵⁰.

In yet another embodiment, R⁵ is selected from H, C₁₋₆ alkyl, C₁₋₆ haloalkyl, C₃₋₆ cycloalkyl, and 4-6 membered heterocycloalkyl. In still another embodiment, R⁵ is H. In another embodiment, R⁵ is selected from H, C₁₋₆ alkyl, and C₁₋₆ haloalkyl; wherein said C₁₋₆ alkyl is optionally substituted by 1 or 2 substituents independently selected from R⁵⁰. In another embodiment R⁵ is selected from H, C₁₋₃ alkyl; wherein said C₁₋₃ alkyl, is optionally substituted with 1 or 2 substituents independently selected from R⁵⁰.

In an embodiment, R⁶ is selected from H, D, C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, CN, OR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, NR^{c6}S(O)₂R^{b6}, S(O)₂R^{b6}, and S(O)₂NR^{c6}R^{d6}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R^{g}.
In another embodiment, R⁶ is selected from H, C₁₋₃ alkyl, C₁₋₃ haloalkyl, C₃₋₆ cycloalkyl, 4-6 membered heterocycloalkyl, phenyl, 5-6 membered heteroaryl, halo, D, CN, OR^{a6}, and NR^{c6}R^{d6}; wherein said C₁₋₃ alkyl, C₃₋₆ cycloalkyl, 4-6 membered heterocycloalkyl, phenyl, and 5-6 membered heteroaryl, are each optionally substituted with 1 or 2 substituents independently selected from R⁶⁰. In another embodiment, R⁶ is selected from H, C₁₋₃ alkyl, 4-6 membered heterocycloalkyl, and 5-6 membered heteroaryl; wherein said C₁₋₃ alkyl, 4-6 membered heterocycloalkyl, and 5-6 membered heteroaryl, are each optionally substituted with 1 or 2 substituents independently selected from R⁶⁰. In yet another embodiment, R⁶ is selected from H, C₁₋₆ alkyl, C₁₋₆ haloalkyl, OR^{a6}, and NR^{c6}R^{d6}.

In another embodiment, R⁶ is selected from H, D, C₁₋₆ alkyl, C₁₋₆ haloalkyl, OR^{a6}, and NR^{c6}R^{d6}; wherein said C₁₋₆ alkyl, is optionally substituted with 1 or 2 substituents independently selected from R^{g}.

In yet another embodiment, R⁶ is selected from H, D, C₁₋₆ alkyl, C₁₋₆ haloalkyl, OR^{a6}, and NR^{c6}R^{d6}. In still another embodiment, R⁶ is H.

In an embodiment, Cy² is 4-14 membered heterocycloalkyl; wherein the 4-14 membered heterocycloalkyl has at least one ring-forming carbon atom and 1, 2, 3, or 4 ring-forming heteroatoms independently selected from N, O, and S; wherein the N and S are optionally oxidized; wherein a ring-forming carbon atom of 4-14 membered heterocycloalkyl is optionally substituted by oxo to form a carbonyl group; and wherein the 4-14 membered heterocycloalkyl, is optionally substituted with 1, 2, 3 or 4 substituents independently selected from R²⁰.

In another embodiment, Cy² is 4-10 membered heterocycloalkyl; wherein the 4-10 membered heterocycloalkyl has at least one ring-forming carbon atom and 1, 2, 3, or 4 ring-forming heteroatoms independently selected from N, O, and S; wherein the N and S are optionally oxidized; wherein a ring-forming carbon atom of 4-10 membered heterocycloalkyl is optionally substituted by oxo to form a carbonyl group; and wherein the 4-10 membered heterocycloalkyl, is optionally substituted with 1, 2, 3 or 4 substituents independently selected from R²⁰.

In yet another embodiment, Cy² is 4-6 membered heterocycloalkyl; wherein the 4-6 membered heterocycloalkyl has at least one ring-forming carbon atom and 1 or 2 ring-forming heteroatoms independently selected from N, O, and S; wherein a ring-forming carbon atom of 4-6 membered heterocycloalkyl is optionally substituted by oxo to form a carbonyl group; and wherein the 4-6 membered heterocycloalkyl, is optionally substituted with 1, 2, 3 or 4 substituents independently selected from R²⁰.

In still another embodiment, Cy² is selected from 4-(piperidin-1-yl)prop-2-en-1-one, 3-(piperidin-1-yl)prop-2-en-1-one, 3-(azetidin-1-yl)prop-2-en-1-one, and 3-(pyrrolidin-1-yl)prop-2-en-1-one. In an embodiment, Cy² is 4-(piperidin-1-yl)prop-2-en-1-one. In another embodiment, Cy² is 3-(piperidin-1-yl)prop-2-en-1-one. In yet another embodiment, Cy² is 3-(azetidin-1-yl)prop-2-en-1-one. In still another embodiment, Cy² is 3-(pyrrolidin-1-yl)prop-2-en-1-one.

In an embodiment, Cy² is 4-6 membered heterocycloalkyl optionally substituted with one or two R²⁰. In yet another embodiment, R²⁰ is C(O)R^{b20}.

In an embodiment, Cy² is selected from

| | | | |
|---|---|---|---|
| 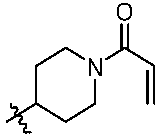 | 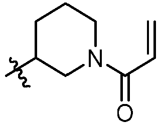 | 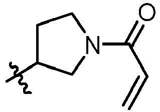 | 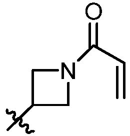 |
| Cy²-a | Cy²-b | Cy²-c, and | Cy²-d. |

In another embodiment, Cy² is selected from

| | | | | |
|---|---|---|---|---|
| 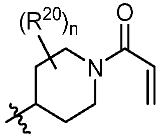 | 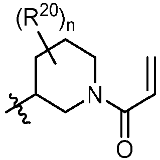 | 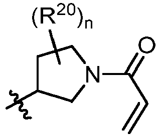 | 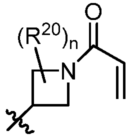 | 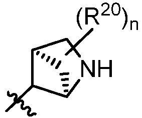 |
| Cy²-a, | Cy²-b, | Cy²-c, | Cy²-d, and | Cy²-e; |

wherein n is 0, 1 or 2.

In yet another embodiment, Cy² is selected from

| | | | |
|---|---|---|---|
| 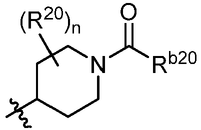 | 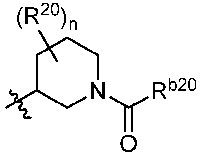 | 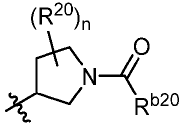 | 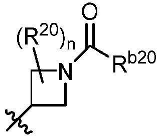 |
| Cy²-a1, | Cy²-b1, | Cy²-c1, and | Cy²-d1 , |

wherein n is 0, 1 or 2.

In an embodiment, Cy² is Cy²-a. In an embodiment, Cy² is Cy²-b. In an embodiment, Cy² is Cy²-c. In an embodiment, Cy² is Cy²-d. In an embodiment, Cy² is Cy²-e.

In an embodiment, n is 0. In an embodiment, n is 1. In an embodiment, n is 2.

In another embodiment, Cy² is selected from Cy²-a, Cy²-b, Cy²-c, Cy²-d, and Cy²-e, wherein n is 0.

In an embodiment, Cy² is Cy²-a1. In another embodiment, Cy² is Cy²-b1. In yet another embodiment, Cy² is Cy²-c1. In still another embodiment, Cy² is Cy²-d1. In an embodiment, n is 0. In another embodiment, n is 1. In yet another embodiment, n is 2.

In another embodiment, each R¹⁰ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, OR^{a10}, C(O)R^{b10}, C(O)NR^{c10}R^{d10}, C(O)OR^{a10}, OC(O)R^{b10}, OC(O)NR^{c10}R^{d10}, NR^{c10}R^{d10}, NR^{C10}C(O)R^{b10}, NR^{c10}C(O)OR^{a10}, NR^{c19}C(O)NR^{c10}R^{d10,} NR^{c10}S(O)₂R^{b10}, S(O)₂R^{b10}, and S(O)₂NR^{c10}R^{d10}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R¹¹.

In another embodiment, each R¹⁰ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, OR^{a10}, C(O)NR^{c19}R^{d10,} C(O)OR^{a10}, NR^{c10}R^{d10}, NR^{c10}C(O)R^{b10}, and S(O)₂R^{b10}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1 or 2 substituents independently selected from R¹¹.

In yet another embodiment, each R¹⁰ is independently selected from C₁₋₆ alkyl, C₂₋₆ alkenyl, C₂₋₆ alkynyl, C₁₋₆ haloalkyl, halo, D, CN, OR^{a10}, and NR^{c10}R^{d10}; wherein said C₁₋₆ alkyl, C₂₋₆ alkenyl, and C₂₋₆ alkynyl, are each optionally substituted with 1 or 2 substituents independently selected from R¹¹.

In still another embodiment, each R¹⁰ is independently selected from C₁₋₆ alkyl, C₁₋₆ haloalkyl, halo, D, CN, OR^{a10}, and NR^{c10}R^{d10}. In an embodiment, each R¹⁰ is independently selected from C₁₋₆ alkyl, halo, and OR^{a10}. In another embodiment, each R¹⁰ is independently selected from methyl, fluoro, and hydroxyl. In still another embodiment, each R¹⁰ is independently selected from F, Cl, CH₃ and CF₃.

In yet another embodiment, each R¹¹ is independently selected from C₁₋₆ alkyl, C₁₋₆ haloalkyl, halo, D, CN, OR^{a11}, C(O)R^{b11}, C(O)NR^{c11}R^{d11}, C(O)ORa¹¹, OC(O)R^{b11}, OC(O)NR^{c11}R^{d11}, NR^{c11}R^{d11}, NR^{c11}C(O)R^{b11}, NR^{c11}C(O)OR^{a11}, S(O)₂R^{b11}, and S(O)₂NR^{c11}R^{d11}. In still another embodiment, each R¹¹ is independently selected from C₁₋₆ alkyl, C₁₋₆ haloalkyl, halo, D, CN, OR^{a11}, and NR^{c11}d¹¹,

In an embodiment, each R²⁰ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, OR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, NR^{c20}C(O)NR^{c20}R^{d20}, NR^{c20}S(O)₂R^{b20}, S(O)₂R^{b20}, and S(O)₂NR^{c20}R^{d20}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R²¹.

In another embodiment, each R²⁰ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, C(O)R^{b20}, C(O)NR^{c20}R^{d20}, C(O)OR^{a20}, S(O)₂R^{b22}, and S(O)₂NR^{c20}R^{d20}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, or 3 substituents independently selected from R²¹.

In another embodiment, each R²⁰ is independently selected from C₁₋₆ alkyl, C₂₋₆ alkenyl, C₂₋₆ alkynyl, C₁₋₆ haloalkyl, halo, D, CN, OR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, and S(O)₂R^{b20}; wherein said C₁₋₆ alkyl, C₂₋₆ alkenyl, and C₂₋₆ alkynyl, are each optionally substituted with 1, 2, 3, or 4 substituents independently selected from R²¹. In another embodiment, each R²⁰ is independently selected from C₁₋₃ alkyl, C₂₋₃ alkenyl, C₂₋₃ alkynyl, C₁₋₃ haloalkyl, halo, D, CN, OR^{a20}, C(O)R^{b20}, C(O)NR^{c20}R^{d20}, C(O)OR^{a20}, and NR^{c20}R^{d20}; wherein said C₁₋₃ alkyl, C₂₋₃ alkenyl, and C₂₋₃ alkynyl, are each optionally substituted with 1 or 2 substituents independently selected from R²¹. In another embodiment, each R²⁰ is independently selected from C₁₋₃ alkyl, CN, OR^{a20}, C(O)R^{b20}, and C(O)NR^{c20}R^{d20}; wherein said C₁₋₃ alkyl, are each optionally substituted with 1 or 2 substituents independently selected from R²¹.

In yet another embodiment, each R²⁰ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, C(O)R^{b20}, C(O)NR^{c20}R^{d20}, C(O)OR^{a22}, S(O)₂R^{b20}, and S(O)₂NR^{c20}R^{d20}.

In an embodiment, each R²⁰ is independently selected from C₁₋₆ alkyl, CN, OR^{a20}, and C(O)NR^{c20}R^{d20}; wherein said C₁₋₆ alkyl is optionally substituted with 1 or 2 substituents independently selected from R²¹.

In still another embodiment, each R²⁰ is independently selected from C₁₋₆ alkyl, CN, C(O)R^{b20}, C(O)OR^{a20}, and S(O)₂R^{b20}. In an embodiment, each R²⁰ is C(O)R^{b20}. In another embodiment, each R²⁰ is prop-2-en-1-one.

In an embodiment, each R²¹ is independently selected from C₁₋₆ alkyl, C₂₋₆ alkenyl, C₂₋₆ alkynyl, C₁₋₆ haloalkyl, halo, D, CN, OR^{a21}, C(O)R^{b21}, C(O)NR^{c21}R^{d21}, C(O)OR^{a21}, OC(O)R^{b21}, OC(O)NR^{c21}R^{d21}, NR^{c21}R^{d21}, NR^{c21}C(O)R^{b21}, NR^{c21}C(O)OR^{a21}, S(O)₂R^{b21}, and S(O)₂NR^{c21}R^{d21}. In another embodiment, each R²¹ is independently selected from C₁₋₆ alkyl, C₁₋₆ haloalkyl, halo, D, CN, and OR^{a21}. In another embodiment, each R²¹ is independently selected from C₁₋₃ alkyl, C₁₋₃ haloalkyl, halo, D, and CN. In another embodiment, each R²¹ is CN.

In yet another embodiment, each R²¹ is independently selected from C₁₋₆ haloalkyl, halo, CN, OR^{a21}, and NR^{c21}R^{d21}. In still another embodiment, each R²¹ is independently selected from CF₃, F, Cl, CN, OH, OCH₃, NH₂, NHCH₃, and N(CH₃)₂.

In an embodiment, R²² is CN.

In yet another embodiment, each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}(O)OR^{a30}, NR^{c30}C(O)NR^{c30}R^{d30}, NR^{c30}S(O)₂R^{b30}, S(O)**₂**R^{b30}**,** and S(O)₂NR^{c30}R^{d30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹.

In still another embodiment, each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a30}, C(O)R^{b30}**,** C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30,} S(O)₂R^{b30}, and S(O)₂NR^{c30}R^{d30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³¹.

In an embodiment, each R³⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a30}, and NR^{c30}R^{d30}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, or 3 substituents independently selected from R³¹.

In another embodiment, each R³⁰ is independently selected from 4-10 membered heterocycloalkyl, 5-10 membered heteroaryl, halo, OR^{a30}, and NR^{c30}R^{d30}; wherein said 4-10 membered heterocycloalkyl, and 5-10 membered heteroaryl, are each optionally substituted with 1 or 2 substituents independently selected from R³¹. In another embodiment, each R³⁰ is independently selected from C₁₋₃ alkyl, C₁₋₃ haloalkyl, C₃₋₆ cycloalkyl, 4-6 membered heterocycloalkyl, phenyl, 5-6 membered heteroaryl, halo, D, CN, OR^{a30}, and NR^{c30}R^{d30}; wherein said C₁₋₃ alkyl, C₃₋₆ cycloalkyl, 4-6 membered heterocycloalkyl, phenyl, and 5-6 membered heteroaryl, are each optionally substituted with 1 or 2 substituents independently selected from R³¹. In another embodiment, each R³⁰ is independently selected from C₁₋₃ alkyl, 4-6 membered heterocycloalkyl, 5-6 membered heteroaryl, halo, OR^{a30}, and NR^{c30}R^{d30}; wherein said C₁₋₃ alkyl, 4-6 membered heterocycloalkyl, and 5-6 membered heteroaryl, are each optionally substituted with 1 or 2 substituents independently selected from R³¹.

In yet another embodiment, each R³¹ is independently selected from C₁₋₆ alkyl, C₂₋₆ alkenyl, C₂₋₆ alkynyl, C₁₋₆ haloalkyl, C₃₋₆ cycloalkyl, 4-6 membered heterocycloalkyl, halo, D, CN, OR^{a31}, C(O)R^{b31}, C(O)NR^{c31}R^{d31}, C(O)OR^{a31}, OC(O)R^{b31}, OC(O)NR^{c31}R^{d31}, NR^{c31}R^{d31}, NR^{c31}C(O)R^{b31}, NR^{c31}C(O)OR^{a31}, NR^{c31}C(O)NR^{c31}R^{d31}, NR^{c31}S(O)₂R^{b31}, S(O)₂R^{b31}, and S(O)₂NR^{c31}R^{d31}; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R³².

In still another embodiment, each R³¹ is independently selected from C₁₋₆ alkyl, halo, D, CN, OR^{a31}, and NR^{c31}R^{d31}. In an embodiment, each R³¹ is independently C₁₋₆ alkyl. In another embodiment, each R³¹ is independently methyl.

In an embodiment, each R⁵⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a50}, and NR^{c50}R^{d50}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁵¹. In another embodiment, each R⁵⁰ is independently selected from C₁₋₃ alkyl, C₁₋₃ haloalkyl, C₃₋₆ cycloalkyl, 4-6 membered heterocycloalkyl, halo, D, and CN; wherein said C₁₋₃ alkyl, C₃₋₆ cycloalkyl, and 4-6 membered heterocycloalkyl, are each optionally substituted with 1 or 2 substituents independently selected from R⁵¹. In another embodiment, each R⁵⁰ is 4-6 membered heterocycloalkyl; wherein said 4-6 membered heterocycloalkyl, are each optionally substituted with 1 or 2 substituents independently selected from R⁵¹.

In an embodiment, each R⁵¹ is independently selected from C₁₋₆ alkyl, C₁₋₆ haloalkyl, halo, D, CN, ORa⁵¹, and NR^{c51}R^{d51}. In another embodiment, each R⁵¹ is independently selected from C₁₋₃ alkyl, C₁₋₃ haloalkyl, halo, D, and CN. In an embodiment, each R⁵¹ is C₁₋₃ alkyl.

In an embodiment, each R⁶⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, and S(O)₂R^{b60}. In an embodiment, each R⁶⁰ is independently selected from C₁₋₃ alkyl, C₁₋₃ haloalkyl, C₃₋₆ cycloalkyl, 4-6 membered heterocycloalkyl, halo, D, CN, OR^{a60}, and NR^{c60}R^{d60}. In an embodiment, each R⁶⁰ is independently selected from C₁₋₃ alkyl, 4-6 membered heterocycloalkyl, OR^{a60}, and NR^{c60}R^{d60}.

In another embodiment, each R⁶⁰ is independently selected from C₁₋₆ alkyl, C₁₋₆ haloalkyl, 4-10 membered heterocycloalkyl, halo, D, CN, OR^{a60}, C(O)NR^{c60}R^{d60} and NR^{c60}R^{d60}; wherein said C₁₋₆ alkyl and 4-10 membered heterocycloalkyl are each optionally substituted with 1, 2, 3, or 4 substituents independently selected from R⁶¹.

In yet another embodiment, each R⁶¹ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a61}, and NR^{c61}R^{d61}. In still another embodiment, each R⁶¹ is independently selected from 4-6 membered heterocycloalkyl.

In an embodiment, each R^{a3}, R^{b3}, R^{c3} and R^{d3} is independently selected from H, C₁₋₆ alkyl, C₂₋₆ alkenyl, C₂₋₆ alkynyl, and C₁₋₆ haloalkyl; wherein said C₁₋₆ alkyl, C₂₋₆ alkenyl, and C₂₋₆ alkynyl, are each optionally substituted with 1, 2, 3, or 4 substituents independently selected from R³⁰.

In an embodiment, each R^{j3} is independently selected from H, C₁₋₆ alkyl, C₂₋₆ alkenyl, C₂₋₆ alkynyl, and C₁₋₆ haloalkyl; wherein said C₁₋₆ alkyl, C₂₋₆ alkenyl, and C₂₋₆ alkynyl, are each optionally substituted with 1, 2, 3, or 4 substituents independently selected from R³⁰.

In another embodiment, each R^{a3}, R^{b3}, R^{c3} and R^{d3} is independently selected from H, C₁₋₆ alkyl, and C₁₋₆ haloalkyl; wherein said C₁₋₆ alkyl, is optionally substituted with 1 or 2 substituents independently selected from R³⁰.

In another embodiment, each R^{j3} is independently selected from H, C₁₋₆ alkyl, and C₁₋₆ haloalkyl; wherein said C₁₋₆ alkyl, is optionally substituted with 1 or 2 substituents independently selected from R³⁰.

In yet another embodiment, each R^{a3}, is independently C₁₋₆ alkyl; wherein said C₁₋₆ alkyl, is optionally substituted with 1 substituent independently selected from R³⁰. In still another embodiment, each R^{a3}, is independently methyl; wherein said methyl, is substituted with 1 substituent independently selected from R³⁰.

In an embodiment, each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R¹¹.

In another embodiment, each R^{a10}, R^{b10}, R^{c10} and R^{d10} is independently selected from H, C₁₋₆ alkyl, and C₁₋₆ haloalkyl. In yet another embodiment, each R^{a10}, is independently selected from H, and C₁₋₆ alkyl. In still another embodiment, each R^{a10}, is independently H.

In an embodiment, each R^{a20}, R^{b20}, R^{c20} and R^{d20} is independently selected from H, C₁₋₆ alkyl, C₂₋₆ alkenyl, C₂₋₆ alkynyl, C₁₋₆ haloalkyl, C₃₋₆ cycloalkyl, and 4-6 membered heterocycloalkyl; wherein said C₁₋₆ alkyl, C₂₋₆ alkenyl, C₂₋₆ alkynyl, C₃₋₆ cycloalkyl, and 4-6 membered heterocycloalkyl, are each optionally substituted with 1, 2, 3, or 4 substituents independently selected from R²¹.

In another embodiment, each R^{a20}, R^{b20}, R^{c20} and R^{d20} is independently selected from H, C₁₋₆ alkyl, C₂₋₆ alkenyl, C₂₋₆ alkynyl, C₁₋₆ haloalkyl; wherein said C₁₋₆ alkyl, C₂₋₆ alkenyl, and C₂₋₆ alkynyl, are each optionally substituted with 1 or 2 substituents independently selected from R²¹.

In yet another embodiment, each R^{b20}, is independently selected from H, C₁₋₆ alkyl, C₂₋₆ alkenyl, C₂₋₆ alkynyl, C₁₋₆ haloalkyl. In still another embodiment, each R^{b20} is independently C₂₋₆ alkenyl. In an embodiment, each R^{b20} is independently ethylenyl (vinyl).

In an embodiment, each R^{b20} is independently selected from C₂₋₆ alkenyl and C₂₋₆ alkynyl; wherein said C₂₋₆ alkenyl and C₂₋₆ alkynyl are each optionally substituted with 1 or 2 substituents independently selected from R²¹. In another embodiment, each R^{b20} is independently selected from C₂₋₃ alkenyl and C₂₋₃ alkynyl; wherein said C₂₋₃ alkenyl, and C₂₋₃ alkynyl are each optionally substituted with 1 or 2 substituents independently selected from R²¹.

In another embodiment, each R^{a30}, R^{b30}, R^{c30} and R^{d30} is independently selected from H, C₁₋₆ alkyl, C₂₋₆ alkenyl, C₂₋₆ alkynyl, and C₁₋₆ haloalkyl; wherein said C₁₋₆ alkyl, C₂₋₆ alkenyl, and C₂₋₆ alkynyl, are each optionally substituted with 1, 2, or 3 substituents independently selected from R³¹.

In yet another embodiment, each R^{a30}, R^{b30}, R^{c30} and R^{d30} is independently selected from H, C₁₋₆ alkyl, and C₁₋₆ haloalkyl; wherein said C₁₋₆ alkyl, is optionally substituted with 1, 2, or 3 substituents independently selected from R³¹. In still another embodiment, each R^{a30}, R^{c30} and R^{d30} is independently selected from H, and C₁₋₆ alkyl. In yet another embodiment, each R^{a30}, R^{c30} and R^{d30} is independently selected from H, and methyl.

In another embodiment, each R^{a60}, R^{b60}, R^{c60} and R^{d60} is independently selected from H, C₁₋₆ alkyl, and C₁₋₆ haloalkyl. In an embodiment, each R^{a60}, R^{c60} and R^{d60} is independently selected from H, C₁₋₃ alkyl, C₁₋₃haloalkyl.

In another embodiment, Cy¹ is other than 3,5-dimethylisoxazol-4-yl, 3,5-dimethyl-1H-pyrazol-4-yl or 4-(1-oxo-2-propen-1-yl)phenyl.

In another embodiment, compounds of the Formulae herein are compounds of the Formulae or pharmaceutically acceptable salts thereof.

In another embodiment, the compound of Formula I is selected from:
1-(4-(8-chloro-6-fluoro-7-(3-hydroxynaphthalen-1-yl)-4-((1-methylpyrrolidin-2-yl)-methoxy)-1H-imidazo[4,5-c]quinolin-1-yl)piperidin-1-yl)prop-2-en-1-one;
1-(4-(8-chloro-6-fluoro-7-(2-fluoro-6-hydroxyphenyl)-4-((1-methylpyrrolidin-2-yl)-methoxy)-1H-imidazo[4,5-c]quinolin-1-yl)piperidin-1-yl)prop-2-en-1-one;
1-(4-(8-chloro-6-fluoro-7-(2-fluoro-3-hydroxyphenyl)-4-((1-methylpyrrolidin-2-yl)-methoxy)-1H-imidazo[4,5-c]quinolin-1-yl)piperidin-1-yl)prop-2-en-1-one;
1-(4-(8-chloro-6-fluoro-7-(5-methyl-1H-indazol-4-yl)-4-((1-methylpyrrolidin-2-yl)-methoxy)-1H-imidazo[4,5-c]quinolin-1-yl)piperidin-1-yl)prop-2-en-1-one;
1-(3-(8-chloro-6-fluoro-7-(3-hydroxynaphthalen-1-yl)-4-((1-methylpyrrolidin-2-yl)-methoxy)-1H-imidazo[4,5-c]quinolin-1-yl)piperidin-1-yl)prop-2-en-1-one;
1-(3-(8-chloro-6-fluoro-7-(3-hydroxynaphthalen-1-yl)-4-((1-methylpyrrolidin-2-yl)-methoxy)-1H-imidazo[4,5-c]quinolin-1-yl)azetidin-1-yl)prop-2-en-1-one;
1-(3-(8-chloro-6-fluoro-7-(3-hydroxynaphthalen-1-yl)-4-((1-methylpyrrolidin-2-yl)-methoxy)-1H-imidazo[4,5-c]quinolin-1-yl)pyrrolidin-1-yl)prop-2-en-1-one;
1-(4-(8-chloro-6-fluoro-7-(3-hydroxynaphthalen-1-yl)-4-((1-methyl-1H-pyrazol-5-yl)-methoxy)-1H-imidazo[4,5-c]quinolin-1-yl)piperidin-1-yl)prop-2-en-1-one;
1-(4-(8-chloro-4-(3-(dimethylamino)azetidin-1-yl)-6-fluoro-7-(3-hydroxynaphthalen-1-yl)-1H-imidazo[4,5-c]quinolin-1-yl)piperidin-1-yl)prop-2-en-1-one;
1-(1-acryloylpiperidin-4-yl)-8-chloro-6-fluoro-7-(3-hydroxynaphthalen-1-yl)-4-((1-methylpyrrolidin-2-yl)ymethoxy)-1,3-dihydro-2H-imidazo[4,5-c]quinolin-2-one;
1-(4-(8-chloro-6-fluoro-4,7-bis(3-hydroxynaphthalen-1-yl)-1H-imidazo[4,5-c]quinolin-1-yl)piperidin-1-yl)prop-2-en-1-one;
1-(4-(8-chloro-6-fluoro-4,7-bis(2-fluoro-6-hydroxyphenyl)-1H-imidazo[4,5-c]quinolin-1-yl)piperidin-1-yl)prop-2-en-1-one;
1-(4-(8-chloro-6-fluoro-7-(2-fluoro-6-hydroxyphenyl)-1H-imidazo[4,5-c]quinolin-1-yl)-piperidin-1-yl)prop-2-en-1-one; and
1-(4-(8-chloro-6-fluoro-7-(3-hydroxynaphthalen-1-yl)-4-((1-methylpyrrolidin-2-yl)methoxy)-1H-[1,2,3]triazolo[4,5-c]quinolin-1-yl)piperidin-1-yl)prop-2-en-1-one.

In another embodiment, the compound of Formula I is selected from:
4-(1-((*endo*)-2-azabicyclo[2.1.1]hexan-5-yl)-8-chloro-4-(3-(dimethylamino)azetidin-1-yl)-6-fluoro-2-methyl-1*H*-imidazo[4,5-c]quinolin-7-yl)naphthalen-2-ol;
2-(1-acryloyl-4-(8-chloro-4-(3-(dimethylamino)azetidin-1-yl)-7-(2,3-dimethylphenyl)-6-fluoro-1*H*-imidazo[4,5-c]quinolin-1-yl)piperidin-2-yl)acetonitrile;
3-(1-(1-acryloyl-2-(cyanomethyl)piperidin-4-yl)-8-chloro-4-(3-(dimethylamino)azetidin-1-yl)-6-fluoro-1*H*-imidazo[4,5-c]quinolin-7-yl)-2-methylbenzonitrile;
2-(1-acryloyl-4-(8-chloro-4-(3-(dimethylamino)azetidin-1-yl)-6-fluoro-7-(3-fluoro-2-methyl-phenyl)-1*H*-imidazo[4,5-c]quinolin-1-yl)piperidin-2-yl)acetonitrile;
2-(1-acryloyl-4-(8-chloro-7-(2-chloro-3-methylphenyl)-4-(3-(dimethylamino)-azetidin-1-yl)-6-fluoro-1*H*-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(*o*-tolyl)-1H-imidazo[4,5-*c*]quinolin-1-yl)piperidin-2-yl)acetonitrile;
1-acryloyl-4-(8-chloro-4-(3-(dimethylamino)azetidin-1-yl)-6-fluoro-7-(3-hydroxy-naphthalen-1-yl)-1*H*-imidazo[4,5-c]quinolin-1-yl)piperidine-2-carboxamide;
1-acryloyl-4-(8-chloro-4-(3-(dimethylamino)azetidin-1-yl)-6-fluoro-7-(3-hydroxy-naphthalen-1-yl)-1*H*-imidazo[4,5-c]quinolin-1-yl)piperidine-2-carbonitrile;
4-(1-((*endo*)-2-azabicyclo[2.1.1]hexan-5-yl)-8-chloro-4-(3-(dimethylamino)azetidin-1-yl)-6-fluoro-1*H*-imidazo[4,5-c]quinolin-7-yl)-6-chloronaphthalen-2-ol;
4-(1-((*endo*)-2-azabicyclo[2.1.1]hexan-5-yl)-8-chloro-4-(3-(dimethylamino)-azetidin-1-yl)-6-fluoro-1*H*-imidazo[4,5-c]quinolin-7-yl)-6-methylnaphthalen-2-ol;
4-(1-((*endo*)-2-azabicyclo[2.1.1]hexan-5-yl)-4-(3-(dimethylamino)azetidin-1-yl)-6-fluoro-8-(1*H*-pyrazol-4-yl)-1*H*-imidazo[4,5-c]quinolin-7-yl)naphthalen-2-ol;
1-(1-acryloylazetidin-3-yl)-8-fluoro-7-(3-hydroxynaphthalen-1-yl)-5-(2-isopropyl-4-methyl-pyridin-3-yl)-3,5-dihydro-1*H*-imidazo[4,5-c][1,8]naphthyridine-2,4-dione;
1-(1-acryloylazetidin-3-yl)-8-fluoro-7-(3-hydroxynaphthalen-1-yl)-5-(2-isopropyl-4-methyl-pyridin-3-yl)-3-((1-methylpyrrolidin-2-yl)methyl)-3,5-dihydro-1*H*-imidazo[4,5-*c*][1,8]naphthyridine-2,4-dione;
1-(1-acryloylazetidin-3-yl)-8-fluoro-7-(3-hydroxynaphthalen-1-yl)-5-phenyl-3,5-dihydro-1*H*-imidazo[4,5-*c*][1,8]naphthyridine-2,4-dione;
1-(3-(8-chloro-6-fluoro-7-(3-hydroxynaphthalen-1-yl)-4-((1-methylpyrrolidin-2-yl)-methoxy)-1*H*-imidazo[4,5-*c*]quinolin-1-yl)-4-methylpyrrolidin-1-yl)prop-2-en-1-one;
1-((*trans*)-3-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)-4-methoxypyrrolidin-1-yl)prop-2-en-1-one;
1-((*trans*)-3-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)-4-methylpyrrolidin-1-yl)prop-2-en-1-one;
2-(1-acryloyl-4-(8-chloro-4-(3-(dimethylamino)azetidin-1-yl)-6-fluoro-7-(3-hydroxy-naphthalen-1-yl)-1H-imidazo[4,5-c]quinolin-1-yl)pyrrolidin-2-yl)acetonitrile;
1-(1-acryloylpyrrolidin-3-yl)-8-chloro-6-fluoro-7-(3-hydroxynaphthalen-1-yl)-5-methyl-1,5-dihydro-4*H*-imidazo[4,5-*c*]quinolin-4-one;
4-(1-((*endo*)-2-azabicyclo[2.1.1]hexan-5-yl)-4-(3-(dimethyl-amino)azetidin-1-yl)-6-fluoro-8-methyl-1*H*-imidazo[4,5-*c*]quinolin-7-yl)naphthalen-2-ol;
4-(1-((*endo*)-2-azabicyclo[2.1.1]hexan-5-yl)-4-(3,8-diazabicyclo[3.2.1]octan-8-yl)-8-chloro-6-fluoro-1*H*-imidazo[4,5-*c*]quinolin-7-yl)naphthalen-2-ol;
2-(1-acryloyl-4-(8-chloro-6-fluoro-7-(3-hydroxynaphthalen-1-yl)-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-6-fluoro-7-(5-methyl-1*H*-indazol-4-yl)-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-6-fluoro-4-(((*S*)-1-methylpyrrolidin-2-yl)methoxy)-7-(naphthalen-1-yl)-1*H*-imidazo[4,5-*c*]quinolin-1-yl)piperidin-2-yl)acetonitrile;
2-(1-acryloyl-4-(8-chloro-6-fluoro-7-(5-hydroxy-2-methylphenyl)-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-7-(6-chloro-5-methyl-1*H*-indazol-4-yl)-6-fluoro-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-7-(3-chloro-2-methylphenyl)-6-fluoro-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-6-fluoro-7-(3-fluoro-2-methylphenyl)-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-7-(2,3-dimethylphenyl)-6-fluoro-4-((1-methyl-pyrrolidin-2-yl)methoxy)-1*H*-imidazo[4,5-*c*]quinolin-1-yl)piperidin-2-yl)acetonitrile;
2-(1-acryloyl-4-(8-chloro-7-(2,3-dihydro-1*H*-inden-4-yl)-6-fluoro-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-phenyl-1*H-*imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(naphthalen-1-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)piperidin-2-yl)acetonitrile;
4-(1-((*endo*)-2-azabicyclo[2.1.1]hexan-5-yl)-8-chloro-4-(3-(dimethylamino)azetidin-1-yl)-6-fluoro-1*H*-imidazo[4,5-*c*]quinolin-7-yl)naphthalen-2-ol;
4-(1-((*endo*)-2-azabicyclo[2.1.1]hexan-5-yl)-8-chloro-4-(3-(dimethylamino)-azetidin-1-yl)-2-((dimethylamino)methyl)-6-fluoro-1*H*-imidazo[4,5-*c*]quinolin-7-yl)naphthalen-2-ol;
1-((*endo*)-2-azabicyclo[2.1.1]hexan-5-yl)-4-(3-(dimethylamino)azetidin-1-yl)-6-fluoro-7-(3-hydroxynaphthalen-1-yl)-1*H*-imidazo[4,5-*c*]quinoline-8-carbonitrile;
4-(1-((*endo*)-2-azabicyclo[2.1.1]hexan-5-yl)-4-(3-(dimethylamino)azetidin-1-yl)-6-fluoro-1*H*-imidazo[4,5-*c*]quinolin-7-yl)naphthalen-2-ol;
2-(1-acryloyl-4-(8-chloro-4-(3-(dimethylamino)azetidin-1-yl)-6-fluoro-7-(5-hydroxy-2,3-dimethylphenyl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(5-hydroxy-2-methylphenyl)-1*H*-imidazo[4,5-*c*]quinolin-1-yl)piperidin-2-yl)acetonitrile;
2-(1-acryloyl-4-(8-chloro-7-(2-chloro-5-hydroxyphenyl)-4-(3-(dimethylamino)azetidin-1-yl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(2-fluoro-5-hydroxyphenyl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(2-fluorophenyl)-1*H*-imidazo[4,5-*c*]quinolin-1-yl)piperidin-2-yl)acetonitrile;
2-(1-acryloyl-4-(8-chloro-7-(2-chlorophenyl)-4-(3-(dimethylamino)-azetidin-1-yl)-6-fluoro-1*H*-imidazo[4,5-*c*]quinolin-1-yl)piperidin-2-yl)acetonitrile;
2-(1-acryloyl-4-(8-chloro-7-(2-chloro-3-fluorophenyl)-4-(3-(dimethyl-amino)azetidin-1-yl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-7-(2,4-dimethylphenyl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-7-(2,5-dimethylphenyl)-6-fluoro-1*H*-imidazo[4,5-*c*]quinolin-1-yl)piperidin-2-yl)acetonitrile;
2-(1-acryloyl-4-(8-chloro-7-(3-chloro-2-methylphenyl)-4-(3-(dimethyl-amino)azetidin-1-yl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-7-(2,3-dimethylphenyl)-6-fluoro-1*H*-imidazo[4,5-*c*]quinolin-1-yl)pyrrolidin-2-yl)acetonitrile;
2-(1-acryloyl-4-(8-chloro-4-(3-(dimethylamino)azetidin-1-yl)-6-fluoro-7-(naphthalen-1-yl)-1*H*-imidazo[4,5-*c*]quinolin-1-yl)pyrrolidin-2-yl)acetonitrile;
2-(1-acryloyl-4-(8-chloro-4-(3-(dimethylamino)azetidin-1-yl)-6-fluoro-7-(5-fluoroquinolin-8-yl)-1*H*-imidazo[4,5-*c*]quinolin-1-yl)piperidin-2-yl)acetonitrile;
2-(1-acryloyl-4-(8-chloro-7-(chroman-8-yl)-4-(3-(dimethylamino)-azetidin-1-yl)-6-fluoro-1*H*-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(2-methoxy-naphthalen-1-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(4-fluoro-naphthalen-1-yl)-1*H*-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(8-methyl-naphthalen-1-yl)-1*H*-imidazo[4,5-c]quinolin-1-yl)piperidin-2-yl)acetonitrile;
2-(1-acryloyl-4-(8-chloro-7-(2,3-dihydrobenzo[b][1,4]dioxin-5-yl)-4-(3-(dimethyl-amino)azetidin-1-yl)-6-fluoro-1H-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(isoquinolin-5-yl)-1*H*-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(isoquinolin-8-yl)-1*H*-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(quinolin-8-yl)-1*H*-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(isoquinolin-4-yl)-1*H*-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(quinolin-4-yl)-1*H-*imidazo[4,5-c]quinolin-1-yl)piperidin-2-yl)acetonitrile;
4-(1-((endo)-2-azabicyclo[2.1.1]hexan-5-yl)-8-chloro-4-(3-(dimethylamino)-azetidin-1-yl)-6-fluoro-2-(hydroxymethyl)-1*H*-imidazo[4,5-c]quinolin-7-yl)naphthalen-2-ol*;*
4-(2-(2-aminoethyl)-1-((endo)-2-azabicyclo[2.1.1]hexan-5-yl)-8-chloro-4-(3-(dimethyl-amino)azetidin-1-yl)-6-fluoro-1*H*-imidazo[4,5-c]quinolin-7-yl)naphthalen-2-ol;
4-(1-((*endo*)-2-azabicyclo[2.1.1]hexan-5-yl)-8-chloro-4-(3-(dimethylamino)azetidin-1-yl)-6-fluoro-2-(piperidin-4-ylmethyl)-1*H*-imidazo[4,5-c]quinolin-7-yl)naphthalen-2-ol;
4-(1-((*endo*)-2-azabicyclo[2.1.1]hexan-5-yl)-8-chloro-4-(3-(dimethylamino)-azetidin-1-yl)-6-fluoro-2-(1-methyl-1*H*-imidazol-4-yl)-1*H*-imidazo[4,5-c]quinolin-7-yl)naphthalen-2-ol; and
4-(1-((*endo*)-2-azabicyclo[2.1.1]hexan-5-yl)-8-chloro-4-(3-(dimethylamino)azetidin-1-yl)-6-fluoro-7-(3-hydroxynaphthalen-1-yl)-1*H*-imidazo[4,5-c]quinolin-2-yl)tetrahydro-2H-thiopyran 1,1-dioxide.

In yet another embodiment, the compound of Formula I is selected from:
2-((2*S*,4*S*)-4-(8-chloro-7-(6-chloro-5-methyl-1H-indazol-4-yl)-4-(3-(dimethylamino)-3-methylazetidin-1-yl)-6-fluoro-1*H*-imidazo[4,5-*c*]quinolin-1-yl)-1-((*E*)-4-methoxybut-2-enoyl)piperidin-2-yl)acetonitrile;
2-((2*S*,4*S*)-1-(but-2-ynoyl)-4-(8-chloro-7-(6-chloro-5-methyl-1*H*-indazol-4-yl)-4-(3-(dimethylamino)-3-methylazetidin-1-yl)-6-fluoro-1*H*-imidazo[4,5-c]quinolin-1-yl)piperidin-2-yl)acetonitrile;
2-((2*S*,4*S*)-1-(but-2-ynoyl)-4-(8-chloro-7-(6-chloro-5-methyl-1H-indazol-4-yl)-4-(3-(dimethylamino)-3-methylazetidin-1-yl)-6-fluoro-1*H*-imidazo[4,5-*c*]quinolin-1-yl)piperidin-2-yl)acetonitrile;
2-((2*S*,4*S*)-1-(but-2-ynoyl)-4-(8-chloro-7-(6-chloro-5-methyl-1H-indazol-4-yl)-6-fluoro-4-(5-methyl-2,5-diazaspiro[3.4]octan-2-yl)-1*H*-imidazo[4,5-*c*]quinolin-1-yl)piperidin-2-yl)acetonitrile;
2-((2*S*,4*S*)-4-(8-chloro-7-(6-chloro-5-methyl-1H-indazol-4-yl)-6-fluoro-4-(5-methyl-2,5-diazaspiro[3.4]octan-2-yl)-1H-imidazo[4,5-c]quinolin-1-yl)-1-((*E*)-4-methoxybut-2-enoyl)piperidin-2-yl)acetonitrile;
2-((2*S*,4*S*)-1-acryloyl-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-c]quinolin-1-yl)piperidin-2-yl)acetonitrile;
2-((2*S*,4*S*)-1-acryloyl-4-(8-chloro-7-(3-chloro-2-methylphenyl)-6-fluoro-4-(((*S*)-1-methylpyrrolidin-2-yl)methoxy)-1*H*-[1,2,3]triazolo[4,5-c]quinolin-1-yl)piperidin-2-yl)acetonitrile;
2-((2*S*,4*S*)-1-(but-2-ynoyl)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-c]quinolin-1-yl)piperidin-2-yl)acetonitrile;
2-((2*S*,4*S*)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-c]quinolin-1-yl)-1-((*E*)-4-(dimethylamino)but-2-enoyl)piperidin-2-yl)acetonitrile;
2-((2*S*,4*S*)-1-acryloyl-4-(8-chloro-7-(5-chloro-4-methylpyridin-3-yl)-4-(3-(dimethylamino)azetidin-1-yl)-6-fluoro-1H-imidazo[4,5-c]quinolin-1-yl)piperidin-2-yl)acetonitrile;
2-((2*S*,4*S*)-1-acryloyl-4-(8-chloro-7-(5-chloro-2-methoxy-4-methylpyridin-3-yl)-4-(3-(dimethylamino)azetidin-1-yl)-6-fluoro-1*H*-imidazo[4,5-c]quinolin-1-yl)piperidin-2-yl)acetonitrile;
2-((2*S*,4*S*)-1-(but-2-ynoyl)-4-(8-chloro-7-(3-chloro-2-methylphenyl)-6-fluoro-4-(3-oxomorpholino)-1*H*-[1,2,3]triazolo[4,5-*c*]quinolin-1-yl)piperidin-2-yl)acetonitrile;
2-((2iS,4*S*)-1-(but-2-ynoyl)-4-(8-chloro-7-(3-chloro-2-methylphenyl)-6-fluoro-4-(4-methyl-2-oxopiperazin-1-yl)-1*H*-[1,2,3]triazolo[4,5*-c*]quinolin-1-yl)piperidin-2-yl)acetonitrile;
2-((2*S*,4*S*)-1-(but-2-ynoyl)-4-(8-chloro-7-(3-chloro-2-methylphenyl)-6-fluoro-4-(2-oxopiperidin-1-yl)-1*H*-[1,2,3]triazolo[4,5-*c*]quinolin-1-yl)piperidin-2-yl)acetonitrile;
2-((2*S*,4*S*)-1-(but-2-ynoyl)-4-(8-chloro-7-(3-chloro-2-methylphenyl)-4-(((S)-1-(dimethylamino)propan-2-yl)oxy)-6-fluoro-1*H*-[1,2,3]triazolo[4,5-*c*]quinolin-1-yl)piperidin-2-yl)acetonitrile;
3-(1-((2*S*,4*S*)-1-acryloyl-2-(cyanomethyl)piperidin-4-yl)-8-chloro-6-fluoro-4-(((S)-1-methylpyrrolidin-2-yl)methoxy)-1*H*-[1,2,3]triazolo[4,5*-c*]quinolin-7-yl)-2-methylbenzonitrile;
3-(1-((2*S*,4*S*)-1-(but-2-ynoyl)-2-(cyanomethyl)piperidin-4-yl)-8-chloro-6-fluoro-4-(((S)-1-methylpyrrolidin-2-yl)methoxy)-1*H*-[1,2,3]triazolo[4,5-*c*]quinolin-7-yl)-2-methylbenzonitrile;
3-(8-chloro-1-((2*S*,4*S*)-2-(cyanomethyl)-1-((E)-4-(dimethylamino)but-2-enoyl)piperidin-4-yl)-6-fluoro-4-(((S)-1-methylpyrrolidin-2-yl)methoxy)-1*H*-[1,2,3]triazolo[4,5-c]quinolin-7-yl)-2-methylbenzonitrile;
2-((2*S*,4*S*)-1-acryloyl-4-(7-(3-chloro-2-methylphenyl)-6-fluoro-8-methyl-4-(((*S*)-1-methylpyrrolidin-2-yl)methoxy)-1*H*-[1,2,3]triazolo[4,5-c]quinolin-1-yl)piperidin-2-yl)acetonitrile;
2-((2*S*,4*S)*-1-(but-2-ynoyl)-4-(7-(3-chloro-2-methylphenyl)-6-fluoro-8-methyl-4-(((*S*)-1-methylpyrrolidin-2-yl)methoxy)-1*H*-[1,2,3]triazolo[4,5-c]quinolin-1-yl)piperidin-2-yl)acetonitrile;
2-((2*S*,4*S*)-4-(7-(3-chloro-2-methylphenyl)-6-fluoro-8-methyl-4-(((*S*)-1-methylpyrrolidin-2-yl)methoxy)-1H-[1,2,3]triazolo[4,5-c]quinolin-1-yl)-1-((*E*)-4-methoxybut-2-enoyl)piperidin-2-yl)acetonitrile;
2-((2*S*,4*S*)-1-acryloyl-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)piperidin-2-yl)acetonitrile;
2-((2*S*,4*S*)-4-(7-(3-chloro-2-methylphenyl)-4-(3-(dimethylamino)-3-methylazetidin-1-yl)-6-fluoro-8-methyl-1*H*-[1,2,3]triazolo[4,5-c]quinolin-1-yl)-1-((*E*)-4,4-difluorobut-2-enoyl)piperidin-2-yl)acetonitrile;
2-((2*S*,4*S*)-1-(but-2-ynoyl)-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)piperidin-2-yl)acetonitrile;
2-((2*S*,4*S*)-4-(7-(3-chloro-2-methylphenyl)-4-(3-(dimethylamino)-3-methylazetidin-1-yl)-6-fluoro-8-methyl-1*H*-[1,2,3]triazolo[4,5-c]quinolin-1-yl)-1-((*E*)-4-fluorobut-2-enoyl)piperidin-2-yl)acetonitrile;
2-((2*S*,4*S*)-4-(7-(3-chloro-2-methylphenyl)-4-(3-(dimethylamino)-3-methylazetidin-1-yl)-6-fluoro-8-methyl-1*H*-[1,2,3]triazolo[4,5-c]quinolin-1-yl)-1-((*E*)-4-(dimethylamino)but-2-enoyl)piperidin-2-yl)acetonitrile;
2-((2*S*,4*S*)-1-acryloyl-4-(8-chloro-4-(3-(dimethylamino)azetidin-1-yl)-6-fluoro-7-(2-(trifluoromethyl)phenyl)-1*H*-imidazo[4,5-c]quinolin-1-yl)piperidin-2-yl)acetonitrile;
2-((2*S*,4*S*)-1-acryloyl-4-(8-chloro-7-(2,3-dichlorophenyl)-4-(3-(dimethylamino)azetidin-1-yl)-6-fluoro-1H-imidazo[4,5-c]quinolin-1-yl)piperidin-2-yl)acetonitrile;
2-((2*S*,4*S*)-1-acryloyl-4-(8-chloro-4-(3-(dimethylamino)azetidin-1-yl)-6-fluoro-7-(4-methylpyridin-3-yl)-1*H*-imidazo[4,5-*c*]quinolin-1-yl)piperidin-2-yl)acetonitrile;
2-((2*S*,4*S*)-1-acryloyl-4-(8-chloro-4-(3-(dimethylamino)azetidin-1-yl)-6-fluoro-7-(m-tolyl)-1*H*-imidazo[4,5-c]quinolin-1-yl)piperidin-2-yl)acetonitrile;
2-((2*S*,4*S*)-1-acryloyl-4-(8-chloro-4-(3-(dimethylamino)azetidin-1-yl)-7-(4,5-dimethylpyridin-3-yl)-6-fluoro-1*H*-imidazo[4,5-*c*]quinolin-1-yl)piperidin-2-yl)acetonitrile;
2-((2*S*,4*S*)-1-acryloyl-4-(8-chloro-4-(3-(dimethylamino)azetidin-1-yl)-7-(2,3-dimethylphenyl)-6-fluoro-1*H*-[1,2,3]triazolo[4,5-*c*]quinolin-1-yl)piperidin-2-yl)acetonitrile;
2-((2*S*,4*S*)-1-acryloyl-4-(8-chloro-7-(3-chloro-2-methylphenyl)-4-(3-(dimethylamino)azetidin-1-yl)-6-fluoro-1*H*-[1,2,3]triazolo[4,5-*c*]quinolin-1-yl)piperidin-2-yl)acetonitrile;
2-((2*S*,4*S*)-1-acryloyl-4-(8-chloro-7-(2,3-dichlorophenyl)-4-(3-(dimethylamino)azetidin-1-yl)-6-fluoro-1*H*-[1,2,3]triazolo[4,5-*c*]quinolin-1-yl)piperidin-2-yl)acetonitrile;
2-((2*S*,4*S*)-1-acryloyl-4-(8-chloro-4-(3-(dimethylamino)azetidin-1-yl)-6-fluoro-7-(*m-*tolyl)-1*H*-[1,2,3]triazolo[4,5-*c*]quinolin-1-yl)piperidin-2-yl)acetonitrile;
3-(1-((2*S*,4*S*)-1-acryloyl-2-(cyanomethyl)piperidin-4-yl)-8-chloro-4-(3-(dimethylamino)-azetidin-1-yl)-6-fluoro-1*H*-[1,2,3]triazolo[4,5-*c*]quinolin-7-yl)-2-methylbenzonitrile;
2-((2*S*,4*S*)-1-acryloyl-4-(8-chloro-4-(3-(dimethylamino)azetidin-1-yl)-6-fluoro-7-(4-fluoro-2,3-dimethylphenyl)-1*H*-[1,2,3]triazolo[4,5-*c*]quinolin-1-yl)piperidin-2-yl)acetonitrile;
2-((2*S*,4*S*)-1-acryloyl-4-(8-chloro-7-(2-cyclopropylphenyl)-4-(3-(dimethylamino)azetidin-1-yl)-6-fluoro-1*H*-[1,2,3]triazolo[4,5-c]quinolin-1-yl)piperidin-2-yl)acetonitrile;
2-((2*S*,4*S*)-1-acryloyl-4-(8-chloro-7-(3-chloro-2-methylphenyl)-6-fluoro-4-(((S)-1-methylpyrrolidin-2-yl)methoxy)-1*H*-imidazo[4,5-*c*]quinolin-1-yl)piperidin-2-yl)acetonitrile;
2-((2*S*,4*S*)-1-acryloyl-4-(8-chloro-7-(3-chloro-2-methylphenyl)-6-fluoro-4-(((R)-1-methylpyrrolidin-2-yl)methoxy)-1*H*-imidazo[4,5-*c*]quinolin-1-yl)piperidin-2-yl)acetonitrile;
2-((2*S*,4*S*)-1-acryloyl-4-(8-chloro-4-(3-(dimethylamino)-3-methylazetidin-1-yl)-6-fluoro-7-(4-fluorophenyl)-1*H*-imidazo[4,5-*c*]quinolin-1-yl)piperidin-2-yl)acetonitrile;
2-((2*S*,4*S*)-1-acryloyl-4-(4-(3-(dimethylamino)-3-methylazetidin-1-yl)-6-fluoro-7-(4-fluorophenyl)-8-methyl-1*H*-imidazo[4,5-*c*]quinolin-1-yl)piperidin-2-yl)acetonitrile;
2-((2*S*,4*S*)-1-acryloyl-4-(8-chloro-4-(((S)-1-(dimethylamino)propan-2-yl)oxy)-6-fluoro-7-(4-fluorophenyl)-1*H*-imidazo[4,5-*c*]quinolin-1-yl)piperidin-2-yl)acetonitrile;
2-((2*S*,4*S*)-1-acryloyl-4-(8-chloro-6-fluoro-7-(4-fluorophenyl)-4-(2-methyl-1H-imidazol-1-yl)-1*H*-imidazo[4,5-*c*]quinolin-1-yl)piperidin-2-yl)acetonitrile;
2-((2*S*,4*S*)-4-(8-chloro-6-fluoro-7-(4-fluorophenyl)-4-(2-methyl-1*H*-imidazol-1-yl)-1*H-*imidazo[4,5-c]quinolin-1-yl)-1-((*E*)-4-(dimethylamino)but-2-enoyl)piperidin-2-yl)acetonitrile;
2-((2*S*,4*S*)-4-(8-chloro-6-fluoro-7-(4-fluorophenyl)-4-(2-methyl-1*H*-imidazol-1-yl)-1 *H-*imidazo[4,5-c]quinolin-1-yl)-1-((*E*)-4-fl uorobut-2-enoyl)piperidin-2-yl)acetonitrile;
2-((2*S*,4*S*)-1-acryloyl-4-(8-chloro-4-(3-(dimethylamino)-3-methylazetidin-1-yl)-6-fluoro-7-(4-fluorophenyl)-1*H*-[1,2,3]triazolo[4,5*-c*]quinolin-1-yl)piperidin-2-yl)acetonitrile;
2-((2*S*,4*S*)-1-acryloyl-4-(4-(3-(dimethylamino)-3-methylazetidin-1-yl)-6-fluoro-7-(4-fluorophenyl)-8-methyl-1*H*-[1,2,3]triazolo[4,5-*c*]quinolin-1-yl)piperidin-2-yl)acetonitrile;
2-((2*S*,4*S*)-1-acryloyl-4-(8-chloro-4-(((S)-1-(dimethylamino)propan-2-yl)oxy)-6-fluoro-7-(4-fluorophenyl)-1*H*-[1,2,3]triazolo[4,5-*c*]quinolin-1-yl)piperidin-2-yl)acetonitrile;
2-((2*S*,4*S*)-1-(but-2-ynoyl)-4-(8-chloro-4-(((S)-1-(dimethylamino)propan-2-yl)oxy)-6-fluoro-7-(4-fluorophenyl)-1*H*-[1,2,3]triazolo[4,5-*c*]quinolin-1-yl)piperidin-2-yl)acetonitrile;
2-((2*S*,4*S*)-4-(8-chloro-4-(((S)-1-(dimethylamino)propan-2-yl)oxy)-6-fluoro-7-(4-fluorophenyl)-1*H*-[1,2,3]triazolo[4,5-*c*]quinolin-1-yl)-1-((*E*)-4-fluorobut-2-enoyl)piperidin-2-yl)acetonitrile;
2-((2*S*,4*S*)-1-acryloyl-4-(8-chloro-6-fluoro-7-(4-fluorophenyl)-4-(((*S*)-1-methylpyrrolidin-2-yl)methoxy)-1*H*-[1,2,3]triazolo[4,5-c]quinolin-1-yl)piperidin-2-yl)acetonitrile;
2-((2S,4S)-1-acryloyl-4-(4-(((*S*)-1-(dimethylamino)propan-2-yl)oxy)-6-fluoro-7-(4-fluorophenyl)-8-methyl-1*H*-[1,2,3]triazolo[4,5-c]quinolin-1-yl)piperidin-2-yl)acetonitrile;
2-((2S,4S)-1-(but-2-ynoyl)-4-(4-(((*S*)-1-(dimethylamino)propan-2-yl)oxy)-6-fluoro-7-(4-fluorophenyl)-8-methyl-1*H*-[1,2,3]triazolo[4,5-c]quinolin-1-yl)piperidin-2-yl)acetonitrile;
2-((2*S*,4*S*)-4-(4-(((S)-1-(dimethylamino)propan-2-yl)oxy)-6-fluoro-7-(4-fluorophenyl)-8-methyl-1H-[1,2,3]triazolo[4,5-c]quinolin-1-yl)-1-((*E*)-4-fluorobut-2-enoyl)piperidin-2-yl)acetonitrile;
2-((2*S*,4*S*)-1-acryloyl-4-(6-fluoro-7-(4-fluorophenyl)-8-methyl-4-(((*S*)-1-methylpyrrolidin-2-yl)methoxy)-1*H*-[1,2,3]triazolo[4,5-c]quinolin-1-yl)piperidin-2-yl)acetonitrile;
2-((2*S*,4*S*)-4-(6-fluoro-7-(4-fluorophenyl)-8-methyl-4-(((*S*)-1-methylpyrrolidin-2-yl)methoxy)-1*H*-[1,2,3]triazolo[4,5-c]quinolin-1-yl)-1-((*E*)-4-fluorobut-2-enoyl)piperidin-2-yl)acetonitrile;
2-((2*S*,4*S*)-1-acryloyl-4-(7-(2,3-dimethylphenyl)-6-fluoro-8-methyl-4-(((*S*)-1-methylpyrrolidin-2-yl)methoxy)-1*H*-[1,2,3]triazolo[4,5-c]quinolin-1-yl)piperidin-2-yl)acetonitrile;
2-((2*S*,4*S*)-4-(7-(2,3-dimethylphenyl)-6-fluoro-8-methyl-4-(((*S*)-1-methylpyrrolidin-2-yl)methoxy)-1*H*-[1,2,3]triazolo[4,5-*c*]quinolin-1-yl)-1-((*E*)-4-fluorobut-2-enoyl)piperidin-2-yl)acetonitrile;
2-((2*S*,4*S*)-1-(but-2-ynoyl)-4-(7-(2,3-dimethylphenyl)-6-fluoro-8-methyl-4-(((*S*)-1-methylpyrrolidin-2-yl)methoxy)-1*H*-[1,2,3]triazolo[4,5-c]quinolin-1-yl)piperidin-2-yl)acetonitrile;
2-((2*S*,4*S*)-4-(7-(2,3-dimethylphenyl)-6-fluoro-8-methyl-4-(((*S*)-1-methylpyrrolidin-2-yl)methoxy)-1*H*-[1,2,3]triazolo[4,5-c]quinolin-1-yl)-1-((*E*)-4-methoxybut-2-enoyl)piperidin-2-yl)acetonitrile;
2-((2*S*,4*S*)-1-((*E*)-4,4-difluorobut-2-enoyl)-4-(7-(2,3-dimethylphenyl)-6-fluoro-8-methyl-4-(((S)-1-methylpyrrolidin-2-yl)methoxy)-1*H*-[1,2,3]triazolo[4,5-c]quinolin-1-yl)piperidin-2-yl)acetonitrile;
2-((2*S*,4*S*)-1-acryloyl-4-(4-(((*S*)-1-(dimethylamino)propan-2-yl)oxy)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4*S*)-4-(4-(((*S*)-1-(dimethylamino)propan-2-yl)oxy)-7-(2,3-dimethylphenyl)-6-fluoro-8-methyl-1*H*-[1,2,3]triazolo[4,5-c]quinolin-1-yl)-1-((*E*)-4-fluorobut-2-enoyl)piperidin-2-yl)acetonitrile;
2-((2*S*,4*S*)-1-((*E*)-4,4-difluorobut-2-enoyl)-4-(4-(((*S*)-1-(dimethylamino)-propan-2-yl)oxy)-7-(2,3-dimethylphenyl)-6-fluoro-8-methyl-1*H*-[1,2,3]triazolo[4,5-c]quinolin-1-yl)piperidin-2-yl)acetonitrile;
2-((2*S*,4*S*)-4-(4-(((*S*)-1-(dimethylamino)propan-2-yl)oxy)-7-(2,3-dimethylphenyl)-6-fluoro-8-methyl-1*H*-[1,2,3]triazolo[4,5-*c*]quinolin-1-yl)-1-((*E*)-4-methoxybut-2-enoyl)piperidin-2-yl)acetonitrile;
2-((2*S*,4*S*)-1-(but-2-ynoyl)-4-(4-(((S)-1-(dimethylamino)propan-2-yl)oxy)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4*S*)-1-acryloyl-4-(4-(3-(dimethylamino)azetidin-1-yl)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4*S*)-1-((*E*)-4,4-difluorobut-2-enoyl)-4-(4-(3-(dimethylamino)-azetidin-1-yl)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4*S*)-4-(8-chloro-4-(3-(dimethylamino)azetidin-1-yl)-7-(2,3-dimethylphenyl)-6-fluoro-1H-imidazo[4,5-c]quinolin-1-yl)-1-((*E*)-4-fluorobut-2-enoyl)piperidin-2-yl)acetonitrile;
2-((2*S*,4*S*)-1-acryloyl-4-(8-chloro-7-(6-chloro-1,5-dimethyl-1*H*-indazol-4-yl)-4-(3-(dimethylamino)azetidin-1-yl)-6-fluoro-1*H*-[1,2,3]triazolo[4,5-c]quinolin-1-yl)piperidin-2-yl)acetonitrile;
2-((2S,4S)-4-(8-chloro-6-fluoro-4-(2-methyl-1H-imidazol-1-yl)-7-phenyl-1H-imidazo[4,5-c]quinolin-1-yl)-1-((E)-4-(dimethylamino)but-2-enoyl)piperidin-2-yl)acetonitrile;
2-((2S,4S)-4-(8-chloro-6-fluoro-4-(2-methyl-1H-imidazol-1-yl)-7-phenyl-1H-imidazo[4,5-c]quinolin-1-yl)-1-((E)-4-fluorobut-2-enoyl)piperidin-2-yl)acetonitrile;
2-((2S,4S)-4-(8-chloro-6-fluoro-4-(2-methyl-1H-imidazol-1-yl)-7-phenyl-1H-[1,2,3]triazolo[4,5-c]quinolin-1-yl)-1-((E)-4-(dimethylamino)but-2-enoyl) piperidin-2-yl)acetonitrile;
2-((2S,4S)-1-acryloyl-4-(8-chloro-4-(3-(dimethylamino)azetidin-1-yl)-6-fluoro-7-phenyl-1H-[1,2,3]triazolo[4,5-c]quinolin-1-yl)piperidin-2-yl)acetonitrile;
2-((2S,4S)-1-acryloyl-4-(8-chloro-6-fluoro-4-(((S)-1-methylpyrrolidin-2-yl)methoxy)-7-phenyl-1H-[1,2,3]triazolo[4,5-c]quinolin-1-yl)piperidin-2-yl)acetonitrile;
2-((2S,4S)-1-acryloyl-4-(6-fluoro-8-methyl-4-(2-methyl-1H-imidazol-1-yl)-7-phenyl-1H-[1,2,3]triazolo[4,5-c]quinolin-1-yl)piperidin-2-yl)acetonitrile;
2-((2S,4S)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)-1-((E)-4-methoxybut-2-enoyl)piperidin-2-yl)acetonitrile;
2-((2S,4S)-1-((E)-4-(dimethylamino)but-2-enoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile;
2-((2S,4S)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)-1-((E)-4-fluorobut-2-enoyl)piperidin-2-yl)acetonitrile;
2-((2S,4S)-1-acryloyl-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile;
2-((2S,4S)-1-((E)-4,4-difluorobut-2-enoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile;
2-((2S,4S)-1-(but-2-ynoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile;
2-((2S,4S)-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)-1-((E)-4-(dimethylamino)but-2-enoyl)piperidin-2-yl)acetonitrile;
2-((2S,4S)-1-((E)-4,4-difluorobut-2-enoyl)-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)piperidin-2-yl)acetonitrile;
2-((2S,4S)-1-acryloyl-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)piperidin-2-yl)acetonitrile;
2-((2S,4S)-1-(but-2-ynoyl)-4-(4-(3-(dimethylamino)a-zetidin-1-yl)-6-fluoro-8-methyl-7-(2-(trifluoromethyl)phenyl)-1H-[1,2,3]triazolo[4,5-c]quinolin-1-yl)piperidin-2-yl)acetonitrile;
2-((2S,4S)-1-((E)-4-(dimethylamino)but-2-enoyl)-4-(6-fluoro-8-methyl-4-(2-methyl-1H-imidazol-1-yl)-7-(2-(trifluoromethyl)phenyl)-1H-[1,2,3]triazolo[4,5-c]quinolin-1-yl)piperidin-2-yl)acetonitrile;
2-((2S,4S)-1-((E)-4,4-difluorobut-2-enoyl)-4-(6-fluoro-8-methyl-4-(((S)-1-methylpyrrolidin-2-yl)methoxy)-7-(2-(trifluoromethyl)pyridin-3-yl)-1H-[1,2,3]triazolo[4,5-c]quinolin-1-yl)piperidin-2-yl)acetonitrile;
2-((2S,4S)-1-(but-2-ynoyl)-4-(6-fluoro-8-methyl-4-(((S)-1-methylpyrrolidin-2-yl)methoxy)-7-(2-(trifluoromethyl)pyridin-3-yl)-1H-[1,2,3]triazolo[4,5-c]quinolin-1-yl)piperidin-2-yl)acetonitrile;
2-((2S,4S)-4-(4-(3-(dimethylamino)azetidin-1-yl)-7-(2,3-dimethylphenyl)-6-fluoro-8-methyl-1H-[1,2,3]triazolo[4,5-c]quinolin-1-yl)-1-((E)-4-fluorobut-2-enoyl)piperidin-2-yl)acetonitrile;
2-((2S,4S)-1-(but-2-ynoyl)-4-(4-(3-(dimethylamino)azetidin-1-yl)-7-(2,3-dimethylphenyl)-6-fluoro-8-methyl-1H-[1,2,3]triazolo[4,5-c]quinolin-1-yl)piperidin-2-yl)acetonitrile;
2-((2S,4S)-1-acryloyl-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile;
2-((2S,4S)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)-1-((E)-4-fluorobut-2-enoyl)piperidin-2-yl)acetonitrile;
2-((2S,4S)-1-(but-2-ynoyl)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile;
1-((2*S*,4*S*)-1-acryloyl-2-(cyanomethyl)piperidin-4-yl)-7-(6-chloro-5-methyl-1*H*-indazol-4-yl)-4-(3-(dimethylamino)azetidin-1-yl)-6-fluoro-1*H*-[1,2,3]triazolo[4,5-*c*]quinoline-8-carbonitrile;
1-((2*S*,4*S*)-1-(but-2-ynoyl)-2-(cyanomethyl)piperidin-4-yl)-7-(6-chloro-5-methyl-1*H-*indazol-4-yl)-4-(3-(dimethylamino)azetidin-1-yl)-6-fluoro-1*H*-[1,2,3]triazolo[4,5-c]quinoline-8-carbonitrile;
2-((2S,4S)-1-acryloyl-4-(8-chloro-7-(3-chloro-2-methylphenyl)-4-(3-(dimethylamino)azetidin-1-yl)-1H-imidazo[4,5-c]quinolin-1-yl)piperidin-2-yl)acetonitrile;
2-((2S,4S)-1-acryloyl-4-(7-(3-chloro-2-methylphenyl)-4-(3-(dimethylamino)azetidin-1-yl)-8-methyl-1H-[1,2,3]triazolo[4,5-c]quinolin-1-yl)piperidin-2-yl)acetonitrile;
2-((2S,4S)-1-acryloyl-4-(8-chloro-7-(3-chloro-2-methylphenyl)-6-fluoro-4-(2-methylpyridin-3-yl)-1*H*-imidazo[4,5-c]quinolin-1-yl)piperidin-2-yl)acetonitrile;
2-((2S,4S)-1-acryloyl-4-(8-chloro-7-(3-chloro-2-methylphenyl)-6-fluoro-4-(2-methylpyridin-3-yl)-1H-[1,2,3]triazolo[4,5-c]quinolin-1-yl)piperidin-2-yl)acetonitrile;
2-((2S,4S)-4-(8-chloro-4-(3-(dimethylamino)azetidin-1-yl)-6-fluoro-7-(isoquinolin-4-yl)-1H-imidazo[4,5-c]quinolin-1-yl)-1-((E)-4-(dimethylamino)but-2-enoyl)piperidin-2-yl)acetonitrile;
2-((2S,4S)-4-(8-chloro-7-(6-chloro-5-methyl-1H-indazol-4-yl)-4-(3-(dimethylamino)-azetidin-1-yl)-6-fluoro-1H-imidazo[4,5-c]quinolin-1-yl)-1-((E)-4-(dimethylamino)but-2-enoyl)piperidin-2-yl)acetonitrile;
2-((2S,4S)-1-(but-2-ynoyl)-4-(8-chloro-7-(6-chloro-5-methyl-1H-indazol-4-yl)-4-(3-(dimethylamino)azetidin-1-yl)-6-fluoro-1H-imidazo[4,5-c]quinolin-1-yl)piperidin-2-yl)acetonitrile;
2-((2S,4S)-1-(but-2-ynoyl)-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-4-(3-(dimethylamino)-azetidin-1-yl)-6-fluoro-1H-[1,2,3]triazolo[4,5-c]quinolin-1-yl)-1-((E)-4-(dimethylamino)but-2-enoyl)piperidin-2-yl)acetonitrile;
2-((2S,4S)-1-(but-2-ynoyl)-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)piperidin-2-yl)acetonitrile;
2-((2S,4S)-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)-1-((E)-4-(dimethylamino)but-2-enoyl)piperidin-2-yl)acetonitrile;
2-((2S,4S)-1-(but-2-ynoyl)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-6-fluoro-4-(((S)-1 - methylpyrrolidin-2-yl)methoxy)-1H-[1,2,3]triazolo[4,5-c]quinolin-1-yl)-1-((E)-4-(dimethylamino)but-2-enoyl)piperidin-2-yl)acetonitrile;
2-((2S,4S)-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)-1-((E)-4-methoxybut-2-enoyl)piperidin-2-yl)acetonitrile;
2-((2S,4S)-1-acryloyl-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)-1-((E)-4-methoxybut-2-enoyl)piperidin-2-yl)acetonitrile;
2-((2S,4S)-1-acryloyl-4-(8-chloro-6-fluoro-7-(4-fluoro-3-methylphenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-(but-2-ynoyl)-4-(8-chloro-7-(6-chloro-5-methyl-1H-indazol-4-yl)-6-fluoro-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-4-(3-(dimethylamino)-azetidin-1-yl)-6-fluoro-1H-[1,2,3]triazolo[4,5-c]quinolin-1-yl)-1-(2-fluoroacryloyl)piperidin-2-yl)acetonitrile;
2-((2S,4S)-1-acryloyl-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-6-fluoro-4-(((S)-1 - methylpyrrolidin-2-yl)methoxy)-1H-[1,2,3]triazolo[4,5-c]quinolin-1-yl)-1-((E)-4-methoxybut-2-enoyl)piperidin-2-yl)acetonitrile;
2-((2S,4S)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)-1-(2-fluoroacryloyl)piperidin-2-yl)acetonitrile;
2-((2S,4S)-1-acryloyl-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-1-(but-2-ynoyl)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4-methoxybut-2-enoyl)piperidin-2-yl)acetonitrile;
2-((2S,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile;
2-((2S,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4,4-difluorobut-2-enoyl)piperidin-2-yl)acetonitrile;
2-((2S,4S)-1-acryloyl-4-(7-(5,6-dimethyl-1H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-1-acryloyl-4-(6-fluoro-7-(4-fluoro-3-methylphenyl)-8-methyl-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(4-(3-(dimethylamino)-3-methylazetidin-1-yl)-6-fluoro-8-methyl-7-(5-methyl-1H-indazol-4-yl)-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-4-(3-(dimethylamino)-azetidin-1-yl)-6-fluoro-1H-[1,2,3]triazolo[4,5-c]quinolin-1-yl)-1-((E)-4,4-difluorobut-2-enoyl)piperidin-2-yl)acetonitrile;
2-((2S,4S)-1-acryloyl-4-(4-(3-(dimethylamino)-3-methylazetidin-1-yl)-6-fluoro-7-(6-fluoro-5-methyl-1H-indazol-4-yl)-8-methyl-1H-[1,2,3]triazolo[4,5-c]quinolin-1-yl)piperidin-2-yl)acetonitrile;
2-((2S,4S)-4-(4-(3-(dimethylamino)-3-methylazetidin-1-yl)-6-fluoro-7-(6-fluoro-5-methyl-1H-indazol-4-yl)-8-methyl-1H-[1,2,3]triazolo[4,5-c]quinolin-1-yl)-1-((E)-4-methoxybut-2-enoyl)piperidin-2-yl)acetonitrile;
2-((2*S*)-1-acryloyl-4-(8-chloro-6-fluoro-4-(((*S*)-1-methylpyrrolidin-2-yl)methoxy)-7-(m-tolyl)-1*H*-[1,2,3]triazolo[4,5-*c*]quinolin-1-yl)piperidin-2-yl)acetonitrile;
2-((2*S*)-1-acryloyl-4-(8-chloro-4-(3-(dimethylamino)-3-methylazetidin-1-yl)-6-fluoro-7-(*m*-tolyl)-1*H*-[1,2,3]triazolo[4,5-*c*]quinolin-1-yl)piperidin-2-yl)acetonitrile;
2-((2*S*)-1-acryloyl-4-(8-chloro-7-(8-chloronaphthalen-1-yl)-4-(3-(dimethylamino)-3-methylazetidin-1-yl)-6-fluoro-1*H*-imidazo[4,5-c]quinolin-1-yl)piperidin-2-yl)acetonitrile;
2-((2*S*)-1-(but-2-ynoyl)-4-(8-chloro-7-(8-chloronaphthalen-1-yl)-4-(3-(dimethylamino)-3-methylazetidin-1-yl)-6-fluoro-1*H*-imidazo[4,5-c]quinolin-1-yl)piperidin-2-yl)acetonitrile;
2-((2*S*)-1-acryloyl-4-(8-chloro-7-(8-chloronaphthalen-1-yl)-4-(3-(ethyl(methyl)amino)azetidin-1-yl)-6-fluoro-1*H*-imidazo[4,5-c]quinolin-1-yl)piperidin-2-yl)acetonitrile;
2-((2*S*)-1-acryloyl-4-(8-chloro-7-(8-chloronaphthalen-1-yl)-6-fluoro-4-(((*S*)-1-methylpyrrolidin-2-yl)methoxy)-1*H*-imidazo[4,5-c]quinolin-1-yl)piperidin-2-yl)acetonitrile;
2-((2*S*)-1-acryloyl-4-(8-chloro-7-(8-chloronaphthalen-1-yl)-4-(3-(dimethylamino)-3-methylazetidin-1-yl)-6-fluoro-1*H*-[1,2,3]triazolo[4,5-c]quinolin-1-yl)piperidin-2-yl)acetonitrile;
2-((2*S*)-4-(8-chloro-7-(8-chloronaphthalen-1-yl)-4-(3-(dimethylamino)-3-methylazetidin-1-yl)-6-fluoro-1*H*-[1,2,3]triazolo[4,5-c]quinolin-1-yl)-1-((*E*)-4-(dimethylamino)but-2-enoyl)piperidin-2-yl)acetonitrile;
2-((2*S*)-1-(but-2-ynoyl)-4-(8-chloro-7-(8-chloronaphthalen-1-yl)-4-(3-(dimethylamino)-3-methylazetidin-1-yl)-6-fluoro-1*H*-[1,2,3]triazolo[4,5-c]quinolin-1-yl)piperidin-2-yl)acetonitrile;
2-((2*S*)-1-acryloyl-4-(8-chloro-7-(8-chloronaphthalen-1-yl)-4-(3-(ethyl(methyl)amino)-azetidin-1-yl)-6-fluoro-1*H*-[1,2,3]triazolo[4,5-c]quinolin-1-yl)piperidin-2-yl)acetonitrile;
8-(1-((2*S*)-1-acryloyl-2-(cyanomethyl)piperidin-4-yl)-8-chloro-4-(3-(dimethylamino)-3-methylazetidin-1-yl)-6-fluoro-1*H*-[1,2,3]triazolo[4,5-c]quinolin-7-yl)-1-naphthonitrile;
2-((2*S*)-1-acryloyl-4-(7-(8-chloronaphthalen-1-yl)-4-(3-(dimethylamino)-3-methylazetidin-1-yl)-6-fluoro-8-methyl-1*H*-imidazo[4,5-c]quinolin-1-yl)piperidin-2-yl)acetonitrile;
2-((2*S*)-1-(but-2-ynoyl)-4-(7-(8-chloronaphthalen-1-yl)-4-(3-(dimethylamino)-3-methylazetidin-1-yl)-6-fluoro-8-methyl-1*H*-imidazo[4,5-c]quinolin-1-yl)piperidin-2-yl)acetonitrile;
2-((2*S*)-1-acryloyl-4-(7-(8-chloronaphthalen-1-yl)-4-(3-(ethyl(methyl)amino)azetidin-1-yl)-6-fluoro-8-methyl-1*H*-imidazo[4,5*-c*]quinolin-1-yl)piperidin-2-yl)acetonitrile;
2-((2*S*)-1-(but-2-ynoyl)-4-(7-(8-chloronaphthalen-1-yl)-4-(3-(ethyl(methyl)amino)azetidin-1-yl)-6-fluoro-8-methyl-1*H*-imidazo[4,5-c]quinolin-1-yl)piperidin-2-yl)acetonitrile;
2-((2*S*)-1-acryloyl-4-(7-(8-chloronaphthalen-1-yl)-4-(3-(dimethylamino)-3-methylazetidin-1-yl)-6-fluoro-8-methyl-1*H*-[1,2,3]triazolo[4,5-c]quinolin-1-yl)piperidin-2-yl)acetonitrile;
2-((2*S*,4*S*)-1-acryloyl-4-(4-(3-(dimethylamino)azetidin-1-yl)-6-fluoro-7-(5-fluoroquinolin-8-yl)-8-(trifluoromethyl)-1*H*-imidazo[4,5-c]quinolin-1-yl)piperidin-2-yl)acetonitrile;
2-((2*S*,4*S*)-4-(4-(3-(dimethylamino)azetidin-1-yl)-6-fluoro-7-(5-fluoroquinolin-8-yl)-8-(trifluoromethyl)-1*H*-imidazo[4,5-c]quinolin-1-yl)-1-((*E*)-4-(dimethylamino)but-2-enoyl)piperidin-2-yl)acetonitrile;
3-(1-((2*S*,4*S*)-1-(but-2-ynoyl)-2-(cyanomethyl)-piperidin-4-yl)-7-(6-chloro-5-methyl-1*H*-indazol-4-yl)-4-(3-(dimethylamino)azetidin-1-yl)-6-fluoro-1*H*-[1,2,3]triazolo[4,5-c]quinolin-8-yl)propanenitrile;
3-(7-(6-chloro-5-methyl-1 H-indazol-4-yl)-1-((2S,4S)-2-(cyanomethyl)-1-((E)-4-(dimethylamino)but-2-enoyl)piperidin-4-yl)-4-(3-(dimethylamino)azetidin-1-yl)-6-fluoro-1H-[1,2,3]triazolo[4,5-c]quinolin-8-yl)propanenitrile;
2-((2S,4S)-1-acryloyl-4-(4-(3-(dimethylamino)-3-methylazetidin-1-yl)-6-fluoro-7-(5-fluoroquinolin-8-yl)-8-(trifluoromethyl)-1H-[1,2,3]triazolo[4,5-c]quinolin-1-yl)piperidin-2-yl)acetonitrile;
2-((2S,4S)-1-(but-2-ynoyl)-4-(4-(3-(dimethylamino)-3-methylazetidin-1-yl)-6-fluoro-7-(5-fluoroquinolin-8-yl)-8-(trifluoromethyl)-1H-[1,2,3]triazolo[4,5-c]quinolin-1-yl)piperidin-2-yl)acetonitrile;
2-((2S,4S)-1-acryloyl-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-1-(but-2-ynoyl)-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile;
2-((2S,4S)-1-acryloyl-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-1-(but-2-ynoyl)-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile;
3-(1-((2S,4S)-1-(but-2-ynoyl)-2-(cyanomethyl)piperidin-4-yl)-7-(3-chloro-2-methylphenyl)-6-fluoro-4-(((S)-1-methylpyrrolidin-2-yl)methoxy)-1H-[1,2,3]triazolo[4,5-c]quinolin-8-yl)propanenitrile;
3-(1-((2S,4S)-1-(but-2-ynoyl)-2-(cyanomethyl)piperidin-4-yl)-7-(2-chloro-3-methylphenyl)-6-fluoro-4-(((S)-1-methylpyrrolidin-2-yl)methoxy)-1H-[1,2,3]triazolo[4,5-c]quinolin-8-yl)propanenitrile;
2-((2S,4S)-1-(but-2-ynoyl)-4-(7-(3-chloro-2-methoxyphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-4-(7-(3-chloro-2-methoxyphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile;
2-((2S,4S)-1-(but-2-ynoyl)-4-(4-(3-(dimethylamino)-3-methylazetidin-1-yl)-6-fluoro-7-(2-methoxy-3-methylphenyl)-8-(trifluoromethyl)-1H-[1 ,2,3]triazolo[4,5-c]quinolin-1-yl)piperidin-2-yl)acetonitrile;
2-((2S,4S)-4-(7-(2-methoxy-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile;
3-(1-((2S,4S)-1-(but-2-ynoyl)-2-(cyanomethyl)piperidin-4-yl)-7-(3-chloro-2-methoxyphenyl)-6-fluoro-4-(((S)-1-methylpyrrolidin-2-yl)methoxy)-1H-[1,2,3]triazolo[4,5-c]quinolin-8-yl)propanenitrile;
2-((2S,4S)-1-acryloyl-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile;
2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-1-((E)-4-fluorobut-2-enoyl)piperidin-2-yl)acetonitrile;
2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile;
2-((2S,4S)-1-(but-2-ynoyl)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile;
2-((2S,4S)-4-(7-(2-chloro-3-methylphenyl)-6-fluoro-8-methyl-4-(2-methyl-1H-imidazol-1-yl)-1 H-[1,2,3]triazolo[4,5-c]quinolin-1-yl)-1-((E)-4-(dimethylamino)but-2-enoyl)piperidin-2-yl)acetonitrile;
2-((2S,4S)-1-acryloyl-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)-1-((E)-4-fluorobut-2-enoyl)piperidin-2-yl)acetonitrile;
2-((2S,4S)-1-(but-2-ynoyl)-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile;
2-((2S,45)-1-acryloyl-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-fluorobut-2-enoyl)piperidin-2-yl)acetonitrile;
2-((2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile;
2-((2S,4S)-1-(but-2-ynoyl)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-1-acryloyl-4-(7-(2-chloro-3-methylphenyl)-6-fluoro-8-methyl-4-(3-oxomorpholino)-1H-[1,2,3]triazolo[4,5-c]quinolin-1-yl)piperidin-2-yl)acetonitrile;
2-((2S,4S)-1-(but-2-ynoyl)-4-(7-(2-chloro-3-methylphenyl)-6-fluoro-8-methyl-4-(3-oxomorpholino)-1H-[1,2,3]triazolo[4,5-c]quinolin-1-yl)piperidin-2-yl)acetonitrile;
2-((2S,4S)-4-(7-(2-chloro-3-methylphenyl)-6-fluoro-8-methyl-4-(3-oxomorpholino)-1H-[1,2,3]triazolo[4,5-c]quinolin-1-yl)-1-((E)-4-fluorobut-2-enoyl)piperidin-2-yl)acetonitrile;
2-((2S,4S)-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)-1-((E)-4-(dimethylamino)but-2-enoyl)piperidin-2-yl)acetonitrile;
2-((2S,4S)-1-(but-2-ynoyl)-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)piperidin-2-yl)acetonitrile;
2-((2S,4S)-1-acryloyl-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)piperidin-2-yl)acetonitrile;
2-((2S,4S)-1-acryloyl-4-(8-chloro-7-(8-chloronaphthalen-1-yl)-4-(3-(dimethylamino)azetidin-1-yl)-6-fluoro-1H-imidazo[4,5-c]quinolin-1-yl)piperidin-2-yl)acetonitrile;
2-((2S,4S)-1-acryloyl-4-(8-chloro-4-(3-(dimethylamino)azetidin-1-yl)-6-fluoro-7-(6-fluoro-5-methyl-1H-indazol-4-yl)-1H-imidazo[4,5-c]quinolin-1-yl)piperidin-2-yl)acetonitrile;
2-((2S,4S)-1-acryloyl-4-(8-chloro-7-(6-chloro-5-methyl-1H-indazol-4-yl)-4-(3-(dimethylamino)azetidin-1-yl)-6-fluoro-1H-imidazo[4,5-c]quinolin-1-yl)piperidin-2-yl)acetonitrile;
2-((2S,4S)-1-acryloyl-4-(8-chloro-4-(3-(dimethylamino)azetidin-1-yl)-6-fluoro-7-(1-methylisoquinolin-4-yl)-1H-imidazo[4,5-c]quinolin-1-yl)piperidin-2-yl)acetonitrile;
2-((2S,4S)-1-acryloyl-4-(8-chloro-4-(3-(dimethylamino)azetidin-1-yl)-6-fluoro-7-(3-methylisoquinolin-4-yl)-1H-imidazo[4,5-c]quinolin-1-yl)piperidin-2-yl)acetonitrile;
2-((2S,4S)-1-(but-2-ynoyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile;
2-((2S,45)-1-acryloyl-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidin-2-yl)acetonitrile;
2-((2S,4S)-1-acryloyl-4-(6,8-dichloro-7-(3-chloro-2-methylphenyl)-4-(3-(dimethylamino)azetidin-1-yl)-1H-[1,2,3]triazolo[4,5-c]quinolin-1-yl)piperidin-2-yl)acetonitrile;
2-((2S,4S)-1-(but-2-ynoyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidin-2-yl)acetonitrile;
2-((2S,4S)-1-acryloyl-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-(but-2-ynoyl)-4-(6,8-dichloro-4-(3-(dimethylamino)azetidin-1-yl)-7-(quinolin-8-yl)-1H-[1,2,3]triazolo[4,5-c]quinolin-1-yl)piperidin-2-yl)acetonitrile;
2-((2S,4S)-1-(but-2-ynoyl)-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-(but-2-ynoyl)-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidin-2-yl)acetonitrile;
2-((2S,4S)-1-(but-2-ynoyl)-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-7-(2,3-dichlorophenyl)-4-(3-(dimethylamino)azetidin-1-yl)-1H-[1,2,3]triazolo[4,5-c]quinolin-1-yl)piperidin-2-yl)acetonitrile;
2-((2S,4S)-1-acryloyl-4-(6,8-dichloro-7-(2-chloro-3-methylphenyl)-4-(3-(dimethylamino)azetidin-1-yl)-1H-[1,2,3]triazolo[4,5-c]quinolin-1-yl)piperidin-2-yl)acetonitrile;
2-((2S,4S)-1-acryloyl-4-(6,8-dichloro-4-(3-(dimethylamino)azetidin-1-yl)-7-(quinolin-8-yl)-1H-[1,2,3]triazolo[4,5-c]quinolin-1-yl)piperidin-2-yl)acetonitrile;
2-((2S,4S)-1-acryloyl-4-(6,8-dichloro-4-(3-(dimethylamino)azetidin-1-yl)-7-(6-fluoroquinolin-8-yl)-1H-[1,2,3]triazolo[4,5-c]quinolin-1-yl)piperidin-2-yl)acetonitrile;
2-((2S,4S)-1-acryloyl-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-7-(2-chloro-3-methylphenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-7-(3-chloro-2-methylphenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidin-2-yl)acetonitrile;
2-((2S,4S)-1-acryloyl-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-7-(3-chloro-2-methoxyphenyl)-4-(3-(dimethylamino)azetidin-1-yl)-1H-[1,2,3]triazolo[4,5-c]quinolin-1-yl)piperidin-2-yl)acetonitrile;
3-(1-((*endo*)-2-azabicyclo[2.1.1]hexan-5-yl)-8-(2-cyanoethyl)-4-(3-(dimethylamino)azetidin-1-yl)-6-fluoro-7-(7-fluoronaphthalen-1-yl)-1H-imidazo[4,5-c]quinolin-2-yl)-N,N-dimethylpropanamide;
3-(1-((*endo*)-2-azabicyclo[2.1.1]hexan-5-yl)-4-(3-(dimethylamino)azetidin-1-yl)-2-ethyl-6-fluoro-7-(7-fluoro-3-hydroxynaphthalen-1-yl)-1H-imidazo[4,5-c]quinolin-8-yl)propanenitrile;
3-(1-((*endo*)-2-azabicyclo[2.1.1]hexan-5-yl)-8-(2-cyanoethyl)-4-(3-(dimethylamino)azetidin-1-yl)-7-(3-hydroxynaphthalen-1-yl)-6-phenoxy-1H-imidazo[4,5-c]quinolin-2-yl)-*N*,*N*-dimethylpropanamide;
3-(6-benzyl-1-((*endo*)-2-azabicyclo[2.1.1]hexan-5-yl)-8-(2-cyanoethyl)-4-(3-(dimethylamino)azetidin-1-yl)-7-(3-hydroxynaphthalen-1-yl)-1*H*-imidazo[4,5-c]quinolin-2-yl)-*N,N-*dimethylpropanamide;
4-(1-((*endo*)-2-azabicyclo[2.1.1]hexan-5-yl)-4-(3-(dimethylamino)azetidin-1-yl)-6-fluoro-2-(2-(methylamino)ethyl)-8-(1*H*-pyrazol-4-yl)-1H-imidazo[4,5-c]quinolin-7-yl)naphthalen-2-ol;
3-(1-((*endo*)-2-((1H-pyrrol-2-yl)methyl)-2-azabicyclo[2.1.1]hexan-5-yl)-4-(3-(dimethylamino)azetidin-1-yl)-2-ethyl-6-fluoro-7-(3-hydroxynaphthalen-1-yl)-1H-imidazo[4,5-c]quinolin-8-yl)propanenitrile;
5-(1-((*endo*)-2-azabicyclo[2.1.1]hexan-5-yl)-8-(2-cyanoethyl)-2-(3-(dimethylamino)-3-oxopropyl)-6-fluoro-7-(3-hydroxynaphthalen-1-yl)-1*H*-imidazo[4,5-c]quinolin-4-yl)-2-fluoro-N-methylbenzamide;
3-(1-((*endo*)-2-azabicyclo[2.1.1]hexan-5-yl)-8-(2-cyanoethyl)-6-fluoro-7-(3-hydroxynaphthalen-1-yl)-4-(1-methyl-6-oxo-1 ,6-dihydropyridin-3-yl)-1*H*-imidazo[4,5-c]quinolin-2-yl)-*N*,*N*-dimethylpropanamide;
3-(1-((*endo*)-2-azabicyclo[2.1.1]hexan-5-yl)-8-(2-cyanobenzyl)-4-(3-(dimethylamino)azetidin-1-yl)-6-fluoro-7-(3-hydroxynaphthalen-1-yl)-1*H*-imidazo[4,5-c]quinolin-2-yl)-*N*,*N*-dimethylpropanamide;
3-(1-((*endo*)-2-azabicyclo[2.1.1]hexan-5-yl)-4-(3-(dimethylamino)azetidin-1-yl)-6-fluoro-7-(3-hydroxynaphthalen-1-yl)-2-((3-oxomorpholino)methyl)-1H-imidazo[4,5-c]quinolin-8-yl)propanenitrile;
3-(1-((*endo*)-2-azabicyclo[2.1.1]hexan-5-yl)-8-(2-cyanoethyl)-4-(3-(dimethylamino)azetidin-1-yl)-6-fluoro-7-(3-hydroxynaphthalen-1-yl)-1H-imidazo[4,5-c]quinolin-2-yl)-N-methyl-N-(pyridin-2-ylmethyl)propanamide; and
3-(1-((*endo*)-2-azabicyclo[2.1.1]hexan-5-yl)-4-(3-(dimethylamino)azetidin-1-yl)-6-fluoro-7-(3-hydroxynaphthalen-1-yl)-2-(2-(piperazin-1-yl)thiazol-4-yl)-1*H*-imidazo[4,5-c]quinolin-8-yl)propanenitrile;
or a pharmaceutically acceptable salt thereof.

It is further appreciated that certain features of the invention, which are, for clarity, described in the context of separate embodiments, can also be provided in combination in a single embodiment (while the embodiments are intended to be combined as if written in multiply dependent form). Conversely, various features of the invention which are, for brevity, described in the context of a single embodiment, can also be provided separately or in any suitable subcombination. Thus, it is contemplated as features described as embodiments of the compounds of Formula I can be combined in any suitable combination.

At various places in the present specification, certain features of the compounds are disclosed in groups or in ranges. It is specifically intended that such a disclosure include each and every individual subcombination of the members of such groups and ranges. For example, the term "C₁₋₆ alkyl" is specifically intended to individually disclose (without limitation) methyl, ethyl, C₃ alkyl, C₄ alkyl, C₅ alkyl and C₆ alkyl.

The term "n-membered," where n is an integer, typically describes the number of ring-forming atoms in a moiety where the number of ring-forming atoms is n. For example, piperidinyl is an example of a 6-membered heterocycloalkyl ring, pyrazolyl is an example of a 5-membered heteroaryl ring, pyridyl is an example of a 6-membered heteroaryl ring and 1,2,3,4-tetrahydro-naphthalene is an example of a 10-membered cycloalkyl group.

At various places in the present specification, variables defining divalent linking groups may be described. It is specifically intended that each linking substituent include both the forward and backward forms of the linking substituent. For example, -NR(CR'R")ₙ- includes both -NR(CR'R")ₙ- and -(CR'R")ₙNR- and is intended to disclose each of the forms individually. Where the structure requires a linking group, the Markush variables listed for that group are understood to be linking groups. For example, if the structure requires a linking group and the Markush group definition for that variable lists "alkyl" or "aryl" then it is understood that the "alkyl" or "aryl" represents a linking alkylene group or arylene group, respectively.

The term "substituted" means that an atom or group of atoms formally replaces hydrogen as a "substituent" attached to another group. The term "substituted," unless otherwise indicated, refers to any level of substitution, e.g., mono-, di-, tri-, tetra- or penta-substitution, where such substitution is permitted. The substituents are independently selected, and substitution may be at any chemically accessible position. It is to be understood that substitution at a given atom is limited by valency. It is to be understood that substitution at a given atom results in a chemically stable molecule. The phrase "optionally substituted" means unsubstituted or substituted. The term "substituted" means that a hydrogen atom is removed and replaced by a substituent. A single divalent substituent, e.g., oxo, can replace two hydrogen atoms.

The term "Cₙ₋ₘ" indicates a range which includes the endpoints, wherein n and m are integers and indicate the number of carbons. Examples include C₁₋₄, C₁₋₆ and the like.

The term "alkyl" employed alone or in combination with other terms, refers to a saturated hydrocarbon group that may be straight-chained or branched. The term "Cₙ₋ₘ alkyl," refers to an alkyl group having n to m carbon atoms. An alkyl group formally corresponds to an alkane with one C-H bond replaced by the point of attachment of the alkyl group to the remainder of the compound. In some embodiments, the alkyl group contains from 1 to 6 carbon atoms, from 1 to 4 carbon atoms, from 1 to 3 carbon atoms, or 1 to 2 carbon atoms. Examples of alkyl moieties include, but are not limited to, chemical groups such as methyl, ethyl, *n*-propyl, isopropyl, *n*-butyl, *tert*-butyl, isobutyl, *sec*-butyl; higher homologs such as 2-methyl-1-butyl, *n*-pentyl, 3-pentyl, *n*-hexyl, 1,2,2-trimethylpropyl and the like.

The term "alkenyl" employed alone or in combination with other terms, refers to a straight-chain or branched hydrocarbon group corresponding to an alkyl group having one or more double carbon-carbon bonds. An alkenyl group formally corresponds to an alkene with one C-H bond replaced by the point of attachment of the alkenyl group to the remainder of the compound. The term "Cₙ₋ₘ alkenyl" refers to an alkenyl group having n to m carbons. In some embodiments, the alkenyl moiety contains 2 to 6, 2 to 4, or 2 to 3 carbon atoms. Example alkenyl groups include, but are not limited to, ethenyl, *n*-propenyl, isopropenyl, *n-*butenyl, sec-butenyl and the like.

The term "alkynyl" employed alone or in combination with other terms, refers to a straight-chain or branched hydrocarbon group corresponding to an alkyl group having one or more triple carbon-carbon bonds. An alkynyl group formally corresponds to an alkyne with one C-H bond replaced by the point of attachment of the alkyl group to the remainder of the compound. The term "Cₙ₋ₘ alkynyl" refers to an alkynyl group having n to m carbons. Example alkynyl groups include, but are not limited to, ethynyl, propyn-1-yl, propyn-2-yl and the like. In some embodiments, the alkynyl moiety contains 2 to 6, 2 to 4, or 2 to 3 carbon atoms.

The term "alkylene," employed alone or in combination with other terms, refers to a divalent alkyl linking group. An alkylene group formally corresponds to an alkane with two C-H bond replaced by points of attachment of the alkylene group to the remainder of the compound. The term "Cₙ₋ₘ alkylene" refers to an alkylene group having n to m carbon atoms. Examples of alkylene groups include, but are not limited to, ethan-1,2-diyl, ethan-1,1-diyl, propan-1,3-diyl, propan-1,2-diyl, propan-1,1-diyl, butan-1,4-diyl, butan-1,3-diyl, butan-1,2-diyl, 2-methyl-propan-1,3-diyl and the like.

The term "alkoxy," employed alone or in combination with other terms, refers to a group of formula -O-alkyl, wherein the alkyl group is as defined above. The term "Cₙ₋ₘ alkoxy" refers to an alkoxy group, the alkyl group of which has n to m carbons. Example alkoxy groups include methoxy, ethoxy, propoxy (e.g., n-propoxy and isopropoxy), t-butoxy and the like. In some embodiments, the alkyl group has 1 to 6, 1 to 4, or 1 to 3 carbon atoms. The term "C ₙ₋ₘ dialkoxy" refers to a linking group of formula -O-(Cₙ₋ₘ alkyl)-O-, the alkyl group of which has n to m carbons. Example dialkyoxy groups include -OCH₂CH₂O- and OCH₂CH₂CH₂O-. In some embodiments, the two O atoms of a C ₙ₋ₘ dialkoxy group may be attached to the same B atom to form a 5- or 6- membered heterocycloalkyl group.

The term "alkylthio," employed alone or in combination with other terms, refers to a group of formula -S-alkyl, wherein the alkyl group is as defined above.

The term "amino," employed alone or in combination with other terms, refers to a group of formula -NH₂, wherein the hydrogen atoms may be substituted with a substituent described herein. For example, "alkylamino" can refer to -NH(alkyl) and -N(alkyl)₂.

The term "carbonyl," employed alone or in combination with other terms, refers to a -C(=O)- group, which also may be written as C(O).

The term "cyano" or "nitrile" refers to a group of formula -C≡N, which also may be written as -CN.

The term "carbamyl," as used herein, refers to a -NHC(O)O- or -OC(O)NH- group, wherein the carbon atom is doubly bound to one oxygen atom, and singly bound to a nitrogen and second oxygen atom.

The terms "halo" or "halogen," used alone or in combination with other terms, refers to fluoro, chloro, bromo and iodo. In some embodiments, "halo" refers to a halogen atom selected from F, Cl, or Br. In some embodiments, halo groups are F.

The term "haloalkyl" as used herein refers to an alkyl group in which one or more of the hydrogen atoms has been replaced by a halogen atom. The term "Cₙ₋ₘ haloalkyl" refers to a Cₙ₋ₘ alkyl group having n to m carbon atoms and from at least one up to {2(n to m)+1} halogen atoms, which may either be the same or different. In some embodiments, the halogen atoms are fluoro atoms. In some embodiments, the haloalkyl group has 1 to 6 or 1 to 4 carbon atoms. Example haloalkyl groups include CF₃, C₂F₅, CHF₂, CH₂F, CCl₃, CHCl₂, C₂Cl₅ and the like. In some embodiments, the haloalkyl group is a fluoroalkyl group.

The term "haloalkoxy," employed alone or in combination with other terms, refers to a group of formula -O-haloalkyl, wherein the haloalkyl group is as defined above. The term "Cₙ₋ₘ haloalkoxy" refers to a haloalkoxy group, the haloalkyl group of which has n to m carbons. Example haloalkoxy groups include trifluoromethoxy and the like. In some embodiments, the haloalkoxy group has 1 to 6, 1 to 4, or 1 to 3 carbon atoms.

The term "oxo" or "oxy" refers to an oxygen atom as a divalent substituent, forming a carbonyl group when attached to carbon, or attached to a heteroatom forming a sulfoxide or sulfone group, or an N-oxide group. In some embodiments, heterocyclic groups may be optionally substituted by 1 or 2 oxo (=O) substituents.

The term "sulfido" refers to a sulfur atom as a divalent substituent, forming a thiocarbonyl group (C=S) when attached to carbon.

The term "sulfonyl" refers to a -SO₂- group wherein a sulfur atom is doubly bound to two oxygen atoms.

The term "oxidized" in reference to a ring-forming N atom refers to a ring-forming N-oxide.

The term "oxidized" in reference to a ring-forming S atom refers to a ring-forming sulfonyl or ring-forming sulfinyl.

The term "aromatic" refers to a carbocycle or heterocycle having one or more polyunsaturated rings having aromatic character (i.e., having (4n + 2) delocalized π (pi) electrons where n is an integer).

The term "aryl," employed alone or in combination with other terms, refers to an aromatic hydrocarbon group, which may be monocyclic or polycyclic (e.g., having 2 fused rings). The term "Cₙ₋ₘ aryl" refers to an aryl group having from n to m ring carbon atoms. Aryl groups include, *e.g*., phenyl, naphthyl, and the like. In some embodiments, aryl groups have from 6 to about 10 carbon atoms. In some embodiments, aryl groups have 6 carbon atoms. In some embodiments, aryl groups have 10 carbon atoms. In some embodiments, the aryl group is phenyl. In some embodiments, the aryl group is naphthyl.

The term "heteroaryl" or "heteroaromatic," employed alone or in combination with other terms, refers to a monocyclic or polycyclic aromatic heterocycle having at least one heteroatom ring member selected from sulfur, oxygen and nitrogen. In some embodiments, the heteroaryl ring has 1, 2, 3 or 4 heteroatom ring members independently selected from nitrogen, sulfur and oxygen. In some embodiments, any ring-forming N in a heteroaryl moiety can be an N-oxide. In some embodiments, the heteroaryl has 5-14 ring atoms including carbon atoms and 1, 2, 3 or 4 heteroatom ring members independently selected from nitrogen, sulfur and oxygen. In some embodiments, the heteroaryl has 5-10 ring atoms including carbon atoms and 1, 2, 3 or 4 heteroatom ring members independently selected from nitrogen, sulfur and oxygen. In some embodiments, the heteroaryl has 5-6 ring atoms and 1 or 2 heteroatom ring members independently selected from nitrogen, sulfur and oxygen. In some embodiments, the heteroaryl is a five-membered or six-membered heteroaryl ring. In other embodiments, the heteroaryl is an eight-membered, nine-membered or ten-membered fused bicyclic heteroaryl ring. Example heteroaryl groups include, but are not limited to, pyridinyl (pyridyl), pyrimidinyl, pyrazinyl, pyridazinyl, pyrrolyl, pyrazolyl, azolyl, oxazolyl, isoxazolyl, thiazolyl, imidazolyl, furanyl, thiophenyl, quinolinyl, isoquinolinyl, naphthyridinyl (including 1,2-, 1,3-, 1,4-, 1,5-, 1,6-, 1,7-, 1,8-, 2,3- and 2,6-naphthyridine), indolyl, isoindolyl, benzothiophenyl, benzofuranyl, benzisoxazolyl, imidazo[1,2-b]thiazolyl, purinyl, and the like. In some embodiments, the heteroaryl group is pyridone (e.g., 2-pyridone).

A five-membered heteroaryl ring is a heteroaryl group having five ring atoms wherein one or more (e.g., 1, 2 or 3) ring atoms are independently selected from N, O and S. Exemplary five-membered ring heteroaryls include thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl and 1,3,4-oxadiazolyl.

A six-membered heteroaryl ring is a heteroaryl group having six ring atoms wherein one or more (*e.g.*, 1, 2 or 3) ring atoms are independently selected from N, O and S. Exemplary six-membered ring heteroaryls are pyridyl, pyrazinyl, pyrimidinyl, triazinyl, isoindolyl, and pyridazinyl.

The term "cycloalkyl," employed alone or in combination with other terms, refers to a non-aromatic hydrocarbon ring system (monocyclic, bicyclic or polycyclic), including cyclized alkyl and alkenyl groups. The term "Cₙ₋ₘ cycloalkyl" refers to a cycloalkyl that has n to m ring member carbon atoms. Cycloalkyl groups can include mono- or polycyclic (*e.g.*, having 2, 3 or 4 fused rings) groups and spirocycles. Cycloalkyl groups can have 3, 4, 5, 6 or 7 ring-forming carbons (C₃₋₇). In some embodiments, the cycloalkyl group has 3 to 6 ring members, 3 to 5 ring members, or 3 to 4 ring members. In some embodiments, the cycloalkyl group is monocyclic. In some embodiments, the cycloalkyl group is monocyclic or bicyclic. In some embodiments, the cycloalkyl group is a C₃₋₆ monocyclic cycloalkyl group. Ring-forming carbon atoms of a cycloalkyl group can be optionally oxidized to form an oxo or sulfido group. Cycloalkyl groups also include cycloalkylidenes. In some embodiments, cycloalkyl is cyclopropyl, cyclobutyl, cyclopentyl or cyclohexyl. Also included in the definition of cycloalkyl are moieties that have one or more aromatic rings fused (i.e., having a bond in common with) to the cycloalkyl ring, e.g., benzo or thienyl derivatives of cyclopentane, cyclohexane and the like. A cycloalkyl group containing a fused aromatic ring can be attached through any ring-forming atom including a ring-forming atom of the fused aromatic ring. Examples of cycloalkyl groups include cyclopropyl, cyclobutyl, cyclopentyl, cyclohexyl, cycloheptyl, cyclopentenyl, cyclohexenyl, cyclohexadienyl, cycloheptatrienyl, norbornyl, norpinyl, norcarnyl, bicyclo[1.1.1]pentanyl, bicyclo[2.1.1]hexanyl, and the like. In some embodiments, the cycloalkyl group is cyclopropyl, cyclobutyl, cyclopentyl, or cyclohexyl.

The term "heterocycloalkyl," employed alone or in combination with other terms, refers to a non-aromatic ring or ring system, which may optionally contain one or more alkenylene groups as part of the ring structure, which has at least one heteroatom ring member independently selected from nitrogen, sulfur, oxygen and phosphorus, and which has 4-10 ring members, 4-7 ring members, or 4-6 ring members. Included within the term "heterocycloalkyl" are monocyclic 4-, 5-, 6- and 7-membered heterocycloalkyl groups. Heterocycloalkyl groups can include mono- or bicyclic (*e.g.,* having two fused or bridged rings) or spirocyclic ring systems. In some embodiments, the heterocycloalkyl group is a monocyclic group having 1, 2 or 3 heteroatoms independently selected from nitrogen, sulfur and oxygen. Ring-forming carbon atoms and heteroatoms of a heterocycloalkyl group can be optionally oxidized to form an oxo or sulfido group or other oxidized linkage (*e.g.,* C(O), S(O), C(S) or S(O)₂, N-oxide *etc.*) or a nitrogen atom can be quaternized. The heterocycloalkyl group can be attached through a ring-forming carbon atom or a ring-forming heteroatom. In some embodiments, the heterocycloalkyl group contains 0 to 3 double bonds. In some embodiments, the heterocycloalkyl group contains 0 to 2 double bonds. Also included in the definition of heterocycloalkyl are moieties that have one or more aromatic rings fused (*i.e.,* having a bond in common with) to the heterocycloalkyl ring, *e.g.,* benzo or thienyl derivatives of piperidine, morpholine, azepine, *etc.* A heterocycloalkyl group containing a fused aromatic ring can be attached through any ring-forming atom including a ring-forming atom of the fused aromatic ring. Examples of heterocycloalkyl groups include 2,5-diazobicyclo[2.2.1]heptanyl; pyrrolidinyl; hexahydropyrrolo[3,4-b]pyrrol-1(2*H*)-yl; 1,6-dihydropyridinyl; morpholinyl; azetidinyl; piperazinyl; and 4,7-diazaspiro[2.5]octan-7-yl.

At certain places, the definitions or embodiments refer to specific rings (*e.g.,* an azetidine ring, a pyridine ring, *etc*.)*.* Unless otherwise indicated, these rings can be attached to any ring member provided that the valency of the atom is not exceeded. For example, an azetidine ring may be attached at any position of the ring, whereas an azetidin-3-yl ring is attached at the 3-position.

The compounds described herein can be asymmetric (*e.g.,* having one or more stereocenters). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically inactive starting materials are known in the art, such as by resolution of racemic mixtures or by stereoselective synthesis. Many geometric isomers of olefins, C=N double bonds and the like can also be present in the compounds described herein, and all such stable isomers are contemplated in the present invention. *Cis* and *trans* geometric isomers of the compounds of the present invention are described and may be isolated as a mixture of isomers or as separated isomeric forms.

Resolution of racemic mixtures of compounds can be carried out by any of numerous methods known in the art. One method includes fractional recrystallization using a chiral resolving acid which is an optically active, salt-forming organic acid. Suitable resolving agents for fractional recrystallization methods are, e.g., optically active acids, such as the D and L forms of tartaric acid, diacetyltartaric acid, dibenzoyltartaric acid, mandelic acid, malic acid, lactic acid or the various optically active camphorsulfonic acids such as β-camphorsulfonic acid. Other resolving agents suitable for fractional crystallization methods include stereoisomerically pure forms of α-methylbenzylamine (*e.g.,* S and R forms, or diastereomerically pure forms), 2-phenylglycinol, norephedrine, ephedrine, *N-*methylephedrine, cyclohexylethylamine, 1,2-diaminocyclohexane and the like.

Resolution of racemic mixtures can also be carried out by elution on a column packed with an optically active resolving agent (*e.g.,* dinitrobenzoylphenylglycine). Suitable elution solvent composition can be determined by one skilled in the art.

In some embodiments, the compounds of the invention have the (*R*)-configuration. In other embodiments, the compounds have the (*S*)-configuration. In compounds with more than one chiral centers, each of the chiral centers in the compound may be independently (*R*) or (*S*), unless otherwise indicated.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, enamine - imine pairs, and annular forms where a proton can occupy two or more positions of a heterocyclic system, e.g., 1*H-* and 3H-imidazole, 1*H-,* 2*H*- and 4*H-*1,2,4-triazole, 1*H*- and 2*H*- isoindole and 1*H*- and 2*H*-pyrazole. Tautomeric forms can be in equilibrium or sterically locked into one form by appropriate substitution.

Compounds of the invention can also include all isotopes of atoms occurring in the intermediates or final compounds. Isotopes include those atoms having the same atomic number but different mass numbers. For example, isotopes of hydrogen include tritium and deuterium. One or more constituent atoms of the compounds of the invention can be replaced or substituted with isotopes of the atoms in natural or non-natural abundance. In some embodiments, the compound includes at least one deuterium atom. For example, one or more hydrogen atoms in a compound of the present disclosure can be replaced or substituted by deuterium. In some embodiments, the compound includes two or more deuterium atoms. In some embodiments, the compound includes 1, 2, 3, 4, 5, 6, 7, 8, 9, 10, 11 or 12 deuterium atoms. Synthetic methods for including isotopes into organic compounds are known in the art (Deuterium Labeling in Organic Chemistry by Alan F. Thomas (New York, N.Y., Appleton-Century-Crofts, 1971; The Renaissance of H/D Exchange by Jens Atzrodt, Volker Derdau, Thorsten Fey and Jochen Zimmermann, Angew. Chem. Int. Ed. 2007, 7744-7765; The Organic Chemistry of Isotopic Labelling by James R. Hanson, Royal Society of Chemistry, 2011). Isotopically labeled compounds can used in various studies such as NMR spectroscopy, metabolism experiments, and/or assays.

Substitution with heavier isotopes such as deuterium, may afford certain therapeutic advantages resulting from greater metabolic stability, for example, increased in vivo half-life or reduced dosage requirements, and hence may be preferred in some circumstances. (A. Kerekes et.al. J. Med. Chem. 2011, 54, 201-210; R. Xu et.al. J. Label Compd. Radiopharm. 2015, 58, 308-312).

The term "compound" as used herein is meant to include all stereoisomers, geometric isomers, tautomers and isotopes of the structures depicted. The term is also meant to refer to compounds of the inventions, regardless of how they are prepared, e.g., synthetically, through biological process (e.g., metabolism or enzyme conversion), or a combination thereof.

All compounds, and pharmaceutically acceptable salts thereof, can be found together with other substances such as water and solvents (*e.g.,* hydrates and solvates) or can be isolated. When in the solid state, the compounds described herein and salts thereof may occur in various forms and may, *e.g.,* take the form of solvates, including hydrates. The compounds may be in any solid state form, such as a polymorph or solvate, so unless clearly indicated otherwise, reference in the specification to compounds and salts thereof should be understood as encompassing any solid state form of the compound.

In some embodiments, the compounds of the invention, or salts thereof, are substantially isolated. By "substantially isolated" is meant that the compound is at least partially or substantially separated from the environment in which it was formed or detected. Partial separation can include, *e.g.,* a composition enriched in the compounds of the invention. Substantial separation can include compositions containing at least about 50%, at least about 60%, at least about 70%, at least about 80%, at least about 90%, at least about 95%, at least about 97%, or at least about 99% by weight of the compounds of the invention, or salt thereof.

The phrase "pharmaceutically acceptable" is employed herein to refer to those compounds, materials, compositions and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The expressions "ambient temperature" and "room temperature," as used herein, are understood in the art, and refer generally to a temperature, *e.g.,* a reaction temperature, that is about the temperature of the room in which the reaction is carried out, *e.g.,* a temperature from about 20 °C to about 30 °C.

The present invention also includes pharmaceutically acceptable salts of the compounds described herein. The term "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the non-toxic salts of the parent compound formed, *e.g.,* from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, alcohols (*e.g.,* methanol, ethanol, iso-propanol or butanol) or acetonitrile (MeCN) are preferred. Lists of suitable salts are found in Remington's Pharmaceutical Sciences, 17th Ed., (Mack Publishing Company, Easton, 1985), p. 1418, Berge et al., J. Pharm. Sci., 1977, 66(1), 1-19 and in Stahl et al., Handbook of Pharmaceutical Salts: Properties, Selection, and Use, (Wiley, 2002). In some embodiments, the compounds described herein include the N-oxide forms.

### Synthesis

Compounds of the invention, including salts thereof, can be prepared using known organic synthesis techniques and can be synthesized according to any of numerous possible synthetic routes, such as those in the Schemes below.

The reactions for preparing compounds of the invention can be carried out in suitable solvents which can be readily selected by one of skill in the art of organic synthesis. Suitable solvents can be substantially non-reactive with the starting materials (reactants), the intermediates or products at the temperatures at which the reactions are carried out, *e.g.,* temperatures which can range from the solvent's freezing temperature to the solvent's boiling temperature. A given reaction can be carried out in one solvent or a mixture of more than one solvent. Depending on the particular reaction step, suitable solvents for a particular reaction step can be selected by the skilled artisan.

Preparation of compounds of the invention can involve the protection and deprotection of various chemical groups. The need for protection and deprotection, and the selection of appropriate protecting groups, can be readily determined by one skilled in the art. The chemistry of protecting groups is described, e.g., in Kocienski, Protecting Groups, (Thieme, 2007); Robertson, Protecting Group Chemistry, (Oxford University Press, 2000); Smith et al., March's Advanced Organic Chemistry: Reactions, Mechanisms, and Structure, 6th Ed. (Wiley, 2007); Peturssion et al., "Protecting Groups in Carbohydrate Chemistry," J. Chem. Educ., 1997, 74(11), 1297; and Wuts et al., Protective Groups in Organic Synthesis, 4th Ed., (Wiley, 2006).

Reactions can be monitored according to any suitable method known in the art. For example, product formation can be monitored by spectroscopic means, such as nuclear magnetic resonance spectroscopy (*e.g.*, ¹H or ¹³C), infrared spectroscopy, spectrophotometry (*e.g.,* UV-visible), mass spectrometry or by chromatographic methods such as high-performance liquid chromatography (HPLC) or thin layer chromatography (TLC).

The Schemes below provide general guidance in connection with preparing the compounds of the invention. One skilled in the art would understand that the preparations shown in the Schemes can be modified or optimized using general knowledge of organic chemistry to prepare various compounds of the invention.

Compounds of formulae disclosed herein can be prepared using a process as illustrated in Scheme 1 below. In the process depicted in Scheme 1, the halo substituent of compounds of Formula 1-1 can be used to install a Cy¹ substituent by a number of methods, e.g., by nucleophilic displacement with an appropriate amine nucleophile with a suitable base (e.g., triethylamine or DIPEA) in a suitable solvent (e.g., DMF, DMSO, dioxane), or by a suitable cross-coupling reaction, to give compounds of Formula 1-12. Suitable cross-coupling reactions include but are not limited to a Buchwald coupling (e.g., in the presence of a palladacycle precatalyst, such as RuPhod Pd G2), and a Negishi or Suzuki coupling (e.g., in the presence of a palladacycle precatalyst, such as Xphos Pd G2). Examples of different cross-coupling procedures include Stille (ACS Catalysis 2015, 5, 3040-3053), Suzuki (Tetrahedron 2002, 58, 9633-9695), Sonogashira (Chem. Soc. Rev. 2011, 40, 5084-5121), Negishi (ACS Catalysis 2016, 6, 1540-1552), Buchwald-Hartwig amination (Chem. Sci. 2011, 2, 27-50), and Cu-catalyzed amination (Org. React. 2014, 85, 1-688), among others.

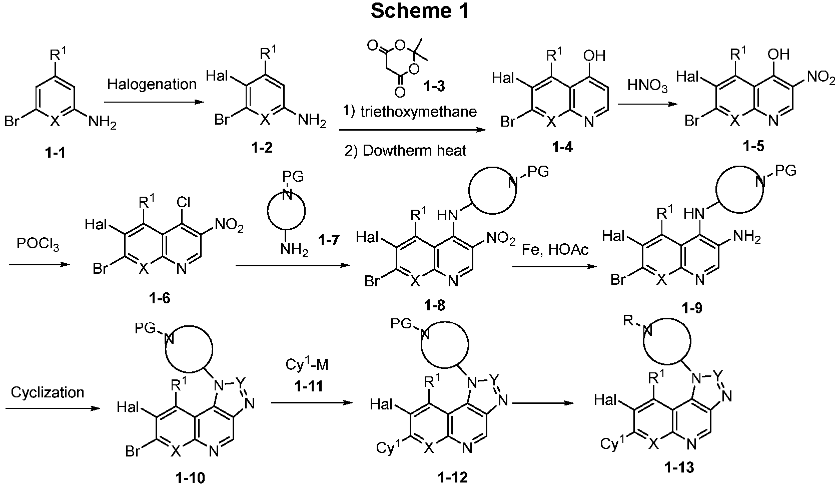

Compounds of formula **1-13** can be prepared *via* the synthetic route outlined in Scheme 1. Halogenation of starting material **1-1** with an appropriate reagent, such as N-chloro-succinimide (NCS), affords intermediate **1-2** (Hal is a halide, such as F, CI, Br, or I). Intermediate **1-4** can then be prepared by condensation of intermediate **1-2** with 2,2-dimethyl-1,3-dioxane-4,6-dione **(1-3)** and triethoxymethane, followed by decarboxylation under thermal conditions. Nitration of intermediate **1-4** with nitric acid gives intermediate **1-5.** Treatment of intermediate **1-5** with POCl₃ yields intermediate **1-6.** S_{N}Ar reaction of intermediate **1-6** with amine **1-7** (PG is an appropriate protecting group, such as Boc), followed by reduction of the nitro group (e.g. Fe in acetic acid), affords intermediate **1-9.** Cyclization of **1-9** in the presence of a suitable reagent, such as triethyl orthoformate, in acetic acid then gives tricyclic adduct **1-10.** Compound **1-12** can then be prepared by coupling of **1-10** with an adduct of formula **1-11,** in which M is a boronic acid, boronic ester or an appropriately substituted metal [*e.g*., M is B(OR)₂, Sn(Alkyl)₃, or Zn-Hal], under standard Suzuki Cross-Coupling conditions (*e.g.,* in the presence of a palladium catalyst and a suitable base) (Tetrahedron 2002, 58, 9633-9695), or standard Stille cross-coupling conditions (*e.g.,* in the presence of a palladium catalyst) (ACS Catalysis 2015, 5, 3040-3053), or standard Negishi cross-coupling conditions (*e.g*., in the presence of a palladium catalyst) (ACS Catalysis 2016, 6, 1540-1552). Removal of the protecting group in **1-12** and subsequent functionalization of the resulting amine (such as coupling with acid chloride, e.g. acryloyl chloride) affords the desired product **1-13.** The order of the above described chemical reactions can be rearranged as appropriate to suit the preparation of different analogues.

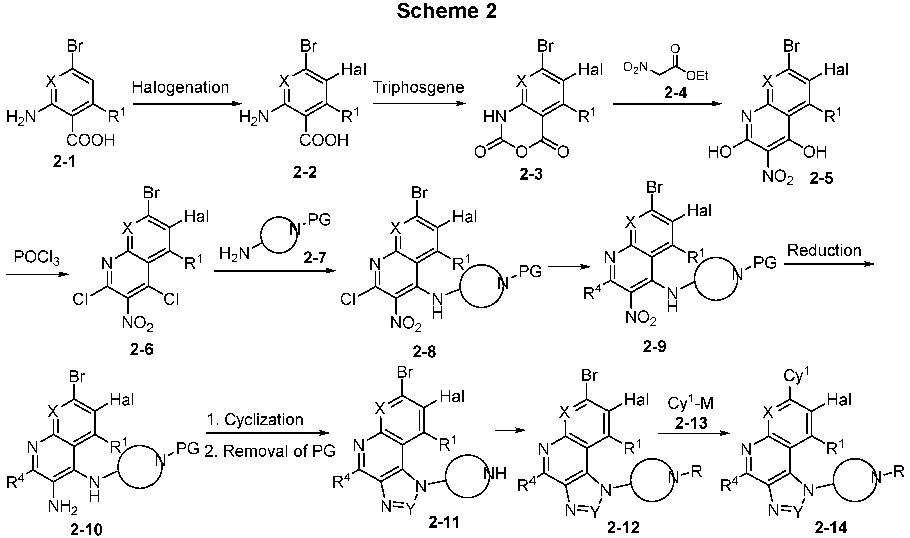

Compounds of formula **2-14** can be prepared *via* the synthetic route outlined in Scheme 2. Halogenation of starting material **2-1** with an appropriate reagent, such as *N-*chloro-succinimide (NCS), affords intermediate **2-2** (Hal is a halide, such as F, CI, Br, or I). Compound **2-3** can be prepared by treating **2-2** with reagents such as triphosgene. Intermediate **2-3** can then react with ester **2-4** to deliver the nitro compound **2-5,** which can be treated with an appropriate reagent (e.g. POCl₃) to afford compound **2-6.** A S_{N}Ar reaction of intermediate **2-6** with amine **2-7** (PG is an appropriate protecting group, such as Boc) can be carried out to generate compound **2-8.** The R⁴ group in **2-9** can then be installed *via* a suitable transformation, such as a S_{N}Ar reaction or a coupling reaction. The nitro group in **2-**9 can be reduced to NH₂ in the presence reducing agents (e.g. Fe in acetic acid). Intermediate **2-10** can first undergo a cyclization reaction (e.g. using triethyl orthoformate), followed by removal of PG, to afford amine **2-11.** Functionalization of the resulting amine (such as coupling with acid chloride, e.g. acryloyl chloride) then affords compound **2-12.** The desired product **2-14** can be prepared by a cross coupling reaction between **2-12** and an adduct of formula **2-13,** in which M is a boronic acid, boronic ester or an appropriately substituted metal [*e*.*g*., M is B(OR)₂, Sn(Alkyl)₃, or Zn-Hal], under standard Suzuki Cross-Coupling conditions (*e.g.,* in the presence of a palladium catalyst and a suitable base), or standard Stille cross-coupling conditions (*e.g.,* in the presence of a palladium catalyst), or standard Negishi cross-coupling conditions (*e.g.,* in the presence of a palladium catalyst). The order of the above described chemical reactions can be rearranged as appropriate to suit the preparation of different analogues.

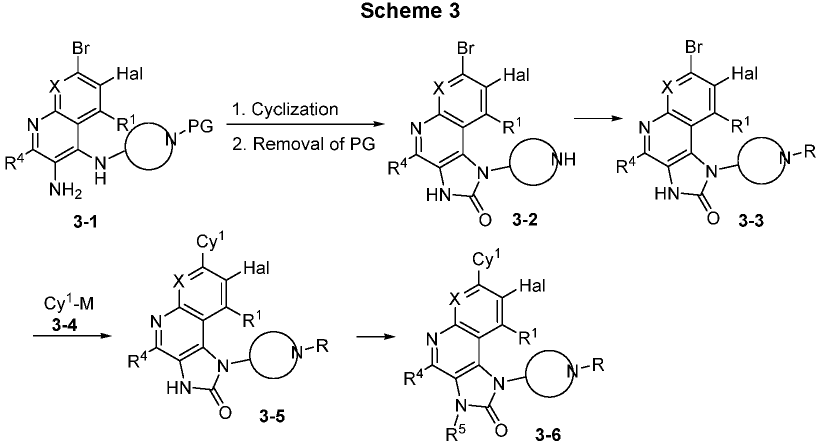

Compounds of formula **3-6** can be prepared *via* the synthetic route outlined in Scheme 3. Intermediate **3-1** (prepared according to procedures outlined in Scheme 2) can undergo a cyclization reaction (e.g. using triphosgene), followed by removal of PG, to afford amine **3-2.** Functionalization of the resulting amine (such as coupling with acid chloride, e.g. acryloyl chloride) affords intermediate **3-3.** Compound **3-5** can be prepared by a cross coupling reaction between **3-3** and an adduct of formula **3-4,** in which M is a boronic acid, boronic ester or an appropriately substituted metal [*e.g.*, M is B(OR)₂, Sn(Alkyl)₃, or Zn-Hal], under standard Suzuki Cross-Coupling conditions (*e.g*., in the presence of a palladium catalyst and a suitable base), or standard Stille cross-coupling conditions (*e.g.*, in the presence of a palladium catalyst), or standard Negishi cross-coupling conditions (*e.g.*, in the presence of a palladium catalyst). Intermediate **3-5** can then be functionalized (e.g. S_{N}2 reaction) to deliver the desired product **3-6.** The order of the above described chemical reactions can be rearranged as appropriate to suit the preparation of different analogues.

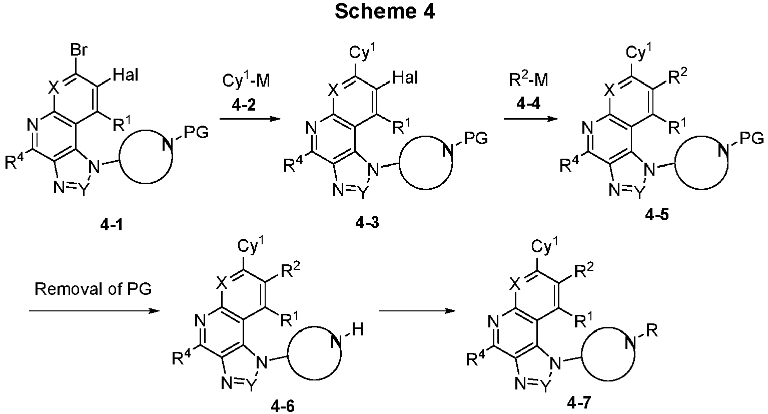

Compounds of formula **4-7** can be prepared *via* the synthetic route outlined in Scheme 4. Intermediate **4-3** can be prepared from a cross coupling reaction between intermediate **4-1** (prepared according to procedures outlined in Scheme 2) and an adduct of formula **4-2,** in which M is a boronic acid, boronic ester or an appropriately substituted metal [*e.g.,* M is B(OR)₂, Sn(Alkyl)₃, or Zn-Hal], under standard Suzuki Cross-Coupling conditions (*e.g*., in the presence of a palladium catalyst and a suitable base) (Tetrahedron 2002, 58, 9633-9695), or standard Stille cross-coupling conditions (*e.g.*, in the presence of a palladium catalyst) (ACS Catalysis 2015, 5, 3040-3053), or standard Negishi cross-coupling conditions (*e.g*., in the presence of a palladium catalyst) (ACS Catalysis 2016, 6, 1540-1552). Subsequent coupling with an adduct of formula **4-4** in which M is a boronic acid, boronic ester or an appropriately substituted metal [*e.g.,* M is B(OR)₂, Sn(Alkyl)₃, or Zn-Hal], under standard Suzuki Cross-Coupling conditions (*e.g.,* in the presence of a palladium catalyst and a suitable base), or standard Stille cross-coupling conditions (*e.g.,* in the presence of a palladium catalyst), or standard Negishi cross-coupling conditions (*e.g.,* in the presence of a palladium catalyst) provides compound **4-5.** Removal of the protecting group in **4-5** affords the amine **4-6.** Functionalization of the resulting amine (such as coupling with acid chloride, e.g. acryloyl chloride) then affords product **4-7.** The order of the above described chemical reactions can be rearranged as appropriate to suit the preparation of different analogues.

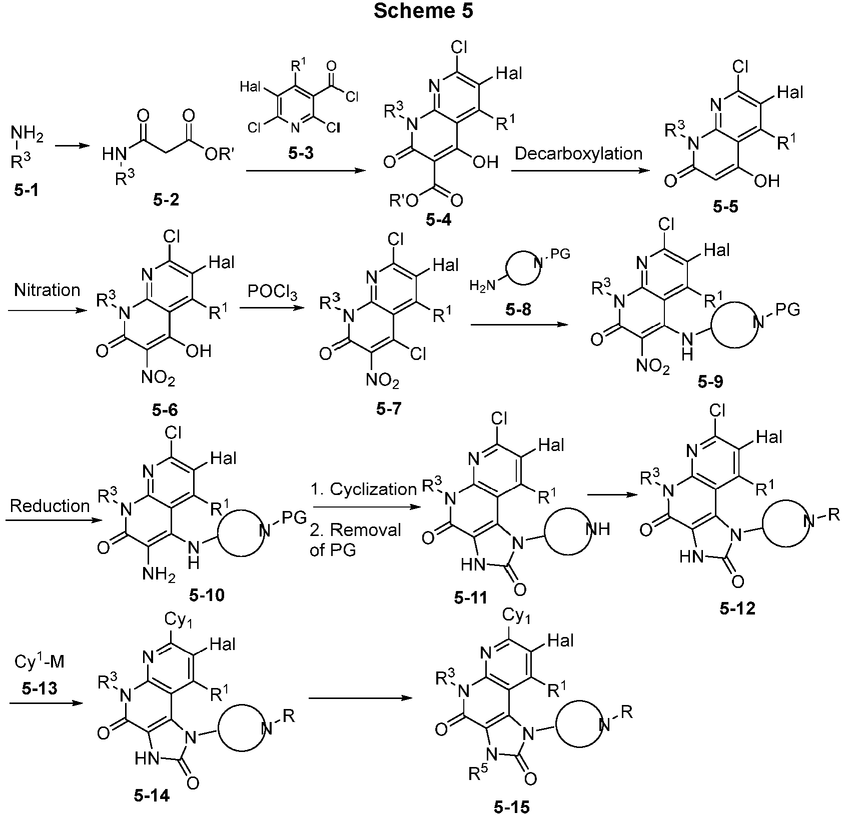

Compounds of formula **5-15** can be prepared *via* the synthetic route outlined in Scheme 5. Acylation of starting material **5-1** with an appropriate reagent, such as an acyl chloride or HATU coupling with a carboxylic acid affords the amide **5-2.** Deprotonation of **5-2** with a suitable base (e.g. sodium hydride) followed by condensation with **5-3** provides intermediate **5-4,** which can undergo decarboxylation to provide **5-5.** Nitration of intermediate **5-5** with nitric acid delivers the nitro compound **5-6.** Treatment of intermediate **5-6** with POCl₃ yields intermediate **5-7.** S_{N}Ar reaction of intermediate **5-7** with amine **5-8** (PG is an appropriate protecting group, such as Boc) can be carried out to generate compound **5-9.** The nitro group in **5-9** is then reduced to furnish amine **5-10.** Intermediate **5-10** can undergo a cyclization reaction (e.g. using CDI), followed by removal of PG, to afford amine **5-11.** Functionalization of the resulting amine (such as coupling with acid chloride, e.g. acryloyl chloride) affords intermediate **5-12.** Compound **5-14** can be prepared by a cross coupling reaction between **5-12** and an adduct of formula **5-13,** in which M is a boronic acid, boronic ester or an appropriately substituted metal [*e.g.*, M is B(OR)₂, Sn(Alkyl)₃, or Zn-Hal], under standard Suzuki Cross-Coupling conditions (*e.g.*, in the presence of a palladium catalyst and a suitable base), or standard Stille cross-coupling conditions (*e.g.,* in the presence of a palladium catalyst), or standard Negishi cross-coupling conditions (*e.g.,* in the presence of a palladium catalyst). **5-14** can then be functionalized (e.g. S_{N}2 reaction) to deliver product **5-15.** The order of the above described chemical reactions can be rearranged as appropriate to suit the preparation of different analogues.

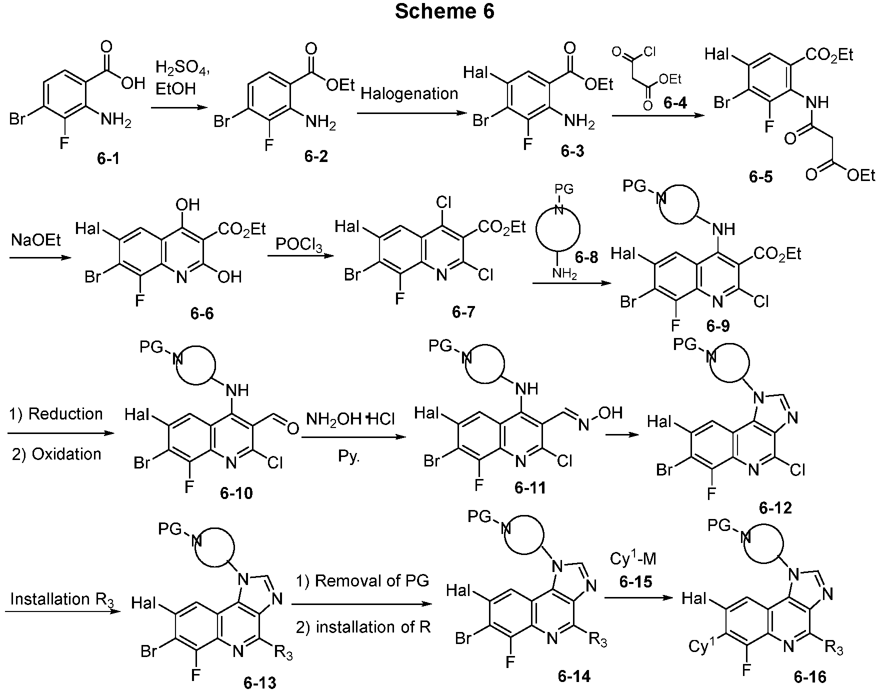

Compounds of formula **6-16** can be prepared *via* the synthetic route outlined in Scheme 6. Esterification of commercially available starting material **6-1** with H₂SO₄ in ethanol. Halogenation of compound **6-2** with an appropriate reagent, such as N-chlorosuccinimide (NCS), affords intermediate **6-3** (Hal is a halide, such as F, CI, Br, or I). Compound **6-5** can be prepared by treating **6-3** with reagents such as ethyl malonyl chloride **(6-4).** Intermediate **6-5** can undergo a cyclization reaction (such as sodium ethoxide in ethanol) to deliver the compound **6-6,** which can be treated with an appropriate reagent (e.g. POCl₃) to afford compound **6-7.** Condensation of intermediate **6-7** with amine **6-8** (PG is an appropriate protecting group, such as Boc) can be carried out to generate compound **6-9.** Reduction of ester with reducing reagent (such as DIBAL), followed by oxidation of intermediate with oxidation reagent (such as Dess-Martin periodinane) to yield aldehyde **6-10.** Treatment of intermediate **6-10** with hydroxylamine hydrochloride and pyridine get compound **6-11.** Intermediate **6-11** can undergo a cyclization reaction (such as methanesulfonyl chloride, aminopyridine in DCM) to deliver the compound **6-12.** The R³ group in **6-13** can then be installed via a suitable transformation, such as a S_{N}Ar reaction or a coupling reaction. Intermediate **6-13** can first undergo a deprotection of protecting group PG, followed by functionalization of the resulting amine (such as coupling with acid chloride, e.g. acryloyl chloride) then afford compound **6-14.** The desired product **6-16** can be prepared by a cross coupling reaction between **6-14** and an adduct of formula **6-15,** in which M is a boronic acid, boronic ester or an appropriately substituted metal [*e.g.*, M is B(OR)₂, Sn(Alkyl)₃, or Zn-Hal], under standard Suzuki Cross-Coupling conditions (*e.g.,* in the presence of a palladium catalyst and a suitable base), or standard Stille cross-coupling conditions (*e.g.,* in the presence of a palladium catalyst), or standard Negishi cross-coupling conditions (*e.g.,* in the presence of a palldium catalyst). The order of the above described chemical reactions can be rearranged as appropriate to suite the preparation of different analogues.

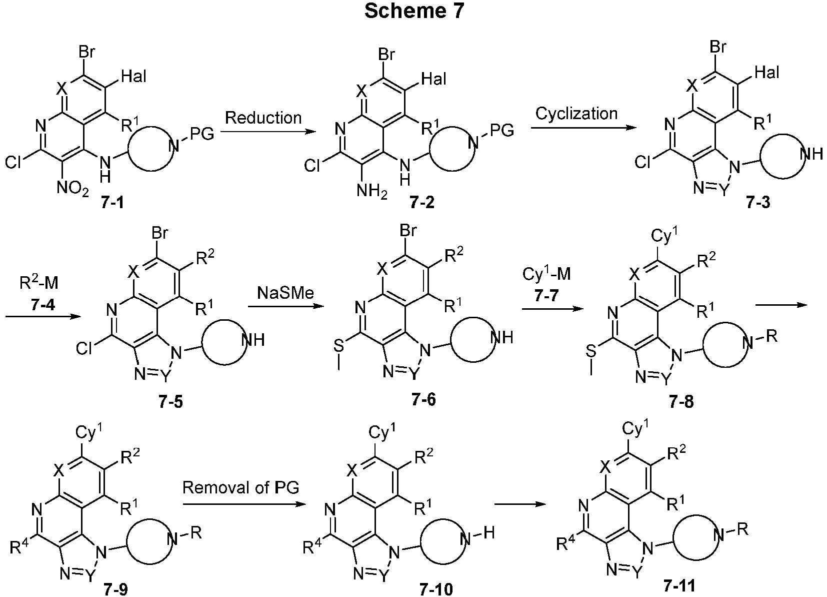

Compounds of formula **7-11** can be prepared *via* the synthetic route outlined in Scheme 7. The nitro group of intermediate **7-1** (prepared according to procedures outlined in Scheme 2) can undergo a reduction in the presence reducing agents (e.g. Fe in acetic acid) to yield **7-2.** Intermediate **7-2** can then undergo a cyclization reaction (e.g. using sodium nitrite) to give **7-3,** which can undergo a cross-coupling reaction with **7-4,** in which M is a boronic acid, boronic ester or an appropriately substituted metal [*e.g.*, M is B(OR)₂, Sn(Alkyl)₃, or Zn-Hal], under standard Suzuki Cross-Coupling conditions (*e.g.*, in the presence of a palladium catalyst and a suitable base), or standard Stille cross-coupling conditions (*e.g.*, in the presence of a palladium catalyst), or standard Negishi cross-coupling conditions (*e.g.*, in the presence of a palladium catalyst). Intermediate **7-5** can undergo a SnAr reaction with sodium thiomethoxide to provide **7-6.** A cross-coupling reaction between **7-6** and an adduct of formula **7-7,** , in which M is a boronic acid, boronic ester or an appropriately substituted metal [*e.g*., M is B(OR)₂, Sn(Alkyl)₃, or Zn-Hal], under standard Suzuki Cross-Coupling conditions (*e.g.,* in the presence of a palladium catalyst and a suitable base), or standard Stille cross-coupling conditions (*e.g.,* in the presence of a palladium catalyst), or standard Negishi cross-coupling conditions (*e.g.,* in the presence of a palladium catalyst) provides compound **7-8.** Intermediate **7-8** can be converted to intermediate **7-9** either through oxidation of the sulfur group with a suitiable oxidant (*e.g.* m-CPBA) followed by an SnAr reaction, or a cross-coupling reaction (Org. Lett. 2002, 4, 979-981). Removal of the protecting group in **7-9** affords the amine **7-10.** Functionalization of the resulting amine (such as coupling with acid chloride, e.g. acryloyl chloride) then affords product **7-11.** The order of the above described chemical reactions can be rearranged or omitted as appropriate to suit the preparation of different analogues.

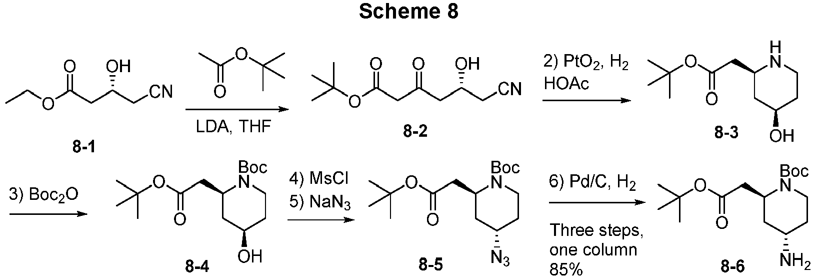

Compound **8-6** can be prepared *via* the synthetic route outlined in Scheme 8. Condensation of commercially available starting material **8-1** with tert-butyl acetate, affords intermediate **8-2** (Reference US 20090216029). Hydrogenation of compound **8-2** in HOAc using PtO₂ as catalyst affords intermediate **8-3,** which is protected with Boc anhydride to give compound **8-4.** Compound **8-5** can be prepared by treating **8-4** with reagents such as methanesulfonyl chloride, followed by displacing with sodium azide. Reduce compound **8-5** under hydrogenation condition (such as 10% palladium on carbon under H2 atomosphere) to give the compound **8-6.**

### KRAS Protein

The Ras family is comprised of three members: KRAS, NRAS and HRAS. RAS mutant cancers account for about 25% of human cancers. KRAS is the most frequently mutated isoform in human cancers: 85% of all RAS mutations are in KRAS, 12% in NRAS, and 3% in HRAS (Simanshu, D. et al. Cell 170.1 (2017):17-33). KRAS mutations are prevalent amongst the top three most deadly cancer types: pancreatic (97%), colorectal (44%), and lung (30%) (Cox, A.D. et al. Nat Rev Drug Discov (2014) 13:828-51). The majority of RAS mutations occur at amino acid residues/codons 12, 13, and 61; Codon 12 mutations are most frequent in KRAS. The frequency of specific mutations varied between RAS genes and G12D mutations are most predominant in KRAS whereas Q61R and G12R mutations are most frequent in NRAS and HRAS. Furthermore, the spectrum of mutations in a RAS isoform differs between cancer types. For example, KRAS G12D mutations predominate in pancreatic cancers (51%), followed by colorectal adenocarcinomas (45%) and lung cancers (17%) (Cox, A.D. et al. Nat Rev Drug Discov (2014) 13:828-51). In contrast, KRAS G12C mutations predominate in non-small cell lung cancer (NSCLC) comprising 11-16% of lung adenocarcinomas (nearly half of mutant KRAS is G12C), as well as 2-5% of pancreatic and colorectal adenocarcinomas, respectively (Cox, A.D. et al. Nat. Rev. Drug Discov. (2014) 13:828-51). Using shRNA knockdown thousands of genes across hundreds of cancer cell lines, genomic studies have demonstrated that cancer cells exhibiting KRAS mutations are highly dependent on KRAS function for cell growth (McDonald, R. et al. Cell 170 (2017): 577-592). Taken together, these findings suggested that KRAS mutations play a critical role in human cancers, therefore development of the inhibitors targeting mutant KRAS may be useful in the clinical treatment of diseases that have characterized by a KRAS mutation.

### Methods of Use

The cancer types in which KRAS harboring G12C, G12V and G12D mutations are implicated include, but are not limited to: carcinomas (e.g., pancreatic, colorectal, lung, bladder, gastric, esophageal, breast, head and neck, cervical skin, thyroid); hematopoietic malignancies (e.g., myeloproliferative neoplasms (MPN), myelodysplastic syndrome (MDS), chronic and juvenile myelomonocytic leukemia (CMML and JMML), acute myeloid leukemia (AML), acute lymphocytic leukemia (ALL) and multiple myeloma (MM)); and other neoplasms (e.g., glioblastoma and sarcomas). In addition, KRAS mutations were found in acquired resistance to anti-EGFR therapy (Knickelbein, K.et al. Genes & Cancer, (2015): 4-12). KRAS mutations were found in immunological and inflammatory disorders (Fernandez-Medarde, A. et al. Genes & Cancer, (2011): 344-358) such as Ras-associated lymphoproliferative disorder (RALD) or juvenile myelomonocytic leukemia (JMML) caused by somatic mutations of KRAS or NRAS.

Compounds of the present disclosure can inhibit the activity of the KRAS protein. For example, compounds of the present disclosure can be used to inhibit activity of KRAS in a cell or in an individual or patient in need of inhibition of the enzyme by administering an inhibiting amount of one or more compounds of the present disclosure to the cell, individual, or patient.

As KRAS inhibitors, the compounds of the present disclosure are useful in the treatment of various diseases associated with abnormal expression or activity of KRAS. Compounds which inhibit KRAS will be useful in providing a means of preventing the growth or inducing apoptosis in tumors, or by inhibiting angiogenesis. It is therefore anticipated that compounds of the present disclosure will prove useful in treating or preventing proliferative disorders such as cancers. In particular, tumors with activating mutants of receptor tyrosine kinases or upregulation of receptor tyrosine kinases may be particularly sensitive to the inhibitors.

In an aspect, provided herein is a method of inhibiting KRAS activity, said method comprising contacting a compound of the instant disclosure with KRAS. In an embodiment, the contacting comprises administering the compound to a patient.

In an aspect, provided herein is a method of inhibiting a KRAS protein harboring a G12C mutation, said method comprising contacting a compound of the instant disclosure with KRAS.

In an aspect, provided herein is a method of inhibiting a KRAS protein harboring a G12D mutation, said method comprising contacting a compound of the instant disclosure with KRAS.

In an aspect, provided herein is a method of inhibiting a KRAS protein harboring a G12V mutation, said method comprising contacting a compound of the instant disclosure with KRAS.

In another aspect, provided herein a is method of treating a disease or disorder associated with inhibition of KRAS interaction, said method comprising administering to a patient in need thereof a therapeutically effective amount of a compound of any of the formulae disclosed herein, or pharmaceutically acceptable salt thereof.

In yet another aspect, provided herein is a method of treating a disease or disorder associated with inhibiting a KRAS protein harboring a G12C mutation, said method comprising administering to a patient in need thereof a therapeutically effective amount of a compound of any of the formulae disclosed herein, or pharmaceutically acceptable salt thereof.

In still another aspect, provided herein is also a method of treating cancer in a patient in need thereof comprising administering to the patient a therapeutically effective amount of the compounds disclosed herein wherein the cancer is characterized by an interaction with a KRAS protein harboring a G12C mutation.

In yet another aspect, provided herein is a method for treating a cancer in a patient, said method comprising administering to the patient a therapeutically effective amount of any one of the compounds disclosed herein, or pharmaceutically acceptable salt thereof.

In an aspect, provided herein is a method for treating a disease or disorder associated with inhibition of KRAS interaction or a mutant thereof, in a patient in need thereof, comprising the step of administering to the patient a compound disclosed herein, or a pharmaceutically acceptable salt thereof, or a composition comprising a compound disclosed herein or a pharmaceutically acceptable salt thereof, in combination with another therapy or therapeutic agent as described herein.

In an embodiment, the cancer is selected from hematological cancers, sarcomas, lung cancers, gastrointestinal cancers, genitourinary tract cancers, liver cancers, bone cancers, nervous system cancers, gynecological cancers, and skin cancers.

In another embodiment, the lung cancer is selected from non-small cell lung cancer (NSCLC), small cell lung cancer, bronchogenic carcinoma, squamous cell bronchogenic carcinoma, undifferentiated small cell bronchogenic carcinoma, undifferentiated large cell bronchogenic carcinoma, adenocarcinoma, bronchogenic carcinoma, alveolar carcinoma, bronchiolar carcinoma, bronchial adenoma, chondromatous hamartoma, mesothelioma, pavicellular and non-pavicellular carcinoma, bronchial adenoma, and pleuropulmonary blastoma.

In yet another embodiment, the lung cancer is non-small cell lung cancer (NSCLC). In still another embodiment, the lung cancer is adenocarcinoma.

In an embodiment, the gastrointestinal cancer is selected from esophagus squamous cell carcinoma, esophagus adenocarcinoma, esophagus leiomyosarcoma, esophagus lymphoma, stomach carcinoma, stomach lymphoma, stomach leiomyosarcoma, exocrine pancreatic carcinoma, pancreatic ductal adenocarcinoma, pancreatic insulinoma, pancreatic glucagonoma, pancreatic gastrinoma, pancreatic carcinoid tumors, pancreatic vipoma, small bowel adenocarcinoma, small bowel lymphoma, small bowel carcinoid tumors, Kaposi's sarcoma, small bowel leiomyoma, small bowel hemangioma, small bowel lipoma, small bowel neurofibroma, small bowel fibroma, large bowel adenocarcinoma, large bowel tubular adenoma, large bowel villous adenoma, large bowel hamartoma, large bowel leiomyoma, colorectal cancer, gall bladder cancer, and anal cancer.

In an embodiment, the gastrointestinal cancer is colorectal cancer.

In another embodiment, the cancer is a carcinoma. In yet another embodiment, the carcinoma is selected from pancreatic carcinoma, colorectal carcinoma, lung carcinoma, bladder carcinoma, gastric carcinoma, esophageal carcinoma, breast carcinoma, head and neck carcinoma, cervical skin carcinoma, and thyroid carcinoma.

In still another embodiment, the cancer is a hematopoietic malignancy. In an embodiment, the hematopoietic malignancy is selected from multiple myeloma, acute myelogenous leukemia, and myeloproliferative neoplasms.

In another embodiment, the cancer is a neoplasm. In yet another embodiment, the neoplasm is glioblastoma or sarcomas.

In certain embodiments, the disclosure provides a method for treating a KRAS-mediated disorder in a patient in need thereof, comprising the step of administering to said patient a compound according to the invention, or a pharmaceutically acceptable composition thereof.

In some embodiments, diseases and indications that are treatable using the compounds of the present disclosure include, but are not limited to hematological cancers, sarcomas, lung cancers, gastrointestinal cancers, genitourinary tract cancers, liver cancers, bone cancers, nervous system cancers, gynecological cancers, and skin cancers.

Exemplary hematological cancers include lymphomas and leukemias such as acute lymphoblastic leukemia (ALL), acute myelogenous leukemia (AML), acute promyelocytic leukemia (APL), chronic lymphocytic leukemia (CLL), chronic myelogenous leukemia (CML), diffuse large B-cell lymphoma (DLBCL), mantle cell lymphoma, Non-Hodgkin lymphoma (including relapsed or refractory NHL and recurrent follicular), Hodgkin lymphoma, myeloproliferative diseases (e.g., primary myelofibrosis (PMF), polycythemia vera (PV), essential thrombocytosis (ET), 8p11 myeloproliferative syndrome, myelodysplasia syndrome (MDS), T-cell acute lymphoblastic lymphoma (T-ALL), multiple myeloma, cutaneous T-cell lymphoma, adult T-cell leukemia, Waldenstrom's Macroglubulinemia, hairy cell lymphoma, marginal zone lymphoma, chronic myelogenic lymphoma and Burkitt's lymphoma.

Exemplary sarcomas include chondrosarcoma, Ewing's sarcoma, osteosarcoma, rhabdomyosarcoma, angiosarcoma, fibrosarcoma, liposarcoma, myxoma, rhabdomyoma, rhabdosarcoma, fibroma, lipoma, harmatoma, lymphosarcoma, leiomyosarcoma, and teratoma.

Exemplary lung cancers include non-small cell lung cancer (NSCLC), small cell lung cancer, bronchogenic carcinoma (squamous cell, undifferentiated small cell, undifferentiated large cell, adenocarcinoma), alveolar (bronchiolar) carcinoma, bronchial adenoma, chondromatous hamartoma, mesothelioma, pavicellular and non-pavicellular carcinoma, bronchial adenoma and pleuropulmonary blastoma.

Exemplary gastrointestinal cancers include cancers of the esophagus (squamous cell carcinoma, adenocarcinoma, leiomyosarcoma, lymphoma), stomach (carcinoma, lymphoma, leiomyosarcoma), pancreas (exocrine pancreatic carcinoma, ductal adenocarcinoma, insulinoma, glucagonoma, gastrinoma, carcinoid tumors, vipoma), small bowel (adenocarcinoma, lymphoma, carcinoid tumors, Kaposi's sarcoma, leiomyoma, hemangioma, lipoma, neurofibroma, fibroma), large bowel (adenocarcinoma, tubular adenoma, villous adenoma, hamartoma, leiomyoma), colorectal cancer, gall bladder cancer and anal cancer.

Exemplary genitourinary tract cancers include cancers of the kidney (adenocarcinoma, Wilm's tumor [nephroblastoma], renal cell carcinoma), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma) and urothelial carcinoma.

Exemplary liver cancers include hepatoma (hepatocellular carcinoma), cholangiocarcinoma, hepatoblastoma, angiosarcoma, hepatocellular adenoma, and hemangioma.

Exemplary bone cancers include, for example, osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma, and giant cell tumors

Exemplary nervous system cancers include cancers of the skull (osteoma, hemangioma, granuloma, xanthoma, osteitis deformans), meninges (meningioma, meningiosarcoma, gliomatosis), brain (astrocytoma, meduoblastoma, glioma, ependymoma, germinoma (pinealoma), glioblastoma, glioblastoma multiform, oligodendroglioma, schwannoma, retinoblastoma, congenital tumors, neuro-ectodermal tumors), and spinal cord (neurofibroma, meningioma, glioma, sarcoma), neuroblastoma, Lhermitte-Duclos disease and pineal tumors.

Exemplary gynecological cancers include cancers of the breast (ductal carcinoma, lobular carcinoma, breast sarcoma, triple-negative breast cancer, HER2-positive breast cancer, inflammatory breast cancer, papillary carcinoma), uterus (endometrial carcinoma), cervix (cervical carcinoma, pre -tumor cervical dysplasia), ovaries (ovarian carcinoma (serous cystadenocarcinoma, mucinous cystadenocarcinoma, unclassified carcinoma), granulosa-thecal cell tumors, Sertoli-Leydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma), and fallopian tubes (carcinoma).

Exemplary skin cancers include melanoma, basal cell carcinoma, squamous cell carcinoma, Kaposi's sarcoma, Merkel cell skin cancer, moles dysplastic nevi, lipoma, angioma, dermatofibroma, and keloids.

Exemplary head and neck cancers include glioblastoma, melanoma, rhabdosarcoma, lymphosarcoma, osteosarcoma, squamous cell carcinomas, adenocarcinomas, oral cancer, laryngeal cancer, nasopharyngeal cancer, nasal and paranasal cancers, thyroid and parathyroid cancers, tumors of the eye, tumors of the lips and mouth and squamous head and neck cancer.

The compounds of the present disclosure can also be useful in the inhibition of tumor metastases.

In addition to oncogenic neoplasms, the compounds of the invention are useful in the treatment of skeletal and chondrocyte disorders including, but not limited to, achrondroplasia, hypochondroplasia, dwarfism, thanatophoric dysplasia (TD) (clinical forms TD I and TD II), Apert syndrome, Crouzon syndrome, Jackson-Weiss syndrome, Beare-Stevenson cutis gyrate syndrome, Pfeiffer syndrome, and craniosynostosis syndromes. In some embodiments, the present disclosure provides a method for treating a patient suffering from a skeletal and chondrocyte disorder.

In some embodiments, compounds described herein can be used to treat Alzheimer's disease, HIV, or tuberculosis.

As used herein, the term "8p11 myeloproliferative syndrome" is meant to refer to myeloid/lymphoid neoplasms associated with eosinophilia and abnormalities of FGFR1.

As used herein, the term "cell" is meant to refer to a cell that is *in vitro, ex vivo* or *in vivo.* In some embodiments, an *ex vivo* cell can be part of a tissue sample excised from an organism such as a mammal. In some embodiments, an *in vitro* cell can be a cell in a cell culture. In some embodiments, an *in vivo* cell is a cell living in an organism such as a mammal.

As used herein, the term "contacting" refers to the bringing together of indicated moieties in an *in vitro* system or an *in vivo* system. For example, "contacting" KRAS with a compound described herein includes the administration of a compound described herein to an individual or patient, such as a human, having KRAS, as well as, for example, introducing a compound described herein into a sample containing a cellular or purified preparation containing KRAS.

As used herein, the term "individual," "subject," or "patient," used interchangeably, refers to any animal, including mammals, preferably mice, rats, other rodents, rabbits, dogs, cats, swine, cattle, sheep, horses, or primates, and most preferably humans.

As used herein, the phrase "therapeutically effective amount" refers to the amount of active compound or pharmaceutical agent such as an amount of any of the solid forms or salts thereof as disclosed herein that elicits the biological or medicinal response in a tissue, system, animal, individual or human that is being sought by a researcher, veterinarian, medical doctor or other clinician. An appropriate "effective" amount in any individual case may be determined using techniques known to a person skilled in the art.

The phrase "pharmaceutically acceptable" is used herein to refer to those compounds, materials, compositions, and/or dosage forms which are, within the scope of sound medical judgment, suitable for use in contact with the tissues of human beings and animals without excessive toxicity, irritation, allergic response, immunogenicity or other problem or complication, commensurate with a reasonable benefit/risk ratio.

As used herein, the phrase "pharmaceutically acceptable carrier or excipient" refers to a pharmaceutically-acceptable material, composition, or vehicle, such as a liquid or solid filler, diluent, solvent, or encapsulating material. Excipients or carriers are generally safe, non-toxic and neither biologically nor otherwise undesirable and include excipients or carriers that are acceptable for veterinary use as well as human pharmaceutical use. In one embodiment, each component is "pharmaceutically acceptable" as defined herein. See, e.g., Remington: The Science and Practice of Pharmacy, 21st ed.; Lippincott Williams & Wilkins: Philadelphia, Pa., 2005; Handbook of Pharmaceutical Excipients, 6th ed.; Rowe et al., Eds.; The Pharmaceutical Press and the American Pharmaceutical Association: 2009; Handbook of Pharmaceutical Additives, 3rd ed.; Ash and Ash Eds.; Gower Publishing Company: 2007; Pharmaceutical Preformulation and Formulation, 2nd ed.; Gibson Ed.; CRC Press LLC: Boca Raton, Fla., 2009.

As used herein, the term "treating" or "treatment" refers to inhibiting a disease; for example, inhibiting a disease, condition, or disorder in an individual who is experiencing or displaying the pathology or symptomology of the disease, condition, or disorder (i.e., arresting further development of the pathology and/or symptomology) or ameliorating the disease; for example, ameliorating a disease, condition, or disorder in an individual who is experiencing or displaying the pathology or symptomology of the disease, condition, or disorder (i.e., reversing the pathology and/or symptomology) such as decreasing the severity of the disease.

The term "prevent," "preventing," or "prevention" as used herein, comprises the prevention of at least one symptom associated with or caused by the state, disease or disorder being prevented.

It is appreciated that certain features of the invention, which are, for clarity, described in the context of separate embodiments, can also be provided in combination in a single embodiment (while the embodiments are intended to be combined as if written in multiply dependent form). Conversely, various features of the invention which are, for brevity, described in the context of a single embodiment, can also be provided separately or in any suitable subcombination.

### Combination Therapy

One or more additional pharmaceutical agents or treatment methods such as, for example, anti-viral agents, chemotherapeutics or other anti-cancer agents, immune enhancers, immunosuppressants, radiation, anti-tumor and anti-viral vaccines, cytokine therapy (e.g., IL2, GM-CSF, *etc*.)*,* and/or tyrosine kinase inhibitors can be used in combination with compounds described herein for treatment of KRAS-associated diseases, disorders or conditions, or diseases or conditions as described herein. The agents can be combined with the present compounds in a single dosage form, or the agents can be administered simultaneously or sequentially as separate dosage forms.

Compounds described herein can be used in combination with one or more other kinase inhibitors for the treatment of diseases, such as cancer, that are impacted by multiple signaling pathways. For example, a combination can include one or more inhibitors of the following kinases for the treatment of cancer: Akt1, Akt2, Akt3, TGF-βR, Pim, PKA, PKG, PKC, CaM-kinase, phosphorylase kinase, MEKK, ERK, MAPK, mTOR, EGFR, FGFR, HER2, HER3, HER4, INS-R, IGF-1R, IR-R, PDGFαR, PDGFβR, CSFIR, KIT, FLK-II, KDR/FLK-1, FLK-4, fit-1, FGFR1, FGFR2, FGFR3, FGFR4, c-Met, Ron, Sea, TRKA, TRKB, TRKC, FLT3, VEGFR/Flt2, Flt4, EphA1, EphA2, EphA3, EphB2, EphB4, Tie2, Src, Fyn, Lck, Fgr, Btk, Fak, SYK, FRK, JAK, ABL, ALK and B-Raf. Additionally, the solid forms of the KRAS inhibitor as described herein can be combined with inhibitors of kinases associated with the PIK3/Akt/mTOR signaling pathway, such as PI3K, Akt (including Akt1, Akt2 and Akt3) and mTOR kinases.

In some embodiments, compounds described herein can be used in combination with one or more inhibitors of the enzyme or protein receptors such as HPK1, SBLB, TUT4, A2A/A2B, CD47, CDK2, STING, ALK2, LIN28, ADAR1, MAT2a, RIOK1, HDAC8, WDR5, SMARCA2, and DCLK1 for the treatment of diseases and disorders. Exemplary diseases and disorders include cancer, infection, inflammation and neurodegenerative disorders.

In some embodiments, compouds described herein can be used in combination with a therapeutic agent that targets an epigenetic regulator. Examples of epigenetic regulators include bromodomain inhibitors, the histone lysine methyltransferases, histone arginine methyl transferases, histone demethylases, histone deacetylases, histone acetylases, and DNA methyltransferases. Histone deacetylase inhibitors include, e.g., vorinostat.

For treating cancer and other proliferative diseases, compounds described herein can be used in combination with targeted therapies, including JAK kinase inhibitors (Ruxolitinib, additional JAK1/2 and JAK1-selective, baricitinib or INCB39110), Pim kinase inhibitors (e.g., LGH447, INCB053914 and SGI-1776), PI3 kinase inhibitors including PI3K-delta selective and broad spectrum PI3K inhibitors (e.g., INCB50465 and INCB54707), PI3K-gamma inhibitors such as PI3K-gamma selective inhibitors, MEK inhibitors, CSF1R inhibitors (e.g., PLX3397 and LY3022855), TAM receptor tyrosine kinases inhibitors (Tyro-3, Axl, and Mer; e.g., INCB81776), angiogenesis inhibitors, interleukin receptor inhibitors, Cyclin Dependent kinase inhibitors, BRAF inhibitors, mTOR inhibitors, proteasome inhibitors (Bortezomib, Carfilzomib), HDAC-inhibitors (panobinostat, vorinostat), DNA methyl transferase inhibitors, dexamethasone, bromo and extra terminal family members inhibitors (for example, bromodomain inhibitors or BET inhibitors, such as OTX015, CPI-0610, INCB54329 or INCB57643), LSD1 inhibitors (e.g., GSK2979552, INCB59872 and INCB60003), arginase inhibitors (e.g., INCB1158), indoleamine 2,3-dioxygenase inhibitors (e.g., epacadostat, NLG919 or BMS-986205), PARP inhibiors (e.g., olaparib or rucaparib), and inhibitors of BTK such as ibrutinib.

In addition, for treating cancer and other proliferative diseases, compounds described herein can be used in combination with targeted therapies such as, e.g., c-MET inhibitors (e.g., capmatinib), an anti-CD19 antibody (e.g., tafasitamab), an ALK2 inhibitor (e.g., INCB00928); or combinations thereof.

For treating cancer and other proliferative diseases, compounds described herein can be used in combination with chemotherapeutic agents, agonists or antagonists of nuclear receptors, or other anti-proliferative agents. Compounds described herein can also be used in combination with a medical therapy such as surgery or radiotherapy, *e.g.,* gamma-radiation, neutron beam radiotherapy, electron beam radiotherapy, proton therapy, brachytherapy, and systemic radioactive isotopes.

Examples of suitable chemotherapeutic agents include any of: abarelix, abiraterone, afatinib, aflibercept, aldesleukin, alemtuzumab, alitretinoin, allopurinol, altretamine, amidox, amsacrine, anastrozole, aphidicolon, arsenic trioxide, asparaginase, axitinib, azacitidine, bevacizumab, bexarotene, baricitinib, bendamustine, bicalutamide, bleomycin, bortezombi, bortezomib, brivanib, buparlisib, busulfan intravenous, busulfan oral, calusterone, camptosar, capecitabine, carboplatin, carmustine, cediranib, cetuximab, chlorambucil, cisplatin, cladribine, clofarabine, crizotinib, cyclophosphamide, cytarabine, dacarbazine, dacomitinib, dactinomycin, dalteparin sodium, dasatinib, dactinomycin, daunorubicin, decitabine, degarelix, denileukin, denileukin diftitox, deoxycoformycin, dexrazoxane, didox, docetaxel, doxorubicin, droloxafine, dromostanolone propionate, eculizumab, enzalutamide, epidophyllotoxin, epirubicin, epothilones, erlotinib, estramustine, etoposide phosphate, etoposide, exemestane, fentanyl citrate, filgrastim, floxuridine, fludarabine, fluorouracil, flutamide, fulvestrant, gefitinib, gemcitabine, gemtuzumab ozogamicin, goserelin acetate, histrelin acetate, ibritumomab tiuxetan, idarubicin, idelalisib, ifosfamide, imatinib mesylate, interferon alfa 2a, irinotecan, lapatinib ditosylate, lenalidomide, letrozole, leucovorin, leuprolide acetate, levamisole, lonafarnib, lomustine, meclorethamine, megestrol acetate, melphalan, mercaptopurine, methotrexate, methoxsalen, mithramycin, mitomycin C, mitotane, mitoxantrone, nandrolone phenpropionate, navelbene, necitumumab, nelarabine, neratinib, nilotinib, nilutamide, niraparib, nofetumomab, oserelin, oxaliplatin, paclitaxel, pamidronate, panitumumab, panobinostat, pazopanib, pegaspargase, pegfilgrastim, pemetrexed disodium, pentostatin, pilaralisib, pipobroman, plicamycin, ponatinib, porfimer, prednisone, procarbazine, quinacrine, ranibizumab, rasburicase, regorafenib, reloxafine, revlimid, rituximab, rucaparib, ruxolitinib, sorafenib, streptozocin, sunitinib, sunitinib maleate, tamoxifen, tegafur, temozolomide, teniposide, testolactone, tezacitabine, thalidomide, thioguanine, thiotepa, tipifarnib, topotecan, toremifene, tositumomab, trastuzumab, tretinoin, triapine, trimidox, triptorelin, uracil mustard, valrubicin, vandetanib, vinblastine, vincristine, vindesine, vinorelbine, vorinostat, veliparib, talazoparib, and zoledronate.

In some embodiments, compounds described herein can be used in combination with immune checkpoint inhibitors. Exemplary immune checkpoint inhibitors include inhibitors against immune checkpoint molecules such as CD27, CD28, CD40, CD122, CD96, CD73, CD47, OX40, GITR, CSF1R, JAK, PI3K delta, PI3K gamma, TAM, arginase, CD137 (also known as 4-1BB), ICOS, A2AR, B7-H3, B7-H4, BTLA, CTLA-4, LAG3 (e.g., INCAGN2385), TIM3 (e.g., INCB2390), VISTA, PD-1, PD-L1 and PD-L2. In some embodiments, the immune checkpoint molecule is a stimulatory checkpoint molecule selected from CD27, CD28, CD40, ICOS, OX40 (e.g., INCAGN1949), GITR (e.g., INCAGN1876) and CD137. In some embodiments, the immune checkpoint molecule is an inhibitory checkpoint molecule selected from A2AR, B7-H3, B7-H4, BTLA, CTLA-4, IDO, KIR, LAG3, PD-1, TIM3, and VISTA. In some embodiments, the compounds provided herein can be used in combination with one or more agents selected from KIR inhibitors, TIGIT inhibitors, LAIR1 inhibitors, CD160 inhibitors, 2B4 inhibitors and TGFR beta inhibitors.

In some embodiments, the inhibitor of an immune checkpoint molecule is a small molecule PD-L1 inhibitor. In some embodiments, the small molecule PD-L1 inhibitor has an IC50 less than 1 µM, less than 100 nM, less than 10 nM or less than 1 nM in a PD-L1 assay described in US Patent Application Publication Nos. US 2017/0107216, US 2017/0145025, US 2017/0174671, US 2017/0174679, US 2017/0320875, US 2017/0342060, US 2017/0362253, and US 2018/0016260, each of which is incorporated by reference in its entirety for all purposes.

In some embodiments, the inhibitor of an immune checkpoint molecule is an inhibitor of PD-1, e.g., an anti-PD-1 monoclonal antibody. In some embodiments, the anti-PD-1 monoclonal antibody is MGA012 (retifanlimab), nivolumab, pembrolizumab (also known as MK-3475), pidilizumab, SHR-1210, PDR001, ipilumimab or AMP-224. In some embodiments, the anti-PD-1 monoclonal antibody is nivolumab or pembrolizumab. In some embodiments, the anti-PD1 antibody is pembrolizumab. In some embodiments, the anti-PD1 antibody is nivolumab. In some embodiments, the anti-PD-1 monoclonal antibody is MGA012 (retifanlimab). In some embodiments, the anti-PD1 antibody is SHR-1210. Other anti-cancer agent(s) include antibody therapeutics such as 4-1BB (e.g. urelumab, utomilumab.

In some embodiments, the compounds of the disclosure can be used in combination with INCB086550.

In some embodiments, the inhibitor of an immune checkpoint molecule is an inhibitor of PD-L1, e.g., an anti-PD-L1 monoclonal antibody. In some embodiments, the anti-PD-L1 monoclonal antibody is BMS-935559, MEDI4736, MPDL3280A (also known as RG7446), or MSB0010718C. In some embodiments, the anti-PD-L1 monoclonal antibody is MPDL3280A or MEDI4736.

In some embodiments, the inhibitor of an immune checkpoint molecule is an inhibitor of CTLA-4, e.g., an anti-CTLA-4 antibody. In some embodiments, the anti-CTLA-4 antibody is ipilimumab, tremelimumab, AGEN1884, or CP-675,206.

In some embodiments, the inhibitor of an immune checkpoint molecule is an inhibitor of LAG3, e.g., an anti-LAG3 antibody. In some embodiments, the anti-LAG3 antibody is BMS-986016, LAG525, or INCAGN2385.

In some embodiments, the inhibitor of an immune checkpoint molecule is an inhibitor of TIM3, e.g., an anti-TIM3 antibody. In some embodiments, the anti-TIM3 antibody is INCAGN2390, MBG453, or TSR-022.

In some embodiments, the inhibitor of an immune checkpoint molecule is an inhibitor of GITR, e.g., an anti-GITR antibody. In some embodiments, the anti-GITR antibody is TRX518, MK-4166, INCAGN1876, MK-1248, AMG228, BMS-986156, GWN323, or MEDI1873.

In some embodiments, the inhibitor of an immune checkpoint molecule is an agonist of OX40, e.g., OX40 agonist antibody or OX40L fusion protein. In some embodiments, the anti-OX40 antibody is MEDI0562, MOXR-0916, PF-04518600, GSK3174998, or BMS-986178. In some embodiments, the OX40L fusion protein is MEDI6383.

In some embodiments, the inhibitor of an immune checkpoint molecule is an inhibitor of CD20, e.g., an anti-CD20 antibody. In some embodiments, the anti-CD20 antibody is obinutuzumab or rituximab.

The compounds of the present disclosure can be used in combination with bispecific antibodies. In some embodiments, one of the domains of the bispecific antibody targets PD-1, PD-L1, CTLA-4, GITR, OX40, TIM3, LAG3, CD137, ICOS, CD3 or TGFβ receptor.

In some embodiments, the compounds of the disclosure can be used in combination with one or more metabolic enzyme inhibitors. In some embodiments, the metabolic enzyme inhibitor is an inhibitor of IDO1, TDO, or arginase. Examples of IDO1 inhibitors include epacadostat, NLG919, BMS-986205, PF-06840003, IOM2983, RG-70099 and LY338196.

In some embodiments, the compounds described herein can be used in combination with one or more agents for the treatment of diseases such as cancer. In some embodiments, the agent is an alkylating agent, a proteasome inhibitor, a corticosteroid, or an immunomodulatory agent. Examples of an alkylating agent include cyclophosphamide (CY), melphalan (MEL), and bendamustine. In some embodiments, the proteasome inhibitor is carfilzomib. In some embodiments, the corticosteroid is dexamethasone (DEX). In some embodiments, the immunomodulatory agent is lenalidomide (LEN) or pomalidomide (POM).

Suitable antiviral agents contemplated for use in combination with compounds of the present disclosure can comprise nucleoside and nucleotide reverse transcriptase inhibitors (NRTIs), non-nucleoside reverse transcriptase inhibitors (NNRTIs), protease inhibitors and other antiviral drugs.

Example suitable NRTIs include zidovudine (AZT); didanosine (ddl); zalcitabine (ddC); stavudine (d4T); lamivudine (3TC); abacavir (1592U89); adefovir dipivoxil [bis(POM)-PMEA]; lobucavir (BMS-180194); BCH-10652; emitricitabine [(-)-FTC]; beta-L-FD4 (also called beta-L-D4C and named beta-L-2', 3'-dicleoxy-5-fluoro-cytidene); DAPD, ((-)-beta-D-2,6,-diamino-purine dioxolane); and lodenosine (FddA). Typical suitable NNRTIs include nevirapine (BI-RG-587); delaviradine (BHAP, U-90152); efavirenz (DMP-266); PNU-142721; AG-1549; MKC-442 (1-(ethoxy-methyl)-5-(1-methylethyl)-6-(phenylmethyl)-(2,4(1H,3H)-pyrimidinedione); and (+)-calanolide A (NSC-675451) and B. Typical suitable protease inhibitors include saquinavir (Ro 31-8959); ritonavir (ABT-538); indinavir (MK-639); nelfnavir (AG-1343); amprenavir (141W94); lasinavir (BMS-234475); DMP-450; BMS-2322623; ABT-378; and AG-1 549. Other antiviral agents include hydroxyurea, ribavirin, IL-2, IL-12, pentafuside and Yissum Project No.11607.

Suitable agents for use in combination with compounds described herein for the treatment of cancer include chemotherapeutic agents, targeted cancer therapies, immunotherapies or radiation therapy. Compounds described herein may be effective in combination with anti-hormonal agents for treatment of breast cancer and other tumors. Suitable examples are anti-estrogen agents including but not limited to tamoxifen and toremifene, aromatase inhibitors including but not limited to letrozole, anastrozole, and exemestane, adrenocorticosteroids (e.g. prednisone), progestins (e.g. megastrol acetate), and estrogen receptor antagonists (e.g. fulvestrant). Suitable anti-hormone agents used for treatment of prostate and other cancers may also be combined with compounds described herein. These include anti-androgens including but not limited to flutamide, bicalutamide, and nilutamide, luteinizing hormone-releasing hormone (LHRH) analogs including leuprolide, goserelin, triptorelin, and histrelin, LHRH antagonists (e.g. degarelix), androgen receptor blockers (e.g. enzalutamide) and agents that inhibit androgen production (e.g. abiraterone).

The compounds described herein may be combined with or in sequence with other agents against membrane receptor kinases especially for patients who have developed primary or acquired resistance to the targeted therapy. These therapeutic agents include inhibitors or antibodies against EGFR, Her2, VEGFR, c-Met, Ret, IGFR1, or Flt-3 and against cancer-associated fusion protein kinases such as Bcr-Abl and EML4-Alk. Inhibitors against EGFR include gefitinib and erlotinib, and inhibitors against EGFR/Her2 include but are not limited to dacomitinib, afatinib, lapitinib and neratinib. Antibodies against the EGFR include but are not limited to cetuximab, panitumumab and necitumumab. Inhibitors of c-Met may be used in combination with KRAS inhibitors. These include onartumzumab, tivantnib, and INC-280. Agents against Abl (or Bcr-Abl) include imatinib, dasatinib, nilotinib, and ponatinib and those against Alk (or EML4-ALK) include crizotinib.

Angiogenesis inhibitors may be efficacious in some tumors in combination with KRAS inhibitors. These include antibodies against VEGF or VEGFR or kinase inhibitors of VEGFR. Antibodies or other therapeutic proteins against VEGF include bevacizumab and aflibercept. Inhibitors of VEGFR kinases and other anti-angiogenesis inhibitors include but are not limited to sunitinib, sorafenib, axitinib, cediranib, pazopanib, regorafenib, brivanib, and vandetanib

Activation of intracellular signaling pathways is frequent in cancer, and agents targeting components of these pathways have been combined with receptor targeting agents to enhance efficacy and reduce resistance. Examples of agents that may be combined with compounds described herein include inhibitors of the PI3K-AKT-mTOR pathway, inhibitors of the Raf-MAPK pathway, inhibitors of JAK-STAT pathway, and inhibitors of protein chaperones and cell cycle progression.

Agents against the PI3 kinase include but are not limited topilaralisib, idelalisib, buparlisib. Inhibitors of mTOR such as rapamycin, sirolimus, temsirolimus, and everolimus may be combined with KRAS inhibitors. Other suitable examples include but are not limited to vemurafenib and dabrafenib (Raf inhibitors) and trametinib, selumetinib and GDC-0973 (MEK inhibitors). Inhibitors of one or more JAKs (e.g., ruxolitinib, baricitinib, tofacitinib), Hsp90 (e.g., tanespimycin), cyclin dependent kinases (e.g., palbociclib), HDACs (e.g., panobinostat), PARP (e.g., olaparib), and proteasomes (e.g., bortezomib, carfilzomib) can also be combined with compounds described herein. In some embodiments, the JAK inhibitor is selective for JAK1 over JAK2 and JAK3.

Other suitable agents for use in combination with compounds described herein include chemotherapy combinations such as platinum-based doublets used in lung cancer and other solid tumors (cisplatin or carboplatin plus gemcitabine; cisplatin or carboplatin plus docetaxel; cisplatin or carboplatin plus paclitaxel; cisplatin or carboplatin plus pemetrexed) or gemcitabine plus paclitaxel bound particles (Abraxane^{®}).

Suitable chemotherapeutic or other anti-cancer agents include, for example, alkylating agents (including, without limitation, nitrogen mustards, ethylenimine derivatives, alkyl sulfonates, nitrosoureas and triazenes) such as uracil mustard, chlormethine, cyclophosphamide (Cytoxan^{™}), ifosfamide, melphalan, chlorambucil, pipobroman, triethylene-melamine, triethylenethiophosphoramine, busulfan, carmustine, lomustine, streptozocin, dacarbazine, and temozolomide.

Other suitable agents for use in combination with compounds described herein include steroids including 17 alpha-ethinylestradiol, diethylstilbestrol, testosterone, prednisone, fluoxymesterone, methylprednisolone, methyltestosterone, prednisolone, triamcinolone, chlorotrianisene, hydroxyprogesterone, aminoglutethimide, and medroxyprogesteroneacetate.

Other suitable agents for use in combination with compounds described herein include: dacarbazine (DTIC), optionally, along with other chemotherapy drugs such as carmustine (BCNU) and cisplatin; the "Dartmouth regimen," which consists of DTIC, BCNU, cisplatin and tamoxifen; a combination of cisplatin, vinblastine, and DTIC; or temozolomide. Compounds described herein may also be combined with immunotherapy drugs, including cytokines such as interferon alpha, interleukin 2, and tumor necrosis factor (TNF) in.

Suitable chemotherapeutic or other anti-cancer agents include, for example, antimetabolites (including, without limitation, folic acid antagonists, pyrimidine analogs, purine analogs and adenosine deaminase inhibitors) such as methotrexate, 5-fluorouracil, floxuridine, cytarabine, 6-mercaptopurine, 6-thioguanine, fludarabine phosphate, pentostatine, and gemcitabine.

Suitable chemotherapeutic or other anti-cancer agents further include, for example, certain natural products and their derivatives (for example, vinca alkaloids, antitumor antibiotics, enzymes, lymphokines and epipodophyllotoxins) such as vinblastine, vincristine, vindesine, bleomycin, dactinomycin, daunorubicin, doxorubicin, epirubicin, idarubicin, ara-C, paclitaxel (TAXOL^{™}), mithramycin, deoxycoformycin, mitomycin-C, L-asparaginase, interferons (especially IFN-a), etoposide, and teniposide.

Other cytotoxic agents include navelbene, CPT-11, anastrazole, letrazole, capecitabine, reloxafine, cyclophosphamide, ifosamide, and droloxafine.

Also suitable are cytotoxic agents such as epidophyllotoxin; an antineoplastic enzyme; a topoisomerase inhibitor; procarbazine; mitoxantrone; platinum coordination complexes such as cis-platin and carboplatin; biological response modifiers; growth inhibitors; antihormonal therapeutic agents; leucovorin; tegafur; and haematopoietic growth factors.

Other anti-cancer agent(s) include antibody therapeutics such as trastuzumab (Herceptin), antibodies to costimulatory molecules such as CTLA-4, 4-1BB, PD-L1 and PD-1 antibodies, or antibodies to cytokines (IL-10, TGF-β, etc.).

Other anti-cancer agents also include those that block immune cell migration such as antagonists to chemokine receptors, including CCR2 and CCR4.

Other anti-cancer agents also include those that augment the immune system such as adjuvants or adoptive T cell transfer.

Anti-cancer vaccines include dendritic cells, synthetic peptides, DNA vaccines and recombinant viruses. In some embodiments, tumor vaccines include the proteins from viruses implicated in human cancers such as Human Papilloma Viruses (HPV), Hepatitis Viruses (HBV and HCV) and Kaposi's Herpes Sarcoma Virus (KHSV). Non-limiting examples of tumor vaccines that can be used include peptides of melanoma antigens, such as peptides of gp100, MAGE antigens, Trp-2, MARTI and/or tyrosinase, or tumor cells transfected to express the cytokine GM-CSF.

The compounds of the present disclosure can be used in combination with bone marrow transplant for the treatment of a variety of tumors of hematopoietic origin.

Methods for the safe and effective administration of most of these chemotherapeutic agents are known to those skilled in the art. In addition, their administration is described in the standard literature. For example, the administration of many of the chemotherapeutic agents is described in the "Physicians' Desk Reference" (PDR, e.g., 1996 edition, Medical Economics Company, Montvale, NJ), the disclosure of which is incorporated herein by reference as if set forth in its entirety.

As provided throughout, the additional compounds, inhibitors, agents, etc. can be combined with the present compound in a single or continuous dosage form, or they can be administered simultaneously or sequentially as separate dosage forms.

### Formulation, Dosage Forms and Administration

When employed as pharmaceuticals, the compounds of the present disclosure can be administered in the form of pharmaceutical compositions. Thus, the present disclosure provides a composition comprising a compound of Formula I, II, or any of the formulas as described herein, a compound as recited in any of the clauses and described herein, or a pharmaceutically acceptable salt thereof, or any of the embodiments thereof, and at least one pharmaceutically acceptable carrier or excipient. These compositions can be prepared in a manner well known in the pharmaceutical art, and can be administered by a variety of routes, depending upon whether local or systemic treatment is indicated and upon the area to be treated. Administration may be topical (including transdermal, epidermal, ophthalmic and to mucous membranes including intranasal, vaginal and rectal delivery), pulmonary (e.g., by inhalation or insufflation of powders or aerosols, including by nebulizer; intratracheal or intranasal), oral or parenteral. Parenteral administration includes intravenous, intraarterial, subcutaneous, intraperitoneal intramuscular or injection or infusion; or intracranial, e.g., intrathecal or intraventricular, administration. Parenteral administration can be in the form of a single bolus dose, or may be, e.g., by a continuous perfusion pump. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

This invention also includes pharmaceutical compositions which contain, as the active ingredient, the compound of the present disclosure or a pharmaceutically acceptable salt thereof, in combination with one or more pharmaceutically acceptable carriers or excipients. In some embodiments, the composition is suitable for topical administration. In making the compositions of the invention, the active ingredient is typically mixed with an excipient, diluted by an excipient or enclosed within such a carrier in the form of, e.g., a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, e.g., up to 10% by weight of the active compound, soft and hard gelatin capsules, suppositories, sterile injectable solutions and sterile packaged powders.

In preparing a formulation, the active compound can be milled to provide the appropriate particle size prior to combining with the other ingredients. If the active compound is substantially insoluble, it can be milled to a particle size of less than 200 mesh. If the active compound is substantially water soluble, the particle size can be adjusted by milling to provide a substantially uniform distribution in the formulation, e.g., about 40 mesh.

The compounds of the invention may be milled using known milling procedures such as wet milling to obtain a particle size appropriate for tablet formation and for other formulation types. Finely divided (nanoparticulate) preparations of the compounds of the invention can be prepared by processes known in the art see, e.g., WO 2002/000196.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup and methyl cellulose. The formulations can additionally include: lubricating agents such as talc, magnesium stearate and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

In some embodiments, the pharmaceutical composition comprises silicified microcrystalline cellulose (SMCC) and at least one compound described herein, or a pharmaceutically acceptable salt thereof. In some embodiments, the silicified microcrystalline cellulose comprises about 98% microcrystalline cellulose and about 2% silicon dioxide w/w.

In some embodiments, the composition is a sustained release composition comprising at least one compound described herein, or a pharmaceutically acceptable salt thereof, and at least one pharmaceutically acceptable carrier or excipient. In some embodiments, the composition comprises at least one compound described herein, or a pharmaceutically acceptable salt thereof, and at least one component selected from microcrystalline cellulose, lactose monohydrate, hydroxypropyl methylcellulose and polyethylene oxide. In some embodiments, the composition comprises at least one compound described herein, or a pharmaceutically acceptable salt thereof, and microcrystalline cellulose, lactose monohydrate and hydroxypropyl methylcellulose. In some embodiments, the composition comprises at least one compound described herein, or a pharmaceutically acceptable salt thereof, and microcrystalline cellulose, lactose monohydrate and polyethylene oxide. In some embodiments, the composition further comprises magnesium stearate or silicon dioxide. In some embodiments, the microcrystalline cellulose is Avicel PH102^{™}. In some embodiments, the lactose monohydrate is Fast-flo 316^{™}. In some embodiments, the hydroxypropyl methylcellulose is hydroxypropyl methylcellulose 2208 K4M (e.g., Methocel K4 M Premier^{™}) and/or hydroxypropyl methylcellulose 2208 K100LV (e.g., Methocel K00LV^{™}). In some embodiments, the polyethylene oxide is polyethylene oxide WSR 1105 (e.g., Polyox WSR 1105^{™}).

In some embodiments, a wet granulation process is used to produce the composition. In some embodiments, a dry granulation process is used to produce the composition.

The compositions can be formulated in a unit dosage form, each dosage containing from about 5 to about 1,000 mg (1 g), more usually about 100 mg to about 500 mg, of the active ingredient. In some embodiments, each dosage contains about 10 mg of the active ingredient. In some embodiments, each dosage contains about 50 mg of the active ingredient. In some embodiments, each dosage contains about 25 mg of the active ingredient. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

The components used to formulate the pharmaceutical compositions are of high purity and are substantially free of potentially harmful contaminants (e.g., at least National Food grade, generally at least analytical grade, and more typically at least pharmaceutical grade). Particularly for human consumption, the composition is preferably manufactured or formulated under Good Manufacturing Practice standards as defined in the applicable regulations of the U.S. Food and Drug Administration. For example, suitable formulations may be sterile and/or substantially isotonic and/or in full compliance with all Good Manufacturing Practice regulations of the U.S. Food and Drug Administration.

The active compound may be effective over a wide dosage range and is generally administered in a therapeutically effective amount. It will be understood, however, that the amount of the compound actually administered will usually be determined by a physician, according to the relevant circumstances, including the condition to be treated, the chosen route of administration, the actual compound administered, the age, weight, and response of the individual patient, the severity of the patient's symptoms and the like.

The therapeutic dosage of a compound of the present invention can vary according to, e.g., the particular use for which the treatment is made, the manner of administration of the compound, the health and condition of the patient, and the judgment of the prescribing physician. The proportion or concentration of a compound of the invention in a pharmaceutical composition can vary depending upon a number of factors including dosage, chemical characteristics (e.g., hydrophobicity), and the route of administration. For example, the compounds of the invention can be provided in an aqueous physiological buffer solution containing about 0.1 to about 10% w/v of the compound for parenteral administration. Some typical dose ranges are from about 1 µg/kg to about 1 g/kg of body weight per day. In some embodiments, the dose range is from about 0.01 mg/kg to about 100 mg/kg of body weight per day. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from in vitro or animal model test systems.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, the active ingredient is typically dispersed evenly throughout the composition so that the composition can be readily subdivided into equally effective unit dosage forms such as tablets, pills and capsules. This solid preformulation is then subdivided into unit dosage forms of the type described above containing from, e.g., about 0.1 to about 1000 mg of the active ingredient of the present invention.

The tablets or pills of the present invention can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compounds and compositions of the present invention can be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* In some embodiments, the compositions are administered by the oral or nasal respiratory route for local or systemic effect. Compositions can be nebulized by use of inert gases. Nebulized solutions may be breathed directly from the nebulizing device or the nebulizing device can be attached to a face mask, tent, or intermittent positive pressure breathing machine. Solution, suspension, or powder compositions can be administered orally or nasally from devices which deliver the formulation in an appropriate manner.

Topical formulations can contain one or more conventional carriers. In some embodiments, ointments can contain water and one or more hydrophobic carriers selected from, *e.g.,* liquid paraffin, polyoxyethylene alkyl ether, propylene glycol, white Vaseline, and the like. Carrier compositions of creams can be based on water in combination with glycerol and one or more other components, *e.g.,* glycerinemonostearate, PEG-glycerinemonostearate and cetylstearyl alcohol. Gels can be formulated using isopropyl alcohol and water, suitably in combination with other components such as, *e.g.,* glycerol, hydroxyethyl cellulose, and the like. In some embodiments, topical formulations contain at least about 0.1, at least about 0.25, at least about 0.5, at least about 1, at least about 2 or at least about 5 wt % of the compound of the invention. The topical formulations can be suitably packaged in tubes of, e.g., 100 g which are optionally associated with instructions for the treatment of the select indication, *e.g.,* psoriasis or other skin condition.

The amount of compound or composition administered to a patient will vary depending upon what is being administered, the purpose of the administration, such as prophylaxis or therapy, the state of the patient, the manner of administration and the like. In therapeutic applications, compositions can be administered to a patient already suffering from a disease in an amount sufficient to cure or at least partially arrest the symptoms of the disease and its complications. Effective doses will depend on the disease condition being treated as well as by the judgment of the attending clinician depending upon factors such as the severity of the disease, the age, weight and general condition of the patient and the like.

The compositions administered to a patient can be in the form of pharmaceutical compositions described above. These compositions can be sterilized by conventional sterilization techniques, or may be sterile filtered. Aqueous solutions can be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers or stabilizers will result in the formation of pharmaceutical salts.

The therapeutic dosage of a compound of the present invention can vary according to, *e.g.,* the particular use for which the treatment is made, the manner of administration of the compound, the health and condition of the patient, and the judgment of the prescribing physician. The proportion or concentration of a compound of the invention in a pharmaceutical composition can vary depending upon a number of factors including dosage, chemical characteristics *(e.g.,* hydrophobicity), and the route of administration. For example, the compounds of the invention can be provided in an aqueous physiological buffer solution containing about 0.1 to about 10% w/v of the compound for parenteral administration. Some typical dose ranges are from about 1 µg/kg to about 1 g/kg of body weight per day. In some embodiments, the dose range is from about 0.01 mg/kg to about 100 mg/kg of body weight per day. The dosage is likely to depend on such variables as the type and extent of progression of the disease or disorder, the overall health status of the particular patient, the relative biological efficacy of the compound selected, formulation of the excipient, and its route of administration. Effective doses can be extrapolated from dose-response curves derived from *in vitro* or animal model test systems.

### Labeled Compounds and Assay Methods

Another aspect of the present invention relates to labeled compounds of the disclosure (radio-labeled, fluorescent-labeled, etc.) that would be useful not only in imaging techniques but also in assays, both *in vitro* and *in vivo,* for localizing and quantitating KRAS protein in tissue samples, including human, and for identifying KRAS ligands by inhibition binding of a labeled compound. Substitution of one or more of the atoms of the compounds of the present disclosure can also be useful in generating differentiated ADME (Adsorption, Distribution, Metabolism and Excretion). Accordingly, the present invention includes KRAS binding assays that contain such labeled or substituted compounds.

The present disclosure further includes isotopically-labeled compounds of the disclosure. An "isotopically" or "radio-labeled" compound is a compound of the disclosure where one or more atoms are replaced or substituted by an atom having an atomic mass or mass number different from the atomic mass or mass number typically found in nature (i.e., naturally occurring). Suitable radionuclides that may be incorporated in compounds of the present disclosure include but are not limited to ²H (also written as D for deuterium), ³H (also written as T for tritium), ¹¹C, ¹³C, ¹⁴C, ¹³N, ¹⁵N, ¹⁵O, ¹⁷O, ¹⁸O, ¹⁸F, ³⁵S, ³⁶Cl, ⁸²Br, ⁷⁵Br, ⁷⁶Br, ⁷⁷Br, ¹²³I, ¹²⁴I, ¹²⁵I and ¹³¹I. For example, one or more hydrogen atoms in a compound of the present disclosure can be replaced by deuterium atoms (e.g., one or more hydrogen atoms of a C₁₋₆ alkyl group of Formula I, II, or any formulae provided herein can be optionally substituted with deuterium atoms, such as -CD₃ being substituted for -CH₃). In some embodiments, alkyl groups in Formula I, II, or any formulae provided herein can be perdeuterated.

One or more constituent atoms of the compounds presented herein can be replaced or substituted with isotopes of the atoms in natural or non-natural abundance. In some embodiments, the compound includes at least one deuterium atom. In some embodiments, the compound includes two or more deuterium atoms. In some embodiments, the compound includes 1-2, 1-3, 1-4, 1-5, or 1-6 deuterium atoms. In some embodiments, all of the hydrogen atoms in a compound can be replaced or substituted by deuterium atoms.

Synthetic methods for including isotopes into organic compounds are known in the art (Deuterium Labeling in Organic Chemistry by Alan F. Thomas (New York, N.Y., Appleton-Century-Crofts, 1971; The Renaissance of H/D Exchange by Jens Atzrodt, Volker Derdau, Thorsten Fey and Jochen Zimmermann, Angew. Chem. Int. Ed. 2007, 7744-7765; The Organic Chemistry of Isotopic Labelling by James R. Hanson, Royal Society of Chemistry, 2011). Isotopically labeled compounds can be used in various studies such as NMR spectroscopy, metabolism experiments, and/or assays.

Substitution with heavier isotopes, such as deuterium, may afford certain therapeutic advantages resulting from greater metabolic stability, for example, increased *in vivo* half-life or reduced dosage requirements, and hence may be preferred in some circumstances. (see *e.g.,* A. Kerekes et. al. J. Med. Chem. 2011, 54, 201-210; R. Xu et. al. J. Label Compd. Radiopharm. 2015, 58, 308-312). In particular, substitution at one or more metabolism sites may afford one or more of the therapeutic advantages.

The radionuclide that is incorporated in the instant radio-labeled compounds will depend on the specific application of that radio-labeled compound. For example, for *in vitro* adenosine receptor labeling and competition assays, compounds that incorporate ³H, ¹⁴C, ⁸²Br, ¹²⁵I, ¹³¹I or ³⁵S can be useful. For radio-imaging applications ¹¹C, ¹⁸F, ¹²⁵I, ¹²³I, ¹²⁴I, ¹³¹I, ⁷⁵Br, ⁷⁶Br or ⁷⁷Br can be useful.

It is understood that a "radio-labeled" or "labeled compound" is a compound that has incorporated at least one radionuclide. In some embodiments, the radionuclide is selected from ³H, ¹⁴C, ¹²¹I, ³⁵S and ⁸²Br.

The present disclosure can further include synthetic methods for incorporating radioisotopes into compounds of the disclosure. Synthetic methods for incorporating radioisotopes into organic compounds are well known in the art, and an ordinary skill in the art will readily recognize the methods applicable for the compounds of disclosure.

A labeled compound of the invention can be used in a screening assay to identify and/or evaluate compounds. For example, a newly synthesized or identified compound (i.e., test compound) which is labeled can be evaluated for its ability to bind a KRAS protein by monitoring its concentration variation when contacting with the KRAS, through tracking of the labeling. For example, a test compound (labeled) can be evaluated for its ability to reduce binding of another compound which is known to bind to a KRAS protein *(i.e.,* standard compound). Accordingly, the ability of a test compound to compete with the standard compound for binding to the KRAS protein directly correlates to its binding affinity. Conversely, in some other screening assays, the standard compound is labeled and test compounds are unlabeled. Accordingly, the concentration of the labeled standard compound is monitored in order to evaluate the competition between the standard compound and the test compound, and the relative binding affinity of the test compound is thus ascertained.

### Kits

The present disclosure also includes pharmaceutical kits useful, *e.g.,* in the treatment or prevention of diseases or disorders associated with the activity of KRAS, such as cancer or infections, which include one or more containers containing a pharmaceutical composition comprising a therapeutically effective amount of a compound of Formula I, II, or any of the embodiments thereof. Such kits can further include one or more of various conventional pharmaceutical kit components, such as, *e.g.,* containers with one or more pharmaceutically acceptable carriers, additional containers, *etc.,* as will be readily apparent to those skilled in the art. Instructions, either as inserts or as labels, indicating quantities of the components to be administered, guidelines for administration, and/or guidelines for mixing the components, can also be included in the kit.

The invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results. The compounds of the Examples have been found to inhibit the activity of KRAS according to at least one assay described herein.

### EXAMPLES

Experimental procedures for compounds of the invention are provided below. Preparatory LC-MS purifications of some of the compounds prepared were performed on Waters mass directed fractionation systems. The basic equipment setup, protocols, and control software for the operation of these systems have been described in detail in the literature. See e.g. "Two-Pump At Column Dilution Configuration for Preparative LC-MS", K. Blom, J. Combi. Chem., 4, 295 (2002); "Optimizing Preparative LC-MS Configurations and Methods for Parallel Synthesis Purification", K. Blom, R. Sparks, J. Doughty, G. Everlof, T. Haque, A. Combs, J. Combi. Chem., 5, 670 (2003); and "Preparative LC-MS Purification: Improved Compound Specific Method Optimization", K. Blom, B. Glass, R. Sparks, A. Combs, J. Combi. Chem., 6, 874-883 (2004). The compounds separated were typically subjected to analytical liquid chromatography mass spectrometry (LCMS) for purity check.

The compounds separated were typically subjected to analytical liquid chromatography mass spectrometry (LCMS) for purity check under the following conditions: Instrument; Agilent 1100 series, LC/MSD, Column: Waters Sunfire^{™} C₁₈ 5 µm particle size, 2.1 x 5.0 mm, Buffers: mobile phase A: 0.025% TFA in water and mobile phase B: acetonitrile; gradient 2% to 80% of B in 3 minutes with flow rate 2.0 mL/minute.

Some of the compounds prepared were also separated on a preparative scale by reverse-phase high performance liquid chromatography (RP-HPLC) with MS detector or flash chromatography (silica gel) as indicated in the Examples. Typical preparative reverse-phase high performance liquid chromatography (RP-HPLC) column conditions are as follows:
pH = 2 purifications: Waters Sunfire^{™} C₁₈ 5 µm particle size, 19 x 100 mm column, eluting with mobile phase A: 0.1% TFA (trifluoroacetic acid) in water and mobile phase B: acetonitrile; the flow rate was 30 mL/minute, the separating gradient was optimized for each compound using the Compound Specific Method Optimization protocol as described in the literature [see "Preparative LCMS Purification: Improved Compound Specific Method Optimization", K. Blom, B. Glass, R. Sparks, A. Combs, J. Comb. Chem., 6, 874-883 (2004)]. Typically, the flow rate used with the 30 x 100 mm column was 60 mL/minute.
pH = 10 purifications: Waters XBridge C₁₈ 5 µm particle size, 19 x 100 mm column, eluting with mobile phase A: 0.15% NH₄OH in water and mobile phase B: acetonitrile; the flow rate was 30 mL/minute, the separating gradient was optimized for each compound using the Compound Specific Method Optimization protocol as described in the literature [See "Preparative LCMS Purification: Improved Compound Specific Method Optimization", K. Blom, B. Glass, R. Sparks, A. Combs, J. Comb. Chem., 6, 874-883 (2004)]. Typically, the flow rate used with 30 x 100 mm column was 60 mL/minute."

The following abbreviations may be used herein: AcOH (acetic acid); Ac₂O (acetic anhydride); aq. (aqueous); atm. (atmosphere(s)); Boc (t-butoxycarbonyl); BOP ((benzotriazol-1-yloxy)tris(dimethylamino)phosphonium hexafluorophosphate); br (broad); Cbz (carboxybenzyl); calc. (calculated); d (doublet); dd (doublet of doublets); DBU (1,8-diazabicyclo[5.4.0]undec-7-ene); DCM (dichloromethane); DIAD (N, N'-diisopropyl azidodicarboxylate); DIEA (N,N-diisopropylethylamine); DIPEA (N, N-diisopropylethylamine); DIBAL (diisobutylaluminium hydride); DMF (N, N-dimethylformamide); Et (ethyl); EtOAc (ethyl acetate); FCC (flash column chromatography); g (gram(s)); h (hour(s)); HATU (*N, N,* N', *N*'-tetramethyl-O-(7-azabenzotriazol-1-yl)uronium hexafluorophosphate); HCl (hydrochloric acid); HPLC (high performance liquid chromatography); Hz (hertz); J (coupling constant); LCMS (liquid chromatography - mass spectrometry); LDA (lithium diisopropylamide); m (multiplet); M (molar); mCPBA (3-chloroperoxybenzoic acid); MS (Mass spectrometry); Me (methyl); MeCN (acetonitrile); MeOH (methanol); mg (milligram(s)); min. (minutes(s)); mL (milliliter(s)); mmol (millimole(s)); N (normal); NCS (N-chlorosuccinimide); NEt₃ (triethylamine); nM (nanomolar); NMP (N-methylpyrrolidinone); NMR (nuclear magnetic resonance spectroscopy); OTf (trifluoromethanesulfonate); Ph (phenyl); pM (picomolar); PPT(precipitate); RP-HPLC (reverse phase high performance liquid chromatography); r.t. (room temperature), s (singlet); t (triplet or tertiary); TBS (tert-butyldimethylsilyl); tert (tertiary); tt (triplet of triplets); TFA (trifluoroacetic acid); THF (tetrahydrofuran); µg (microgram(s)); µL (microliter(s)); µM (micromolar); wt % (weight percent). Brine is saturated aqueous sodium chloride. *In vacuo* is under vacuum.

### Example 1a and Example 1b. 1-(4-(8-chloro-6-fluoro-7-(3-hydroxynaphthalen-1-yl)-4-(((S)-1-methylpyrrolidin-2-yl)methoxy)-1H-imidazo[4,5-c]quinolin-1-yl)piperidin-1-yl)prop-2-en-1-one

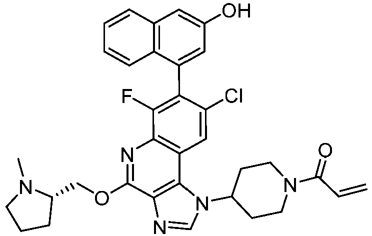

### Step 1. 2-Amino-4-bromo-5-chloro-3-fluorobenzoic acid

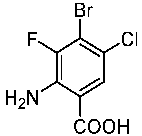

NCS (6.28 g, 47.0 mmol) was added to a solution of 2-amino-4-bromo-3-fluorobenzoic acid (10.0 g, 42.7 mmol) in DMF (100 ml) and then the reaction was stirred at 70 °C for 6 h. The mixture was cooled with ice water and then water (150 mL) was added and stirred for 20 min, the PPT was filtered and washed with water, dried to provide the desired product as a solid. LC-MS calculated for C₇H₅BrClFNO₂ (M+H)⁺: m/z = 269.9; found 269.9.

### Step 2. 7-Bromo-6-chloro-8-fluoro-2H-benzo[d][1,3]oxazine-2,4(1H)-dione

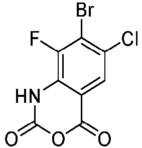

Triphosgene (7.27 g, 24.50 mmol) was added to a solution of 2-amino-4-bromo-5-chloro-3-fluorobenzoic acid (9.40 g, 35.0 mmol) in THF (100 ml) and then the reaction was stirred at 60 °C for 2 h. The reaction mixture was cooled with ice water and then 1 N HCl (40 mL) was added and stirred for 10 min and then filtered. The solid was washed with ethyl acetate to provide the desired product as a solid (5.0 g). The filtrate was diluted with ethyl acetate and separated, and the organic phase was dried and concentrated. The residue was washed with hexane and filtered, and dried to provide another batch of the product (4.3 g).

### Step 3. 7-Bromo-6-chloro-8-fluoro-3-nitroquinoline-2,4-diol

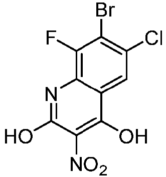

DIPEA (3.49 ml, 19.97 mmol) was added to a solution of ethyl 2-nitroacetate (2.66 g, 19.97 mmol) in toluene (40.0 ml) at r.t. and stirred for 10 min. 7-Bromo-6-chloro-8-fluoro-2H-benzo[*d*][1,3]oxazine-2,4(1*H*)-dione (2.94g, 9.98 mmol) was then added to the reaction mixture and the the reaction was stirred at 95 °C for 3 h. The reaction was cooled with ice water and then 1 N HCl (30 mL) was added and then filtered, then washed with small amount of ethyl acetate to provide the desired as a yellow solid (1.15 g, 34%). LC-MS calculated for C₉H₄BrClFN₂O₄ (M+H)⁺: m/z = 338.9; found 338.8.

### Step 4. 7-Bromo-2,4,6-trichloro-8-fluoro-3-nitroquinoline

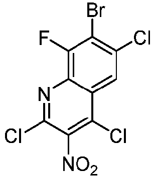

DIPEA (2.070 ml, 11.85 mmol) was added to a mixture of 7-bromo-6-chloro-8-fluoro-3-nitroquinoline-2,4-diol (1.0 g, 2.96 mmol) in POCl₃ (6.0 ml, 64.4 mmol) and then the reaction was stirred at 105 °C for 3 h. The solvent was removed under vacuum and then azeotroped with toluene 3 times to provide the crude material which was used in the next step directly.

### Step 5. tert-Butyl 4-((7-bromo-2,6-dichloro-8-fluoro-3-nitroquinolin-4-yl)amino)piperidine-1-carboxylate

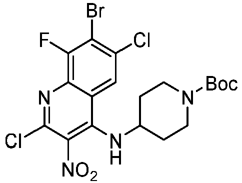

DIPEA (2.333 ml, 13.36 mmol) was added to a solution of 7-bromo-2,4,6-trichloro-8-fluoro-3-nitroquinoline (1.0 g, 2.67 mmol) and *tert-butyl* 4-aminopiperidine-1-carboxylate (1.0 g, 5.0 mmol) in CH₂Cl₂ (20.0 ml) and then the reaction was stirred at 50 °C overnight. The product was purified on silica gel (40 g, 0-40% EtOAc in CH₂Cl₂) to yield a yellow solid (1.08 g, 60% over two steps). LCMS calculated for C₁₉H₂₁BrCl₂FN₄O₄ (M+H)⁺: m/z = 539.0; found: 539.0.

### Step 6. tert-Butyl (S)-4-((7-bromo-6-chloro-8-fluoro-2-((1-methylpyrrolidin-2-yl)methoxy)-3-nitroquinolin-4-yl)amino)piperidine-1-carboxylate

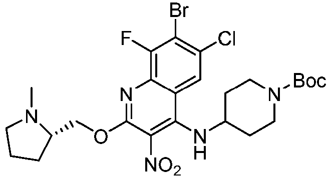

Sodium hydride (0.033 g, 1.394 mmol) was added to a solution of (S)-(1-methylpyrrolidin-2-yl)methanol (0.161 g, 1.394 mmol) in THF (3.0 ml) at 0 °C and then stirred at r.t. for 10 min. The mixture was then added to a solution of *tert-butyl* 4-((7-bromo-2,6-dichloro-8-fluoro-3-nitroquinolin-4-yl)amino)piperidine-1-carboxylate (0.50 g, 0.929 mmol) in THF (1.5 ml) at r.t. and stirred for 2 h. The mixture was quenched with saturated NaHCO₃ and extracted with CH₂Cl₂. The organic solvent was dried and concentrated to provide the product which was used in the next step directly. LCMS calculated for C₂₅H₃₃BrClFN₅O₅ (M+H)⁺: m/z = 618.1; found: 618.2.

### Step 7. tert-Butyl (S)-4-((3-amino-7-bromo-6-chloro-8-fluoro-2-((1-methylpyrrolidin-2-yl)methoxy)quinolin-4-yl)amino)piperidine-1-carboxylate

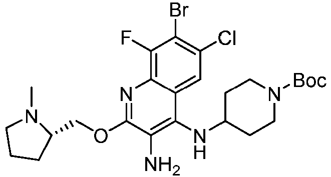

A mixture of *tert-butyl* (S)-4-((7-bromo-6-chloro-8-fluoro-2-((1-methylpyrrolidin-2-yl)-methoxy)-3-nitroquinolin-4-yl)amino)piperidine-1-carboxylate (100.0 mg, 0.162 mmol), iron (50.0 mg, 0.895 mmol) in acetic acid (3.00 ml) was stirred at 60 °C for 1 h. The mixture was diluted with CH₂Cl₂/MeOH and then filtered. The filtrate was concentrated and then dissolved in CH₂Cl₂ and washed with aqueous sodium carbonate. The organic phase was concentrated. The residue was purified on silica gel (12 g, 0-15% MeOH in CH₂Cl₂) to provide the desired product. LCMS calculated for C₂₅H₃₅BrClFN₅O₃ (M+H)⁺: m/z = 588.2; found: 588.2.

### Step 8. tert-Butyl (S)-4-(7-bromo-8-chloro-6-fluoro-4-((1-methylpyrrolidin-2-yl)methoxy)-1H-imidazo[4,5-c]quinolin-1-yl)piperidine-1-carboxylate

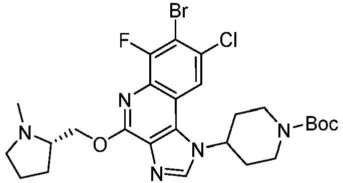

Triethylorthoformate (0.3 mL) was added to a mixture of tert-butyl (S)-4-((3-amino-7-bromo-6-chloro-8-fluoro-2-((1-methylpyrrolidin-2-yl)methoxy)quinolin-4-yl)amino)piperidine-1-carboxylate (0.3 g, 0.51 mmol) in toluene (1.2 mL) and the the reaction was stirred at 105 °C for 2 h. The solvent was removed to provide the desired product which was used in the next step directly. LCMS calculated for LCMS calculated for C₂₆H₃₃BrClFN₅O₃ (M+H)⁺: m/z = 598.2; found: 598.2.

### Step 9. (S)-1-(4-(7-Bromo-8-chloro-6-fluoro-4-((1-methylpyrrolidin-2-yl)methoxy)-1H-imidazo[4, 5-c]quinolin-1-yl) piperidin-1-yl)prop-2-en-1-one

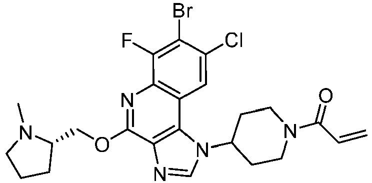

4N HCl (2.0 mL) was added to a solution of tert-butyl (S)-4-(7-bromo-8-chloro-6-fluoro-4-((1-methylpyrrolidin-2-yl)methoxy)-1*H*-imidazo[4,5-*c*]quinolin-1-yl)piperidine-1-carboxylate (300.0 mg, 0.503 mmol)in CH₂Cl₂/MeOH (1.5/0.5 mL) and stirred at r.t. for 30 min. The solvent was removed under vacuum. The residue was dissolved in CH₂Cl₂ (4.0 ml) and cooled to 0 °C, to this was added triethylamine (420 µl, 3.02 mmol) followed by acryloyl chloride (227 mg, 2.51 mmol) and the reaction was stirred at 0 °C for 20 min. The reaction was diluted with CH₂Cl₂, washed with saturated NaHCO₃, and the organic solvent was dried and concentrated. The residue was purified on silica gel (24 g, 0-15% MeOH in CH₂Cl₂ with 0.4% NEt₃) to provide the desired product. LCMS calculated for C₂₄H₂₇BrClFN₅O₂ (M+H)⁺: m/z = 552.1; found: 552.1.

### Step 10. 1-(4-(8-Chloro-6-fluoro-7-(3-hydroxynaphthalen-1-yl)-4-(((S)-1-methylpyrrolidin-2-yl)methoxy)-1H-imidazo[4,5-c]quinolin-1-yl)piperidin-1-yl)prop-2-en-1-one

A screw-cap vial equipped with a magnetic stir bar was charged (S)-1-(4-(7-bromo-8-chloro-6-fluoro-4-((1-methylpyrrolidin-2-yl)methoxy)-1*H*-imidazo[4,5-*c*]quinolin-1-yl)piperidin-1-yl)prop-2-en-1-one (30.0 mg, 0.05 mmol) , 4-(4,4,5,5-tetramethyl-1,3,2-dioxaborolan-2-yl)naphthalen-2-ol (30.2 mg, 0.112 mmol), sodium carbonate (17.77 mg, 0.168 mmol), Pd(Ph₃P)₄ (6.46 mg, 5.59 µmol) and dioxane (0.8 ml)/water (0.2 ml). The vial was sealed with a Teflon-lined septum, evacuated and backfilled with nitrogen (this process was repeated a total of three times). Then the reaction was stirred at 95 °C for 2 h. The mixture was diluted with acetonitrile/water and purified using prep-LCMS (XBridge C18 column, eluting with a gradient of acetonitrile/water containing 0.1% TFA, at flow rate of 60 mL/min) to afford the desired products as TFA salt.

**Example 1a***.* Diastereomer 1. Peak 1. LC-MS calculated for C₃₄H₃₄ClFN₅O₃ (M+H)⁺: m/z = 614.3; found 614.4. ¹H-NMR (500 MHz, DMSO-*d*₆) δ 10.03 (s, 1H), 9.85 (s, 1H), 8.70 (s, 1H), 8.42 (s, 1H), 7.84 (d, *J* = 8.3 Hz, 1H), 7.46 (t, *J* = 7.4 Hz, 1H), 7.32 (s, 1H), 7.23 (t, *J* = 7.5 Hz, 1H), 7.19 (d, *J* = 8.3 Hz, 1H), 7.12 (s, 1H), 6.91 (dd, *J* = 16.6, 10.5 Hz, 1H), 6.18 (d, *J* = 16.7 Hz, 1H), 5.75 (d, *J* = 10.6 Hz, 1H), 5.42 (t, *J* = 11.5 Hz, 1H), 5.01 (brs, 1H), 4.80 - 4.73 (m, 1H), 4.71 (d, *J* = 14.8 Hz, 1H), 4.32 (d, *J* = 12.4 Hz, 1H), 3.99 (s, 1H), 3.64 (d, *J* = 5.3 Hz, 1H), 3.54 (t, *J* = 12.8 Hz, 1H), 3.22 - 3.13 (m, 1H), 3.12 (d, *J* = 16.0 Hz, 1H), 3.07 (s, 3H), 2.40 - 2.26 (m, 3H), 2.17 - 2.02 (m, 3H), 2.02 - 1.87 (m, 2H).

**Example 1b***.* Diastereomer 2. Peak 2. LC-MS calculated for C₃₄H₃₄ClFN₅O₃ (M+H)⁺: m/z = 614.3; found 614.4.

### Example 2a and Example 2b. 1-(4-(8-chloro-6-fluoro-7-(2-fluoro-6-hydroxyphenyl)-4-(((S)-1-methylpyrrolidin-2-yl)methoxy)-1H-imidazo[4,5-c]quinolin-1-yl)piperidin-1-yl)prop-2-en-1-one

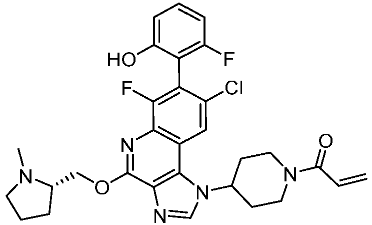

This compound was prepared according to the procedure described in **Example 1 step 10,** replacing 4-(4,4,5,5-tetramethyl-1,3,2-dioxaborolan-2-yl)naphthalen-2-ol with (2-fluoro-6-hydroxyphenyl)boronic acid.

**Example 2a.** Diastereomer 1. Peak 1. LCMS calculated for C₃₀H₃₁ClF₂N₅O₃ (M+H)+ m/z = 582.2; found 582.3.

**Example 2b.** Diastereomer 2. Peak 2. LCMS calculated for C₃₀H₃₁ClF₂N₅O₃ (M+H)+ m/z = 582.2; found 582.3.

### Example 3a and Example 3b. 1-(4-(8-Chloro-6-fluoro-7-(2-fluoro-3-hydroxyphenyl)-4-(((S)-1-methylpyrrolidin-2-yl)methoxy)-1H-imidazo[4,5-c]quinolin-1-yl)piperidin-1-yl)prop-2-en-1-one

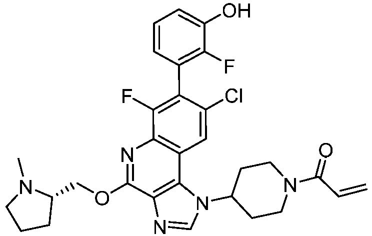

This compound was prepared according to the procedure described in **Example 1 step 10,** replacing 4-(4,4,5,5-tetramethyl-1,3,2-dioxaborolan-2-yl)naphthalen-2-ol with (2-fluoro-3-hydroxyphenyl)boronic acid.

**Example 3a.** Diastereomer 1. Peak 1. LCMS calculated for C₃₀H₃₁ClF_{Z}N₅O₃ (M+H)+ m/z = 582.2; found 582.2.

**Example 3b.** Diastereomer 2. Peak 2. LCMS calculated for C₃₀H₃₁ClF₂N₅O₃ (M+H)+ m/z = 582.2; found 582.2.

### Example 4a and Example 4b. 1-(4-(8-Chloro-6-fluoro-7-(5-methyl-1H-indazol-4-yl)-4-(((S)-1-methylpyrrolidin-2-yl)methoxy)-1H-imidazo[4,5-c]quinolin-1-yl)piperidin-1-yl)prop-2-en-1-one

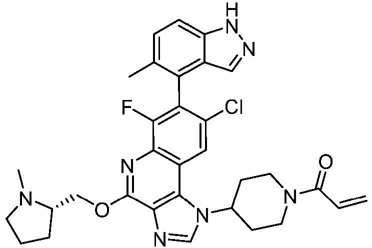

This compound was prepared according to the procedure described in **Example 1 step 10,** replacing 4-(4,4,5,5-tetramethyl-1,3,2-dioxaborolan-2-yl)naphthalen-2-ol with 5-methyl-4-(4,4,5,5-tetramethyl-1,3,2-dioxaborolan-2-yl)-1*H*-indazole.

**Example 4a.** Diastereomer 1. Peak 1. LCMS calculated for C₃₂H₃₄ClFN₇O₂ (M+H)⁺ m/z = 602.2; found 602.2.

**Example 4b.** Diastereomer 2. Peak 2. LCMS calculated for C₃₂H₃₄ClFN₇O₂ (M+H)⁺ m/z = 602.2; found 602.2.

### Example 5. 1-(3-(8-chloro-6-fluoro-7-(3-hydroxynaphthalen-1-yl)-4-(((S)-1-methylpyrrolidin-2-yl)methoxy)-1H-imidazo[4,5-c]quinolin-1-yl)piperidin-1-yl)prop-2-en-1-one

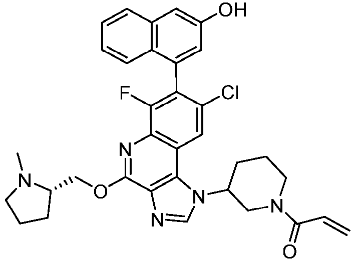

This compound was prepared according to the procedure described in **Example** 1, replacing tert-butyl 4-aminopiperidine-1-carboxylate with tert-butyl 3-aminopiperidine-1-carboxylate in **Step 5.** The product was isolated as a mixture of diastereomers. LCMS calculated for C₃₄H₃₄ClFN₅O₃ (M+H)⁺ m/z = 614.2 ; found 614.1.

### Example 6a and Example 6b. 1-(3-(8-chloro-6-fluoro-7-(3-hydroxynaphthalen-1-yl)-4-(((S)-1-methyl-pyrrolidin-2-yl)methoxy)-1H-imidazo[4,5-c]quinolin-1-yl)azetidin-1-yl)prop-2-en-1-one

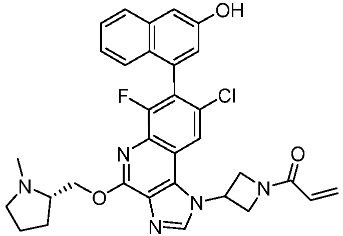

This compound was prepared according to the procedure described in **Example 1,** replacing tert-butyl 4-aminopiperidine-1-carboxylate with tert-butyl 3-aminoazetidine-1-carboxylate in **Step 5.**

**Example 6a.** Diastereomer 1. Peak 1. LCMS calculated for C₃₂H₃₀ClFN₅O₃ (M+H)⁺ m/z = 586.2 ; found 586.3.

**Example 6b.** Diastereomer 2. Peak 2. LCMS calculated for C₃₂H₃₀ClFN₅O₃ (M+H)⁺ m/z = 586.2 ; found 586.3.

### Example 7. 1-(3-(8-chloro-6-fluoro-7-(3-hydroxynaphthalen-1-yl)-4-(((S)-1-methylpyrrolidin-2-yl)methoxy)-1H-imidazo[4,5-c]quinolin-1-yl)pyrrolidin-1-yl)prop-2-en-1-one

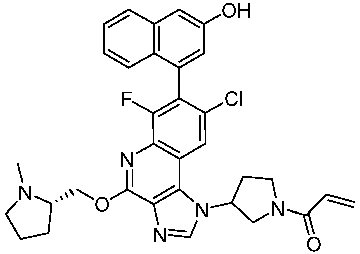

This compound was prepared according to the procedure described in **Example 1,** replacing tert-butyl 4-aminopiperidine-1-carboxylate with tert-butyl 3-aminopyrrolidine-1-carboxylate in **Step 5.** The product was isolated as a mixture of diastereomers. LCMS calculated for C₃₃H₃₂ClFN₅O₃ (M+H)⁺ m/z = 600.2 ; found 600.3.

### Example 8. 1-(4-(8-chloro-6-fluoro-7-(3-hydroxynaphthalen-1-yl)-4-((1-methyl-1H-pyrazol-5-yl)methoxy)-1H-imidazo[4,5-c]quinolin-1-yl)piperidin-1-yl)prop-2-en-1-one

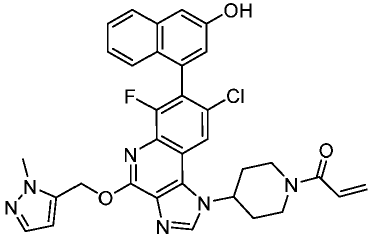

This compound was prepared according to the procedure described in **Example 1,** replacing (S)-(1-methylpyrrolidin-2-yl)methanol with (1-methyl-1H-pyrazol-5-yl)methanol in **Step 6.** The product was isolated as a mixture of atropisomers. LCMS calculated for C₃₃H₂₉ClFN₆O₃ (M+H)⁺ m/z = 611.2; found 611.2.

### Example 9. 1-(4-(8-chloro-4-(3-(dimethylamino)azetidin-1-yl)-6-fluoro-7-(3-hydroxy-naphthalen-1-yl)-1H-imidazo[4,5-c]quinolin-1-yl)piperidin-1-yl)prop-2-en-1-one

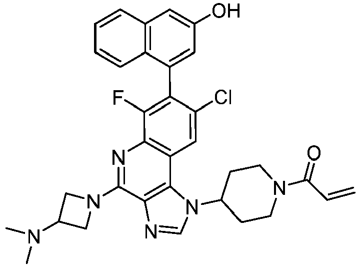

### Step 1. tert-butyl 4-((7-bromo-6-chloro-2-(3-(dimethylamino)azetidin-1-yl)-8-fluoro-3-nitroquinolin-4-yl)amino)piperidine-1-carboxylate

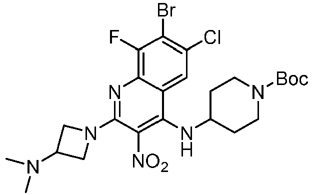

A mixture of *tert-butyl* 4-((7-bromo-2,6-dichloro-8-fluoro-3-nitroquinolin-4-yl)amino)-piperidine-1-carboxylate (0.20 g, 0.19 mmol), *N*,*N*-dimethylazetidin-3-amine (2 HCl salt, 47.0 mg, 0.30 mmol) in 1-butanol (1.5 ml) was stirred at 100 °C for 1 h. The solvent was removed and the residue was purified on silica gel (24 g, 0-15% MeOH in CH₂Cl₂) to provide the desired product. LCMS calculated for C₂₄H₃₂BrClFN₆O₄ (M+H)⁺: m/z = 601.1; found 601.0.

### Step 2. tert-butyl 4-((3-amino-7-bromo-6-chloro-2-(3-(dimethylamino)azetidin-1-yl)-8-fluoroquinolin-4-yl)amino)piperidine-1-carboxylate

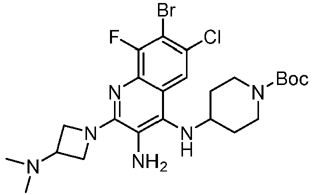

This compound was prepared according to the procedure described in **Example 1 step 7.** LCMS calculated for C₂₄H₃₄BrClFN₃O₂ (M+H)⁺ m/z = 571.2; found 571.2.

### Step 3. tert-butyl 4-(7-bromo-8-chloro-4-(3-(dimethylamino)azetidin-1-yl)-6-fluoro-1H-imidazo[4,5-c]quinolin-1-yl)piperidine-1-carboxylate

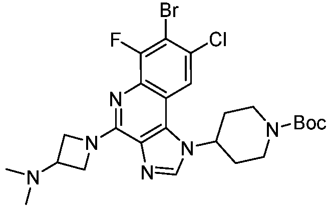

This compound was prepared according to the procedure described in **Example 1 step 8.** LCMS calculated for C₂₅H₃₂BrClFN₆O₂ (M+H)⁺ m/z = 581.1; found 581.1.

### Step 4. 1-(4-(7-bromo-8-chloro-4-(3-(dimethylamino)azetidin-1-yl)-6-fluoro-1H-imidazo[4,5-c]quinolin-1-yl)piperidin-1-yl)prop-2-en-1-one

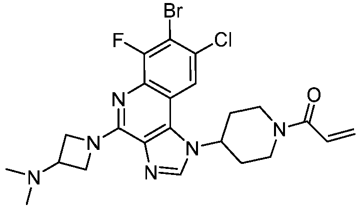

This compound was prepared according to the procedure described in **Example 1 step 9.** LCMS calculated for C₂₃H₂₃BrClFN₆O (M+H)⁺ m/z = 535.1; found 535.2 .

### Step 5. 1-(4-(8-chloro-4-(3-(dimethylamino)azetidin-1-yl)-6-fluoro-7-(3-hydroxynaphthalen-1-yl)-1H-imidazo[4,5-c]quinolin-1-yl)piperidin-1-yl)prop-2-en-1-one

This compound was prepared according to the procedure described in **Example 1 step 10,** replacing (S)-1-(4-(7-bromo-8-chloro-6-fluoro-4-((1-methylpyrrolidin-2-yl)methoxy)-1H-imidazo[4,5-c]quinolin-1-yl)piperidin-1-yl)prop-2-en-1-one with 1-(4-(7-bromo-8-chloro-4-(3-(dimethylamino)azetidin-1-yl)-6-fluoro-1H-imidazo[4,5-c]quinolin-1-yl)piperidin-1-yl)prop-2-en-1-one. The product was isolated as a mixture of atropisomers. LCMS calculated for C₃₃H₃₃ClFN₆O₂ (M+H)⁺ m/z = 599.2; found 599.3.

### Example 10. 1-(1-Acryloylpiperidin-4-yl)-8-chloro-6-fluoro-7-(3-hydroxynaphthalen-1-yl)-4-(((S)-1-methylpyrrolidin-2-yl)methoxy)-1,3-dihydro-2H-imidazo[4,5-c]quinolin-2-one

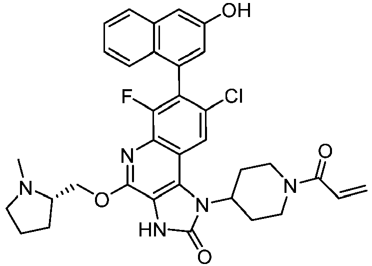

### Step 1. tert-butyl (S)-4-(7-bromo-8-chloro-6-fluoro-4-((1-methylpyrrolidin-2-yl)methoxy)-2-oxo-2,3-dihydro-1H-imidazo[4,5-c]quinolin-1-yl)piperidine-1-carboxylate

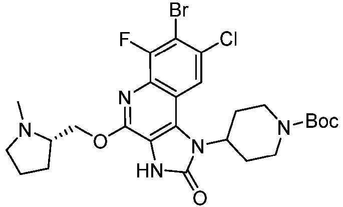

Triphosgene (30.0 mg, 0.1 mmol) was added to a solution of tert-butyl (S)-4-((3-amino-7-bromo-6-chloro-8-fluoro-2-((1-methylpyrrolidin-2-yl)methoxy)quinolin-4-yl)amino)piperidine-1-carboxylate (60.0 mg, 0.1 mmol) and triethylamine (70 uL, 0.4 mmol) in acetrontrile (2.0 mL) and then the reaction was stirred at rt for 2 h. The mixture was diluted with CH₂Cl₂ and washed saturated NaHCO₃, the organic phase was dried and concentrated and the crude was used in the next step directly. LCMS calculated for C₂₆H₃₃BrClFN₅O₄ (M+H)⁺ m/z = 612.1; found 612.2.

### Step 2. (S)-1-(1-acryloylpiperidin-4-yl)-7-bromo-8-chloro-6-fluoro-4-((1-methylpyrrolidin-2-yl)methoxy)-1, 3-dihydro-2H-imidazo[4, 5-c]quinolin-2-one

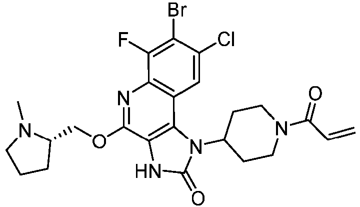

This compound was prepared according to the procedure described in **Example 1 step 9,** replacing tert-butyl (S)-4-(7-bromo-8-chloro-6-fluoro-4-((1-methylpyrrolidin-2-yl)methoxy)-1H-imidazo[4,5-c]quinolin-1-yl)piperidine-1-carboxylate with tert-butyl (S)-4-(7-bromo-8-chloro-6-fluoro-4-((1-methylpyrrolidin-2-yl)methoxy)-2-oxo-2,3-dihydro-1*H-*imidazo[4,5-c]quinolin-1-yl)piperidine-1-carboxylate. LCMS calculated for C₂₄H₂₇BrClFN₅O₃ (M+H)⁺ m/z = 566.1; found 566.1.

### Step 3. 1-(1-acryloylpiperidin-4-yl)-8-chloro-6-fluoro-7-(3-hydroxynaphthalen-1-yl)-4-(((S)-1-methylpyrrolidin-2-yl)methoxy)-1,3-dihydro-2H-imidazo[4,5-c]quinolin-2-one

This compound was prepared according to the procedure described in **Example 1 step 10,** replacing (S)-1-(4-(7-bromo-8-chloro-6-fluoro-4-((1-methylpyrrolidin-2-yl)methoxy)-1*H*-imidazo[4,5-*c*]quinolin-1-yl)piperidin-1-yl)prop-2-en-1-one with (S)-1-(1-acryloylpiperidin-4-yl)-7-bromo-8-chloro-6-fluoro-4-((1-methylpyrrolidin-2-yl)methoxy)-1,3-dihydro-2H-imidazo[4,5-*c*]quinolin-2-one. The product was isolated as a mixture of diastereomers. LCMS calculated for C₃₄H₃₄ClFN₅O₄ (M+H)⁺ m/z = 630.2; found 630.2.

### Example 11. 1-(4-(8-chloro-6-fluoro-4,7-bis(3-hydroxynaphthalen-1-yl)-1H-imidazo[4,5-c]quinolin-1-yl)piperidin-1-yl)prop-2-en-1-one

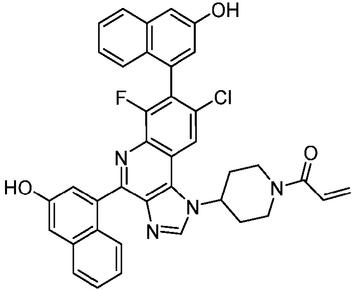

### Step 1. tert-butyl 4-((3-amino-7-bromo-2,6-dichloro-8-fluoroquinolin-4-yl)amino)piperidine-1-carboxylate

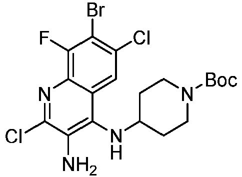

A mixture tert-butyl 4-((7-bromo-2,6-dichloro-8-fluoro-3-nitroquinolin-4-yl)amino)piperidine-1-carboxylate (100.0 mg, 0.186 mmol), iron (50.0 mg, 0.895 mmol) in acetic acid (3.00 ml) was stirred at 60 °C for 1 h. The mixture was diluted with CH₂Cl₂/MeOH and then filtered. The filtrate was concentrated and then dissolved in CH₂Cl₂ and washed with aqueous sodium carbonate. The organic phase was concentrated. The residue was purified on silica gel (12 g, 0-60% EtOAc in CH₂Cl₂) to provide the desired product. LCMS calculated for C₁₉H₂₃BrCl₂FN₄O₂ (M+H)⁺: m/z = 507.0; found 507.1.

### Step 2. tert-butyl 4-(7-bromo-4,8-dichloro-6-fluoro-1H-imidazo[4,5-c]quinolin-1-yl)piperidine-1-carboxylate

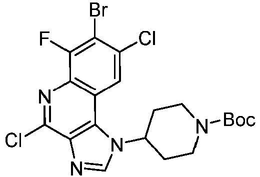

Triethylorthoformate (0.3 mL) was added to a mixture of tert-butyl (S)-4-((3-amino-7-bromo-6-chloro-8-fluoro-2-((1-methylpyrrolidin-2-yl)methoxy)quinolin-4-yl)amino)piperidine-1-carboxylate (0.1 g, 0.20 mmol) in toluene (0.9 mL) and the reaction was stirred at 105 °C for 2 h. To this mixture was added hexane (2.0 mL). The solution was filtered, washed with heaxane, and dried to provide the desired product as a white solid. LCMS calculated for LCMS calculated for C₂₀H₂₁BrCl₂FN₄O₂ (M+H)⁺: m/z = 517.0; found 517.1.

### Step 3. 1-(4-(7-bromo-4,8-dichloro-6-fluoro-1H-imidazo[4,5-c]quinolin-1-yl)piperidin-1-yl)prop-2-en-1-one

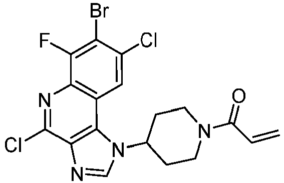

This compound was prepared according to the procedure described in **Example 1 step 9,** replacing tert-butyl (S)-4-(7-bromo-8-chloro-6-fluoro-4-((1-methylpyrrolidin-2-yl)methoxy)-1H-imidazo[4,5-c]quinolin-1-yl)piperidine-1-carboxylate with tert-butyl 4-(7-bromo-4,8-dichloro-6-fluoro-1*H*-imidazo[4,5-*c*]quinolin-1-yl)piperidine-1-carboxylate. LCMS calculated for C₁₈H₁₅BrCl₂FN₄O (M+H)⁺ m/z = 471.0; found 471.1.

### Step 4. 1-(4-(8-chloro-6-fluoro-4,7-bis(3-hydroxynaphthalen-1-yl)-1H-imidazo[4,5-c]quinolin-1-yl) piperidin-1-yl)prop-2-en-1-one

A screw-cap vial equipped with a magnetic stir bar was charged with 1-(4-(7-bromo-4,8-dichloro-6-fluoro-1*H*-imidazo[4,5-c]quinolin-1-yl)piperidin-1-yl)prop-2-en-1-one (25.0 mg, 0.05 mmol), 4-(4,4,5,5-tetramethyl-1,3,2-dioxaborolan-2-yl)naphthalen-2-ol (45.2 mg, 0.162 mmol), sodium carbonate (30.0 mg, 0.28 mmol), Pd(Ph₃P)₄ (6.46 mg, 5.59 µmol) and dioxane (0.8 ml)/water (0.2 ml). The vial was sealed with a Teflon-lined septum and evacuated and backfilled with nitrogen (this process was repeated a total of three times). Then the reaction was stirred at 100 °C for 2 h. The mixture was diluted with acetonitrile/water and purified using prep-LCMS (XBridge C18 column, eluting with a gradient of acetonitrile/water containing 0.1% TFA, at flow rate of 60 mL/min) to afford the desired product as its TFA salt. The product was isolated as a mixture of atropisomers. LC-MS calculated for C₃₈H₂₉ClFN₄O₃ (M+H)⁺: m/z = 643.2; found 643.2.

### Example 12. 1-(4-(8-chloro-6-fluoro-4,7-bis(2-fluoro-6-hydroxyphenyl)-1H-imidazo[4,5-c]-quinolin-1-yl)piperidin-1-yl)prop-2-en-1-one

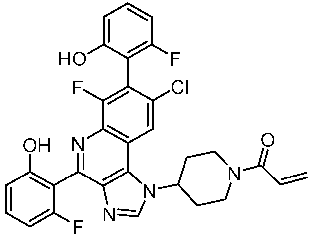

This compound was prepared according to the procedure described in **Example 11 step 4,** replacing 4-(4,4,5,5-tetramethyl-1,3,2-dioxaborolan-2-yl)naphthalen-2-ol with (2-fluoro-6-hydroxyphenyl)boronic acid. The product was isolated as a mixture of atropisomers. LCMS calculated for C₃₀H₂₃ClF₃N₄O₃ (M+H)⁺ m/z = 579.1; found 579.2 .

### Example 13. 1-(4-(8-Chloro-6-fluoro-7-(2-fluoro-6-hydroxyphenyl)-1H-imidazo[4,5-c]-quinolin-1-yl)piperidin-1-yl)prop-2-en-1-one

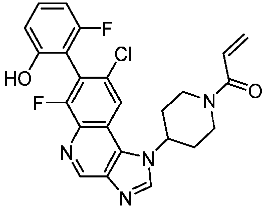

### Step 1. 3-bromo-4-chloro-2-fluoroaniline

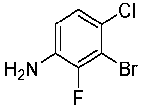

To a solution of 3-bromo-2-fluoroaniline (46.8g, 246 mmol) in DMF (246 ml) was added NCS (34.5 g, 259 mmol) portionwise, and the resultant mixture stirred at room temperature overnight. The mixture was poured onto ice-water (400 mL) and extracted with ethyl acetate. The organic layer was washed with water (2x), brine, dried over Na₂SO₄, filtered and concentrated. The crude was purified with silica gel column (0-30% ethyl acetate in hexanes) to give the desired product as brown oil which solidified on standing (38 g, 69%). LC-MS calculated for C₆H₅BrClFN (M+H)⁺: m/z = 223.9, 225.9; found 223.9, 225.9.

### Step 2. 7-Bromo-6-chloro-8-fluoroquinolin-4(1H)-one

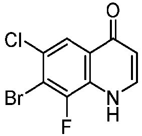

The mixture of 3-bromo-4-chloro-2-fluoroaniline (1.3 g, 5.79 mmol), 5-(methoxymethylene)-2,2-dimethyl-1,3-dioxane-4,6-dione (1.186 g, 6.37 mmol) and 2-propanol (12 ml) was heated at 90 °C for 2 h. The mixture was cooled to room temperature, and the solid formed in the mixture was collected by filtration, and washed with IPA (20 mL) and diethyl ether (20 mL) to give a colorless solid. The solid and DOWTHERM (25 mL) was heated at 220 °C -vigorous evolution of gas. The orange solution was stirred at 220 °C for 40 min then cooled to room temperature. To the mixture was added heptane (25 mL), and the mixture was filtered, with the solid collected, washed with heptane and ethyl ether and dried under vacuum to give the desired product as a tan solid (2.1 g, 96%). LC-MS calculated for C₉H₅BrClFNO (M+H)⁺: m/z = 275.9; found 275.9.

### Step 3. 7-bromo-6-chloro-8-fluoro-3-nitroquinolin-4(1H)-one

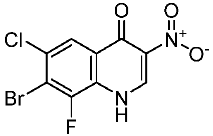

7-bromo-6-chloro-8-fluoroquinolin-4(1H)-one (804 mg, 2.91 mmol) was added to stirred propionic acid (7.86 ml) and the mixture was heated at 125 °C with stirring. Nitric acid (260 µl, 5.82 mmol) was added dropwise, and the solution was stirred for 2 hours at 125 °C before being allowed to cool to room temperature. Water was added, and the mixture was filtered. The solid collected was washed with water and diethyl ether then dried to give the desired product as a pale solid (0.57 g, 61%). LC-MS calculated for C₉H₄BrClFN₂O₃ (M+H)⁺: m/z = 320.9, 322.9; found 320.9, 322.8.

### Step 4. 7-bromo-4,6-dichloro-8-fluoro-3-nitroquinoline

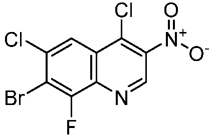

POCl₃ (0.893 ml, 9.58 mmol) was added to 7-bromo-6-chloro-8-fluoro-3-nitroquinolin-4-ol (0.77 g, 2.395 mmol) in toluene (14 ml) at room temperature. The mixture was heated at 110 °C with stirring, at which point DMF (0.1 mL) was added and the mixture was stirred at 110 °C overnight. The solvents were removed by evaporation. Toluene (15 mL) was added and the solvents evaporated. The residue was taken up in DCM (100 mL) and poured into ice-cold sat. NaHCO₃ (150 mL). The mixture was extracted with DCM (3x). The combined organic layers were washed with brine, dried and evaporated to give the desired product as light brown solid (0.80 g, 98%). LC-MS calculated for C₉H₃BrCl₂FN₂O₂ (M+H)⁺: m/z = 338.9, 340.9; found 338.9, 340.9.

### Step 5. tert-butyl 4-((7-bromo-6-chloro-8-fluoro-3-nitroquinolin-4-yl)amino)piperidine-1-carboxylate

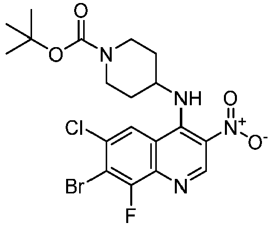

To a solution of 7-bromo-4,6-dichloro-8-fluoro-3-nitroquinoline (78.2 mg, 0.230 mmol) in DMF (1.0 ml) was added tert-butyl 4-aminopiperidine-1-carboxylate (101 mg, 0.506 mmol) and DIEA (100 µl, 0.575 mmol). The resulting mixture was stirred at ambient temperature for 1h. The solvent was diluted with water and the resulting precipitate was collected and washed with hexane, dried under vacuum to give the desired product as brown solid (0.11 g, 95%). LC-MS calculated for C₁₉H₂₂BrClFN₄O₄ (M+H)⁺: m/z = 503.0, 505.0; found 503.0, 505.0.

### Step 6. tert-Butyl 4-((3-amino-7-bromo-6-chloro-8-fluoroquinolin-4-yl)amino)piperidine-1-carboxylate

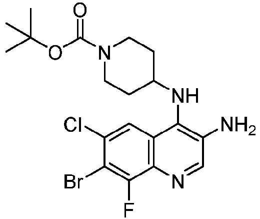

To a solution of tert-butyl 4-((7-bromo-6-chloro-8-fluoro-3-nitroquinolin-4-yl)amino)-piperidine-1-carboxylate (110 mg, 0.218 mmol) in acetic acid (2.0 ml) was added iron (61.0 mg, 1.092 mmol). The resulting mixture was stirred at 80 °C for 1h. LCMS showed completion of reaction. the mixture was filtered through a pad of Celite and washed with methanol. The filtrate was concentrated and purified with silica gel column (80 mg, 77%). LC-MS calculated for C₁₉H₂₄BrClFN₄O₂ (M+H)⁺: m/z = 473.1, 475.1; found 473.1, 475.1.

### Step 7. tert-butyl 4-(7-bromo-8-chloro-6-fluoro-1H-imidazo[4,5-c]quinolin-1-yl)piperidine-1-carboxylate

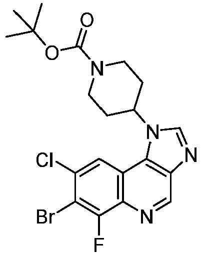

The mixture of tert-butyl 4-((3-amino-7-bromo-6-chloro-8-fluoroquinolin-4-yl)amino)piperidine-1-carboxylate (80 mg, 0.169 mmol), triethyl orthoformate (56.2 µl, 0.338 mmol) and ethanol (1.0 ml) was stirred at 80 °C for 1 h. The mixture was concentrated and purified with silica gel column (80 mg, 98%). LC-MS calculated for C₂₀H₂₂BrClFN₄O₂ (M+H)⁺: m/z = 483.1, 485.1; found 483.1, 485.1.

### Step 8. tert-butyl 4-(8-chloro-6-fluoro-7-(2-fluoro-6-hydroxyphenyl)-1H-imidazo[4,5-c]quinolin-1-yl)piperidine-1-carboxylate

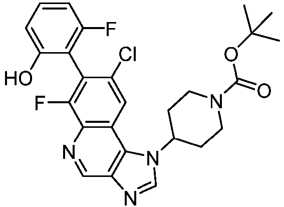

A mixture of *tert-butyl* 4-(7-bromo-8-chloro-6-fluoro-1H-imidazo[4,5-c]quinolin-1-yl)piperidine-1-carboxylate (80 mg, 0.165 mmol), (2-fluoro-6-hydroxyphenyl)boronic acid (56.7 mg, 0.364 mmol), dicyclohexyl(2',4',6'-triisopropylbiphenyl-2-yl)phosphine - (2'-aminobiphenyl-2-yl)(chloro)palladium (1:1) (13.01 mg, 0.017 mmol) and tripotassium phosphate hydrate (84 mg, 0.364 mmol) in 1,4-dioxane (3.0mL)/Water (0.600 mL) was stirred at 80 °C for 1 h. The residue was dissolved in methanol and 1 N HCl and purified with prep-LCMS (pH 2, acetonitrile/ water+TFA) to give the desired product (69 mg, 81%). LC-MS calculated for C₂₆H₂₆ClF₂N₄O₃ (M+H)⁺: m/z = 515.2; found 515.2.

### Step 9. 1-(4-(8-chloro-6-fluoro-7-(2-fluoro-6-hydroxyphenyl)-1H-imidazo[4,5-c]quinolin-1-yl)piperidin-1-yl)prop-2-en-1-one

To a reaction mixture of tert-butyl 4-(8-chloro-6-fluoro-7-(2-fluoro-6-hydroxyphenyl)-1H-imidazo[4,5-c]quinolin-1-yl)piperidine-1-carboxylate (69 mg, 0.134 mmol) and DCM (1.0 mL) was added TFA (413 µl, 5.36 mmol). After stirring for 1 hour at room temperature, the volatiles were removed under reduced pressure. The residue was dissolved in DCM (1.0 mL) and 1.0 M acryloyl chloride in DCM (134 µl, 0.134 mmol) was added, followed by DIEA (117 µl, 0.670 mmol) at 0 °C. After stirring at same temperature for 0.5 h, the reaction mixture was concentrated *in vacuo.* The residue was dissolved in methanol and 1 N HCl and purified with prep-LCMS (pH 2, acetonitrile/water+TFA) to give the desired product (10 mg, 16%). The product was isolated as a mixture of atropisomers. LC-MS calculated for C₂₄H₂₀ClF₂N₄O₂ (M+H)⁺: m/z = 469.1; found 469.1.

### Example 14a and Example 14b. 1-(4-(8-Chloro-6-fluoro-7-(3-hydroxynaphthalen-1-yl)-4-(((S)-1-methylpyrrolidin-2-yl)methoxy)-1H-[1,2,3]triazolo[4,5-c]quinolin-1-yl)piperidin-1-yl)prop-2-en-1-one

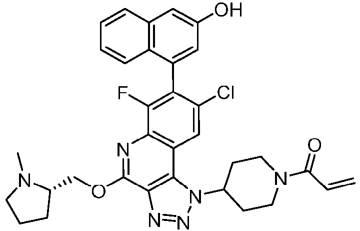

### Step 1. tert-Butyl (S)-4-(7-bromo-8-chloro-6-fluoro-4-((1-methylpyrrolidin-2-yl)methoxy)-1H-[1,2, 3]triazolo[4,5-c]quinolin-1-yl)piperidine-1-carboxylate

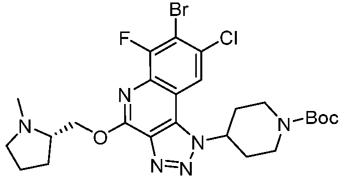

Sodium nitrite (11.7 mg, 0.17 mmol) was added to a mixture of tert-butyl (S)-4-((3-amino-7-bromo-6-chloro-8-fluoro-2-((1-methylpyrrolidin-2-yl)methoxy)quinolin-4-yl)amino)piperidine-1-carboxylate (50.0 mg, 0.085 mmol) in acetic acid (1.0 mL) and the reaction was stirred at rt for 2 h. The reaction mixture was then diluted with CH₂Cl₂ and filtered. The filtrate was concentrated and the resulting material was used directly in the next step. LCMS calculated for C₂₅H₃₂BrClFN₆O₃ (M+H)⁺ m/z = 597.1; found 597.1.

### Step 2. (S)-1-(4-(7-Bromo-8-chloro-6-fluoro-4-((1-methylpyrrolidin-2-yl)methoxy)-1H-[1,2,3]triazolo[4,5-c]quinolin-1-yl)piperidin-1-yl)prop-2-en-1-one

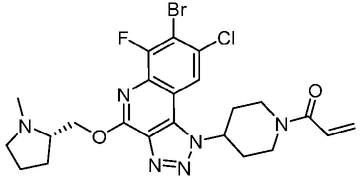

This compound was prepared according to the procedure described in **Example 1 step 9,** replacing tert-butyl (S)-4-(7-bromo-8-chloro-6-fluoro-4-((1-methylpyrrolidin-2-yl)methoxy)-1H-imidazo[4,5-c]quinolin-1-yl)piperidine-1-carboxylate with tert-butyl (S)-4-(7-bromo-8-chloro-6-fluoro-4-((1-methylpyrrolidin-2-yl)methoxy)-1H-[1,2,3]triazolo[4,5-c]quinolin-1-yl)piperidine-1-carboxylate. LCMS calculated for C₂₃H₂₆BrClFN₆O₂ (M+H)⁺ m/z = 551.1; found 551.0.

### Step 3. 1-(4-(8-Chloro-6-fluoro-7-(3-hydroxynaphthalen-1-yl)-4-(((S)-1-methylpyrrolidin-2-yl)methoxy)-1H-[1,2,3]triazolo[4,5-c]quinolin-1-yl)piperidin-1-yl)prop-2-en-1-one

This compound was prepared according to the procedure described in **Example 1 step 10,** replacing (S)-1-(4-(7-bromo-8-chloro-6-fluoro-4-((1-methylpyrrolidin-2-yl)methoxy)-1*H*-imidazo[4,5-*c*]quinolin-1-yl)piperidin-1-yl)prop-2-en-1-one with (S)-1-(4-(7-bromo-8-chloro-6-fluoro-4-((1-methylpyrrolidin-2-yl)methoxy)-1*H*-[1,2,3]triazolo[4,5-*c*]quinolin-1-yl)piperidin-1-yl)prop-2-en-1-one.

**Example 14a.** Diastereomer 1. Peak 1. LCMS calculated for C₃₃H₃₃ClFN₆O₃ (M+H)⁺ m/z = 615.2; found 615.2. ¹H-NMR (500MHz in DMSO-*d*₆) δ 10.09 (s, 1H), 9.95 (s, 1H), 8.63 (s, 1H), 7.85 (d, *J* = 8.3 Hz, 1H), 7.49 - 7.44 (m, 1H), 7.33 (d, *J* = 2.1 Hz, 1H), 7.28 - 7.22 (m, 1H), 7.18 (d, *J* = 8.3 Hz, 1H), 7.13 (d, *J* = 2.2 Hz, 1H), 6.93 (dd, *J* = 16.7, 10.5 Hz, 1H), 6.19 (dd, *J* = 16.7, 2.3 Hz, 1H), 5.84 (dt, *J* = 10.8, 6.7 Hz, 1H), 5.75 (dd, *J* = 10.4, 2.3 Hz, 1H), 5.11 = 5.04 (m, 1H), 4.93 - 4.80 (m, 1H), 4.58 (d, *J* = 9.9 Hz, 1H), 4.28 (d, *J* = 10.8 Hz, 1H), 4.02 (s, 1H), 3.72 - 3.56 (m, 2H), 3.29 - 3.23 (m, 1H), 3.21 - 3.13 (m, 1H), 3.06 (s, 3H), 2.46 - 2.38 (m, 2H), 2.37 - 2.30 (m, 1H), 2.28 - 2.20 (m, 1H), 2.20 - 2.06 (m, 2H), 2.04 - 1.96 (m, 2H).

**Example 14b.** Diastereomer 2. Peak 2. LCMS calculated for C₃₃H₃₃ClFN₆O₃ (M+H)⁺ m/z = 615.2; found 615.2.

### Example 15. 4-(1-((endo)-2-azabicyclo[2.1.1]hexan-5-yl)-8-chloro-4-(3-(dimethylamino)-azetidin-1-yl)-6-fluoro-2-methyl-1H-imidazo[4,5-c]quinolin-7-yl)naphthalen-2-ol

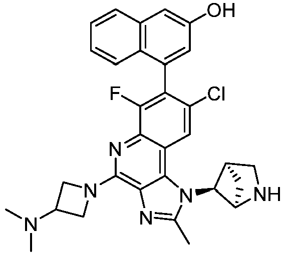

### Step 1. tert-butyl (endo)-5-((7-bromo-6-chloro-2-(3-(dimethylamino)azetidin-1-yl)-8-fluoro-3-nitroquinolin-4-yl)amino) -2-azabicyclo[2. 1.1]hexane-2-carboxylate

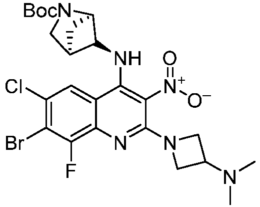

DIPEA (4.62 ml, 26.4 mmol) was added to a solution of 7-bromo-2,4,6-trichloro-8-fluoro-3-nitroquinoline **(Example 1, step 4,** 1.8 g, 4.81 mmol) and tert-butyl (endo)-5-amino-2-azabicyclo[2.1.1]hexane-2-carboxylate (1.0 g, 5.0 mmol) in CH₂Cl₂ (20.0 ml) and then the reaction was stirred at 50 °C for 4 h. Then *N*,*N*-dimethylazetidin-3-amine dihydrochloride (1.2 g, 7.2 mmol) was added. After heating at 60 °C for another 4 h, the mixture was concentrated to dryness and used in the next step without further purification. LCMS calculated for C₂₄H₃₀BrClFN₆O₄ (M+H)⁺: m/z = 599.1; found: 599.1.

### Step 2. tert-butyl (endo)-5-((3-amino-7-bromo-6-chloro-2-(3-(dimethylamino)azetidin-1-yl)-8-fluoroquinolin-4-yl)amino)-2-azabicyclo[2.1.1]hexane-2-carboxylate

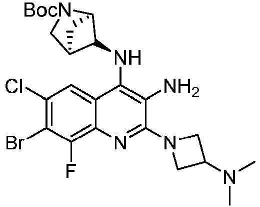

Sodium hydrosulfite (3.01 g, 17.31 mmol) in water (0.5 mL) was added to a solution of tert-butyl (endo)-5-((7-bromo-6-chloro-2-(3-(dimethylamino)azetidin-1-yl)-8-fluoro-3-nitroquinolin-4-yl)amino)-2-azabicyclo[2.1.1]hexane-2-carboxylate (2.3 g, 3.84 mmol) and 30% aq. ammonium hydroxide (520 µL, 4.01 mmol) in MeOH (3 mL) at 0 °C. After 10 min, water was added to the reaction mixture followed by extraction with CH₂Cl₂. The combined organic layers were dried over Na₂SO₄, filtered, concentrated, and used in the next step without further purification. LCMS calculated for C₂₄H₃₂BrClFN₆O₂ (M+H)⁺: m/z = 569.1; found: 569.1.

### Step 3. 4-(1-((endo)-2-azabicyclo[2.1.1]hexan-5-yl)-8-chloro-4-(3-(dimethylamino)azetidin-1-yl)-6-fluoro-2-methyl-1H-imidazo[4,5-c]quinolin-7-yl)naphthalen-2-ol

tert-butyl (endo)-5-((3-amino-7-bromo-6-chloro-2-(3-(dimethylamino)azetidin-1-yl)-8-fluoroquinolin-4-yl)amino)-2-azabicyclo[2.1.1]hexane-2-carboxylate (40 mg, 0.070 mmol) was dissolved in triethyl orthoacetate (12.94 µl, 0.070 mmol) and heated to 100 °C for 2 hr. A few drops of acetic acid were then added and stirring was continued at 100 °C overnight. The mixture was concentrated to dryness and used in the next step without further purification.

To the crude mixture was added 4-(4,4,5,5-tetramethyl-1,3,2-dioxaborolan-2-yl)naphthalen-2-ol (24.65 mg, 0.091 mmol), Pd(Ph₃P)₄ (8.11 mg, 7.02 µmol), sodium carbonate (14.88 mg, 0.140 mmol), followed by dioxane (0.8 mL)/water (0.4 mL). The reaction flask was evacuated, back filled with nitrogen, and then stirred at 100 °C overnight. HCl (4 N in dioxane, 1 mL) was added and the mixture was stirred at 60 °C for 30 min. The mixture was then diluted with MeOH/water/TFA and purified by prep-LCMS (XBridge C18 column, eluting with a gradient of acetonitrile/water containing 0.1% TFA, at flow rate of 60 mL/min) to give the desired product as a TFA salt. The product was isolated as a mixture of diastereomers. LCMS calculated for C₃₁H₃₁ClFN₆O (M+H)⁺: m/z = 557.2; found 557.2.

### Example 16. 2-((2S,4S)-1-acryloyl-4-(8-chloro-4-(3-(dimethylamino)azetidin-1-yl)-7-(2,3-dimethylphenyl)-6-fluoro-1H-imidazo[4,5-c]quinolin-1-yl)piperidin-2-yl)acetonitrile

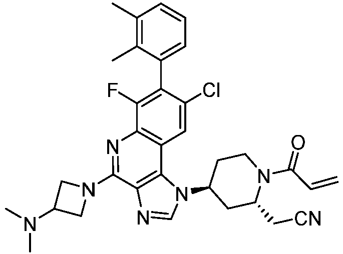

### Step 1. 1-(tert-butyl) 2-methyl (2S,4S)-4-((7-bromo-6-chloro-2-(3-(dimethylamino)azetidin-1-yl)-8-fluoro-3-nitroquinolin-4-yl)amino)piperidine-1,2-dicarboxylate

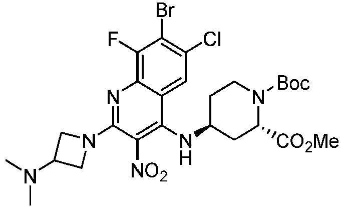

DIPEA (2.93 ml, 16.8 mmol) was added to a solution of 7-bromo-2,4,6-trichloro-8-fluoro-3-nitroquinoline **(Example 1, step 4,** 630 mg, 1.68 mmol) and 1-(tert-butyl) 2-methyl (2S,4S)-4-aminopiperidine-1,2-dicarboxylate (478 mg, 1.85 mmol) in CH₂Cl₂ (10.0 ml). and then the reaction was stirred at room temperature overnight. After completion, N,N-dimethylazetidin-3-amine dihydrochloride (378 mg, 2.1 mmol) was added at r.t. the reaction mixture was stirred at r.t. for 20 h. The mixture was concentrated to dryness and purified on silica gel (25 g, 0-40% EtOAc in CH₂Cl₂) to yield a white solid (705 mg, 70% over two steps). LCMS calculated for C₂₆H₃₄BrClFN₆O₆ (M+H)⁺: m/z = 659.1; found: 659.2.

### Step 2. 1-(tert-butyl) 2-methyl (2S,4S)-4-(7-bromo-8-chloro-4-(3-(dimethylamino)azetidin-1-yl)-6-fluoro-1H-imidazo[4,5-c]quinolin-1-yl)piperidine-1,2-dicarboxylate

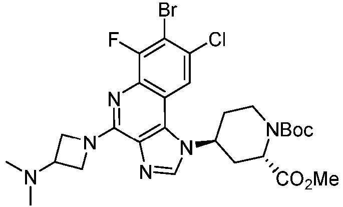

A mixture of *tert-butyl* (S)-4-((7-bromo-6-chloro-8-fluoro-2-((1-methylpyrrolidin-2-yl)-methoxy)-3-nitroquinolin-4-yl)amino)piperidine-1-carboxylate (300.0 mg, 0.455 mmol), iron (254.0 mg, 4.55 mmol), and ammonium chloride (243 mg, 4.55 mmol) in 15 mL MeOH (5 mL)/THF (5 mL)/water (5 mL) was stirred at 70 °C for 15 min. The mixture was diluted with THF/MeOH and then filtered over a pad of celite. The filtrate was concentrated and then dissolved in CH₂Cl₂ and washed with sat'd sodium carbonate. The organic layer was concentrated to dryness and used in the next step without further purification.

The crude mixture was dissolved in toluene (0.3 mL) and Triethylorthoformate (0.3 mL) was added, the mixture was stirred at 100 °C for 1 hour until its completion. The solvent was removed and the residue was purified by silica gel column (12 g, 0-100% EtOAc in CH₂Cl₂) to yield a white solid (230 mg, 80% over two steps). LCMS calculated for C₂₇H₃₄BrClFN₆O₄ (M+H)⁺: m/z = 639.1; found: 639.1.

### Step 3. tert-butyl (2S,4S)-4-(7-bromo-8-chloro-4-(3-(dimethylamino)azetidin-1-yl)-6-fluoro-1H-imidazo[4,5-c]quinolin-1-yl)-2-(hydroxymethyl)piperidine-1-carboxylate

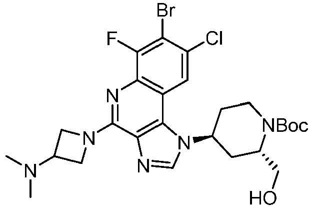

To a solution of 1-(tert-butyl) 2-methyl (2S,4S)-4-(7-bromo-8-chloro-4-(3-(dimethyl-amino)azetidin-1-yl)-6-fluoro-1H-imidazo[4,5-c]quinolin-1-yl)piperidine-1,2-dicarboxylate (100 mg, 0.16 mmol) in THF (2.0 mL) was added DIBAL-H (1 N in toluene, 0.8 mL, 0.8 mmol) at - 30 °C. The reaction was allowed to warm up to -10 °C over 1 hour before quenching with sat'd ammonium chloride. The mixture was extracted with CH₂Cl₂ and the combined organic layers were dried over Na₂SO₄, filtered, concentrated, and purified on silica gel (12 g, 0-100% (3:1 EtOAc:MeOH) in CH₂Cl₂) to yield the desired product (58 mg, 60%). LCMS calculated for C₂₃H₃₄BrClFN₆O₃ (M+H)⁺: m/z = 611.1; found: 611.1.

### Step 4. tert-butyl (2S,4S)-4-(7-bromo-8-chloro-4-(3-(dimethylamino)azetidin-1-yl)-6-fluoro-1H-imidazo[4,5-c]quinolin-1-yl)-2-(cyanomethyl)piperidine-1-carboxylate

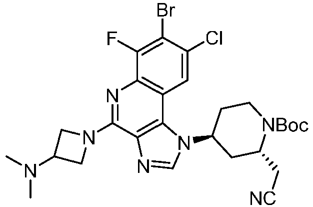

To a solution of tert-butyl (2S,4S)-4-(7-bromo-8-chloro-4-(3-(dimethylamino)azetidin-1-yl)-6-fluoro-1*H*-imidazo[4,5-*c*]quinolin-1-yl)-2-(hydroxymethyl)piperidine-1-carboxylate (58 mg, 0.095 mmol) in CH₂Cl₂ (2.0 mL) and trimethylamine (0.033 mL, 0.24 mmol) was added Ms-Cl (0.011 mL, 0.14 mmol) at 0 °C. The mixture was allowed to warm to r.t. and stirred for 1 h, then the reaction was diluted with CH₂Cl₂ and washed with sat'd NaHCO₃. The combined organic layers were dried over Na₂SO₄ and concentrated to dryness.

To the crude residue was added sodium cyanide (49 mg, 1 mmol) followed by DMSO (1 mL). The reaction was sealed and heated to 65 °C for 24 h, then diluted with CH₂Cl₂ and washed with sat'd NaHCO₃ and brine. The combined organic layers were dried over Na₂SO₄, filtered, concentrated, and purified on silica gel (12 g, 0-100% (3:1 EtOAc:MeOH) in CH₂Cl₂) to yield the desired product (15 mg, 25%). LCMS calculated for C₂₇H₃₃BrClFN₇O₂ (M+H)⁺: m/z = 620.2; found: 620.2.

### Step 5. 2-((2S,4S)-1-acryloyl-4-(7-bromo-8-chloro-4-(3-(dimethylamino)azetidin-1-yl)-6-fluoro-1H-imidazo[4,5-c]quinolin-1-yl)piperidin-2-yl)acetonitrile

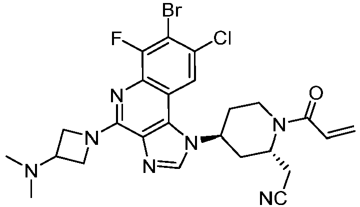

To a solution of tert-butyl (2S,4S)-4-(7-bromo-8-chloro-4-(3-(dimethylamino)azetidin-1-yl)-6-fluoro-1*H*-imidazo[4,5-*c*]quinolin-1-yl)-2-(cyanomethyl)piperidine-1-carboxylate (15 mg, 0.024 mmol) in CH₂Cl₂ (2.0 mL) was added TFA (0.5 mL). The mixture was stirred at r.t. for 30 min then concentrated to dryness. To the residue was added CH₂Cl₂ (2.0 mL) and an excess amount of trimethylamine (0.5 mL). The solution was coold to 0 °C and acryloyl chloride (10 mg, 0.11 mmol) was added. After stirring for 15 min, the mixture was diluted with MeOH/water and purified by prep-LCMS (XBridge C18 column, eluting with a gradient of acetonitrile/water containing 0.1% TFA, at flow rate of 60 mL/min) to give the desired product as a TFA salt. LCMS calculated for C₂₅H₂₇ClFN₇O (M+H)⁺: m/z = 574.1; found 574.1.

### Step 6. 2-((2S,4S)-1-acryloyl-4-(8-chloro-4-(3-(dimethylamino)azetidin-1-yl)-7-(2,3-dimethylphenyl)-6-fluoro-1H-imidazo[4,5-c]quinolin-1-yl)piperidin-2-yl)acetonitrile

A screw-cap vial equipped with a magnetic stir bar was charged 2-((2S,4S)-1-acryloyl-4-(7-bromo-8-chloro-4-(3-(dimethylamino)azetidin-1-yl)-6-fluoro-1H-imidazo[4,5-c]quinolin-1-yl)piperidin-2-yl)acetonitrile (14.0 mg, 0.025 mmol), (2,3-dimethylphenyl)boronic acid (7.5 mg, 0.05 mmol), sodium carbonate (10.0 mg, 0.09 mmol), and Pd(Ph₃P)₄ (6.46 mg, 5.59 µmol) followed by dioxane (0.8 ml)/water (0.2 ml). The vial was sealed with a Teflon-lined septum, evacuated and backfilled with nitrogen (this process was repeated a total of three times). Then the reaction was stirred at 105 °C for 2 h. The mixture was diluted with acetonitrile/water and purified using prep-LCMS (XBridge C18 column, eluting with a gradient of acetonitrile/water containing 0.1% TFA, at flow rate of 60 mL/min) to afford the desired products as a TFA salt. The product was isolated as a mixture of diastereomers. LCMS calculated for C₃₃H₃₆ClFN₇O (M+H)⁺ m/z = 600.2; found 600.2.

### Example 17. 3-(1-((2S,4S)-1-acryloyl-2-(cyanomethyl)piperidin-4-yl)-8-chloro-4-(3-(dimethylamino)azetidin-1-yl)-6-fluoro-1H-imidazo[4,5-c]quinolin-7-yl)-2-methylbenzonitrile

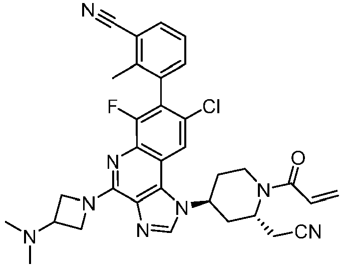

This compound was prepared according to the procedure described in **Example 16,** replacing (2,3-dimethylphenyl)boronic acid with (3-cyano-2-methylphenyl)boronic acid in **Step 6.** The product was isolated as a mixture of diastereomers. LCMS calculated for C₃₃H₃₃ClFN₈O (M+H)⁺ m/z = 611.2; found 611.2.

### Example 18. 2-((2S,4S)-1-acryloyl-4-(8-chloro-4-(3-(dimethylamino)azetidin-1-yl)-6-fluoro-7-(3-fluoro-2-methylphenyl)-1H-imidazo[4,5-c]quinolin-1-yl)piperidin-2-yl)acetonitrile

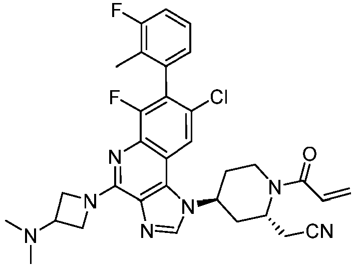

This compound was prepared according to the procedure described in **Example 16,** replacing (2,3-dimethylphenyl)boronic acid with (3-fluoro-2-methylphenyl)boronic acid in **Step 6.** The product was isolated as a mixture of diastereomers. LCMS calculated for C₃₂H₃₃ClF₂N₇O (M+H)⁺ m/z = 604.2; found 604.3.

### Example 19. 2-((2S,4S)-1-acryloyl-4-(8-chloro-7-(2-chloro-3-methylphenyl)-4-(3-(dimethylamino)azetidin-1-yl)-6-fluoro-1H-imidazo[4,5-c]quinolin-1-yl)piperidin-2-yl)acetonitrile

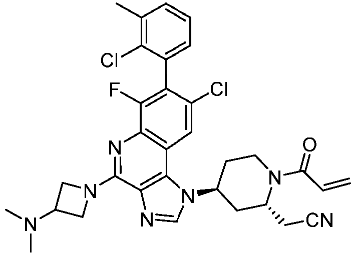

This compound was prepared according to the procedure described in **Example 16,** replacing (2,3-dimethylphenyl)boronic acid with (2-chloro-3-methylphenyl)boronic acid in **Step 6.** The product was isolated as a mixture of diastereomers. LCMS calculated for C₃₂H₃₃Cl₂FN₇O (M+H)⁺ m/z = 620.2; found 620.2.

### Example 20. 2-((2S,4S)-1-acryloyl-4-(8-chloro-4-(3-(dimethylamino)azetidin-1-yl)-6-fluoro-7-(o-tolyl)-1H-imidazo[4,5-c]quinolin-1-yl)piperidin-2-yl)acetonitrile

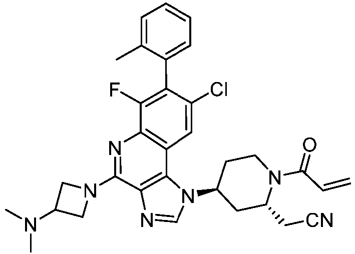

This compound was prepared according to the procedure described in **Example 16,** replacing (2,3-dimethylphenyl)boronic acid with o-tolylboronic acid in **Step 6.** The product was isolated as a mixture of diastereomers. LCMS calculated for C₃₂H₃₄ClFN₇O (M+H)⁺ m/z = 586.2; found 586.2.

### Example 21a and 21b. (2S,4S)-1-acryloyl-4-(8-chloro-4-(3-(dimethylamino)azetidin-1-yl)-6-fluoro-7-(3-hydroxynaphthalen-1-yl)-1H-imidazo[4,5-c]quinolin-1-yl)piperidine-2-carboxamide

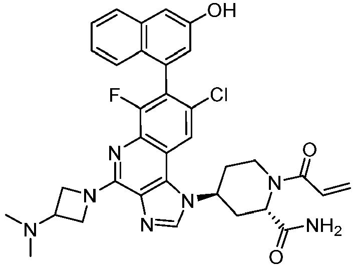

### Step 1. (2S,4S)-4-(7-bromo-8-chloro-4-(3-(dimethylamino)azetidin-1-yl)-6-fluoro-1H-imidazo[4,5-c]quinolin-1-yl)-1-(tert-butoxycarbonyl)piperidine-2-carboxylic acid

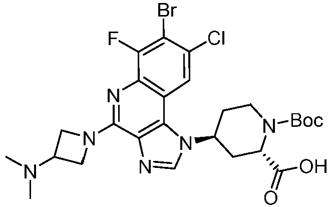

LiOH hydrate (41 mg, 1.0 mmol) was added to a solution of 1-(tert-butyl) 2-methyl (2*S*,4*S*)-4-(7-bromo-8-chloro-4-(3-(dimethylamino)azetidin-1-yl)-6-fluoro-1*H*-imidazo[4,5-c]quinolin-1-yl)piperidine-1,2-dicarboxylate **(Example 16, step 2, 0.13** g, 0.02 mmol) in MeOH (1.0 mL)/THF (1.0 mL)/water (0.5 mL). The reaction was stirred at r.t. for 2 h. HCl (1.0 N, 1.5 mL) was added to the reaction mixture. The reaction was then concentrated to dryness and used in the next step without further purification. LCMS calculated for C₂₆H₃₂BrClFN₆O₄ (M+H)⁺: m/z = 625.1; found: 625.1.

### Step 2. tert-butyl (2S,4S)-4-(7-bromo-8-chloro-4-(3-(dimethylamino)azetidin-1-yl)-6-fluoro-1H-imidazo[4,5-c]quinolin-1-yl)-2-carbamoylpiperidine-1-carboxylate

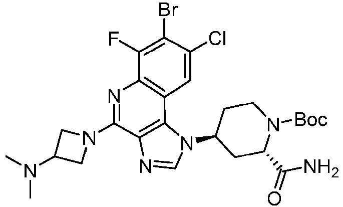

To a solution of (2S,4S)-4-(7-bromo-8-chloro-4-(3-(dimethylamino)azetidin-1-yl)-6-fluoro-1H-imidazo[4,5-c]quinolin-1-yl)-1-(tert-butoxycarbonyl)-piperidine-2-carboxylic acid (0.12 g, 0.2 mmol), (NH₄)₂CO₃ (0.2 g, 2 mmol), and BOP (133 mg, 0.3 mmol) in DMF (2.0 mL) was added triethylamine (0.34 ml, 2 mmol). The reaction was stirred at r.t. for 2 h, then diluted with EtOAc, washed with sat'd NaHCO₃, water and brine. dried over Na₂SO₄, and filtered. The combined organic layers were dried over Na₂SO₄, filtered, concentrated, and purified on silica gel (12 g, 0-15% MeOH in CH₂Cl₂) to provide the desired product. LCMS calculated for C₂₆H₃₃BrClFN₇O₃ (M+H)⁺: m/z = 624.1; found: 624.2.

### Step 3. (2S,4S)-1-acryloyl-4-(7-bromo-8-chloro-4-(3-(dimethylamino)azetidin-1-yl)-6-fluoro-1H-imidazo[4,5-c]quinolin-1-yl)piperidine-2-carboxamide

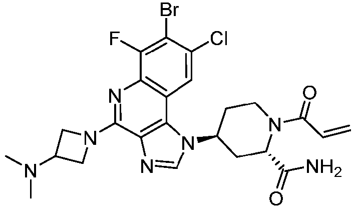

This compound was prepared according to the procedure described in **Example 16,** Step 5, replacing tert-butyl (2S,4S)-4-(7-bromo-8-chloro-4-(3-(dimethylamino)azetidin-1-yl)-6-fluoro-1H-imidazo[4,5-c]quinolin-1-yl)-2-(cyanomethyl)piperidine-1-carboxylate with *tert-*butyl (2*S*,4*S*)-4-(7-bromo-8-chloro-4-(3-(dimethylamino)azetidin-1-yl)-6-fluoro-1*H-*imidazo[4,5-c]quinolin-1-yl)-2-carbamoylpiperidine-1-carboxylate. LCMS calculated for C₂₄H₂₇BrClFN₇O₂ (M+H)⁺: m/z = 578.1.1; found: 578.2.

### Step 4. (2S,4S)-1-acryloyl-4-(8-chloro-4-(3-(dimethylamino)azetidin-1-yl)-6-fluoro-7-(3-hydroxynaphthalen-1-yl)-1H-imidazo[4,5-c]quinolin-1-yl)piperidine-2-carboxamide

This compound was prepared according to the procedure described in **Example 1, step 10,** replacing (S)-1-(4-(7-bromo-8-chloro-6-fluoro-4-((1-methylpyrrolidin-2-yl)methoxy)-1*H*-imidazo[4,5-*c*]quinolin-1-yl)piperidin-1-yl)prop-2-en-1-one with (2S,4S)-1-acryloyl-4-(7-bromo-8-chloro-4-(3-(dimethylamino)azetidin-1-yl)-6-fluoro-1*H*-imidazo[4,5-*c*]quinolin-1-yl)piperidine-2-carboxamide.

**Example 21a.** Diastereomer 1. Peak 1. LCMS calculated for C₃₄H₃₄ClFN₇O₃ (M+H)⁺ m/z = 642.2; found 642.3.

**Example 21b.** Diastereomer 2. Peak 2. LCMS calculated for C₃₄H₃₄ClFN₇O₃ (M+H)⁺ m/z = 642.2; found 642.3.

### Example 22a and 22b (2S,4S)-1-acryloyl-4-(8-chloro-4-(3-(dimethylamino)azetidin-1-yl)-6-fluoro-7-(3-hydroxynaphthalen-1-yl)-1H-imidazo[4,5-c]quinolin-1-yl)piperidine-2-carbonitrile

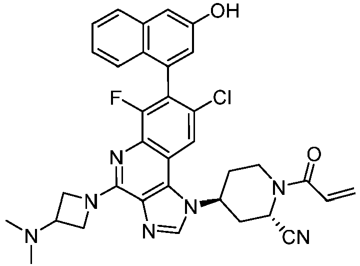

### Step 1. (2S,4S)-1-acryloyl-4-(7-bromo-8-chloro-4-(3-(dimethylamino)azetidin-1-yl)-6-fluoro-1H-imidazo[4, 5-c]quinolin-1-yl)piperidine-2-carbonitrile

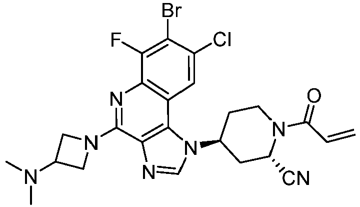

To a solution of (2S,4S)-1-acryloyl-4-(7-bromo-8-chloro-4-(3-(dimethylamino)azetidin-1-yl)-6-fluoro-1H-imidazo[4,5-c]quinolin-1-yl)piperidine-2-carboxamide (Example 22, step 3, 58 mg, 0.1 mmol) and trimethylamine (56 ul, 0.4 mmol) in THF (1.0 mL) at 0 °C was added trifluoroacetic anhydride (28 uL, 0.2 mmol). The mixture was stirred at 0 °C for 1 h, then quenched with the addition of sat'd NaHCO₃ and extracted with CH₂Cl₂. The combined organic layers were dried over Na₂SO₄, filtered, concentrated, and used directly in the next step without further purification. LCMS calculated for C₂₄H₂₅BrClFN₇O (M+H)⁺: m/z = 560.1; found: 560.1.

### Step 2. (2S,4S)-1-acryloyl-4-(8-chloro-4-(3-(dimethylamino)azetidin-1-yl)-6-fluoro-7-(3-hydroxy-naphthalen-1-yl)-1H-imidazo[4,5-c]quinolin-1-yl)piperidine-2-carbonitrile

This compound was prepared according to the procedure described in **Example 1, step 10,** replacing (S)-1-(4-(7-bromo-8-chloro-6-fluoro-4-((1-methylpyrrolidin-2-yl)methoxy)-1*H*-imidazo[4,5-*c*]quinolin-1-yl)piperidin-1-yl)prop-2-en-1-one with (2S,4S)-1-acryloyl-4-(7-bromo-8-chloro-4-(3-(dimethylamino)azetidin-1-yl)-6-fluoro-1*H*-imidazo[4,5-*c*]quinolin-1-yl)piperidine-2-carbonitrile.

**Example 22a.** Diastereomer 1. Peak 1. LCMS calculated for C₃₄H₃₂ClFN₇O₂ (M+H)⁺ m/z = 624.2; found 624.2.

**Example 22b.** Diastereomer 2. Peak 2. LCMS calculated for C₃₄H₃₂ClFN₇O₂ (M+H)⁺ m/z = 624.2; found 624.2.

### Example 23a and Example 23b. 4-(1-((endo)-2-azabicyclo[2.1.1]hexan-5-yl)-8-chloro-4-(3-(dimethylamino)azetidin-1-yl)-6-fluoro-1H-imidazo[4,5-c]quinolin-7-yl)-6-chloronaphthalen-2-ol

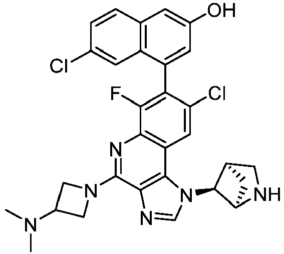

### Step 1. tert-butyl (endo)-5-(7-bromo-8-chloro-4-(3-(dimethylamino)azetidin-1-yl)-6-fluoro-1H-imidazo[4,5-c]quinolin-1-yl)-2-azabicyclo[2.1.1]hexane-2-carboxylate

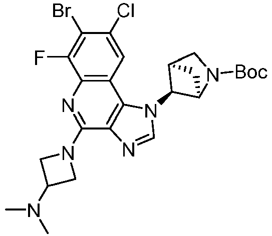

To a solution of tert-butyl (endo)-5-((3-amino-7-bromo-6-chloro-2-(3-(dimethylamino)-azetidin-1-yl)-8-fluoroquinolin-4-yl)amino)-2-azabicyclo[2.1.1]hexane-2-carboxylate **(Example 15, step 2,** 2.19 g, 3.84 mmol) in toluene (3 mL) was added triethyl orthoformate (2.4 ml, 14.4 mmol). The reaction mixture was heated to 110 °C. After 5 h, the reaction mixture was concentrated to dryness. The product was purified on silica gel (0-10% MeOH in CH₂Cl₂) to yield the desired product as a yellow solid. LCMS calculated for C₂₅H₃₀BrClFN₆O₂ (M+H)⁺: m/z = 581.1; found 581.2.

### Step 2. 2,4-dibromo-6-chloronaphthalen-1-amine

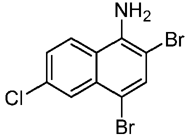

To a solution of 6-chloronaphthalen-1-amine (1.120 g, 6.30 mmol) in acetic acid (100 mL) was added bromine (0.7 ml, 13.9 mmol) at r.t., and the reaction was stirred at 70 °C for 1 h. After cooling to r.t., the reaction mixture was filtered and the filter cake was washed with acetic acid and dried under vacuum to provide the desired product which was used in next step without further purification. LCMS calculated for C₁₀H₇Br₂ClN (M+H)⁺: m/z = 333.8; found: 333.9.

### Step 3. 4-bromo-6-chloronaphthalen-2-ol

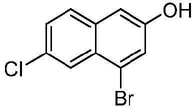

To a solution of 2,4-dibromo-6-chloronaphthalen-1-amine (459 mg, 1.369 mmol) in acetic acid (9 mL) and propionic acid (1.5 mL) was added sodium nitrite (113 mg, 1.6 mmol) portionwise at 0 °C over 30 min. Following complete addition, the reaction mixture was stirred at 0 °C for an additional 30 min. The reaction mixture was then poured into ice (100 mL). The resulting solid was collected and washed with water to provide a crude product as gray solid which was used directly in the next step without further purification.

To a solution of the crude product in EtOH (50 mL) was added sodium borohydride (55.4 mg, 1.4 mmol) portionwise at 0 °C. Following complete addition, the reaction allowed to warm to r.t. and stirred for an additional 3 h. Water was then added and the reaction mixture, and the solution was extracted with CH₂Cl₂. The combined organic layers were dried over Na₂SO₄, filtered, concentrated, and purified on silica gel to provide desired product as gray solid. LCMS calculated for C₁₀H₇BrClO (M+H)⁺: m/z = 257.0; found: 257.0.

### Step 4. 6-chloro-4-(4,4,5,5-tetramethyl-1,3,2-dioxaborolan-2-yl)naphthalen-2-ol

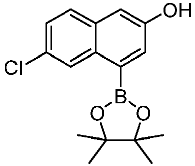

A solution of 4-bromo-6-chloronaphthalen-2-ol (55 mg, 0.214 mmol), Pd(dppf)Cl₂•CH₂Cl₂ (17.44 mg, 0.021 mmol), potassium acetate (62.9 mg, 0.641 mmol), and bis(pinacolato)diboron (54.2 mg, 0.214 mmol) in dioxane (2.0 mL) was heated at 100 °C for 2 h. The reaction mixture was cooled to r.t., diluted with EtOAc and filtered through celite. The organic layer was concentrated and t to provide the desired product. LCMS calculated for C₁₆H₁₉BClO₃ (M+H)⁺: m/z = 305.1; found 305.1.

### Step 5. 4-(1-((endo)-2-azabicyclo[2.1. 1]hexan-5-yl)-8-chloro-4-(3-(dimethylamino)azetidin-1-yl)-6-fluoro-1H-imidazo[4,5-c]quinolin-7-yl)-6-chloronaphthalen-2-ol

A screw-cap vial equipped with a magnetic stir bar was charged with tert-butyl (*endo*)-5-(7-bromo-8-chloro-4-(3-(dimethylamino)azetidin-1-yl)-6-fluoro-1*H*-imidazo[4,5-*c*]quinolin-1-yl)-2-azabicyclo[2.1.1]hexane-2-carboxylate (10 mg, 0.0172 mmol), 6-chloro-4-(4,4,5,5-tetramethyl-1,3,2-dioxaborolan-2-yl)naphthalen-2-ol (6.3 mg, 0.0207 mmol), K₃PO₄ (7.1 mg, 0.0345 mmol), Pd(Ph₃P)₄ (1.93 mg, 0.0017 mmol) and dioxane (0.8 ml)/water (0.2 ml). The vial was sealed with a Teflon-lined septum, evacuated and backfilled with nitrogen (this process was repeated a total of three times), and stirred at 100 °C for 2 h. After cooling to r.t. the reaction was concentrated to dryness, then TFA (1.0 mL) was added. The mixture was stirred at r.t. for 5 min, then diluted with acetonitrile/water and purified using prep-LCMS (XBridge C18 column, eluting with a gradient of acetonitrile/water containing 0.1% TFA, at flow rate of 60 mL/min) to afford the desired product as a TFA salt. The products were isolated as pairs of enantiomers.

**Example 23a.** Diastereomer 1. Peak 1. LCMS calculated for C₃₀H₂₈Cl₂FN₆O (M+H)⁺: m/z = 577.2; found 577.2.

**Example 23b.** Diastereomer 2. Peak 2. LCMS calculated for C₃₀H₂₈Cl₂FN₆O (M+H)⁺: m/z = 577.2; found 577.2.

### Example 24. 4-(1-((endo)-2-azabicyclo[2.1.1]hexan-5-yl)-8-chloro-4-(3-(dimethylamino)-azetidin-1-yl)-6-fluoro-1H-imidazo[4,5-c]quinolin-7-yl)-6-methylnaphthalen-2-ol

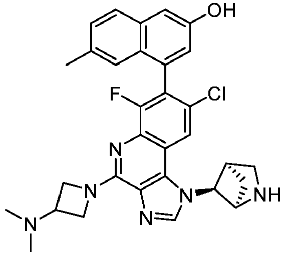

This compound was prepared according to the procedure described in **Example 23a and Example 23b,** replacing 6-chloronaphthalen-1-amine with 6-methylnaphthalen-1-amine in **Step 3.** The product was isolated as a mixture of diastereomers. LCMS calculated for C₃₁H₃₁CIFN₆O (M+H)+: m/z = 557.2; found: 557.2.

### Example 25a, Example 25b, and Example 25c. 4-(1-((endo)-2-azabicyclo[2.1.1]hexan-5-yl)-4-(3-(dimethylamino)azetidin-1-yl)-6-fluoro-8-(1H-pyrazol-4-yl)-1H-imidazo[4,5-c]quinolin-7-yl)naphthalen-2-ol

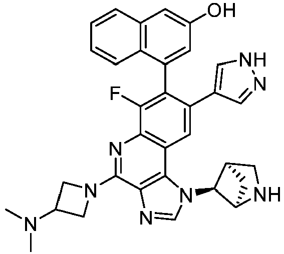

### Step 1. tert-butyl (endo)-5-(8-chloro-4-(3-(dimethylamino)azetidin-1-yl)-6-fluoro-7-(3-hydroxy-naphthalen-1-yl)-1H-imidazo[4, 5-c]quinolin-1-yl)-2-azabicyclo[2. 1.1]hexane-2-carboxylate

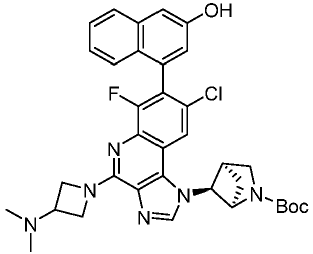

A screw-cap vial equipped with a magnetic stir bar was charged with tert-butyl (*endo*)-5-(7-bromo-8-chloro-4-(3-(dimethylamino)azetidin-1-yl)-6-fluoro-1*H*-imidazo[4,5-c]quinolin-1-yl)-2-azabicyclo[2.1.1]hexane-2-carboxylate **(Example 23a and Example 23b, Step 1,** 200 mg, 0.345 mmol), (3-hydroxynaphthalen-1-yl)boronic acid (97 mg, 0.517 mmol), Pd(Ph₃P)₄ (120 mg, 0.103 mmol), K₃PO₄ (220 mg, 1.035 mmol), and dioxane (5.75 mL)/water (1.15 mL). The resultant mixture was flushed with nitrogen for 2 min and then stirred at 120 °C for 1 h. After cooling to r.t., the mixture was concentrated to dryness and purified on silica gel to yield the desired product. LCMS calculated for C₃₅H₃₇ClFN₆O₃⁺ (M+H)⁺: m/z = 643.3; found: 643.3.

### Step 2. 4-(1-((endo)-2-azabicyclo[2.1.1]hexan-5-yl)-4-(3-(dimethylamino)azetidin-1-yl)-6-fluoro-8-(1H-pyrazol-4-yl)-1H-imidazo[4,5-c]quinolin-7-yl)naphthalen-2-ol

To a mixture of tert-butyl (endo)-5-(8-chloro-4-(3-(dimethylamino)azetidin-1-yl)-6-fluoro-7-(3-hydroxynaphthalen-1-yl)-1H-imidazo[4,5-c]quinolin-1-yl)-2-azabicyclo[2.1.1]hexane-2-carboxylate (20 mg, 0.031 mmol), 4-(4,4,5,5-tetramethyl-1,3,2-dioxaborolan-2-yl)-1H-pyrazole (9.05 mg, 0.047 mmol), XPhos Pd G2 (7.34 mg, 9.33 µmol), and K₃PO₄ (19.8 mg, 0.093 mmol) was added dioxane (0.5 mL)/water (0.1 mL). The resultant mixture was flushed with nitrogen for 2 min and stirred at 120 °C for 1 h. The resulting mixture was cooled to r.t., filtered through a thiocartridge, washed with MeOH/CH₂Cl₂ and concentrated to dryness. The residue was redissolved in CH₂Cl₂ (1.0 mL) and TFA (1.0 mL) was added slowly. After stirring at r.t. for 30 min, the mixture was diluted with MeOH/acetonitrile and purified using prep-LCMS (XBridge C18 column, eluting with a gradient of acetonitrile/water containing 0.1% TFA, at flow rate of 60 mL/min) to afford the desired product as a TFA salt. The products were isolated as either a pair of enantiomers or a mixture of diastereomers.

**Example 25a.** Diastereomer 1. Peak 1. LCMS calculated for C₃₃H₃₂FN₈O⁺ (M+H)⁺: m/z = 575.3; found: 575.4.

**Example 25b.** Diastereomer 2. Peak 2. LCMS calculated for C₃₃H₃₂FN₈O⁺ (M+H)⁺: m/z = 575.3; found: 575.4.

**Example 25c.** Diastereomer 3. Peak 3. LCMS calculated for C₃₃H₃₂FN₈O⁺ (M+H)⁺: m/z = 575.3; found: 575.4.

### Example 26. 1-(1-acryloylazetidin-3-yl)-8-fluoro-7-(3-hydroxynaphthalen-1-yl)-5-(2-isopropyl-4-methylpyridin-3-yl)-3,5-dihydro-1H-imidazo[4,5-c][1,8]naphthyridine-2,4-dione

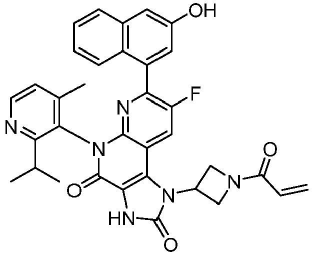

### Step 1. methyl 3-((2-isopropyl-4-methylpyridin-3-yl)amino)-3-oxopropanoate

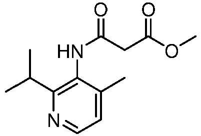

A solution of methyl 3-chloro-3-oxopropanoate (3.6 mL, 33.5 mmol) in CH₂Cl₂ (128 mL) was slowly added to a stirred mixture of 2-isopropyl-4-methylpyridin-3-amine (4.8 g, 32 mmol) and triethylamine (5.34 mL, 38.3 mmol) in CH₂Cl₂ (128 mL) at 0 °C. After complete addition, the mixture was removed from the ice/water bath and stirred at r.t. for an additional 1 h. The reaction was quenched by addition of sat'd NaHCO₃ then extracted with CH₂Cl₂. The combined organic were dried over MgSO₄, concentrated to dryness, and purified on silica gel (120 g, 0-50% EtOAc in hexanes) to yield a white solid (6.07 g, 76%). LCMS calculated for C₁₃H₁₉N₂O₃ (M+H)⁺: m/z = 251.1; found: 251.1.

### Step 2. methyl 7-chloro-6-fluoro-4-hydroxy-1-(2-isopropyl-4-methylpyridin-3-yl)-2-oxo-1,2-dihydro-1,8-naphthyridine-3-carboxylate

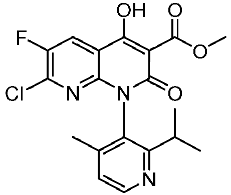

To a solution of methyl 3-((2-isopropyl-4-methylpyridin-3-yl)amino)-3-oxopropanoate (6.07 g, 24.25 mmol) in THF (120 mL) was added sodium hydride (60 wt%, 3.88 g, 97 mmol) in several portions. The mixture was stirred at r.t. for 15 min then cooled to 0 °C in an ice/water bath. A solution of 2,6-dichloro-5-fluoronicotinoyl chloride in THF (0.5 M) was added dropwise. Following complete addition, the mixture was stirred at r.t. for an additional 2 h before heating to a reflux overnight. The reaction was cooled to r.t., poured into water (250 mL)/1 N HCl (50 mL), and extracted with EtOAc. The combined organic layers were concentrated and used directly in the next step without further purification. LCMS calculated for C₁₉H₁₈ClFN₃O₄ (M+H)⁺: m/z = 406.1; found: 406.1.

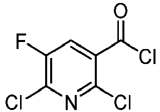

To a solution of 2,6-dichloro-5-fluoronicotinic acid (5.35 g, 25.5 mmol) in CH₂Cl₂ (75 mL) was added 1 drop of DMF followed by slow addition of oxalyl chloride (6.16 mL, 72.8 mmol). The mixture was stirred at r.t. for 1 h then concentrated under reduced pressure. The residue was taken up in THF (50 mL) and used immediately.

### Step 3. 7-chloro-6-fluoro-4-hydroxy-1-(2-isopropyl-4-methylpyridin-3-yl)-1,8-naphthyridin-2(1H)-one

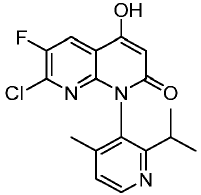

The concentrated residue from **Step 2** was taken up in TFA (40 mL)/concentrated HCl (40 mL) and heated at 100 °C for 4 h. The mixture was cooled to r.t., concentrated under reduced pressure, and neutralized to pH 6 with sodium carbonate. The mixture was then extracted with EtOAc. The combined organic layers were concentrated and used directly in the next step without further purification. LCMS calculated for C₁₇H₁₆ClFN₃O₂ (M+H)⁺: m/z = 348.1; found: 348.0.

### Step 4. 7-chloro-6-fluoro-4-hydroxy-1-(2-isopropyl-4-methylpyridin-3-yl)-3-nitro-1,8-naphthyridin-2(1H)-one

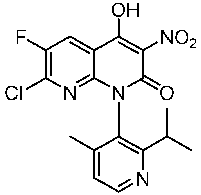

The concentrated residue from **Step 3** was taken up in acetic acid (40 mL). Nitric acid (concentrated, 5.42 mL) was added dropwise and the mixture was heated at 50 °C for 20 min. After cooling to r.t., ice was slowly added to the reaction mixture, followed by water (200 mL). The precipitate was collected via filtration, washed with water, and dried to provide the desired product as a bright yellow solid (8.39 g, 88% over 3 steps) which was used directly in the next step without further purification. LCMS calculated for C₁₇H₁₅ClFN₄O₄ (M+H)⁺: m/z = 393.1; found 393.1.

### Step 5. 4,7-dichloro-6-fluoro-1-(2-isopropyl-4-methylpyridin-3-yl)-3-nitro-1,8-naphthyridin-2(1H)-one

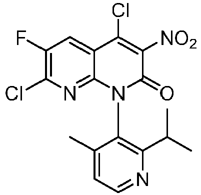

A solution of 7-chloro-6-fluoro-4-hydroxy-1-(2-isopropyl-4-methylpyridin-3-yl)-3-nitro-1,8-naphthyridin-2(1H)-one (1.5 g, 3.82 mmol), DIPEA (2.0 mL, 11.46 mmol), and POCl₃ (2.136 mL, 22.91 mmol) in toluene (7.64 mL) was heated at 100 °C for 2 h. After cooling to r.t., the mixture was concentrated. The residue was redissolved in CH₂Cl₂ and neutralized with DIPEA, then purified by silica gel (25 g, 0-50% EtOAc in hexanes) to yield a brown solid (1.2 g, 76%). LCMS calculated for C₁₇H₁₄Cl₂FN₄O₃ (M+H)⁺: m/z = 411.0; found: 411.0.

### Step 6. tert-butyl 3-((7-chloro-6-fluoro-1-(2-isopropyl-4-methylpyridin-3-yl)-3-nitro-2-oxo-1,2-dihydro-1,8-naphthyridin-4-yl)amino)azetidine-1-carboxylate

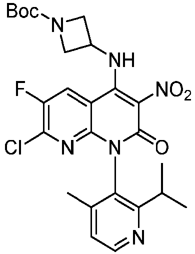

A solution of 4,7-dichloro-6-fluoro-1-(2-isopropyl-4-methylpyridin-3-yl)-3-nitro-1,8-naphthyridin-2(1H)-one (150 mg, 0.365 mmol), tert-butyl 3-aminoazetidine-1-carboxylate (75 mg, 0.438 mmol), and DIPEA (0.1 mL, 0.547 mmol) was stirred in acetonitrile (1.0 mL) at r.t. for 3 h. The mixture was concentrated and used directly in the next step without further purification. LCMS calculated for C₂₅H₂₉ClFN₆O₅ (M+H)⁺: m/z = 547.2; found: 547.2.

### Step 7. tert-butyl 3-((3-amino-7-chloro-6-fluoro-1-(2-isopropyl-4-methylpyridin-3-yl)-2-oxo-1,2-dihydro-1,8-naphthyridin-4-yl)amino)azetidine-1-carboxylate

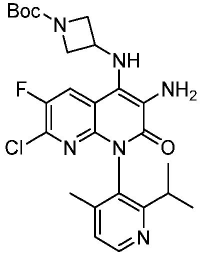

The concentrated residue from **Step 6** was taken up in MeOH (0.4 mL)/THF (0.4 mL)/water (0.4 mL). To the solution was added iron (81 mg, 1.459 mmol), and ammonium chloride (117 mg, 2.189 mmol) and the mixture was stirred at 60 °C for 30 min. After cooling to r.t., the mixture was diluted with EtOAc and then filtered over a pad of celite. The filtrate was dried over MgSO₄, concentrated, and purified by silica gel (20 g, 0-50% EtOAc in hexanes) to provide the desired product (120.1 mg, 64% over two steps). LCMS calculated for C₂₅H₃₁ClFN₆O₃ (M+H)⁺: m/z = 517.2; found: 517.2.

### Step 8. tert-butyl 3-(7-chloro-8-fluoro-5-(2-isopropyl-4-methylpyridin-3-yl)-2,4-dioxo-2,3,4,5-tetrahydro-1H-imidazo[4,5-c][1,8]naphthyridin-1-yl)azetidine-1-carboxylate

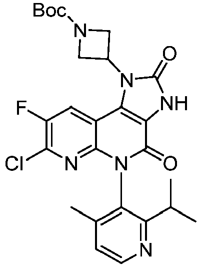

To a solution of tert-butyl 3-((3-amino-7-chloro-6-fluoro-1-(2-isopropyl-4-methylpyridin-3-yl)-2-oxo-1,2-dihydro-1,8-naphthyridin-4-yl)amino)azetidine-1-carboxylate (60 mg, 0.116 mmol) in THF (1.0 mL) was added CDI (56.5 mg, 0.348 mmol). The mixture was refluxed for 5 h then cooled to r.t., concentrated, and used directly in the next step. LCMS calculated for C₂₆H₂₉ClFN₆O₄ (M+H)⁺: m/z = 543.2; found: 543.2.

### Step 9. 1-(azetidin-3-yl)-8-fluoro-7-(3-hydroxynaphthalen-1-yl)-5-(2-isopropyl-4-methylpyridin-3-yl)-3,5-dihydro-1H-imidazo[4,5-c][1,8]naphthyridine-2,4-dione

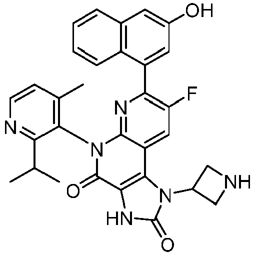

A screw-cap vial equipped with a magnetic stir bar was charged tert-butyl 3-(7-chloro-8-fluoro-5-(2-isopropyl-4-methylpyridin-3-yl)-2,4-dioxo-2,3,4,5-tetrahydro-1*H-*imidazo[4,5-c][1,8]naphthyridin-1-yl)azetidine-1-carboxylate (61 mg, 0.116 mmol), 4-(4,4,5,5-tetramethyl-1,3,2-dioxaborolan-2-yl)naphthalen-2-ol (47 mg, 0.174 mmol), sodium carbonate (61 mg, 0.58 mmol), XPhos Pd G2 (9.13 mg, 12 µmol) and dioxane (0.84 ml)/water (0.16 ml). The headspace of the vial briefly sparged with nitrogen then sealed with a Teflon-lined septum. The reaction was stirred at 90 °C overnight. After cooling to r.t. the mixture was diluted with EtOAc and extracted with brine. The combined organic layers were dried over MgSO₄, filtered, and concentrated. The concentrated residue was dissolved in CH₂Cl₂ (1.5 mL)/TFA (0.5 mL) and stirred at r.t. for 30 min. The mixture was purified using prep-LCMS (XBridge C18 column, eluting with a gradient of acetonitrile/water containing 0.1% TFA, at flow rate of 60 mL/min) to afford the desired product as a TFA salt. LCMS calculated for C₃₁H₂₈FN₆O₃ (M+H)⁺: m/z = 551.2; found: 551.2.

### Step 10. 1-(1-acryloylazetidin-3-yl)-8-fluoro-7-(3-hydroxynaphthalen-1-yl)-5-(2-isopropyl-4-methylpyridin-3-yl)-3,5-dihydro-1H-imidazo[4,5-c][1,8]naphthyridine-2,4-dione

A solution 1-(azetidin-3-yl)-8-fluoro-7-(3-hydroxynaphthalen-1-yl)-5-(2-isopropyl-4-methylpyridin-3-yl)-3,5-dihydro-1H-imidazo[4,5-c][1,8]naphthyridine-2,4-dione (TFA salt, 17.4 mg, 0.031 mmol) and triethylamine (17.3 µL, 0.124 mmol) in THF (1.0 mL) was cooled to -78 °C in a dry ice/acetone bath. A solution of acryloyl chloride (5.8 µL, 71.4 µmol) in THF (1.0 mL) was added slowly and the reaction was stirred at -78 °C for 5 min. The reaction was diluted with MeOH then purified using prep-LCMS (XBridge C18 column, eluting with a gradient of acetonitrile/water containing 0.1% TFA, at flow rate of 60 mL/min) to afford the desired products as a TFA salt. The product was isolated as a mixture of diastereomers. LCMS calculated for C₃₄H₃₀FN₆O₄ (M+H)⁺ m/z = 605.2 ; found 605.3.

### Example 27. (S)-1-(1-acryloylazetidin-3-yl)-8-fluoro-7-(3-hydroxynaphthalen-1-yl)-5-(2-isopropyl-4-methylpyridin-3-yl)-3-((1-methylpyrrolidin-2-yl)methyl)-3,5-dihydro-1H-imidazo[4,5-c][1,8]naphthyridine-2,4-dione

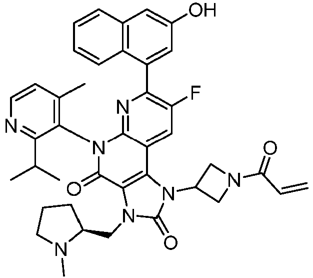

### Step 1. tert-butyl (S)-3-(7-chloro-8-fluoro-5-(2-isopropyl-4-methylpyridin-3-yl)-3-((1-methylpyrrolidin-2-yl)methyl)-2,4-dioxo-2,3,4,5-tetrahydro-1H-imidazo[4,5-c][1,8]naphthyridin-1-yl)azetidine-1-carboxylate

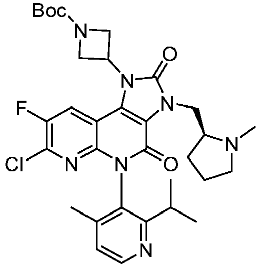

To a solution of tert-butyl 3-(7-chloro-8-fluoro-5-(2-isopropyl-4-methylpyridin-3-yl)-2,4-dioxo-2,3,4,5-tetrahydro-1*H*-imidazo[4,5-*c*][1,8]naphthyridin-1-yl)azetidine-1-carboxylate

**(Example 26, step 8,** 30 mg, 55 µmol), (S)-(1-methylpyrrolidin-2-yl)methanol (13 mg, 0.11 mmol), and PPh₃ (29 mg, 0.11 mmol) in THF (0.4 mL) was added DEAD (40 wt% in toluene, 50 µL, 0.11 mmol) dropwise. The mixture was stirred at r.t. for 1 h. The reaction mixture was then extracted between sat'd NaHCO₃ and EtOAc. The combined organic layers were dried over MgSO₄, filtered, concentrated, and used directly in the next step without further purification. LCMS calculated for C₃₂H₄₀ClFN₇O₄ (M+H)⁺: m/z = 640.3; found: 640.3.

### Step 2. (S)-1-(1-acryloylazetidin-3-yl)-8-fluoro-7-(3-hydroxynaphthalen-1-yl)-5-(2-isopropyl-4-methylpyridin-3-yl)-3-((1-methylpyrrolidin-2-yl)methyl)-3,5-dihydro-1H-imidazo[4,5-c][1,8]-naphthyridine-2,4-dione

To the concentrated residue from **Step 1,** 4-(4,4,5,5-tetramethyl-1,3,2-dioxaborolan-2-yl)naphthalen-2-ol (18 mg, 66 µmol), sodium carbonate (23 mg, 0.22 mmol), and Pd(dppf)Cl2•CH2Cl2 (8.98 mg, 11 µmol) was added dioxane (0.84 ml)/water (0.16 ml). The headspace of the vial briefly sparged with nitrogen then sealed with a Teflon-lined septum. The reaction was stirred at 100 °C overnight. After cooling to r.t. the mixture was diluted with EtOAc and extracted with brine. The combined organic layers were dried over MgSO₄, filtered, concentrated, and used directly in the next step without further purification. LCMS calculated for C₄₂H₄₇FN₇O₅ (M+H)⁺: m/z = 748.4; found: 748.5.

The crude residue was redissolved in CH₂Cl₂ (1.5 mL)/TFA (0.5 mL) and stirred at r.t. for 30 min, then concentrated to dryness. To the concentrated residue was added an excess amount of trimethylamine (0.5 mL) and THF (1.0 m). The mixture was cooled to -78 °C in a dry ice/acetone bath, then a solution of acryloyl chloride (6.7 µL, 83 µmol) in THF (1.0 mL) was added slowly. After stirring at -78 °C for 10 min, the reaction was diluted with MeOH then purified using prep-LCMS (XBridge C18 column, eluting with a gradient of acetonitrile/water containing 0.1% TFA, at flow rate of 60 mL/min) to afford the desired products as a TFA salt. The product was isolated as a mixture of diastereomers. LCMS calculated for C₄₀H₄₁FN₇O₄ (M+H)+ m/z = 702.3; found 702.4.

### Example 28. 1-(1-acryloylazetidin-3-yl)-8-fluoro-7-(3-hydroxynaphthalen-1-yl)-5-phenyl-3,5-dihydro-1H-imidazo[4,5-c][1,8]naphthyridine-2,4-dione

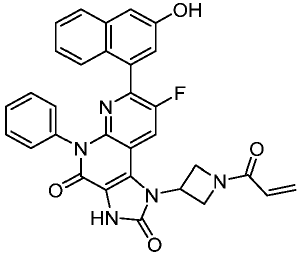

This compound was prepared according to the procedure described in **Example 26,** replacing 2-isopropyl-4-methylpyridin-3-amine with aniline in **Step 1.** LCMS calculated for C₃₁H₂₃FN₅O₄ (M+H)⁺ m/z = 548.2 ; found 548.2.

### Example 29a and Example 29b. 1-((3R,4S)-3-(8-chloro-6-fluoro-7-(3-hydroxynaphthalen-1-yl)-4-(((S)-1-methylpyrrolidin-2-yl)methoxy)-1H-imidazo[4,5-c]quinolin-1-yl)-4-methylpyrrolidin-1-yl)prop-2-en-1-one

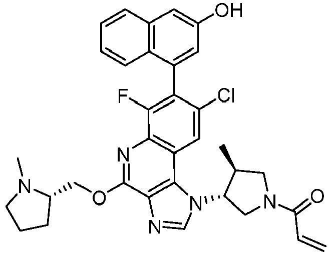

This compound was prepared according to the procedure described in **Example 1,** replacing tert-butyl 4-aminopiperidine-1-carboxylate with tert-butyl (3R,4S)-3-amino-4-methylpyrrolidine-1-carboxylate in **Step 5.**

**Example 29a.** Diastereomer 1. Peak 1. LCMS calculated for C₃₄H₃₄ClFN₅O₃ (M+H)+ m/z = 614.2; found 614.2.

**Example 29b.** Diastereomer 2. Peak 2. LCMS calculated for C₃₄H₃₄ClFN₅O₃ (M+H)+ m/z = 614.2; found 614.2.

### Example 30a and Example 30b. 1-((trans)-3-(8-chloro-4-(3-(dimethylamino)azetidin-1-yl)-6-fluoro-7-(3-hydroxynaphthalen-1-yl)-1H-imidazo[4,5-c]quinolin-1-yl)-4-methoxypyrrolidin-1-yl)prop-2-en-1-one

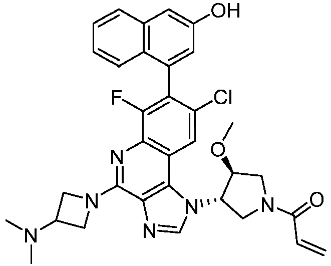

### Step 1. tert-butyl-(trans)-3-((7-bromo-6-chloro-2-(3-(dimethylamino)azetidin-1-yl)-8-fluoro-3-nitroquinolin-4-yl)amino)-4-methoxypyrrolidine-1-carboxylate

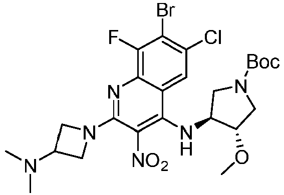

This compound was prepared according to the procedure described in **Example 15, step 1,** replacing *tert*-butyl (endo)-5-amino-2-azabicyclo[2.1.1]hexane-2-carboxylate with *tert*-butyl (*trans*)-3-amino-4-methoxypyrrolidine-1-carboxylate. LCMS calculated for C₂₄H₃₂BrClFN₆O₅ (M+H)⁺ m/z = 619.1; found 619.1.

### Step 2. tert-butyl (trans)-3-(7-bromo-8-chloro-4-(3-(dimethylamino)azetidin-1-yl)-6-fluoro-1H-imidazo[4,5-c]quinolin-1-yl)-4-methoxypyrrolidine-1-carboxylate

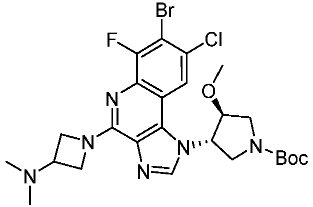

This compound was prepared according to the procedure described in **Example 16, step 2,** replacing *tert*-butyl (S)-4-((7-bromo-6-chloro-8-fluoro-2-((1-methylpyrrolidin-2-yl)-methoxy)-3-nitroquinolin-4-yl)amino)piperidine-1-carboxylate with *tert-butyl-(trans)-3-((7-*bromo-6-chloro-2-(3-(dimethylamino)azetidin-1-yl)-8-fluoro-3-nitroquinolin-4-yl)amino)-4-methoxypyrrolidine-1-carboxylate. LCMS calculated for C₂₅H₃₂BrClFN₆O₃ (M+H)⁺ m/z = 599.1; found 599.1.

### Step 3. 4-(8-chloro-4-(3-(dimethylamino)azetidin-1-yl)-6-fluoro-1-((trans)-4-methoxypyrrolidin-3-yl)-1H-imidazo[4,5-c]quinolin-7-yl)naphthalen-2-ol

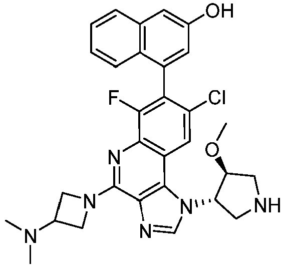

A screw-cap vial equipped with a magnetic stir bar was charged *tert*-butyl (*trans*)*-3-*(7-bromo-8-chloro-4-(3-(dimethylamino)azetidin-1-yl)-6-fluoro-1*H*-imidazo[4,5-*c*]quinolin-1-yl)-4-methoxypyrrolidine-1-carboxylate (112 mg, 0.187 mmol), 4-(4,4,5,5-tetramethyl-1,3,2-dioxaborolan-2-yl)naphthalen-2-ol (56 mg, 0.206 mmol), sodium carbonate (99 mg, 0.935 mmol), Pd(Ph₃P)₄ (22 mg, 19 µmol) and dioxane (0.84 ml)/water (0.16 ml). The headspace of the vial briefly sparged with nitrogen then sealed with a Teflon-lined septum. The reaction was stirred at 90 °C overnight. After cooling to r.t. the mixture was diluted with EtOAc and extracted with brine. The combined organic layers were dried over MgSO₄, filtered, and concentrated. The concentrated residue was dissolved in CH₂Cl₂ (1.5 mL)/TFA (0.5 mL) and stirred at r.t. for 30 min. The mixture was purified using prep-LCMS (XBridge C18 column, eluting with a gradient of acetonitrile/water containing 0.1% TFA, at flow rate of 60 mL/min) to afford the desired products as a TFA salt. LCMS calculated for C₃₀H₃₁ClFN₆O₂ (M+H)⁺ m/z = 561.2; found 561.2.

### Step 4. 1-((trans)-3-(8-chloro-4-(3-(dimethylamino)azetidin-1-yl)-6-fluoro-7-(3-hydroxy-naphthalen-1-yl)-1H-imidazo[4,5-c]quinolin-1-yl)-4-methoxypyrrolidin-1-yl)prop-2-en-1-one

A solution of 4-(8-chloro-4-(3-(dimethylamino)azetidin-1-yl)-6-fluoro-1-((*trans*)-4-methoxypyrrolidin-3-yl)-1*H*-imidazo[4,5-*c*]quinolin-7-yl)naphthalen-2-ol (TFA salt, 17.4 mg, 0.031 mmol) and triethylamine (17.3 µL, 0.124 mmol) in THF (1.0 mL) was cooled to -78 °C in a dry ice/acetone bath. A solution of acryloyl chloride (5.8 µL, 71.4 µmol) in THF (1.0 mL) was added slowly and the reaction was stirred at -78 °C for 5 min. The reaction was diluted with MeOH then purified using prep-LCMS (XBridge C18 column, eluting with a gradient of acetonitrile/water containing 0.1% TFA, at flow rate of 60 mL/min) to afford the desired products as a TFA salt. The products were isolated as pairs of enantiomers.

**Example 30a.** Diastereomer 1. Peak 1. LCMS calculated for C₃₃H₃₃ClFN₆O₃ (M+H)+ m/z = 615.2; found 615.2.

**Example 30b.** Diastereomer 2. Peak 2. LCMS calculated for C₃₃H₃₃ClFN₆O₃ (M+H)+ m/z = 615.27; found 615.2.

### Example 31a and Example 31b. 1-((trans)-3-(8-chloro-4-(3-(dimethylamino)azetidin-1-yl)-6-fluoro-7-(3-hydroxynaphthalen-1-yl)-1H-imidazo[4,5-c]quinolin-1-yl)-4-methylpyrrolidin-1-yl)prop-2-en-1-one

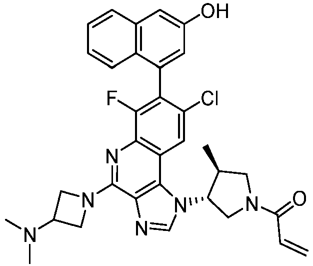

This compound was prepared according to the procedure described in **Example 30,** replacing tert-butyl (*trans*)-3-amino-4-methoxypyrrolidine-1-carboxylate with *tert*-butyl (*trans*)-3-amino-4-methylpyrrolidine-1-carboxylate in **Step 1.** The products were isolated as pairs of enantiomers.

**Example 31a.** Diastereomer 1. Peak 1. LCMS calculated for C₃₃H₃₃ClFN₆O₂ (M+H)+ m/z = 599.2; found 599.3.

**Example 31b.** Diastereomer 2. Peak 2. LCMS calculated for C₃₃H₃₃ClFN₆O₂ (M+H)+ m/z = 599.2; found 599.2.

### Example 32. 2-((2R,4R)-1-acryloyl-4-(8-chloro-4-(3-(dimethylamino)azetidin-1-yl)-6-fluoro-7-(3-hydroxynaphthalen-1-yl)-1H-imidazo[4,5-c]quinolin-1-yl)pyrrolidin-2-yl)acetonitrile

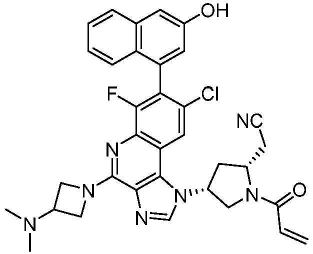

### Step 1. 1-(tert-butyl) 2-methyl (2R,4R)-4-((7-bromo-6-chloro-2-(3-(dimethylamino)azetidin-1-yl)-8-fluoro-3-nitroquinolin-4-yl)amino)pyrrolidine-1,2-dicarboxylate

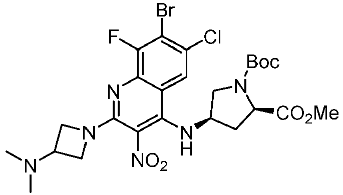

This compound was prepared according to the procedure described in **Example 15, step 1,** replacing *tert*-butyl (*endo*)-5-amino-2-azabicyclo[2.1.1]hexane-2-carboxylate with 1-(*tert*-butyl) 2-methyl (2*R*,4*R*)-4-aminopyrrolidine-1,2-dicarboxylate hydrochloride. LCMS calculated for C₂₅H₃₂BrClFN₆O₆ (M+H)⁺ m/z = 647.1; found 647.1.

### Step 2. 1-(tert-butyl) 2-methyl (2R,4R)-4-((3-amino-7-bromo-6-chloro-2-(3-(dimethylamino)-azetidin-1-yl)-8-fluoroquinolin-4-yl)amino)pyrrolidine-1,2-dicarboxylate

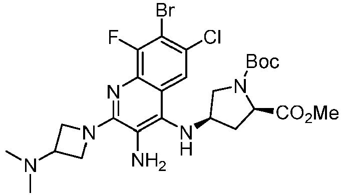

This compound was prepared according to the procedure described in **Example 1, step 7,** replacing *tert*-Butyl (S)-4-((7-bromo-6-chloro-8-fluoro-2-((1-methylpyrrolidin-2-yl)methoxy)-3-nitroquinolin-4-yl)amino)piperidine-1-carboxylate with 1-(*tert*-butyl) 2-methyl (2*R*,4*R*)-4-((7-bromo-6-chloro-2-(3-(dimethylamino)azetidin-1-yl)-8-fluoro-3-nitroquinolin-4-yl)amino)-pyrrolidine-1,2-dicarboxylate. LCMS calculated for C₂₅H₃₄BrClFN₆O₄ (M+H)⁺ m/z = 617.2; found 617.2.

### Step 3. 1-(tert-butyl) 2-methyl (2R,4R)-4-(7-bromo-8-chloro-4-(3-(dimethylamino)azetidin-1-yl)-6-fluoro-1H-imidazo[4,5-c]quinolin-1-yl)pyrrolidine-1,2-dicarboxylate

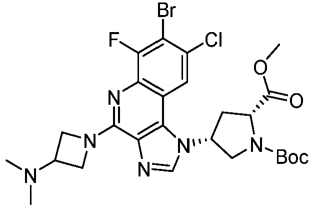

This compound was prepared according to the procedure described in **Example 1, step 8,** replacing *tert*-butyl (S)-4-((3-amino-7-bromo-6-chloro-8-fluoro-2-((1-methylpyrrolidin-2-yl)methoxy)quinolin-4-yl)amino)piperidine-1-carboxylate with 1-(*tert*-butyl) 2-methyl (2*R*,4*R*)-4-((3-amino-7-bromo-6-chloro-2-(3-(dimethylamino)azetidin-1-yl)-8-fluoroquinolin-4-yl)amino)pyrrolidine-1,2-dicarboxylate and stirring at 120 °C overnight. LCMS calculated for C₂₃H₃₂BrClFN₆O₄ (M+H)⁺ m/z = 627.1; found 627.1.

### Step 4. tert-butyl (2R,4R)-4-(7-bromo-8-chloro-4-(3-(dimethylamino)azetidin-1-yl)-6-fluoro-1H-imidazo[4,5-c]quinolin-1-yl)-2-(hydroxymethyl)pyrrolidine-1-carboxylate

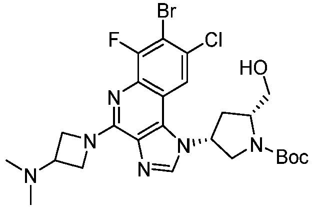

To a solution of 1-(*tert*-butyl) 2-methyl (2*R*,4*R*)-4-(7-bromo-8-chloro-6-fluoro-4-(3-isopropylazetidin-1-yl)-1*H*-imidazo[4,5-*c*]quinolin-1-yl)pyrrolidine-1,2-dicarboxylate (250 mg, 0.4 mmol) in MeOH (0.1 mL)/THF (1.0 mL) was slowly added LiBH₄ (2.0 M in THF, 0.3 mL, 0.6 mmol). The progress of the reaction was monitored and additional portions of LiBH₄ were added until no starting material remained. The mixture was then diluted with water and extracted with EtOAc. The combined organic layers were dried over MgSO₄, filtered, concentrated, and used directly in the next step. LCMS calculated for C₂₅H₃₂BrClFN₆O₃ (M+H)⁺ m/z = 599.1; found 599.1.

### Step 5. tert-butyl (2R,4R)-4-(7-bromo-8-chloro-4-(3-(dimethylamino)azetidin-1-yl)-6-fluoro-1H-imidazo[4,5-c]quinolin-1-yl)-2-(cyanomethyl)pyrrolidine-1-carboxylate

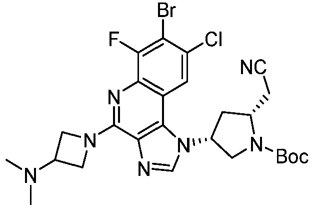

To a solution of tert-butyl (2*R*,4*R*)-4-(7-bromo-8-chloro-4-(3-(dimethylamino)azetidin-1-yl)-6-fluoro-1*H*-imidazo[4,5-*c*]quinolin-1-yl)-2-(hydroxymethyl)pyrrolidine-1-carboxylate (200 mg, 0.334 mmol) and triethylamine (140 µL, 0.502 mmol) in CH₂Cl₂ (1.0 mL) was slowly added MsCl (40 µL, 0.513 mmol). After stirring at r.t. for 30 min, silica gel was added to the reaction mixture, followed by MeOH (0.5 mL). The mixture was stirred for another 10 min and the solids were removed via filtration. The solids were washed with additional 10% MeOH/CH₂Cl₂ and the combined filtrate was concentrated under reduced pressure.

To the concentrated residue was added NaCN (131 mg, 2.676 mmol) and DMSO (1.0 mL). The mixture was stirred at 50 °C for 72 h, then cooled to r.t. and extracted between sat'd NaHCO₃ and EtOAc. The combined organic layers were dried over MgSO₄, filtered, concentrated, and used directly in the next step. LCMS calculated for C₂₆H₃₁BrClFN₇O₂ (M+H)⁺ m/z = 608.1; found 608.1.

### Step 6. 2-((2R,4R)-1-acryloyl-4-(7-bromo-8-chloro-4-(3-(dimethylamino)azetidin-1-yl)-6-fluoro-1H-imidazo[4,5-c]quinolin-1-yl)pyrrolidin-2-yl)acetonitrile

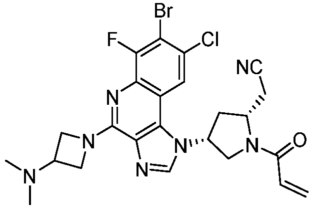

This compound was prepared according to the procedure described in **Example 16, step 5,** replacing *tert*-butyl (2S,4S)-4-(7-bromo-8-chloro-4-(3-(dimethylamino)azetidin-1-yl)-6-fluoro-1*H*-imidazo[4,5-*c*]quinolin-1-yl)-2-(cyanomethyl)piperidine-1-carboxylate with *tert-*butyl (2*R*,4*R*)-4-(7-bromo-8-chloro-4-(3-(dimethylamino)azetidin-1-yl)-6-fluoro-1*H-*imidazo[4,5-*c*]quinolin-1-yl)-2-(cyanomethyl)pyrrolidine-1-carboxylate. LCMS calculated for C₂₄H₂₅BrClFN₇O (M+H)⁺ m/z = 562.1; found 562.1.

### Step 7. 2-((2R,4R)-1-acryloyl-4-(8-chloro-4-(3-(dimethylamino)azetidin-1-yl)-6-fluoro-7-(3-hydroxynaphthalen-1-yl)-1H-imidazo[4,5-c]quinolin-1-yl)pyrrolidin-2-yl)acetonitrile

This compound was prepared according to the procedure described in **Example 1, step 10,** replacing (*S*)-1-(4-(7-bromo-8-chloro-6-fluoro-4-((1-methylpyrrolidin-2-yl)methoxy)-1*H*-imidazo[4,5-*c*]quinolin-1-yl)piperidin-1-yl)prop-2-en-1-one with 2-((2*R*,4*R*)-1-acryloyl-4-(7-bromo-8-chloro-4-(3-(dimethylamino)azetidin-1-yl)-6-fluoro-1*H*-imidazo[4,5-*c*]quinolin-1-yl)pyrrolidin-2-yl)acetonitrile. The product was isolated as a mixture of diastereomers. LCMS calculated for C₃₄H₃₂ClFN₇O₂ (M+H)⁺ m/z = 624.2 ; found 624.3.

### Example 33. 1-(1-acryloylpyrrolidin-3-yl)-8-chloro-6-fluoro-7-(3-hydroxynaphthalen-1-yl)-5-methyl-1,5-dihydro-4H-imidazo[4,5-c]quinolin-4-one

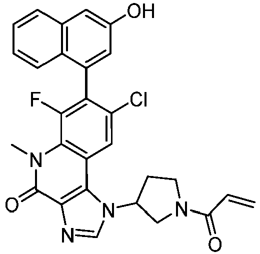

### Step 1: tert-butyl 3-((7-bromo-2,6-dichloro-8-fluoro-3-nitroquinolin-4-yl)amino)pyrrolidine-1-carboxylate

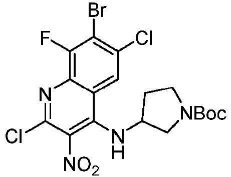

This compound was prepared according to the procedure described in **Example 1, step** 5 replacing *tert*-butyl 4-aminopiperidine-1-carboxylate with tert-butyl 3-aminopyrrolidine-1-carboxylate. LCMS calculated for C₁₈H₁₉BrCl₂FN₄O₄ (M+H)⁺ m/z = 523.0; found 523.1.

### Step 2: tert-butyl 3-((7-bromo-6-chloro-8-fluoro-2-methoxy-3-nitroquinolin-4-yl)amino)pyrrolidine-1-carboxylate

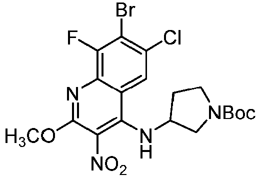

A flask was charged with tert-butyl 3-((7-bromo-2,6-dichloro-8-fluoro-3-nitroquinolin-4-yl)amino)pyrrolidine-1-carboxylate (0.905 g, 1.73 mmol), THF (8.2 mL), and sodium methoxide (25% w/w solution in MeOH, 1.12 g, 5.18 mmol). The reaction mixture was stirred at r.t. for 30 min, then diluted with water. The layers were separated, and the aqueous layer was extracted with CH₂Cl₂. The combined organic fractions were dried over MgSO₄, concentrated to dryness, and use directly in next step without further purification. LCMS calculated for C₁₉H₂₂BrClFN₄O₅ (M+H)⁺ m/z = 519.0; found 519.1.

### Step 3: tert-butyl 3-((3-amino-7-bromo-6-chloro-8-fluoro-2-methoxyquinolin-4-yl)amino)-pyrrolidine-1-carboxylate

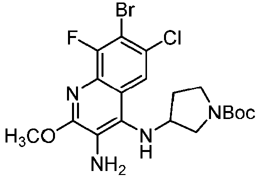

A mixture of *tert*-butyl 3-((7-bromo-6-chloro-8-fluoro-2-methoxy-3-nitroquinolin-4-yl)amino)pyrrolidine-1-carboxylate (0.9 g, 1.732 mmol), iron powder (0.967 g, 17.32 mmol), ammonium chloride (0.926 g, 17.32 mmol) in EtOH (17.3 mL)/water (17.3 mL) was stirred at 80 °C for 2 h. The mixture was diluted with dichloromethane and sat'd NaHCO₃, stirred for 10 minutes, and then filtered over a pad of celite. The filtrate was concentrated to dryness and purified on silica gel (0-20% EtOAc in CH₂Cl₂) to afford the desired product (0.387 g, 0.79 mmol, 46%). LCMS calculated for C₁₉H₂₄BrClFN₄O₃ (M+H)⁺ m/z = 489.1; found 489.1.

### Step 4: tert-butyl 3-(7-bromo-8-chloro-6-fluoro-4-methoxy-1H-imidazo[4,5-c]quinolin-1-yl)pyrrolidine-1-carboxylate

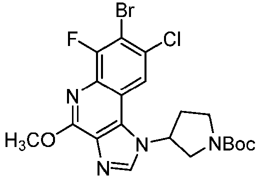

A mixture of *tert*-butyl 3-((3-amino-7-bromo-6-chloro-8-fluoro-2-methoxyquinolin-4-yl)amino)pyrrolidine-1-carboxylate (387 mg, 0.790 mmol), PPTS (1.986 mg, 7.90 µmol) in triethyl orthoformate (4 ml) was stirred at 100 °C for 5 min. The reaction mixture was cooled then diluted with hexanes, filtered, washed with hexanes, and dried to provide the desired product (0.286 g, 72%) which was used in the next step without further purification. LCMS calculated for C₂₀H₂₂BrClFN₄O₃ (M+H)⁺ m/z = 499.1; found 499.1.

### Step 5: tert-butyl 3-(7-bromo-8-chloro-6-fluoro-4-oxo-4,5-dihydro-1H-imidazo[4,5-c]quinolin-1-yl) pyrrolidine-1-carboxylate

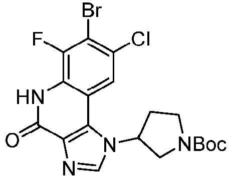

A vial was charged with *tert*-butyl 3-(7-bromo-8-chloro-6-fluoro-4-methoxy-1*H-*imidazo[4,5-*c*]quinolin-1-yl)pyrrolidine-1-carboxylate (.286 g, 0.572 mmol), potassium iodide (95 mg, 0.572 mmol), and acetic acid (5.72 ml). The reaction mixture was stirred at 80 °C for 2 h, quenched with sat'd NaHCO₃/Na₂S₂O₃ solution, then diluted with CH₂Cl₂. The layers were separated, and the aqueous layer was extracted with CH₂Cl₂. The combined organic fractions were dried over MgSO_{4,} concentrated to dryness, and purified on silica gel to yield the desired product (80 mg, 29%). LCMS calculated for C₁₉H₂₀BrClFN₄O₃ (M+H)⁺ m/z = 485.0; found 485.1.

### Step 6: tert-butyl 3-(7-bromo-8-chloro-6-fluoro-5-methyl-4-oxo-4,5-dihydro-1H-imidazo[4,5-c]quinolin-1-yl)pyrrolidine-1-carboxylate

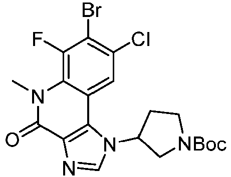

A vial was charged with *tert*-butyl 3-(7-bromo-8-chloro-6-fluoro-4-oxo-4,5-dihydro-1*H-*imidazo[4,5-*c*]quinolin-1-yl)pyrrolidine-1-carboxylate (30 mg, 62 µmol), DMF (0.309 ml), cesium carbonate (40 mg, 0.124 mmol), and methyl iodide (34 µl, 68 µmol). The reaction mixture was stirred at r.t. for 30 min then concentrated to dryness. The crude residue was purified on silica gel (0-10% MeOH in CH₂Cl₂) to afford the desired product (20 mg, 65%). LCMS calculated for C₂₀H₂₂BrClFN₄O₃ (M+H)⁺ m/z = 499.1; found 499.0.

### Step 7: 1-(1-acryloylpyrrolidin-3-yl)-7-bromo-8-chloro-6-fluoro-5-methyl-1,5-dihydro-4H-imidazo-[4,5-c]quinolin-4-one

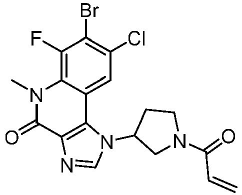

A vial was charged with tert-butyl 3-(7-bromo-8-chloro-6-fluoro-5-methyl-4-oxo-4,5-dihydro-1*H*-imidazo[4,5-*c*]quinolin-1-yl)pyrrolidine-1-carboxylate (20 mg, 0.04 mmol) and CH₂Cl₂ (0.5 mL)/TFA (0.5 mL). The reaction mixture was stirred for 30 min at r.t. then the mixture was concentrated to dryness. The crude residue was dissolved into CH₂Cl₂ (0.4 mL) and DIPEA (6.99 µl, 0.040 mmol) followed by acryloyl chloride (3.25 µl, 0.040 mmol) were added. The reaction mixture was stirred at r.t. for 30 min, concentrated to dryness. The crude residue was purified on silica gel (0-10% MeOH in CH₂Cl₂) to afford the desired product (10 mg, 55%). LCMS calculated for C₁₈H₁₆BrClFN₄O₂ (M+H)⁺ m/z = 453.0; found 452.8.

### Step 8: 1-(1-acryloylpyrrolidin-3-yl)-8-chloro-6-fluoro-7-(3-hydroxynaphthalen-1-yl)-5-methyl-1,5-dihydro-4H-imidazo[4,5-c]quinolin-4-one

This compound was prepared according to the procedure described in **Example 1, step 10,** replacing (*S*)-1-(4-(7-bromo-8-chloro-6-fluoro-4-((1-methylpyrrolidin-2-yl)methoxy)-1*H*-imidazo[4,5-*c*]quinolin-1-yl)piperidin-1-yl)prop-2-en-1-one with 1-(1-acryloylpyrrolidin-3-yl)-7-bromo-8-chloro-6-fluoro-5-methyl-1,5-dihydro-4*H*-imidazo[4,5-*c*]quinolin-4-one. The product was isolated as a mixture of diastereomers. LCMS calculated for C₂₃H₂₃ClFN₄O₃ (M+H)⁺ m/z = 517.1; found 517.2.

### Example 34a and Example 34b. 4-(1-((endo)-2-azabicyclo[2.1.1]hexan-5-yl)-4-(3-(dimethyl-amino)azetidin-1-yl)-6-fluoro-8-methyl-1H-imidazo[4,5-c]quinolin-7-yl)naphthalen-2-ol

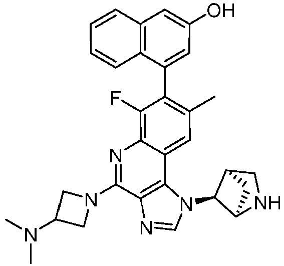

A screw-cap vial equipped with a magnetic stir bar was charged with *tert*-butyl (*endo*)-5-(8-chloro-4-(3-(dimethylamino)azetidin-1-yl)-6-fluoro-7-(3-hydroxynaphthalen-1-yl)-1*H*-imidazo-[4,5-*c*]quinolin-1-yl)-2-azabicyclo[2.1.1]hexane-2-carboxylate **(Example 25, step 1,** 18 mg, 0.028 mmol), potassium methyltrifluoroborate (10 mg, 0.084 mmol), Di(1-adamantyl)-n-butyl-phosphine (4.0 mg, 0.011 mmol), Cs₂CO₃ (27 mg, 0.084 mmol), Pd(OAc)₂ (1.3 mg, 0.0056 mmol) and toluene (0.4 ml)/water (0.1 ml). The vial was sealed with a Teflon-lined septum, evacuated and backfilled with nitrogen. The reaction was stirred at 120 °C for 1 h, then cooled to r.t. and concentrated to dryness. The residue was redissolved in TFA (1.0 mL) and stirred at r.t. for 5 min, then diluted with acetonitrile/water and purified using prep-LCMS (XBridge C18 column, eluting with a gradient of acetonitrile/water containing 0.1% TFA, at flow rate of 60 mL/min) to afford the desired product as a TFA salt. The products were isolated as pairs of enantiomers.

**Example 34a.** Diastereomer 1. Peak 1. LCMS calculated for C₃₁H₃₂FN₆O (M+H)⁺: m/z = 523.3; found: 523.2.

**Example 34b.** Diastereomer 2. Peak 2. LCMS calculated for C₃₁H₃₂FN₆O (M+H)⁺: m/z = 523.3; found: 523.2.

### Example 35a and Example 35b. 4-(1-((endo)-2-azabicyclo[2.1.1]hexan-5-yl)-4-(3,8-diazabicyclo[3.2.1]octan-8-yl)-8-chloro-6-fluoro-1H-imidazo[4,5-c]quinolin-7-yl)naphthalen-2-ol

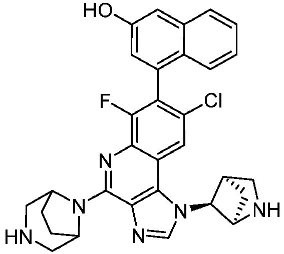

### Step 1. tert-butyl (endo)-5-((7-bromo-2, 6-dichloro-8-fluoro-3-nitroquinolin-4-yl)amino)-2-azabicyclo[2.1.1]hexane-2-carboxylate

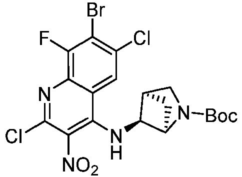

DIPEA (0.56 ml, 3.21 mmol) was added to a solution of 7-bromo-2,4,6-trichloro-8-fluoro-3-nitroquinoline **(Example 1, step 4,** 600 mg, 1.60 mmol) and *tert*-butyl (*endo*)-5-amino-2-azabicyclo[2.1.1]hexane-2-carboxylate (318 mg, 1.60 mmol) in CH₂Cl₂ (10.0 ml). The reaction was stirred at room temperature for 3 h, concentrated to dryness, and used directly in the next step without further purification.

### Step 2. tert-butyl (endo)-5-((3-amino-7-bromo-2,6-dichloro-8-fluoroquinolin-4-yl)amino)-2-azabicyclo[2.1.1]hexane-2-carboxylate

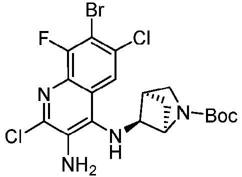

This compound was prepared according to the procedure described in **Example 15, step 2,** replacing *tert*-butyl (*endo*)-5-((7-bromo-6-chloro-2-(3-(dimethylamino)azetidin-1-yl)-8-fluoro-3-nitroquinolin-4-yl)amino)-2-azabicyclo[2.1.1]hexane-2-carboxylate with *tert*-butyl (*endo*)-5-((7-bromo-2,6-dichloro-8-fluoro-3-nitroquinolin-4-yl)amino)-2-azabicyclo[2.1.1]hexane-2-carboxylate.

### Step 3. tert-butyl (endo)-5-(7-bromo-4,8-dichloro-6-fluoro-1H-imidazo[4,5-c]quinolin-1-yl)-2-azabicyclo[2.1.1]hexane-2-carboxylate

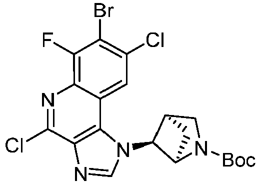

This compound was prepared according to the procedure described in **Example 23, step 1,** replacing *tert*-butyl (*endo*)-5-((3-amino-7-bromo-6-chloro-2-(3-(dimethylamino)azetidin-1-yl)-8-fluoroquinolin-4-yl)amino)-2-azabicyclo[2.1.1]hexane-2-carboxylate with *tert*-butyl (endo)-5-((3-amino-7-bromo-2,6-dichloro-8-fluoroquinolin-4-yl)amino)-2-azabicyclo[2.1.1]hexane-2-carboxylate. LCMS calculated for C₂₀H₁₉BrCl₂FN₄O₂ (M+H)⁺: m/z = 515.0; found: 515.1.

### Step 4. 4-(1-((endo)-2-azabicyclo[2.1.1]hexan-5-yl)-4-(3,8-diazabicyclo[3.2.1]octan-8-yl)-8-chloro-6-fluoro-1H-imidazo[4,5-c]quinolin-7-yl)naphthalen-2-ol

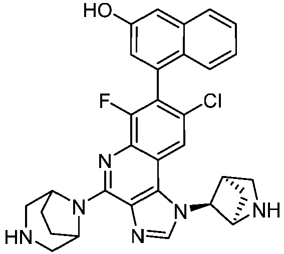

A vial charged with *tert*-butyl 5-(7-bromo-4,8-dichloro-6-fluoro-1*H*-imidazo[4,5-c]quinolin-1-yl)-2-azabicyclo[2.1.1]hexane-2-carboxylate (20 mg, 0.039 mmol), *tert*-butyl 3,8-diazabicyclo[3.2.1]octane-3-carboxylate (12 mg, 0.058 mmol), DIPEA (0.014 mL, 0.077 mmol) and *n*-BuOH (0.5 mL) was heated to 120 °C with vigorous stirring. Upon completion, the reaction mixture was diluted with EtOAc (3 mL) and washed with sat'd ammonium chloride (1 mL) and water (3 mL × 3). The combined organic fractions were dried over Na₂SO₄, concentrated to dryness, and use directly in next step without further purification.

The crude residue was combined with 4-(4,4,5,5-tetramethyl-1,3,2-dioxaborolan-2-yl)naphthalen-2-ol (12 mg, 43 µmol), K₃PO₄ (17 mg, 78 µmol), Pd(Ph₃P)₄ (5 mg, 3.9 µmol) and dioxane (0.8 ml)/water (0.2 ml). The vial was sealed with a Teflon-lined septum, evacuated and backfilled with nitrogen. The reaction was stirred at 100 °C for 3 h, , then cooled to r.t. and concentrated to dryness. The residue was redissolved in TFA (1.0 mL) and stirred at r.t. for 5 min, then diluted with acetonitrile/water and purified using prep-LCMS (XBridge C18 column, eluting with a gradient of acetonitrile/water containing 0.1% TFA, at flow rate of 60 mL/min) to afford the desired product as a TFA salt. The products were isolated as pairs of enantiomers.

**Example 35a.** Diastereomer 1. Peak 1. LCMS calculated for C₃₁H₂₉ClFN₆O (M+H)⁺: m/z = 555.2; found: 555.2.

**Example 35b.** Diastereomer 2. Peak 2. LCMS calculated for C₃₁H₂₉ClFN₆O (M+H)⁺: m/z = 555.2; found: 555.2.

### Example 36a, Example 36b, and Example 36c. 2-(1-acryloyl-4-(8-chloro-6-fluoro-7-(3-hydroxynaphthalen-1-yl)-4-(((S)-1-methylpyrrolidin-2-yl)methoxy)-1H-imidazo[4,5-c]quinolin-1-yl)piperidin-2-yl)acetonitrile

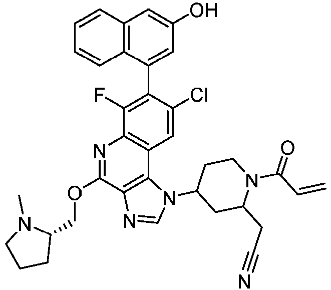

### Step 1. 2-allyl-1-benzoyl-2,3-dihydropyridin-4(1H)-one

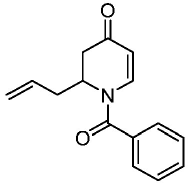

To a -23 °C solution of 4-methoxypyridine (5.20 g, 47.7 mmol) in THF (200 ml) was added dropwise phenyl chloroformate (6.58 ml, 52.4 mmol). The resulting slurry was stirred at this temperature for 1 h, then cooled to -78 °C. allylmagnesium chloride (2.0 M in THF, 26.2 ml, 52.4 mmol) was added dropwise. The mixture was stirred at -78 °C for 2 h, then quenched by the addition of 1 N HCl (42 mL). The solution was allowed to warm to room temperature and stirred for 12 h. The aqueous layer was extracted with EtOAc (3 times). The combined organic layers were washed successively with sat'd NaHCO₃ and brine. The resulting solution was dried over MgSO₄, filtered, and concentrated *under reduced pressure.* The remaining oil was purified using flash chromatography (0-50% EtOAc in hexanes) to give the desired product (6.3 g, 55%). LCMS calculated for C₁₅H₁₈N₂O₂ (M+NH₃)⁺: m/z = 258.1; found 258.1.

### Step 2. tert-butyl 2-allyl-4-oxo-3,4-dihydropyridine-1(2H)-carboxylate

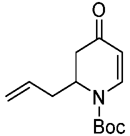

To a solution of 2-allyl-1-benzoyl-2,3-dihydropyridin-4(1*H*)-one (6.3 g, 26.1 mmol) in MeOH (60 ml) was added sodium methoxide (25% w/w/ in MeOH, 6.21 ml, 27.2 mmol). After stirring at 70 °C for 30 min, the reaction was neutralized by the slow addition of 12 M HCl at 0 °C. The remaining slurry was concentrated and purified by flash chromatography (100% ethyl acetate) to give the desired product.

The compound (3.1 g) from previous step was dissolved in acetonitrile (50 mL). Boc-anhydride (6.67 ml, 28.7 mmol) and DMAP (0.159 g, 1.305 mmol) were added and the resulting mixture was stirred at r.t. overnight. After which the solvent was removed and the remaining residue was diluted with EtOAc and washed with 1 M HCl, sat'd NaHCO₃, and brine. The combined organic layers were dried over Na₂SO₄, filtered, concentrated, and purified on silica gel (0-50% EtOAc in hexanes) to give the desired product as colorless oil. (4.66 g, 75 %). LCMS calculated for C₁₃H₂₀NO₃ (M+H)⁺: m/z = 238.1; found 238.1.

### Step 3. tert-butyl 2-allyl-4-oxopiperidine-1-carboxylate

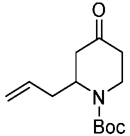

To a solution of *tert*-butyl 2-allyl-4-oxo-3,4-dihydropyridine-1(2*H*)-carboxylate (4.15 g, 17.49 mmol) in THF (39.7 ml) at -78 °C was added L-selectride (1.0 M in THF, 21.86 ml, 21.86 mmol) dropwise. The reaction was stirred at -78 °C for 1 h, then quenched by dropwise addition of water and warming to r.t.. The solvent was removed and the residue was purified on silica gel (0-50% EtOAc in hexanes) to provide the desired product (3.5 g, 84 %). LCMS calculated for C₁₃H₂₂NO₃ (M+H)⁺: m/z = 240.2; found 240.2.

### Step 4. tert-butyl 2-allyl-4-aminopiperidine-1-carboxylate

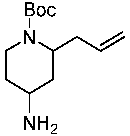

To a solution of *tert*-butyl 2-allyl-4-oxopiperidine-1-carboxylate (450 mg, 1.880 mmol) in ammonia (2.0 M in EtOH, 9.40 mmol) was added titanium(IV) isopropoxide (1.1 mL, 3.76 mmol). The solution was stirred at 60 °C for 3 h, then cooled to 0 °C. Sodium borohydride (2.0 M in triethylene glycol dimethyl ether, 113 µL, 2.82 mmol) was added to the reaction at 0°C. The resulting mixture was stirred for 1 h, quenched with 2 M ammonium in water, and filtered. The solids were washed with acetonitrile. The filtrate was concentrated under reduced pressure and the residue was extracted with EtOAc. The combined organic layers was washed with brine, dried over MgSO₄, filtered, and concentrated to give the desired product which was used without further purification. LCMS calculated for C₁₃H₂₅N₂O₂ (M+H)⁺: m/z = 241.2; found 241.2.

### Step 5. tert-butyl 2-allyl-4-((7-bromo-2,6-dichloro-8-fluoro-3-nitroquinolin-4-yl)amino)piperidine-1-carboxylate

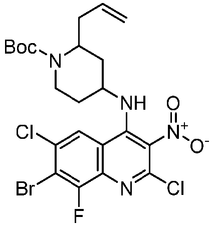

To a solution of 7-bromo-2,4,6-trichloro-8-fluoro-3-nitroquinoline **(Example 1, step 4,** 2.2 g, 5.88 mmol) in DMF (20 ml) was added tert-butyl 2-allyl-4-aminopiperidine-1-carboxylate (1.695 g, 7.05 mmol) and DIPEA (3.59 ml, 20.57 mmol). The resulting mixture was stirred at rt for 1 h. The reaction mixture was diluted with EtOAc and water. The organic layer was separated and washed with water and brine, dried over Na2SO4, filtered and concentrated. The residue was purified on silica gel (2.9 g, 85 %). LCMS calculated for C₂₂H₂₅BrCl₂FN₄O₄ (M+H)⁺: m/z = 577.0; found 577.0.

### Step 6. tert-butyl 2-allyl-4-((7-bromo-6-chloro-8-fluoro-2-(((S)-1-methylpyrrolidin-2-yl)methoxy)-3-nitroquinolin-4-yl)amino)piperidine-1-carboxylate

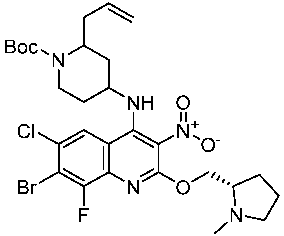

This compound was prepared according to the procedure described in **Example 1, step 6,** replacing *tert*-butyl 4-((7-bromo-2,6-dichloro-8-fluoro-3-nitroquinolin-4-yl)amino)piperidine-1-carboxylate with *tert*-butyl 2-allyl-4-((7-bromo-2,6-dichloro-8-fluoro-3-nitroquinolin-4-yl)amino)piperidine-1-carboxylate. LCMS calculated for C₂₈H₃₇BrClFN₅O₅ (M+H)⁺: m/z = 656.2; found 658.2.

### Step 7. tert-butyl 2-allyl-4-((3-amino-7-bromo-6-chloro-8-fluoro-2-(((S)-1-methylpyrrolidin-2-yl)methoxy)quinolin-4-yl)amino)piperidine-1-carboxylate

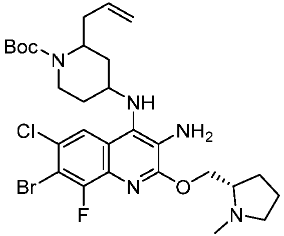

This compound was prepared according to the procedure described in **Example 1, step 7,** replacing *tert*-butyl (*S*)-4-((7-bromo-6-chloro-8-fluoro-2-((1-methylpyrrolidin-2-yl)-methoxy)-3-nitroquinolin-4-yl)amino)piperidine-1-carboxylate with *tert*-butyl 2-allyl-4-((7-bromo-6-chloro-8-fluoro-2-(((*S*)-1-methylpyrrolidin-2-yl)methoxy)-3-nitroquinolin-4-yl)amino)piperidine-1-carboxylate. LCMS calculated for C₂₈H₃₉BrClFN₅O₃ (M+H)⁺: m/z = 626.2; found 626.2,.

### Step 8. tert-butyl 2-allyl-4-(7-bromo-8-chloro-6-fluoro-4-(((S)-1-methylpyrrolidin-2-yl)methoxy)-1H-imidazo[4, 5-c]quinolin-1-yl)piperidine-1-carboxylate

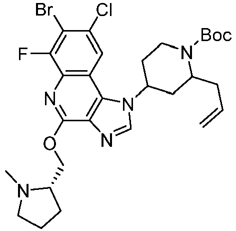

This compound was prepared according to the procedure described in **Example** 1, **step 8,** replacing of *tert-butyl* (S)-4-((3-amino-7-bromo-6-chloro-8-fluoro-2-((1-methylpyrrolidin-2-yl)methoxy)quinolin-4-yl)amino)piperidine-1-carboxylate with tert-butyl 2-allyl-4-((3-amino-7-bromo-6-chloro-8-fluoro-2-(((S)-1-methylpyrrolidin-2-yl)methoxy)quinolin-4-yl)amino)piperidine-1-carboxylate. LCMS calculated for C₂₉H₃₇BrClFN₅O₃ (M+H)⁺: m/z = 636.2; found 636.2.

### Step 9. tert-butyl 4-(7-bromo-8-chloro-6-fluoro-4-(((S)-1-methylpyrrolidin-2-yl)methoxy)-1H-imidazo[4, 5-c]quinolin-1-yl)-2-(2-oxoethyl)piperidine-1-carboxylate

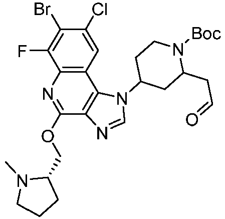

To a solution of tert-butyl 2-allyl-4-(7-bromo-8-chloro-6-fluoro-4-(((S)-1-methylpyrrolidin-2-yl)methoxy)-1H-imidazo[4,5-c]quinolin-1-yl)piperidine-1-carboxylate (1.30 g, 2.041 mmol) in 1,4-dioxane (38 ml)/water (12 ml) was added osmium tetraoxide in water (0.16 M, 0.64 ml, 0.102 mmol) and sodium metaperiodate (2.18 g, 10.20 mmol). The reaction was stirred at r.t. for 1.5 h, then filtered through a plug of celite. The plug was rinsed with THFand the filtrate was concentrated under vacuum. The residue was purified on silica gel (0-80% EtOAc in hexanes) to give the desired product as yellow oil (1.3 g, 97 %). LCMS calculated for C₂₈H₃₅BrClFN₅O₄ (M+H)⁺: m/z = 638.2; found 638.2.

### Step 10. tert-butyl 4-(7-bromo-8-chloro-6-fluoro-4-(((S)-1-methylpyrrolidin-2-yl)methoxy)-1H-imidazo[4,5-c]quinolin-1-yl)-2-(cyanomethyl)piperidine-1-carboxylate

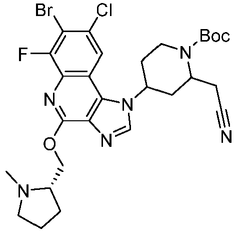

To a stirred solution of *tert*-butyl 4-(7-bromo-8-chloro-6-fluoro-4-(((S)-1-methylpyrrolidin-2-yl)methoxy)-1*H*-imidazo[4,5-c]quinolin-1-yl)-2-(2-oxoethyl) piperidine- 1-carboxylate (1.30 g, 2.035 mmol) in THF (20 ml) at r.t. was added ammonia in water (28 w/w, 2.227 ml, 33.0 mmol), followed by iodine (0.52 g, 2.035 mmol). The reaction mixture was stirred at r.t. for 2 h then quenched with 10% Na₂S₂O₃ (30 mL). The mixture was diluted with water and extracted with EtOAc. The organic layer was washed with brine, dried over MgSO₄, filtered and concentrated under reduced pressure. The residue was purified on silica gel (0-20% MeOH in CH₂Cl₂) to provide the desired product as a viscous yellow oil (705 mg, 55 %). LCMS calculated for C₂₈H₃₄BrClFN₆O₃ (M+H)⁺: m/z = 635.2, 637.2; found: 635.2, 637.2.

### Step 11. 2-(1-acryloyl-4-(7-bromo-8-chloro-6-fluoro-4-(((S)-1-methylpyrrolidin-2-yl)methoxy)-1H-imidazo[4,5-c]quinolin-1-yl)piperidin-2-yl)acetonitrile

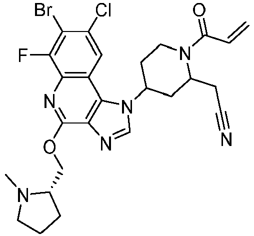

This compound was prepared according to the procedure described in **Example 16, step 5,** replacing of *tert*-butyl (2*S*,4*S*)-4-(7-bromo-8-chloro-4-(3-(dimethylamino)azetidin-1-yl)-6-fluoro-1*H*-imidazo[4,5-*c*]quinolin-1-yl)-2-(cyanomethyl)piperidine-1-carboxylate with *tert-*butyl 4-(7-bromo-8-chloro-6-fluoro-4-(((*S*)-1-methylpyrrolidin-2-yl)methoxy)-1*H*-imidazo[4,5-*c*]quinolin-1-yl)-2-(cyanomethyl)piperidine-1-carboxylate. LCMS calculated for C₂₆H₂₈BrClFN₆O₂ (M+H)⁺: m/z = 589.1; found: 589.1.

### Step 12. 2-(1-acryloyl-4-(8-chloro-6-fluoro-7-(3-hydroxynaphthalen-1-yl)-4-(((S)-1-methylpyrrolidin-2- yl)methoxy)-1H-imidazo[4,5-c]quinolin-1-yl)piperidin-2-yl)acetonitrile

A mixture of 2-(1-acryloyl-4-(7-bromo-8-chloro-6-fluoro-4-(((S)-1-methylpyrrolidin-2-yl)methoxy)-1*H*-imidazo[4,5-c]quinolin-1-yl)piperidin-2-yl)acetonitrile (15.0 mg, 0.025 mmol), 4-(4,4,5,5-tetramethyl-1,3,2-dioxaborolan-2-yl)naphthalen-2-ol (13.74 mg, 0.051 mmol), Pd(Ph₃P)₄ (2.94 mg, 2.54 µmol) and sodium carbonate (6.74 mg, 0.064 mmol) in 1,4-dioxane (1.0 mL)/water (0.2 mL) was stirred at 90 °C for 2 h. After cooling to r.t. the mixture was concentrated under reduced pressure. The residue was dissolved in MeOH and 1 N HCl, stirred for 30 min, then purified with prep-LCMS (XBridge C18 column, eluting with a gradient of acetonitrile/water containing 0.1% TFA, at flow rate of 60 mL/min) to afford the desired products as TFA salt (5 mg, 29 %). The products were isolated as either a pair of enantiomers or a mixture of diastereomers. LCMS calculated for C₃₆H₃₅ClFN₆O₃ (M+H)⁺: m/z = 653.2; found: 653.2.

**Example 36a.** Diastereomers 1. Peak 1. LCMS calculated for C₃₆H₃₅ClFN₆O₃ (M+H)⁺: m/z = 653.2; found: 653.2.

**Example 36b.** Diastereomers 2. Peak 2. LCMS calculated for C₃₆H₃₅ClFN₆O₃ (M+H)⁺: m/z = 653.2; found: 653.2.

**Example 36c.** Diastereomers 3. Peak 3. LCMS calculated for C₃₆H₃₅ClFN₆O₃ (M+H)⁺: m/z = 653.2; found: 653.2.

### Example 37a, Example 37b, and Example 37c. 2-(1-acryloyl-4-(8-chloro-6-fluoro-7-(5-methyl-1H-indazol-4-yl)-4-(((S)-1-methylpyrrolidin-2-yl)methoxy)-1H-imidazo[4,5-c]quinolin-1-yl)piperidin-2-yl)acetonitrile

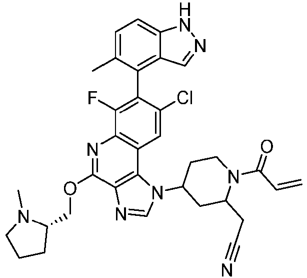

This compound was prepared according to the procedure described in **Example 36, step 12,** replacing 4-(4,4,5,5-tetramethyl-1,3,2-dioxaborolan-2-yl)naphthalen-2-ol with (5-methyl-1H-indazol-4-yl)boronic acid. The products were isolated as either a pair of enantiomers or a mixture of diastereomers. LCMS calculated for C₃₄H₃₅ClFN₈O₂ (M+H)⁺: m/z = 641.3; found: 641.3.

**Example 37a.** Diastereomers 1. Peak 1. LCMS calculated for C₃₄H₃₅ClFN₈O₂ (M+H)⁺: m/z = 641.3; found: 641.3.

**Example 37b.** Diastereomers 2. Peak 2. LCMS calculated for C₃₄H₃₅ClFN₈O₂ (M+H)⁺: m/z = 641.3; found: 641.3.

**Example 37c.** Diastereomers 3. Peak 3. LCMS calculated for C₃₄H₃₅ClFN₈O₂ (M+H)⁺: m/z = 641.3; found: 641.3.

### Example 38. 2-(1-acryloyl-4-(8-chloro-6-fluoro-4-(((S)-1-methylpyrrolidin-2-yl)methoxy)-7-(naphthalen-1-yl)-1H-imidazo[4,5-c]quinolin-1-yl)piperidin-2-yl)acetonitrile

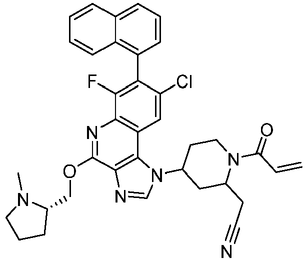

This compound was prepared according to the procedure described in **Example 36, step 12,** replacing 4-(4,4,5,5-tetramethyl-1,3,2-dioxaborolan-2-yl)naphthalen-2-ol with naphthalen-1-ylboronic acid. The product was isolated as a mixture of diastereomers. LCMS calculated for C₃₆H₃₅ClFN₆O₂ (M+H)⁺: m/z = 637.2; found: 637.2.

### Example 39a, Example 39b, and Example 39c. 2-(1-acryloyl-4-(8-chloro-6-fluoro-7-(5-hydroxy-2-methylphenyl)-4-(((S)-1-methylpyrrolidin-2-yl)methoxy)-1H-imidazo[4,5-c]quinolin-1-yl)piperidin-2-yl)acetonitrile

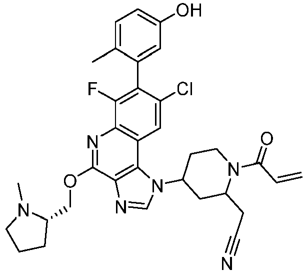

This compound was prepared according to the procedure described in **Example 36, step 12,** replacing 4-(4,4,5,5-tetramethyl-1,3,2-dioxaborolan-2-yl)naphthalen-2-ol with (5-hydroxy-2-methylphenyl)boronic acid. The products were isolated as either a pair of enantiomers or a mixture of diastereomers. LCMS calculated for C₃₃H₃₅ClFN₆O₃ (M+H)⁺: m/z = 617.2; found: 617.2.

**Example 39a.** Diastereomers 1. Peak 1. LCMS calculated for C₃₃H₃₅ClFN₆O₃ (M+H)⁺: m/z = 617.2; found: 617.2.

**Example 39b.** Diastereomers 2. Peak 2. LCMS calculated for C₃₃H₃₅ClFN₆O₃ (M+H)⁺: m/z = 617.2; found: 617.2.

**Example 39c.** Diastereomers 3. Peak 3. LCMS calculated for C₃₃H₃₅ClFN₆O₃ (M+H)⁺: m/z = 617.2; found: 617.2.

### Example 40a, Example 40b, and Example 40c. 2-(1-acryloyl-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)piperidin-2-yl)acetonitrile

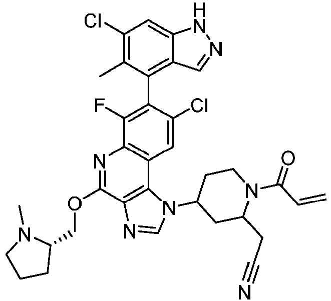

This compound was prepared according to the procedure described in **Example 36, step 12,** replacing 4-(4,4,5,5-tetramethyl-1,3,2-dioxaborolan-2-yl)naphthalen-2-ol with 6-chloro-5-methyl-1-(tetrahydro-2H-pyran-2-yl)-4-(4,4,5,5-tetramethyl-1,3,2-dioxaborolan-2-yl)-1H-indazole. The products were isolated as either a pair of enantiomers or a mixture of diastereomers. LCMS calculated for C₃₄H₃₄ClFN₈O₂ (M+H)⁺: m/z = 675.2; found: 675.2.

**Example 40a.** Diastereomers 1. Peak 1. LCMS calculated for C₃₄H₃₄ClFN₈O₂ (M+H)⁺: m/z = 675.2; found: 675.2.

**Example 40b.** Diastereomers 2. Peak 2. LCMS calculated for C₃₄H₃₄ClFN₈O₂ (M+H)⁺: m/z = 675.2; found: 675.2.

**Example 40c.** Diastereomers 3. Peak 3. LCMS calculated for C₃₄H₃₄ClFN₈O₂ (M+H)⁺: m/z = 675.2; found: 675.2.

### Example 41. 2-(1-acryloyl-4-(8-chloro-7-(3-chloro-2-methylphenyl)-6-fluoro-4-(((S)-1-methylpyrrolidin-2-yl)methoxy)-1H-imidazo[4,5-c]quinolin-1-yl)piperidin-2-yl)acetonitrile

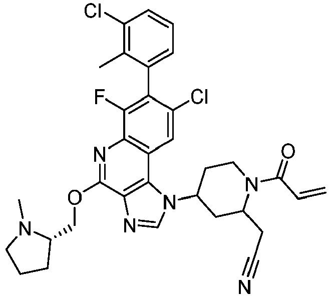

This compound was prepared according to the procedure described in **Example 36, step 12,** replacing 4-(4,4,5,5-tetramethyl-1,3,2-dioxaborolan-2-yl)naphthalen-2-ol with (3-chloro-2-methylphenyl)boronic acid. The product was isolated as a mixture of diastereomers. LCMS calculated for C₃₃H₃₄Cl₂FN₆O₂ (M+H)⁺: m/z = 635.2; found: 635.2.

### Example 42. 2-(1-acryloyl-4-(8-chloro-6-fluoro-7-(3-fluoro-2-methylphenyl)-4-(((S)-1-methylpyrrolidin-2-yl)methoxy)-1H-imidazo[4,5-c]quinolin-1-yl)piperidin-2-yl)acetonitrile

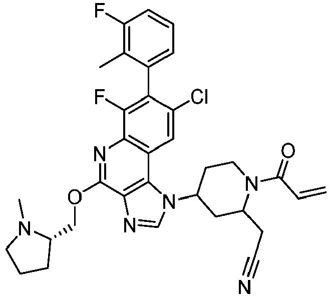

This compound was prepared according to the procedure described in **Example 36, step 12,** replacing 4-(4,4,5,5-tetramethyl-1,3,2-dioxaborolan-2-yl)naphthalen-2-ol with (3-fluoro-2-methylphenyl)boronic acid. The product was isolated as a mixture of diastereomers. LCMS calculated for C₃₃H₃₄ClF₂N₆O₂ (M+H)⁺: m/z = 619.2; found: 619.2.

### Example 43. 2-(1-acryloyl-4-(8-chloro-7-(2,3-dimethylphenyl)-6-fluoro-4-(((S)-1-methylpyrrolidin-2-yl)methoxy)-1H-imidazo[4,5-c]quinolin-1-yl)piperidin-2-yl)acetonitrile

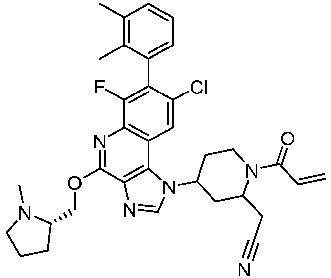

This compound was prepared according to the procedure described in **Example 36, step 12,** replacing 4-(4,4,5,5-tetramethyl-1,3,2-dioxaborolan-2-yl)naphthalen-2-ol with (2,3-dimethylphenyl)boronic acid. The product was isolated as a mixture of diastereomers. LCMS calculated for C₃₄H₃₇ClFN₆O₂ (M+H)⁺: m/z = 615.3; found: 615.3.

### Example 44. 2-(1-acryloyl-4-(8-chloro-7-(2,3-dihydro-1H-inden-4-yl)-6-fluoro-4-(((S)-1-methylpyrrolidin-2-yl)methoxy)-1H-imidazo[4,5-c]quinolin-1-yl)piperidin-2-yl)acetonitrile

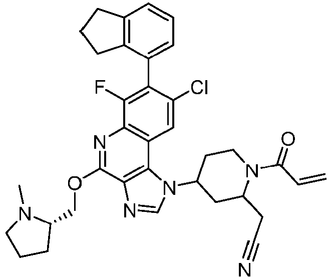

This compound was prepared according to the procedure described in **Example 36, step 12,** replacing 4-(4,4,5,5-tetramethyl-1,3,2-dioxaborolan-2-yl)naphthalen-2-ol with (2,3-dihydro-1H-inden-4-yl)boronic acid. The product was isolated as a mixture of diastereomers. LCMS calculated for C₃₅H₃₇ClFN₆O₂ (M+H)⁺: m/z = 627.3; found: 627.3.

### Example 45. 2-((2S,4S)-1-acryloyl-4-(8-chloro-4-(3-(dimethylamino)azetidin-1-yl)-6-fluoro-7-phenyl-1H-imidazo[4,5-c]quinolin-1-yl)piperidin-2-yl)acetonitrile

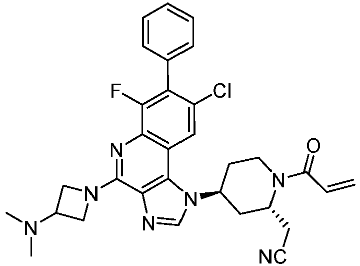

This compound was prepared according to the procedure described in **Example 16,** replacing (2,3-dimethylphenyl)boronic acid with phenylboronic acid in **Step** 6. LCMS calculated for C₃₁H₃₂ClFN₇O (M+H)⁺: m/z = 572.2; found 572.2.

### Example 46. 2-((2S,4S)-1-acryloyl-4-(8-chloro-4-(3-(dimethylamino)azetidin-1-yl)-6-fluoro-7-(naphthalen-1-yl)-1H-imidazo[4,5-c]quinolin-1-yl)piperidin-2-yl)acetonitrile

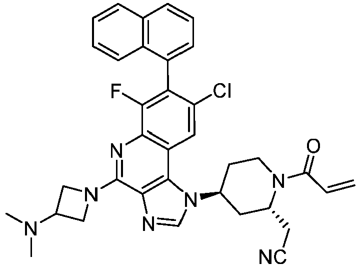

This compound was prepared according to the procedure described in **Example 16,** replacing (2,3-dimethylphenyl)boronic acid with 1-Naphthylboronic acid in **Step 6.** The product was isolated as a mixture of diastereomers. LCMS calculated for C₃₅H₃₄ClFN₇O (M+H)⁺: m/z = 622.2; found 622.2.

### Example 47a and 47b. 2-((2S,4S)-1-acryloyl-4-(8-chloro-4-(3-(dimethylamino)azetidin-1-yl)-6-fluoro-7-(3-hydroxynaphthalen-1-yl)-1H-imidazo[4,5-c]quinolin-1-yl)piperidin-2-yl)acetonitrile

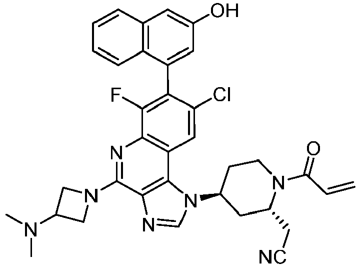

This compound was prepared according to the procedure described in **Example 16,** replacing (2,3-dimethylphenyl)boronic acid with 4-(4,4,5,5-tetramethyl-1,3,2-dioxaborolan-2-yl)naphthalen-2-ol in **Step 6.**

**Example 47a.** Diastereomer 1. Peak 1. LCMS calculated for C₃₅H₃₄ClFN₇O₂ (M+H)⁺: m/z = 638.2; found 638.2.

**Example 47b.** Diastereomer 2. Peak 2. LCMS calculated for C₃₅H₃₄ClFN₇O₂ (M+H)⁺: m/z = 638.2; found 638.2.

### Example 48a and Example 48b. 4-(1-((endo)-2-azabicyclo[2.1.1]hexan-5-yl)-8-chloro-4-(3-(dimethylamino)azetidin-1-yl)-6-fluoro-1H-imidazo[4,5-c]quinolin-7-yl)naphthalen-2-ol

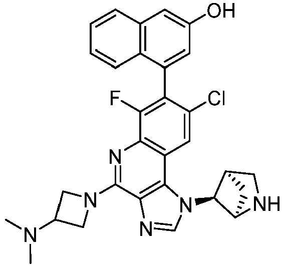

A screw-cap vial equipped with a magnetic stir bar was charged with *tert*-butyl (*endo*)-5-(7-bromo-8-chloro-4-(3-(dimethylamino)azetidin-1-yl)-6-fluoro-1*H*-imidazo[4,5-*c*]quinolin-1-yl)-2-azabicyclo[2.1.1]hexane-2-carboxylate **(Example 23a and Example 23b, step 1,** 92 mg, 0.159 mmol), 4-(4,4,5,5-tetramethyl-1,3,2-dioxaborolan-2-yl)naphthalen-2-ol (64.3 mg, 0.238 mmol), Pd(Ph₃P)₄ (18.3 mg, 0.016 mmol), sodium carbonate (50.4 mg, 0.476 mmol), and dioxane (0.8 mL)/water (0.2 mL). The reaction mixture was heated to 100 °C for 2.5 h. After cooling to r.t., the reaction mixture was diluted with CH₂Cl₂ and washed with sat'd NaHCO₃. The combined organic layers were dried over MgSO₄, filtered, and concentrated. The concentrated residue was dissolved in TFA (0.5 mL) and stirred at r.t. for 15 min. The mixture diluted with MeOH and purified using prep-LCMS (XBridge C18 column, eluting with a gradient of acetonitrile/water containing 0.1% TFA, at flow rate of 60 mL/min) to afford the desired product as a TFA salt. The products were isolated as pairs of enantiomers.

**Example 48a.** Diastereomer 1. Peak 1. LCMS calculated for C₃₀H₂₉ClFN₆O (M+H)⁺: m/z = 543.2; found 543.3.

**Example 48b.** Diastereomer 2. Peak 2. LCMS calculated for C₃₀H₂₉ClFN₆O (M+H)⁺: m/z = 543.2; found 543.3.

### Example 49. 4-(1-((endo)-2-azabicyclo[2.1.1]hexan-5-yl)-8-chloro-4-(3-(dimethylamino)-azetidin-1-yl)-2-((dimethylamino)methyl)-6-fluoro-1H-imidazo[4,5-c]quinolin-7-yl)naphthalen-2-ol

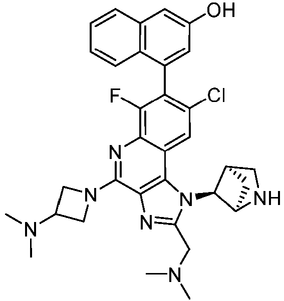

### Step 1. tert-butyl (endo)-5-(7-bromo-8-chloro-2-(chloromethyl)-4-(3-(dimethylamino)azetidin-1-yl)-6-fluoro-1H-imidazo[4,5-c]quinolin-1-yl)-2-azabicyclo[2.1.1]hexane-2-carboxylate

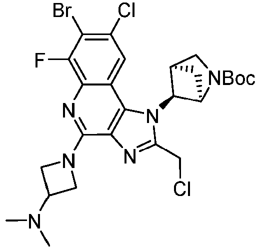

This compound was prepared according to the procedure described in **Example 23a and Example 23b, step 1,** replacing triethyl orthoformate with 2-chloro-1,1,1-trimethoxyethane. LCMS calculated for C₂₆H₃₁BrCl₂FN₆O₂ (M+H)⁺: m/z = 627.1; found 627.2.

### Step 2. tert-butyl (endo)-5-(7-bromo-8-chloro-4-(3-(dimethylamino)azetidin-1-yl)-2-((dimethylamino)methyl)-6-fluoro-1H-imidazo[4,5-c]quinolin-1-yl)-2-azabicyclo[2.1.1]hexane-2-carboxylate

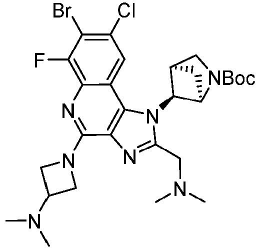

To a solution of *tert*-butyl (endo)-5-(7-bromo-8-chloro-2-(chloromethyl)-4-(3-(dimethylamino)azetidin-1-yl)-6-fluoro-1*H*-imidazo[4,5-c]quinolin-1-yl)-2-azabicyclo[2.1.1]hexane-2-carboxylate (25 mg, 0.04 mmol) in acetonitrile (0.5 mL) was added dimethylamine (2.0 M solution in THF, 0.1 mL, 0.2 mmol). The reaction mixture was heated at 50 °C for 4 h. The mixture was then concentrated to dryness and used without further purification. LCMS calculated for C₂₈H₃₇BrClFN₇O₂ (M+H)⁺: m/z = 638.2; found 638.2.

### Step 3. 4-(1-((endo)-2-azabicyclo[2.1. 1]hexan-5-yl)-8-chloro-4-(3-(dimethylamino)azetidin-1-yl)-2-((dimethylamino)methyl)-6-fluoro-1H-imidazo[4,5-c]quinolin-7-yl)naphthalen-2-ol

This compound was prepared according to the procedure described in **Example 48,** replacing *tert*-butyl (*endo*)-5-(7-bromo-8-chloro-4-(3-(dimethylamino)azetidin-1-yl)-6-fluoro-1*H*-imidazo[4,5-c]quinolin-1-yl)-2-azabicyclo[2.1.1]hexane-2-carboxylate with tert-butyl (*endo*)-5-(7-bromo-8-chloro-4-(3-(dimethylamino)azetidin-1-yl)-2-((dimethylamino)methyl)-6-fluoro-1*H*-imidazo[4,5-*c*]quinolin-1-yl)-2-azabicyclo[2.1.1]hexane-2-carboxylate. The product was isolated as a mixture of diastereomers. LCMS calculated for C₃₃H₃₆ClFN₇O (M+H)⁺: m/z = 600.3; found 600.2.

### Example 50. 1-((endo)-2-azabicyclo[2.1.1]hexan-5-yl)-4-(3-(dimethylamino)azetidin-1-yl)-6-fluoro-7-(3-hydroxynaphthalen-1-yl)-1H-imidazo[4,5-c]quinoline-8-carbonitrile

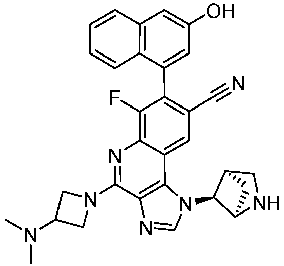

### Example 51. 4-(1-((endo)-2-azabicyclo[2.1.1]hexan-5-yl)-4-(3-(dimethylamino)azetidin-1-yl)-6-fluoro-1H-imidazo[4,5-c]quinolin-7-yl)naphthalen-2-ol

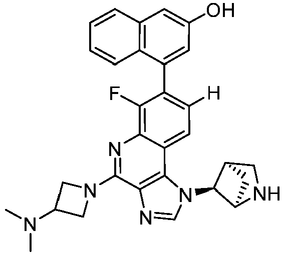

A 1 dram vial was charged with 4-(1-((*endo*)-2-azabicyclo[2.1.1]hexan-5-yl)-8-chloro-4-(3-(dimethylamino)azetidin-1-yl)-6-fluoro-1*H*-imidazo[4,5-*c*]quinolin-7-yl)naphthalen-2-ol **(Example 48, 2** mg, 3.68 µmol), potassium ferrocyanide(II) hydrate (1.87 mg, 4.42 µmol), potassium acetate (0.361 mg, 3.68 µmol), tBuXPhos Pd G3 (0.3 mg, 0.4 µmol), and 1,4-dioxane (0.3 mL)/water (0.2 mL). The reaction mixture was heated at 100 °C for 2 h, then cooled to r.t. and diluted with MeOH. The mixture was purified using prep-LCMS (XBridge C18 column, eluting with a gradient of acetonitrile/water containing 0.1% TFA, at flow rate of 60 mL/min) to afford the desired products as a TFA salt. The products were isolated as pairs of enantiomers.

**Example 50.** LCMS calculated for C₃₀H₂₉ClFN₆O (M+H)⁺: m/z = C₃₁H₂₉FN₇O (M+H)⁺: m/z = 534.2; found 534.2.

**Example 51.** LCMS calculated for C₃₀H₃₀FN₆O (M+H)⁺: m/z = 509.2; found 509.2.

### Example 52a and 52b. 2-((2S,4S)-1-acryloyl-4-(8-chloro-4-(3-(dimethylamino)azetidin-1-yl)-6-fluoro-7-(5-hydroxy-2,3-dimethylphenyl)-1H-imidazo[4,5-c]quinolin-1-yl)piperidin-2-yl)acetonitrile

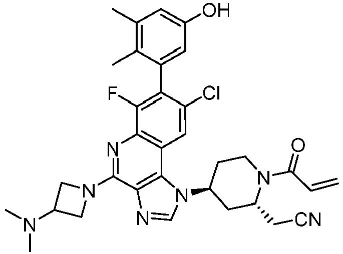

This compound was prepared according to the procedure described in **Example 16,** replacing (2,3-dimethylphenyl)boronic acid with 3,4-dimethyl-5-(4,4,5,5-tetramethyl-1,3,2-dioxaborolan-2-yl)phenol in **Step 6.**

**Example 52a.** Diastereomer 1. Peak 1. LCMS calculated for C₃₃H₃₆ClFN₇O₂ (M+H)⁺ m/z = 616.3; found 616.4.

**Example 52b.** Diastereomer 2. Peak 2. LCMS calculated for C₃₃H₃₆ClFN₇O₂ (M+H)⁺ m/z = 616.3; found 616.4.

### Example 53a and 53b. 2-((2S,4S)-1-acryloyl-4-(8-chloro-4-(3-(dimethylamino)azetidin-1-yl)-6-fluoro-7-(5-hydroxy-2-methylphenyl)-1H-imidazo[4,5-c]quinolin-1-yl)piperidin-2-yl)acetonitrile

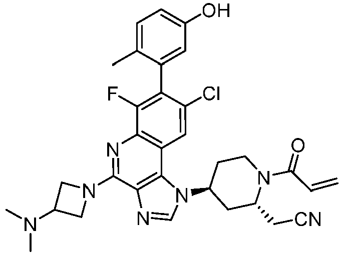

This compound was prepared according to the procedure described in **Example 16,** replacing (2,3-dimethylphenyl)boronic acid with (5-hydroxy-2-methylphenyl)boronic acid **Step 6.**

**Example 53a.** Diastereomer 1. Peak 1. LCMS calculated for C₃₂H₃₄ClFN₇O₂ (M+H)⁺ m/z = 602.2; found 602.3.

**Example 53b.** Diastereomer 2. Peak 1. LCMS calculated for C₃₂H₃₄ClFN₇O₂ (M+H)⁺ m/z = 602.2; found 602.3.

### Example 54a and 54b. 2-((2S,4S)-1-acryloyl-4-(8-chloro-7-(2-chloro-5-hydroxyphenyl)-4-(3-(dimethylamino)azetidin-1-yl)-6-fluoro-1H-imidazo[4,5-c]quinolin-1-yl)piperidin-2-yl)acetonitrile

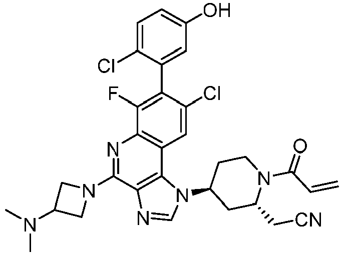

This compound was prepared according to the procedure described in **Example 16,** replacing (2,3-dimethylphenyl)boronic acid with (2-chloro-5-hydroxyphenyl)boronic acid in **Step 6.**

**Example 54a.** Diastereomer 1. Peak 1. LCMS calculated for C₃₁H₃₁Cl₂FN₇O₂ (M+H)⁺ m/z = 622.2; found 622.3.

**Example 54b.** Diastereomer 2. Peak 1. LCMS calculated for C₃₁H₃₁Cl₂FN₇O₂ (M+H)⁺ m/z = 622.2; found 622.3.

### Example 55. 2-((2S,4S)-1-acryloyl-4-(8-chloro-4-(3-(dimethylamino)azetidin-1-yl)-6-fluoro-7-(2-fluoro-5-hydroxyphenyl)-1H-imidazo[4,5-c]quinolin-1-yl)piperidin-2-yl)acetonitrile

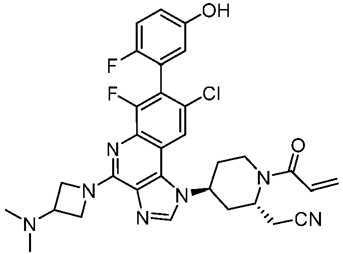

This compound was prepared according to the procedure described in **Example 16,** replacing (2,3-dimethylphenyl)boronic acid with (2-fluoro-5-hydroxyphenyl)boronic acid in **Step 6.** The product was isolated as a mixture of diastereomers. LCMS calculated for C₃₁H₃₁ClF₂N₇O₂ (M+H)⁺ m/z = 606.2; found 606.3.

### Example 56. 2-((2S,4S)-1-acryloyl-4-(8-chloro-4-(3-(dimethylamino)azetidin-1-yl)-6-fluoro-7-(2-fluorophenyl)-1H-imidazo[4,5-c]quinolin-1-yl)piperidin-2-yl)acetonitrile

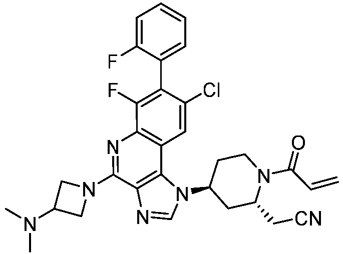

This compound was prepared according to the procedure described in **Example 16,** replacing (2,3-dimethylphenyl)boronic acid with (2-fluorophenyl)boronic acid in **Step 6.** The product was isolated as a mixture of diastereomers. LCMS calculated for C₃₁H₃₁ClF₂N₇O (M+H)⁺ m/z = 590.2; found 590.2.

### Example 57. 2-((2S,4S)-1-acryloyl-4-(8-chloro-7-(2-chlorophenyl)-4-(3-(dimethylamino)-azetidin-1-yl)-6-fluoro-1H-imidazo[4,5-c]quinolin-1-yl)piperidin-2-yl)acetonitrile

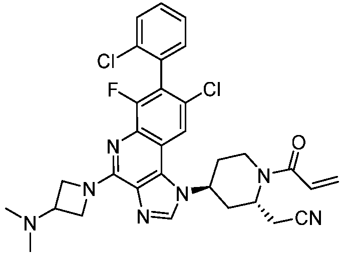

This compound was prepared according to the procedure described in **Example 16,** replacing (2,3-dimethylphenyl)boronic acid with (2-chlorophenyl)boronic acid in **Step 6.** The product was isolated as a mixture of diastereomers. LCMS calculated for C₃₁H₃₁Cl₂FN₇O (M+H)⁺ m/z = 606.2; found 606.2.

### Example 58. 2-((2S,4S)-1-acryloyl-4-(8-chloro-7-(2-chloro-3-fluorophenyl)-4-(3-(dimethyl-amino)azetidin-1-yl)-6-fluoro-1H-imidazo[4,5-c]quinolin-1-yl)piperidin-2-yl)acetonitrile

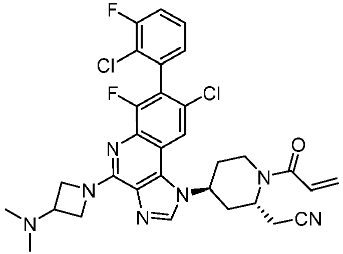

This compound was prepared according to the procedure described in **Example 16,** replacing (2,3-dimethylphenyl)boronic acid with (2-chloro-3-fluorophenyl)boronic acid in **Step 6.** The product was isolated as a mixture of diastereomers. LCMS calculated for C₃₁H₃₀Cl₂F₂N₇O (M+H)⁺ m/z = 624.2; found 624.2.

### Example 59. 2-((2S,4S)-1-acryloyl-4-(8-chloro-4-(3-(dimethylamino)azetidin-1-yl)-7-(2,4-dimethylphenyl)-6-fluoro-1H-imidazo[4,5-c]quinolin-1-yl)piperidin-2-yl)acetonitrile

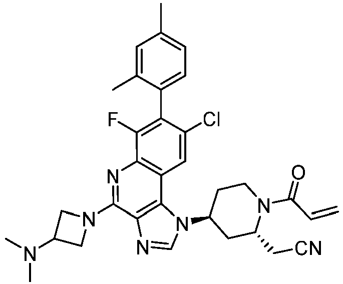

This compound was prepared according to the procedure described in **Example 16,** replacing (2,3-dimethylphenyl)boronic acid with (2,4-dimethylphenyl)boronic acid in **Step 6.** The product was isolated as a mixture of diastereomers. LCMS calculated for C₃₃H₃₆ClFN₇O (M+H)⁺ m/z = 600.3; found 600.3.

### Example 60. 2-((2S,4S)-1-acryloyl-4-(8-chloro-4-(3-(dimethylamino)azetidin-1-yl)-7-(2,5-dimethylphenyl)-6-fluoro-1H-imidazo[4,5-c]quinolin-1-yl)piperidin-2-yl)acetonitrile

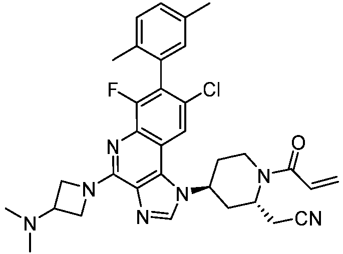

This compound was prepared according to the procedure described in **Example 16,** replacing (2,3-dimethylphenyl)boronic acid with (2,5-dimethylphenyl)boronic acid in **Step 6.** The product was isolated as a mixture of diastereomers. LCMS calculated for C₃₃H₃₆ClFN₇O (M+H)⁺ m/z = 600.3; found 600.3

### Example 61. 2-((2S,4S)-1-acryloyl-4-(8-chloro-7-(3-chloro-2-methylphenyl)-4-(3-(dimethyl-amino)azetidin-1-yl)-6-fluoro-1H-imidazo[4,5-c]quinolin-1-yl)piperidin-2-yl)acetonitrile

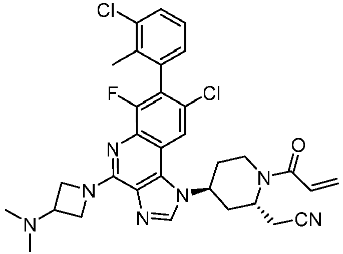

This compound was prepared according to the procedure described in **Example 16,** replacing (2,3-dimethylphenyl)boronic acid with (3-chloro-2-methylphenyl)boronic acid in **Step 6.** The product was isolated as a mixture of diastereomers. LCMS calculated for C₃₂H₃₃Cl₂FN₇O (M+H)⁺ m/z = 620.2 ; found 620.3

### Example 62. 2-((2R,4R)-1-acryloyl-4-(8-chloro-4-(3-(dimethylamino)azetidin-1-yl)-7-(2,3-dimethylphenyl)-6-fluoro-1H-imidazo[4,5-c]quinolin-1-yl)pyrrolidin-2-yl)acetonitrile

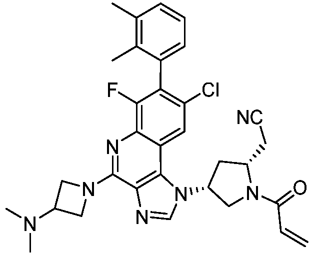

This compound was prepared according to the procedure described in **Example 32,** replacing 4-(4,4,5,5-tetramethyl-1,3,2-dioxaborolan-2-yl)naphthalen-2-ol with (2,3-dimethylphenyl)boronic acid in **Step 7.** The product was isolated as a mixture of diastereomers. LCMS calculated for C₃₂H₃₄ClFN₇O (M+H)⁺ m/z = 586.3; found 586.3.

### Example 63. 2-((2R,4R)-1-acryloyl-4-(8-chloro-4-(3-(dimethylamino)azetidin-1-yl)-6-fluoro-7-(naphthalen-1-yl)-1H-imidazo[4,5-c]quinolin-1-yl)pyrrolidin-2-yl)acetonitrile

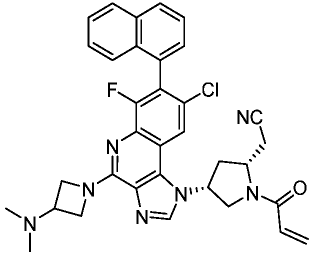

This compound was prepared according to the procedure described in **Example 32,** replacing 4-(4,4,5,5-tetramethyl-1,3,2-dioxaborolan-2-yl)naphthalen-2-ol with 4,4,5,5-tetramethyl-2-(naphthalen-1-yl)-1,3,2-dioxaborolane in **Step 7.** The product was isolated as a mixture of diastereomers. LCMS calculated for C₃₄H₃₂ClFN₇O (M+H)⁺ m/z = 608.2; found 608.2.

### Example 64a and 64b. 2-((2S,4S)-1-acryloyl-4-(8-chloro-4-(3-(dimethylamino)azetidin-1-yl)-6-fluoro-7-(5-fluoroquinolin-8-yl)-1H-imidazo[4,5-c]quinolin-1-yl)piperidin-2-yl)acetonitrile

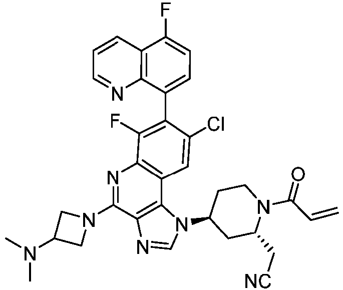

This compound was prepared according to the procedure described in **Example 16,** replacing (2,3-dimethylphenyl)boronic acid with (5-fluoroquinolin-8-yl)boronic acid in **Step 6.**

**Example 64a.** Diastereomer 1. Peak 1. LCMS calculated for C₃₄H₃₂ClF₂N₈O (M+H)⁺ m/z = 641.2; found 641.2.

**Example 64b.** Diastereomer 2. Peak 2. LCMS calculated for C₃₄H₃₂ClF₂N₈O (M+H)⁺ m/z = 641.2; found 641.2.

### Example 65. 2-((2S,4S)-1-acryloyl-4-(8-chloro-7-(chroman-8-yl)-4-(3-(dimethylamino)-azetidin-1-yl)-6-fluoro-1H-imidazo[4,5-c]quinolin-1-yl)piperidin-2-yl)acetonitrile

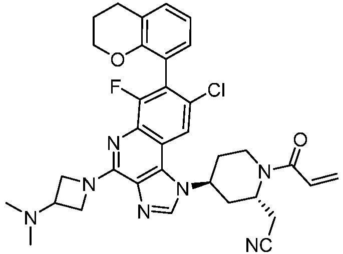

This compound was prepared according to the procedure described in **Example 16,** replacing (2,3-dimethylphenyl)boronic acid with chroman-8-ylboronic acid in **Step 6.** The product was isolated as a mixture of diastereomers. LCMS calculated for C₃₄H₃₆ClFN₇O₂ (M+H)⁺ m/z = 628.2; found 628.2.

### Example 66. 2-((2S,4S)-1-acryloyl-4-(8-chloro-4-(3-(dimethylamino)azetidin-1-yl)-6-fluoro-7-(2-methoxynaphthalen-1-yl)-1H-imidazo[4,5-c]quinolin-1-yl)piperidin-2-yl)acetonitrile

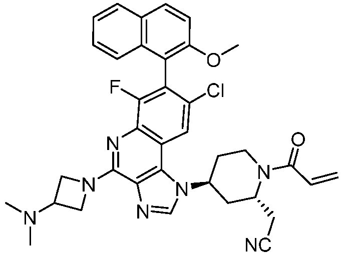

This compound was prepared according to the procedure described in **Example 16,** replacing (2,3-dimethylphenyl)boronic acid with (2-methoxynaphthalen-1-yl)boronic acid in **Step 6.** The product was isolated as a mixture of diastereomers. LCMS calculated for C₃₆H₃₆ClFN₇O₂ (M+H)⁺ m/z = 652.2; found 652.2.

### Example 67. 2-((2S,4S)-1-acryloyl-4-(8-chloro-4-(3-(dimethylamino)azetidin-1-yl)-6-fluoro-7-(4-fluoronaphthalen-1-yl)-1H-imidazo[4,5-c]quinolin-1-yl)piperidin-2-yl)acetonitrile

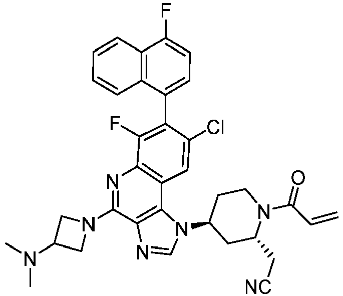

This compound was prepared according to the procedure described in **Example 16,** replacing (2,3-dimethylphenyl)boronic acid with (4-fluoronaphthalen-1-yl)boronic acid in **Step 6.** The product was isolated as a mixture of diastereomers. LCMS calculated for C₃₅H₃₃ClF₂N₇O (M+H)⁺ m/z = 640.2; found 640.2.

### Example 68. 2-((2S,4S)-1-acryloyl-4-(8-chloro-4-(3-(dimethylamino)azetidin-1-yl)-6-fluoro-7-(8-methylnaphthalen-1-yl)-1H-imidazo[4,5-c]quinolin-1-yl)piperidin-2-yl)acetonitrile

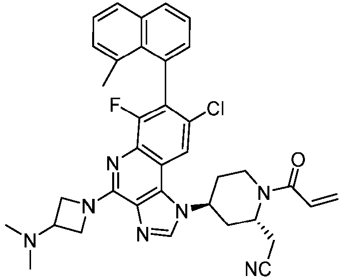

This compound was prepared according to the procedure described in **Example 16,** replacing (2,3-dimethylphenyl)boronic acid with (8-methylnaphthalen-1-yl)boronic acid in **Step 6.** The product was isolated as a mixture of diastereomers. LCMS calculated for C₃₆H₃₆ClFN₇O (M+H)⁺ m/z = 636.3; found 636.3.

### Example 69. 2-((2S,4S)-1-acryloyl-4-(8-chloro-7-(2,3-dihydrobenzo[b][1,4]dioxin-5-yl)-4-(3-(dimethylamino)azetidin-1-yl)-6-fluoro-1H-imidazo[4,5-c]quinolin-1-yl)piperidin-2-yl)acetonitrile

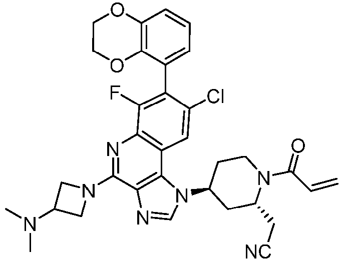

This compound was prepared according to the procedure described in **Example 16,** replacing (2,3-dimethylphenyl)boronic acid with (2,3-dihydrobenzo[b][1,4]dioxin-5-yl)boronic acid in **Step 6.** The product was isolated as a mixture of diastereomers. LCMS calculated for C₃₃H₃₄ClFN₇O₃ (M+H)⁺ m/z = 630.2; found 630.2.

### Example 70a and 70b. 2-((2S,4S)-1-acryloyl-4-(8-chloro-4-(3-(dimethylamino)azetidin-1-yl)-6-fluoro-7-(isoquinolin-5-yl)-1H-imidazo[4,5-c]quinolin-1-yl)piperidin-2-yl)acetonitrile

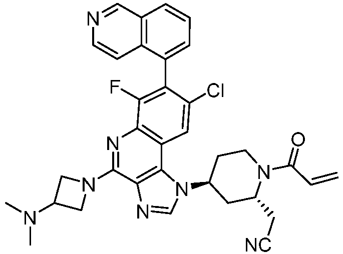

This compound was prepared according to the procedure described in **Example 16,** replacing (2,3-dimethylphenyl)boronic acid with isoquinolin-5-ylboronic acid in **Step 6.**

**Example 70a.** Diastereomer 1. Peak 1. LCMS calculated for C₃₄H₃₃ClFN₈O (M+H)⁺ m/z = 623.2; found 623.2.

**Example 70b.** Diastereomer 2. Peak 2. LCMS calculated for C₃₄H₃₃ClFN₈O (M+H)⁺ m/z = 623.2; found 623.2.

### Example 71a and 71b. 2-((2S,4S)-1-acryloyl-4-(8-chloro-4-(3-(dimethylamino)azetidin-1-yl)-6-fluoro-7-(isoquinolin-8-yl)-1H-imidazo[4,5-c]quinolin-1-yl)piperidin-2-yl)acetonitrile

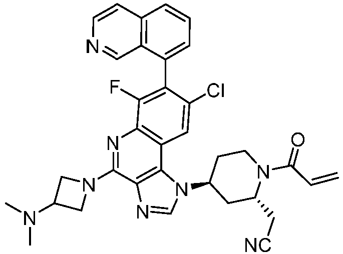

This compound was prepared according to the procedure described in **Example 16,** replacing (2,3-dimethylphenyl)boronic acid with isoquinolin-8-ylboronic acid in **Step 6.**

**Example 71a.** Diastereomer 1. Peak 1. LCMS calculated for C₃₄H₃₃ClFN₈O (M+H)⁺ m/z = 623.2; found 623.2.

**Example 71b.** Diastereomer 2. Peak 2. LCMS calculated for C₃₄H₃₃ClFN₈O (M+H)⁺ m/z = 623.2; found 623.2.

### Example 72a and 72b. 2-((2S,4S)-1-acryloyl-4-(8-chloro-4-(3-(dimethylamino)azetidin-1-yl)-6-fluoro-7-(quinolin-8-yl)-1H-imidazo[4,5-c]quinolin-1-yl)piperidin-2-yl)acetonitrile

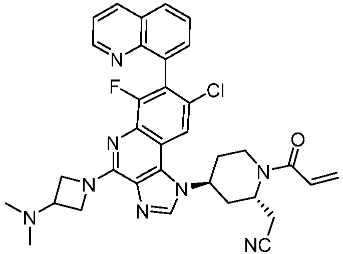

This compound was prepared according to the procedure described in **Example 16,** replacing (2,3-dimethylphenyl)boronic acid with quinolin-8-ylboronic acid in **Step 6.**

**Example 72a.** Diastereomer 1. Peak 1. LCMS calculated for C₃₄H₃₃ClFN₈O (M+H)⁺: m/z = 623.2; found 623.2.

**Example 72b.** Diastereomer 2. Peak 2. LCMS calculated for C₃₄H₃₃ClFN₈O (M+H)⁺: m/z = 623.2; found 623.2.

### Example 73a and 73b. 2-((2S,4S)-1-acryloyl-4-(8-chloro-4-(3-(dimethylamino)azetidin-1-yl)-6-fluoro-7-(isoquinolin-4-yl)-1H-imidazo[4,5-c]quinolin-1-yl)piperidin-2-yl)acetonitrile

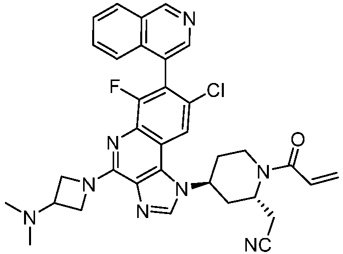

This compound was prepared according to the procedure described in **Example 16,** replacing (2,3-dimethylphenyl)boronic acid with isoquinolin-4-ylboronic acid in **Step 6.**

**Example 73a.** Diastereomer 1. Peak 1. LCMS calculated for C₃₄H₃₃ClFN₈O (M+H)⁺: m/z = 623.2; found 623.2.

**Example 73b.** Diastereomer 2. Peak 2. LCMS calculated for C₃₄H₃₃ClFN₈O (M+H)⁺: m/z = 623.2; found 623.2.

### Example 74a and 74b. 2-((2S,4S)-1-acryloyl-4-(8-chloro-4-(3-(dimethylamino)azetidin-1-yl)-6-fluoro-7-(quinolin-4-yl)-1H-imidazo[4,5-c]quinolin-1-yl)piperidin-2-yl)acetonitrile

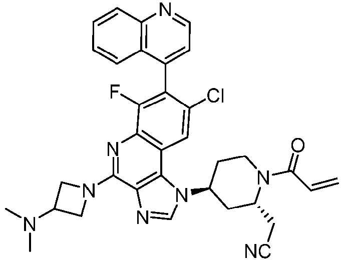

This compound was prepared according to the procedure described in **Example 16,** replacing (2,3-dimethylphenyl)boronic acid with quinolin-4-ylboronic acid in **Step 6.**

**Example 74a.** Diastereomer 1. Peak 1. LCMS calculated for C₃₄H₃₃ClFN₈O (M+H)⁺: m/z = 623.2; found 623.2.

**Example 74b.** Diastereomer 2. Peak 2. LCMS calculated for C₃₄H₃₃ClFN₈O (M+H)⁺: m/z = 623.2; found 623.2.

### Example 75. 4-(1-((endo)-2-azabicyclo[2.1.1]hexan-5-yl)-8-chloro-4-(3-(dimethylamino)-azetidin-1-yl)-6-fluoro-2-(hydroxymethyl)-1H-imidazo[4,5-c]quinolin-7-yl)naphthalen-2-ol

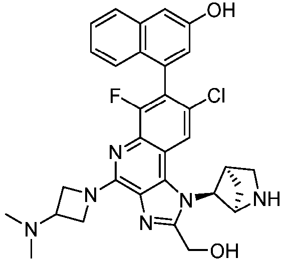

### Step 1. 4-(4-(((endo)-2-azabicyclo[2.1.1]hexan-5-yl)amino)-3-amino-6-chloro-2-(3-(dimethylamino)azetidin-1-yl)-8-fluoroquinolin-7-yl)naphthalen-2-ol

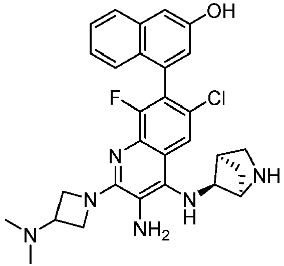

To a solution of *tert*-butyl (*endo*)-5-((3-amino-7-bromo-6-chloro-2-(3-(dimethylamino)-azetidin-1-yl)-8-fluoroquinolin-4-yl)amino)-2-azabicyclo[2.1.1]hexane-2-carboxylate **(Example 15, step 2,** 338 mg, 0.593 mmol) in dioxane (2.4 mL)/water (0.6 mL) was added 4-(4,4,5,5-tetramethyl-1,3,2-dioxaborolan-2-yl)naphthalen-2-ol (352 mg, 1.30 mmol), Pd(Ph₃P)₄ (69 mg, 5.9 µmol) and sodium carbonate (189 mg, 1.78 mmol). The reaction flask was evacuated, back filled with nitrogen, and then stirred at 100 °C for 5 h. HCl (4 N in dioxane, 5 mL) was added and the mixture was stirred at 60 °C for 30 min. The mixture was then diluted with MeOH/water/TFA and purified by prep-LCMS (XBridge C18 column, eluting with a gradient of acetonitrile/water containing 0.1% TFA, at flow rate of 60 mL/min) to give the desired product as a TFA salt. The product was isolated as a mixture of diastereomers. LCMS calculated for C₂₉H₃₁ClFN₆O (M+H)⁺: m/z = 533.2; found 533.2.

### Step 2. 4-(1-((endo)-2-azabicyclo[2.1.1]hexan-5-yl)-8-chloro-4-(3-(dimethylamino)azetidin-1-yl)-6-fluoro-2-(hydroxymethyl)-1H-imidazo[4,5-c]quinolin-7-yl)naphthalen-2-ol

A screw-cap vial equipped with a magnetic stir bar was charged 4-(4-(((*endo*)-2-azabicyclo[2.1.1]hexan-5-yl)amino)-3-amino-6-chloro-2-(3-(dimethylamino)azetidin-1-yl)-8-fluoro-quinolin-7-yl)naphthalen-2-ol (5.0 mg, 9.4 µmol) and 2-((*tert*butyldimethylsilyl)oxy)acetaldehyde (1.6 mg, 9.4 µmol) followed by ethanol (0.5 ml). The reaction was stirred for 18 h, then was treated with conc. HCl (50 µL). After another 4 h, the mixture was diluted with acetonitrile/water and purified using prep-LCMS (XBridge C18 column, eluting with a gradient of acetonitrile/water containing 0.1% TFA, at flow rate of 60 mL/min) to afford the desired products as a TFA salt. The product was isolated as a mixture of diastereomers. LCMS calculated for C₃₁H₃₁ClFN₆O₂ (M+H)⁺ m/z = 573.2; found 573.2.

### Example 76. 4-(2-(2-aminoethyl)-1-((endo)-2-azabicyclo[2.1.1]hexan-5-yl)-8-chloro-4-(3-(dimethylamino)azetidin-1-yl)-6-fluoro-1H-imidazo[4,5-c]quinolin-7-yl)naphthalen-2-ol

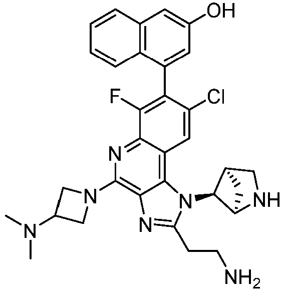

This compound was prepared according to the procedure described in **Example 77,** replacing 2-((*tert*-butyldimethylsilyl)oxy)acetaldehyde with tert-butyl (3-oxopropyl)carbamate in **Step 2.** The product was isolated as a mixture of diastereomers. LCMS calculated for C₃₂H₃₄ClFN₇O (M+H)⁺ m/z = 586.2; found 586.2.

### Example 77. 4-(1-((endo)-2-azabicyclo[2.1.1]hexan-5-yl)-8-chloro-4-(3-(dimethylamino)azetidin-1-yl)-6-fluoro-2-(piperidin-4-ylmethyl)-1H-imidazo[4,5-c]quinolin-7-yl)naphthalen-2-ol

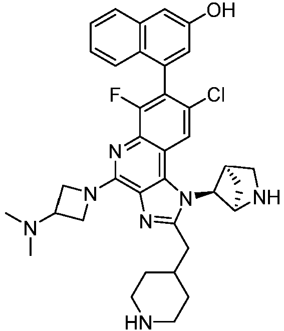

This compound was prepared according to the procedure described in **Example 77,** replacing 2-((*tert*-butyldimethylsilyl)oxy)acetaldehyde with tert-butyl 4-(2-oxoethyl)piperidine-1-carboxylate in **Step 2.** The product was isolated as a mixture of diastereomers. LCMS calculated for C₃₆H₄₀ClFN₇O (M+H)⁺ m/z = 640.3; found 640.2.

### Example 78. 4-(1-((endo)-2-azabicyclo[2.1.1]hexan-5-yl)-8-chloro-4-(3-(dimethylamino)-azetidin-1-yl)-6-fluoro-2-(1-methyl-1H-imidazol-4-yl)-1H-imidazo[4,5-c]quinolin-7-yl)naphthalen-2-ol

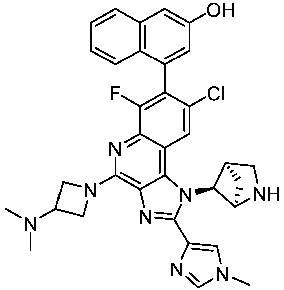

This compound was prepared according to the procedure described in **Example 77,** replacing 2-((tert-butyldimethylsilyl)oxy)acetaldehyde with 1-methyl-1*H*-imidazole-4-carbaldehyde in **Step 2.** The product was isolated as a mixture of diastereomers. LCMS calculated for C₃₄H₃₃ClFN₈O (M+H)⁺ m/z = 623.2; found 623.2.

### Example 79a and Example 79b. 4-(1-((endo)-2-azabicyclo[2.1.1]hexan-5-yl)-8-chloro-4-(3-(dimethylamino)azetidin-1-yl)-6-fluoro-7-(3-hydroxynaphthalen-1-yl)-1H-imidazo[4,5-c]quinolin-2-yl)tetrahydro-2H-thiopyran 1,1-dioxide

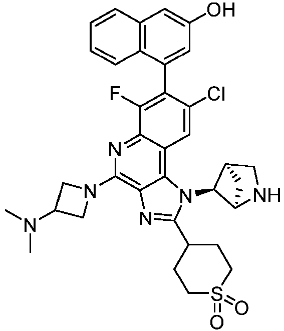

This compound was prepared according to the procedure described in **Example 77,** replacing 2-((*tert*-butyldimethylsilyl)oxy)acetaldehyde with tetrahydro*-2H*-thiopyran-4-carbaldehyde 1,1-dioxide in **Step 2.**

**Example 79a.** Diastereomer 1. Peak 1. LCMS calculated for C₃₅H₃₇ClFN₆O₃S (M+H)⁺ m/z = 675.2; found 675.2.

**Example 79b.** Diastereomer 2. Peak 2. LCMS calculated for LCMS calculated for C₃₅H₃₇ClFN₆O₃S (M+H)⁺ m/z = 675.2; found 675.2.

### Example 80a and Example 80b. 2-((2S,4S)-4-(8-chloro-7-(6-chloro-5-methyl-1H-indazol-4-yl)-4-(3-(dimethylamino)-3-methylazetidin-1-yl)-6-fluoro-1H-imidazo[4,5-c]quinolin-1-yl)-1-((E)-4-methoxybut-2-enoyl)piperidin-2-yl)acetonitrile

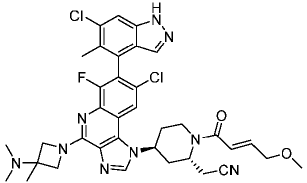

### Step 1. tert-butyl (2S,4S)-4-((7-bromo-2,6-dichloro-8-fluoro-3-nitroquinolin-4-yl)amino)-2-(2-(tert-butoxy)-2-oxoethyl)piperidine-1-carboxylate

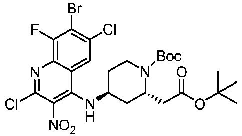

This compound was prepared according to the procedure described in **Example 35a and Example 35b, step 1,** replacing *tert*-butyl (endo)-5-amino-2-azabicyclo[2.1.1]hexane-2-carboxylate with *tert*-butyl (2S,4S)-4-amino-2-(2-(*tert*-butoxy)-2-oxoethyl)piperidine-1-carboxylate. LCMS calculated for C₂₅H₃₁BrCl₂FN₄O₆ (M+H)⁺: m/z = 651.1; found: 651.1.

### Step 2. tert-butyl (2S,4S)-4-((3-amino-7-bromo-2,6-dichloro-8-fluoroquinolin-4-yl)amino)-2-(2-(tert-butoxy)-2-oxoethyl)piperidine-1-carboxylate

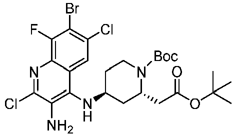

This compound was prepared according to the procedure described in **Example 15, step 2,** replacing *tert*-butyl (endo)-5-((7-bromo-6-chloro-2-(3-(dimethylamino)azetidin-1-yl)-8-fluoro-3-nitroquinolin-4-yl)amino)-2-azabicyclo[2.1.1]hexane-2-carboxylate with *tert*-butyl (2*S*,4*S*)-4-((3-amino-7-bromo-2,6-dichloro-8-fluoroquinolin-4-yl)amino)-2-(2-(*tert*-butoxy)-2-oxoethyl)piperidine-1-carboxylate. LCMS calculated for C₂₅H₃₃BrCl₂FN₄O₄ (M+H)⁺: m/z = 621.1; found: 621.1.

### Step 3. tert-butyl (2S,4S)-4-(7-bromo-4,8-dichloro-6-fluoro-1H-imidazo[4,5-c]quinolin-1-yl)-2-(2-(tert-butoxy)-2-oxoethyl)piperidine-1-carboxylate

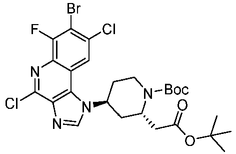

This compound was prepared according to the procedure described in **Example 23, step 1,** replacing *tert*-butyl (*endo*)-5-((3-amino-7-bromo-6-chloro-2-(3-(dimethylamino)azetidin-1-yl)-8-fluoroquinolin-4-yl)amino)-2-azabicyclo[2.1.1]hexane-2-carboxylate with *tert*-butyl (2S,4S)-4-((3-amino-7-bromo-2,6-dichloro-8-fluoroquinolin-4-yl)amino)-2-(2-(*tert*-butoxy)-2-oxoethyl)piperidine-1-carboxylate. LCMS calculated for C₂₆H₃₁BrCl₂FN₄O₄ (M+H)⁺: m/z = 631.1; found: 631.1.

### Step 4. tert-butyl (2S,4S)-4-(7-bromo-8-chloro-6-fluoro-4-(methylthio)-1H-imidazo[4,5-cjquinolin-1-yl)-2-(2-(tert-butoxy)-2-oxoethyl)piperidine-1-carboxylate

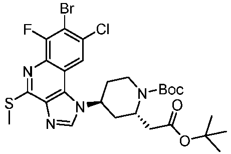

To a stirred solution of *tert*-butyl (2*S*,4*S*)-4-(7-bromo-4,8-dichloro-6-fluoro-1*H-*imidazo[4,5-*c*]quinolin-1-yl)-2-(2-(*tert*-butoxy)-2-oxoethyl)piperidine-1-carboxylate (3.46 g, 5.52 mmol) in MeOH (20.0 mL) and THF (20.0 mL) was added sodium thiomethoxide (1.16 g, 16.6 mmol). The mixture was stirred at room temperature for 15 minutes, then diluted with sat'd ammomium chloride and extracted with EtOAc. The combined organic layers were dried over MgSO4, filtered, concentrated to dryness, and purified on silica gel to yield the desired product (1.64 g, 46%). LCMS calculated for C₂₇H₃₄BrClFN₄O₄S (M+H)⁺: m/z = 643.1; found: 643.1.

### Step 5. 2-((2S,4S)-4-(7-bromo-8-chloro-6-fluoro-4-(methylthio)-1H-imidazo[4,5-c]quinolin-1-yl)piperidin-2-yl)acetic acid

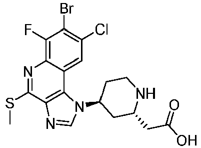

A vial was charged with *tert*-butyl (2*S*,4*S*)-4-(7-bromo-8-chloro-6-fluoro-4-(methylthio)-1*H*-imidazo[4,5-c]quinolin-1-yl)-2-(2-(*tert*-butoxy)-2-oxoethyl)piperidine-1-carboxylate (1.64 g, 2.55 mmol), CH₂Cl₂ (10.0 mL), and TFA (5.0 mL). The mixtute was stirred at room temperature for 30 minutes then concentrated to dryness to yield the desired product as a TFA-salt which was used directly in the next step without further purification. LCMS calculated for C₁₈H₁₈BrClFN₄O₂S (M+H)⁺: m/z = 487.0; found: 486.9.

### Step 6. 2-((2S,4S)-4-(7-bromo-8-chloro-6-fluoro-4-(methylthio)-1H-imidazo[4,5-c]quinolin-1-yl)-1-(tert-butoxycarbonyl)piperidin-2-yl)acetic acid

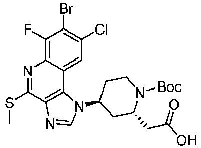

The concentrated residue from **Step 5** was taken up in CH₂Cl₂ (20 mL) and triethylamine (3.55 mL, 25.5 mmol) was added slowly. The mixture was stirred at room temperature for 5 minutes, then Boc-anhydride (611 mg, 2.80 mmol) was added. The mixture was stirred at room temperature for another 30 minutes. Additional Boc-anhydride was added as necessary. Upon full conversion, the mixture was acidified to pH 4-5 then extracted with EtOAc. The combined organic layers were dried over MgSO₄, filtered, concentrated, and used directly in the next step without further purification. LCMS calculated for C₂₃H₂₆BrClFN₄O₄S (M+H)⁺: m/z = 587.1; found: 587.1.

### Step 7. tert-butyl (2S,4S)-2-(2-amino-2-oxoethyl)-4-(7-bromo-8-chloro-6-fluoro-4-(methylthio)-1H-imidazo[4,5-c]quinolin-1-yl)piperidine-1-carboxylate

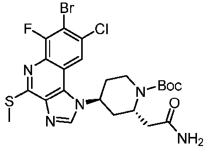

The concentrated residue from **Step** 6 was taken up in THF (20.0 mL) and DIPEA (2.67 mL, 15.3 mmol). The mixture was cooled to 0 °C, then isobutyl chloroformate (836 µL, 6.37 mmol) was added slowly. The mixture was stirred at 0 °C for another 20 minutes before ammonium hydroxide (28% in water, 3.54 mL, 25.5 mmol) was added to the mixture. After stirring for another 5 minutes, the mixture was diluted with brine and extracted with EtOAc. The combined organic layers were dried over MgSO₄, filtered, concentrated, and used directly in the next step without further purification. LCMS calculated for C₂₃H₂₇BrClFN₅O₃S (M+H)⁺: m/z = 586.1; found: 586.0.

### Step 8. tert-butyl (2S,4S)-4-(7-bromo-8-chloro-6-fluoro-4-(methylthio)-1H-imidazo[4,5-c]quinolin-1-yl)-2-(cyanomethyl)piperidine-1-carboxylate

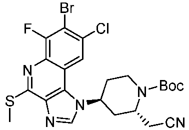

The concentrated residue from **Step 7** was taken up in THF (20.0 mL) and triethylamine (710 µL, 5.09 mmol). The mixture was cooled to 0 °C, then TFAA (540 µL, 3.82 mmol) was added slowly. The mixture was stirred at 0 °C for another 30 minutes before diluting with EtOAc and extracting with brine. The combined organic layers were dried over MgSO₄, filtered, concentrated to dryness, and purified on silica gel to yield the desired product (1.14 g, 79% over 4 steps). LCMS calculated for C₂₃H₂₅BrClFN₅O₂S (M+H)⁺: m/z = 568.1; found: 568.0.

### Step 9. tert-butyl (2S,4S)-4-(8-chloro-7-(6-chloro-5-methyl-1-(tetrahydro-2H-pyran-2-yl)-1H-indazol-4-yl)-6-fluoro-4-(methylthio)-1H-imidazo[4,5-c]quinolin-1-yl)-2-(cyanomethyl)piperidine-1-carboxylate

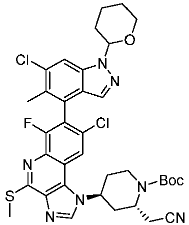

A screw-cap vial equipped with a magnetic stir bar was charged tert-butyl (2*S*,4*S*)-4-(7-bromo-8-chloro-6-fluoro-4-(methylthio)-1*H*-imidazo[4,5-c]quinolin-1-yl)-2-(cyanomethyl)-piperidine-1-carboxylate (250.0 mg, 0.439 mmol), 6-chloro-5-methyl-1-(tetrahydro-2*H*-pyran-2-yl)-4-(4,4,5,5-tetramethyl-1,3,2-dioxaborolan-2-yl)-1*H*-indazole (199 mg, 0.527 mmol), sodium carbonate (186 mg, 1.758 mmol), and Pd(Ph₃P)₄ (76 mg, 66 µmol) followed by dioxane (2.5 mL) and water (0.5 mL). The vial was sealed with a Teflon-lined septum, evacuated and backfilled with nitrogen (this process was repeated a total of three times). Then the reaction was stirred at 105 °C overnight. After cooling to room temperature, the mixture was diluted with brine and extracted with EtOAc. The combined organic layers were dried over MgSO₄, filtered, concentrated to dryness, and purified on silica gel to yield the desired product (314 mg, 97%). LCMS calculated for C₃₆H₃₉Cl₂FN₇O₃S (M+H)⁺: m/z = 738.2; found: 738.2.

### Step 10. tert-butyl (2S,4S)-4-(8-chloro-7-(6-chloro-5-methyl-1-(tetrahydro-2H-pyran-2-yl)-1H-indazol-4-yl)-4-(3-(dimethylamino)-3-methylazetidin-1-yl)-6-fluoro-1H-imidazo[4,5-c]quinolin-1-yl)-2-(cyanomethyl)piperidine-1-carboxylate

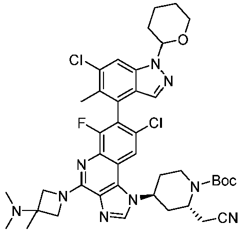

A solution of *tert-*butyl (2S,4S)-4-(8-chloro-7-(6-chloro-5-methyl-1-(tetrahydro-2*H-*pyran-2-yl)-1*H*-indazol-4-yl)-6-fluoro-4-(methylthio)-1*H*-imidazo[4,5-*c*]quinolin-1-yl)-2-(cyanomethyl)piperidine-1-carboxylate (20.0 mg, 0.027 mmol) in CH₂Cl₂ (1.0 mL) was cooled to 0 °C. m-CPBA (7.89 mg, 0.035 mmol) was added in one portion and the mixture was stirred at 0 °C for an additional 30 minutes. The mixture was then diluted with sat'd NaHCO₃ and extracted with CH₂Cl₂. The combined organic layers were dried over MgSO₄, filtered, and concentrated to dryness.

To the concentrate residue was added *N,N*,3-trimethylazetidin-3-amine hydrochloride (8.16 mg, 0.054 mmol), DIPEA (100 µL, 0.573 mmol), and dioxane (1.0 mL). The mixture was heated at 90 °C overnight before cooling to room temperature and extracting with sat'd ammonium chloride. The combined organic layers were dried over MgSO₄, filtered, concentrated, and used directly in the next step without further purification. LCMS calculated for C₄₁H₄₉Cl₂FN₉O₃ (M+H)⁺: m/z = 804.3; found: 804.3.

### Step 11. 2-((2S, 4S)-4-(8-chloro-7-(6-chloro-5-methyl-1H-indazol-4-yl)-4-(3-(dimethylamino)-3-methylazetidin-1-yl)-6-fluoro-1H-imidazo[4,5-c]quinolin-1-yl)-1-((E)-4-methoxybut-2-enoyl)piperidin-2-yl)acetonitrile

The concentrated residue from **Step 10** was redissolved in CH₂Cl₂ (2.0 mL) and TFA (1.0 mL). The mixture was stirred at room temperature for 30 minutes before concentrating to dryness. The concentreate residue was redissolved in acetonitrile (1.0 mL) and DIPEA (100 µL) was added. The mixture was stirred for 5 minutes before HATU (20.6 mg, 0.054 mmol) and (*E*)-4-methoxybut-2-enoic acid (6.29 mg, 0.054 mmol) were added. Upon completion, the reaction was diluted with acetonitrile and purified using prep-LCMS (XBridge C18 column, eluting with a gradient of acetonitrile/water containing 0.1% TFA, at flow rate of 60 mL/min) then purified again using prep-LCMS (XBridge C18 column, eluting with a gradient of acetonitrile/water containing 0.15% NH₄OH, at flow rate of 60 mL/min) to afford the desired product.

**Example 80a.** Diastereomer 1. Peak 1. LCMS calculated for C₃₆H₃₉Cl₂FN₉O₂ (M+H)⁺ m/z = 718.3; found 718.4.

**Example 80b.** Diastereomer 2. Peak 2. LCMS calculated for C₃₆H₃₉Cl₂FN₉O₂ (M+H)⁺ m/z = 718.3; found 718.4.

### Example 85. 2-((2S,4S)-1-acryloyl-4-(8-chloro-7-(3-chloro-4-fluorophenyl)-6-fluoro-4-(((S)-1-methylpyrrolidin-2-yl)methoxy)-1H-[1,2,3]triazolo[4,5-c]quinolin-1-yl)piperidin-2-yl)acetonitrile

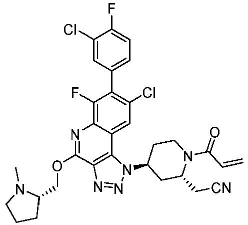

### Step 1. tert-butyl (2S,4S)-4-(((benzyloxy)carbonyl)amino)-2-(2-(tert-butoxy)-2-oxoethyl)piperidine-1-carboxylate

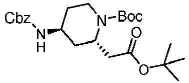

To a stirred solution of tert-butyl (2S,4S)-4-amino-2-(2-(tert-butoxy)-2-oxoethyl)-piperidine-1-carboxylate (5.2 g, 16.54 mmol) in CH₂Cl₂ (80.0 mL) was added *N*-(benzyloxy-carbonyloxy)succinimide (4.95 g, 19.85 mmol) followed by DIPEA (4.33 mL, 24.81 mmol). The mixture stirred at room temperature for 2 hours, then diluted with water. The mixture was extracted with water and brine. The combined organic layers were dried over MgSO₄, filtered, concentrated, and used directly in the next step without further purification.

### Step 2. 2-((2S,4S)-4-(((benzyloxy)carbonyl)amino)piperidin-2-yl)acetic acid

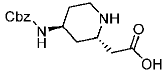

The concentrated residue from **Step 1** was taken up in CH₂Cl₂ (80.0 mL) and TFA (50.0 mL). The mixture was stirred at room temperature overnight then concentrated to dryness and used directly in the next step without further purification.

### Step 3. 2-((2S,4S)-4-(((benzyloxy)carbonyl)amino)-1-(tert-butoxycarbonyl)piperidin-2-yl)acetic acid

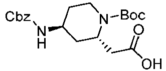

The concentrated residue from **Step 2** was taken up in CH₂Cl₂ (80.0 mL) and triethylamine (23.1 mL, 165 mmol) was added slowly. The mixture was stirred at room temperature for 5 minutes, then Boc-anhydride (4.33 g, 19.85 mmol) was added. The mixture was stirred at room temperature for another 30 minutes. Additional Boc-anhydride was added as necessary. Upon full conversion, the mixture was acidified to pH 4-5 then extracted with EtOAc. The combined organic layers were dried over MgSO₄, filtered, concentrated, and used directly in the next step without further purification.

### Step 4. tert-butyl (2S,4S)-2-(2-amino-2-oxoethyl)-4-(((benzyloxy)carbonyl)amino)piperidine-1-carboxylate

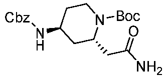

The concentrated residue from **Step 3** was taken up in THF (80.0 mL) and DIPEA (8.67 mL, 49.6 mmol). The mixture was cooled to 0 °C, then isobutyl chloroformate (5.43 mL, 41.3 mmol) was added slowly. The mixture was stirred at 0 °C for another 20 minutes before ammonium hydroxide (28% in water, 23.0 mL, 165 mmol) was added to the mixture. After stirring for another 5 minutes, the mixture was diluted with brine and extracted with EtOAc. The combined organic layers were dried over MgSO₄, filtered, concentrated, and used directly in the next step without further purification.

### Step 5. tert-butyl (2S,4S)-4-(((benzyloxy)carbonyl)amino)-2-(cyanomethyl)piperidine-1-carboxylate

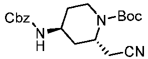

The concentrated residue from **Step 4** was taken up in THF (80.0 mL) and triethylamine (6.0 mL, 43 mmol). The mixture was cooled to 0 °C, then TFAA (3.5 mL, 24.8 mmol) was added slowly. The mixture was stirred at 0 °C for another 30 minutes before diluting with EtOAc and extracting with brine. The combined organic layers were dried over MgSO₄, filtered, concentrated to dryness, and purified on silica gel to yield the desired product (5.12 g, 83% over 5 steps). LCMS calculated for C₁₆H₂₀N₃O₄ (M+H-tert-butyl)⁺: m/z = 318.1; found: 318.1.

### Step 6. tert-butyl (2S,4S)-4-amino-2-(cyanomethyl)piperidine-1-carboxylate

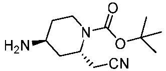

A round-bottom flask containing a stir bar was charged with *tert-*butyl (2*S*,4*S*)-4-(((benzyloxy)carbonyl)amino)-2-(cyanomethyl)piperidine-1-carboxylate (5.12 g, 13.71 mmol), palladium on carbon (10 wt%, 2.92 g, 2.74 mmol), and MeOH (45 mL). The round-bottom was evacuated and backfilled with H₂ (this process was repeated a total of three times) and the mixture was stirred vigorously at room temperature for 1.5 hours with a balloon of H₂ attached. The mixture was then filtered over celite and the solids were washed with EtOAc. The filtrate was concentrated and used directly in the next step without further purification.

### Step 7. tert-butyl (2S,4S)-4-((7-bromo-2,6-dichloro-8-fluoro-3-nitroquinolin-4-yl)amino)-2-(cyanomethyl)piperidine-1-carboxylate

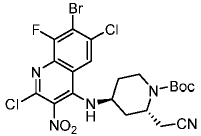

This compound was prepared according to the procedure described in **Example 1a and Example 1b, step 5,** replacing *tert*-butyl 4-aminopiperidine-1-carboxylate with *tert*-butyl (2*S*,4*S*)-4-amino-2-(cyanomethyl)piperidine-1-carboxylate. LCMS calculated for C₂₁H₂₂BrCl₂FN₅O₄ (M+H)⁺: m/z = 576.0; found: 576.0

### Step 8. tert-butyl (2S,4S)-4-((7-bromo-6-chloro-8-fluoro-2-(((S)-1-methylpyrrolidin-2-yl)methoxy)-3-nitroquinolin-4-yl)amino)-2-(cyanomethyl)piperidine-1-carboxylate

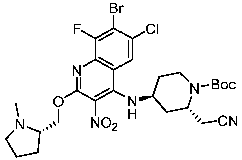

This compound was prepared according to the procedure described in **Example 1a and Example 1b, step 6,** replacing *tert*-butyl 4-((7-bromo-2,6-dichloro-8-fluoro-3-nitroquinolin-4-yl)amino)piperidine-1-carboxylate with tert-butyl (2*S*,4*S*)-4-((7-bromo-2,6-dichloro-8-fluoro-3-nitroquinolin-4-yl)amino)-2-(cyanomethyl)piperidine-1-carboxylate. LCMS calculated for C₂₇H₃₄BrClFN₆O₅ (M+H)⁺: m/z = 655.1; found: 655.1.

### Step 9. tert-butyl (2S,4S)-4-((3-amino-7-bromo-6-chloro-8-fluoro-2-(((S)-1-methylpyrrolidin-2-yl)methoxy)quinolin-4-yl)amino)-2-(cyanomethyl)piperidine-1-carboxylate

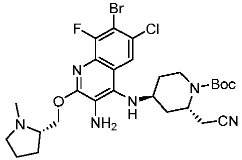

This compound was prepared according to the procedure described in **Example 1a and Example 1b, step 7,** replacing *tert-butyl* (*S*)-4-((7-bromo-6-chloro-8-fluoro-2-((1-methylpyrrolidin-2-yl)-methoxy)-3-nitroquinolin-4-yl)amino)piperidine-1-carboxylate with *tert-*butyl (2S,4S)-4-((7-bromo-6-chloro-8-fluoro-2-(((*S*)-1-methylpyrrolidin-2-yl)methoxy)-3-nitroquinolin-4-yl)amino)-2-(cyanomethyl)piperidine-1-carboxylate. LCMS calculated for C₂₇H₃₃BrClFN₃O₃ (M+H)⁺: m/z = 625.2; found: 625.2.

### Step 10. tert-butyl (2S,4S)-4-(7-bromo-8-chloro-6-fluoro-4-(((S)-1-methylpyrrolidin-2-yl)methoxy)-1H-[1,2,3]triazolo[4,5-c]quinolin-1-yl)-2-(cyanomethyl)piperidine-1-carboxylate

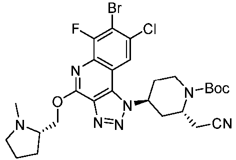

This compound was prepared according to the procedure described in **Example 14, Step** 1, replacing tert-butyl (S)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2*S*,4*S*)-4-((3-amino-7-bromo-6-chloro-8-fluoro-2-(((*S*)-1-methylpyrrolidin-2-yl)methoxy)quinolin-4-yl)amino)-2-(cyanomethyl)piperidine-1-carboxylate. LCMS calculated for C₂₇H₃₃BrClFN₇O₃ (M+H)⁺: m/z = 636.2; found: 636.1

### Step 11. tert-butyl (2S,4S)-4-(8-chloro-7-(3-chloro-4-fluorophenyl)-6-fluoro-4-(((S)-1-methylpyrrolidin-2-yl)methoxy)-1H-[1,2,3]triazolo[4,5-c]quinolin-1-yl)-2-(cyanomethyl) piperidine-1-carboxylate

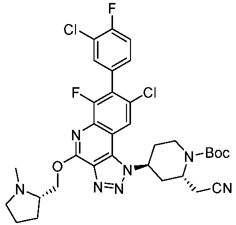

A screw-cap vial equipped with a magnetic stir bar was charged tert-butyl (2*S*,4*S*)-4-(7-bromo-8-chloro-6-fluoro-4-(((*S*)-1-methylpyrrolidin-2-yl)methoxy)-1*H*-[1,2,3]triazolo[4,5-c]quinolin-1-yl)-2-(cyanomethyl)piperidine-1-carboxylate (130.0 mg, 0.204 mmol), (3-chloro-4-fluorophenyl)boronic acid (39.1 mg, 0.225 mmol), sodium carbonate (87 mg, 0.816 mmol), and Pd(Ph₃P)₄ (18.9 mg, 16 µmol) followed by dioxane (3.33 mL) and water (0.67 mL). The vial was sealed with a Teflon-lined septum, evacuated and backfilled with nitrogen (this process was repeated a total of three times). Then the reaction was stirred at 70 °C overnight. After cooling to room temperature, the mixture was diluted with brine and extracted with EtOAc. The combined organic layers were dried over MgSO₄, filtered, concentrated to dryness, and purified on silica gel to yield the desired product (85.4 mg, 61%). LCMS calculated for C₃₃H₃₃Cl₂F₂N₇O₃ (M+H)⁺: m/z = 686.2; found: 686.1.

### Step 12. 2-((2S,4S)-1-acryloyl-4-(8-chloro-7-(3-chloro-4-fluorophenyl)-6-fluoro-4-(((S)-1-methylpyrrolidin-2-yl)methoxy)-1H-[1,2,3]triazolo[4,5-c]quinolin-1-yl)piperidin-2-yl)acetonitrile

*tert*-butyl (2S,4S)-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-c]quinolin-1-yl)-2-(cyanomethyl)-piperidine-1-carboxylate (21.3 mg, 0.031 mmol) was redissolved in CH₂Cl₂ (2.0 mL) and TFA (1.0 mL). The mixture was stirred at room temperature for 30 minutes before concentrating to dryness. The concentreate residue was redissolved in THF (1.0 mL) and triethylamine (100 µL) was added. The mixture was stirred for 5 minutes before acryloyl chloride (6.6 µL, 0.082 mmol) was added. The reaction was diluted with acetonitrile and purified using prep-LCMS (XBridge C18 column, eluting with a gradient of acetonitrile/water containing 0.1% TFA, at flow rate of 60 mL/min) then purified again using prep-LCMS (XBridge C18 column, eluting with a gradient of acetonitrile/water containing 0.15% NH₄OH, at flow rate of 60 mL/min) to afford the desired product. LCMS calculated for C₃₁H₃₀Cl₂F₂N₇O₂ (M+H)⁺: m/z = 640.2; found: 640.2.

### Example 86. 2-((2S,4S)-1-acryloyl-4-(8-chloro-7-(3-chloro-2-methylphenyl)-6-fluoro-4-(((S)-1-methylpyrrolidin-2-yl)methoxy)-1H-[1,2,3]triazolo[4,5-c]quinolin-1-yl)piperidin-2-yl)acetonitrile

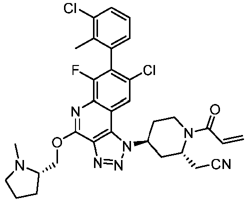

### Step 1. tert-butyl (2S,4S)-4-(8-chloro-7-(3-chloro-2-methylphenyl)-6-fluoro-4-(((S)-1-methylpyrrolidin-2-yl)methoxy)-1H-[1,2,3]triazolo[4,5-c]quinolin-1-yl)-2-(cyanomethyl)piperidine-1-carboxylate

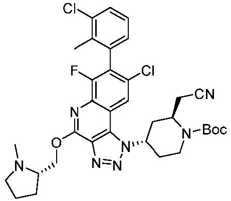

This compound was prepared according to the procedure described in **Example 85, Step 11,** replacing (3-chloro-4-fluorophenyl)boronic acid with (3-chloro-2-methylphenyl)-boronic acid. LCMS calculated for C₃₄H₃₉Cl₂FN₇O₃ (M+H)⁺ m/z = 682.3; found 682.2.

### Step 2. 2-((2S,4S)-1-acryloyl-4-(8-chloro-7-(3-chloro-2-methylphenyl)-6-fluoro-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1*H*-[1,2,3]triazolo[4,5-*c*]quinolin-1-yl)-2-(cyanomethyl)-piperidine-1-carboxylate with tert-butyl (2S,4S)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate. The product was isolated as a mixture of diastereomers. LCMS calculated for C₃₂H₃₃Cl₂FN₇O₂ (M+H)⁺ m/z = 636.2; found 636.2.

### Example 87a and Example 87b. 2-((2S,4S)-1-(but-2-ynoyl)-4-(8-chloro-7-(3-chloro-2-methylphenyl)-6-fluoro-4-(((S)-1-methylpyrrolidin-2-yl)methoxy)-1H-[1,2,3]triazolo[4,5-c]quinolin-1-yl)piperidin-2-yl)acetonitrile

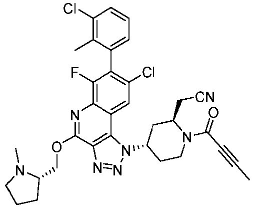

A solution of *tert-butyl* (2*S*,4*S*)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate (27.0 mg, 0.047 mmol, prepared according to **Example 86, Step 1)** in CH₂Cl₂ (2.0 mL) and TFA (1.0 mL) was stirred at room temperature for 30 minutes before concentrating to dryness. The concentreate residue was redissolved in acetonitrile (1.0 mL) and DIPEA (100 µL) was added. The mixture was stirred for 5 minutes before HATU (28.7 mg, 0.075 mmol) and but-2-ynoic acid (6.34 mg, 0.075 mmol) were added. Upon completion, the reaction was diluted with acetonitrile and purified using prep-LCMS (XBridge C18 column, eluting with a gradient of acetonitrile/water containing 0.1% TFA, at flow rate of 60 mL/min) then purified again using prep-LCMS (XBridge C18 column, eluting with a gradient of acetonitrile/water containing 0.15% NH₄OH, at flow rate of 60 mL/min) to afford the desired product.

**Example 87a.** Diastereomer 1. Peak 1. LCMS calculated for C₃₃H₃₃Cl₂FN₇O₂ (M+H)⁺ m/z = 648.2; found 648.2. ¹H NMR (600 MHz, DMSO-*d*₆) δ 8.63 - 8.48 (m, 1H), 7.63 (d, *J =* 8.1 Hz, 1H), 7.43 (t, *J* = 7.8 Hz, 1H), 7.28 (dd, *J* = 9.7, 7.7 Hz, 1H), 5.96 - 5.70 (m, 1H), 5.18 (q, *J* = 8.8 Hz, 1H), 5.06 (tdd, *J* = 12.2, 9.6, 3.5 Hz, 1H), 4.85 (tdd, *J* = 12.3, 7.3, 4.1 Hz, 1H), 4.64 - 4.48 (m, 1H), 4.01 (d, *J =* 10.2 Hz, 1H), 3.79 - 3.70 (m, 2H), 3.66 (m, 1H), 3.51 (ddd, *J* = 16.6, 9.4, 7.2 Hz, 1H), 3.35 - 3.15 (m, 3H), 3.06 (m, 4H), 2.44 - 2.21 (m, 2H), 2.17 - 2.06 (m, 7H), 1.98 (q, *J* = 7.7 Hz, 2H).

**Example 87b.** Diastereomer 2. Peak 2. LCMS calculated for C₃₃H₃₃Cl₂FN₇O₂ (M+H)⁺ m/z = 648.2; found 648.2.

### Example 88. 2-((2S,4S)-4-(8-chloro-7-(3-chloro-2-methylphenyl)-6-fluoro-4-(((S)-1-methylpyrrolidin-2-yl)methoxy)-1H-[1,2,3]triazolo[4,5-c]quinolin-1-yl)-1-((E)-4-(dimethylamino)but-2-enoyl)piperidin-2-yl)acetonitrile

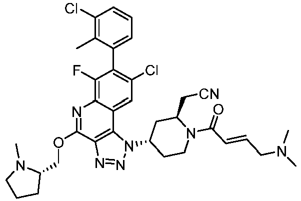

This compound was prepared according to the procedure described in **Example 87a and Example 87b** replacing but-2-ynoic acid with 4-(dimethylamino)but-2-ynoic acid hydrochloride. The product was isolated as a mixture of diastereomers. LCMS calculated for C₃₅H₄₀Cl₂FN₈O₂ (M+H)⁺ m/z = 693.3; found 693.3

### Example 89. 2-((2S,4S)-1-acryloyl-4-(8-chloro-7-(5-chloro-4-methylpyridin-3-yl)-4-(3-(dimethylamino)azetidin-1-yl)-6-fluoro-1H-imidazo[4,5-c]quinolin-1-yl)piperidin-2-yl)acetonitrile

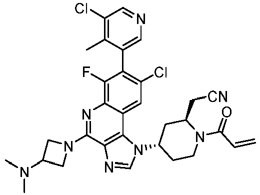

### Step 1. tert-butyl (2S,4S)-4-(7-bromo-8-chloro-4-(3-(dimethylamino)azetidin-1-yl)-6-fluoro-1H-imidazo[4,5-c]quinolin-1-yl)-2-(2-(tert-butoxy)-2-oxoethyl)piperidine-1-carboxylate

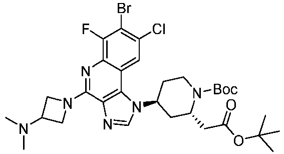

A solution of *tert*-butyl (2*S*,4*S*)-4-(7-bromo-4,8-dichloro-6-fluoro-1*H*-imidazo[4,5-c]quinolin-1-yl)-2-(2-(*tert*-butoxy)-2-oxoethyl)piperidine-1-carboxylate (prepared according to **Example 80a and Example 80b, Steps 1-3,** 800 mg, 1.27 mmol), *N,N*-dimethylazetidin-3-amine dihydrochloride (263 mg, 1.52 mmol), and DIPEA (0.773 mL, 4.43 mmol) in dioxane (5 mL) was heated to 100 °C overnight. Upon cooling to room temperature, the mixture was diluted with EtOAc and extracted with sat'd ammonium chloride. The combined organic layers were dried over MgSO₄, filtered, concentrated, and used directly in the next step without further purification. LCMS calculated for C₃₁H₄₂BrClFN₆O₄ (M+H)⁺ m/z = 695.2; found 695.2.

### Step 2. tert-butyl (2S,4S)-4-(7-bromo-8-chloro-4-(3-(dimethylamino)azetidin-1-yl)-6-fluoro-1H-imidazo[4,5-c]quinolin-1-yl)-2-(cyanomethyl)piperidine-1-carboxylate

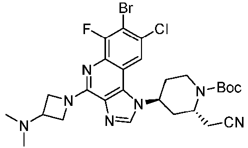

This compound was prepared according to the procedure described in **Example 80a and Example 80b, Steps 5-8,** replacing tert-butyl (2S,4S)-4-(7-bromo-8-chloro-6-fluoro-4-(methylthio)-1H-imidazo[4,5-c]quinolin-1-yl)-2-(2-(tert-butoxy)-2-oxoethyl)piperidine-1-carboxylate with tert-butyl (2S,4S)-4-(7-bromo-8-chloro-4-(3-(dimethylamino)azetidin-1-yl)-6-fluoro-1*H*-imidazo[4,5-*c*]quinolin-1-yl)-2-(2-(tert-butoxy)-2-oxoethyl)piperidine-1-carboxylate. LCMS calculated for C₂₇H₃₃BrClFN₇O₂ (M+H)⁺ m/z = 620.2; found 620.2.

### Step 3. 3-chloro-4-methyl-5-(4,4,5,5-tetramethyl-1,3,2-dioxaborolan-2-yl)pyridine

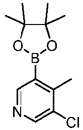

A solution of 3-bromo-5-chloro-4-methylpyridine (300 mg, 1.45 mmol), Pd(dppf)Cl₂•CH₂Cl₂ (53.2 mg, 0.073 mmol), potassium acetate (428 mg, 4.63 mmol), and bis(pinacolato)diboron (738 mg, 2.91 mmol) in dioxane (6.0 mL) was heated at 90 °C overnight. The reaction mixture was cooled to r.t., diluted with EtOAc and filtered through celite. The organic layer was concentrated to provide the desired product. LCMS calculated for C₁₂H₁₈BClNO₂ (M+H)⁺: m/z = 254.1; found 254.1.

### Step 4. tert-butyl (2S,4S)-4-(8-chloro-7-(5-chloro-4-methylpyridin-3-yl)-4-(3-(dimethylamino)azetidin-1-yl)-6-fluoro-1H-imidazo[4,5-c]quinolin-1-yl)-2-(cyanomethyl)piperidine-1-carboxylate

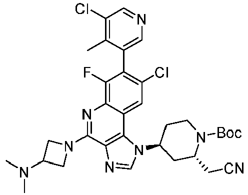

This compound was prepared according to the procedure described in **Example 85, Step 11,** replacing (3-chloro-4-fluorophenyl)boronic acid with 3-chloro-4-methyl-5-(4,4,5,5-tetramethyl-1,3,2-dioxaborolan-2-yl)pyridine and tert-butyl (2S,4S)-4-(7-bromo-8-chloro-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with *tert*-butyl (2*S*,4*S*)-4-(7-bromo-8-chloro-4-(3-(dimethylamino)azetidin-1-yl)-6-fluoro-1*H*-imidazo[4,5-*c*]quinolin-1-yl)-2-(cyanomethyl)-piperidine-1-carboxylate. LCMS calculated for C₃₃H₃₃Cl₂FN₈O₂ (M+H)⁺ m/z = 667.3; found 667.3.

### Step 5. 2-((2S,4S)-4-(8-chloro-7-(3-chloro-2-methylphenyl)-6-fluoro-4-(((S)-1-methylpyrrolidin-2-yl)methoxy)-1H-[1,2,3]triazolo[4,5-c]quinolin-1-yl)-1-((E)-4-(dimethylamino)but-2-enoyl)piperidin-2-yl)acetonitrile

This compound was prepared according to the procedure described in **Example 85, Step 12,** replacing tert-butyl (2S,4S)-4-(8-chloro-7-(3-chloro-4-fluorophenyl)-6-fluoro-4-(((S)-1-methylpyrrolidin-2-yl)methoxy)-1*H*-[1,2,3]triazolo[4,5-*c*]quinolin-1-yl)-2-(cyanomethyl)-piperidine-1-carboxylate with *tert*-butyl (2*S*,4*S*)-4-(8-chloro-7-(5-chloro-4-methylpyridin-3-yl)-4-(3-(dimethylamino)azetidin-1-yl)-6-fluoro-1*H*-imidazo[4,5-*c*]quinolin-1-yl)-2-(cyanomethyl)-piperidine-1-carboxylate. The product was isolated as a mixture of diastereomers. LCMS calculated for C₃₁H₃₂Cl₂FN₈O (M+H)⁺ m/z = 621.2; found 621.2.

### Example 91. 2-((2S,4S)-1-(but-2-ynoyl)-4-(8-chloro-7-(3-chloro-2-methylphenyl)-6-fluoro-4-(3-oxomorpholino)-1H-[1,2,3]triazolo[4,5-c]quinolin-1-yl)piperidin-2-yl)acetonitrile

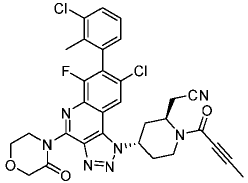

### Step 1. tert-butyl (2S,4S)-4-((3-amino-7-bromo-2,6-dichloro-8-fluoroquinolin-4-yl)amino)-2-(cyanomethyl)piperidine-1-carboxylate

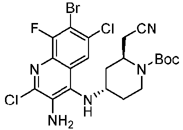

This compound was prepared according to the procedure described in **Example 15, step 2,** replacing *tert*-butyl (*endo*)-5-((7-bromo-6-chloro-2-(3-(dimethylamino)azetidin-1-yl)-8-fluoro-3-nitroquinolin-4-yl)amino)-2-azabicyclo[2.1.1]hexane-2-carboxylate with *tert-butyl* (2*S*,4*S*)-4-((7-bromo-2,6-dichloro-8-fluoro-3-nitroquinolin-4-yl)amino)-2-(cyanomethyl)-piperidine-1-carboxylate (prepared according to **Example 85, Steps 1-7**). LCMS calculated for C₂₁H₂₄BrCl₂FN₅O₂ (M+H)⁺: m/z = 546.1; found: 545.9.

### Step 2. tert-butyl (2S,4S)-4-(7-bromo-4,8-dichloro-6-fluoro-1H-[1,2,3]triazolo[4,5-c]quinolin-1-yl)-2-(cyanomethyl)piperidine-1-carboxylate

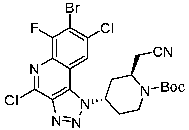

This compound was prepared according to the procedure described in **Example 14, Step** 1, replacing *tert*-butyl (*S*)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2*S*,4*S*)-4-((3-amino-7-bromo-2,6-dichloro-8-fluoroquinolin-4-yl)amino)-2-(cyanomethyl)piperidine-1-carboxylate. LCMS calculated for C₂₁H₂₁BrCl₂FN₆O₂ (M+H)⁺: m/z = 557.0; found: 557.0

### Step 3. tert-butyl (2S,4S)-4-(7-bromo-8-chloro-6-fluoro-4-(methylthio)-1H-[1,2,3]triazolo[4,5-c]quinolin-1-yl)-2-(cyanomethyl)piperidine-1-carboxylate

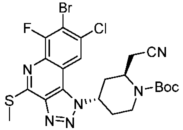

This compound was prepared according to the procedure described in **Example 80a and Example 80b, Step 4,** *tert-*butyl (2S,4S)-4-(7-bromo-4,8-dichloro-6-fluoro-1*H-*imidazo[4,5-c]quinolin-1-yl)-2-(2-(tert-butoxy)-2-oxoethyl)piperidine-1-carboxylate with *tert-*butyl (2S,4S)-4-(7-bromo-4,8-dichloro-6-fluoro-1*H*-[1,2,3]triazolo[4,5-c]quinolin-1-yl)-2-(cyanomethyl)piperidine-1-carboxylate. LCMS calculated for C₂₂H₂₄BrClFN₆O₂S (M+H)⁺: m/z = 569.1; found: 569.0

### Step 4. tert-butyl (2S,4S)-4-(8-chloro-7-(3-chloro-2-methylphenyl)-6-fluoro-4-(methylthio)-1H-[1,2,3]triazolo[4,5-c]quinolin-1-yl)-2-(cyanomethyl)piperidine-1-carboxylate

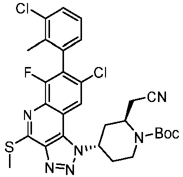

This compound was prepared according to the procedure described in **Example 86, Step** 1, replacing *tert*-butyl (2S,4S)-4-(7-bromo-8-chloro-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with *tert*-butyl (2*S*,4*S*)-4-(7-bromo-8-chloro-6-fluoro-4-(methylthio)-1*H*-[1,2,3]triazolo[4,5-*c*]quinolin-1-yl)-2-(cyanomethyl)piperidine-1-carboxylate. LCMS calculated for C₂₉H₃₀Cl₂FN₆O₂S (M+H)⁺ m/z = 615.2; found 615.1.

### Step 5. tert-butyl (2S,4S)-4-(8-chloro-7-(3-chloro-2-methylphenyl)-6-fluoro-4-(3-oxomorpholino)-1H-[1,2,3]triazolo[4,5-c]quinolin-1-yl)-2-(cyanomethyl) piperidine-1-carboxylate

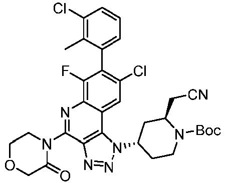

A solution of *tert-butyl* (2S,4S)-4-(8-chloro-7-(3-chloro-2-methylphenyl)-6-fluoro-4-(methylthio)-1H-[1,2,3]triazolo[4,5-c]quinolin-1-yl)-2-(cyanomethyl) piperidine-1-carboxylate (20.0 mg, 0.032 mmol) in CH₂Cl₂ (1.0 mL) was cooled to 0 °C. m-CPBA (9.47 mg, 0.042 mmol) was added in one portion and the mixture was stirred at 0 °C for an additional 30 minutes. The mixture was then diluted with sat'd NaHCO₃ and extracted with CH₂Cl₂. The combined organic layers were dried over MgSO₄, filtered, and concentrated to dryness.

The concentrated residue was taken up in THF (1.0 mL) and added to a solution of morpholin-3-one (6.57 mg, 0.065 mmol) and LHMDS (1.0 M in THF, 65 µL, 0.065 mmol) in THF (1.0 mL) at 0 °C. The mixture was stirred at 0 °C for 1 hour before sat'd ammonium chloride was added, then the mixture was extracted with EtOAc. The combined organic layers were dried over MgSO₄, filtered, concentrated, and used directly in the next step without further purification. LCMS calculated for C₃₂H₃₃Cl₂FN₇O₄ (M+H)⁺: m/z = 668.2; found: 668.2.

### Step 6. 2-((2S,4S)-1-(but-2-ynoyl)-4-(8-chloro-7-(3-chloro-2-methylphenyl)-6-fluoro-4-(3-oxomorpholino)-1H-[1,2,3]triazolo[4,5-c]quinolin-1-yl)piperidin-2-yl)acetonitrile

This compound was prepared according to the procedure described in **Example 87a and Example 87b,** replacing tert-butyl (2S,4S)-4-(8-chloro-7-(3-chloro-2-methylphenyl)-6-*fluoro-4-(((S)-1-methylpyrrolidin-2-yl)methoxy)-1H-[1,2,3]triazolo[4,5-c]quinolin-1-yl)-2-*(cyanomethyl)piperidine-1-carboxylate with tert-butyl (2S,4S)-4-(8-chloro-7-(3-chloro-2-methylphenyl)-6-fluoro-4-(3-oxomorpholino)-1*H*-[1 ,2,3]triazolo[4,5-c]quinolin-1-yl)-2-(cyanomethyl)piperidine-1-carboxylate. The product was isolated as a mixture of diastereomers. LCMS calculated for C₃₁H₂₇Cl₂FN₇O₃ (M+H)⁺ m/z = 634.2; found 634.2.

### Example 92. 2-((2S,4S)-1-(but-2-ynoyl)-4-(8-chloro-7-(3-chloro-2-methylphenyl)-6-fluoro-4-(4-methyl-2-oxopiperazin-1-yl)-1H-[1,2,3]triazolo[4,5-c]quinolin-1-yl)piperidin-2-yl)acetonitrile

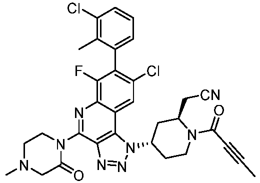

This compound was prepared according to the procedure described in **Example 91,** replacing morpholin-3-one with 4-methylpiperazin-2-one in **Step 5.** The product was isolated as a mixture of diastereomers. LCMS calculated for C₃₂H₃₀Cl₂FN₈O₂ (M+H)⁺ m/z = 647.2; found 647.2.

### Example 95. 3-(1-((2S,4S)-1-acryloyl-2-(cyanomethyl)piperidin-4-yl)-8-chloro-6-fluoro-4-(((S)-1-methylpyrrolidin-2-yl)methoxy)-1H-[1,2,3]triazolo[4,5-c]quinolin-7-yl)-2-methylbenzonitrile

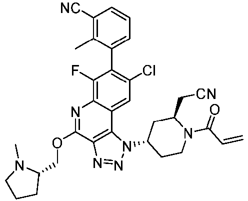

This compound was prepared according to the procedure described in **Example 86,** replacing (3-chloro-2-methylphenyl)boronic acid with (3-cyano-2-methylphenyl)boronic acid in **Step 1.** The product was isolated as a mixture of diastereomers. LCMS calculated for C₃₃H₃₃ClFN₃O₂ (M+H)⁺ m/z = 627.2; found 627.2.

### Example 96. 3-(1-((2S,4S)-1-(but-2-ynoyl)-2-(cyanomethyl)piperidin-4-yl)-8-chloro-6-fluoro-4-(((S)-1-methylpyrrolidin-2-yl)methoxy)-1H-[1,2,3]triazolo[4,5-c]quinolin-7-yl)-2-methylbenzonitrile

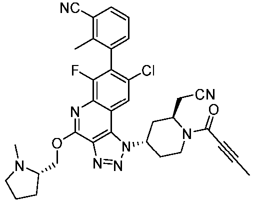

This compound was prepared according to the procedure described in **Example 87a and Example 87b,** replacing *tert*-butyl (2S,4S)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with tert-butyl (2S,4S)-4-(8-chloro-7-(3-cyano-2-methylphenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate. The product was isolated as a mixture of diastereomers. LCMS calculated for C₃₄H₃₃ClFN₃O₂ (M+H)⁺ m/z = 639.2; found 639.2.

### Example 97. 3-(8-chloro-1-((2S,4S)-2-(cyanomethyl)-1-((E)-4-(dimethylamino)but-2-enoyl)piperidin-4-yl)-6-fluoro-4-(((S)-1-methylpyrrolidin-2-yl)methoxy)-1H-[1,2,3]triazolo[4,5-c]quinolin-7-yl)-2-methylbenzonitrile

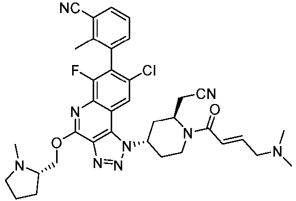

This compound was prepared according to the procedure described in **Example 96,** replacing but-2-ynoic acid with 4-(dimethylamino)but-2-ynoic acid hydrochloride. The product was isolated as a mixture of diastereomers. LCMS calculated for C₃₆H₄₀ClFN₉O₂ (M+H)⁺ m/z = 684.3; found 684.2.

### Example 98. 2-((2S,4S)-1-acryloyl-4-(7-(3-chloro-2-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile

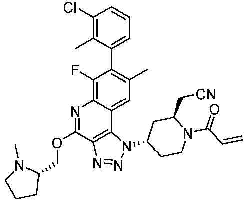

### Step 1. tert-butyl (2S,4S)-4-((7-bromo-2-chloro-8-fluoro-6-iodo-3-nitroquinolin-4-yl)amino)-2-(cyanomethyl)piperidine-1-carboxylate

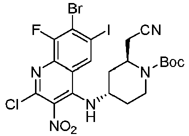

This compound was prepared according to the procedure described in **Example 85, step 7,** replacing 7-bromo-2,4,6-trichloro-8-fluoro-3-nitroquinoline with 7-bromo-2,4-dichloro-8-fluoro-6-iodo-3-nitroquinoline. LCMS calculated for C₂₁H₂₂BrClFlN₅O₄ (M+H)⁺: m/z = 668.0; found: 668.0.

### Step 2. tert-butyl (2S,4S)-4-((3-amino-7-bromo-2-chloro-8-fluoro-6-iodoquinolin-4-yl)amino)-2-(cyanomethyl)piperidine-1-carboxylate

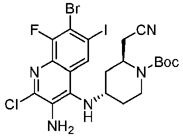

This compound was prepared according to the procedure described in **Example 1a and Example 1b, step 7,** replacing *tert-*butyl (*S*)-4-((7-bromo-6-chloro-8-fluoro-2-((1-methylpyrrolidin-2-yl)-methoxy)-3-nitroquinolin-4-yl)amino)piperidine-1-carboxylate with *tert-*butyl (2S,4S)-4-((7-bromo-2-chloro-8-fluoro-6-iodo-3-nitroquinolin-4-yl)amino)-2-(cyanomethyl)piperidine-1-carboxylate. LCMS calculated for C₂₁H₂₄BrClFlN₅O₂ (M+H)⁺: m/z = 638.0; found: 638.0.

### Step 3. tert-butyl (2S,4S)-4-(7-bromo-4-chloro-6-fluoro-8-iodo-1H-[1,2,3]triazolo[4,5-c]quinolin-1-yl)-2-(cyanomethyl)piperidine-1-carboxylate

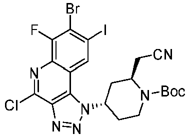

This compound was prepared according to the procedure described in **Example 14, Step** 1, replacing *tert*-butyl (*S*)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2S,4S)-4-((3-amino-7-bromo-2-chloro-8-fluoro-6-iodoquinolin-4-yl)amino)-2-(cyanomethyl)piperidine-1-carboxylate. LCMS calculated for C₂₁H₂₁BrClFlN₆O₂ (M+H)⁺: m/z = 649.0; found: 648.8.

### Step 4. tert-butyl (2S,4S)-4-(7-bromo-6-fluoro-8-iodo-4-(methylthio)-1H-[1,2,3]triazolo[4,5-c]quinolin-1-yl)-2-(cyanomethyl)piperidine-1-carboxylate

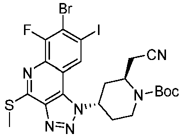

This compound was prepared according to the procedure described in **Example 80a and Example 80b, Step 4,** replacing *tert*-butyl (2*S*,4*S*)-4-(7-bromo-4,8-dichloro-6-fluoro-1*H-*imidazo[4,5-c]quinolin-1-yl)-2-(2-(*tert*-butoxy)-2-oxoethyl)piperidine-1-carboxylate with *tert-*butyl (2*S*,4*S*)-4-(7-bromo-4-chloro-6-fluoro-8-iodo-1*H*-[1,2,3]triazolo[4,5-*c*]quinolin-1-yl)-2-(cyanomethyl)piperidine-1-carboxylate. LCMS calculated for C₂₂H₂₄BrFlN₃O₂S (M+H)⁺: m/z = 661.0; found: 660.9.

### Step 5. tert-butyl (2S,4S)-4-(7-bromo-6-fluoro-8-methyl-4-(methylthio)-1H-[1,2,3]triazolo[4,5-c]quinolin-1-yl)-2-(cyanomethyl)piperidine-1-carboxylate

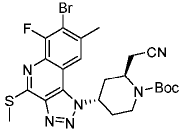

A screw-cap vial equipped with a magnetic stir bar was charged *tert*-butyl (2S,4S)-4-(7-bromo-6-fluoro-8-iodo-4-(methylthio)-1*H*-[1,2,3]triazolo[4,5-c]quinolin-1-yl)-2-(cyanomethyl)piperidine-1-carboxylate (1.19 g, 1.832 mmol), methylboronic acid (1.096 g, 18.32 mmol), tripotassium phosphate (1.166 g, 5.49 mmol), and bis(triphenylphosphine)-palladium(II) chloride (257 mg, 0.366 mmol) followed by dioxane (10.0 mL) and water (2.0 mL). The vial was sealed with a Teflon-lined septum, evacuated and backfilled with nitrogen (this process was repeated a total of three times). Then the reaction was stirred at 90 °C for 3 hours. After cooling to room temperature, the mixture was diluted with brine and extracted with EtOAc. The combined organic layers were dried over MgSO₄, filtered, concentrated to dryness, and purified on silica gel to yield the desired product. LCMS calculated for C₂₃H₂₇BrFN₃O₂S (M+H)⁺: m/z = 549.1; found: 549.1.

### Step 6. tert-butyl (2S,4S)-4-(7-(3-chloro-2-methylphenyl)-6-fluoro-8-methyl-4-(methylthio)-1H-[1,2,3]triazolo[4,5-c]quinolin-1-yl)-2-(cyanomethyl)piperidine-1-carboxylate

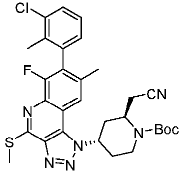

This compound was prepared according to the procedure described in **Example 91, Step 4,** replacing *tert*-butyl (2*S*,4*S*)-4-(7-bromo-8-chloro-6-fluoro-4-(methylthio)-1*H-*[1,2,3]triazolo[4,5-*c*]quinolin-1-yl)-2-(cyanomethyl)piperidine-1-carboxylate with *tert*-butyl (2*S*,4*S*)-4-(7-bromo-6-fluoro-8-methyl-4-(methylthio)-1*H*-[1,2,3]triazolo[4,5-*c*]quinolin-1-yl)-2-(cyanomethyl)piperidine-1-carboxylate. LCMS calculated for C₃₀H₃₃ClFN₆O₂S (M+H)⁺ m/z = 595.2; found 595.0.

### Step 7. tert-butyl (2S,4S)-4-(7-(3-chloro-2-methylphenyl)-6-fluoro-8-methyl-4-(((S)-1-methylpyrrolidin-2-yl)methoxy)-1H-[1,2,3]triazolo[4,5-c]quinolin-1-yl)-2-(cyanomethyl)piperidine-1-carboxylate

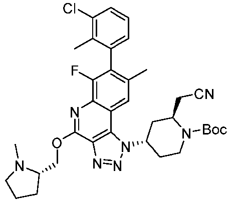

This compound was prepared according to the procedure described in **Example 91,** replacing morpholin-3-one with (S)-(1-methylpyrrolidin-2-yl)methanol and *tert*-butyl(2*S,*4*S*)-4-(8-chloro-7-(3-chloro-2-methylphenyl)-6-fluoro-4-(methylthio)-1H-[1,2,3]triazolo[4,5-c]quinolin-1-yl)-2-(cyanomethyl)piperidine-1-carboxylate with *tert*-butyl (2S,4S)-4-(7-(3-chloro-2-methylphenyl)-6-fluoro-8-methyl-4-(methylthio)-1*H*-[1,2,3]triazolo[4,5-*c*]quinolin-1-yl)-2-(cyanomethyl)piperidine-1-carboxylate in **Step 5.** LCMS calculated for C₃₅H₄₂ClFN₇O₃ (M+H)⁺ m/z = 662.3; found 662.2.

### Step 8. 2-((2S,4S)-1-acryloyl-4-(7-(3-chloro-2-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1*H*-[1,2,3]triazolo[4,5-*c*]quinolin-1-yl)-2-(cyanomethyl)-piperidine-1-carboxylate with tert-butyl (2S,4S)-4-(7-(3-chloro-2-methylphenyl)-6-fluoro-8-methyl-4-(((*S*)-1-methylpyrrolidin-2-yl)methoxy)-1*H*-[1 ,2,3]triazolo[4,5-c]quinolin-1-yl)-2-(cyanomethyl)piperidine-1-carboxylate. The product was isolated as a mixture of diastereomers. LCMS calculated for C₃₃H₃₆ClFN₇O₂ (M+H)⁺ m/z = 616.3; found 616.3.

### Example 101. 2-((2S,4S)-1-acryloyl-4-(7-(3-chloro-2-methylphenyl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile

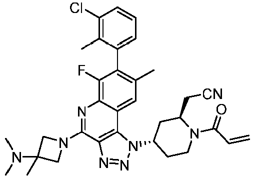

### Step 1. tert-butyl (2S,4S)-4-(7-(3-chloro-2-methylphenyl)-4-(3-(dimethylamino)-3-methylazetidin-1-yl)-6-fluoro-8-methyl-1H-[1,2,3]triazolo[4,5-c]quinolin-1-yl)-2-(cyanomethyl)piperidine-1-carboxylate

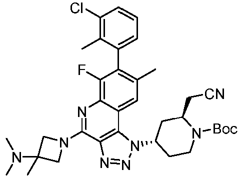

This compound was prepared according to the procedure described in **Example 80a and Example 80b, Step 10,** replacing *tert*-butyl (2*S*,4*S*)-4-(8-chloro-7-(6-chloro-5-methyl-1-(tetrahydro-2H-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with tert-butyl (2S,4S)-4-(7-(3-chloro-2-methylphenyl)-6-fluoro-8-methyl-4-(methylthio)-1*H*-[1,2,3]triazolo[4,5-*c*]quinolin-1-yl)-2-(cyanomethyl)piperidine-1-carboxylate (prepared according to **Example 98, Steps 1-6**). LCMS calculated for C₃₅H₄₃ClFN₈O₂ (M+H)⁺ m/z = 661.3; found 661.3.

### Step 2. 2-((2S,4S)-1-acryloyl-4-(7-(3-chloro-2-methylphenyl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1*H*-[1,2,3]triazolo[4,5-*c*]quinolin-1-yl)-2-(cyanomethyl)-piperidine-1-carboxylate with *tert*-butyl (2S,4S)-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)-2-(cyanomethyl)piperidine-1-carboxylate. The product was isolated as a mixture of diastereomers. LCMS calculated for C₃₃H₃₇ClFN₈O (M+H)⁺ m/z = 615.3; found 615.2.

### Example 105. 2-((2S,4S)-4-(7-(3-chloro-2-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile

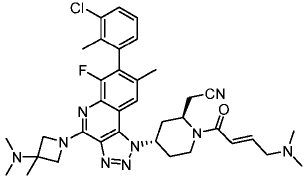

This compound was prepared according to the procedure described in **Example 88,** replacing *tert*-butyl (2S,4S)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-c]quinolin-1-yl)-2-(cyanomethyl)piperidine-1-carboxylate with *tert*-butyl (2S,4S)-4-(7-(3-chloro-2-methylphenyl)-4-(3-(dimethylamino)-3-methylazetidin-1-yl)-6-fluoro-8-methyl-1*H*-[1,2,3]triazolo[4,5-c]quinolin-1-yl)-2-(cyanomethyl)piperidine-1-carboxylate (as prepared from **Example 101, Step 1**). The product was isolated as a mixture of diastereomers. LCMS calculated for C₃₆H₄₄ClFN₉O (M+H)⁺ m/z = 672.3; found 672.2.

### Example 106. 2-((2S,4S)-1-acryloyl-4-(8-chloro-4-(3-(dimethylamino)azetidin-1-yl)-6-fluoro-7-(2-(trifluoromethyl)phenyl)-1H-imidazo[4,5-c]quinolin-1-yl)piperidin-2-yl)acetonitrile

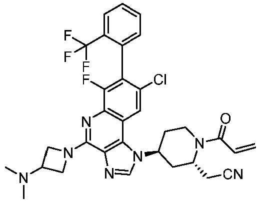

This compound was prepared according to the procedure described in **Example 16,** replacing (2,3-dimethylphenyl)boronic acid with (2-(trifluoromethyl)phenyl)boronic acid in **Step 6.** The product was isolated as a mixture of diastereomers. LCMS calculated for C₃₂H₃₁CIF₄N₇0 (M+H)⁺ m/z = 640.2; found 640.3

### Example 107. 2-((2S,4S)-1-acryloyl-4-(8-chloro-7-(2,3-dichlorophenyl)-4-(3-(dimethylamino)azetidin-1-yl)-6-fluoro-1H-imidazo[4,5-c]quinolin-1-yl)piperidin-2-yl)acetonitrile

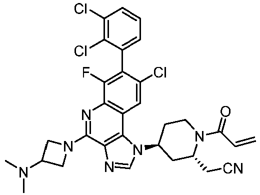

This compound was prepared according to the procedure described in **Example 16,** replacing (2,3-dimethylphenyl)boronic acid with (2,3-dichlorophenyl)boronic acid in **Step 6.** The product was isolated as a mixture of diastereomers. LCMS calculated for C₃₁H₃₀Cl₃FN₇O (M+H)⁺ m/z = 640.2 ; found 640.2

### Example 108. 2-((2S,4S)-1-acryloyl-4-(8-chloro-4-(3-(dimethylamino)azetidin-1-yl)-6-fluoro-7-(4-methylpyridin-3-yl)-1H-imidazo[4,5-c]quinolin-1-yl)piperidin-2-yl)acetonitrile

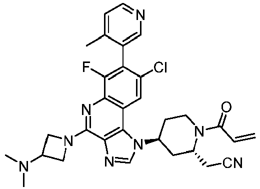

This compound was prepared according to the procedure described in **Example 16,** replacing (2,3-dimethylphenyl)boronic acid with 4-methyl-3-(4,4,5,5-tetramethyl-1,3,2-dioxaborolan-2-yl)pyridine in **Step 6.** The product was isolated as a mixture of diastereomers. LCMS calculated for C₃₁H₃₃ClFN₈O (M+H)⁺ m/z = 587.2 ; found 587.3

### Example 111. 2-((2S,4S)-1-acryloyl-4-(8-chloro-4-(3-(dimethylamino)azetidin-1-yl)-7-(2,3-dimethylphenyl)-6-fluoro-1H-[1,2,3]triazolo[4,5-c]quinolin-1-yl)piperidin-2-yl)acetonitrile

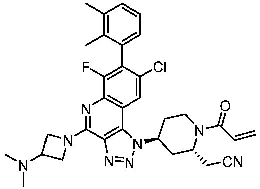

### Step 1. tert-butyl (2S,4S)-4-((7-bromo-6-chloro-2-(3-(dimethylamino)azetidin-1-yl)-8-fluoro-3-nitroquinolin-4-yl)amino)-2-(2-(tert-butoxy)-2-oxoethyl)piperidine-1-carboxylate

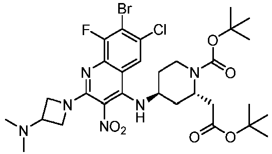

Et₃N (2.79 ml, 20.0 mmol) was added to a mixture of tert-butyl (2S,4S)-4-((7-bromo-2,6-dichloro-8-fluoro-3-nitroquinolin-4-yl)amino)-2-(2-(*tert*-butoxy)-2-oxoethyl)piperidine-1-carboxylate (3.26 g, 5.0 mmol) **(Example 80, Step 1)** and *N,N*-dimethylazetidin-3-amine (2 HCl salt) (0.751 g, 7.50 mmol) in AcCN (15.0 ml) and stirred at 50 °C for 1 h. The mixture was diluted with ethyl acetate and washed with aqueous Na₂CO₃, water, dried and concentrated to provide the desired product which was used in the next step directly. LCMS calculated for C₃₀H₄₂BrClFN₆O₆ (M+H)⁺ m/z = 717.2 ; found 717.4

### Step 2. tert-butyl (2S,4S)-4-((7-bromo-6-chloro-2-(3-(dimethylamino)azetidin-1-yl)-8-fluoro-3-nitroquinolin-4-yl)amino)-2-(2-(tert-butoxy)-2-oxoethyl)piperidine-1-carboxylate

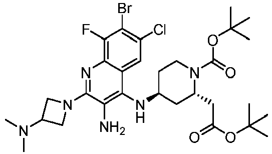

This compound was prepared according to the procedure described in **Example 15, step 2,** replacing tert-butyl (endo)-5-((7-bromo-6-chloro-2-(3-(dimethylamino)azetidin-1-yl)-8-fluoro-3-nitroquinolin-4-yl)amino)-2-azabicyclo[2.1.1]hexane-2-carboxylate with *tert-butyl* (2S,4S)-4-((7-bromo-6-chloro-2-(3-(dimethylamino)azetidin-1-yl)-8-fluoro-3-nitroquinolin-4-yl)amino)-2-(2-(*tert*-butoxy)-2-oxoethyl)piperidine-1-carboxylate. LCMS calculated for C₃₀H₄₄BrClFN₆O₄ (M+H)⁺: m/z = 687.1; found: 687.3.

### Step 3. tert-butyl (2S,4S)-4-(7-bromo-8-chloro-4-(3-(dimethylamino)azetidin-1-yl)-6-fluoro-1H-[1,2,3]triazolo[4,5-c]quinolin-1-yl)-2-(2-(tert-butoxy)-2-oxoethyl)piperidine-1-carboxylate

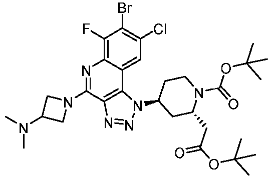

This compound was prepared according to the procedure described in **Example 14, Step** 1, replacing *tert*-butyl (S)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2S,4S)-4-((7-bromo-6-chloro-2-(3-(dimethylamino)azetidin-1-yl)-8-fluoro-3-nitroquinolin-4-yl)amino)-2-(2-(*tert*-butoxy)-2-oxoethyl)piperidine-1-carboxylate. LCMS calculated for C₃₀H₄₁BrClFN₇O₄ (M+H)⁺: m/z = 698.2; found: 698.1.

### Step 4. tert-butyl (2S,4S)-2-(2-amino-2-oxoethyl)-4-(7-bromo-8-chloro-4-(3-(dimethylamino)azetidin-1-yl)-6-fluoro-1H-[1,2,3]triazolo[4,5-c]quinolin-1-yl)piperidine-1-carboxylate

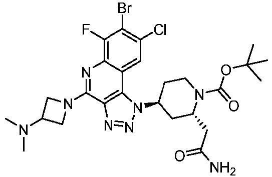

TFA (2.0 mL) was added to a solution of tert-butyl (2S,4S)-4-(7-bromo-8-chloro-4-(3-(dimethylamino)azetidin-1-yl)-6-fluoro-1*H*-[1,2,3]triazolo[4,5-c]quinolin-1-yl)-2-(2-(*tert-*butoxy)-2-oxoethyl)piperidine-1-carboxylate (0.5 g, 0.717 mmol) in CH₂Cl₂ (2.0 mL) and then stirred at rt for 5 h. The solvent was removed (switch with toluene for 3 times). The residue was redissoved in CH₂Cl₂ (8.0 ml) and then Boc₂O (0.250 ml, 1.076 mmol) was added followed by adding triethylamine (0.600 ml, 4.30 mmol) and stirred at rt for 2 h. The solvent was concentrated. The crude was redissolved in THF (10.0 ml) and triethylamine (0.600 ml, 4.30 mmol) was added followed by adding isobutyl chloroformate (0.141 ml, 1.076 mmol) at 0 °C and stirred for 15 min. At this time ammonia (1.0 mL) was added at 0 °C and the reaction was stirred for 10 min. To this mixture was added saturated NaHCO₃ and then extracted with CH₂Cl₂ the organic phase was dried and concentrated to provide the desired product which was used in the next step directly. LCMS calculated for C₂₃H₃₄BrClFN₃O₃ (M+H)⁺: m/z = 641.1; found: 641.1.

### Step 5. tert-butyl (2S,4S)-4-(7-bromo-8-chloro-4-(3-(dimethylamino)azetidin-1-yl)-6-fluoro-1H-[1,2,3]triazolo[4,5-c]quinolin-1-yl)-2-(cyanomethyl)piperidine-1-carboxylate

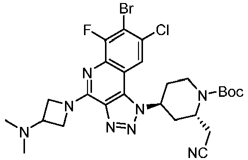

This compound was prepared according to the procedure described in **Example 80a and Example 80b, Step 8,** replacing tert-butyl (2S,4S)-2-(2-amino-2-oxoethyl)-4-(7-bromo-8-chloro-6-fluoro-4-(methylthio)-1H-imidazo[4,5-c]quinolin-1-yl)piperidine-1-carboxylate with tert-butyl (2S,4S)-2-(2-amino-2-oxoethyl)-4-(7-bromo-8-chloro-4-(3-(dimethylamino)azetidin-1-yl)-6-fluoro-1H-[1,2,3]triazolo[4,5-c]quinolin-1-yl)piperidine-1-carboxylate. The product was purified on silica gel (25 g, 0-15% MeOH in CH₂Cl₂). LCMS calculated for C₂₃H₃₂BrClFN₈O₂ (M+H)⁺: m/z = 623.1; found: 623.1.

### Step 6. 2-((2S,4S)-1-acryloyl-4-(7-bromo-8-chloro-4-(3-(dimethylamino)azetidin-1-yl)-6-fluoro-1H-[1,2,3]triazolo[4,5-c]quinolin-1-yl)piperidin-2-yl)acetonitrile

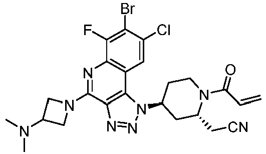

TFA (1.0 mL) was added to a solution of *tert*-butyl (2S,4S)-4-(7-bromo-8-chloro-4-(3-(dimethylamino)azetidin-1-yl)-6-fluoro-1H-[1,2,3]triazolo[4,5-c]quinolin-1-yl)-2-(cyanomethyl)piperidine-1-carboxylate (100.0 mg, 0.161 mmol) in CH₂Cl₂/MeOH (1.0/0.2 mL) and stirred at rt for 30 min. The solvent was removed under vacuum. The residue was dissolved in CH₂Cl₂ (4.0 ml) and cooled to 0 °C, to this was added triethylamine (134 µl, 0.965 mmol) followed by acryloyl chloride (29.1 mg, 0.322 mmol) and the reaction was stirred at 0 °C for 10 min. The reaction was quenched by adding saturated NaHCO₃ and extracted with CH₂Cl₂. The organic was dried and concentrated to provide the desired product which was used without further purification. LCMS calculated for C₂₄H₂₆BrClFN₈O (M+H)⁺: m/z = 577.1; found: 577.0.

### Step 7. 2-((2S,4S)-1-acryloyl-4-(8-chloro-4-(3-(dimethylamino)azetidin-1-yl)-7-(2,3-dimethylphenyl)-6-fluoro-1H-imidazo[4,5-c]quinolin-1-yl)piperidin-2-yl)acetonitrile

A screw-cap vial equipped with a magnetic stir bar was charged 2-((2*S*,4*S*)-1-acryloyl-4-(7-bromo-8-chloro-4-(3-(dimethylamino)azetidin-1-yl)-6-fluoro-1H-[1,2,3]triazolo[4,5-c]quinolin-1-yl)piperidin-2-yl)acetonitrile (10.0 mg, 0.017 mmol), (2,3-dimethylphenyl)boronic acid (7.5 mg, 0.05 mmol), potassium phosphate (11.06 mg, 0.052 mmol) and Pd(Ph₃P)₄ (2.7 mg, 1.736 µmol) in dioxane (0.8 mL)/water (0.2 mL). The vial was sealed with a Teflon-lined septum, evacuated and backfilled with nitrogen (this process was repeated a total of three times). And then the reaction was stirred at 105 °C for 1 h. The mixture was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₂H₃₅ClFN₈O (M+H)⁺ m/z = 601.3 ; found 601.3.

### Example 114. 2-((2S,4S)-1-acryloyl-4-(8-chloro-4-(3-(dimethylamino)azetidin-1-yl)-6-fluoro-7-(m-tolyl)-1H-[1,2,3]triazolo[4,5-c]quinolin-1-yl)piperidin-2-yl)acetonitrile

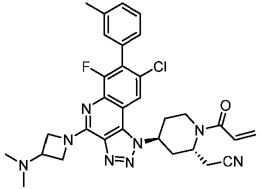

This compound was prepared according to the procedure described in **Example 111,** replacing (2,3-dimethylphenyl)boronic acid with m-tolylboronic acid in **Step 7.** LCMS calculated for C₃₁H₃₃ClFN₈O (M+H)⁺ m/z = 587.2 ; found 587.3.

### Example 115. 3-(1-((2S,4S)-1-acryloyl-2-(cyanomethyl)piperidin-4-yl)-8-chloro-4-(3-(dimethylamino)azetidin-1-yl)-6-fluoro-1H-[1,2,3]triazolo[4,5-c]quinolin-7-yl)-2-methylbenzonitrile

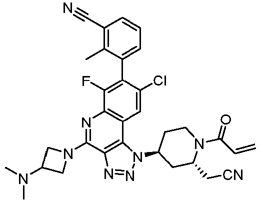

This compound was prepared according to the procedure described in **Example 111,** replacing (2,3-dimethylphenyl)boronic acid with (3-cyano-2-methylphenyl)boronic acid in **Step 7.** The product was isolated as a mixture of diastereomers. LCMS calculated for C₃₂H₃₂ClFN₉O (M+H)⁺ m/z = 612.3 ; found 612.4.

### Example 116. 2-((2S,4S)-1-acryloyl-4-(8-chloro-4-(3-(dimethylamino)azetidin-1-yl)-6-fluoro-7-(4-fluoro-2,3-dimethylphenyl)-1H-[1,2,3]triazolo[4,5-c]quinolin-1-yl)piperidin-2-yl)acetonitrile

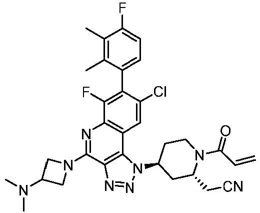

This compound was prepared according to the procedure described in **Example 111,** replacing (2,3-dimethylphenyl)boronic acid with (4-fluoro-2,3-dimethylphenyl)boronic acid in **Step 7.** The product was isolated as a mixture of diastereomers. LCMS calculated for C₃₂H₃₄ClF₂N₈O (M+H)⁺ m/z = 619.3 ; found 619.4.

### Example 118a and Example 118b. 2-((2S,4S)-1-acryloyl-4-(8-chloro-7-(3-chloro-2-methylphenyl)-6-fluoro-4-(((S)-1-methylpyrrolidin-2-yl)methoxy)-1H-imidazo[4,5-c]quinolin-1-yl)piperidin-2-yl)acetonitrile

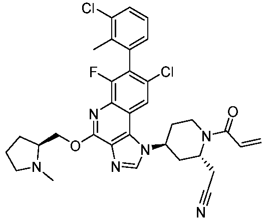

### Step 1. tert-butyl (2S,4S)-4-(8-chloro-7-(3-chloro-2-methylphenyl)-6-fluoro-4-(methylthio)-1H-imidazo[4, 5-c]quinolin-1-yl)-2-(cyanomethyl)piperidine-1-carboxylate

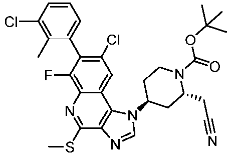

This compound was prepared according to the procedure described in **Example 80, Step 9,** replacing 6-chloro-5-methyl-1-(tetrahydro-2H-pyran-2-yl)-4-(4,4,5,5-tetramethyl-1,3,2-dioxaborolan-2-yl)-1H-indazole with (3-chloro-2-methylphenyl)boronic acid. The product was purified on silica gel (25 g, 0-80% EtOAc in hexane). LCMS calculated for C₃₀H₃₁C₂FN₅O₂S (M+H)⁺: m/z = 614.2; found: 614.3.

### Step 2. tert-butyl (2S,4S)-4-(8-chloro-7-(3-chloro-2-methylphenyl)-6-fluoro-4-(methylsulfonyl)-1H-imidazo[4,5-c]quinolin-1-yl)-2-(cyanomethyl)piperidine-1-carboxylate

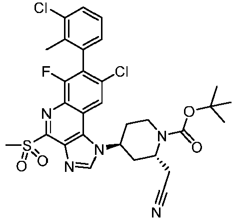

m-CPBA (95 mg, 0.553 mmol) was added to a solution of *tert*-butyl (2S,4S)-4-(8-chloro-7-(3-chloro-2-methylphenyl)-6-fluoro-4-(methylthio)-1H-imidazo[4,5-c]quinolin-1-yl)-2-(cyanomethyl)piperidine-1-carboxylate (170.0 mg, 0.277 mmol) in CH₂Cl₂ (3.0 ml) at 0 °C and then the reaction was stirred at this temperature for 10 min. The reaction was quenched by adding saturated Na₂S₂O₃, diluted with ethyl acetate and washed with saturated NaHCO₃, brine, filtered, dried and concentrated and the crude was used in the next step directly. LCMS calculated for C₃₀H₃₁C₂FN₅O₄S (M+H)⁺: m/z = 646.2; found: 646.2.

### Step 3. 2-((2S,4S)-1-acryloyl-4-(8-chloro-7-(3-chloro-2-methylphenyl)-6-fluoro-4-(((S)-1-methylpyrrolidin-2-yl)methoxy)-1H-imidazo[4,5-c]quinolin-1-yl)piperidin-2-yl)acetonitrile

Sodium *tert*-butoxide (9.0 mg, 0.093 mmol) was added to a solution of *tert*-butyl (2*S*,4*S*)-4-(8-chloro-7-(3-chloro-2-methylphenyl)-6-fluoro-4-(methylsulfonyl)-1*H*-imidazo[4,5-c]quinolin-1-yl)-2-(cyanomethyl)piperidine-1-carboxylate (15.0 mg, 0.023 mmol) and (S)-(1-methylpyrrolidin-2-yl)methanol (5.34 mg, 0.046 mmol) in THF (1.0 mL) and then the reaction was stirred at rt for 1 h. The mixture was diluted with ethyl acetate and washed with saturated NaHCO₃, water, filtered and concentrated.

The residue was dissolved in CH₂Cl₂ (0.4 mL) and then TFA (0.5 mL) was added and stirred at rt for 20 min. The solvent was removed. the residue was dissolved in CH₂Cl₂ (1.0 ml) and then triethylamine (16.17 µl, 0.116 mmol) was added followed by adding acryloyl chloride (6.30 mg, 0.070 mmol) at 0 °C and stirred for 10 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt.

**Example 118a.** Diastereomer 1. Peak 1. LCMS calculated for C₃₃H₃₄Cl₂FN₃O₂ (M+H)⁺ m/z = 635.2; found 635.3.

**Example 118b.** Diastereomer 2. Peak 2. LCMS calculated for C₃₃H₃₄Cl₂FN₃O₂ (M+H)⁺ m/z = 635.2; found 635.3

### Example 119a and Example 119b. 2-((2S,4S)-1-acryloyl-4-(8-chloro-7-(3-chloro-2-methylphenyl)-6-fluoro-4-(((R)-1-methylpyrrolidin-2-yl)methoxy)-1H-imidazo[4,5-c]quinolin-1-yl)piperidin-2-yl)acetonitrile

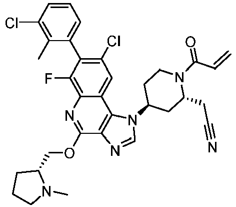

This compound was prepared according to the procedure described in **Example 118a and Example 118b,** replacing (*S*)-(1-methylpyrrolidin-2-yl)methanol with (R)-(1-methylpyrrolidin-2-yl)methanol in **Step 3.**

**Example 119a.** Diastereomer 1. Peak 1. LCMS calculated for C₃₃H₃₄Cl₂FN₃O₂ (M+H)⁺ m/z = 635.2; found 635.3.

**Example 119b.** Diastereomer 1. Peak 1. LCMS calculated for C₃₃H₃₄Cl₂FN₃O₂ (M+H)⁺ m/z = 635.2; found 635.3.

### Example 120. 2-((2S,4S)-1-acryloyl-4-(8-chloro-4-(3-(dimethylamino)-3-methylazetidin-1-yl)-6-fluoro-7-(4-fluorophenyl)-1H-imidazo[4,5-c]quinolin-1-yl)piperidin-2-yl)acetonitrile

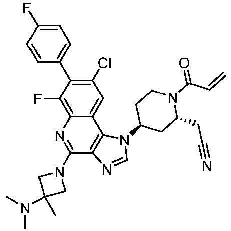

### Step 1. tert-butyl (2S,4S)-4-(8-chloro-6-fluoro-7-(4-fluorophenyl)-4-(methylthio)-1H-imidazo[4, 5-c]quinolin-1-yl)-2-(cyanomethyl)piperidine-1-carboxylate

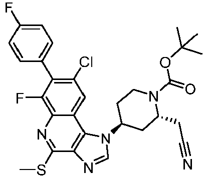

This compound was prepared according to the procedure described in **Example 118a and Example 118b,** replacing (3-chloro-2-methylphenyl)boronic acid with (4-fluorophenyl)boronic acid in **Step** 1. LCMS calculated for C₂₉H₂₉ClF₂N₅O₂S (M+H)⁺: m/z = 584.2; found: 584.2.

### Step 2. tert-butyl (2S,4S)-4-(8-chloro-6-fluoro-7-(4-fluorophenyl)-4-(methylsulfonyl)-1H-imidazo[4,5-c]quinolin-1-yl)-2-(cyanomethyl)piperidine-1-carboxylate

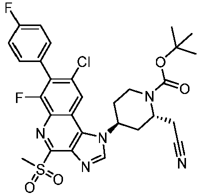

m-CPBA (100 mg, 0.582 mmol) was added to a solution of *tert*-butyl (2*S*,4*S*)-4-(8-chloro-6-fluoro-7-(4-fluorophenyl)-4-(methylthio)-1*H*-imidazo[4,5-*c*]quinolin-1-yl)-2-(cyanomethyl)piperidine-1-carboxylate (170.0 mg, 0.291 mmol) in CH₂Cl₂ (4.0 ml) at 0 °C and then the reaction was stirred at this temperature for 10 min. The reaction was quenched by adding saturated Na₂S₂O₃, diluted with ethyl acetate and washed with saturated NaHCO₃, brine, filtered, dried and concentrated to provide the crude product which was used in the next step directly. LCMS calculated for C₂₉H₂₉ClF₂N₅O₄S (M+H)⁺: m/z = 616.2; found: 616.2.

### Step 3. tert-butyl (2S,4S)-4-(8-chloro-4-(3-(dimethylamino)-3-methylazetidin-1-yl)-6-fluoro-7-(4-fluorophenyl)-1H-imidazo[4,5-c]quinolin-1-yl)-2-(cyanomethyl) piperidine-1-carboxylate

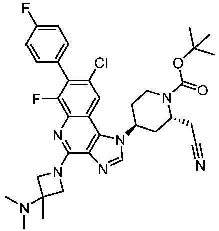

Et₃N (0.054 ml, 0.390 mmol) was added to a solution of *tert*-butyl (2S,4S)-4-(8-chloro-6-fluoro-7-(4-fluorophenyl)-4-(methylsulfonyl)-1*H*-imidazo[4,5-c]quinolin-1-yl)-2-(cyanomethyl)piperidine-1-carboxylate (0.060 g, 0.097 mmol) and *N,N*,3-trimethylazetidin-3-amine (2-HCI salt) (0.027 g, 0.146 mmol) in AcCN (1.5 mL) and then stirred at 70 °C for 2 h. The product was purified on silica gel (12 g, 0-15% MeOH in CH₂Cl₂). LCMS calculated for C₃₄H₃₉ClF₂N₇O₂ (M+H)⁺: m/z = 650.3; found: 650.4.

### Step 4. 2-((2S,4S)-4-(8-chloro-4-(3-(dimethylamino)-3-methylazetidin-1-yl)-6-fluoro-7-(4-fluorophenyl)-1H-imidazo[4,5-c]quinolin-1-yl)piperidin-2-yl)acetonitrile

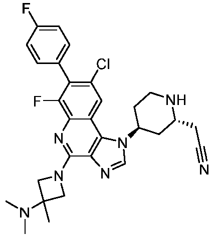

*tert*-Butyl (2*S*,4*S*)-4-(8-chloro-4-(3-(dimethylamino)-3-methylazetidin-1-yl)-6-fluoro-7-(4-fluorophenyl)-1*H*-imidazo[4,5-*c*]quinolin-1-yl)-2-(cyanomethyl)piperidine-1-carboxylate (60.0 mg) in CH₂Cl₂ (0.5 mL) was added TFA (0.5 mL) and the mixture was stirred at rt for 20 min. The solvent was removed to provide the desired product was TFA salt which was used in the next step directly. LCMS calculated for C₂₉H₃₁ClF₂N₇ (M+H)⁺: m/z = 550.2; found: 550.3.

### Step 5. 2-((2S,4S)-1-acryloyl-4-(8-chloro-4-(3-(dimethylamino)-3-methylazetidin-1-yl)-6-fluoro-7-(4-fluorophenyl)-1H-imidazo[4,5-c]quinolin-1-yl)piperidin-2-yl)acetonitrile

Acryloyl chloride (3.95 mg, 0.044 mmol) was added to a solution of 2-((2*S*,4*S*)-4-(8-chloro-4-(3-(dimethylamino)-3-methylazetidin-1-yl)-6-fluoro-7-(4-fluorophenyl)-1*H-*imidazo[4,5-c]quinolin-1-yl)piperidin-2-yl)acetonitrile (8.0 mg, 0.015 mmol) and Et₃N (12.16 µl, 0.087 mmol) in CH₂Cl₂ (1.0 mL) at 0 °C and then stirred for 10 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. LCMS calculated for C₃₂H₃₃ClF₂N₇O (M+H)⁺ m/z = 604.2; found 604.3.

### Example 121. 2-((2S,4S)-1-acryloyl-4-(4-(3-(dimethylamino)-3-methylazetidin-1-yl)-6-fluoro-7-(4-fluorophenyl)-8-methyl-1H-imidazo[4,5-c]quinolin-1-yl)piperidin-2-yl)acetonitrile

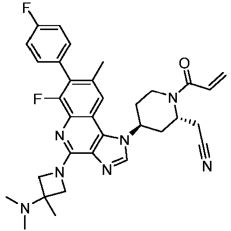

### Step 1. tert-butyl (2S,4S)-2-(cyanomethyl)-4-(4-(3-(dimethylamino)-3-methylazetidin-1-yl)-6-fluoro-7-(4-fluorophenyl)-8-methyl-1H-imidazo[4,5-c]quinolin-1-yl)piperidine-1-carboxylate

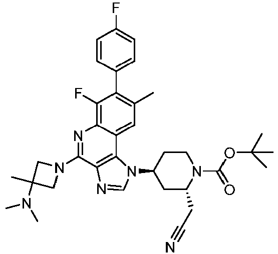

A mixture of tert-butyl (2S,4S)-4-(8-chloro-4-(3-(dimethylamino)-3-methylazetidin-1-yl)-6-fluoro-7-(4-fluorophenyl)-1H-imidazo[4,5-c]quinolin-1-yl)-2-(cyanomethyl)piperidine-1-carboxylate (50.0 mg, 0.077 mmol), methylboronic acid (48.0 mg, 0.8 mmol), potassium phosphate (49.0 mg, 0.231 mmol) and methanesulfonato(2-dicyclohexylphosphino-2',4',6'-tri-i-propyl-1,1'-biphenyl)(2'-methylamino-1,1'-biphenyl-2-yl)palladium(II) (XPhos Pd G4) (66.3 mg, 0.077 mmol) in dioxane (2.0 ml)/water (0.5 ml) was evacuated and backfilled with nitrogen (this process was repeated a total of three times). The reaction was then stirred at 90 °C 3 h. The mixture was diluted with ethyl acetate and washed with water. The organic phase was dried and concentrated. The product was purified on silica gel (12 g, 0-15% MeOH in CH₂Cl₂). LCMS calculated for C₃₅H₄₂F₂N₇O₂ (M+H)⁺ m/z = 630.3; found 630.2.

### Step 2. 2-((2S,4S)-4-(4-(3-(dimethylamino)-3-methylazetidin-1-yl)-6-fluoro-7-(4-fluorophenyl)-8-methyl-1H-imidazo[4,5-c]quinolin-1-yl)piperidin-2-yl)acetonitrile

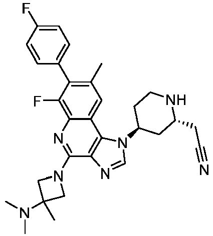

This compound was prepared according to the procedure described in **Example 120, Step 4,** replacing *tert*-Butyl (2*S*,4*S*)-4-(8-chloro-4-(3-(dimethylamino)-3-methylazetidin-1-yl)-6-fluoro-7-(4-fluorophenyl)-1*H*-imidazo[4,5-c]quinolin-1-yl)-2-(cyanomethyl)piperidine-1-carboxylate with *tert*-butyl (2S,4S)-2-(cyanomethyl)-4-(4-(3-(dimethylamino)-3-methylazetidin-1-yl)-6-fluoro-7-(4-fluorophenyl)-8-methyl-1*H*-imidazo[4,5-*c*]quinolin-1-yl)piperidine-1-carboxylate. LCMS calculated for C₃₀H₃₄F₂N₇ (M+H)⁺: m/z = 530.3; found: 530.3.

### Step 3. 2-((2S,4S)-1-acryloyl-4-(4-(3-(dimethylamino)-3-methylazetidin-1-yl)-6-fluoro-7-(4-fluorophenyl)-8-methyl-1H-imidazo[4,5-c]quinolin-1-yl)piperidin-2-yl)acetonitrile

This compound was prepared according to the procedure described in **Example 120, Step 5,** replacing 2-((2*S*,4*S*)-4-(8-chloro-4-(3-(dimethylamino)-3-methylazetidin-1-yl)-6-fluoro-7-(4-fluorophenyl)-1*H*-imidazo[4,5-c]quinolin-1-yl)piperidin-2-yl)acetonitrile with 2-((2*S*,4*S*)-4-(4-(3-(dimethylamino)-3-methylazetidin-1-yl)-6-fluoro-7-(4-fluorophenyl)-8-methyl-1*H*-imidazo[4,5-c]quinolin-1-yl)piperidin-2-yl)acetonitrile. LCMS calculated for C₃₃H₃₆F₂N₇O (M+H)⁺ m/z = 584.3 ; found 584.3. ¹H NMR (500 MHz, DMSO) δ 8.52 (s, 1H), 7.85 (s, 1H), 7.35 (s, 2H), 7.45 (s, 2H), 6.95 (m, 1H), 6.18 (d, 1H), 5.80 (d, 1H), 5.50 (m, 1H), 5.28 (m, 1H), 4.98 (m, 1H), 4.75 (m, 1H), 4.65 (m, 2H), 4.30 (m, 3H), 3.42 (m, 1H), 3.25 (m, 2H), 2.82 (s, 6H), 2.28 (s, 3H), 2.15 (m, 2H), 1.60 (s, 3H).

### Example 123. 2-((2S,4S)-1-acryloyl-4-(8-chloro-6-fluoro-7-(4-fluorophenyl)-4-(2-methyl-1H-imidazol-1-yl)-1H-imidazo[4,5-c]quinolin-1-yl)piperidin-2-yl)acetonitrile

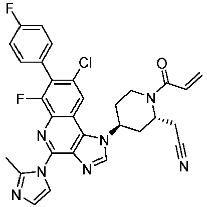

### Step 1. 2-((2S,4S)-4-(8-chloro-6-fluoro-7-(4-fluorophenyl)-4-(2-methyl-1H-imidazol-1-yl)-1H-imidazo[4, 5-c]quinolin-1-yl)piperidin-2-yl)acetonitrile

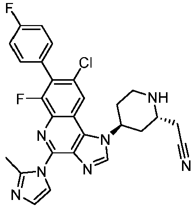

Sodium *tert*-butoxide (46.8 mg, 0.487 mmol) was added to a solution of *tert*-butyl (2S,4S)-4-(8-chloro-6-fluoro-7-(4-fluorophenyl)-4-(methylsulfonyl)-1*H*-imidazo[4,5-c]quinolin-1-yl)-2-(cyanomethyl)piperidine-1-carboxylate (75.0 mg, 0.122 mmol) and 2-methyl-1*H-*imidazole (19.99 mg, 0.243 mmol) in THF (2.0 mL) and then the reaction was stirred at rt for 1 h. The reaction was quenched by adding saturated NaHCO₃ and extracted with ethyl acetate. The organic was dried and concentrated. The crude was treated with CH₂Cl₂/TFA (0.8/0.8 mL) and stirred at rt for 30 min. The solvent was removed to provide the desired product as TFA salt which was used in the next step directly. LCMS calculated for C₂₇H₂₂ClF₂N₇ (M+H)⁺ m/z = 518.2; found 518.2

### Step 2. 2-((2S,4S)-1-acryloyl-4-(8-chloro-6-fluoro-7-(4-fluorophenyl)-4-(2-methyl-1H-imidazol-1-yl)-1H-imidazo[4,5-c]quinolin-1-yl)piperidin-2-yl)acetonitrile

This compound was prepared according to the procedure described in **Example 120, Step 5,** replacing 2-((2*S*,4*S*)-4-(8-chloro-4-(3-(dimethylamino)-3-methylazetidin-1-yl)-6-fluoro-7-(4-fluorophenyl)-1H-imidazo[4,5-c]quinolin-1-yl)piperidin-2-yl)acetonitrile with 2-((2*S*,4*S*)-4-(8-chloro-6-fluoro-7-(4-fluorophenyl)-4-(2-methyl-1*H*-imidazol-1-yl)-1H-imidazo[4,5-*c*]quinolin-1-yl)piperidin-2-yl)acetonitrile. LCMS calculated for C₃₀H₂₅ClF₂N₇O (M+H)⁺ m/z = 572.2 ; found 572.3.

### Example 126. 2-((2S,4S)-1-acryloyl-4-(8-chloro-4-(3-(dimethylamino)-3-methylazetidin-1-yl)-6-fluoro-7-(4-fluorophenyl)-1H-[1,2,3]triazolo[4,5-c]quinolin-1-yl)piperidin-2-yl)acetonitrile

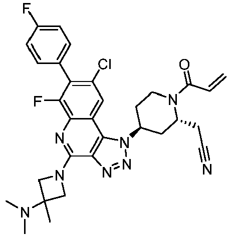

### Step 1. tert-butyl (2S,4S)-4-(8-chloro-6-fluoro-7-(4-fluorophenyl)-4-(methylthio)-1H-[1,2,3]triazolo[4,5-c]quinolin-1-yl)-2-(cyanomethyl)piperidine-1-carboxylate

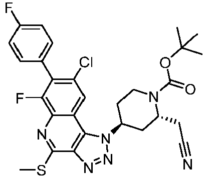

A mixture of *tert-*butyl (2*S*,4*S*)-4-(7-bromo-8-chloro-6-fluoro-4-(methylthio)-1*H-*[1,2,3]triazolo[4,5-c]quinolin-1-yl)-2-(cyanomethyl)piperidine-1-carboxylate (200 mg, 0.351 mmol) , (4-fluorophenyl)boronic acid (73.7 mg, 0.526 mmol), potassium phosphate (223 mg, 1.053 mmol) and Pd(Ph₃P)₄ (40.6 mg, 0.035 mmol) in dioxane (3 mL)/water (0.6 mL) was evacuated and backfilled with nitrogen (this process was repeated a total of three times). And then the reaction was stirred at 105 °C for 1 h. The mixture was diluted with ethyl acetate and washed with water, brine. The organic phase was filtered, dried and concentrated. The product was purified on silica gel (25 g, 0-80% EtOAc in hexane). LCMS calculated for C₂₃H₂₃ClF₂N₃O₂S (M+H)⁺: m/z = 585.2; found: 585.2.

### Step 2. tert-butyl (2S,4S)-4-(8-chloro-6-fluoro-7-(4-fluorophenyl)-4-(methylsulfonyl)-1H-[1,2,3]triazolo[4,5-c]quinolin-1-yl)-2-(cyanomethyl)piperidine-1-carboxylate

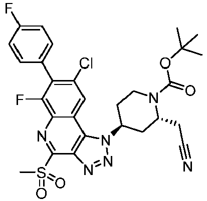

This compound was prepared according to the procedure described in **Example 120, Step 2,** replacing of *tert*-butyl (2S,4S)-4-(8-chloro-6-fluoro-7-(4-fluorophenyl)-4-(methylthio)-1*H*-imidazo[4,5-*c*]quinolin-1-yl)-2-(cyanomethyl)piperidine-1-carboxylate with tert-butyl (2*S*,4*S*)-4-(8-chloro-6-fluoro-7-(4-fluorophenyl)-4-(methylthio)-1*H*-[1,2,3]triazolo[4,5-*c*]quinolin-1-yl)-2-(cyanomethyl)piperidine-1-carboxylate. LCMS calculated for C₂₃H₂₃ClF₂N₃O₄S (M+H)⁺ m/z = 617.2 ; found 617.3.

### Step 3. tert-butyl (2S,4S)-4-(8-chloro-4-(3-(dimethylamino)-3-methylazetidin-1-yl)-6-fluoro-7-(4-fluorophenyl)-1H-[1,2,3]triazolo[4,5-c]quinolin-1-yl)-2-(cyanomethyl)piperidine-1-carboxylate

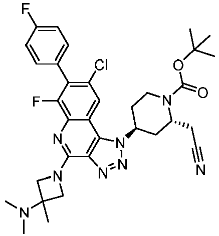

This compound was prepared according to the procedure described in **Example 120, Step 3,** replacing of of *tert*-butyl (2*S*,4*S*)-4-(8-chloro-6-fluoro-7-(4-fluorophenyl)-4-(methylsulfonyl)-1*H*-imidazo[4,5-*c*]quinolin-1-yl)-2-(cyanomethyl)piperidine-1-carboxylate with *tert*-butyl (2S,4S)-4-(8-chloro-6-fluoro-7-(4-fluorophenyl)-4-(methylsulfonyl)-1*H-*[1,2,3]triazolo[4,5-*c*]quinolin-1-yl)-2-(cyanomethyl)piperidine-1-carboxylate. LCMS calculated for C₃₃H₃₃ClF₂N₃O₂ (M+H)⁺ m/z = 651.3 ; found 651.3.

### Step 4. 2-((2S,4S)-4-(8-chloro-4-(3-(dimethylamino)-3-methylazetidin-1-yl)-6-fluoro-7-(4-fluorophenyl)-1H-[1,2,3]triazolo[4,5-c]quinolin-1-yl)piperidin-2-yl)acetonitrile

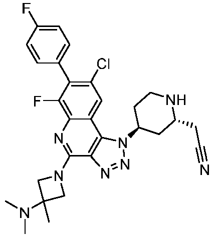

*tert*-butyl (2*S*,4*S*)-4-(8-chloro-4-(3-(dimethylamino)-3-methylazetidin-1-yl)-6-fluoro-7-(4-fluorophenyl)-1*H*-[1,2,3]triazolo[4,5-*c*]quinolin-1-yl)-2-(cyanomethyl)piperidine-1-carboxylate (30.0 mg) in CH₂Cl₂ (0.5 mL) was added TFA (0.5 mL) and the mixture was stirred at rt for 20 min. The solvent was removed to provide the desired product was TFA salt which was used in the next step directly. LCMS calculated for C₂₈H₃₀ClF₂N₈ (M+H)⁺ m/z = 551.2 ; found 551.2.

### Step 5. 2-((2S,4S)-1-acryloyl-4-(8-chloro-4-(3-(dimethylamino)-3-methylazetidin-1-yl)-6-fluoro-7-(4-fluorophenyl)-1H-[1,2,3]triazolo[4,5-c]quinolin-1-yl)piperidin-2-yl)acetonitrile

This compound was prepared according to the procedure described in **Example 120, Step 5,** replacing 2-((2*S*,4*S*)-4-(8-chloro-4-(3-(dimethylamino)-3-methylazetidin-1-yl)-6-fluoro-7-(4-fluorophenyl)-1*H*-imidazo[4,5-*c*]quinolin-1-yl)piperidin-2-yl)acetonitrile with 2-((2*S*,4*S*)-4-(8-chloro-4-(3-(dimethylamino)-3-methylazetidin-1-yl)-6-fluoro-7-(4-fluorophenyl)-1*H*-[1,2,3]triazolo[4,5-*c*]quinolin-1-yl)piperidin-2-yl)acetonitrile in. LCMS calculated for C₃₁H₃₂ClF₂N₈O (M+H)⁺ m/z = 605.2 ; found 605.3.

### Example 127. 2-((2S,4S)-1-acryloyl-4-(4-(3-(dimethylamino)-3-methylazetidin-1-yl)-6-fluoro-7-(4-fluorophenyl)-8-methyl-1H-[1,2,3]triazolo[4,5-c]quinolin-1-yl)piperidin-2-yl)acetonitrile

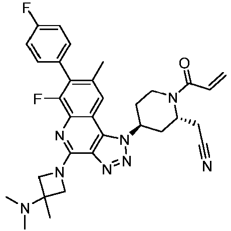

### Step 1. tert-butyl (2S,4S)-2-(cyanomethyl)-4-(4-(3-(dimethylamino)-3-methylazetidin-1-yl)-6-fluoro-7-(4-fluorophenyl)-8-methyl-1H-[1, 2, 3]triazolo[4, 5-c]quinolin-1-yl)piperidine-1-carboxylate

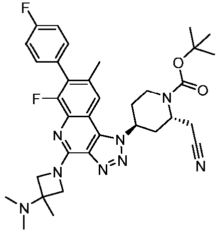

This compound was prepared according to the procedure described in **Example 121,** replacing *tert*-butyl (2*S*,4*S*)-4-(8-chloro-4-(3-(dimethylamino)-3-methylazetidin-1-yl)-6-fluoro-7-(4-fluorophenyl)-1*H*-imidazo[4,5-*c*]quinolin-1-yl)-2-(cyanomethyl)piperidine-1-carboxylate with tert-butyl (2*S*,4*S*)-4-(8-chloro-4-(3-(dimethylamino)-3-methylazetidin-1-yl)-6-fluoro-7-(4-fluorophenyl)-1*H*-[1,2,3]triazolo[4,5-*c*]quinolin-1-yl)-2-(cyanomethyl)piperidine-1-carboxylate in **Step** 1. LCMS calculated for C₃₄H₄₁F₂N₈O₂ (M+H)⁺ m/z = 631.3 ; found 631.3.

### Step 2. 2-((2S,4S)-4-(4-(3-(dimethylamino)-3-methylazetidin-1-yl)-6-fluoro-7-(4-fluorophenyl)-8-methyl-1H-[1,2,3]triazolo[4,5-c]quinolin-1-yl)piperidin-2-yl)acetonitrile

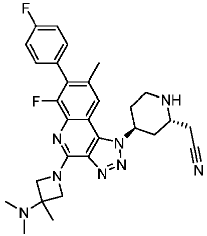

*tert*-butyl (2*S*,4*S*)-2-(cyanomethyl)-4-(4-(3-(dimethylamino)-3-methylazetidin-1-yl)-6-fluoro-7-(4-fluorophenyl)-8-methyl-1*H*-[1,2,3]triazolo[4,5-*c*]quinolin-1-yl)piperidine-1-carboxylate (40.0 mg) in CH₂Cl₂ (0.5 mL) was added TFA (0.5 mL) and the mixture was stirred at rt for 20 min. The solvent was removed to provide the desired product was TFA salt which was used in the next step directly. LCMS calculated for C₂₉H₃₃F₂N₈ (M+H)⁺ m/z = 531.3 ; found 531.3.

### Step 3. 2-((2S,4S)-1-acryloyl-4-(4-(3-(dimethylamino)-3-methylazetidin-1-yl)-6-fluoro-7-(4-fluorophenyl)-8-methyl-1H-[1,2,3]triazolo[4,5-c]quinolin-1-yl)piperidin-2-yl)acetonitrile

This compound was prepared according to the procedure described in **Example 120, Step 5,** replacing 2-((2*S*,4*S*)-4-(8-chloro-4-(3-(dimethylamino)-3-methylazetidin-1-yl)-6-fluoro-7-(4-fluorophenyl)-1*H*-imidazo[4,5-*c*]quinolin-1-yl)piperidin-2-yl)acetonitrile with 2-((2*S*,4*S*)-4-(4-(3-(dimethylamino)-3-methylazetidin-1-yl)-6-fluoro-7-(4-fluorophenyl)-8-methyl-1*H*-[1,2,3]triazolo[4,5-*c*]quinolin-1-yl)piperidin-2-yl)acetonitrile. LCMS calculated for C₃₂H₃₅F₂N₈O (M+H)⁺ m/z = 585.3 ; found 585.4.

### Example 128. 2-((2S,4S)-1-acryloyl-4-(8-chloro-4-(((S)-1-(dimethylamino)propan-2-yl)oxy)-6-fluoro-7-(4-fluorophenyl)-1H-[1,2,3]triazolo[4,5-c]quinolin-1-yl)piperidin-2-yl)acetonitrile

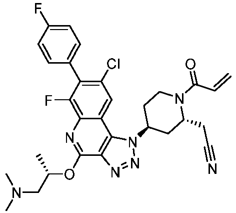

### Step 1. 2-((2S,4S)-4-(8-chloro-4-(((S)-1-(dimethylamino)propan-2-yl)oxy)-6-fluoro-7-(4-fluorophenyl)-1H-[1, 2, 3]triazolo[4, 5-c]quinolin-1-yl)piperidin-2-yl)acetonitrile

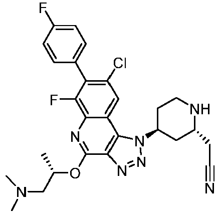

Sodium tert-butoxide (46.7 mg, 0.486 mmol) was added to a solution of tert-butyl (2*S*,4*S*)-4-(8-chloro-6-fluoro-7-(4-fluorophenyl)-4-(methylsulfonyl)-1*H*-[1,2,3]triazolo[4,5-c]quinolin-1-yl)-2-(cyanomethyl)piperidine-1-carboxylate (75.0 mg, 0.122 mmol) and (S)-1-(dimethylamino)propan-2-ol (25.08 mg, 0.243 mmol) in THF (2.0 mL) and then the reaction was stirred at rt for 1 h. The mixture was diluted with ethyl acetate and washed with saturated NaHCO₃, water, filtered and concentrated. The residue was dissolved in CH₂Cl₂ (0.4 mL) and then TFA (0.5 mL) was added and stirred at rt for 20 min. The solvent was removed and the residue was used in the next step directly. LCMS calculated for C₂₇H₂₀ClF₂N₇O (M+H)⁺ m/z = 540.2 ; found 540.3.

### Step 2. 2-((2S,4S)-1-acryloyl-4-(8-chloro-4-(((S)-1-(dimethylamino)propan-2-yl)oxy)-6-fluoro-7-(4-fluorophenyl)-1H-[1,2,3]triazolo[4,5-c]quinolin-1-yl)piperidin-2-yl)acetonitrile

This compound was prepared according to the procedure described in **Example 120, Step 5,** replacing 2-((2*S*,4*S*)-4-(8-chloro-4-(3-(dimethylamino)-3-methylazetidin-1-yl)-6-fluoro-7-(4-fluorophenyl)-1*H*-imidazo[4,5-*c*]quinolin-1-yl)piperidin-2-yl)acetonitrile with 2-((2*S*,4*S*)-4-(8-chloro-4-(((*S*)-1-(dimethylamino)propan-2-yl)oxy)-6-fluoro-7-(4-fluorophenyl)-1*H*-[1,2,3]triazolo[4,5-*c*]quinolin-1-yl)piperidin-2-yl)acetonitrile in. LCMS calculated for C₃₀H₃₁ClF₂N₇O₂ (M+H)⁺ m/z = 594.2 ; found 594.3.

### Example 129. 2-((2S,4S)-1-(but-2-ynoyl)-4-(8-chloro-4-(((S)-1-(dimethylamino)propan-2-yl)oxy)-6-fluoro-7-(4-fluorophenyl)-1H-[1,2,3]triazolo[4,5-c]quinolin-1-yl)piperidin-2-yl)acetonitrile

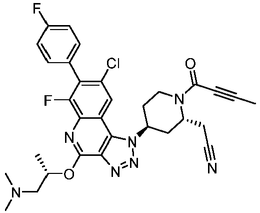

Triethylamine (10.32 µl, 0.074 mmol) was added to a solution of 2-((2S,4S)-4-(8-chloro-4-(((*S*)-1-(dimethylamino)propan-2-yl)oxy)-6-fluoro-7-(4-fluorophenyl)-1*H-*[1,2,3]triazolo[4,5-c]quinolin-1-yl)piperidin-2-yl)acetonitrile (8.0 mg, 0.015 mmol), but-2-ynoic acid (3.74 mg, 0.044 mmol) and 1-propanephosphonic acid cyclic anhydride (T₃P 50% in EtOAc) (13.23 µl, 0.044 mmol) in ethyl acetate (0.8 mL) at 0 °C and then the reaction was stirred for 30 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. LCMS calculated for C₃₁H₃₁ClF₂N₇O₂ (M+H)⁺ m/z = 606.2; found 606.3.

### Example 130. 2-((2S,4S)-4-(8-chloro-4-(((S)-1-(dimethylamino)propan-2-yl)oxy)-6-fluoro-7-(4-fluorophenyl)-1H-[1,2,3]triazolo[4,5-c]quinolin-1-yl)-1-((E)-4-fluorobut-2-enoyl)piperidin-2-yl)acetonitrile

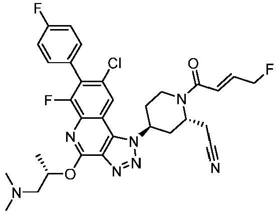

This compound was prepared according to the procedure described in **Example 129,** replacing but-2-ynoic acid with (E)-4-fluorobut-2-enoic acid. LCMS calculated for C₃₁H₃₂ClF₃N₇O₂ (M+H)⁺ m/z = 626.2 ; found 626.3.

### Example 131. 2-((2S,4S)-1-acryloyl-4-(8-chloro-6-fluoro-7-(4-fluorophenyl)-4-(((S)-1-methylpyrrolidin-2-yl)methoxy)-1H-[1,2,3]triazolo[4,5-c]quinolin-1-yl)piperidin-2-yl)acetonitrile

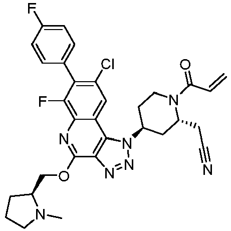

### Step 1. 2-((2S,4S)-4-(8-chloro-6-fluoro-7-(4-fluorophenyl)-4-(((S)-1-methylpyrrolidin-2-yl)methoxy)-1H-[1,2,3]triazolo[4,5-c]quinolin-1-yl)piperidin-2-yl)acetonitrile

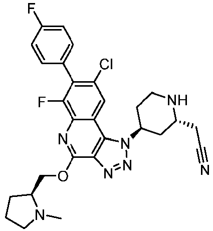

This compound was prepared according to the procedure described in **Example 128,** replacing (S)-1-(dimethylamino)propan-2-ol with (S)-(1-methylpyrrolidin-2-yl)methanol in **Step 1.** LCMS calculated for C₂₈H₂₉ClF₂N₇O (M+H)⁺ m/z = 552.2 ; found 552.3.

### Step 2 2-((2S,4S)-1-acryloyl-4-(8-chloro-6-fluoro-7-(4-fluorophenyl)-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 120, Step 5,** replacing 2-((2*S*,4*S*)-4-(8-chloro-4-(3-(dimethylamino)-3-methylazetidin-1-yl)-6-fluoro-7-(4-fluorophenyl)-1H-imidazo[4,5-c]quinolin-1-yl)piperidin-2-yl)acetonitrile with 2-((2*S*,4*S*)-4-(8-chloro-6-fluoro-7-(4-fluorophenyl)-4-(((*S*)-1-methylpyrrolidin-2-yl)methoxy)-1*H-*[1,2,3]triazolo[4,5-*c*]quinolin-1-yl)piperidin-2-yl)acetonitrile. LCMS calculated for C₃₁H₃₁ClF₂N₇O₂ (M+H)⁺ m/z = 606.2 ; found 606.3.

### Example 132. 2-((2S,4S)-1-acryloyl-4-(4-(((S)-1-(dimethylamino)propan-2-yl)oxy)-6-fluoro-7-(4-fluorophenyl)-8-methyl-1H-[1,2,3]triazolo[4,5-c]quinolin-1-yl)piperidin-2-yl)acetonitrile

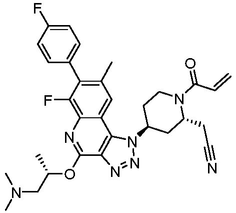

### Step 1. tert-butyl (2S,4S)-2-(cyanomethyl)-4-(6-fluoro-7-(4-fluorophenyl)-8-methyl-4-(methylthio)-1H-[1,2,3]triazolo[4,5-c]quinolin-1-yl)piperidine-1-carboxylate

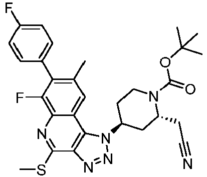

A mixture of *tert*-butyl (2*S*,4*S*)-4-(7-bromo-6-fluoro-8-methyl-4-(methylthio)-1*H-*[1,2,3]triazolo[4,5-c]quinolin-1-yl)-2-(cyanomethyl)piperidine-1-carboxylate **(Example 98, Step 5)** (200 mg, 0.364 mmol), (4-fluorophenyl)boronic acid (76 mg, 0.546 mmol), potassium phosphate (232 mg, 1.092 mmol) and Pd(Ph₃P)₄ (42.1 mg, 0.036 mmol) in dioxane (3 mL)/water (0.6 mL) was evacuated and backfilled with nitrogen (this process was repeated a total of three times). And then the reaction was stirred at 105 °C for 1 h. The mixture was diluted with ethyl acetate and washed with water, brine. The organic phase was filtered, dried and concentrated. The product was purified on silica gel (12 g, 0-60% EtOAc in hexane). LCMS calculated for C₂₉H₃₁F₂N₆O₂S (M+H)⁺: m/z = 565.2; found: 565.1.

### Step 2. tert-butyl (2S,4S)-2-(cyanomethyl)-4-(6-fluoro-7-(4-fluorophenyl)-8-methyl-4-(methylsulfonyl)-1H-[1,2,3]triazolo[4, 5-c]quinolin-1-yl)piperidine-1-carboxylate

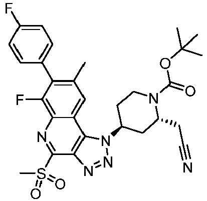

This compound was prepared according to the procedure described in **Example 120, Step 2,** replacing of tert-butyl (2S,4S)-4-(8-chloro-6-fluoro-7-(4-fluorophenyl)-4-(methylthio)-1*H*-imidazo[4,5-*c*]quinolin-1-yl)-2-(cyanomethyl)piperidine-1-carboxylate with tert-butyl (2*S*,4*S*)-2-(cyanomethyl)-4-(6-fluoro-7-(4-fluorophenyl)-8-methyl-4-(methylthio)-1*H-*[1,2,3]triazolo[4,5-*c*]quinolin-1-yl)piperidine-1-carboxylate. LCMS calculated for C₂₉H₃₁F₂N₆O₄S (M+H)⁺ m/z = 597.2 ; found 597.3.

### Step 3. 2-((2S,4S)-4-(4-(((S)-1-(dimethylamino)propan-2-yl)oxy)-6-fluoro-7-(4-fluorophenyl)-8-methyl-1H-[1,2,3]triazolo[4,5-c]quinolin-1-yl)piperidin-2-yl)acetonitrile

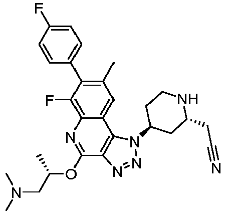

Sodium tert-butoxide (46.7 mg, 0.486 mmol) was added to a solution of *tert*-butyl (2*S*,4*S*)-2-(cyanomethyl)-4-(6-fluoro-7-(4-fluorophenyl)-8-methyl-4-(methylsulfonyl)-1*H-*[1,2,3]triazolo[4,5-*c*]quinolin-1-yl)piperidine-1-carboxylate (75.0 mg, 0.122 mmol) and (S)-1-(dimethylamino)propan-2-ol (25.08 mg, 0.243 mmol) in THF (2.0 ml) and then the reaction was stirred at rt for 1 h. The mixture was diluted with ethyl acetate and washed with saturated NaHCO₃, water, filtered and concentrated. The residue was dissolved in CH₂Cl₂ (1.0 mL) and then TFA (1.0 mL) was added and stirred at rt for 20 min. The solvent was removed and the residue was used in the next step directly. LCMS calculated for C₂₃H₃₂F₂N₇O (M+H)⁺ m/z = 520.3 ; found 520.3.

### Step 4. 2-((2S,4S)-1-acryloyl-4-(4-(((S)-1-(dimethylamino)propan-2-yl)oxy)-6-fluoro-7-(4-fluorophenyl)-8-methyl-1H-[1,2,3]triazolo[4,5-c]quinolin-1-yl)piperidin-2-yl)acetonitrile

Acryloyl chloride (4.18 mg, 0.046 mmol) was added to a solution of 2-((2S,4S)-4-(4-(((S)-1-(dimethylamino)propan-2-yl)oxy)-6-fluoro-7-(4-fluorophenyl)-8-methyl-1H-[1,2,3]triazolo[4,5-c]quinolin-1-yl)piperidin-2-yl)acetonitrile (8.0 mg, 0.015 mmol) and triethylamine (12.88 µl, 0.092 mmol) in CH₂Cl₂ (1.0 ml) at 0 °C and then stirred for 10 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. LCMS calculated for C₃₁H₃₄F₂N₇O₂ (M+H)⁺ m/z = 574.3; found 574.3. ¹H NMR (500 MHz, DMSO) δ 8.20 (s, 1H), 7.48 (s, 2H), 7.40 (s, 2H), 6.95 (m, 1H), 6.22 (d, 1H), 6.05 (m, 1H), 5.80 (m, 1H), 5.75 (d, 1H), 5.21 (m, 1H), 4.50 (m, 1H), 3.52 (m, 7H), 2.92 (s, 3H), 2.88 (s, 3H), 2.42 (m, 2H), 2.38 (s, 3H), 1.51 (s, 3H).

### Example 133. 2-((2S,4S)-1-(but-2-ynoyl)-4-(4-(((S)-1-(dimethylamino)propan-2-yl)oxy)-6-fluoro-7-(4-fluorophenyl)-8-methyl-1H-[1,2,3]triazolo[4,5-c]quinolin-1-yl)piperidin-2-yl)acetonitrile

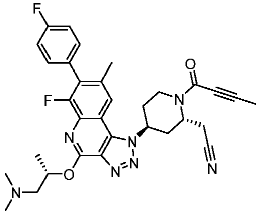

Triethylamine (10.73 µl, 0.077 mmol) was added to a solution of 2-((2S,4S)-4-(4-(((S)-1-(dimethylamino)propan-2-yl)oxy)-6-fluoro-7-(4-fluorophenyl)-8-methyl-1H-[1,2,3]triazolo[4,5-c]quinolin-1-yl)piperidin-2-yl)acetonitrile (8.0 mg, 0.015 mmol), but-2-ynoic acid (3.88 mg, 0.046 mmol) and 1-propanephosphonic acid cyclic anhydride (T₃P 50% in EtOAc) (13.75 µl, 0.046 mmol) in ethyl acetate (0.8 ml) at 0 °C and then the reaction was stirred for 30 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. LCMS calculated for C₃₂H₃₄F₂N₇O₂ (M+H)⁺ m/z = 586.3; found 586.4.

### Example 134. 2-((2S,4S)-4-(4-(((S)-1-(dimethylamino)propan-2-yl)oxy)-6-fluoro-7-(4-fluorophenyl)-8-methyl-1H-[1,2,3]triazolo[4,5-c]quinolin-1-yl)-1-((E)-4-fluorobut-2-enoyl)piperidin-2-yl)acetonitrile

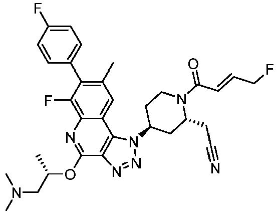

This compound was prepared according to the procedure described in **Example 133,** replacing but-2-ynoic acid with (*E*)-4-fluorobut-2-enoic acid. LCMS calculated for C₃₂H₃₅F₃N₇O₂ (M+H)⁺ m/z = 606.3 ; found 606.4.

### Example 135. 2-((2S,4S)-1-acryloyl-4-(6-fluoro-7-(4-fluorophenyl)-8-methyl-4-(((S)-1-methylpyrrolidin-2-yl)methoxy)-1H-[1,2,3]triazolo[4,5-c]quinolin-1-yl)piperidin-2-yl)acetonitrile

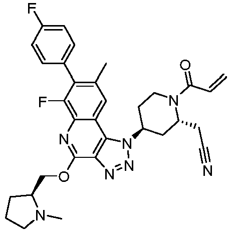

### Step 1. 2-((2S,4S)-4-(6-fluoro-7-(4-fluorophenyl)-8-methyl-4-(((S)-1-methylpyrrolidin-2-yl)methoxy)-1H-[1,2,3]triazolo[4,5-c]quinolin-1-yl)piperidin-2-yl)acetonitrile

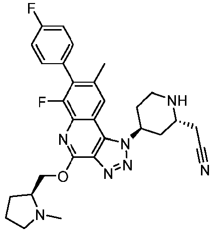

The compound was prepared according to the procedure described in **Example 132, Step 3,** replacing (*S*)-1-(dimethylamino)propan-2-ol with (*S*)-(1-methylpyrrolidin-2-yl)methanol. LCMS calculated for C₂₉H₃₂F₂N₇O (M+H)⁺ m/z = 532.3 ; found 532.3.

### Step 2. 2-((2S,4S)-1-acryloyl-4-(6-fluoro-7-(4-fluorophenyl)-8-methyl-4-(((S)-1-methylpyrrolidin-2-yl)methoxy)-1H-[1,2,3]triazolo[4,5-c]quinolin-1-yl)piperidin-2-yl)acetonitrile

The compound was prepared according to the procedure described in **Example 132, Step 4,** replacing of 2-((2S,4S)-4-(4-(((S)-1-(dimethylamino)propan-2-yl)oxy)-6-fluoro-7-(4-fluorophenyl)-8-methyl-1H-[1,2,3]triazolo[4,5-*c*]quinolin-1-yl)piperidin-2-yl)acetonitrile with 2-((2*S*,4*S*)-4-(6-fluoro-7-(4-fluorophenyl)-8-methyl-4-(((*S*)-1-methylpyrrolidin-2-yl)methoxy)-1H-[1,2,3]triazolo[4,5-*c*]quinolin-1-yl)piperidin-2-yl)acetonitrile. LCMS calculated for C₃₂H₃₄F₂N₇O₂ (M+H)⁺ m/z = 586.3 ; found 586.4.

### Example 136. 2-((2S,4S)-4-(6-fluoro-7-(4-fluorophenyl)-8-methyl-4-(((S)-1-methylpyrrolidin-2-yl)methoxy)-1H-[1,2,3]triazolo[4,5-c]quinolin-1-yl)-1-((E)-4-fluorobut-2-enoyl)piperidin-2-yl)acetonitrile

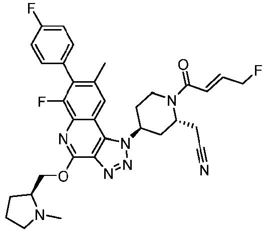

Triethylamine (10.49 µl, 0.075 mmol) was added to a solution of 2-((2*S*,4*S*)-4-(6-fluoro-7-(4-fluorophenyl)-8-methyl-4-(((*S*)-1-methylpyrrolidin-2-yl)methoxy)-1*H-*[1,2,3]triazolo[4,5-c]quinolin-1-yl)piperidin-2-yl)acetonitrile (8.0 mg, 0.015 mmol), (*E*)-4-fluorobut-2-enoic acid (4.70 mg, 0.045 mmol) and 1-propanephosphonic acid cyclic anhydride (T₃P 50% in EtOAc) (13.44 µl, 0.045 mmol) in ethyl acetate (0.8 mL) at 0 °C and then the reaction was stirred for 30 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. LCMS calculated for C₃₃H₃₅F₃N₇O₂ (M+H)⁺ m/z = 618.3; found 618.4.

### Example 137. 2-((2S,4S)-1-acryloyl-4-(7-(2,3-dimethylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile

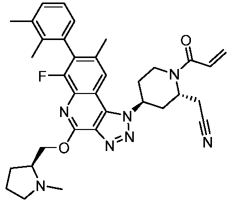

### Step 1. tert-butyl (2S,4S)-2-(cyanomethyl)-4-(7-(2,3-dimethylphenyl)-6-fluoro-8-methyl-4-(methylthio)-1H-[1,2,3]triazolo[4, 5-c]quinolin-1-yl)piperidine-1-carboxylate

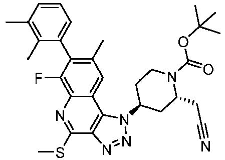

The compound was prepared according to the procedure described in **Example 132, Step 1,** replacing (4-fluorophenyl)boronic acid with (2,3-dimethylphenyl)boronic acid in. LCMS calculated for C₃₁H₃₆FN₆O₂S (M+H)⁺ m/z = 575.3 ; found 575.3.

### Step 2. tert-butyl (2S,4S)-2-(cyanomethyl)-4-(7-(2,3-dimethylphenyl)-6-fluoro-8-methyl-4-(methylsulfonyl)-1H-[1,2,3]triazolo[4,5-c]quinolin-1-yl)piperidine-1-carboxylate

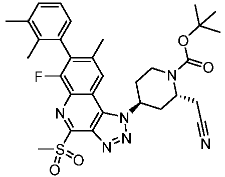

m-CPBA (102 mg, 0.592 mmol) was added to a solution of *tert*-butyl (2*S*,4*S*)-2-(cyanomethyl)-4-(7-(2,3-dimethylphenyl)-6-fluoro-8-methyl-4-(methylthio)-1*H-*[1,2,3]triazolo[4,5-c]quinolin-1-yl)piperidine-1-carboxylate (170.0 mg, 0.296 mmol) in CH₂Cl₂ (4.0 mL) at 0 °C and then the reaction was stirred at this temperature for 20 min. The reaction was quenched by adding saturated Na₂S₂O₃, diluted with ethyl acetate and washed with saturated NaHCO₃, brine, filtered, dried and concentrated and the crude was used in the next step directly. LCMS calculated for C₃₁H₃₆FN₆O₄S (M+H)⁺ m/z = 607.2 ; found 607.3.

### Step 3. 2-((2S,4S)-4-(7-(2,3-dimethylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile

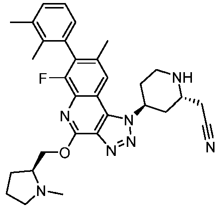

Sodium *tert*-butoxide (47.5 mg, 0.494 mmol) was added to a solution of tert-butyl (2*S*,4*S*)-2-(cyanomethyl)-4-(7-(2,3-dimethylphenyl)-6-fluoro-8-methyl-4-(methylsulfonyl)-1*H-*[1,2,3]triazolo[4,5-c]quinolin-1-yl)piperidine-1-carboxylate (75.0 mg, 0.124 mmol) and (*S*)-(1-methylpyrrolidin-2-yl)methanol (28.5 mg, 0.247 mmol) in THF (2.0 mL) and then the reaction was stirred at rt for 1 h. The mixture was diluted with ethyl acetate and washed with saturated NaHCO₃, water, filtered and concentrated. The residue was dissolved in CH₂Cl₂ (0.8 mL) and then TFA (1.0 mL) was added and stirred at rt for 20 min. The solvent was removed and the residue was used in the next step directly. LCMS calculated for C₃₁H₃₇FN₇O (M+H)⁺ m/z = 542.3 ; found 542.4.

### Step 4. 2-((2S,4S)-1-acryloyl-4-(7-(2,3-dimethylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile

Acryloyl chloride (4.01 mg, 0.044 mmol) was added to a solution of 2-((2*S*,4*S*)-4-(7-(2,3-dimethylphenyl)-6-fluoro-8-methyl-4-(((*S*)-1-methylpyrrolidin-2-yl)methoxy)-1*H-*[1,2,3]triazolo[4,5-c]quinolin-1-yl)piperidin-2-yl)acetonitrile (8.0 mg, 0.015 mmol) and triethylamine (12.5 µl, 0.089 mmol) in CH₂Cl₂ (1.0 ml) at 0 °C and then stirred for 10 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₄H₃₉FN₇O₂ (M+H)⁺ m/z = 596.3; found 596.4.

### Example 138. 2-((2S,4S)-4-(7-(2,3-dimethylphenyl)-6-fluoro-8-methyl-4-(((S)-1-methylpyrrolidin-2-yl)methoxy)-1H-[1,2,3]triazolo[4,5-c]quinolin-1-yl)-1-((E)-4-fluorobut-2-enoyl)piperidin-2-yl)acetonitrile

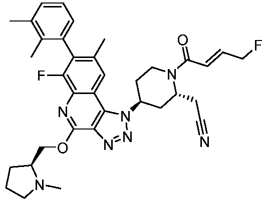

Triethylamine (10.29 µl, 0.074 mmol) was added to a solution of 2-((2*S*,4*S*)-4-(7-(2,3-dimethylphenyl)-6-fluoro-8-methyl-4-(((*S*)-1-methylpyrrolidin-2-yl)methoxy)-1*H-*[1,2,3]triazolo[4,5-*c*]quinolin-1-yl)piperidin-2-yl)acetonitrile (8.0 mg, 0.015 mmol), (*E*)-4-fluorobut-2-enoic acid (4.61 mg, 0.044 mmol) and 1-propanephosphonic acid cyclic anhydride (T₃P 50% in EtOAc) (13.19 µl, 0.044 mmol) in ethyl acetate (0.8 ml) at 0 °C and then the reaction was stirred for 30 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₅H₄₀F₂N₇O₂ (M+H)⁺ m/z = 628.3; found 628.4.

### Example 142. 2-((2S,4S)-1-acryloyl-4-(4-(((S)-1-(dimethylamino)propan-2-yl)oxy)-7-(2,3-dimethylphenyl)-6-fluoro-8-methyl-1H-[1,2,3]triazolo[4,5-c]quinolin-1-yl)piperidin-2-yl)acetonitrile

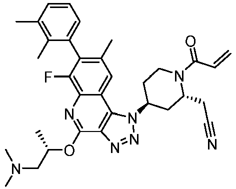

### Step 1. 2-((2S,4S)-4-(4-(((S)-1-(dimethylamino)propan-2-yl)oxy)-7-(2,3-dimethylphenyl)-6-fluoro-8-methyl-1H-[1,2,3]triazolo[4,5-c]quinolin-1-yl)piperidin-2-yl)acetonitrile

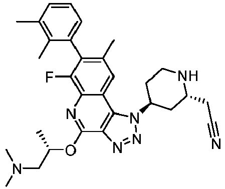

The compound was prepared according to the procedure described in **Example 137,** replacing (*S*)-(1-methylpyrrolidin-2-yl)methanol with (S)-1-(dimethylamino)propan-2-ol in **Step 3.** LCMS calculated for C₃₀H₃₇FN₇O (M+H)⁺ m/z = 530.3 ; found 530.3.

### Step 2 2-((2S,4S)-1-acryloyl-4-(4-(((S)-1-(dimethylamino)propan-2-yl)oxy)-7-(2,3-dimethylphenyl)-6-fluoro-8-methyl-1H-[1,2,3]triazolo[4,5-c]quinolin-1-yl)piperidin-2-yl)acetonitrile

Acryloyl chloride (4.10 mg, 0.045 mmol) was added to a solution of 2-((2*S*,4*S*)-4-(4-*(((S)-1-(dimethylamino)propan-2-yl)oxy)-7-(2,3-dimethylphenyl)-6-fluoro-8-methyl-1H-*[1,2,3]triazolo[4,5-c]quinolin-1-yl)piperidin-2-yl)acetonitrile (8.0 mg, 0.015 mmol) and triethylamine (12.63 µl, 0.091 mmol) in CH₂Cl₂ (1.0 ml) at 0 °C and then stirred for 10 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₃H₃₉FN₇O₂ (M+H)⁺ m/z = 584.3; found 584.3.

### Example 143. 2-((2S,4S)-4-(4-(((S)-1-(dimethylamino)propan-2-yl)oxy)-7-(2,3-dimethylphenyl)-6-fluoro-8-methyl-1H-[1,2,3]triazolo[4,5-c]quinolin-1-yl)-1-((E)-4-fluorobut-2-enoyl)piperidin-2-yl)acetonitrile

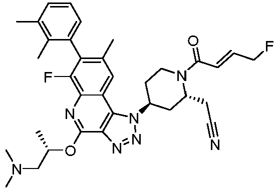

Triethylamine (10.53 µl, 0.076 mmol) was added to a solution of 2-((2*S*,4*S*)-4-(4-(((*S*)-1-(dimethylamino)propan-2-yl)oxy)-7-(2,3-dimethylphenyl)-6-fluoro-8-methyl-1*H-*[1,2,3]triazolo[4,5-c]quinolin-1-yl)piperidin-2-yl)acetonitrile (8.0 mg, 0.015 mmol), (*E*)-4-fluorobut-2-enoic acid (4.72 mg, 0.045 mmol) and 1-Propanephosphonic acid cyclic anhydride (T₃P 50% in EtOAc) (13.49 µl, 0.045 mmol) in ethyl acetate (0.8 mL) at 0 °C and then the reaction was stirred for 30 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₄H₄₀F₂N₇O₂ (M+H)⁺ m/z = 616.3; found 616.4.

### Example 145. 2-((2S,4S)-4-(4-(((S)-1-(dimethylamino)propan-2-yl)oxy)-7-(2,3-dimethylphenyl)-6-fluoro-8-methyl-1H-[1,2,3]triazolo[4,5-c]quinolin-1-yl)-1-((E)-4-methoxybut-2-enoyl)piperidin-2-yl)acetonitrile

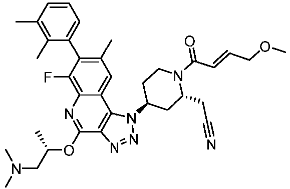

The compound was prepared according to the procedure described in **Example 143,** replacing (*E*)-4-fluorobut-2-enoic acid with (*E*)-4-methoxybut-2-enoic acid. The product was isolated as a mixture of diastereomers. LCMS calculated for C₃₅H₄₃FN₇O₃ (M+H)⁺ m/z = 628.3 ; found 628.5.

### Example 146. 2-((2S,4S)-1-(but-2-ynoyl)-4-(4-(((S)-1-(dimethylamino)propan-2-yl)oxy)-7-(2,3-dimethylphenyl)-6-fluoro-8-methyl-1H-[1,2,3]triazolo[4,5-c]quinolin-1-yl)piperidin-2-yl)acetonitrile

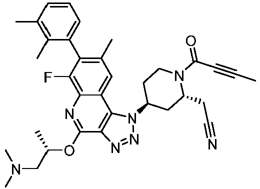

The compound was prepared according to the procedure described in **Example 143,** replacing (E)-4-fluorobut-2-enoic acid with but-2-ynoic acid. The product was isolated as a mixture of diastereomers. LCMS calculated for C₃₄H₃₉FN₇O₂ (M+H)⁺ m/z = 596.3 ; found 596.4.

### Example 147. 2-((2S,4S)-1-acryloyl-4-(4-(3-(dimethylamino)azetidin-1-yl)-7-(2,3-dimethylphenyl)-6-fluoro-8-methyl-1H-[1,2,3]triazolo[4,5-c]quinolin-1-yl)piperidin-2-yl)acetonitrile

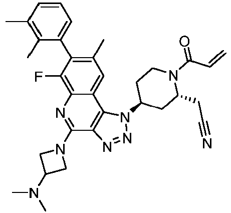

### Step 1. 2-((2S,4S)-4-(4-(3-(dimethylamino)azetidin-1-yl)-7-(2,3-dimethylphenyl)-6-fluoro-8-methyl-1H-[1,2,3]triazolo[4,5-c]quinolin-1-yl)piperidin-2-yl)acetonitrile

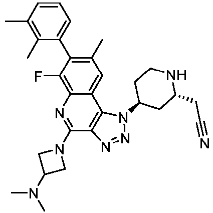

Triethylamine (0.074 ml, 0.527 mmol) was added to a solution of tert-butyl (2S,4S)-2-(cyanomethyl)-4-(7-(2,3-dimethylphenyl)-6-fluoro-8-methyl-4-(methylsulfonyl)-1*H-*[1,2,3]triazolo[4,5-c]quinolin-1-yl)piperidine-1-carboxylate (0.080 g, 0.132 mmol) and *N,N,-*dimethylazetidin-3-amine (0.023 g, 0.21 mmol) and then stirred at 70 °C for 2 h. The Boc protected intermediate was purified on silica gel (12 g, 0-15% MeOH in CH₂Cl₂) which was then treated with CH₂Cl₂/TFA (1.0/1.0 mL) at rt for 30 min. The solvent was removed and the product was used in the next step directly. LCMS calculated for C₃₀H₃₆FN₈ (M+H)⁺ m/z = 527.3 ; found 527.3.

### Step 2. 2-((2S,4S)-1-acryloyl-4-(4-(3-(dimethylamino)azetidin-1-yl)-7-(2,3-dimethylphenyl)-6-fluoro-8-methyl-1H-[1,2,3]triazolo[4,5-c]quinolin-1-yl)piperidin-2-yl)acetonitrile

Acryloyl chloride (5.16 mg, 0.057 mmol) was added to a solution of 2-((2*S*,4*S*)-4-(4-*(3-(dimethylamino)azetidin-1-yl)-7-(2,3-dimethylphenyl)-6-fluoro-8-methyl-1H-*[1,2,3]triazolo[4,5-c]quinolin-1-yl)piperidin-2-yl)acetonitrile (10.0 mg, 0.019 mmol) and triethylamine (15.88 µl, 0.114 mmol) in CH₂Cl₂ (1.0 mL) at 0 °C and then stirred for 10 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₃H₃₃FN₃O (M+H)⁺ m/z = 518.3; found 518.4.

### Example 149. 2-((2S,4S)-4-(8-chloro-4-(3-(dimethylamino)azetidin-1-yl)-7-(2,3-dimethylphenyl)-6-fluoro-1H-imidazo[4,5-c]quinolin-1-yl)-1-((E)-4-fluorobut-2-enoyl)piperidin-2-yl)acetonitrile

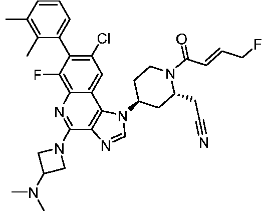

### Step 1. tert-butyl (2S,4S)-4-(8-chloro-4-(3-(dimethylamino)azetidin-1-yl)-7-(2,3-dimethylphenyl)-6-fluoro-1H-imidazo[4,5-c]quinolin-1-yl)-2-(cyanomethyl)piperidine-1-carboxylate

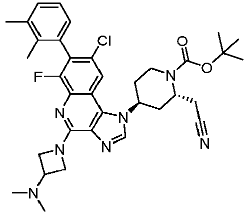

A mixture of *tert*-butyl (2S,4S)-4-(7-bromo-8-chloro-4-(3-(dimethylamino)azetidin-1-yl)-6-fluoro-1H-imidazo[4,5-c]quinolin-1-yl)-2-(cyanomethyl)piperidine-1-carboxylate (60.0 mg, 0.097 mmol), (2,3-dimethylphenyl)boronic acid (21.74 mg, 0.145 mmol), potassium phosphate (61.5 mg, 0.290 mmol) and Pd(Ph₃P)₄ (11.17 mg, 9.66 µmol) in dioxane (3 ml)/water (0.6 ml) evacuated and backfilled with nitrogen (this process was repeated a total of three times). And then the reaction was stirred at 105 °C for 1 h. The mixture was diluted with ethyl acetate and washed with water, brine. The organic phase was filtered, dried and concentrated. The product was purified on silica gel (12 g, 0-15% MeOH in CH₂Cl₂). LCMS calculated for C₃₄H₄₁ClFN₈O2 (M+H)⁺: m/z = 646.3; found: 646.2.

### Step 2. 2-((2S,4S)-4-(8-chloro-4-(3-(dimethylamino)azetidin-1-yl)-7-(2,3-dimethylphenyl)-6-fluoro-1H-imidazo[4, 5-c]quinolin-1-yl)piperidin-2-yl)acetonitrile

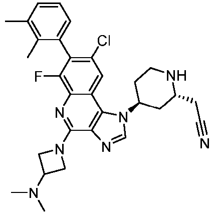

TFA (1.0 mL) was added to a solution of *tert*-butyl (2*S*,4*S*)-4-(8-chloro-4-(3-(dimethylamino)azetidin-1-yl)-7-(2,3-dimethylphenyl)-6-fluoro-1*H*-imidazo[4,5-*c*]quinolin-1-yl)-2-(cyanomethyl)piperidine-1-carboxylate (60.0 mg, 0.093 mmol) in CH₂Cl₂/MeOH (1.0/0.1 mL) and stirred at rt for 30 min. The solvent was removed and the product was used in the next step directly. LCMS calculated for C₂₉H₃₃ClFN₈ (M+H)⁺: m/z = 546.3; found: 546.5.

### Step 3. 2-((2S,4S)-4-(8-chloro-4-(3-(dimethylamino)azetidin-1-yl)-7-(2,3-dimethylphenyl)-6-fluoro-1H-imidazo[4,5-c]quinolin-1-yl)-1-((E)-4-fluorobut-2-enoyl)piperidin-2-yl)acetonitrile

Triethylamine (10.21 µl, 0.073 mmol) was added to a solution of 2-((2*S*,4*S*)-4-(8-chloro-4-(3-(dimethylamino)azetidin-1-yl)-7-(2,3-dimethylphenyl)-6-fluoro-1*H*-imidazo[4,5-*c*]quinolin-1-yl)piperidin-2-yl)acetonitrile (8.0 mg, 0.015 mmol), (*E*)-4-fluorobut-2-enoic acid (4.57 mg, 0.044 mmol) and 1-Propanephosphonic acid cyclic anhydride (T₃P 50% in EtOAc) (13.08 µl, 0.044 mmol) in ethyl acetate (0.8 ml) at 0 °C and then the reaction was warmed to rt for 30 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₄H₃₇ClF₂N₇O (M+H)⁺ m/z = 632.3; found 632.2.

### Example 150a and Example 150b. 2-((2S,4S)-1-acryloyl-4-(8-chloro-7-(6-chloro-1,5-dimethyl-1H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile

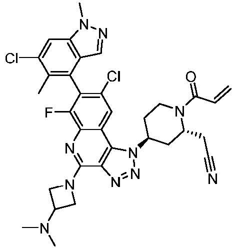

### Step 1 4-bromo-6-chloro-1,5-dimethyl-1H-indazole

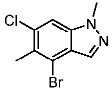

NaH (0.036 g, 1.500 mmol) was added to a solution of 4-bromo-6-chloro-5-methyl-1H-indazole (0.246 g, 1.0 mmol) in DMF (4.0 ml) at 0 °C and then stirred for 15 min, at this time iodomethane (0.2 ml, 3.0 mmol) was added to the mixture and the reaction was stirred for 30 min.The reaction was quenched by adding saturated NH₄Cl and extracted with ethyl acetate. The product was purified on silica gel (12 g, 0-50% EtOAc in hexane). LCMS calculated for C₉H₉BrClN₂ (M+H)⁺: m/z = 261.0; found: 261.0.

### Step 2 6-chloro-1,5-dimethyl-4-(4,4,5,5-tetramethyl-1,3,2-dioxaborolan-2-yl)-1H-indazole

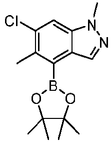

A mixture of 4,4,5,5,4',4',5',5'-Octamethyl-[2,2']bi[[1,3,2]dioxaborolanyl] (0.044 g, 0.173 mmol), potassium acetate (0.024 g, 0.241 mmol), 4-bromo-6-chloro-1,5-dimethyl-1H-indazole (0.025 g, 0.096 mmol) and PdCl₂(dppf) (7.05 mg, 9.63 µmol) in 1,4-dioxane (4.0 mL) was evacuated and backfilled with nitrogen (this process was repeated a total of three times). And then the reaction was stirred at 105 °C for 3 h. The mixture was diluted with CH₂Cl₂ and filtered. The filtrate was concentrated and the product was purified on silica gel (12 g, 0-60% EtOAc in hexane). LCMS calculated for C₁₅H₂₁BClN₂O₂ (M+H)⁺: m/z = 307.1; found: 307.1.

### Step 3 2-((2S,4S)-1-acryloyl-4-(8-chloro-7-(6-chloro-1,5-dimethyl-1H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile

A mixture of 2-((2*S*,4*S*)-1-acryloyl-4-(7-bromo-8-chloro-4-(3-(dimethylamino)azetidin-1-yl)-6-fluoro-1*H*-[1,2,3]triazolo[4,5-*c*]quinolin-1-yl)piperidin-2-yl)acetonitrile (30.0 mg, 0.052 mmol) , 6-chloro-1,5-dimethyl-4-(4,4,5,5-tetramethyl-1,3,2-dioxaborolan-2-yl)-1*H*-indazole (23.96 mg, 0.078 mmol), potassium phosphate (33.2 mg, 0.156 mmol) and Pd(Ph₃P)₄ (6.02 mg, 5.21 µmol) in dioxane (1.8 ml)/ater (0.4 ml) was evacuated and backfilled with nitrogen (this process was repeated a total of three times) and then the reaction was stirred at 105 °C for 3 h. The mixture was diluted with acetonitrile/water and purified using prep-LCMS (XBridge C18 column, eluting with a gradient of acetonitrile/water containing 0.1% TFA, at flow rate of 60 mL/min) to afford the desired products as TFA salt.

**Example 150a.** Diastereomer 1. Peak 1. LCMS calculated for C₃₃H₃₄Cl₂FN₁₀O (M+H)⁺ m/z = 675.2; found 675.4.

**Example 150b.** Diastereomer 2. Peak 2.. LCMS calculated for C₃₃H₃₄Cl₂FN₁₀O (M+H)⁺ m/z = 675.2; found 675.4.

### Example 151. 2-((2S,4S)-4-(8-chloro-6-fluoro-4-(2-methyl-1H-imidazol-1-yl)-7-phenyl-1H-imidazo[4,5-c]quinolin-1-yl)-1-((E)-4-(dimethylamino)but-2-enoyl)piperidin-2-yl)acetonitrile

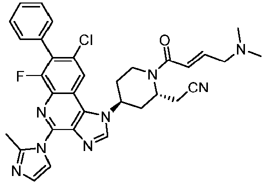

### Step 1. tert-butyl (2S,4S)-4-(8-chloro-6-fluoro-4-(methylthio)-7-phenyl-1H-imidazo[4,5-c]quinolin-1-yl)-2-(cyanomethyl)piperidine-1-carboxylate

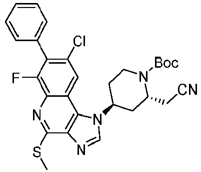

This compound was prepared according to the procedure described in **Example 80a and Example 80b, step 9.** LCMS calculated for C₂₉H₃₀ClFN₅O₂S (M+H)⁺: m/z = 566.1; found: 566.3.

### Step 2. 2-((2S,4S)-4-(8-chloro-6-fluoro-4-(2-methyl-1H-imidazol-1-yl)-7-phenyl-1H-imidazo[4,5-c]quinolin-1-yl)piperidin-2-yl)acetonitrile

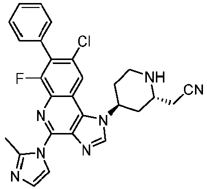

A solution of *tert-*butyl (2*S*,4*S*)-4-(8-chloro-7-(6-chloro-5-methyl-1-(tetrahydro-2*H-*pyran-2-yl)-1*H*-indazol-4-yl)-6-fluoro-4-(methylthio)-1*H*-imidazo[4,5-*c*]quinolin-1-yl)-2-(cyanomethyl)piperidine-1-carboxylate (350.0 mg, 0.618 mmol) in CH₂Cl₂ (10.0 mL) was cooled to 0 °C. *m*-CPBA (160 mg, 0.927 mmol) was added in one portion and the mixture was stirred at 0 °C for an additional 10 minutes. The mixture was then diluted with sat'd NaHCO₃ and extracted with CH₂Cl₂. The combined organic layers were washed with 0.5 N NaOH aqueous solution, dried over MgSO₄, filtered, and concentrated to dryness.

To a solution of above residue (75 mg, 0.129 mmol) in Dioxane (3.0 ml) was added 2-methyl-1H-imidazole (21.16 mg, 0.258 mmol) and tripotassium phosphate (82 mg, 0.387 mmol). The mixture was heated at 70 °C overnight. After cooling to room temperature, the mixture was filtered and concentrated to dryness. The residue was purified by silica gel column eluted with 0 to 10 % MeOH/DCM to afford the desired product.

The desired product was dissolved in DCM (2mL), then added TFA (2.0 mL) and stir at r.t. 1 hour to remove Boc. The mixture was concentrated to dryness and go to next step directly. LCMS calculated for C₂₇H₂₄ClFN₇ (M+H)⁺: m/z = 500.2; found: 500.2.

### Step 3. 2-((2S,4S)-4-(8-chloro-6-fluoro-4-(2-methyl-1H-imidazol-1-yl)-7-phenyl-1H-imidazo[4,5-c]quinolin-1-yl)-1-((E)-4-(dimethylamino)but-2-enoyl)piperidin-2-yl)acetonitrile

To a solution of2-((2S,4S)-4-(8-chloro-6-fluoro-4-(2-methyl-1H-imidazol-1-yl)-7-phenyl-1H-imidazo[4,5-c]quinolin-1-yl)piperidin-2-yl)acetonitrile (10 mg, 0.020 mmol) in CH₂Cl₂ (2.0 ml) was added (*E*)-4-(dimethylamino)but-2-enoic acid (7.8 mg, 0.060 mmol) , T3P (23.57 µl, 0.040 mmol) and TEA (16.73 µl, 0.120 mmol). The reaction was stirred at r.t. 20 min, the reaction was concentrated, then diluted with methanol and purified using prep-LCMS (pH=10) to afford the desired product. LCMS calculated for C₃₃H₃₃ClFN₃O (M+H)⁺ m/z = 611.2; found 611.2.

### Example 155. 2-((2S,4S)-1-acryloyl-4-(8-chloro-6-fluoro-4-(((S)-1-methylpyrrolidin-2-yl)methoxy)-7-phenyl-1H-[1,2,3]triazolo[4,5-c]quinolin-1-yl)piperidin-2-yl)acetonitrile

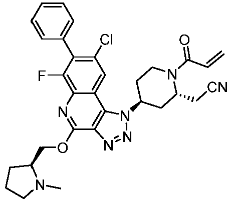

### Step 1. 2-((2S,4S)-4-(8-chloro-6-fluoro-4-(((S)-1-methylpyrrolidin-2-yl)methoxy)-7-phenyl-1H-[1,2, 3]triazolo[4, 5-c]quinolin-1-yl)piperidin-2-yl)acetonitrile

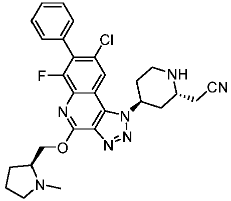

A solution of tert-butyl (2S,4S)-4-(8-chloro-6-fluoro-4-(methylthio)-7-phenyl-1H-[1,2,3]triazolo[4,5-c]quinolin-1-yl)-2-(cyanomethyl)piperidine-1-carboxylate (65.0 mg, 0.115 mmol) in CH₂Cl₂ (5.0 mL) was cooled to 0 °C. *m*-CPBA (29.7 mg, 0.172 mmol) was added in one portion and the mixture was stirred at 0 °C for an additional 10 minutes. The mixture was then diluted with sat'd NaHCO₃ and extracted with CH₂Cl₂. The combined organic layers were washed with 0.5N NaOH aqueous solution, dried over MgSO₄, filtered, and concentrated to dryness.

To a solution of above residue (77.0 mg, 0.129 mmol) and (S)-(1-methylpyrrolidin-2-yl)methanol (29.6 mg, 0.257 mmol) in THF (2.0 ml) was added Sodium tert-butoxide (30.9 mg, 0.321 mmol). The reaction mixture was stirred at r.t. for 1 h. The mixture was diluted with ethyl acetate and washed with saturated NaHCO₃, water, the organic phase was dried over MgSO₄, filtered and concentrated. The residue was purified by silica gel column eluted with 0 to 15% MeOH/DCM to afford the desired product.

The desired product was dissolved in DCM (2.0 mL), then added TFA (2.0 mL) and stir at r.t. 1 hour to remove Boc. The mixture was concentrated to dryness and go to next step directly. LCMS calculated for C₂₈H₃₀ClFN₇O (M+H)⁺: m/z 534.2; found: 534.3

### Step 2. 2-((2S,4S)-1-acryloyl-4-(8-chloro-6-fluoro-4-(((S)-1-methylpyrrolidin-2-yl)methoxy)-7-phenyl-1H-[1,2,3]triazolo[4,5-c]quinolin-1-yl)piperidin-2-yl)acetonitrile

This compound was prepared according to the procedure described in **Example 151, Steps 3.** LCMS calculated for C₃₁H₃₂ClFN₇O₂ (M+H)⁺: m/z = 588.2; found: 588.3.

### Example 157a and Example 157b. 2-((2S,4S)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)-1-((E)-4-methoxybut-2-enoyl)piperidin-2-yl)acetonitrile

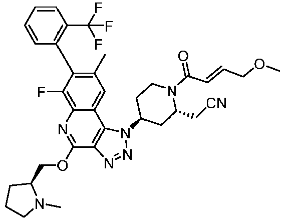

### Step 1. tert-butyl (2S,4S)-2-(cyanomethyl)-4-(6-fluoro-8-methyl-4-(methylthio)-7-(2-(trifluoromethyl) phenyl)-1H-[1, 2, 3]triazolo[4, 5-c]quinolin-1-yl)piperidine-1-carboxylate

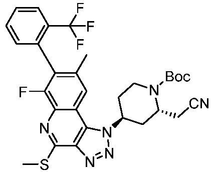

The mixture of tert-butyl (2S,4S)-4-(7-bromo-6-fluoro-8-methyl-4-(methylthio)-1H-[1,2,3]triazolo[4,5-c]quinolin-1-yl)-2-(cyanomethyl)piperidine-1-carboxylate (200 mg, 0.364 mmol, as prepared from **Example 98**) , (2-(trifluoromethyl)phenyl)boronic acid (138 mg, 0.728 mmol),dicyclohexyl(2',4',6'-triisopropylbiphenyl-2-yl)phosphine - (2'-aminobiphenyl-2-yl)(chloro)palladium (1:1) (28.6 mg, 0.036 mmol) (XPhos G2) and tripotassium phosphate (232 mg, 1.092 mmol) in Dioxane (10mL) and water (1mL) was degassed with N2 three times, then heated at 90 °C for 3 hours. After the reaction mixture was cooled to r.t, it was pured into water, extracted with AcOEt three times, the organic phase was washed with NaHCO3 aqueous solution, brine. The organic phase was dried over MgSO₄, filtered and concentrated. The residue was purified by column eluted with 0 to 10% AcOEt in DCM to afford the desired product. LCMS calculated for C₃₀H₃₁F₄N₆O₂S (M+H)⁺: m/z = 615.2; found: 615.2

### Step 2. 2-((2S,4S)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)-1-((E)-4-methoxybut-2-enoyl)piperidin-2-yl)acetonitrile

This compound was prepared according to the procedure described in **Example 155.**

**Example 157a.** Diastereomer 1. Peak 1. LCMS calculated for C₃₅H₃₈F₄N₇O₃ (M+H)⁺: m/z = 680.3; found: 680.2.

**Example 157b.** Diastereomer 2. Peak 2. LCMS calculated for C₃₅H₃₈F₄N₇O₃ (M+H)⁺: m/z = 680.3; found: 680.2.

### Example 159a and Example 159b. 2-((2S,4S)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)-1-((E)-4-fluorobut-2-enoyl)piperidin-2-yl)acetonitrile

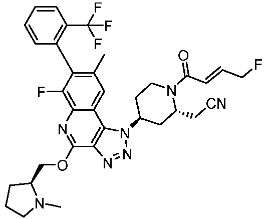

This compound was prepared according to the procedure described in **Example 157.**

**Example 159a.** Diastereomer 1. Peak 1. LCMS calculated for C₃₄H₃₅F₅N₇O₂ (M+H)⁺: m/z = 668.3; found: 668.2.

**Example 159b.** Diastereomer 2. Peak 2. LCMS calculated for C₃₄H₃₅F₅N₇O₂ (M+H)⁺: m/z = 668.3; found: 668.2.

### Example 160a and Example 160b. 2-((2S,4S)-1-acryloyl-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile

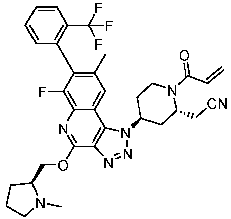

This compound was prepared according to the procedure described in **Example 157.**

**Example 160a.** Diastereomer 1. Peak 1. LCMS calculated for C₃₃H₃₄F₄N₇O₂ (M+H)⁺: m/z = 636.3; found: 636.2.

**Example 160b.** Diastereomer 2. Peak 2. LCMS calculated for C₃₃H₃₄F₄N₇O₂ (M+H)⁺: m/z = 636.3; found: 636.2.

### Example 163a and 163b. 2-((2S,4S)-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)-1-((E)-4-(dimethylamino)but-2-enoyl)piperidin-2-yl)acetonitrile

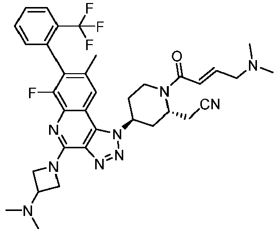

### Step 1. tert-butyl (2S,4S)-2-(cyanomethyl)-4-(6-fluoro-8-methyl-4-(methylthio)-7-(2-(trifluoromethyl) phenyl)-1H-[1, 2, 3]triazolo[4, 5-c]quinolin-1-yl)piperidine-1-carboxylate

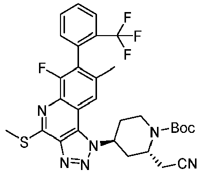

This compound was prepared according to the procedure described in **Example 157.** LCMS calculated for C₃₀H₃₁F₄N₆O₂S (M+H)⁺: m/z = 615.2; found: 615.2

### Step 2. 2-((2S,4S)-4-(4-(3-(dimethylamino)azetidin-1-yl)-6-fluoro-8-methyl-7-(2-(trifluoromethyl) phenyl)-1H-[1,2,3]triazolo[4,5-c]quinolin-1-yl)-1-((E)-4-(dimethylamino)but-2-enoyl)piperidin-2-yl)acetonitrile

This compound was prepared according to the procedure described in **Example 151 Step 2 to 3.**

**Example 163a.** Diastereomer 1. Peak 1. LCMS calculated for C₃₅H₄₀F₄N₉O (M+H)⁺: m/z = 678.3; found: 678.2

**Example 163b.** Diastereomer 2. Peak 2. LCMS calculated for C₃₅H₄₀F₄N₉O (M+H)⁺: m/z = 678.3; found: 678.2

### Example 165a and Example 165b. 2-((2S,4S)-1-acryloyl-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)piperidin-2-yl)acetonitrile

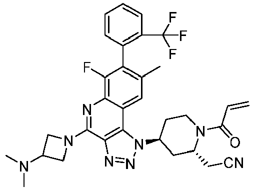

This compound was prepared according to the procedure described in **Example 163.**

**Example 165a.** Diastereomer 1. Peak 1. LCMS calculated for C₃₂H₃₃F₄N₈O (M+H)⁺: m/z = 621.3; found: 621.2. ¹H NMR (600 MHz, DMSO) δ 8.03 (s, 1H), 7.96 (d, J = 8.0 Hz, 1H), 7.85 (t, J = 7.6 Hz, 1H), 7.74 (t, J = 7.8 Hz, 1H), 7.45 (d, J = 7.6 Hz, 1H), 6.95 (s, 1H), 6.21 (dd, J = 16.7, 2.3 Hz, 1H), 5.79 (dd, J = 10.4, 2.3 Hz, 2H), 5.31 (s, 1H), 5.02 (s, 1H), 4.87 - 4.53 (m, 4H), 4.33 (s, 1H), 3.63 (d, J = 14.2 Hz, 1H), 3.38 (d, J = 8.7 Hz, 1H), 3.32 (s, 1H), 2.89 (s, 6H), 2.45 (s, 1H), 2.40 (d, J = 12.6 Hz, 2H), 2.24 (d, J = 12.7 Hz, 1H), 2.19 (s, 3H).

**Example 165b.** Diastereomer 2. Peak 2. LCMS calculated for C₃₂H₃₃F₄N₈O (M+H)⁺: m/z = 621.3; found: 621.2

### Example 167a and Example 167b. 2-((2S,4S)-1-((E)-4-(dimethylamino)but-2-enoyl)-4-(6-fluoro-8-methyl-4-(2-methyl-1H-imidazol-1-yl)-7-(2-(trifluoromethyl)phenyl)-1H-[1,2,3]triazolo[4,5-c]quinolin-1-yl)piperidin-2-yl)acetonitrile

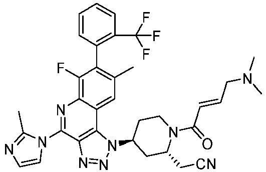

### Step 1. tert-butyl (2S,4S)-2-(cyanomethyl)-4-(6-fluoro-8-methyl-4-(methylthio)-7-(2-(trifluoromethyl) phenyl)-1H-[1, 2, 3]triazolo[4, 5-c]quinolin-1-yl)piperidine-1-carboxylate

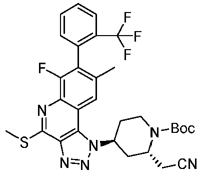

This compound was prepared according to the procedure described in **Example 157 Step1 to** 6. LCMS calculated for C₃₀H₃₁F₄N₆O₂S (M+H)⁺: m/z = 615.2; found: 615.2

### Step 2. 2-((2S,4S)-1-((E)-4-(dimethylamino)but-2-enoyl)-4-(6-fluoro-8-methyl-4-(2-methyl-1H-imidazol-1-yl)-7-(2-(trifluoromethyl)phenyl)-1H-[1,2,3]triazolo[4,5-c]quinolin-1-yl)piperidin-2-yl)acetonitrile

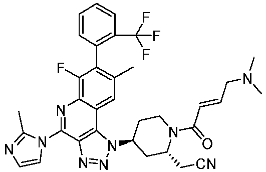

This compound was prepared according to the procedure described in **Example 151.**

**Example 167a.** Diastereomer 1. Peak 1. LCMS calculated for C₃₄H₃₄F₄N₉O (M+H)⁺: m/z = 660.3; found: 660.3

**Example 167b.** Diastereomer 2. Peak 2. LCMS calculated for C₃₄H₃₄F₄N₉O (M+H)⁺: m/z = 660.3; found: 660.3

### Example 168a and Example 168b. 2-((2S,4S)-1-((E)-4,4-difluorobut-2-enoyl)-4-(6-fluoro-8-methyl-4-(((S)-1-methylpyrrolidin-2-yl)methoxy)-7-(2-(trifluoromethyl)pyridin-3-yl)-1H-[1,2,3]triazolo[4,5-c]quinolin-1-yl)piperidin-2-yl)acetonitrile

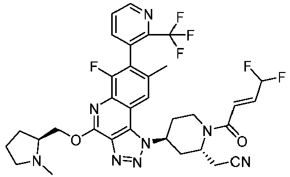

### Step 1. tert-butyl (2S,4S)-4-(7-bromo-6-fluoro-8-methyl-4-(methylthio)-1H-[1,2,3]triazolo[4,5-c]quinolin-1-yl)-2-(cyanomethyl)piperidine-1-carboxylate

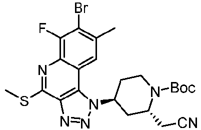

This compound was prepared according to the procedure described in **Example 98.** LCMS calculated for C₂₃H₂₇BrFN₃O₂S (M+H)⁺: m/z = 549.1; found: 549.1

### Step 2. tert-butyl (2S,4S)-4-(7-bromo-6-fluoro-8-methyl-4-(((S)-1-methylpyrrolidin-2-yl)methoxy)-1H-[1,2,3]triazolo[4,5-c]quinolin-1-yl)-2-(cyanomethyl)piperidine-1-carboxylate

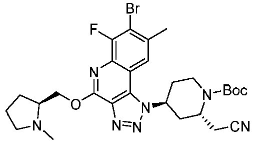

This compound was prepared according to the procedure described in **Example 155.** LCMS calculated for C₂₈H₃₆BrFN₇O₃ (M+H)⁺: m/z = 616.2; found: 616.2

### Step 3. 2-((2S,4S)-4-(6-fluoro-8-methyl-4-(((S)-1-methylpyrrolidin-2-yl)methoxy)-7-(2-(trifluoromethyl)pyridin-3-yl)-1H-[1,2,3]triazolo[4,5-c]quinolin-1-yl)piperidin-2-yl)acetonitrile

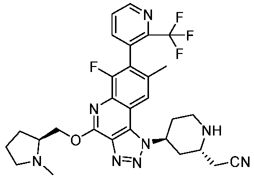

The mixture of tert-butyl (2S,4S)-4-(7-bromo-6-fluoro-8-methyl-4-(((S)-1-methylpyrrolidin-2-yl)methoxy)-1H-[1,2,3]triazolo[4,5-c]quinolin-1-yl)-2-(cyanomethyl)piperidine-1-carboxylate (100 mg, 0.162 mmol) , (2-(trifluoromethyl)pyridin-3-yl)boronic acid (77 mg, 0.405 mmol), XPhos Pd G4 (13.96 mg, 0.016 mmol) and tripotassium phosphate (103 mg, 0.487 mmol) ) in Dioxane (5mL) and water (1mL) was degassed with N2 three times, then heated at 90 °C for 3 hours. After the reaction mixture was cooled to r.t, it was pured into water, extracted with AcOEt three times, the organic phase was washed with NaHCO3 aqueous solution, brine. The organic phase was dried over MgSO₄, filtered and concentrated. The residue was purified by column eluted with 0 to 15% MeOH in DCM (0.1%NH3) to afford the desired product.

The purified compound was dissolved in DCM (2mL), then added TFA 2mL, the reaction mixture was stirred at r.t. 1 hour to remove Boc, the reaction solution was concentrated to dryness, go to next step directly. LCMS calculated for C₂₉H₃₁F₄N₈O (M+H)⁺: m/z = 583.3; found: 583.2.

### Step 4. 2-((2S,4S)-1-((E)-4,4-difluorobut-2-enoyl)-4-(6-fluoro-8-methyl-4-(((S)-1-methylpyrrolidin-2-yl)methoxy)-7-(2-(trifluoromethyl)pyridin-3-yl)-1H-[1,2,3]triazolo[4,5-c]quinolin-1-yl) piperidin-2-yl)acetonitrile

This compound was prepared according to the procedure described in **Example 151, step 3.**

**Example 168a.** Diastereomer 1. Peak 1. LCMS calculated for C₃₃H₃₃F₃N₃O₂ (M+H)⁺: m/z = 687.3; found: 687.2.

**Example 168b.** Diastereomer 2. Peak 2. LCMS calculated for C₃₃H₃₃F₃N₃O₂ (M+H)⁺: m/z = 687.3; found: 687.2.

### Example 205a and Example 205b: 2-((2S,4S)-1-(but-2-ynoyl)-4-(6-fluoro-8-methyl-4-(((S)-1-methylpyrrolidin-2-yl)methoxy)-7-(2-(trifluoromethyl)pyridin-3-yl)-1H-[1,2,3]triazolo[4,5-c]quinolin-1-yl)piperidin-2-yl)acetonitrile

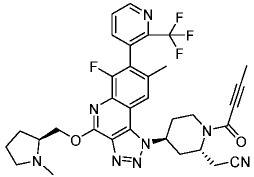

This compound was prepared according to the procedure described in **Example 168.**

**Example 205a.** Diastereomer 1. Peak 1. LCMS calculated for C₃₃H₃₃F₄N₃O₂ (M+H)⁺: m/z = 649.3; found: 649.2

**Example 205b.** Diastereomer 2. Peak 2. LCMS calculated for C₃₃H₃₃F₄N₃O₂ (M+H)⁺: m/z = 649.3; found: 649.2.

### Example 206. 2-((2S,4S)-4-(4-(3-(dimethylamino)azetidin-1-yl)-7-(2,3-dimethylphenyl)-6-fluoro-8-methyl-1H-[1,2,3]triazolo[4,5-c]quinolin-1-yl)-1-((E)-4-fluorobut-2-enoyl)piperidin-2-yl)acetonitrile

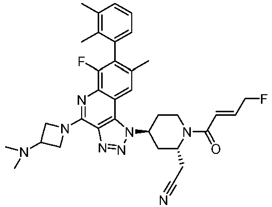

Triethylamine (10.59 µl, 0.076 mmol) was added to a solution of 2-((2*S*,4*S*)-4-(4-(3-(dimethylamino)azetidin-1-yl)-7-(2,3-dimethylphenyl)-6-fluoro-8-methyl-1*H*-[1,2,3]triazolo[4,5-c]quinolin-1-yl)piperidin-2-yl)acetonitrile (8.0 mg, 0.015 mmol), (*E*)-4-fluorobut-2-enoic acid (4.79 mg, 0.046 mmol) and 1-propanephosphonic acid cyclic anhydride (T₃P 50% in EtOAc)

(13.56 µl, 0.046 mmol) in ethyl acetate (0.8 ml) at 0 °C and then the reaction was stirred for 30 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₄H₃₉F₂N₈O (M+H)⁺ m/z = 613.3 ; found 613.4.

### Example 207. 2-((2S,4S)-1-(but-2-ynoyl)-4-(4-(3-(dimethylamino)azetidin-1-yl)-7-(2,3-dimethylphenyl)-6-fluoro-8-methyl-1H-[1,2,3]triazolo[4,5-c]quinolin-1-yl)piperidin-2-yl)acetonitrile

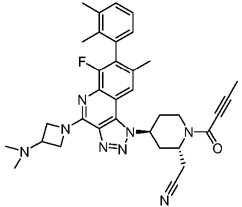

The compound was prepared according to the procedure described in **Example 206,** replacing (*E*)-4-fluorobut-2-enoic acid with but-2-ynoic acid. The product was isolated as a mixture of diastereomers. LCMS calculated for C₃₄H₃₃FN₃O (M+H)⁺ m/z = 593.3 ; found 593.4.

### Example 208. 2-((2S,4S)-1-acryloyl-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile

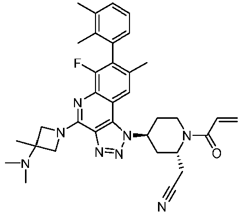

### Step 1. 2-((2S,4S)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile

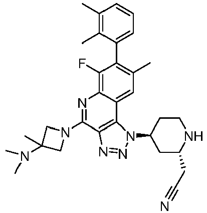

The compound was prepared according to the procedure described in **Example 147,** replacing N,N,-dimethylazetidin-3-amine with N,N,3-trimethylazetidin-3-amine in **Step 1.** LCMS calculated for C₃₁H₃₈FN₈ (M+H)⁺ m/z = 541.3 ; found 541.4.

### Step 2. 2-((2S,4S)-1-acryloyl-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile

Acryloyl chloride (5.02 mg, 0.055 mmol) was added to a solution of 2-((2S,4S)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile (10.0 mg, 0.018 mmol) and triethylamine (15.47 µl, 0.111 mmol) in CH₂Cl₂ (1.0 ml) at 0 °C and then stirred for 10 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₄H₄₀FN₈O (M+H)⁺ m/z = 595.3; found 595.5.

### Example 209. 2-((2S,4S)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)-1-((E)-4-fluorobut-2-enoyl)piperidin-2-yl)acetonitrile

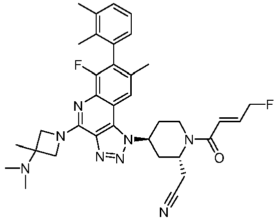

Triethylamine (10.31 µl, 0.074 mmol) was added to a solution of 2-((2S,4S)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile (8.0 mg, 0.015 mmol), (E)-4-fluorobut-2-enoic acid (4.62 mg, 0.044 mmol) and 1-propanephosphonic acid cyclic anhydride (T₃P 50% in EtOAc) (13.21 µl, 0.044 mmol) in EtOAc (0.8 ml) at 0 °C and then the reaction was stirred for 30 min. The solvent was dried and the residue was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. The product was isolated as a mixture of diastereomers. LCMS calculated for C₃₅H₄₁F₂N₈O (M+H)⁺ m/z = 627.3; found 627.5.

### Example 226. 2-((2S,4S)-1-(but-2-ynoyl)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile

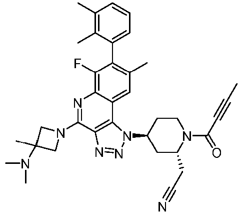

The compound was prepared according to the procedure described in **Example 209,** replacing (E)-4-fluorobut-2-enoic acid with but-2-ynoic acid. The product was isolated as a mixture of diastereomers. LCMS calculated for C₃₅H₄₀FN₈O (M+H)⁺ m/z = 607.3 ; found 607.4.

### Example 169. 1-((2S,4S)-1-acryloyl-2-(cyanomethyl)piperidin-4-yl)-7-(6-chloro-5-methyl-1H-indazol-4-yl)-4-(3-(dimethylamino)azetidin-1-yl)-6-fluoro-1H-[1,2,3]triazolo[4,5-c]quinoline-8-carbonitrile

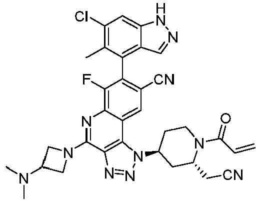

### Step 1. tert-butyl (2S,4S)-4-((7-bromo-2-chloro-8-fluoro-6-iodo-3-nitroquinolin-4-yl)amino)-2-(2-(tert-butoxy)-2-oxoethyl)piperidine-1-carboxylate

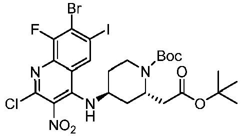

This compound was prepared according to the procedure described in **Example 80a and Example 80b, step 1,** replacing 7-bromo-2,4,6-trichloro-8-fluoro-3-nitroquinoline with 7-bromo-2,4-dichloro-8-fluoro-6-iodo-3-nitroquinoline. LCMS calculated for C₂₅H₃₁BrClFIN₄O₆(M+H)⁺: m/z = 743.0; found: 743.2.

### Step 2. tert-butyl (2S,4S)-4-((7-bromo-2-(3-(dimethylamino)azetidin-1-yl)-8-fluoro-6-iodo-3-nitroquinolin-4-yl)amino)-2-(2-(tert-butoxy)-2-oxoethyl)piperidine-1-carboxylate

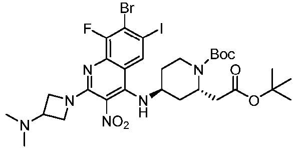

This compound was prepared according to the procedure described in **Example 9, step** 1, replacing tert-butyl 4-((7-bromo-2,6-dichloro-8-fluoro-3-nitroquinolin-4-yl)amino)-piperidine-1-carboxylate with *tert*-butyl (2S,4S)-4-((7-bromo-2-chloro-8-fluoro-6-iodo-3-nitroquinolin-4-yl)amino)-2-(2-(*tert*-butoxy)-2-oxoethyl)piperidine-1-carboxylate. LCMS calculated for C₃₀H₄₂BrFIN₆O₆ (M+H)⁺: m/z = 807.1; found: 807.2.

### Step 3. tert-butyl (2S,4S)-4-((3-amino-7-bromo-2-(3-(dimethylamino)azetidin-1-yl)-8-fluoro-6-iodoquinolin-4-yl)amino)-2-(2-(tert-butoxy)-2-oxoethyl)piperidine-1-carboxylate

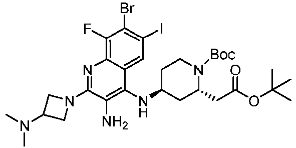

This compound was prepared according to the procedure described in **Example 15, step 2,** replacing *tert*-butyl (*endo*)-5-((7-bromo-6-chloro-2-(3-(dimethylamino)azetidin-1-yl)-8-fluoro-3-nitroquinolin-4-yl)amino)-2-azabicyclo[2.1.1]hexane-2-carboxylate with *tert*-butyl (2S,4S)-4-((7-bromo-2-(3-(dimethylamino)azetidin-1-yl)-8-fluoro-6-iodo-3-nitroquinolin-4-yl)amino)-2-(2-(*tert*-butoxy)-2-oxoethyl)piperidine-1-carboxylate. LCMS calculated for C₃₀H₄₄BrFIN₆O₄ (M+H)⁺: m/z = 777.2; found: 777.2.

### Step 4. tert-butyl (2S,4S)-4-(7-bromo-4-(3-(dimethylamino)azetidin-1-yl)-6-fluoro-8-iodo-1H-[1,2,3]triazolo[4,5-c]quinolin-1-yl)-2-(2-(tert-butoxy)-2-oxoethyl)piperidine-1-carboxylate

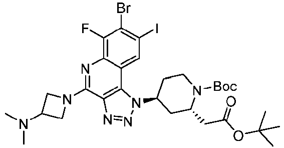

This compound was prepared according to the procedure described in **Example 14a and Example 14b, step 1,** replacing *tert*-butyl (*S*)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2*S*,4*S*)-4-((3-amino-7-bromo-2-(3-(dimethylamino)azetidin-1-yl)-8-fluoro-6-iodoquinolin-4-yl)amino)-2-(2-(*tert*-butoxy)-2-oxoethyl)piperidine-1-carboxylate.The crude material was purified by column chromatography (0-20% MeOH:DCM) to afford the desired product as a solid. LCMS calculated for C₃₀H₄₁BrFIN₇O₄ (M+H)⁺: m/z = 788.1; found: 788.2.

### Step 5. tert-butyl (2S,4S)-4-(7-bromo-8-cyano-4-(3-(dimethylamino)azetidin-1-yl)-6-fluoro-1H-[1,2,3]triazolo[4,5-c]quinolin-1-yl)-2-(2-(tert-butoxy)-2-oxoethyl) piperidine-1-carboxylate

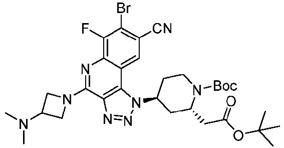

A solution of tert-butyl (2*S*,4*S*)-4-(7-bromo-4-(3-(dimethylamino)azetidin-1-yl)-6-fluoro-8-iodo-1*H*-[1,2,3]triazolo[4,5-*c*]quinolin-1-yl)-2-(2-(*tert*-butoxy)-2-oxoethyl)piperidine-1-carboxylate (580 mg, 0.736 mmol), Zn(CN)₂ (432 mg, 3.68 mmol), Pd(dppf)₂Cl₂.DCM (120 mg, 0.147 mmol), KOAc (144 mg, 1.47 mmol) in DMA (3.7 mL) was flushed with nitrogen for ca. 2 min. The resultant mixture was heated at 150 °C for overnight then quenched by water at room temp. The mixture was extracted with DCM (x3). The combined organic extracts were dried, concentrated under reduced pressure and purified by column chromatography (0-20% MeOH:DCM) to afford the product as an oil. LC-MS calculated for C₃₁H₄₁BrFN₈O₄ (M+H)⁺: m/z = 687.2; found 687.3.

### Step 6. 2-((2S,4S)-4-(7-bromo-8-cyano-4-(3-(dimethylamino)azetidin-1-yl)-6-fluoro-1H-[1,2,3]triazolo[4,5-c]quinolin-1-yl)piperidin-2-yl)acetic acid

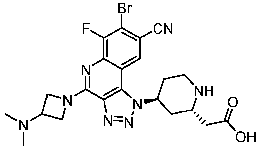

This compound was prepared according to the procedure described in **Example 80a and Example 80b, step 5,** replacing *tert*-butyl (2*S*,4*S*)-4-(7-bromo-8-chloro-6-fluoro-4-(methylthio)-1*H*-imidazo[4,5-*c*]quinolin-1-yl)-2-(2-(*tert*-butoxy)-2-oxoethyl)piperidine-1-carboxylate with *tert*-butyl (2*S*,4*S*)-4-(7-bromo-8-cyano-4-(3-(dimethylamino)azetidin-1-yl)-6-fluoro-1*H*-[1,2,3]triazolo[4,5-*c*]quinolin-1-yl)-2-(2-(*tert*-butoxy)-2-oxoethyl)piperidine-1-carboxylate. LCMS calculated for C₂₂H₂₅BrFN₈O₂ (M+H)⁺: m/z = 531.1; found: 531.2.

### Step 7. 2-((2S,4S)-4-(7-bromo-8-cyano-4-(3-(dimethylamino)azetidin-1-yl)-6-fluoro-1H-[1,2,3]triazolo[4,5-c]quinolin-1-yl)-1-(tert-butoxycarbonyl) piperidin-2-yl) acetic acid

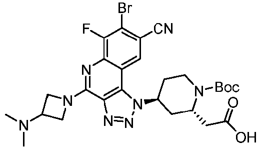

This compound was prepared according to the procedure described in **Example 80a and Example 80b, step 6,** replacing 2-((2*S*,4*S*)-4-(7-bromo-8-chloro-6-fluoro-4-(methylthio)-1H-imidazo[4,5-c]quinolin-1-yl)piperidin-2-yl)acetic acid with 2-((2*S*,4*S*)-4-(7-bromo-8-cyano-4-(3-(dimethylamino)azetidin-1-yl)-6-fluoro-1*H*-[1,2,3]triazolo[4,5-*c*]quinolin-1-yl)piperidin-2-yl)acetic acid. LCMS calculated for C₂₇H₃₃BrFN₈O₄ (M+H)⁺: m/z = 631.2; found: 631.3.

### Step 8. tert-butyl (2S,4S)-2-(2-amino-2-oxoethyl)-4-(7-bromo-8-cyano-4-(3-(dimethylamino)azetidin-1-yl)-6-fluoro-1H-[1,2,3]triazolo[4,5-c]quinolin-1-yl)piperidine-1-carboxylate

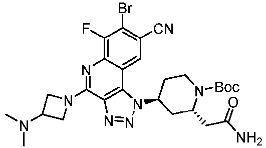

This compound was prepared according to the procedure described in **Example 80a and Example 80b, step 7,** replacing 2-((2*S*,4*S*)-4-(7-bromo-8-chloro-6-fluoro-4-(methylthio)-1*H*-imidazo[4,5-c]quinolin-1-yl)-1-(*tert*-butoxycarbonyl)piperidin-2-yl)acetic acid with 2-((2*S*,4*S*)-4-(7-bromo-8-cyano-4-(3-(dimethylamino)azetidin-1-yl)-6-fluoro-1*H-*[1,2,3]triazolo[4,5-c]quinolin-1-yl)-1-(*tert*-butoxycarbonyl)piperidin-2-yl)acetic acid. LCMS calculated for C₂₇H₃₄BrFN₉O₃ (M+H)⁺: m/z = 630.2; found: 630.3.

### Step 9. tert-butyl (2S,4S)-4-(7-bromo-8-cyano-4-(3-(dimethylamino)azetidin-1-yl)-6-fluoro-1H-[1,2,3]triazolo[4,5-c]quinolin-1-yl)-2-(cyanomethyl)piperidine-1-carboxylate

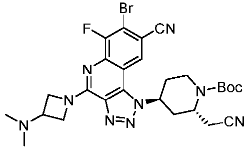

This compound was prepared according to the procedure described in **Example 80a and Example 80b, step 8,** replacing *tert*-butyl (2*S*,4*S*)-2-(2-amino-2-oxoethyl)-4-(7-bromo-8-chloro-6-fluoro-4-(methylthio)-1*H*-imidazo[4,5-*c*]quinolin-1-yl)piperidine-1-carboxylate with *tert*-butyl (2*S*,4*S*)-2-(2-amino-2-oxoethyl)-4-(7-bromo-8-cyano-4-(3-(dimethylamino)azetidin-1-yl)-6-fluoro-1*H*-[1,2,3]triazolo[4,5-*c*]quinolin-1-yl)piperidine-1-carboxylate. LCMS calculated for C₂₇H₃₂BrFN₉O₂ (M+H)⁺: m/z = 612.2; found: 612.3.

### Step 10. tert-butyl (2S,4S)-4-(7-(6-chloro-5-methyl-1-(tetrahydro-2H-pyran-2-yl)-1H-indazol-4-yl)-8-cyano-4-(3-(dimethylamino)azetidin-1-yl)-6-fluoro-1H-[1,2,3]triazolo[4,5-c]quinolin-1-yl)-2-(cyanomethyl)piperidine-1-carboxylate

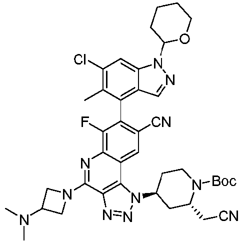

This compound was prepared according to the procedure described in **Example 80a and Example 80b, step 9,** replacing *tert*-butyl (2*S*,4*S*)-4-(7-bromo-8-chloro-6-fluoro-4-(methylthio)-1*H*-imidazo[4,5-*c*]quinolin-1-yl)-2-(cyanomethyl)piperidine-1-carboxylate with *tert*-butyl (2*S*,4*S*)-4-(7-bromo-8-cyano-4-(3-(dimethylamino)azetidin-1-yl)-6-fluoro-1*H-*[1,2,3]triazolo[4,5-*c*]quinolin-1-yl)-2-(cyanomethyl)piperidine-1-carboxylate. The crude material was not isolated and used in the next step without further purification. LCMS calculated for C₄₀H₄₆ClFN₁₁O₃ (M+H)⁺: m/z = 782.4; found: 782.4..

### Step 11. 7-(6-chloro-5-methyl-1H-indazol-4-yl)-1-((2S,4S)-2-(cyanomethyl)piperidin-4-yl)-4-(3-(dimethylamino)azetidin-1-yl)-6-fluoro-1H-[1,2,3]triazolo[4,5-c]quinoline-8-carbonitrile hydrochloride

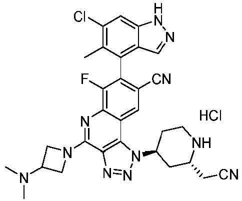

The crude material from **step 10** (100 mg, 0.127 mmol) was dissolved in dioxane (1 mL) and then HCl (1 mL, 4M in dioxane) was added. The resultant mixture was stirred at room temp for 30 min and concentrated to afford the crude product as a HCl salt which was used in the next step without further purification. LCMS calculated for C₃₀H₃₀ClFN₁₁ (M+H)⁺: m/z = 598.2; found: 598.3.

### Step 12. 1-((2S,4S)-1-acryloyl-2-(cyanomethyl)piperidin-4-yl)-7-(6-chloro-5-methyl-1H-indazol-4-yl)-4-(3-(dimethylamino)azetidin-1-yl)-6-fluoro-1H-[1,2,3]triazolo[4,5-c]quinoline-8-carbonitrile

To a solution of crude 7-(6-chloro-5-methyl-1*H*-indazol-4-yl)-1-((2*S*,4*S*)-2-(cyanomethyl)piperidin-4-yl)-4-(3-(dimethylamino)azetidin-1-yl)-6-fluoro-1*H*-[1,2,3]triazolo[4,5-c]quinoline-8-carbonitrile hydrochloride (29 mg, 0.046 mmol) in DCM (1 mL) was added DIEA (159 µl, 0.914 mmol) dropwise at room temp. The resultant mixture was cooled to -78 °C and acryloyl chloride (3.7 µL, 0.046 mmol) in DCM (1 mL) was added dropwise. The resulting mixture was stirred at -78 °C for 10 min. Upon completion, the reaction was diluted with methanol and purified using prep-LCMS (XBridge C18 column, eluting with a gradient of acetonitrile/water containing 0.1% TFA, at flow rate of 60 mL/min) to afford the desired product as a TFA salt. The product was isolated as a mixture of diastereomers. LC-MS calculated for C₃₃H₃₂ClFN₁₁O (M+H)⁺: m/z = 652.3; found 652.3.

### Example 170. 1-((2S,4S)-1-(but-2-ynoyl)-2-(cyanomethyl)piperidin-4-yl)-7-(6-chloro-5-methyl-1H-indazol-4-yl)-4-(3-(dimethylamino)azetidin-1-yl)-6-fluoro-1H-[1,2,3]triazolo[4,5-c]quinoline-8-carbonitrile

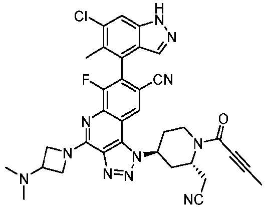

To a solution of crude 7-(6-chloro-5-methyl-1*H*-indazol-4-yl)-1-((2*S*,4*S*)-2-(cyanomethyl)piperidin-4-yl)-4-(3-(dimethylamino)azetidin-1-yl)-6-fluoro-1*H*-[1,2,3]triazolo[4,5-c]quinoline-8-carbonitrile hydrochloride (**Example 169, step 11,** 30 mg, 0.047 mmol) and but-2-ynoic acid (19.9 mg, 0.236 mmol) in DCM (1 mL) was added DIEA (165 µL, 0.946 mmol) then propane phosphonic acid anhydride (30.1 mg, 0.0950 mmol) (in 0.5 mL DCM) dropwise at room temp. The resulting mixture was stirred at room temp for 1 h. Upon completion, the reaction was diluted with methanol and purified using prep-LCMS (XBridge C18 column, eluting with a gradient of acetonitrile/water containing 0.1% TFA, at flow rate of 60 mL/min) to afford the desired product as a TFA salt. The product was isolated as a mixture of diastereomers. LC-MS calculated for C₃₄H₃₂ClFN₁₁O (M+H)⁺: m/z = 664.3; found 664.4.

### Example 171. 2-((2S,4S)-1-acryloyl-4-(8-chloro-7-(3-chloro-2-methylphenyl)-4-(3-(dimethylamino)azetidin-1-yl)-1H-imidazo[4,5-c]quinolin-1-yl)piperidin-2-yl)acetonitrile

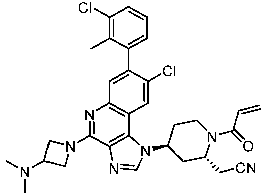

### Step 1. 7-bromo-6-chloro-2H-benzo[d][1,3]oxazine-2,4(1H)-dione

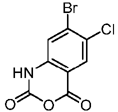

This compound was prepared according to the procedure described in **Example 1a and Example 1b, Step 2,** replacing 2-amino-4-bromo-5-chloro-3-fluorobenzoic acid with 2-amino-4-bromo-5-chlorobenzoic acid. LC-MS calculated for C₃H₄BrClNO₃ (M+H)⁺: m/z = 275.9; found 275.9.

### Step 2. 7-bromo-6-chloro-3-nitroquinoline-2,4-diol

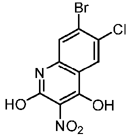

This compound was prepared according to the procedure described in **Example 1a and Example 1b, Step 3,** replacing 7-bromo-6-chloro-8-fluoro-2H-benzo[d][1,3]oxazine-2,4(1*H*)-dione with *7-bromo-6-chloro-2H-benzo[d][1,3]oxazine-2,4(1H)-dione.* LC-MS calculated for C₉H₅BrClN₂O₄ (M+H)⁺: m/z = 318.9; found 318.9.

### Step 3. 7-bromo-2,4,6-trichloro-3-nitroquinoline

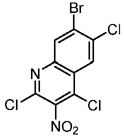

This compound was prepared according to the procedure described in **Example 1a and Example 1b, Step 4,** replacing 7-bromo-6-chloro-8-fluoro-3-nitroquinoline-2,4-diol with 7-bromo-6-chloro-3-nitroquinoline-2,4-diol. LC-MS calculated for C₉H₃BrCl₃N₂O₂ (M+H)⁺: m/z = 354.8; found 354.8.

### Step 4. tert-butyl (2S,4S)-4-((7-bromo-2,6-dichloro-3-nitroquinolin-4-yl)amino)-2-(2-(tert-butoxy)-2-oxoethyl)piperidine-1-carboxylate

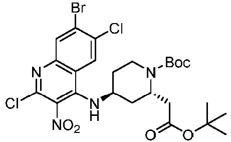

This compound was prepared according to the procedure described in **Example 1a and Example 1b, step 5,** replacing 7-bromo-2,4,6-trichloro-8-fluoro-3-nitroquinoline and *tert-*butyl 4-aminopiperidine-1-carboxylate with 7-bromo-2,4,6-trichloro-3-nitroquinoline and *tert-*butyl (2S,4S)-4-amino-2-(2-(tert-butoxy)-2-oxoethyl)piperidine-1-carboxylate respectively. LCMS calculated for C₂₅H₃₂BrCl₂N₄O₆ (M+H)⁺: m/z = 633.1; found: 633.1.

### Step 5. tert-butyl (2S,4S)-4-((3-amino-7-bromo-2,6-dichloroquinolin-4-yl)amino)-2-(2-(tert-butoxy)-2-oxoethyl)piperidine-1-carboxylate

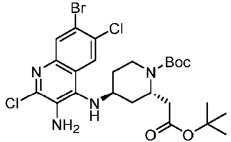

This compound was prepared according to the procedure described in **Example 15, step 2,** replacing tert-butyl (endo)-5-((7-bromo-6-chloro-2-(3-(dimethylamino)azetidin-1-yl)-8-fluoro-3-nitroquinolin-4-yl)amino)-2-azabicyclo[2.1.1]hexane-2-carboxylate with tert-butyl (2S,4S)-4-((7-bromo-2,6-dichloro-3-nitroquinolin-4-yl)amino)-2-(2-(tert-butoxy)-2-oxoethyl)piperidine-1-carboxylate. LCMS calculated for C₂₅H₃₄BrCl₂N₄O₄ (M+H)⁺: m/z = 603.1; found: 603.1.

### Step 6. tert-butyl (2S,4S)-4-(7-bromo-4,8-dichloro-1H-imidazo[4,5-c]quinolin-1-yl)-2-(2-(tert-butoxy)-2-oxoethyl)piperidine-1-carboxylate

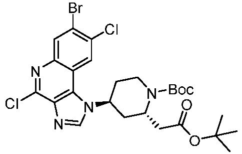

This compound was prepared according to the procedure described in **Example 23, step 1,** replacing *tert*-butyl (*endo*)-5-((3-amino-7-bromo-6-chloro-2-(3-(dimethylamino)azetidin-1-yl)-8-fluoroquinolin-4-yl)amino)-2-azabicyclo[2.1.1]hexane-2-carboxylate with *tert*-butyl (2S,4S)-4-((3-amino-7-bromo-2,6-dichloroquinolin-4-yl)amino)-2-(2-(*tert*-butoxy)-2-oxoethyl)piperidine-1-carboxylate. LCMS calculated for C₂₆H₃₂BrCl₂N₄O₄ (M+H)⁺: m/z = 613.1; found: 613.1.

### Step 7. tert-butyl (2S,4S)-4-(7-bromo-8-chloro-4-(3-(dimethylamino)azetidin-1-yl)-1H-imidazo[4,5-c]quinolin-1-yl)-2-(2-(tert-butoxy)-2-oxoethyl)piperidine-1-carboxylate

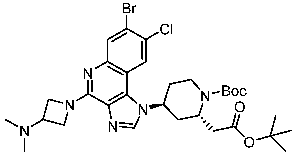

This compound was prepared according to the procedure described in **Example 89, Step 1,** replacing *tert*-butyl (2*S*,4*S*)-4-(7-bromo-4,8-dichloro-6-fluoro-1*H*-imidazo[4,5-c]quinolin-1-yl)-2-(2-(*tert*-butoxy)-2-oxoethyl)piperidine-1-carboxylate with *tert*-butyl (2S,4S)-4-(7-bromo-4,8-dichloro-1H-imidazo[4,5-c]quinolin-1-yl)-2-(2-(*tert*-butoxy)-2-oxoethyl)piperidine-1-carboxylate. LCMS calculated for C₃₁H₄₃BrClN₆O₄ (M+H)⁺: m/z = 677.2; found: 677.3.

### Step 8. tert-butyl (2S,4S)-4-(7-bromo-8-chloro-4-(3-(dimethylamino)azetidin-1-yl)-1H-imidazo[4,5-c]quinolin-1-yl)-2-(cyanomethyl)piperidine-1-carboxylate

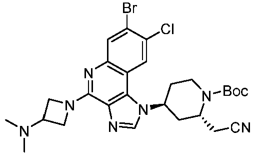

This compound was prepared according to the procedure described in **Example 80a and Example 80b, Steps 5-8,** replacing *tert*-butyl (2*S*,4*S*)-4-(7-bromo-8-chloro-6-fluoro-4-(methylthio)-1*H*-imidazo[4,5-*c*]quinolin-1-yl)-2-(2-(*tert*-butoxy)-2-oxoethyl)piperidine-1-carboxylate with *tert*-butyl (2S,4S)-4-(7-bromo-8-chloro-4-(3-(dimethylamino)azetidin-1-yl)-1H-imidazo[4,5-c]quinolin-1-yl)-2-(2-(*tert*-butoxy)-2-oxoethyl)piperidine-1-carboxylate. LCMS calculated for C₂₇H₃₄BrClN₇O₂ (M+H)⁺ m/z = 602.2; found 602.2.

### Step 9. 2-((2S,4S)-1-acryloyl-4-(7-bromo-8-chloro-4-(3-(dimethylamino)azetidin-1-yl)-1H-imidazo[4,5-c]quinolin-1-yl)piperidin-2-yl)acetonitrile

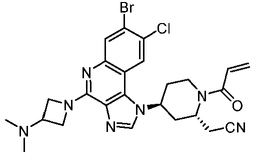

This compound was prepared according to the procedure described in **Example 85, Step 12,** replacing *tert*-butyl (2*S*,4*S*)-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-c]quinolin-1-yl)-2-(cyanomethyl)piperidine-1-carboxylate with *tert*-butyl (2S,4S)-4-(7-bromo-8-chloro-4-(3-(dimethylamino)azetidin-1-yl)-1H-imidazo[4,5-c]quinolin-1-yl)-2-(cyanomethyl)piperidine-1-carboxylate. LCMS calculated for C₂₅H₂₈ClN₇O (M+H)⁺ m/z = 556.2; found 556.1.

### Step 10. 2-((2S,4S)-1-acryloyl-4-(8-chloro-7-(3-chloro-2-methylphenyl)-4-(3-(dimethylamino)azetidin-1-yl)-1H-imidazo[4,5-c]quinolin-1-yl)piperidin-2-yl)acetonitrile

This compound was prepared according to the procedure described in **Example 85, Step 11,** replacing (3-chloro-4-fluorophenyl)boronic acid with (3-chloro-2-methylphenyl)boronic acid and tert-butyl (2*S*,4*S*)-4-(7-bromo-8-chloro-6-fluoro-4-(((*S*)-1-methylpyrrolidin-2-yl)methoxy)-1*H*-[1,2,3]triazolo[4,5-*c*]quinolin-1-yl)-2-(cyanomethyl) piperidine-1-carboxylate with 2-((2S,4S)-1-acryloyl-4-(7-bromo-8-chloro-4-(3-(dimethylamino)azetidin-1-yl)-1H-imidazo[4,5-c]quinolin-1-yl)piperidin-2-yl)acetonitrile. LCMS calculated for C₃₂H₃₄Cl₂N₇O (M+H)⁺ m/z = 602.2; found 602.2.

### Example 172. 2-((2S,4S)-1-acryloyl-4-(7-(3-chloro-2-methylphenyl)-4-(3-(dimethylamino)azetidin-1-yl)-8-methyl-1H-[1,2,3]triazolo[4,5-c]quinolin-1-yl)piperidin-2-yl)acetonitrile

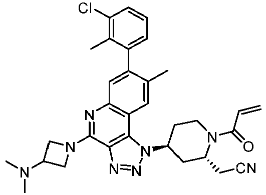

### Step 1. tert-butyl (2S,4S)-4-((3-amino-7-bromo-2-chloro-6-methylquinolin-4-yl)amino)-2-(2-(tert-butoxy)-2-oxoethyl)piperidine-1-carboxylate

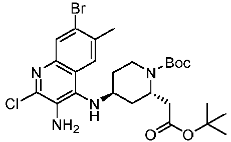

This compound was prepared according to the procedure described in **Example 171, step 1-5,** replacing 2-amino-4-bromo-5-chlorobenzoic acid with 2-amino-4-bromo-5-methylbenzoic acid. LCMS calculated for C₂₆H₃₇BrClN₄O₄ (M+H)⁺: m/z = 583.2; found: 583.2.

### Step 2. tert-butyl (2S,4S)-4-(7-bromo-4-chloro-8-methyl-1H-[1,2,3]triazolo[4,5-c]quinolin-1-yl)-2-(2-(tert-butoxy)-2-oxoethyl) piperidine-1-carboxylate

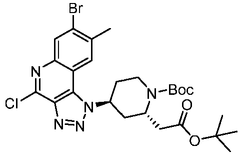

This compound was prepared according to the procedure described in **Example 14, Step 1**, replacing *tert*-butyl (*S*)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2S,4S)-4-((3-amino-7-bromo-2-chloro-6-methylquinolin-4-yl)amino)-2-(2-(*tert*-butoxy)-2-oxoethyl)-piperidine-1-carboxylate. LCMS calculated for C₂₆H₃₄BrClN₅O₄ (M+H)⁺: m/z = 594.1; found: 594.1

### Step 3. tert-butyl (2S,4S)-4-(7-bromo-4-(3-(dimethylamino)azetidin-1-yl)-8-methyl-1H-[1,2,3]triazolo[4,5-c]quinolin-1-yl)-2-(cyanomethyl)piperidine-1-carboxylate

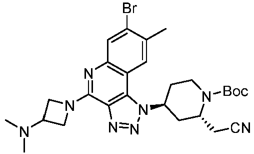

This compound was prepared according to the procedure described in **Example 80a and Example 80b, Steps 5-8,** replacing *tert*-butyl (2*S*,4*S*)-4-(7-bromo-8-chloro-6-fluoro-4-(methylthio)-1*H*-imidazo[4,5-*c*]quinolin-1-yl)-2-(2-(*tert*-butoxy)-2-oxoethyl)piperidine-1-carboxylate with *tert*-butyl (2S,4S)-4-(7-bromo-4-chloro-8-methyl-1H-[1,2,3]triazolo[4,5-c]quinolin-1-yl)-2-(2-(*tert*-butoxy)-2-oxoethyl)piperidine-1-carboxylate. LCMS calculated for C₂₇H₃₆BrN₈O₂ (M+H)⁺ m/z = 583.2; found 583.2.

### Step 4. tert-butyl (2S,4S)-4-(7-(3-chloro-2-methylphenyl)-4-(3-(dimethylamino)azetidin-1-yl)-8-methyl-1H-[1,2,3]triazolo[4,5-c]quinolin-1-yl)-2-(cyanomethyl)piperidine-1-carboxylate

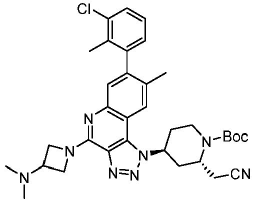

This compound was prepared according to the procedure described in **Example 85, Step 11,** replacing (3-chloro-4-fluorophenyl)boronic acid with (3-chloro-2-methylphenyl)-boronic acid and tert-butyl (2*S*,4*S*)-4-(7-bromo-8-chloro-6-fluoro-4-(((*S*)-1-methylpyrrolidin-2-yl)methoxy)-1*H-*[1,2,3]triazolo[4,5-*c*]quinolin-1-yl)-2-(cyanomethyl)piperidine-1-carboxylate with tert-butyl (2S,4S)-4-(7-bromo-4-(3-(dimethylamino)azetidin-1-yl)-8-methyl-1H-[1,2,3]triazolo[4,5-c]quinolin-1-yl)-2-(cyanomethyl)piperidine-1-carboxylate. LCMS calculated for C₃₄H₄₂ClN₃O₂ (M+H)⁺ m/z = 629.3; found 629.3

### Step 5. 2-((2S, 4S)-1-acryloyl-4-(7-(3-chloro-2-methylphenyl)-4-(3-(dimethylamino)azetidin-1-yl)-8-methyl-1H-[1,2,3]triazolo[4,5-c]quinolin-1-yl)piperidin-2-yl)acetonitrile

This compound was prepared according to the procedure described in **Example 85, Step 12,** replacing *tert*-butyl (2*S*,4*S*)-4-(8-chloro-7-(3-chloro-4-fluorophenyl)-6-fluoro-4-(((*S*)-1-methylpyrrolidin-2-yl)methoxy)-1*H-*[1,2,3]triazolo[4,5-*c*]quinolin-1-yl)-2-(cyanomethyl)-piperidine-1-carboxylate with tert-butyl (2S,4S)-4-(7-(3-chloro-2-methylphenyl)-4-(3-(dimethylamino)azetidin-1-yl)-8-methyl-1H-[1,2,3]triazolo[4,5-c]quinolin-1-yl)-2-(cyanomethyl)piperidine-1-carboxylate. LCMS calculated for C₃₂H₃₆ClN₈O (M+H)⁺ m/z = 583.3; found 583.3.

### Example 173. 2-((2S,4S)-1-acryloyl-4-(8-chloro-7-(3-chloro-2-methylphenyl)-6-fluoro-4-(2-methylpyridin-3-yl)-1H-imidazo[4,5-c]quinolin-1-yl)piperidin-2-yl)acetonitrile

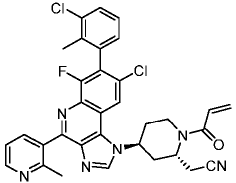

### Step 1. tert-butyl (2S,4S)-4-(7-bromo-4,8-dichloro-6-fluoro-1H-imidazo[4,5-c]quinolin-1-yl)-2-(cyanomethyl)piperidine-1-carboxylate

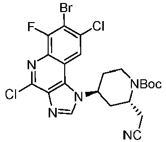

*tert*-butyl (2*S*,4*S*)-4-(7-bromo-4,8-dichloro-6-fluoro-1*H*-imidazo[4,5-c]quinolin-1-yl)-2-(2-(*tert*-butoxy)-2-oxoethyl)piperidine-1-carboxylate (prepared in **Example 80a, step 3**) was subjected to the procedure described in **Example 80a and Example 80b, Steps 5-8,** replacing *tert*-butyl (2*S*,4*S*)-4-(7-bromo-8-chloro-6-fluoro-4-(methylthio)-1*H*-imidazo[4,5-c]quinolin-1-yl)-2-(2-(*tert*-butoxy)-2-oxoethyl)piperidine-1-carboxylate with *tert*-butyl (2S,4S)-4-(7-bromo-4,8-dichloro-6-fluoro-1H-imidazo[4,5-c]quinolin-1-yl)-2-(2-(*tert*-butoxy)-2-oxoethyl)piperidine-1-carboxylate. LCMS calculated for C₂₂H₂₂BrCl₂FN₅O₂ (M+H)⁺: m/z = 556.0; found: 556.0.

### Step 2. tert-butyl (2S,4S)-4-(7-bromo-8-chloro-6-fluoro-4-(2-methylpyridin-3-yl)-1H-imidazo[4,5-c]quinolin-1-yl)-2-(cyanomethyl)piperidine-1-carboxylate

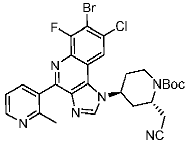

A screw-cap vial equipped with a magnetic stir bar was charged tert-butyl (2S,4S)-4-(7-bromo-4,8-dichloro-6-fluoro-1H-imidazo[4,5-c]quinolin-1-yl)-2-(cyanomethyl)piperidine-1-carboxylate (100 mg, 0.179 mmol), (2-methylpyridin-3-yl)boronic acid (37.0 mg, 0.269 mmol), sodium carbonate (38.0 mg, 0.359 mmol), and Pd(Ph₃P)₄ (21 mg, 18 µmol) followed by dioxane (2.0 mL) and water (0.2 mL). The vial was sealed with a Teflon-lined septum, evacuated and backfilled with nitrogen (this process was repeated a total of three times). Then the reaction was concentrated in vacuo and subjected to the next reaction without further purification. LCMS calculated for C₂₈H₂₈BrClFN₆O₂ (M+H)⁺: m/z = 613.1; found: 613.1.

### Step 3. 2-((2S,4S)-1-acryloyl-4-(7-bromo-8-chloro-6-fluoro-4-(2-methylpyridin-3-yl)-1H-imidazo[4,5-c]quinolin-1-yl)piperidin-2-yl)acetonitrile

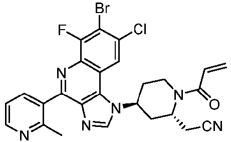

This compound was prepared according to the procedure described in **Example 85, Step 12,** replacing *tert*-butyl (2*S*,4*S*)-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with *tert-butyl (2S,4S)-4-(7-bromo-8-chloro-6-fluoro-4-(2-methylpyridin-3-yl)-1H-imidazo[4, 5-c]quinolin-1-yl)-2-(cyanomethyl) piperidine-1-carboxylate.* The product was purified using prep-LCMS (XBridge C18 column, eluting with a gradient of acetonitrile/water containing 0.1% TFA, at flow rate of 60 mL/min) without the second purification. LCMS calculated for C₂₆H₂₂BrClFN₆O (M+H)⁺ m/z = 567.1; found 567.1.

### Step 4. 2-((2S,4S)-1-acryloyl-4-(8-chloro-7-(3-chloro-2-methylphenyl)-6-fluoro-4-(2-methylpyridin-3-yl)-1H-imidazo[4,5-c]quinolin-1-yl)piperidin-2-yl)acetonitrile

This compound was prepared according to the procedure described in **Example 85, Step 11,** replacing (3-chloro-4-fluorophenyl)boronic acid with (3-chloro-2-methylphenyl)-boronic acid and *tert*-butyl (2*S*,4*S*)-4-(7-bromo-8-chloro-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with 2-((2S,4S)-1-acryloyl-4-(7-bromo-8-chloro-6-fluoro-4-(2-methylpyridin-3-yl)-1H-imidazo[4,5-c]quinolin-1-yl)piperidin-2-yl)acetonitrile. The product was purified and was isolated as a mixture of diastereomers using prep-LCMS (XBridge C18 column, eluting with a gradient of acetonitrile/water containing 0.1% TFA, at flow rate of 60 mL/min). LCMS calculated for C₃₃H₂₈Cl₂FN₆O (M+H)⁺ m/z = 613.2; found 613.2

### Example 174: 2-((2S,4S)-1-acryloyl-4-(8-chloro-7-(3-chloro-2-methylphenyl)-6-fluoro-4-(2-methylpyridin-3-yl)-1H-[1,2,3]triazolo[4,5-c]quinolin-1-yl)piperidin-2-yl)acetonitrile

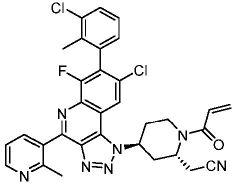

### Step 1 tert-butyl (2S,4S)-4-(7-bromo-8-chloro-6-fluoro-4-(2-methylpyridin-3-yl)-1H-[1,2,3]triazolo[4,5-c]quinolin-1-yl)-2-(cyanomethyl)piperidine-1-carboxylate

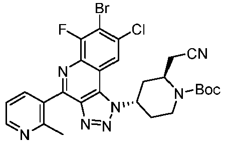

This compound was prepared according to the procedure described **in Example 173, step 2,** replacing tert-butyl (2S,4S)-4-(7-bromo-4,8-dichloro-6-fluoro-1H-imidazo[4,5-c]quinolin-1-yl)-2-(cyanomethyl)piperidine-1-carboxylate with *tert*-butyl (2*S*,4*S*)-4-(7-bromo-4,8-dichloro-6-fluoro-1*H*-[1,2,3]triazolo[4,5-*c*]quinolin-1-yl)-2-(cyanomethyl)piperidine-1-carboxylate (prepared in **Example 91, step 2**). LCMS calculated for C₂₇H₂₇BrClFN₇O₂ (M+H)⁺: m/z = 614.1; found: 614.1.

### Step 2. tert-butyl (2S,4S)-4-(8-chloro-7-(3-chloro-2-methylphenyl)-6-fluoro-4-(2-methylpyridin-3-yl)-1H-[1,2,3]triazolo[4,5-c]quinolin-1-yl)-2-(cyanomethyl)piperidine-1-carboxylate

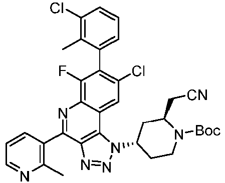

This compound was prepared according to the procedure described in **Example 173, step 4,** replacing 2-((2S,4S)-1-acryloyl-4-(7-bromo-8-chloro-6-fluoro-4-(2-methylpyridin-3-yl)-1H-imidazo[4,5-c]quinolin-1-yl)piperidin-2-yl)acetonitrile with *tert*-butyl (2S,4S)-4-(7-bromo-8-chloro-6-fluoro-4-(2-methylpyridin-3-yl)-1H-[1,2,3]triazolo[4,5-c]quinolin-1-yl)-2-(cyanomethyl)piperidine-1-carboxylate. LCMS calculated for C₃₄H₃₃Cl₂FN₇O₂ (M+H)⁺: m/z = 660.2; found: 660.2.

### Step 3. 2-((2S,4S)-1-acryloyl-4-(8-chloro-7-(3-chloro-2-methylphenyl)-6-fluoro-4-(2-methylpyridin-3-yl)-1H-[1,2,3]triazolo[4,5-c]quinolin-1-yl) piperidin-2-yl)acetonitrile

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1*H-*[1,2,3]triazolo[4,5-*c*]quinolin-1-yl)-2-(cyanomethyl)-piperidine-1-carboxylate with *tert*-butyl (2S,4S)-4-(8-chloro-7-(3-chloro-2-methylphenyl)-6-fluoro-4-(2-methylpyridin-3-yl)-1H-[1,2,3]triazolo[4,5-c]quinolin-1-yl)-2-(cyanomethyl)-piperidine-1-carboxylate. The product was purified and was isolated as a mixture of diastereomers using prep-LCMS (XBridge C18 column, eluting with a gradient of acetonitrile/water containing 0.1% TFA, at flow rate of 60 mL/min) without the second purification. LCMS calculated for C₃₂H₂₇Cl₂FN₇O (M+H)⁺ m/z = 614.2; found 614.2.

### Example 178a and 178b. 2-((2S,4S)-1-(but-2-ynoyl)-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile

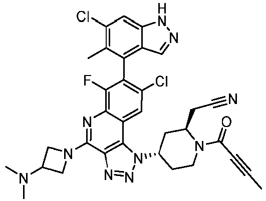

### Step 1. tert-butyl (2S,4S)-4-(8-chloro-7-(6-chloro-5-methyl-1-(tetrahydro-2H-pyran-2-yl)-1H-indazol-4-yl)-6-fluoro-4-(methylthio)-1H-[1,2,3]triazolo[4, 5-c]quinolin-1-yl)-2-(cyanomethyl)piperidine-1-carboxylate

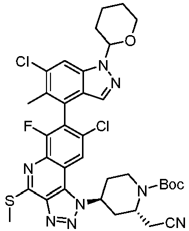

This compound was prepared according to the procedure described in **Example 80a and Example 80b, step 9,** replacing *tert*-butyl (2*S*,4*S*)-4-(7-bromo-8-chloro-6-fluoro-4-(methylthio)-1H-imidazo[4,5-c]quinolin-1-yl)-2-(cyanomethyl)piperidine-1-carboxylate with tert-butyl (2S,4S)-4-(7-bromo-8-chloro-6-fluoro-4-(methylthio)-1H-[1,2,3]triazolo[4,5-c]quinolin-1-yl)-2-(cyanomethyl)piperidine-1-carboxylate (**Example 91, step 3**). LCMS calculated for C₃₅H₃₈Cl₂FN₈O₃S (M+H)⁺ m/z = 739.2; found 739.2.

### Step 2. tert-butyl (2S,4S)-4-(8-chloro-7-(6-chloro-5-methyl-1-(tetrahydro-2H-pyran-2-yl)-1H-indazol-4-yl)-4-(3-(dimethylamino)azetidin-1-yl)-6-fluoro-1H-[1,2,3]triazolo[4, 5-c]quinolin-1-yl)-2-(cyanomethyl) piperidine-1-carboxylate

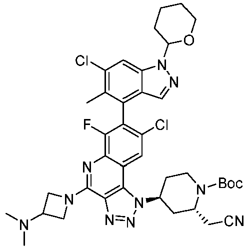

This compound was prepared according to the procedure described in **Example 80a and Example 80b, step 10,** replacing *tert*-butyl (2*S*,4*S*)-4-(8-chloro-7-(6-chloro-5-methyl-1-(tetrahydro-2*H*-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with *tert*-butyl (2S,4S)-4-(8-chloro-7-(6-chloro-5-methyl-1-(tetrahydro-2H-pyran-2-yl)-1H-indazol-4-yl)-6-fluoro-4-(methylthio)-1H-[1,2,3]triazolo[4,5-c]quinolin-1-yl)-2-(cyanomethyl)piperidine-1-carboxylate and replacing N,N,3-trimethylazetidin-3-amine hydrochloride with *N,N*-dimethylazetidin-3-amine hydrochloride. LCMS calculated for C₃₉H₄₆Cl₂FN₁₀O₃ (M+H)⁺ m/z = 791.3; found 791.3.

### Step 3. 2-((2S,4S)-1-(but-2-ynoyl)-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile

This compound was prepared according to the procedure described in **Example 87a and Example 87b,** replacing *tert*-butyl (2*S*,4*S*)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-c]quinolin-1-yl)-2-(cyanomethyl)piperidine-1-carboxylate with *tert*-butyl (2S,4S)-4-(8-chloro-7-(6-chloro-5-methyl-1-(tetrahydro-2H-pyran-2-yl)-1H-indazol-4-yl)-4-(3-(dimethylamino)azetidin-1-yl)-6-fluoro-1H-[1,2,3]triazolo[4,5-c]quinolin-1-yl)-2-(cyanomethyl)piperidine-1-carboxylate.

**Example 178a**. Diastereomer 1. Peak 1. LCMS calculated for C₃₃H₃₂Cl₂FN₁₀O (M+H)⁺ m/z = 673.2; found 673.2.

**Example 178b.** Diastereomer 2. Peak 2. LCMS calculated for C₃₃H₃₂Cl₂FN₁₀O (M+H)⁺ m/z = 673.2; found 673.2.

### Example 182. 2-((2S,4S)-1-(but-2-ynoyl)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)piperidin-2-yl)acetonitrile

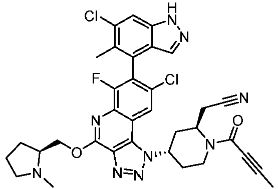

### Step 1. tert-butyl (2S,4S)-4-(8-chloro-7-(6-chloro-5-methyl-1-(tetrahydro-2H-pyran-2-yl)-1H-indazol-4-yl)-6-fluoro-4-(((S)-1-methylpyrrolidin-2-yl)methoxy)-1H-[1,2,3]triazolo[4,5-c]quinolin-1-yl)-2-(cyanomethyl)piperidine-1-carboxylate

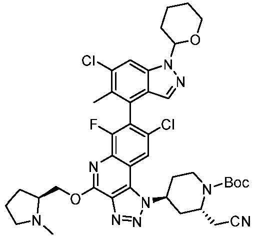

This compound was prepared according to the procedure described in **Example 85, Step 11,** replacing (3-chloro-4-fluorophenyl)boronic acid with 6-chloro-5-methyl-1-(tetrahydro-2H-pyran-2-yl)-4-(4,4,5,5-tetramethyl-1,3,2-dioxaborolan-2-yl)-1H-indazole. LCMS calculated for C₄₀H₄₇Cl₂FN₉O₄ (M+H)⁺ m/z = 806.3; found 806.3.

### Step 2. 2-((2S,4S)-1-(but-2-ynoyl)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 87a and Example 87b,** replacing *tert*-butyl (2*S*,4*S*)-4-(8-chloro-7-(3-chloro-2-methylphenyl)-6-fluoro-4-(((S)-1-methylpyrrolidin-2-yl)methoxy)-1*H*-[1,2,3]triazolo[4,5-*c*]quinolin-1-yl)-2-(cyanomethyl)piperidine-1-carboxylate with *tert*-butyl (2S,4S)-4-(8-chloro-7-(6-chloro-5-methyl-1-(tetrahydro-2H-pyran-2-yl)-1H-indazol-4-yl)-6-fluoro-4-(((S)-1-methylpyrrolidin-2-yl)methoxy)-1H-[1,2,3]triazolo[4,5-c]quinolin-1-yl)-2-(cyanomethyl)piperidine-1-carboxylate. LCMS calculated for C₃₄H₃₃Cl₂FN₉O₂ (M+H)⁺ m/z = 688.2; found 688.2.

### Example 185a and 185b. 2-((2S,4S)-1-acryloyl-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile

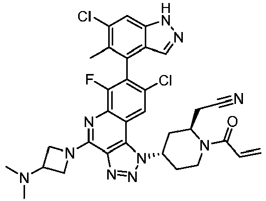

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1H-[1,2,3]triazolo[4,5-c]quinolin-1-yl)-2-(cyanomethyl)-piperidine-1-carboxylate with *tert*-butyl (2S,4S)-4-(8-chloro-7-(6-chloro-5-methyl-1-(tetrahydro-2H-pyran-2-yl)-1H-indazol-4-yl)-4-(3-(dimethylamino)azetidin-1-yl)-6-fluoro-1H-[1,2,3]triazolo[4,5-c]quinolin-1-yl)-2-(cyanomethyl)piperidine-1-carboxylate (**Example 178a and Example 178b, Step 2**)**.**

**Example 185a.** Diastereomer 1. Peak 1. LCMS calculated for C₃₂H₃₂Cl₂FN₁₀O (M+H)⁺ m/z = 661.2; found 661.2. ¹H NMR (500 MHz, DMSO) δ 13.35 (s, 1H), 10.47 (s, 1H), 8.43 (s, 1H), 7.85 (s, 1H), 7.54 (d, *J =* 4.4 Hz, 1H), 6.95 (m, 1H), 6.21 (dd, *J* = 16.7, 2.1 Hz, 1H), 5.85-5.79 (m, 2H), 5.32 (s, 1H), 5.01 (s, 1H), 4.73 (d, *J* = 12.8 Hz, 1H), 4.40-4.30 (m, 2H), 3.61 (dd, *J* = 13.5, 13.2 Hz, 1H), 3.47 (dd, J = 18.0, 8.0 Hz, 1H), 3.27 -3.20 (m, 3H), 2.89 (s, 6H), 2.50-2.41 (m, 2H), 2.26 (d, *J* = 9.8 Hz, 1H), 2.19 (s, 3H), 2.15-2.10 (m, 1H).

**Example 185b.** Diastereomer 2. Peak 2. LCMS calculated for C₃₂H₃₂Cl₂FN₁₀O (M+H)⁺ m/z = 661.2; found 661.2.

### Example 189. 2-((2S,4S)-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)-1-(2-fluoroacryloyl)piperidin-2-yl)acetonitrile

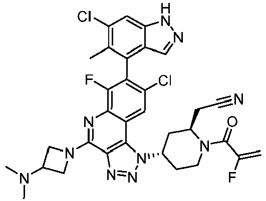

This compound was prepared according to the procedure described in **Example 178a and Example 178b, Step 3** replacing but-2-ynoic acid with 2-fluoroacrylic acid. The product was isolated as a mixture of diastereomers. LCMS calculated for C₃₂H₃₁Cl₂F₂N₁₀O (M+H)⁺ m/z = 679.2; found 679.2. ¹H NMR (500 MHz, DMSO) δ 13.34 (s, 1H), 10.49 (s, 1H), 8.43 (s, 1H), 7.86 (s, 1H), 7.54 (d, *J* = 4.4 Hz, 1H), 5.85 (m, 1H), 5.42-5.28 (m, 2H), 4.78-4.65 (m, 3H), 4.48-4.32 (m, 2H), 3.47 (dd, J = 18.0, 8.0 Hz, 1H), 3.68-3.53 (m, 3H), 3.28 (ddd, *J* = 16.8, 6.1, 3.3 Hz, 1H), 2.90 (s, 6H), 2.50-2.41 (m, 2H), 2.2 (d, *J* = 9.8 Hz, 1H), 2.19 (s, 3H), 2.20-2.15 (m, 1H).

### Example 191a and Example 191b. 2-((2S,4S)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)-1-((E)-4-methoxybut-2-enoyl)piperidin-2-yl)acetonitrile

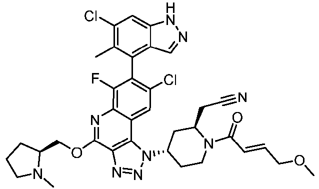

This compound was prepared according to the procedure described in **Example 80a and Example 80b, Step 11,** replacing *tert*-butyl (2S,4S)-4-(8-chloro-7-(6-chloro-5-methyl-1-(tetrahydro-2H-pyran-2-yl)-1H-indazol-4-yl)-4-(3-(dimethylamino)-3-methylazetidin-1-yl)-6-fluoro-1H-imidazo[4,5-c]quinolin-1-yl)-2-(cyanomethyl)piperidine-1-carboxylate with *tert*-butyl (2S,4S)-4-(8-chloro-7-(6-chloro-5-methyl-1-(tetrahydro-2H-pyran-2-yl)-1H-indazol-4-yl)-6-fluoro-4-(((S)-1-methylpyrrolidin-2-yl)methoxy)-1H-[1,2,3]triazolo[4,5-c]quinolin-1-yl)-2-(cyanomethyl)piperidine-1-carboxylate (**Example 182, Step** 1).

**Example 191a.** Diastereomer 1. Peak 1. LCMS calculated for C₃₅H₃₇Cl₂FN₉O₃ (M+H)⁺ m/z = 720.2; found 720.2.

**Example 191b.** Diastereomer 2. Peak 2. LCMS calculated for C₃₅H₃₇Cl₂FN₉O₃ (M+H)⁺ m/z = 720.2; found 720.2.

### Example 193a and Example 193b. 2-((2S,4S)-1-acryloyl-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile

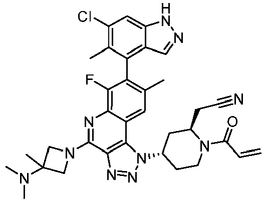

### Step 1. tert-butyl (2S,4S)-4-(7-(6-chloro-5-methyl-1-(tetrahydro-2H-pyran-2-yl)-1H-indazol-4-yl)-6-fluoro-8-methyl-4-(methylthio)-1H-[1,2,3]triazolo[4,5-c]quinolin-1-yl)-2-(cyanomethyl)piperidine-1-carboxylate

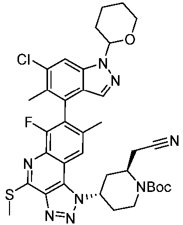

This compound was prepared according to the procedure described in **Example 98, Step 6,** replacing (3-chloro-2-methylphenyl)boronic acid with 6-chloro-5-methyl-1-(tetrahydro-2H-pyran-2-yl)-4-(4,4,5,5-tetramethyl-1,3,2-dioxaborolan-2-yl)-1H-indazole. LCMS calculated for C₃₆H₄₁ClFN₈O₃S (M+H)⁺ m/z = 719.3; found 719.3.

### Step 2. tert-butyl (2S,4S)-4-(7-(6-chloro-5-methyl-1-(tetrahydro-2H-pyran-2-yl)-1H-indazol-4-yl)-4-(3-(dimethylamino)-3-methylazetidin-1-yl)-6-fluoro-8-methyl-1H-[1,2,3]triazolo[4,5-cjquinolin-1-yl)-2-(cyanomethyl)piperidine-1-carboxylate

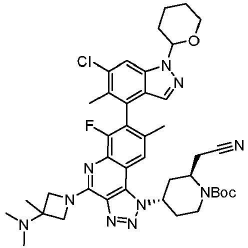

This compound was prepared according to the procedure described in **Example 80a and Example 80b, Step 10,** replacing *tert*-butyl (2*S*,4*S*)-4-(8-chloro-7-(6-chloro-5-methyl-1-(tetrahydro-2*H*-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with *tert*-butyl (2S,4S)-4-(7-(6-chloro-5-methyl-1-(tetrahydro-2H-pyran-2-yl)-1H-indazol-4-yl)-6-fluoro-8-methyl-4-(methylthio)-1H-[1,2,3]triazolo[4,5-c]quinolin-1-yl)-2-(cyanomethyl)piperidine-1-carboxylate. LCMS calculated for C₄₁H₅₁ClFN₁₀O₃ (M+H)⁺ m/z = 785.4; found 785.4.

### Step 3. 2-((2S,4S)-1-acryloyl-4-(7-(6-chloro-5-methyl-1H-indazol-4-yl)-4-(3-(dimethylamino)-3-methylazetidin-1-yl)-6-tluoro-8-methyl-1H-[1,2,3]triazolo[4,5-c]quinolin-1-yl)piperidin-2-yl)acetonitrile

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1H-[1 ,2,3]triazolo[4,5-c]quinolin-1-yl)-2-(cyanomethyl)piperidine-1-carboxylate with *tert*-butyl (2S,4S)-4-(7-(6-chloro-5-methyl-1-(tetrahydro-2H-pyran-2-yl)-1H-indazol-4-yl)-4-(3-(dimethylamino)-3-methylazetidin-1-yl)-6-fluoro-8-methyl-1H-[1,2,3]triazolo[4,5-c]quinolin-1-yl)-2-(cyanomethyl)piperidine-1-carboxylate.

**Example 193a.** Diastereomer 1. Peak 1. LCMS calculated for C₃₄H₃₇ClFN₁₀O (M+H)⁺ m/z = 655.3; found 655.3.

**Example 193b.** Diastereomer 2. Peak 2. LCMS calculated for C₃₄H₃₇ClFN₁₀O (M+H)⁺ m/z = 655.3; found 655.3.

### Example 194a and Example 194b. 2-((2S,4S)-1-(but-2-ynoyl)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile

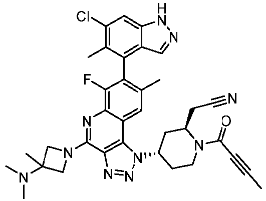

This compound was prepared according to the procedure described in **Example 87a and Example 87b,** replacing *tert*-butyl (2*S*,4*S*)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with *tert*-butyl (2S,4S)-4-(7-(6-chloro-5-methyl-1-(tetrahydro-2H-pyran-2-yl)-1H-indazol-4-yl)-4-(3-(dimethylamino)-3-methylazetidin-1-yl)-6-fluoro-8-methyl-1H-[1,2,3]triazolo[4,5-c]quinolin-1-yl)-2-(cyanomethyl)piperidine-1-carboxylate (**Example 193, Step 2**).

**Example 194a.** Diastereomer 1. Peak 1. LCMS calculated for C₃₅H₃₇ClFN₁₀O (M+H)⁺ m/z = 667.3; found 667.3.

**Example 194b.** Diastereomer 2. Peak 2. LCMS calculated for C₃₅H₃₇ClFN₁₀O (M+H)⁺ m/z = 667.3; found 667.3.

### Example 198a and Example 198b. 2-((2S,4S)-1-acryloyl-4-(7-(5,6-dimethyl-1H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile

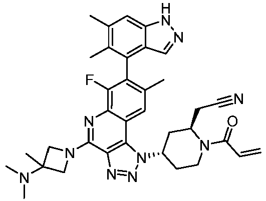

This compound was prepared according to the procedure described in **Example 193a and Example 193b, Steps 1 to 3,** replacing 6-chloro-5-methyl-1-(tetrahydro-2H-pyran-2-yl)-4-(4,4,5,5-tetramethyl-1,3,2-dioxaborolan-2-yl)-1H-indazole with 5,6-dimethyl-1-(tetrahydro-2H-pyran-2-yl)-4-(4,4,5,5-tetramethyl-1,3,2-dioxaborolan-2-yl)-1H-indazole in **Steps 2.**

**Example 198a.** Diastereomer 1. Peak 1. LCMS calculated for C₃₅H₄₀FN₁₀O (M+H)⁺ m/z = 635.3; found 635.3.

**Example 198b.** Diastereomer 2. Peak 2. LCMS calculated for C₃₅H₄₀FN₁₀O (M+H)⁺ m/z = 635.3; found 635.3.

### Example 199. 2-((2S,4S)-1-acryloyl-4-(6-fluoro-7-(4-fluoro-3-methylphenyl)-8-methyl-4-(((S)-1-methylpyrrolidin-2-yl)methoxy)-1H-[1,2,3]triazolo[4,5-c]quinolin-1-yl)piperidin-2-yl)acetonitrile

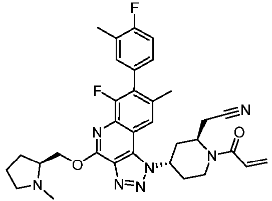

### Step 1. tert-butyl (2S,4S)-2-(cyanomethyl)-4-(6-fluoro-7-(4-fluoro-3-methylphenyl)-8-methyl-4-(methylthio)-1H-[1,2,3]triazolo[4,5-c]quinolin-1-yl)piperidine-1-carboxylate

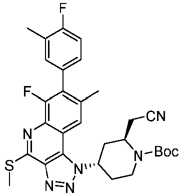

This compound was prepared according to the procedure described in **Example 98, Step 6,** replacing (3-chloro-2-methylphenyl)boronic acid with (4-fluoro-3-methylphenyl)boronic acid. LCMS calculated for C₃₀H₃₃F₂N₆O₂S (M+H)⁺ m/z = 579.2; found 579.2.

### Step 2. tert-butyl (2S,4S)-2-(cyanomethyl)-4-(6-fluoro-7-(4-fluoro-3-methylphenyl)-8-methyl-4-(((S)-1-methylpyrrolidin-2-yl)methoxy)-1H-[1,2,3]triazolo[4,5-c]quinolin-1-yl)piperidine-1-carboxylate

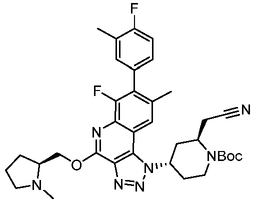

This compound was prepared according to the procedure described in **Example 98, Step 7,** replacing *tert*-butyl (2S,4S)-4-(7-(3-chloro-2-methylphenyl)-6-fluoro-8-methyl-4-(methylthio)-1H-[1,2,3]triazolo[4,5-c]quinolin-1-yl)-2-(cyanomethyl)piperidine-1-carboxylate with tert-butyl (2S,4S)-2-(cyanomethyl)-4-(6-fluoro-7-(4-fluoro-3-methylphenyl)-8-methyl-4-(methylthio)-1H-[1,2,3]triazolo[4,5-c]quinolin-1-yl)piperidine-1-carboxylate. LCMS calculated for C₃₅H₄₂F₂N₇O₃ (M+H)⁺ m/z = 646.3; found 646.2.

### Step 3. 2-((2S,4S)-1-acryloyl-4-(6-fluoro-7-(4-fluoro-3-methylphenyl)-8-methyl-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1H-[1,2,3]triazolo[4,5-c]quinolin-1-yl)-2-(cyanomethyl)piperidine-1-carboxylate with tert-butyl (2S,4S)-2-(cyanomethyl)-4-(6-fluoro-7-(4-fluoro-3-methylphenyl)-8-methyl-4-(((S)-1-methylpyrrolidin-2-yl)methoxy)-1H-[1,2,3]triazolo[4,5-c]quinolin-1-yl)piperidine-1-carboxylate. LCMS calculated for C₃₃H₃₆F₂N₇O₂ (M+H)⁺ m/z = 600.2; found 600.0.

### Example 200a and Example 200b. 2-((2S,4S)-1-acryloyl-4-(4-(3-(dimethylamino)-3-methylazetidin-1-yl)-6-fluoro-8-methyl-7-(5-methyl-1H-indazol-4-yl)-1H-[1,2,3]triazolo[4,5-c]quinolin-1-yl)piperidin-2-yl)acetonitrile

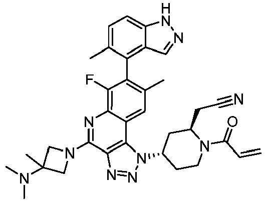

This compound was prepared according to the procedure described in **Example 193a and Example 193b, Steps 1 to 3,** replacing 6-chloro-5-methyl-1-(tetrahydro-2H-pyran-2-yl)-4-(4,4,5,5-tetramethyl-1,3,2-dioxaborolan-2-yl)-1H-indazole with 5-methyl-1-(tetrahydro-2H-pyran-2-yl)-4-(4,4,5,5-tetramethyl-1,3,2-dioxaborolan-2-yl)-1H-indazole in **Step 2.**

**Example 200a.** Diastereomer 1. Peak 1. LCMS calculated for C₃₄H₃₈FN₁₀O (M+H)⁺ m/z = 621.3; found 621.3.

**Example 200b.** Diastereomer 2. Peak 2. LCMS calculated for C₃₄H₃₈FN₁₀O (M+H)⁺ m/z = 621.3; found 621.3.

### Example 210. 2-((2S)-1-acryloyl-4-(8-chloro-6-fluoro-4-(((S)-1-methylpyrrolidin-2-yl)methoxy)-7-(m-tolyl)-1H-[1,2,3]triazolo[4,5-c]quinolin-1-yl)piperidin-2-yl)acetonitrile

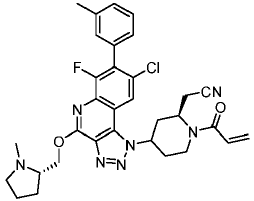

### Step 1. tert-butyl (2S)-4-(8-chloro-6-fluoro-4-(methylthio)-7-(m-tolyl)-1H-[1,2,3]triazolo[4,5-c]quinolin-1-yl)-2-(cyanomethyl)piperidine-1-carboxylate

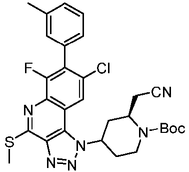

This compound was prepared according to the procedure described in **Example 91, step 4,** replacing (3-chloro-2-methylphenyl)boronic acid with *m-tolylboronic acid.* LCMS calculated for C₂₉H₃₁ClFN₆O₂S (M+H)⁺: m/z = 581.2; found: 581.2.

### Step 2. tert-butyl (2S)-4-(8-chloro-6-fluoro-4-(((S)-1-methylpyrrolidin-2-yl)methoxy)-7-(m-tolyl)-1H-[1,2,3]triazolo[4,5-c]quinolin-1-yl)-2-(cyanomethyl)piperidine-1-carboxylate

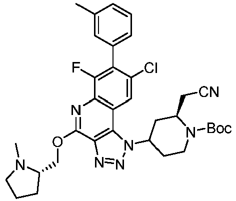

This compound was prepared according to the procedure described in **Example 91, step 5,** replacing tert-butyl (2*S*,4*S*)-4-(8-chloro-7-(3-chloro-2-methylphenyl)-6-fluoro-4-(methylthio)-1*H*-[1,2,3]triazolo[4,5-*c*]quinolin-1-yl)-2-(cyanomethyl)piperidine-1-carboxylate with tert-butyl (2*S*)-4-(8-chloro-6-fluoro-4-(methylthio)-7-(*m*-tolyl)-1*H*-[1,2,3]triazolo[4,5-c]quinolin-1-yl)-2-(cyanomethyl)piperidine-1-carboxylate.LCMS calculated for C₃₄H₄₀ClFN₇O₃ (M+H)⁺: m/z = 648.3; found: 648.3.

### Step 3. 2-((2S)-1-acryloyl-4-(8-chloro-6-fluoro-4-(((S)-1-methylpyrrolidin-2-yl)methoxy)-7-(m-tolyl)-1H-[1,2,3triazolo[4,5-c]quinolin-1-yl)piperidin-2-yl)acetonitrile

This compound was prepared according to the procedure described in **Example 85, step 12,** replacing *tert*-butyl (2*S*,4*S*)-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with *tert*-butyl (2*S*)-4-(8-chloro-6-fluoro-4-(((*S*)-1-methylpyrrolidin-2-yl)methoxy)-7-(*m*-tolyl)-1*H*-[1,2,3]triazolo[4,5-*c*]quinolin-1-yl)-2-(cyanomethyl)piperidine-1-carboxylate. The reaction was diluted with acetonitrile and purified using prep-LCMS (XBridge C18 column, eluting with a gradient of acetonitrile/water containing 0.1% TFA, at flow rate of 60 mL/min) to afford the desired product. LCMS calculated for C₃₂H₃₄ClFN₇O₂ (M+H)⁺: m/z = 602.2; found: 602.2.

### Example 211. 2-((2S)-1-acryloyl-4-(8-chloro-4-(3-(dimethylamino)-3-methylazetidin-1-yl)-6-fluoro-7-(m-tolyl)-1H-[1,2,3]triazolo[4,5-c]quinolin-1-yl)piperidin-2-yl)acetonitrile

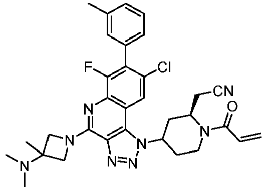

### Step 1. tert-butyl (2S)-4-(8-chloro-4-(3-(dimethylamino)-3-methylazetidin-1-yl)-6-fluoro-7-(m-tolyl)-1H-[1,2,3]triazolo[4,5-c]quinolin-1-yl)-2-(cyanomethyl)piperidine-1-carboxylate

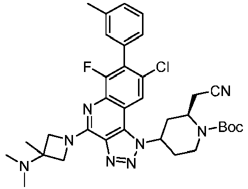

This compound was prepared according to the procedure described in **Example 80a and Example 80b, Step 10,** replacing *tert*-butyl (2*S*,4*S*)-4-(8-chloro-7-(6-chloro-5-methyl-1-(tetrahydro-2*H*-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with *tert*-butyl (2*S*)-4-(8-chloro-6-fluoro-4-(methylthio)-7-(m-tolyl)-1*H*-[1,2,3]triazolo[4,5-*c*]quinolin-1-yl)-2-(cyanomethyl)-piperidine-1-carboxylate (prepared according to **Example 210, Steps 1**). LCMS calculated for C₃₄H₄₁ClFN₈O₂ (M+H)⁺ m/z = 647.3; found 647.3.

### Step 2. tert-butyl (2S)-2-(cyanomethyl)-4-(4-(3-(dimethylamino)-3-methylazetidin-1-yl)-6-fluoro-8-methyl-7-(m-tolyl)-1H-[1,2,3]triazolo[4,5-c]quinolin-1-yl)piperidine-1-carboxylate

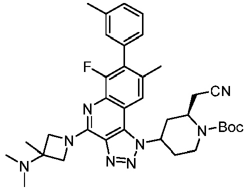

A screw-cap vial equipped with a magnetic stir bar was charged *tert*-butyl (2*S*)-4-(8-chloro-4-(3-(dimethylamino)-3-methylazetidin-1-yl)-6-fluoro-7-(*m*-tolyl)-1*H*-[1,2,3]triazolo[4,5-c]quinolin-1-yl)-2-(cyanomethyl)piperidine-1-carboxylate (55.0 mg, 0.085 mmol), methylboronic acid (25.4 mg, 0.425 mmol), K₃PO₄ (54.1 mg, 0.255 mmol), and XPhosPd G4 (36.6 mg, 0.042 mmol) followed by dioxane (3.5 mL) and water (1 mL). The vial was purged with nitrogen for 5 minutes. Then the reaction was stirred at 100 °C 45 minutes. After cooling to room temperature, the mixture was diluted with brine and extracted with EtOAc. The combined organic layers were dried over MgSO₄, filtered, concentrated to dryness, and purified on silica gel to yield the desired product. LCMS calculated for C₃₅H₄₄FN₈O₂ (M+H)⁺: m/z = 627.4; found: 627.4.

### Step 3. 2-((2S)-1-acryloyl-4-(8-chloro-4-(3-(dimethylamino)-3-methylazetidin-1-yl)-6-fluoro-7-(m-tolyl)-1H-[1,2,3]triazolo[4,5-c]quinolin-1-yl)piperidin-2-yl)acetonitrile

This compound was prepared according to the procedure described in **Example 85, step 12,** replacing *tert*-butyl (2*S*,4*S*)-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)-piperidine-1-carboxylate with *tert*-butyl (2*S*)-2-(cyanomethyl)-4-(4-(3-(dimethylamino)-3-methylazetidin-1-yl)-6-fluoro-8-methyl-7-(*m*-tolyl)-1*H*-[1,2,3]triazolo[4,5-*c*]quinolin-1-yl)piperidine-1-carboxylate. The reaction was diluted with acetonitrile and purified using prep-LCMS (XBridge C18 column, eluting with a gradient of acetonitrile/water containing 0.1% TFA, at flow rate of 60 mL/min) to afford the desired product. LCMS calculated for C₃₂H₃₅ClFN₈O (M+H)⁺: m/z = 601.3; found: 601.3. ¹H NMR (600 MHz, DMSO-*d*₆) δ 7.96 (s, 1H), 7.42 (d, *J* = 7.6 Hz, 1H), 7.27 (d, *J =* 7.6 Hz, 1H), 7.15 (d, *J =* 7.6 Hz, 1H), 6.95 (dd, *J* = 16.7, 10.6 Hz, 1H), 6.20 (dd, J = 16.7, 2.3 Hz, 1H), 5.83-5.70 (m, 2H), 5.30-5.22 (m, 1H), 5.10-4.94 (m, 1H), 4.77-4.07 (m, 1H), 4.50-3.70 (m, 4H), 3.67-3.55 (m, 1H), 3.44-3.17 (m, 2H), 2.45-2.10 (m, 16H), 1.33 (s, 3H).

### Example 212a and Example 212b. 2-((2S)-1-acryloyl-4-(8-chloro-7-(8-chloronaphthalen-1-yl)-4-(3-(dimethylamino)-3-methylazetidin-1-yl)-6-fluoro-1H-imidazo[4,5-c]quinolin-1-yl)piperidin-2-yl)acetonitrile

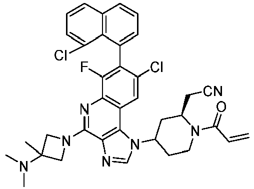

### Step 1. tert-butyl (2S)-4-(8-chloro-7-(8-chloronaphthalen-1-yl)-6-fluoro-4-(methylthio)-1H-imidazo[4, 5-c]quinolin-1-yl)-2-(cyanomethyl)piperidine-1-carboxylate

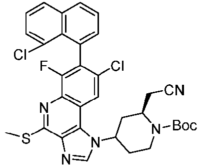

This compound was prepared according to the procedure described in **Example 80a and Example 80b, Step 9,** replacing 6-chloro-5-methyl-1-(tetrahydro-2*H*-pyran-2-yl)-4-(4,4,5,5-tetramethyl-1,3,2-dioxaborolan-2-yl)-1*H*-indazole with 2-(8-chloronaphthalen-1-yl)-4,4,5,5-tetramethyl-1,3,2-dioxaborolane. LCMS calculated for C₃₃H₃₁Cl₂FN₅O₂S (M+H)⁺ m/z = 650.2; found 650.2.

### Step 2. tert-butyl (2S)-4-(8-chloro-7-(8-chloronaphthalen-1-yl)-4-(3-(dimethylamino)-3-methylazetidin-1-yl)-6-fluoro-1H-imidazo[4,5-c]quinolin-1-yl)-2-(cyanomethyl)piperidine-1-carboxylate

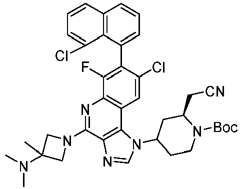

This compound was prepared according to the procedure described in **Example 80a and Example 80b, Step 10,** replacing *tert*-butyl (2*S*,4*S*)-4-(8-chloro-7-(6-chloro-5-methyl-1-(tetrahydro-2*H*-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with *tert*-butyl (2*S*)-4-(8-chloro-7-(8-chloronaphthalen-1-yl)-6-fluoro-4-(methylthio)-1H-imidazo[4,5-c]quinolin-1-yl)-2-(cyanomethyl)piperidine-1-carboxylate. LCMS calculated for C₃₈H₄₁Cl₂FN₇O₂ (M+H)⁺ m/z = 716.3; found 716.3.

### Step 3. 2-((2S)-1-acryloyl-4-(8-chloro-7-(8-chloronaphthalen-1-yl)-4-(3-(dimethylamino)-3-methylazetidin-1-yl)-6-fluoro-1H-imidazo[4,5-c]quinolin-1-yl)piperidin-2-yl)acetonitrile

This compound was prepared according to the procedure described in **Example 85, step 12,** replacing *tert*-butyl (2*S*,4*S*)-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with *tert*-butyl (2*S*)-4-(8-chloro-7-(8-chloronaphthalen-1-yl)-4-(3-(dimethylamino)-3-methylazetidin-1-yl)-6-fluoro-1*H*-imidazo[4,5-*c*]quinolin-1-yl)-2-(cyanomethyl)piperidine-1-carboxylate. The reaction was diluted with acetonitrile and purified using prep-LCMS (XBridge C18 column, eluting with a gradient of acetonitrile/water containing 0.1% TFA, at flow rate of 60 mL/min) to afford the desired product.

**Example 212a.** Diastereomer 1. Peak 1. LCMS calculated for C₃₆H₃₅Cl₂FN₇O (M+H)⁺ m/z = 670.2; found 670.2.

**Example 212b.** Diastereomer 2. Peak 2. LCMS calculated for C₃₆H₃₅Cl₂FN₇O (M+H)⁺ m/z = 670.2; found 670.2.

### Example 215a and Example 215b. 2-((2S)-1-acryloyl-4-(8-chloro-7-(8-chloronaphthalen-1-yl)-6-fluoro-4-(((S)-1-methylpyrrolidin-2-yl)methoxy)-1H-imidazo[4,5-c]quinolin-1-yl)piperidin-2-yl)acetonitrile

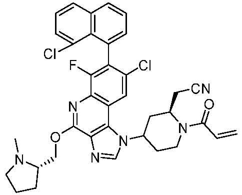

### Step 1. tert-butyl (2S)-4-(8-chloro-7-(8-chloronaphthalen-1-yl)-6-fluoro-4-(methylthio)-1H-imidazo[4, 5-c]quinolin-1-yl)-2-(cyanomethyl)piperidine-1-carboxylate

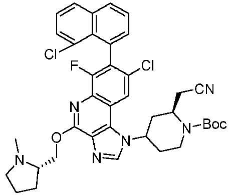

This compound was prepared according to the procedure described in **Example 91, step 5,** replacing *tert*-butyl (2*S*,4*S*)-4-(8-chloro-7-(3-chloro-2-methylphenyl)-6-fluoro-4-(methylthio)-1H-[1,2,3]triazolo[4,5-*c*]quinolin-1-yl)-2-(cyanomethyl)piperidine-1-carboxylate with *tert*-butyl (2S)-4-(8-chloro-7-(8-chloronaphthalen-1-yl)-6-fluoro-4-(methylthio)-1*H-*imidazo[4,5-c]quinolin-1-yl)-2-(cyanomethyl)piperidine-1-carboxylate (prepared from **Example 212a and Example 212b, step 1).** LCMS calculated for C₃₈H₄₀Cl₂FNₑO₃ (M+H)⁺ m/z = 717.3; found 717.3.

### Step 2. 2-((2S)-1-acryloyl-4-(8-chloro-7-(8-chloronaphthalen-1-yl)-6-fluoro-4-(((S)-1-methylpyrrolidin-2-yl)methoxy)-1H-imidazo[4,5-c]quinolin-1-yl)piperidin-2-yl)acetonitrile

This compound was prepared according to the procedure described in **Example 80a and Example 80b, Step 11,** replacing 2-((2*S*,4*S*)-4-(8-chloro-7-(6-chloro-5-methyl-1H-indazol-4-yl)-4-(3-(dimethylamino)-3-methylazetidin-1-yl)-6-fluoro-1H-imidazo[4,5-*c*]quinolin-1-yl)-1-((*E*)-4-methoxybut-2-enoyl)piperidin-2-yl)acetonitrile with *tert-butyl (2S)-4-(8-chloro-7-(8-chloronaphthalen-1-yl)-6-fluoro-4-(methylthio)-1H-imidazo[4,5-c]quinolin-1-yl)-2-(cyanomethyl)piperidine-1-carboxylate.* The reaction was diluted with acetonitrile and purified using prep-LCMS (XBridge C18 column, eluting with a gradient of acetonitrile/water containing 0.1% TFA, at flow rate of 60 mL/min) to afford the desired product.

**Example 215a.** Diastereomer 1. Peak 1. LCMS calculated for C₃₆H₃₄Cl₂FN₆O₂ (M+H)⁺ m/z = 671.2; found 671.2.

**Example 215b.** Diastereomer 2. Peak 2. LCMS calculated for C₃₆H₃₄Cl₂FN₆O₂ (M+H)⁺ m/z = 671.2; found 671.2.

### Example 216a and Example 216b. 2-((2S)-1-acryloyl-4-(8-chloro-7-(8-chloronaphthalen-1-yl)-4-(3-(dimethylamino)-3-methylazetidin-1-yl)-6-fluoro-1H-[1,2,3]triazolo[4,5-c]quinolin-1-yl)piperidin-2-yl)acetonitrile

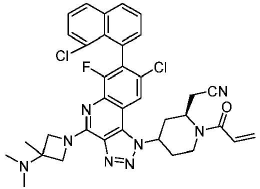

### Step 1. tert-butyl (2S)-4-(8-chloro-7-(8-chloronaphthalen-1-yl)-6-fluoro-4-(methylthio)-1H-[1,2,3]triazolo[4,5-c]quinolin-1-yl)-2-(cyanomethyl)piperidine-1-carboxylate

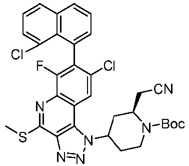

This compound was prepared according to the procedure described in **Example 91, step 4,** replacing (3-chloro-2-methylphenyl)boronic acid with 2-(8-chloronaphthalen-1-yl)-4,4,5,5-tetramethyl-1,3,2-dioxaborolane. LCMS calculated for C₃₂H₃₀Cl₂FN₆O₂S (M+H)⁺ m/z = 651.2; found 651.2.

### Step 2. tert-butyl (2S)-4-(8-chloro-7-(8-chloronaphthalen-1-yl)-4-(3-(dimethylamino)-3-methylazetidin-1-yl)-6-fluoro-1H-[1,2,3]triazolo[4,5-c]quinolin-1-yl)-2-(cyanomethyl)piperidine-1-carboxylate

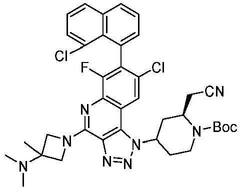

This compound was prepared according to the procedure described in **Example 80a and Example 80b, Step 10,** replacing *tert*-butyl (2*S*,4*S*)-4-(8-chloro-7-(6-chloro-5-methyl-1-(tetrahydro-2*H*-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with *tert*-butyl (2*S*)-4-(8-chloro-7-(8-chloronaphthalen-1-yl)-6-fluoro-4-(methylthio)-1*H*-[1,2,3]triazolo[4,5-*c*]quinolin-1-yl)-2-(cyanomethyl)piperidine-1-carboxylate. LCMS calculated for C₃₇H₄₀Cl₂FN₈O₂ (M+H)⁺ m/z = 717.3; found 717.3.

### Step 3. 2-((2S)-1-acryloyl-4-(8-chloro-7-(8-chloronaphthalen-1-yl)-4-(3-(dimethylamino)-3-methylazetidin-1-yl)-6-fluoro-1H-[1,2,3]triazolo[4,5-c]quinolin-1-yl)piperidin-2-yl)acetonitrile

This compound was prepared according to the procedure described in **Example 80a and Example 80b, Step 11,** replacing 2-((2*S*,4*S*)-4-(8-chloro-7-(6-chloro-5-methyl-1H-indazol-4-yl)-4-(3-(dimethylamino)-3-methylazetidin-1-yl)-6-fluoro-1H-imidazo[4,5-c]quinolin-1-yl)-1-((*E*)-4-methoxybut-2-enoyl)piperidin-2-yl)acetonitrile with *tert*-butyl (2*S*)-4-(8-chloro-7-(8-chloronaphthalen-1-yl)-4-(3-(dimethylamino)-3-methylazetidin-1-yl)-6-fluoro-1*H-*[1,2,3]triazolo[4,5-*c*]quinolin-1-yl)-2-(cyanomethyl)piperidine-1-carboxylate. The reaction was diluted with acetonitrile and purified using prep-LCMS (XBridge C18 column, eluting with a gradient of acetonitrile/water containing 0.1% TFA, at flow rate of 60 mL/min) to afford the desired product.

**Example 216a.** Diastereomer 1. Peak 1. LCMS calculated for C₃₅H₃₄Cl₂FN₈O (M+H)⁺ m/z = 671.2; found 671.2.

**Example 216b.** Diastereomer 2. Peak 2. LCMS calculated for C₃₅H₃₄Cl₂FN₈O (M+H)⁺ m/z = 671.2; found 671.2.

### Example 217. 2-((2S)-4-(8-chloro-7-(8-chloronaphthalen-1-yl)-4-(3-(dimethylamino)-3-methylazetidin-1-yl)-6-fluoro-1H-[1,2,3]triazolo[4,5-c]quinolin-1-yl)-1-((E)-4-(dimethylamino)but-2-enoyl)piperidin-2-yl)acetonitrile

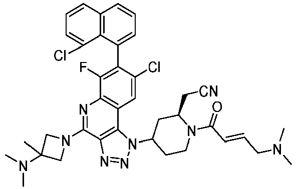

This compound was prepared according to the procedure described in **Example 87a and Example 87b,** replacing *tert*-butyl (2*S*,4*S*)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)piperidine-1-carboxylate with *tert*-butyl (2*S*)-4-(8-chloro-7-(8-chloronaphthalen-1-yl)-4-(3-(dimethylamino)-3-methylazetidin-1-yl)-6-fluoro-1*H*-[1,2,3]triazolo[4,5-*c*]quinolin-1-yl)-2-(cyanomethyl)piperidine-1-carboxylate (prepared from **Example 216a and Example 216b, step 3**) and but-2-ynoic acid with (*E*)-4-(dimethylamino)but-2-enoic acid. The reaction was diluted with acetonitrile and purified using prep-LCMS (XBridge C18 column, eluting with a gradient of acetonitrile/water containing 0.1% TFA, at flow rate of 60 mL/min) to afford the desired product as a mixture of diasteromers. LCMS calculated for C₃₈H₄₁Cl₂FN₉O (M+H)⁺ m/z = 728.3; found 728.3.

### Example 220a and Example 220b. 8-(1-((2S)-1-acryloyl-2-(cyanomethyl)piperidin-4-yl)-8-chloro-4-(3-(dimethylamino)-3-methylazetidin-1-yl)-6-fluoro-1H-[1,2,3]triazolo[4,5-c]quinolin-7-yl)-1-naphthonitrile

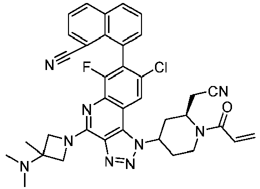

### Step 1. tert-butyl (2S)-4-(8-chloro-7-(8-cyanonaphthalen-1-yl)-6-fluoro-4-(methylthio)-1H-[1,2,3]triazolo[4,5-c]quinolin-1-yl)-2-(cyanomethyl)piperidine-1-carboxylate

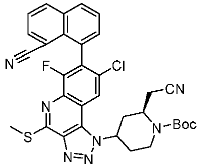

This compound was prepared according to the procedure described in **Example 91, step 4,** replacing (3-chloro-2-methylphenyl)boronic acid with 8-(4,4,5,5-tetramethyl-1,3,2-dioxaborolan-2-yl)-1-naphthonitrile. LCMS calculated for C₃₃H₃₀ClN₇O₂S (M+H)⁺ m/z = 642.2; found 642.2.

### Step 2. tert-butyl (2S)-4-(8-chloro-7-(8-cyanonaphthalen-1-yl)-4-(3-(dimethylamino)-3-methylazetidin-1-yl)-6-fluoro-1H-[1,2,3]triazolo[4,5-c]quinolin-1-yl)-2-(cyanomethyl)piperidine-1-carboxylate

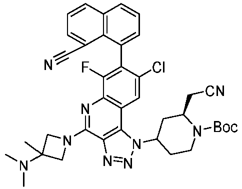

This compound was prepared according to the procedure described in **Example 80a and Example 80b, Step 10,** replacing *tert*-butyl (2*S*,4*S*)-4-(8-chloro-7-(6-chloro-5-methyl-1-(tetrahydro-2*H*-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with *tert*-butyl (2*S*)-4-(8-chloro-7-(8-cyanonaphthalen-1-yl)-6-fluoro-4-(methylthio)-1*H*-[1,2,3]triazolo[4,5-*c*]quinolin-1-yl)-2-(cyanomethyl)piperidine-1-carboxylate. The reaction was diluted with acetonitrile and purified using prep-LCMS (XBridge C18 column, eluting with a gradient of acetonitrile/water containing 0.1% TFA, at flow rate of 60 mL/min) to isolate the two diastereomers, which are each separately carried onto the next step.

Diastereomer 1. Peak 1. LCMS calculated for C₃₈H₄₀ClFN₉O₂ (M+H)⁺ m/z = 708.3; found 708.3.

Diastereomer 2. Peak 2. LCMS calculated for C₃₈H₄₀ClFN₉O₂ (M+H)⁺ m/z = 708.3; found 708.3.

### Step 3. 8-(1-((2S)-1-acryloyl-2-(cyanomethyl)piperidin-4-yl)-8-chloro-4-(3-(dimethylamino)-3-methylazetidin-1-yl)-6-fluoro-1H-[1,2,3]triazolo[4,5-c]quinolin-7-yl)-1-naphthonitrile

This compound was prepared according to the procedure described in **Example 80a and Example 80b, Step 11,** replacing 2-((2*S*,4*S*)-4-(8-chloro-7-(6-chloro-5-methyl-1H-indazol-4-yl)-4-(3-(dimethylamino)-3-methylazetidin-1-yl)-6-fluoro-1H-imidazo[4,5-*c*]quinolin-1-yl)-1-((*E*)-4-methoxybut-2-enoyl)piperidin-2-yl)acetonitrile with *tert*-butyl (2*S*)-4-(8-chloro-7-(8-chloronaphthalen-1-yl)-4-(3-(dimethylamino)-3-methylazetidin-1-yl)-6-fluoro-1*H-*[1,2,3]triazolo[4,5-*c*]quinolin-1-yl)-2-(cyanomethyl)piperidine-1-carboxylate (Diastereomer 1 and Diastereomer 2, Step 2 each run upon separately). The reaction was diluted with acetonitrile and purified using prep-LCMS (XBridge C18 column, eluting with a gradient of acetonitrile/water containing 0.1% TFA, at flow rate of 60 mL/min) to afford the desired product.

**Example 220a.** Diastereomer 1. Peak 1. LCMS calculated for C₃₆H₃₄ClFN₉O (M+H)⁺ m/z = 662.3; found 662.3.

**Example 220b.** Diastereomer 2. Peak 2. LCMS calculated for C₃₆H₃₄ClFN₉O (M+H)⁺ m/z = 662.3; found 662.3.

### Example 221a and Example 221b. 2-((2S)-1-acryloyl-4-(7-(8-chloronaphthalen-1-yl)-4-(3-(dimethylamino)-3-methylazetidin-1-yl)-6-fluoro-8-methyl-1H-imidazo[4,5-c]quinolin-1-yl)piperidin-2-yl)acetonitrile

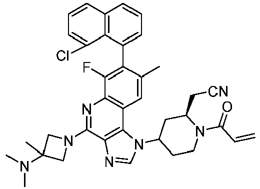

### Step 1. tert-butyl (2S,4S)-4-(7-bromo-4-chloro-6-fluoro-8-iodo-1H-imidazo[4,5-c]quinolin-1-yl)-2-(cyanomethyl) piperidine-1-carboxylate

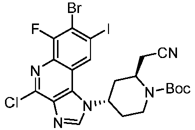

This compound was prepared according to the procedure described in **Example 23, Step 1**, replacing *tert*-butyl (*endo*)-5-((3-amino-7-bromo-6-chloro-2-(3-(dimethylamino)-azetidin-1-yl)-8-fluoroquinolin-4-yl)amino)-2-azabicyclo[2.1.1]hexane-2-carboxylate with *tert-*butyl (2*S*,4*S*)-4-((3-amino-7-bromo-2-chloro-8-fluoro-6-iodoquinolin-4-yl)amino)-2-(cyanomethyl)piperidine-1-carboxylate (prepared from **Example 98, Step 2**). LCMS calculated for C₂₂H₂₂BrClFlN₅O₂ (M+H)⁺: m/z = 648.0; found: 648.0.

### Step 2. tert-butyl (2S,4S)-4-(7-bromo-6-fluoro-8-iodo-4-(methylthio)-1H-imidazo[4,5-c]quinolin-1-yl)-2-(cyanomethyl)piperidine-1-carboxylate

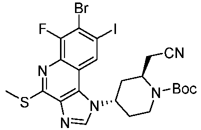

This compound was prepared according to the procedure described in **Example 80a and Example 80b, Step 4,** replacing *tert*-butyl (2*S*,4*S*)-4-(7-bromo-4,8-dichloro-6-fluoro-1*H-*imidazo[4,5-*c*]quinolin-1-yl)-2-(2-(*tert*-butoxy)-2-oxoethyl)piperidine-1-carboxylate with *tert-*butyl (2*S*,4*S*)-4-(7-bromo-4-chloro-6-fluoro-8-iodo-1*H*-imidazo[4,5-*c*]quinolin-1-yl)-2-(cyanomethyl)piperidine-1-carboxylate. LCMS calculated for C₂₃H₂₅BrFlN₅O₂S (M+H)⁺: m/z = 660.0; found: 660.0.

### Step 3. tert-butyl (2S)-4-(7-bromo-6-fluoro-8-methyl-4-(methylthio)-1H-imidazo[4,5-c]quinolin-1-yl)-2-(cyanomethyl)piperidine-1-carboxylate

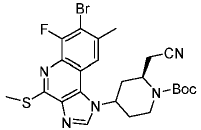

This compound was prepared according to the procedure described in **Example 98, Step 5,** replacing *tert*-butyl (2*S*,4*S*)-4-(7-bromo-6-fluoro-8-iodo-4-(methylthio)-1*H-*[1,2,3]triazolo[4,5-*c*]quinolin-1-yl)-2-(cyanomethyl)piperidine-1-carboxylate with *tert*-butyl (2*S*,4*S*)-4-(7-bromo-6-fluoro-8-iodo-4-(methylthio)-1H-imidazo[4,5-*c*]quinolin-1-yl)-2-(cyanomethyl)piperidine-1-carboxylate. LCMS calculated for C₂₄H₂₈BrFN₅O₂S (M+H)⁺: m/z = 548.1; found: 548.1.

### Step 4. tert-butyl (2S)-4-(7-(8-chloronaphthalen-1-yl)-6-fluoro-8-methyl-4-(methylthio)-1H-imidazo[4,5-c]quinolin-1-yl)-2-(cyanomethyl)piperidine-1-carboxylate

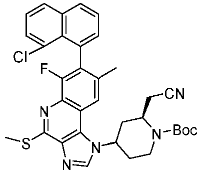

This compound was prepared according to the procedure described in **Example 80a and Example 80b, Step 9,** replacing *tert*-butyl (2*S*,4*S*)-4-(7-bromo-8-chloro-6-fluoro-4-(methylthio)-1*H*-imidazo[4,5-c]quinolin-1-yl)-2-(cyanomethyl)piperidine-1-carboxylate with *tert*-butyl (2*S*)-4-(7-bromo-6-fluoro-8-methyl-4-(methylthio)-1H-imidazo[4,5-*c*]quinolin-1-yl)-2-(cyanomethyl)piperidine-1-carboxylate and 6-chloro-5-methyl-1-(tetrahydro-2*H*-pyran-2-yl)-4-(4,4,5,5-tetramethyl-1,3,2-dioxaborolan-2-yl)-1*H*-indazole with 2-(8-chloronaphthalen-1-yl)-4,4,5,5-tetramethyl-1,3,2-dioxaborolane. LCMS calculated for C₃₄H₃₄ClFN₅O₂S (M+H)⁺: m/z = 630.2; found: 630.2.

### Step 5. tert-butyl (2S)-4-(7-(8-chloronaphthalen-1-yl)-4-(3-(dimethylamino)-3-methylazetidin-1-yl)-6-fluoro-8-methyl-1H-imidazo[4,5-c]quinolin-1-yl)-2-(cyanomethyl)piperidine-1-carboxylate

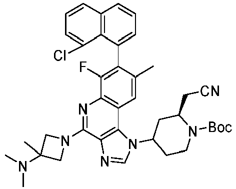

This compound was prepared according to the procedure described in **Example 80a and Example 80b, Step 10,** replacing *tert*-butyl (2*S*,4*S*)-4-(8-chloro-7-(6-chloro-5-methyl-1-(tetrahydro-2*H*-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with *tert*-butyl (2*S*)-4-(7-(8-chloronaphthalen-1-yl)-6-fluoro-8-methyl-4-(methylthio)-1H-imidazo[4,5-*c*]quinolin-1-yl)-2-(cyanomethyl)piperidine-1-carboxylate. LCMS calculated for C₃₉H₄₄ClFN₇O₂ (M+H)⁺ m/z = 696.3; found 696.3.

### Step 6. 2-((2S)-1-acryloyl-4-(7-(8-chloronaphthalen-1-yl)-4-(3-(dimethylamino)-3-methylazetidin-1-yl)-6-fluoro-8-methyl-1H-imidazo[4,5-c]quinolin-1-yl)piperidin-2-yl)acetonitrile

This compound was prepared according to the procedure described in **Example 80a and Example 80b, Step 11,** replacing 2-((2*S*,4*S*)-4-(8-chloro-7-(6-chloro-5-methyl-1H-indazol-4-yl)-4-(3-(dimethylamino)-3-methylazetidin-1-yl)-6-fluoro-1H-imidazo[4,5-c]quinolin-1-yl)-1-((*E*)-4-methoxybut-2-enoyl)piperidin-2-yl)acetonitrile with *tert*-butyl (2*S*)-4-(7-(8-chloronaphthalen-1-yl)-4-(3-(dimethylamino)-3-methylazetidin-1-yl)-6-fluoro-8-methyl-1*H-*imidazo[4,5-c]quinolin-1-yl)-2-(cyanomethyl)piperidine-1-carboxylate. The reaction was diluted with acetonitrile and purified using prep-LCMS (XBridge C18 column, eluting with a gradient of acetonitrile/water containing 0.1% TFA, at flow rate of 60 mL/min) to afford the desired product.

**Example 221a.** Diastereomer 1. Peak 1. LCMS calculated for C₃₇H₃₈ClFN₇O (M+H)⁺ m/z = 650.3; found 650.3.

**Example 221b.** Diastereomer 2. Peak 2. LCMS calculated for C₃₇H₃₈ClFN₇O (M+H)⁺ m/z = 650.3; found 650.3.

### Example 229. 2-((2S,4S)-1-acryloyl-4-(4-(3-(dimethylamino)azetidin-1-yl)-6-fluoro-7-(5-fluoroquinolin-8-yl)-8-(trifluoromethyl)-1H-imidazo[4,5-c]quinolin-1-yl)piperidin-2-yl)acetonitrile

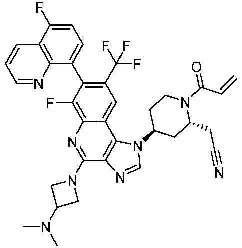

### Step 1. tert-butyl (2S,4R)-4-((7-bromo-2-(3-(dimethylamino)azetidin-1-yl)-8-fluoro-6-iodo-3-nitroquinolin-4-yl)amino)-2-(2-(tert-butoxy)-2-oxoethyl)piperidine-1-carboxylate

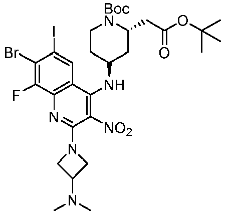

This compound was prepared according to the procedure described in **Example 15, step 1,** replacing 7-bromo-2,4,6-trichloro-8-fluoro-3-nitroquinoline with 7-bromo-2,4-dichloro-8-fluoro-6-iodo-3-nitroquinoline, and *tert*-butyl (*endo*)-5-amino-2-azabicyclo[2.1.1]hexane-2-carboxylate with *tert*-butyl (2S,4S)-4-amino-2-(2-(*tert*-butoxy)-2-oxoethyl)piperidine-1-carboxylate. LCMS calculated for C₃₀H₄₂BrFIN₆O₆ (M+H)⁺: m/z = 807.1; found: 807.1.

### Step 2. tert-butyl (2S,4R)-4-((3-amino-7-bromo-2-(3-(dimethylamino)azetidin-1-yl)-8-fluoro-6-iodoquinolin-4-yl)amino)-2-(2-(tert-butoxy)-2-oxoethyl)piperidine-1-carboxylate

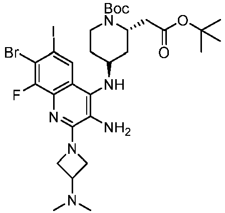

This compound was prepared according to the procedure described in **Example 15, step 2,** replacing *tert*-butyl (endo)-5-((7-bromo-6-chloro-2-(3-(dimethylamino)azetidin-1-yl)-8-fluoro-3-nitroquinolin-4-yl)amino)-2-azabicyclo[2.1.1]hexane-2-carboxylate with *tert*-butyl (2*S*,4*R*)-4-((7-bromo-2-(3-(dimethylamino)azetidin-1-yl)-8-fluoro-6-iodo-3-nitroquinolin-4-yl)amino)-2-(2-(*tert*-butoxy)-2-oxoethyl)piperidine-1-carboxylate. LCMS calculated for C₃₀H₄₄BrFIN₆O₄ (M+H)⁺: m/z = 777.2; found: 777.1.

### Step 3. tert-butyl (2S,4S)-4-(7-bromo-4-(3-(dimethylamino)azetidin-1-yl)-6-fluoro-8-iodo-1H-imidazo[4,5-c]quinolin-1-yl)-2-(2-(tert-butoxy)-2-oxoethyl)piperidine-1-carboxylate

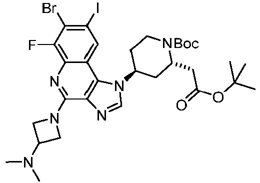

This compound was prepared according to the procedure described in **Example 1, step 8,** replacing *tert*-butyl (S)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2*S*,4*R*)-4-((3-amino-7-bromo-2-(3-(dimethylamino)azetidin-1-yl)-8-fluoro-6-iodoquinolin-4-yl)amino)-2-(2-(*tert*-butoxy)-2-oxoethyl)piperidine-1-carboxylate. LCMS calculated for C₃₁H₄₂BrFIN₆O₄ (M+H)⁺: m/z = 787.2; found: 787.1.

### Step 4. tert-butyl (2S,4S)-4-(7-bromo-4-(3-(dimethylamino)azetidin-1-yl)-6-fluoro-8-(trifluoromethyl)-1H-imidazo[4,5-c]quinolin-1-yl)-2-(2-(tert-butoxy)-2-oxoethyl)piperidine-1-carboxylate

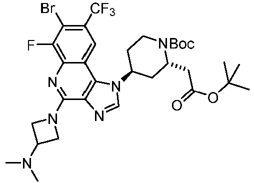

A vial was charged with *tert*-butyl (2S,4S)-4-(7-bromo-4-(3-(dimethylamino)azetidin-1-yl)-6-fluoro-8-iodo-1*H*-imidazo[4,5-*c*]quinolin-1-yl)-2-(2-(*tert*-butoxy)-2-oxoethyl)piperidine-1-carboxylate (1.37 g, 1.74 mmol), Cul (0.050 g, 0.26 mmol), 1,10-phenanthroline (0.047 g, 0.26 mmol), and KF (0.303 g, 5.22 mmol). DMSO (3 mL) was added and the mixture was degassed for 5 min before trimethyl borate (0.58 mL, 0.26 mmol) and trimethyl(trifluoromethyl)silane (2.61 mL, 5.22 mmol) were added. The mixture was degassed for another 5 min, then heated to 80 °C for 3 h. Additional trimethyl(trifluoromethyl)silane was added (2.61 mL, 5.22 mmol), then the reaction was stirred at 80 °C for 15 h. The mixture was cooled to room temperature and diluted with EtOAc (10 mL). The organic phase was washed by water three times (10 mL x 3), concentrated and purified on silica gel to yield the desired product (1.04 g, 82%). LCMS calculated for C₃₂H₄₂BrF₄N₆O₄ (M+H)⁺: m/z = 729.2; found: 729.2.

### Step 5. tert-butyl (2S,4S)-4-(7-bromo-4-(3-(dimethylamino)azetidin-1-yl)-6-fluoro-8-(trifluoromethyl)-1H-imidazo[4,5-c]quinolin-1-yl)-2-(cyanomethyl)piperidine-1-carboxylate

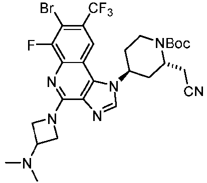

This compound was prepared according to the procedure described in **Example 80a and Example 80b, Steps 5-8,** replacing *tert*-butyl (2S,4S)-4-(7-bromo-8-chloro-6-fluoro-4-(methylthio)-1*H*-imidazo[4,5-*c*]quinolin-1-yl)-2-(2-(*tert*-butoxy)-2-oxoethyl)piperidine-1-carboxylate with *tert*-butyl (2*S*,4*S*)-4-(7-bromo-4-(3-(dimethylamino)azetidin-1-yl)-6-fluoro-8-(trifluoromethyl)-1*H*-imidazo[4,5-*c*]quinolin-1-yl)-2-(2-(*tert*-butoxy)-2-oxoethyl)piperidine-1-carboxylate. LCMS calculated for C₂₈H₃₃BrF₄N₇O₂ (M+H)⁺ m/z = 654.2; found 654.1.

### Step 6. tert-butyl (2S,4S)-2-(cyanomethyl)-4-(4-(3-(dimethylamino)azetidin-1-yl)-6-fluoro-7-(5-fluoroquinolin-8-yl)-8-(trifluoromethyl)-1H-imidazo[4,5-c]quinolin-1-yl)piperidine-1-carboxylate

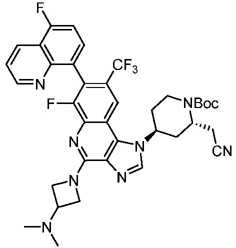

This compound was prepared according to the procedure described in **Example 16, Steps 6,** replacing 2-((2*S*,4*S*)-1-acryloyl-4-(7-bromo-8-chloro-4-(3-(dimethylamino)azetidin-1-yl)-6-fluoro-1*H*-imidazo[4,5-c]quinolin-1-yl)piperidin-2-yl)acetonitrile with *tert*-butyl (2*S*,4*S*)-4-(7-bromo-4-(3-(dimethylamino)azetidin-1-yl)-6-fluoro-8-(trifluoromethyl)-1*H*-imidazo[4,5-*c*]quinolin-1-yl)-2-(cyanomethyl)piperidine-1-carboxylate. LCMS calculated for C₃₇H₃₈F₅N₈O₂ (M+H)⁺ m/z = 721.3; found 721.2.

### Step 7. 2-((2S,4S)-1-acryloyl-4-(4-(3-(dimethylamino)azetidin-1-yl)-6-fluoro-7-(5-fluoroquinolin-8-yl)-8-(trifluoromethyl)-1H-imidazo[4,5-c]quinolin-1-yl)piperidin-2-yl)acetonitrile

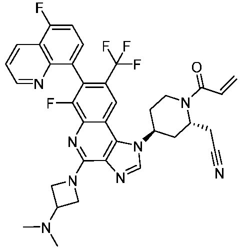

This compound was prepared according to the procedure described in **Example 16, Steps 5,** replacing *tert*-butyl (2S,4S)-4-(7-bromo-8-chloro-4-(3-(dimethylamino)azetidin-1-yl)-6-fluoro-1*H*-imidazo[4,5-*c*]quinolin-1-yl)-2-(cyanomethyl)piperidine-1-carboxylate with *tert-*butyl (2*S*,4*S*)-2-(cyanomethyl)-4-(4-(3-(dimethylamino)azetidin-1-yl)-6-fluoro-7-(5-fluoroquinolin-8-yl)-8-(trifluoromethyl)-1*H*-imidazo[4,5-*c*]quinolin-1-yl)piperidine-1-carboxylate. LCMS calculated for C₃₅H₃₂F₅N₈O (M+H)⁺ m/z = 675.3; found 675.2.

### Example 230a and Example 230b. 2-((2S,4S)-4-(4-(3-(dimethylamino)azetidin-1-yl)-6-fluoro-7-(5-fluoroquinolin-8-yl)-8-(trifluoromethyl)-1H-imidazo[4,5-c]quinolin-1-yl)-1-((E)-4-(dimethylamino)but-2-enoyl)piperidin-2-yl)acetonitrile

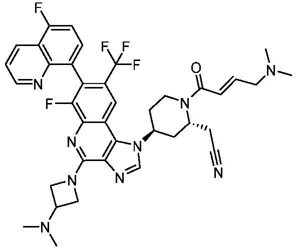

A solution of *tert*-butyl (2S,4S)-2-(cyanomethyl)-4-(4-(3-(dimethylamino)azetidin-1-yl)-6-fluoro-7-(5-fluoroquinolin-8-yl)-8-(trifluoromethyl)-1*H*-imidazo[4,5-*c*]quinolin-1-yl)piperidine-1-carboxylate (100 mg, 0.15 mmol) in TFA (1.0 mL) was stirred at room temperature for 10 minutes before concentrating to dryness. The concentrated residue was re-dissolved in acetonitrile (1.0 mL) and DIPEA (0.27 mL, 1.5 mmol) was added. The mixture was cooled in an ice bath and (*E*)-4-(dimethylamino)but-2-enoic acid (30 mg, 0.23 mmol), propylphosphonic anhydride solution (50 wt. % in ethyl acetate, 0.19 mL. 0.31 mmol) were added. Upon completion, the reaction was diluted with acetonitrile and purified using prep-LCMS (XBridge C18 column, eluting with a gradient of acetonitrile/water containing 0.1% TFA, at flow rate of 60 mL/min) then purified again using prep-LCMS (XBridge C18 column, eluting with a gradient of acetonitrile/water containing 0.1% TFA, at flow rate of 60 mL/min) to afford the desired product.

**Example 230a.** Diastereomer 1. Peak 1. LCMS calculated for C₃₈H₃₉F₅N₉O (M+H)⁺ m/z = 732.3; found 732.3.

**Example 230b.** Diastereomer 2. Peak 2. LCMS calculated for C₃₈H₃₉F₅N₉O (M+H)⁺ m/z = 732.3; found 732.3.

### Example 231a and Example 231b. 3-(1-((2S,4S)-1-(but-2-ynoyl)-2-(cyanomethyl)piperidin-4-yl)-7-(6-chloro-5-methyl-1H-indazol-4-yl)-4-(3-(dimethylamino)azetidin-1-yl)-6-fluoro-1H-[1,2,3]triazolo[4,5-c]quinolin-8-yl)propanenitrile

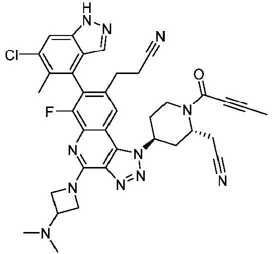

### Step 1. tert-butyl (2S,4S)-4-(7-bromo-4-(3-(dimethylamino)azetidin-1-yl)-6-fluoro-8-iodo-1H-[1,2,3]triazolo[4,5-c]quinolin-1-yl)-2-(2-(tert-butoxy)-2-oxoethyl)piperidine-1-carboxylate

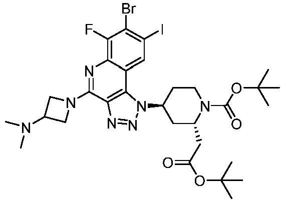

This compound was prepared according to the procedure described in **Example 14, Step 1,** replacing *tert*-butyl (S)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2S,4R)-4-((3-amino-7-bromo-2-(3-(dimethylamino)azetidin-1-yl)-8-fluoro-6-iodoquinolin-4-yl)amino)-2-(2-(*tert*-butoxy)-2-oxoethyl)piperidine-1-carboxylate. LCMS calculated for C₃₀H₄₁BrFIN₇O₄ (M+H)⁺: m/z = 788.1; found: 788.3.

### Step 2. tert-butyl (2S,4S)-4-(7-bromo-8-(2-cyanoethyl)-4-(3-(dimethylamino)azetidin-1-yl)-6-fluoro-1H-[1,2,3]triazolo[4,5-c]quinolin-1-yl)-2-(2-(tert-butoxy)-2-oxoethyl)piperidine-1-carboxylate

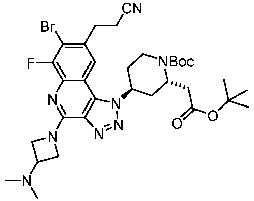

A solution of *tert*-butyl (2*S*,4*S*)-4-(7-bromo-4-(3-(dimethylamino)azetidin-1-yl)-6-fluoro-8-iodo-1*H*-[1,2,3]triazolo[4,5-*c*]quinolin-1-yl)-2-(2-(*tert*-butoxy)-2-oxoethyl)piperidine-1-carboxylate (650 mg, 0.82 mmol) in DMF (3.0 mL) was added palladium(II) acetate (18.5 mg, 0.082 mmol), tri-*o*-tolylphosphine (50 mg, 0.16 mmol), triethylamine (0.17 mL, 1.24 mmol) and acrylonitrile (87 mg, 1.65 mmol). The mixture was degassed for 5 min, before heated to 80 °C for 1 h. The mixture was cooled to room temperature and diluted with EtOAc (3 mL). The organic phase was washed by water three times (3 mL x 3), concentrated, re-dissolved in THF (3.0 mL), and cooled to the 0 °C. Superhydride (1.0 M in THF, 0.29 mL, 1.65 mmol) was added dropwise, and stirred for 30 min. The reaction was then quenched with the addition of saturated NaHCO₃ solution, and extracted with EtOAc (10 mL). The organic phase was concentrated and purified on silica gel (MeOH in DCM, 0-20% gradient) to yield the desired product (480 mg, 91%). LCMS calculated for C₃₃H₄₅BrFN₈O₄ (M+H)⁺: m/z = 715.3; found: 715.4.

### Step 3. tert-butyl (2S,4S)-4-(7-bromo-8-(2-cyanoethyl)-4-(3-(dimethylamino)azetidin-1-yl)-6-fluoro-1H-[1,2,3]triazolo[4,5-c]quinolin-1-yl)-2-(cyanomethyl)piperidine-1-carboxylate

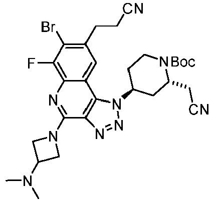

This compound was prepared according to the procedure described in **Example 80a and Example 80b, Steps 5-8,** replacing tert-butyl (2S,4S)-4-(7-bromo-8-chloro-6-fluoro-4-(methylthio)-1*H*-imidazo[4,5-*c*]quinolin-1-yl)-2-(2-(*tert*-butoxy)-2-oxoethyl)piperidine-1-carboxylate with *tert*-butyl (2*S*,4*S*)-4-(7-bromo-8-(2-cyanoethyl)-4-(3-(dimethylamino)azetidin-1-yl)-6-fluoro-1*H*-[1,2,3]triazolo[4,5-*c*]quinolin-1-yl)-2-(2-(*tert-*butoxy)-2-oxoethyl)piperidine-1-carboxylate. LCMS calculated for C₂₉H₃₆BrFN₉O₂ (M+H)⁺: m/z = 640.2; found: 640.2.

### Step 4. tert-butyl (2S,4S)-4-(7-(6-chloro-5-methyl-1-(tetrahydro-2H-pyran-2-yl)-1H-indazol-4-yl)-8-(2-cyanoethyl)-4-(3-(dimethylamino)azetidin-1-yl)-6-fluoro-1H-[1,2,3]triazolo[4,5-c]quinolin-1-yl)-2-(cyanomethyl)piperidine-1-carboxylate

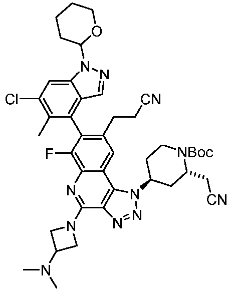

This compound was prepared according to the procedure described in **Example 80a and Example 80b, Steps 9,** replacing *tert*-butyl (2*S*,4*S*)-4-(7-bromo-8-chloro-6-fluoro-4-(methylthio)-1*H*-imidazo[4,5-*c*]quinolin-1-yl)-2-(cyanomethyl)piperidine-1-carboxylate with *tert*-butyl (2*S*,4*S*)-4-(7-bromo-8-(2-cyanoethyl)-4-(3-(dimethylamino)azetidin-1-yl)-6-fluoro-1*H*-[1,2,3]triazolo[4,5-*c*]quinolin-1-yl)-2-(cyanomethyl)piperidine-1-carboxylate. LCMS calculated for C₄₂H₅₀ClFN₁₁O₃ (M+H)⁺: m/z = 810.4; found: 810.3.

### Step 5. 3-(1-((2S,4S)-1-(but-2-ynoyl)-2-(cyanomethyl)piperidin-4-yl)-7-(6-chloro-5-methyl-1H-indazol-4-yl)-4-(3-(dimethylamino)azetidin-1-yl)-6-fluoro-1H-[1,2,3]triazolo[4,5-c]quinolin-8-yl)propanenitrile

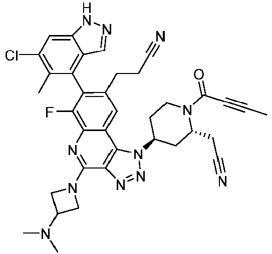

This compound was prepared according to the procedure described in **Example 230a and Example 230b,** replacing *tert*-butyl (2S,4S)-2-(cyanomethyl)-4-(4-(3-(dimethylamino)azetidin-1-yl)-6-fluoro-7-(5-fluoroquinolin-8-yl)-8-(trifluoromethyl)-1*H-*imidazo[4,5-*c*]quinolin-1-yl)piperidine-1-carboxylate with *tert*-butyl (2S,4S)-4-(7-(6-chloro-5-methyl-1-(tetrahydro-2H-pyran-2-yl)-1*H*-indazol-4-yl)-8-(2-cyanoethyl)-4-(3-(dimethylamino)azetidin-1-yl)-6-fluoro-1*H*-[1,2,3]triazolo[4,5-c]quinolin-1-yl)-2-(cyanomethyl)piperidine-1-carboxylate, and (*E*)-4-(dimethylamino)but-2-enoic acid with 2-butynoic acid.

**Example 231a.** Diastereomer 1. Peak 1. LCMS calculated for C₃₆H₃₆ClFN₁₁O (M+H)⁺ m/z = 692.3; found 692.4.

**Example 231b.** Diastereomer 2. Peak 2. LCMS calculated for C₃₆H₃₆ClFN₁₁O (M+H)⁺ m/z = 692.3; found 692.4.

### Example 232. 3-(7-(6-chloro-5-methyl-1H-indazol-4-yl)-1-((2S,4S)-2-(cyanomethyl)-1-((E)-4-(dimethylamino)but-2-enoyl)piperidin-4-yl)-4-(3-(dimethylamino)azetidin-1-yl)-6-fluoro-1H-[1,2,3]triazolo[4,5-c]quinolin-8-yl)propanenitrile

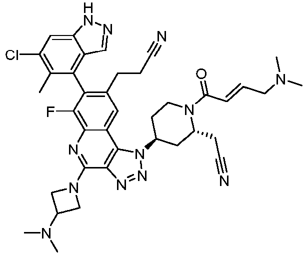

This compound was prepared according to the procedure described in **Example 231a and Example 231b,** replacing 2-butynoic acid with (E)-4-(dimethylamino)but-2-enoic acid. LCMS calculated for C₃₈H₄₃ClFN₁₂O (M+H)⁺: m/z = 737.3; found: 737.2.

### Example 233a and Example 233b. 2-((2S,4S)-1-acryloyl-4-(4-(3-(dimethylamino)-3-methylazetidin-1-yl)-6-fluoro-7-(5-fluoroquinolin-8-yl)-8-(trifluoromethyl)-1H-[1,2,3]triazolo[4,5-c]quinolin-1-yl)piperidin-2-yl)acetonitrile

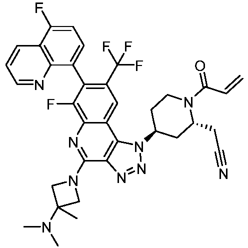

### Step 1. tert-butyl (2S,4R)-4-((3-amino-7-bromo-2-(3-(dimethylamino)-3-methylazetidin-1-yl)-8-fluoro-6-iodoquinolin-4-yl)amino)-2-(2-(tert-butoxy)-2-oxoethyl)piperidine-1-carboxylate

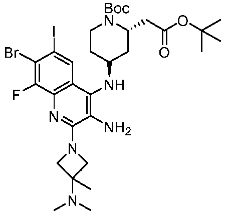

This compound was prepared according to the procedure described in **Example 229, Step 1-2,** replacing *N,N*-dimethylazetidin-3-amine dihydrochloride with *N*,*N*,3-trimethylazetidin-3-amine dihydrochloride. LCMS calculated for C₃₁H₄₆BrFIN₆O₄ (M+H)⁺: m/z = 791.2; found: 791.1.

### Step 2. tert-butyl (2S,4S)-4-(7-bromo-4-(3-(dimethylamino)-3-methylazetidin-1-yl)-6-fluoro-8-iodo-1H-[1, 2, 3]triazolo[4,5-c]quinolin-1-yl)-2-(2-(tert-butoxy)-2-oxoethyl)piperidine-1-carboxylate

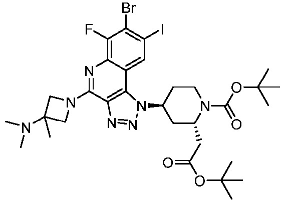

This compound was prepared according to the procedure described in **Example 231a and Example 231b, Step 1,** replacing *tert*-butyl (2*S*,4*R*)-4-((3-amino-7-bromo-2-(3-(dimethylamino)azetidin-1-yl)-8-fluoro-6-iodoquinolin-4-yl)amino)-2-(2-(*tert*-butoxy)-2-oxoethyl)piperidine-1-carboxylate with *tert*-butyl (2*S*,4*R*)-4-((3-amino-7-bromo-2-(3-(dimethylamino)-3-methylazetidin-1-yl)-8-fluoro-6-iodoquinolin-4-yl)amino)-2-(2-(*tert*-butoxy)-2-oxoethyl)piperidine-1-carboxylate. LCMS calculated for C₃₁H₄₃BrFIN₇O₄ (M+H)⁺: m/z = 802.3; found: 802.3.

### Step 3. tert-butyl (2S,4S)-4-(7-bromo-4-(3-(dimethylamino)-3-methylazetidin-1-yl)-6-fluoro-8-(trifluoromethyl)-1H-[1,2,3]triazolo[4,5-c]quinolin-1-yl)-2-(cyanomethyl) piperidine-1-carboxylate

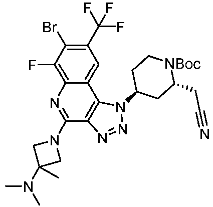

This compound was prepared according to the procedure described in **Example 229, step 4-5,** replacing *tert*-butyl (2S,4S)-4-(7-bromo-4-(3-(dimethylamino)azetidin-1-yl)-6-fluoro-8-iodo-1*H*-imidazo[4,5-*c*]quinolin-1-yl)-2-(2-(*tert*-butoxy)-2-oxoethyl)piperidine-1-carboxylate with *tert*-butyl (2*S*,4*S*)-4-(7-bromo-4-(3-(dimethylamino)-3-methylazetidin-1-yl)-6-fluoro-8-iodo-1*H*-[1,2,3]triazolo[4,5-*c*]quinolin-1-yl)-2-(2-(tert-butoxy)-2-oxoethyl)piperidine-1-carboxylate. LCMS calculated for C₂₃H₃₄BrF₄N₃O₂ (M+H)⁺: m/z = 669.2; found: 669.3.

### Step 4. tert-butyl (2S,4S)-2-(cyanomethyl)-4-(4-(3-(dimethylamino)-3-methylazetidin-1-yl)-6-fluoro-7-(5-fluoroquinolin-8-yl)-8-(trifluoromethyl)-1H-[1,2,3]triazolo[4, 5-c]quinolin-1-yl)piperidine-1-carboxylate

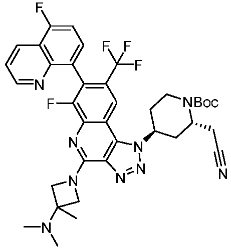

A solution of *tert*-butyl (2*S*,4*S*)-4-(7-bromo-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl)-piperidine-1-carboxylate (80 mg, 0.12 mmol) in dioxane (1.0 mL) and water (0.2 mL) was added Pd(PPh₃)₄ (13.9 mg, 0.012 mmol), tripotassium phosphate (76 mg, 0.36 mmol), and (5-fluoroquinolin-8-yl)boronic acid (46 mg, 0.24 mmol). The mixture was degassed for 5 min, before heated to 100 °C for 2 h. The mixture was cooled to room temperature, diluted with EtOAc (3 mL) and saturated NH₄Cl solution (3 mL). The organic phase was concentrated, and purified on silica gel (MeOH in DCM, 0-20% gradient) to yield the desired product (62 mg, 70%). LCMS calculated for C₃₇H₃₉F₅N₉O₂ (M+H)⁺: m/z = 736.3; found: 736.4.

### Step 5. 2-((2S,4S)-1-acryloyl-4-(4-(3-(dimethylamino)-3-methylazetidin-1-yl)-6-fluoro-7-(5-fluoroquinolin-8-yl)-8-(trifluoromethyl)-1H-[1,2,3]triazolo[4,5-c]quinolin-1-yl)piperidin-2-yl)acetonitrile

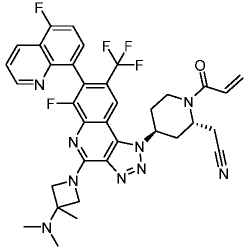

This compound was prepared according to the procedure described in **Example 16, Steps 5,** replacing *tert*-butyl (2*S*,4*S*)-4-(7-bromo-8-chloro-4-(3-(dimethylamino)azetidin-1-yl)-6-fluoro-1*H*-imidazo[4,5-*c*]quinolin-1-yl)-2-(cyanomethyl)piperidine-1-carboxylate with *tert-*butyl (2*S*,4*S*)-2-(cyanomethyl)-4-(4-(3-(dimethylamino)-3-methylazetidin-1-yl)-6-fluoro-7-(5-fluoroquinolin-8-yl)-8-(trifluoromethyl)-1*H*-[1,2,3]triazolo[4,5-*c*]quinolin-1-yl)piperidine-1-carboxylate.

**Example 233a.** Diastereomer 1. Peak 1. LCMS calculated for C₃₅H₃₃F₅N₉O (M+H)⁺ m/z = 690.3; found 690.2.

**Example 233b.** Diastereomer 2. Peak 2. LCMS calculated for C₃₅H₃₃F₅N₉O (M+H)⁺ m/z = 690.3; found 690.2.

### Example 234. 2-((2S,4S)-1-(but-2-ynoyl)-4-(4-(3-(dimethylamino)-3-methylazetidin-1-yl)-6-fluoro-7-(5-fluoroquinolin-8-yl)-8-(trifluoromethyl)-1H-[1,2,3]triazolo[4,5-c]quinolin-1-yl)piperidin-2-yl)acetonitrile

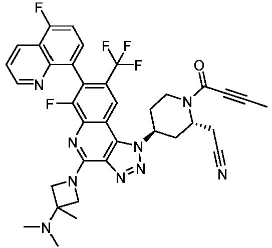

This compound was prepared according to the procedure described in **Example 231a and Example 231b, step 5,** replacing tert-butyl (2*S*,4*S*)-4-(7-(6-chloro-5-methyl-1-(tetrahydro-2H-pyran-2-yl)-1*H*-indazol-4-yl)-8-(2-cyanoethyl)-4-(3-(dimethylamino)azetidin-1-yl)-6-fluoro-1*H*-[1,2,3]triazolo[4,5-*c*]quinolin-1-yl)-2-(cyanomethyl)piperidine-1-carboxylate with tert-butyl (2*S*,4*S*)-2-(cyanomethyl)-4-(4-(3-(dimethylamino)-3-methylazetidin-1-yl)-6-fluoro-7-(5-fluoroquinolin-8-yl)-8-(trifluoromethyl)-1*H*-[1,2,3]triazolo[4,5-*c*]quinolin-1-yl)piperidine-1-carboxylate. LCMS calculated for C₃₆H₃₃F₅N₉O (M+H)⁺: m/z = 702.3; found: 702.1. ¹H NMR (TFA salt) (600 MHz, DMSO-*d*₆) δ 10.78 (s, 1H), 8.88 (ddd, *J* = 7.5, 4.2, 1.9 Hz, 1H), 8.62 (dt, *J* = 8.4, 1.9 Hz, 1H), 8.42 - 8.37 (m, 1H), 7.82 (t, *J = 6.9* Hz, 1H), 7.70 (ddd, *J* = 8.3, 4.1, 2.0 Hz, 1H), 7.65 (dq, *J* = 11.0, 3.7 Hz, 1H), 5.94 (dq, *J* = 30.1, 14.2, 13.1 Hz, 1H), 5.16 (ddd, *J* = 24.4, 11.6, 5.9 Hz, 1H), 4.97 (s, 1H), 4.75 (s, 1H), 4.71 - 4.50 (m, 3H), 4.28 (s, 1H), 3.74 - 3.62 (m, 1H), 3.47 (ddd, *J* = 35.2, 16.8, 9.5 Hz, 1H), 3.19 (dddd, *J* = 20.9, 16.7, 8.7, 5.8 Hz, 2H), 2.84 (s, 6H), 2.46 - 2.41 (m, 2H), 2.12 - 2.05 (m, 3H), 1.70 (s, 3H).

### Example 235a and Example 235b. 2-((2S,4S)-1-acryloyl-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile

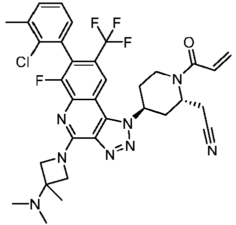

### Step 1. tert-butyl (2S,4S)-4-(7-(2-chloro-3-methylphenyl)-4-(3-(dimethylamino)-3-methylazetidin-1-yl)-6-fluoro-8-(trifluoromethyl)-1H-[1, 2, 3]triazolo[4, 5-c]quinolin-1-yl)-2-(cyanomethyl)piperidine-1-carboxylate

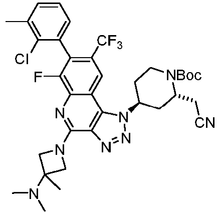

This compound was prepared according to the procedure described in **Example 233a and Example 233b, step 4,** replacing (5-fluoroquinolin-8-yl)boronic acid with (2-chloro-3-methylphenyl)boronic acid. LCMS calculated for C₃₅H₄₀ClF₄N₈O₂ (M+H)⁺: m/z = 715.3; found: 715.1.

### Step 2. 2-((2S,4S)-1-acryloyl-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile

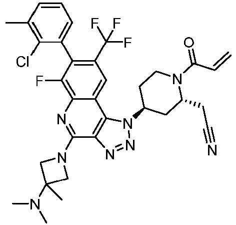

This compound was prepared according to the procedure described in **Example 16, Steps 5,** replacing *tert*-butyl (2*S*,4*S*)-4-(7-bromo-8-chloro-4-(3-(dimethylamino)azetidin-1-yl)-6-fluoro-1*H*-imidazo[4,5-*c*]quinolin-1-yl)-2-(cyanomethyl)piperidine-1-carboxylate with *tert-*butyl (2*S*,4*S*)-4-(7-(2-chloro-3-methylphenyl)-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl)piperidine-1-carboxylate.

**Example 235a.** Diastereomer 1. Peak 1. LCMS calculated for C₃₃H₃₄ClF₄N₈O (M+H)⁺ m/z = 669.2; found 669.2.

**Example 235b.** Diastereomer 2. Peak 2. LCMS calculated for C₃₃H₃₄ClF₄N₈O (M+H)⁺ m/z = 669.2; found 669.2.

### Example 237. 2-((2S,4S)-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile

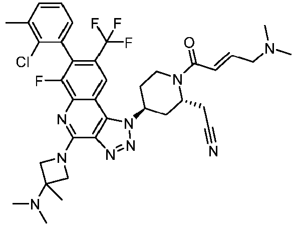

This compound was prepared according to the procedure described in **Example 232,** replacing *tert*-butyl (2*S*,4*S*)-4-(7-(6-chloro-5-methyl-1-(tetrahydro-2H-pyran-2-yl)-1*H*-indazol-4-yl)-8-(2-cyanoethyl)-4-(3-(dimethylamino)azetidin-1-yl)-6-fluoro-1*H*-[1,2,3]triazolo[4,5-*c*]quinolin-1-yl)-2-(cyanomethyl)piperidine-1-carboxylate with *tert*-butyl (2*S*,4*S*)-4-(7-(2-chloro-3-methylphenyl)-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl)piperidine-1-carboxylate. LCMS calculated for C₃₆H₄₁ClF₄N₉O (M+H)⁺ m/z = 726.3; found 726.2.

### Example 238a and Example 238b. 2-((2S,4S)-1-acryloyl-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile

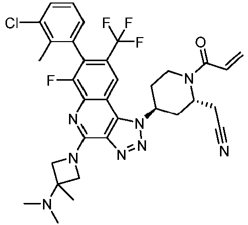

### Step 1. tert-butyl (2S,4S)-4-(7-(3-chloro-2-methylphenyl)-4-(3-(dimethylamino)-3-methylazetidin-1-yl)-6-fluoro-8-(trifluoromethyl)-1H-[1, 2, 3]triazolo[4, 5-c]quinolin-1-yl)-2-(cyanomethyl)piperidine-1-carboxylate

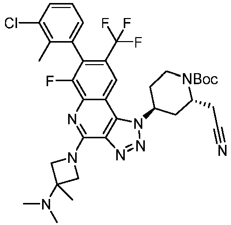

This compound was prepared according to the procedure described in **Example 233a and Example 233b, step 4,** replacing (5-fluoroquinolin-8-yl)boronic acid with (3-chloro-2-methylphenyl)boronic acid. LCMS calculated for C₃₅H₄₀ClF₄N₈O₂ (M+H)⁺: m/z = 715.3; found: 715.1.

### Step 2. 2-((2S,4S)-1-acryloyl-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile

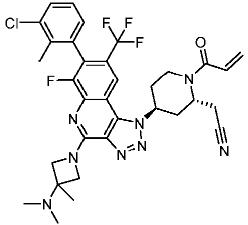

This compound was prepared according to the procedure described in **Example 16, Steps 5,** replacing *tert*-butyl (2*S*,4*S*)-4-(7-bromo-8-chloro-4-(3-(dimethylamino)azetidin-1-yl)-6-fluoro-1*H*-imidazo[4,5-*c*]quinolin-1-yl)-2-(cyanomethyl)piperidine-1-carboxylate with *tert-*butyl (2*S*,4*S*)-4-(7-(3-chloro-2-methylphenyl)-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl)piperidine-1-carboxylate.

**Example 238a.** Diastereomer 1. Peak 1. LCMS calculated for C₃₃H₃₄ClF₄N₈O (M+H)⁺ m/z = 669.2; found 669.4.

**Example 238b.** Diastereomer 2. Peak 2. LCMS calculated for C₃₃H₃₄ClF₄N₈O (M+H)⁺ m/z = 669.2; found 669.4.

### Example 240. 2-((2S,4S)-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile

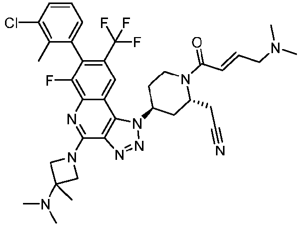

This compound was prepared according to the procedure described in **Example 232,** replacing *tert*-butyl (2S,4S)-4-(7-(6-chloro-5-methyl-1-(tetrahydro-2H-pyran-2-yl)-1H-indazol-4-yl)-8-(2-cyanoethyl)-4-(3-(dimethylamino)azetidin-1-yl)-6-fluoro-1*H*-[1,2,3]triazolo[4,5-c]quinolin-1-yl)-2-(cyanomethyl)piperidine-1-carboxylate with *tert*-butyl (2*S*,4*S*)-4-(7-(3-chloro-2-methylphenyl)-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl)piperidine-1-carboxylate. LCMS calculated for C₃₆H₄₁ClF₄N₉O (M+H)⁺ m/z = 726.3; found 726.3.

### Example 242a and Example 242b. 3-(1-((2S,4S)-1-(but-2-ynoyl)-2-(cyanomethyl)piperidin-4-yl)-7-(2-chloro-3-methylphenyl)-6-fluoro-4-(((S)-1-methylpyrrolidin-2-yl)methoxy)-1H-[1,2,3]triazolo[4,5-c]quinolin-8-yl)propanenitrile

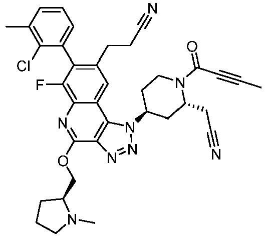

### Step 1. tert-butyl (2S,4S)-4-(7-(2-chloro-3-methylphenyl)-8-(2-cyanoethyl)-6-fluoro-4-(((S)-1-methylpyrrolidin-2-yl)methoxy)-1H-[1, 2, 3]triazolo[4, 5-c]quinolin-1-yl)-2-(cyanomethyl)piperidine-1-carboxylate

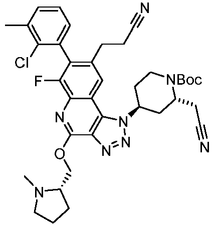

This compound was prepared according to the procedure described in **Example 235a and Example 235b, Steps 1,** replacing *tert*-butyl (2S,4S)-4-(7-bromo-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl)piperidine-1-carboxylate with *tert*-butyl (2S,4S)-4-(7-bromo-8-(2-cyanoethyl)-6-fluoro-4-(((*S*)-1-methylpyrrolidin-2-yl)methoxy)-1H-[1,2,3]triazolo[4,5-*c*]quinolin-1-yl)-2-(cyanomethyl)piperidine-1-carboxylate. LCMS calculated for C₃₇H₄₃ClFN₈O₃ (M+H)⁺ m/z = 701.3; found 701.2.

### Step 2. 3-(1-((2S,4S)-1-(but-2-ynoyl)-2-(cyanomethyl)piperidin-4-yl)-7-(2-chloro-3-methylphenyl)-6-fluoro-4-(((S)-1-methylpyrrolidin-2-yl)methoxy)-1H-[1,2,3]triazolo[4,5-c]quinolin-8-yl)propanenitrile

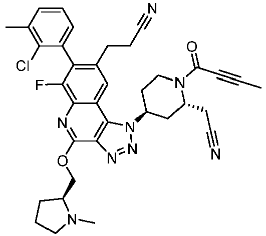

This compound was prepared according to the procedure described in **Example 231a and Example 231b, step 5,** replacing tert-butyl (2*S*,4*S*)-4-(7-(6-chloro-5-methyl-1-(tetrahydro-2H-pyran-2-yl)-1*H*-indazol-4-yl)-8-(2-cyanoethyl)-4-(3-(dimethylamino)azetidin-1-yl)-6-fluoro-1*H*-[1,2,3]triazolo[4,5-*c*]quinolin-1-yl)-2-(cyanomethyl)piperidine-1-carboxylate with tert-butyl (2*S*,4*S*)-4-(7-(2-chloro-3-methylphenyl)-8-(2-cyanoethyl)-6-fluoro-4-(((*S*)-1-methylpyrrolidin-2-yl)methoxy)-1*H*-[1,2,3]triazolo[4,5-*c*]quinolin-1-yl)-2-(cyanomethyl) piperidine-1-carboxylate

**Example 242a.** Diastereomer 1. Peak 1. LCMS calculated for C₃₃H₃₇ClFN₈O₂ (M+H)⁺ m/z = 667.3; found 667.4.

**Example 242b.** Diastereomer 2. Peak 2. LCMS calculated for C₃₃H₃₇ClFN₈O₂ (M+H)⁺ m/z = 667.3; found 667.4.

### Example 243a and Example 243b. 2-((2S,4S)-1-(but-2-ynoyl)-4-(7-(3-chloro-2-methoxyphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile

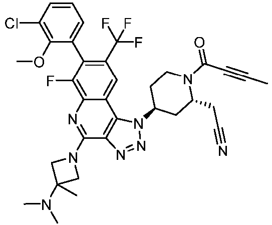

### Step 1. tert-butyl (2S,4S)-4-(7-(3-chloro-2-methoxyphenyl)-4-(3-(dimethylamino)-3-methylazetidin-1-yl)-6-fluoro-8-(trifluoromethyl)-1H-[1, 2, 3]triazolo[4, 5-c]quinolin-1-yl)-2-(cyanomethyl)piperidine-1-carboxylate

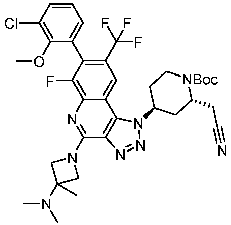

This compound was prepared according to the procedure described in **Example 233a and Example 233b, step 4,** replacing (5-fluoroquinolin-8-yl)boronic acid with (3-chloro-2-methoxyphenyl)boronic acid. LCMS calculated for C₃₅H₄₀ClF₄N₈O₃ (M+H)⁺: m/z = 731.3; found: 731.4.

### Step 2. 2-((2S,4S)-1-(but-2-ynoyl)-4-(7-(3-chloro-2-methoxyphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile

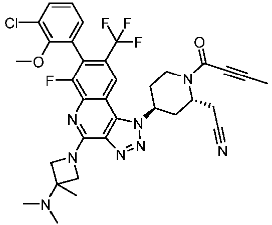

This compound was prepared according to the procedure described in **Example 231a and Example 231b, step 5,** replacing *tert*-butyl (2S,4S)-4-(7-(6-chloro-5-methyl-1-(tetrahydro-2H-pyran-2-yl)-1*H*-indazol-4-yl)-8-(2-cyanoethyl)-4-(3-(dimethylamino)azetidin-1-yl)-6-fluoro-1*H*-[1,2,3]triazolo[4,5-*c*]quinolin-1-yl)-2-(cyanomethyl)piperidine-1-carboxylate with tert-butyl (2*S*,4*S*)-4-(7-(3-chloro-2-methoxyphenyl)-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl) piperidine-1-carboxylate.

**Example 243a.** Diastereomer 1. Peak 1. LCMS calculated for C₃₄H₃₄ClF₄N₈O₂ (M+H)⁺ m/z = 697.2; found 697.2.

**Example 243b.** Diastereomer 2. Peak 2. LCMS calculated for C₃₄H₃₄ClF₄N₈O₂ (M+H)⁺ m/z = 697.2; found 697.2.

### Example 244. 2-((2S,4S)-4-(7-(3-chloro-2-methoxyphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile

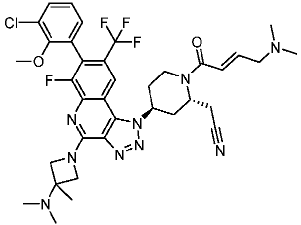

This compound was prepared according to the procedure described in **Example 232,** replacing *tert*-butyl (2*S*,4*S*)-4-(7-(6-chloro-5-methyl-1-(tetrahydro-2H-pyran-2-yl)-1*H*-indazol-4-yl)-8-(2-cyanoethyl)-4-(3-(dimethylamino)azetidin-1-yl)-6-fluoro-1*H*-[1,2,3]triazolo[4,5-c]quinolin-1-yl)-2-(cyanomethyl)piperidine-1-carboxylate with *tert*-butyl (2S,4S)-4-(7-(3-chloro-2-methoxyphenyl)-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl)piperidine-1-carboxylate. LCMS calculated for C₃₆H₄₁ClF₄N₉O₂ (M+H)⁺ m/z = 742.3; found 742.2.

### Example 245a and Example 245b. 2-((25,45)-1-(but-2-ynoyl)-4-(4-(3-(dimethylamino)-3-methylazetidin-1-yl)-6-fluoro-7-(2-methoxy-3-methylphenyl)-8-(trifluoromethyl)-1H-[1,2,3]triazolo[4,5-c]quinolin-1-yl)piperidin-2-yl)acetonitrile

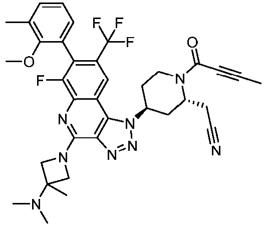

### Step 1. tert-butyl (2S,4S)-2-(cyanomethyl)-4-(4-(3-(dimethylamino)-3-methylazetidin-1-yl)-6-fluoro-7-(2-methoxy-3-methylphenyl)-8-(trifluoromethyl)-1H-[1,2,3]triazolo[4,5-c]quinolin-1-yl)piperidine-1-carboxylate

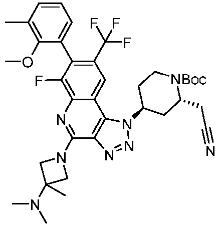

This compound was prepared according to the procedure described in **Example 233a and Example 233b, step 4,** replacing (5-fluoroquinolin-8-yl)boronic acid with (2-methoxy-3-methylphenyl)boronic acid. LCMS calculated for C₃₃H₄₃F₄N₈O₃ (M+H)⁺: m/z = 711.3; found: 711.5.

### Step 2. 2-((2S,4S)-1-(but-2-ynoyl)-4-(4-(3-(dimethylamino)-3-methylazetidin-1-yl)-6-fluoro-7-(2-methoxy-3-methylphenyl)-8-(trifluoromethyl)-1H-[1,2,3]triazolo[4,5-c]quinolin-1-yl)piperidin-2-yl)acetonitrile

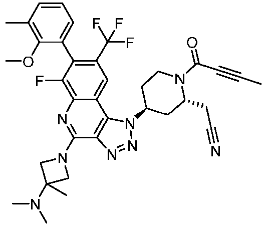

This compound was prepared according to the procedure described in **Example 231a and Example 231b, step 5,** replacing tert-butyl (2*S*,4*S*)-4-(7-(6-chloro-5-methyl-1-(tetrahydro-2H-pyran-2-yl)-1*H*-indazol-4-yl)-8-(2-cyanoethyl)-4-(3-(dimethylamino)azetidin-1-yl)-6-fluoro-1*H*-[1,2,3]triazolo[4,5-*c*]quinolin-1-yl)-2-(cyanomethyl)piperidine-1-carboxylate with *tert*-butyl (2S,4S)-4-(7-(2-methoxy-3-methylphenyl)-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl) piperidine-1-carboxylate.

**Example 245a.** Diastereomer 1. Peak 1. LCMS calculated for C₃₅H₃₇F₄N₈O₂ (M+H)⁺ m/z = 677.3; found 677.4.

**Example 245b.** Diastereomer 2. Peak 2. LCMS calculated for C₃₅H₃₇F₄N₈O₂ (M+H)⁺ m/z = 677.3; found 677.4.

### Example 246. 2-((2S,4S)-4-(7-(2-methoxy-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile

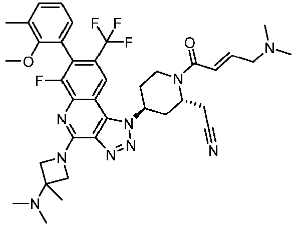

This compound was prepared according to the procedure described in **Example 232,** replacing *tert*-butyl (2*S*,4*S*)-4-(7-(6-chloro-5-methyl-1-(tetrahydro-2H-pyran-2-yl)-1*H*-indazol-4-yl)-8-(2-cyanoethyl)-4-(3-(dimethylamino)azetidin-1-yl)-6-fluoro-1*H*-[1,2,3]triazolo[4,5-*c*]quinolin-1-yl)-2-(cyanomethyl)piperidine-1-carboxylate with *tert*-butyl (2S,4S)-4-(7-(2-methoxy-3-methylphenyl)-4-(3-(dimethylamino)-3-methylazetidin-1-yl)-6-fluoro-8-(trifluoromethyl)-1*H*-[1,2,3]triazolo[4,5-*c*]quinolin-1-yl)-2-(cyanomethyl)piperidine-1-carboxylate. LCMS calculated for C₃₇H₄₄F₄N₉O₂ (M+H)⁺ m/z = 722.4; found 722.5.

### Example 204. 3-(1-((2S,4S)-1-(but-2-ynoyl)-2-(cyanomethyl)piperidin-4-yl)-7-(3-chloro-2-methoxyphenyl)-6-fluoro-4-(((S)-1-methylpyrrolidin-2-yl)methoxy)-1H-[1,2,3]triazolo[4,5-c]quinolin-8-yl)propanenitrile

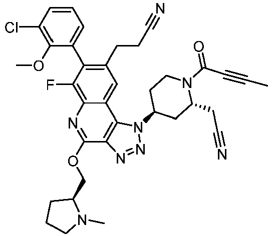

This compound was prepared according to the procedure described in **Example 242a and Example 242b, step 1-2,** replacing (2-chloro-3-methylphenyl)boronic acid with (3-chloro-2-methoxyphenyl)boronic acid.

**Example 204a.** Diastereomer 1. Peak 1. LCMS calculated for C₃₃H₃₇ClFN₈O₃ (M+H)⁺ m/z = 683.3; found 683.5.

**Example 204b.** Diastereomer 2. Peak 2. LCMS calculated for C₃₃H₃₇ClFN₈O₃ (M+H)⁺ m/z = 683.3; found 683.5.

### Example 247. 2-((2S,4S)-1-acryloyl-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile

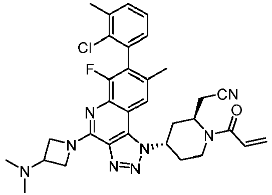

### Step 1. tert-butyl (2S,4S)-4-((7-bromo-2-chloro-8-fluoro-6-iodo-3-nitroquinolin-4-yl)amino)-2-(2-(tert-butoxy)-2-oxoethyl)piperidine-1-carboxylate

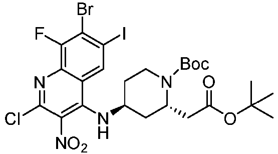

This compound was prepared according to the procedure described in **Example 80a and Example 80b, step 1,** replacing 7-bromo-2,4,6-trichloro-8-fluoro-3-nitroquinoline with 7-bromo-2,4-dichloro-8-fluoro-6-iodo-3-nitroquinoline. LCMS calculated for C₂₅H₃₁BrClFlN₄O₆ (M+H)⁺: m/z = 743.1; found: 743.1.

### Step 2. tert-butyl (2S,4S)-4-((3-amino-7-bromo-2-chloro-8-fluoro-6-iodoquinolin-4-yl)amino)-2-(2-(tert-butoxy)-2-oxoethyl)piperidine-1-carboxylate

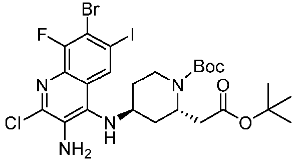

This compound was prepared according to the procedure described in **Example 80a and Example 80b, step 2,** replacing tert-butyl (2S,4S)-4-((3-amino-7-bromo-2,6-dichloro-8-fluoroquinolin-4-yl)amino)-2-(2-(tert-butoxy)-2-oxoethyl)piperidine-1-carboxylate with *tert-butyl (2S,4S)-4-((7-bromo-2-chloro-8-fluoro-6-iodo-3-nitroquinolin-4-yl)amino)-2-(2-(tert-butoxy)-2-oxoethyl)piperidine-1-carboxylate*. LCMS calculated for C₂₅H₃₃BrClFIN₄O₄ (M+H)⁺: m/z = 713.1; found: 713.1.

### Step 3. tert-butyl (2S,4S)-4-(7-bromo-4-chloro-6-fluoro-8-iodo-1H-[1,2,3]triazolo[4,5-c]quinolin-1-yl)-2-(2-(tert-butoxy)-2-oxoethyl)piperidine-1-carboxylate

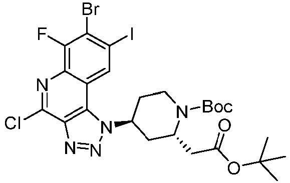

This compound was prepared according to the procedure described in **Example 98, step 3,** replacing *tert*-butyl (2*S*,4*S*)-4-((3-amino-7-bromo-2-chloro-8-fluoro-6-iodoquinolin-4-yl)amino)-2-(cyanomethyl)piperidine-1-carboxylate with tert-butyl (2S,4S)-4-((3-amino-7-bromo-2-chloro-8-fluoro-6-iodoquinolin-4-yl)amino)-2-(2-(tert-butoxy)-2-oxoethyl)piperidine-1-carboxylate. LCMS calculated for C₂₅H₃₀BrClFN₅O₄ (M+H)⁺: m/z = 724.0; found: 724.1.

### Step 4. tert-butyl (2S,4S)-4-(7-bromo-6-fluoro-8-iodo-4-(methylthio)-1H-[1,2,3]triazolo[4,5-c]quinolin-1-yl)-2-(2-(tert-butoxy)-2-oxoethyl)piperidine-1-carboxylate

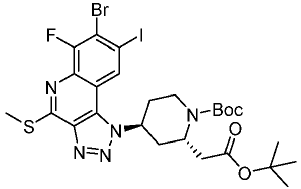

This compound was prepared according to the procedure described in **Example 98, Step 4,** replacing *tert*-butyl (2S,4S)-4-(7-bromo-4-chloro-6-fluoro-8-iodo-1*H-*[1,2,3]triazolo[4,5-*c*]quinolin-1-yl)-2-(cyanomethyl)piperidine-1-carboxylate with tert-butyl (2S,4S)-4-(7-bromo-4-chloro-6-fluoro-8-iodo-1H-[1,2,3]triazolo[4,5-c]quinolin-1-yl)-2-(2-(tert-butoxy)-2-oxoethyl)piperidine-1-carboxylate . LCMS calculated for C₂₆H₃₃BrFIN₅O₄S (M+H)⁺: m/z = 736.0; found: 736.1.

### Step 5. 2-((2S,4S)-4-(7-bromo-6-fluoro-8-iodo-4-(methylthio)-1H-[1,2,3]triazolo[4,5-c]quinolin-1-yl)piperidin-2-yl)acetic acid

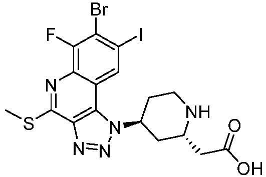

This compound was prepared according to the procedure described in **Example 80a and Example 80b, Step 5,** replacing tert-butyl (2S,4S)-4-(7-bromo-8-chloro-6-fluoro-4-(methylthio)-1H-imidazo[4,5-c]quinolin-1-yl)-2-(2-(tert-butoxy)-2-oxoethyl)piperidine-1-carboxylate with tert-butyl (2S,4S)-4-(7-bromo-6-fluoro-8-iodo-4-(methylthio)-1H-[1,2,3]triazolo[4,5-c]quinolin-1-yl)-2-(2-(tert-butoxy)-2-oxoethyl)piperidine-1-carboxylate. LCMS calculated for C₁₇H₁₇BrFIN₅O₂S (M+H)⁺: m/z = 580.0; found: 580.1.

### Step 6. 2-((2S,4S)-4-(7-bromo-6-fluoro-8-iodo-4-(methylthio)-1H-[1,2,3]triazolo[4,5-c]quinolin-1-yl)-1-(tert-butoxycarbonyl)piperidin-2-yl)acetic acid

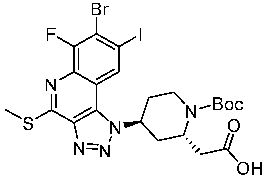

This compound was prepared according to the procedure described in **Example 80a and Example 80b, Step 6,** replacing 2-((2S,4S)-4-(7-bromo-8-chloro-6-fluoro-4-(methylthio)-1H-imidazo[4,5-c]quinolin-1-yl)-1-(tert-butoxycarbonyl)piperidin-2-yl)acetic acid with 2-((2S,4S)-4-(7-bromo-6-fluoro-8-iodo-4-(methylthio)-1H-[1,2,3]triazolo[4,5-c]quinolin-1-yl)piperidin-2-yl)acetic acid. LCMS calculated for C₂₂H₂₅BrFIN₅O₄S (M+H)⁺: m/z = 680.0; found: 680.1.

### Step 7. tert-butyl (2S,4S)-2-(2-amino-2-oxoethyl)-4-(7-bromo-6-fluoro-8-iodo-4-(methylthio)-1H-[1,2,3]triazolo[4,5-c]quinolin-1-yl)piperidine-1-carboxylate

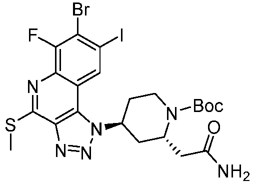

This compound was prepared according to the procedure described in **Example 80a and Example 80b, Step 7,** replacing 2-((2S,4S)-4-(7-bromo-8-chloro-6-fluoro-4-(methylthio)-1H-imidazo[4,5-c]quinolin-1-yl)-1-(tert-butoxycarbonyl)piperidin-2-yl)acetic acid with *tert-butyl (2S,4S)-4-(7-bromo-6-fluoro-8-iodo-4-(methylthio)-1H-[1,2,3]triazolo[4,5-c]quinolin-1-yl)-2-(cyanomethyl)piperidine-1-carboxylate.* LCMS calculated for C₂₂H₂₆BrFIN₆O₃S (M+H)⁺: m/z = 679.0; found: 679.1.

### Step 8. tert-butyl (2S,4S)-4-(7-bromo-6-fluoro-8-iodo-4-(methylthio)-1H-[1,2,3]triazolo[4,5-c]quinolin-1-yl)-2-(cyanomethyl)piperidine-1-carboxylate

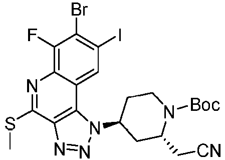

This compound was prepared according to the procedure described in **Example 80a and Example 80b, Step 8,** replacing tert-butyl (2S,4S)-2-(2-amino-2-oxoethyl)-4-(7-bromo-8-chloro-6-fluoro-4-(methylthio)-1H-imidazo[4,5-c]quinolin-1-yl)piperidine-1-carboxylate with tert-butyl (2S,4S)-2-(2-amino-2-oxoethyl)-4-(7-bromo-6-fluoro-8-iodo-4-(methylthio)-1H-[1,2,3]triazolo[4,5-c]quinolin-1-yl)piperidine-1-carboxylate. LCMS calculated for C₂₂H₂₄BrFIN₆O₂S (M+H)⁺: m/z = 661.0; found: 661.1.

### Step 9. tert-butyl (2S,4S)-4-(7-bromo-6-fluoro-8-methyl-4-(methylthio)-1H-[1,2,3]triazolo[4,5-c]quinolin-1-yl)-2-(cyanomethyl)piperidine-1-carboxylate

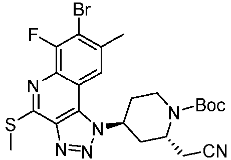

This compound was prepared according to the procedure described in **Example 98, Step 5,** replacing *tert*-butyl (2S,4S)-4-(7-bromo-4-chloro-6-fluoro-8-iodo-1*H-*[1,2,3]triazolo[4,5-*c*]quinolin-1-yl)-2-(cyanomethyl)piperidine-1-carboxylate with tert-butyl (2S,4S)-4-(7-bromo-4-chloro-6-fluoro-8-iodo-1H-[1,2,3]triazolo[4,5-c]quinolin-1-yl)-2-(2-(tert-butoxy)-2-oxoethyl)piperidine-1-carboxylate. LCMS calculated for C₂₃H₂₇BrFN₆O₂S (M+H)⁺: m/z = 549.1; found: 549.1.

### Step 10. tert-butyl (2S,4S)-4-(7-(2-chloro-3-methylphenyl)-6-fluoro-8-methyl-4-(methylthio)-1H-[1,2,3]triazolo[4,5-c]quinolin-1-yl)-2-(cyanomethyl)piperidine-1-carboxylate

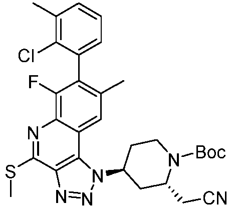

This compound was prepared according to the procedure described in **Example 91, Step 4,** replacing *tert*-butyl (2*S*,4*S*)-4-(7-bromo-8-chloro-6-fluoro-4-(methylthio)-1*H-*[1,2,3]triazolo[4,5-*c*]quinolin-1-yl)-2-(cyanomethyl)piperidine-1-carboxylate with *tert*-butyl (2*S*,4*S*)-4-(7-bromo-6-fluoro-8-methyl-4-(methylthio)-1*H*-[1,2,3]triazolo[4,5-*c*]quinolin-1-yl)-2-(cyanomethyl)piperidine-1-carboxylate. LCMS calculated for C₃₀H₃₃ClFN₆O₂S (M+H)⁺ m/z = 595.2; found 595.0.

### Step 11. tert-butyl (2S, 4S)-4-(7-(2-chloro-3-methylphenyl)-4-(3-(dimethylamino)azetidin-1-yl)-6-fluoro-8-methyl-1H-[1,2,3]triazolo[4, 5-c]quinolin-1-yl)-2-(cyanomethyl) piperidine-1-carboxylate

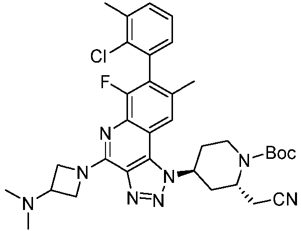

This compound was prepared according to the procedure described in **Example 80a and Example 80b, Step 10,** replacing tert-butyl (2*S*,4*S*)-4-(8-chloro-7-(6-chloro-5-methyl-1-(tetrahydro-2H-pyran-2-yl)-1*H*-indazol-4-yl)-6-fluoro-4-(methylthio)-1*H*-imidazo[4,5-c]quinolin-1-yl)-2-(cyanomethyl)piperidine-1-carboxylate with tert-butyl (2S,4S)-4-(7-(2-chloro-3-methylphenyl)-6-fluoro-8-methyl-4-(methylthio)-1H-[1,2,3]triazolo[4,5-c]quinolin-1-yl)-2-(cyanomethyl)piperidine-1-carboxylate and *N,N*-dimethylazetidin-3-amine dihydrochloride. LCMS for C₃₄H₄₁ClFN₈O₂ (M+H)⁺ m/z = 647.3; found 647.2.

### Step 12. 2-((2S, 4S)-1-acryloyl-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile

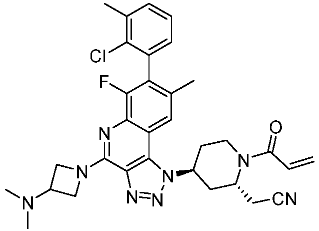

This compound was prepared according to the procedure described in **Example 85, Step 12,** replacing *tert*-butyl (2*S*,4*S*)-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-*c*]quinolin-1-yl)-2-(cyanomethyl)-piperidine-1-carboxylate with tert-butyl (2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-2-(cyanomethyl)piperidine-1-carboxylate . The product was isolated as a mixture of diastereomers. LCMS calculated for C₃₂H₃₅ClFN₈O (M+H)⁺ m/z = 601.3; found 601.2.

### Example 248. 2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-1-((E)-4-fluorobut-2-enoyl)piperidin-2-yl)acetonitrile

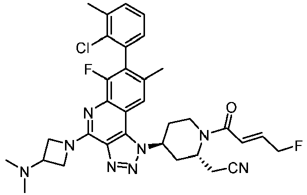

This compound was prepared according to the procedure described in **Example 247, Step 1** - **11,** to give tert-butyl (2S,4S)-4-(7-(2-chloro-3-methylphenyl)-4-(3-(dimethylamino)-azetidin-1-yl)-6-fluoro-8-methyl-1H-[1,2,3]triazolo[4,5-c]quinolin-1-yl)-2-(cyanomethyl)-piperidine-1-carboxylate.

### Step 1. tert-butyl (2S,4S)-4-(7-(2-chloro-3-methylphenyl)-4-(3-(dimethylamino)azetidin-1-yl)-6-fluoro-8-methyl-1H-[1,2,3]triazolo[4, 5-c]quinolin-1-yl)-2-(cyanomethyl) piperidine-1-carboxylate

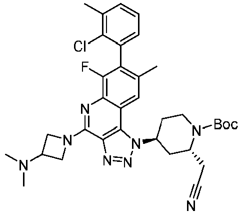

This compound was prepared according to the procedure described in **Example 247, Step 1 - 11,** to give tert-butyl (2S,4S)-4-(7-(2-chloro-3-methylphenyl)-4-(3-(dimethylamino)-azetidin-1-yl)-6-fluoro-8-methyl-1H-[1,2,3]triazolo[4,5-c]quinolin-1-yl)-2-(cyanomethyl)-piperidine-1-carboxylate. LC/MS calculated for C₃₄H₄₁ClFN₈O₂ (M+H)⁺ m/z = 647.3; found 647.2

### Step 2. 2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile

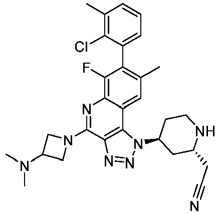

A solution of tert-butyl (2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-2-(cyanomethyl)piperidine-1-carboxylate(27.0 mg, 0.047 mmol) in CH₂Cl₂ (2.0 mL) and TFA (1.0 mL) was stirred at room temperature for 30 minutes before concentrating to dryness to give 2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile. LCMS calculated for C₂₉H₃₃ClFN₈ (M+H)⁺ m/z = 547.3; found 547.2.

### Step 3. 2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-1-((E)-4-fluorobut-2-enoyl)piperidin-2-yl)acetonitrile

The concentrated residue from Step 2 (10.0 mg, 0.018 mmol) was redissolved in EtOAc (1.0 mL) and (E)-4-fluorobut-2-enoic acid (5.71 mg, 0.055 mmol) and 1-Propanephosphonic acid cyclic anhydride (T3P 50% in EtOAc) (16.32 µl, 0.055 mmol) in Ethyl acetate (0.8 ml) was cooled at 0 °C followed by the addition of triethylamine (12.74 µl, 0.091 mmol). The reaction was warmed to room temperature and stirred for 30 min. Upon completion, the reaction was diluted with acetonitrile and purified using prep-LCMS (XBridge C18 column, eluting with a gradient of acetonitrile/water containing 0.1% TFA, at flow rate of 60 mL/min) to afford the desired product. The product was isolated as a mixture of diastereomers. LCMS calculated for C₃₃H₃₆ClF₂N₈O (M+H)⁺ m/z = 633.3; found 633.2. ¹H NMR (500 MHz, DMSO-*d*₆) δ 8.04 (s, 1H), 7.50 (d, *J* = 7.6 Hz, 1H), 7.42 (td, *J* = 7.6, 1.4 Hz, 1H), 7.23 (dt, *J* = 7.7, 2.3 Hz, 1H), 6.90 - 6.76 (m, 2H), 5.80 (m, 1H), 5.34 - 5.24 (m, 1H), 5.22 (d, *J* = 2.0 Hz, 1H), 5.12 (d, *J =* 3.3 Hz, 1H), 4.98 (s, 1H), 4.71 (d, *J =* 47.9 Hz, 3H), 4.34 (m, 3H), 3.62 (d, *J* = 14.1 Hz,3H), 3.45 - 3.17 (m, 3H), 2.90 (s, 6H), 2.46 (s, 3H), 2.24 (d, *J* = 3.3 Hz, 3H).

### Example 249. 2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile

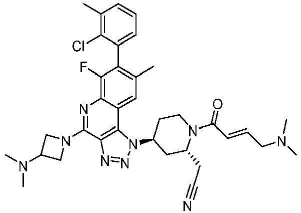

### Step 1. 2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile

This compound was prepared according to the procedure described in **Example 248, Step 3,** replacing (E)-4-fluorobut-2-enoic acid with (E)-4-(dimethylamino)but-2-enoic acid

HCl and DIPEA. The product was isolated as a mixture of diastereomers. LCMS calculated for C₃₅H₄₂ClFN₉O (M+H)⁺ m/z = 658.3; found 658.2.

### Example 251. 2-((2S,4S)-1-acryloyl-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile

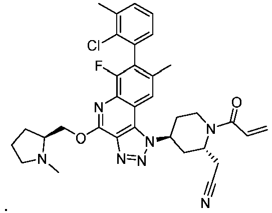

### Step 1. tert-butyl (2S,4S)-4-(7-(2-chloro-3-methylphenyl)-6-fluoro-8-methyl-4-(((S)-1-methylpyrrolidin-2-yl)methoxy)-1H-[1, 2, 3]triazolo[4, 5-c]quinolin-1-yl)-2-(cyanomethyl)piperidine-1-carboxylate

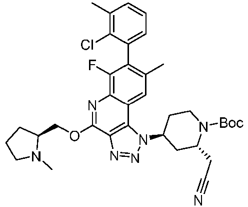

To a solution of tert-butyl (2S,4S)-4-(8-chloro-7-(4-chloro-3-methylphenyl)-6-fluoro-4-(methylsulfonyl)-1H-[1,2,3]triazolo[4,5-c]quinolin-1-yl)-2-(cyanomethyl)piperidine-1-carboxylate (134.7 mg, 0.226 mmol) from **Example 247, Step 10** in CH₂Cl₂ (1.0 mL) was cooled to 0 °C. *m*-CPBA (78 mg, 0.453 mmol) was added in one portion and the mixture was stirred at 0 °C for an additional 30 minutes. The mixture was then diluted with sat'd NaHCO₃ and extracted with CH₂Cl₂. The combined organic layers were dried over MgSO₄, filtered, and concentrated to dryness.

To the concentrated residue (47 mg, 0.075 mmoles), (S)-(1-methylpyrrolidin-2-yl)methanol (17.34 mg, 0.151 mmol), Sodium tert-butoxide (18.08 mg, 0.188 mmol) and THF (3.0 ml) were added. The mixture was stirred at room temperature for 1 hr. The reaction was quenched with water and extracted with EtOAc. EtOac extracts were washed with saturated NaHCO3 and brine and dried down and used as such in the next step. LCMS calculated for C₃₅H₄₂ClFN₇O₃ (M+H)⁺ m/z = 662.2; found 662.2.

### Step 2. 2-((2S,4S)-1-acryloyl-4-(7-(2-chloro-3-methylphenyl)-6-fluoro-8-methyl-4-(((S)-1-methylpyrrolidin-2- yl)methoxy)-1H-[1,2,3]triazolo[4,5-c]quinolin-1- yl)piperidin-2-yl)acetonitrile

This compound was prepared according to the procedure described in **Example 85, Step 12,** replacing tert-butyl (2S,4S)-4-(7-(2-chloro-3-methylphenyl)-4-(3-(dimethylamino)-azetidin-1-yl)-6-fluoro-8-methyl-1H-[1,2,3]triazolo[4,5-c]quinolin-1-yl)-2-(cyanomethyl)-piperidine-1- carboxylate with tert-butyl (2S,4S)-4-(7-(2-chloro-3-methylphenyl)-6-fluoro-8-methyl-4-(((S)-1-methylpyrrolidin-2-yl)methoxy)-1H-[1 ,2,3]triazolo[4,5-c]quinolin-1-yl)-2-(cyanomethyl)piperidine-1-carboxylate. The product was isolated as a mixture of diastereomers. LCMS calculated for C₃₃H₃₆ClFN₇O₂ (M+H)⁺ m/z = 616.2; found 616.2.

### Example 253. 2-((2S,4S)-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)-1-((E)-4-fluorobut-2-enoyl)piperidin-2-yl)acetonitrile

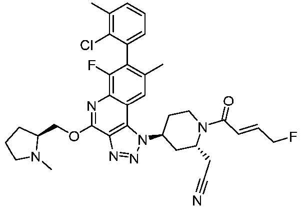

### Step 1. 2-((2S,4S)-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)-1-((E)-4-fluorobut-2-enoyl) piperidin-2-yl)acetonitrile

This compound was prepared from **Example 251, Step 1** according to the procedure described in **Example 248, Step 2-3** to give the desired product as a mixture of diastereomers, LCMS calculated for C₃₄H₃₇ClF2N₇O₂ (M+H)⁺ m/z = 648.2; found 648.2.

### Example 254. 2-((2S,4S)-1-(but-2-ynoyl)-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile

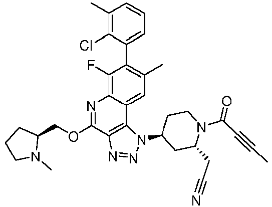

### Step 1. 2-((2S,4S)-1-(but-2-ynoyl)-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile

This compound was prepared from **Example 251, Step 1** according to the procedure described in **Example 248, Step 2 and 3,** replacing (E)-4-fluorobut-2-enoic acid with but-2-ynoic acid to give the desired product as a mixture of diastereomers, LCMS calculated for C₃₄H₃₆ClFN₇O₂ (M+H)⁺ m/z = 628.2; found 628.2.

### Example 255. 2-((2S,4S)-1-acryloyl-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)piperidin-2- yl)acetonitrile

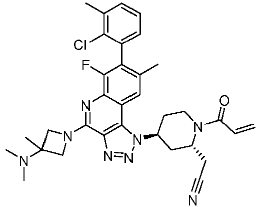

### Step 1. tert-butyl (2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-2-(cyanomethyl)piperidine-1-carboxylate

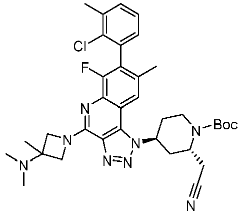

This compound was prepared according to the procedure described in **Example 247, Step 11,** replacing *N,N*-dimethylazetidin-3-amine dihydrochloride with *N*,*N*-3-triimethylazetidin-3-amine hydrochloride. LCMS for C₃₅H₄₃ClFN₈O₂ (M+H)⁺ m/z = 661.2; found 661.2.

### Step 2. 2-((2S,4S)-1-acryloyl-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile

This compound was prepared from **Example 255, Step 1** according to the procedure described in **Example 247, Step 12,.** The product was isolated as a mixture of diastereomers. LCMS calculated for C₃₃H₃₇ClFN₈O (M+H)⁺ m/z = 615.3; found 615.2.

### Example 256. 2-((2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-fluorobut-2-enoyl)piperidin-2-yl)acetonitrile

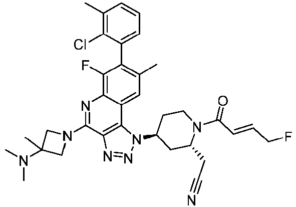

### Step 1. 2-((2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-fluorobut-2-enoyl)piperidin-2-yl)acetonitrile

This compound was prepared from **Example 255, Step 1** according to the procedure described in **Example 248, Steps 2-3.** The product was isolated as a mixture of diastereomers. LCMS calculated for C₃₄H₃₈ClF₂N₈O (M+H)⁺ m/z = 647.2; found 647.2.

### Example 257. 2-((2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile

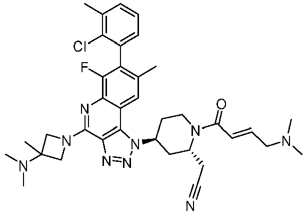

### Step 1. 2-((2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-(dimethylamino)but-2-enoyl) piperidin-2-yl)acetonitrile

This compound was prepared from **Example 255, Step 1** according to the procedure described in **Example 87a and Example 87b** replacing but-2-ynoic acid with 4-(dimethylamino)but-2-ynoic acid hydrochloride. The product was isolated as a mixture of diastereomers. LCMS calculated for C₃₆H₄₄ClFN₉O (M+H)⁺ m/z = 672.2; found 672.2.

### Example 259. 2-((2S,4S)-1-acryloyl-4-(7-(2-chloro-3-methylphenyl)-6-fluoro-8-methyl-4-(3-oxomorpholino)-1H-[1,2,3]tri azolo[4,5-c]quinolin-1-yl)piperidin-2-yl)acetonitrile

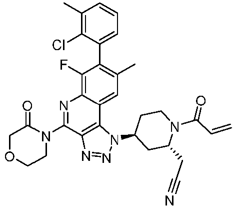

### Step 1. tert-butyl (2S,4S)-4-(7-(2-chloro-3-methylphenyl)-6-fluoro-8-methyl-4-(3-oxomorpholino)-1H-[1, 2, 3]triazolo[4, 5-c]quinolin-1-yl)-2-(cyanomethyl)piperidine-1-carboxylate

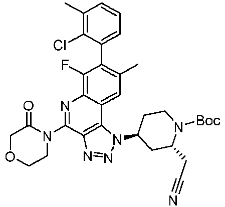

To a solution of tert-butyl (2S,4S)-4-(8-chloro-7-(4-chloro-3-methylphenyl)-6-fluoro-4-(methylsulfonyl)-1H-[1,2,3]triazolo[4,5-c]quinolin-1-yl)-2-(cyanomethyl)piperidine-1-carboxylate (134.7 mg, 0.226 mmol) from **Example 247, Step 10** in CH₂Cl₂ (1.0 mL) was cooled to 0 °C. *m*-CPBA (78 mg, 0.453 mmol) was added in one portion and the mixture was stirred at 0 °C for an additional 30 minutes. The mixture was then diluted with sat'd NaHCO₃ and extracted with CH₂Cl₂. The combined organic layers were dried over MgSO₄, filtered, and concentrated to dryness.

To the concentrated residue (30 mg, 0.048 mmoles), morpholin-3-one (9.67 mg mg, 0.096 mmol), Sodium tert-butoxide (4.6 mg, 0.048 mmol) and THF (2.0 ml)was added. The mixture was stirred at room temperature for 1 hr. The reaction was quenched with water and extracted with EtOAc. EtOac extracts were washed with saturated NaHCO₃ and brine and dried down and used as such in the next step. LCMS calculated for C₃₃H₃₆ClFN₇O₄ (M+H)⁺ m/z = 648.2; found 648.2

### Step 2. 2-((2S,4S)-1-acryloyl-4-(7-(2-chloro-3-methylphenyl)-6-fluoro-8-methyl-4-(3-oxomorpholino)-1H-[1,2,3]tri azolo[4,5-c]quinolin-1-yl)piperidin-2-yl)acetonitrile

This compound was prepared from **Example 259, Step 1** according to the procedure described in **Example 247, Step 12.** The product was isolated as a mixture of diastereomers. LCMS calculated for C₃₁H₃₀ClFN₇O₃. (M+H)⁺ m/z = 602.2; found 602.2.

### Example 260. 2-((2S,4S)-1-(but-2-ynoyl)-4-(7-(2-chloro-3-methylphenyl)-6-fluoro-8-methyl-4-(3-oxomorpholino)-1H-[1,2,3]triazolo[4,5-c]quinolin-1-yl)piperidin-2-yl)acetonitrile

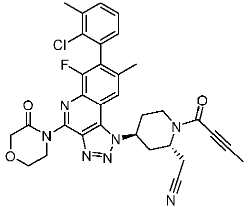

### Step 1. 2-((2S,4S)-1-(but-2-ynoyl)-4-(7-(2-chloro-3-methylphenyl)-6-fluoro-8-methyl-4-(3-oxomorpholino)-1H-[1,2,3]triazolo[4,5-c]quinolin-1-yl)piperidin-2-yl)acetonitrile

This compound was prepared from **Example 259, Step 1** according to the procedure described in **Example 254, Step 2,.** The product was isolated as a mixture of diastereomers. LCMS calculated for C₃₂H₃₀ClFN₇O₃. (M+H)⁺ m/z = 614.2; found 614.2.

### Example 262. 2-((2S,4S)-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)-1-((E)-4-(dimethylamino)but-2-enoyl)piperidin-2-yl)acetonitrile

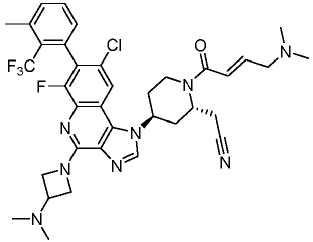

### Step 1. tert-butyl (R)-6-cyano-5-hydroxy-3-oxohexanoate

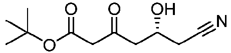

To a solution of 2.0 M LDA (100 ml, 200 mmol) in anhydrous THF (223 ml) was cooled to -78°C for 1 h, and then tert-butyl acetate (26.9 ml, 200 mmol) was added dropwise with stirring over 20 min. After an additional 40 minutes maintained at -78°C, a solution of ethyl (R)-4-cyano-3-hydroxybutanoate (10.5 g, 66.8 mmol) was added dropwise. The mixture was allowed to stir at -40°C for 4 h, and then an appropriate amount of HCl (2 M) was added to the mixture, keeping pH ~6. During this quench, the temperature of the mixture was maintained at -10°C. Upon completion, the temperature of the mixture was cooled to 0°C. The mixture was extracted with ethyl acetate (3 × 100 mL). The combined organic layer was washed with NaHCO₃ (100 mL) and brine (100 mL), dried over anhydrous Na₂SO₄, and evaporated to provide the material as yellow oil (15.0 g, 99% yield).

### Step 2. tert-butyl (2S,4R)-2-(2-(tert-butoxy)-2-oxoethyl)-4-hydroxypiperidine-1-carboxylate

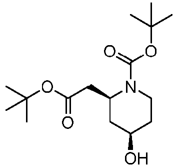

A solution of tert-butyl (R)-6-cyano-5-hydroxy-3-oxohexanoate (15.0 g, 66.0 mmol) in acetic acid (110 ml) was treated with platinum (IV) oxide hydrate (0.868 g, 3.30 mmol). The Parr bottle was evacuated and backfilled with H₂ three times and stirred under a H₂ atmosphere (45 psi, recharged 4 times) at 22 °C for 3h. The mixture was filtered through Celite and the filter cake was washed with EtOH. The filtrate was concentrated to yield product with a ~9:1 cis:trans diastereomer ratio.

The residue was dissolved in methanol (100 mL) then Boc-anhydride (15.32 ml, 66.0 mmol), sodium carbonate (13.99 g, 132 mmol) was added. The reaction mixture was stirred at room temperature overnight. The mixture was filtered and concentrated. The residue was purified with silica gel column to give the desired product (11.7 g, 56%). LCMS calculated for C₁₆H₂₉NNaO₅ (M+Na)⁺: m/z = 338.2; found: 338.2.

### Step 3. tert-butyl (2S,4S)-4-azido-2-(2-(tert-butoxy)-2-oxoethyl)piperidine-1-carboxylate

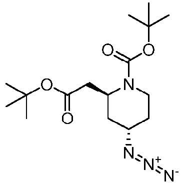

To a solution of tert-butyl (2S,4R)-2-(2-(tert-butoxy)-2-oxoethyl)-4-hydroxypiperidine-1-carboxylate (2.10 g, 6.66 mmol) in DCM (33 ml) at 0 °C was added Ms-Cl (0.67 mL, 8.66 mmol), After stirring for 1 h, The reaction was diluted with water and organic layer was separated and dried over Na₂SO₄, filtered and concentrated. The resulting residue was dissolved in DMF and sodium azide (1.3 g, 20 mmol) was added and the reaction mixture was heated at 70 °C for 5 h. After cooling to rt, the reaction was diluted with EtOAc and water. The organic layer was separated and dried over Na₂SO₄, filtered and concentrated. The residue was purified with silica gel column to give the desired product (1.90 g, 84%). LCMS calculated for (Product-Boc) C₁₁H₂₁N₄O₂ (M+H)⁺: m/z = 241.2; found: 241.2.

### Step 4. tert-butyl (2S,4S)-4-amino-2-(2-(tert-butoxy)-2-oxoethyl)piperidine-1-carboxylate

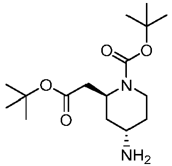

To a solution of tert-butyl (2S,4S)-4-azido-2-(2-(tert-butoxy)-2-oxoethyl)piperidine-1-carboxylate (1.9 g, 5.58 mmol) in methanol (27.9 ml) was added 10 % palladium on carbon (0.594 g, 0.558 mmol). The reaction mixture was evacuated under vacuum and refilled with H₂, stirred at rt for 2 h. The reaction mixture was filtered through a pad of Celite and washed with methanol. The filtrate was concentrated and used as is (1.5 g, 85%). LCMS calculated for C₁₆H₃₁N₂O₄ (M+H)⁺: m/z = 315.2; found: 315.2.

### Step 5. methyl 2-amino-3-fluoro-4-(4,4,5,5-tetramethyl-1,3,2-dioxaborolan-2-yl)benzoate

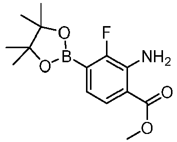

A mixture of methyl 2-amino-4-bromo-3-fluorobenzoate (349 mg, 1.407 mmol), bis(pinacolato)diboron (429 mg, 1.688 mmol), dichloro[1,1'-bis(diphenylphosphino)-ferrocene]palladium (II) dichloromethane adduct (115 mg, 0.141 mmol) and acetic acid, potassium salt, anhydrous (304 mg, 3.10 mmol) was charged with nitrogen and stirred at 100 °C for 4 h. The mixture was filtered through a pad of Celite and washed with DCM. The filtrate was concentrated. The residue was purified by flash chromatography to give the desired product (0.40 g, 96%). LCMS calculated for C₁₄H₂₀BFNO₄ (M+H)⁺: m/z = 296.1; found: 296.1.

### Step 6. methyl 3-amino-2-fluoro-3'-methyl-2'-(trifluoromethyl)-[1,1'-biphenyl]-4-carboxylate

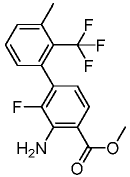

A mixture of 1-bromo-3-methyl-2-(trifluoromethyl)benzene (280 mg, 1.171 mmol), methyl 2-amino-3-fluoro-4-(4,4,5,5-tetramethyl-1,3,2-dioxaborolan-2-yl)benzoate (380 mg, 1.289 mmol), tetrakis (135 mg, 0.117 mmol) and sodium bicarbonate (197 mg, 2.343 mmol) in 1,4-dioxane (8.0 mL)/water (1.6 mL) was stirred at 90 °C for 6 h. The reaction mixture was diluted with ethyl acetate and water. The organic layer was separated and dried over Na₂SO₄, filtered and concentrated and used directly in the next step without further purification. LCMS calculated for C₁₆H₁₄F₄NO₂ (M+H)⁺: m/z = 328.1; found: 328.1.

### Step 7. methyl 3-amino-6-chloro-2-fluoro-3'-methyl-2'-(trifluoromethyl)-[1,1'-biphenyl]-4-carboxylate

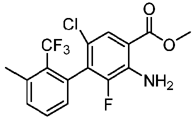

To a solution of methyl 3-amino-2-fluoro-3'-methyl-2'-(trifluoromethyl)-[1,1'-biphenyl]-4-carboxylate (380 mg, 1.161 mmol) in DMF (3.87 ml) was added NCS (171 mg, 1.277 mmol) at rt. The mixture was stirred at room temperature for 10 min. The reaction mixture was diluted with water and DCM. The organic layer was separated and dried over Na₂SO₄, filtered and concentrated.and used directly in the next step without further purification. LCMS calculated for C₁₆H₁₃ClF₄NO₂ (M+H)⁺: m/z = 362.1; found: 362.1.

### Step 8. methyl 6-chloro-3-(3-ethoxy-3-oxopropanamido)-2-fluoro-3'-methyl-2'-(trifluoromethyl)-[1,1'-biphenyl]-4-carboxylate

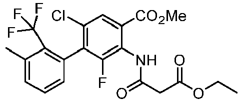

Ethyl 3-chloro-3-oxopropanoate (0.178 ml, 1.393 mmol) was added dropwise to a solution of methyl 3-amino-6-chloro-2-fluoro-3'-methyl-2'-(trifluoromethyl)-[1,1'-biphenyl]-4-carboxylate (0.420 g, 1.161 mmol) and TEA (0.194 ml, 1.393 mmol) in DCM (10 mL) at rt. The resulting mixture was stirred at rt for 4 h, The reaction was diluted with water and DCM. The organic layer was separated and dried over Na₂SO₄, filtered and concentrated. The residue was purified with flash chromatography to give the desired product (0.32 g, 58% over 3 steps). LCMS calculated for C₂₁H₁₉ClF₄NO₅ (M+H)⁺: m/z = 476.1; found: 476.1.

### Step 9. ethyl 2,4,6-trichloro-8-fluoro-7-(3-methyl-2-(trifluoromethyl)phenyl)quinoline-3-carboxylate

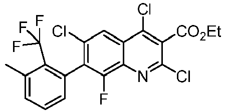

21 % sodium ethoxide (0.741 ml, 1.986 mmol) in EtOH was added dropwise to a solution of methyl 6-chloro-3-(3-ethoxy-3-oxopropanamido)-2-fluoro-3'-methyl-2'-(trifluoromethyl)-[1,1'-biphenyl]-4-carboxylate (0.315 g, 0.662 mmol) in EtOH (4 mL). Precipitates appeared during the addition process. The reaction was stirred at rt for 30 min. The solvent was removed under vacuum, and the crude product was used in next step without further purification.

The crude product from last step was dissolved in POCl₃ (1.24 mL, 13.3 mmol), and DIEA (0.23 ml, 1.33 mmol) was added. The resulting mixture was stirred at 100 °C for 2h. POCl₃ was removed by azeotrope with PhMe (3 times), and the residue was purified on silica gel column (EtOAc in hexanes, 0 ~ 20% gradient) to yield the product as white solid (184 mg, 58%). LCMS calculated for C₂₀H₁₃Cl₃F₄NO₂ (M+H)⁺: m/z = 480.0, 482.0; found: 480.0, 482.0.

### Step 10. ethyl 4-(((2S,4S)-1-(tert-butoxycarbonyl)-2-(cyanomethyl)piperidin-4-yl)amino)-2,6-dichloro-8-fluoro-7-(3-methyl-2-(trifluoromethyl)phenyl)quinoline-3-carboxylate

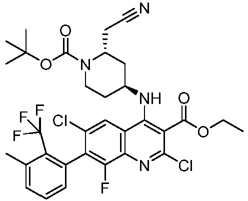

To a solution of ethyl 2,4,6-trichloro-8-fluoro-7-(3-methyl-2-(trifluoromethyl)phenyl)-quinoline-3-carboxylate (184 mg, 0.383 mmol) in DMF (2 ml) was added tert-butyl (2S,4S)-4-amino-2-(cyanomethyl)piperidine-1-carboxylate (110 mg, 0.459 mmol) and DIEA (134 µl, 0.766 mmol). The resulting mixture was stirred at 55 °C for 5h. After cooling to room temperature, ethyl acetate and water were added. The organic layer was washed with water (2x) and brine, dried over Na₂SO₄, filtered and concentrated. The residue was purified with flash chromatography (eluting with 0%-25% ethyl acetate in hexanes) to give the desired product as foam (230 mg, 88 %). LCMS calculated for C₃₂H₃₃Cl₂F₄N₄O₄ (M+H)⁺: m/z = 683.2; found: 683.2.

### Step 11. ethyl 4-(((2S,4S)-1-(tert-butoxycarbonyl)-2-(2-hydroxyethyl)piperidin-4-yl)amino)-2,6-dichloro-8-fluoro-7-(3-methyl-2-(trifluoromethyl) phenyl) quinoline-3-carboxylate

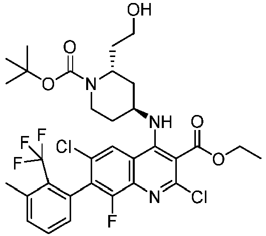

To a solution of ethyl 4-(((2S,4S)-1-(tert-butoxycarbonyl)-2-(cyanomethyl)piperidin-4-yl)amino)-2,6-dichloro-8-fluoro-7-(3-methyl-2-(trifluoromethyl)phenyl)quinoline-3-carboxylate (230 mg, 0.336 mmol) in toluene (1.7 ml) at -78 °C was added 1.0 M DIBAL-H in DCM (841 µl, 0.841 mmol). The resulting mixture was stirred at -78 °C for 40 min and warm to 0 °C for 1 h, quenched with methanol (0.5 ml). Aqueous Rochelle salt solution (prepared from 1.5 g of Rochelle salt and 10 mL of water) was added to the solution at ≤10 °C. The biphasic mixture was stirred vigorously for ≥1 h at rt and separated to give organic layer. The organic layer was washed with aqueous NaCl (×2), The organic layer was dried over Na₂SO₄, filtered and concentrated and used as is.

To a solution of crude from last step in ethanol (1.8 ml) at 0 °C was added sodium borohydride (6.94 mg, 0.184 mmol). The resulting mixture was stirred at 0 °C for 40 min and quenched with water and diluted with ethyl acetate. The organic layer was washed with water (2x) and brine, dried over Na₂SO₄, filtered and concentrated. The residue was purified with flash chromatography (eluting with 0%-25% ethyl acetate in hexanes) to give the desired product as foam (100 mg, 43 %). LCMS calculated for C₃₂H₃₆Cl₂F₄N₃O₅ (M+H)⁺: m/z = 688.2; found: 688.2.

### Step 12. ethyl 4-(((2S,4S)-1-(tert-butoxycarbonyl)-2-(2-((tertbutyldimethylsilyl)oxy)ethyl)piperidin-4-yl)amino)-2,6-dichloro-8-fluoro-7-(3-methyl-2-(trifluoromethyl)phenyl)quinoline-3-carboxylate

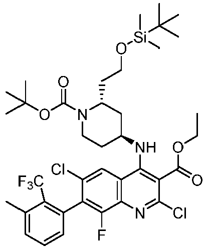

To a solution of ethyl 4-(((2S,4S)-1-(tert-butoxycarbonyl)-2-(2-hydroxyethyl)piperidin-4-yl)amino)-2,6-dichloro-8-fluoro-7-(3-methyl-2-(trifluoromethyl)phenyl)quinoline-3-carboxylate (101 mg, 0.147 mmol) in DMF (0.73 ml) was added imidazole (15.0 mg, 0.220 mmol) and TBS-Cl (28.7 mg, 0.191 mmol). The resulting mixture was stirred at 60 °C for 1 h 15 min. The reaction was diluted with EtOAc and water. The organic layer was washed with water and brine, dried over Na₂SO₄, filtered and concentrated. The residue was purified with flash chromatography (eluting with 0%-25% ethyl acetate in hexanes) to give the desired product as foam (110 mg, 93 %). LCMS calculated for C₃₈H₅₀Cl₂F₄N₃O₅Si (M+H)⁺: m/z = 802.3; found: 802.3.

### Step 13. tert-butyl (2S,4S)-2-(2-((tert-butyldimethylsilyl)oxy)ethyl)-4-((2,6-dichloro-8-fluoro-3-(hydroxymethyl)-7-(3-methyl-2-(trifluoromethyl)phenyl)quinolin-4-yl)amino)piperidine-1-carboxylate

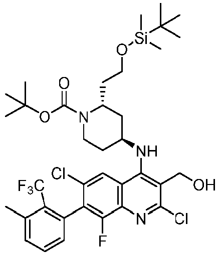

To a solution of ethyl 4-(((2S,4S)-1-(tert-butoxycarbonyl)-2-(2-((tertbutyldimethylsilyl)oxy)ethyl)piperidin-4-yl)amino)-2,6-dichloro-8-fluoro-7-(3-methyl-2-(trifluoromethyl)phenyl)quinoline-3-carboxylate (0.95 g, 1.183 mmol) in toluene (6.0 ml) at - 78 °C was added 1.0 M DIBAL-H in DCM (4.14 ml, 4.14 mmol). The resulting mixture was stirred at -78 °C for 40 min and warm to 0 °C for 1 h and 20 min, quenched with methanol (0.5 ml). Aqueous Rochelle salt solution (prepared from 4.8 g of Rochelle salt and 30 mL of water) was added to the solution at ≤10 °C. The biphasic mixture was stirred vigorously for ≥1 h and separated to give organic layer. The organic layer was washed with aqueous NaCl (×2). The organic layer was dried over Na₂SO₄, filtered and concentrated, and used as is. LCMS calculated for C₃₆H₄₈Cl₂F₄N₃O₄Si (M+H)⁺: m/z = 760.3; found: 760.3.

### Step 14. tert-butyl (2S,4S)-2-(2-((tert-butyldimethylsilyl)oxy)ethyl)-4-((2,6-dichloro-8-fluoro-3-formyl-7-(3-methyl-2-(trifluoromethyl)phenyl)quinolin-4-yl)amino)piperidine-1-carboxylate

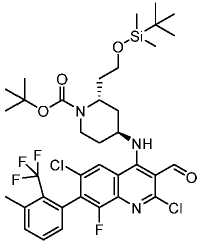

To a solution of tert-butyl (2S,4S)-2-(2-((tert-butyldimethylsilyl)oxy)ethyl)-4-((2,6-dichloro-8-fluoro-3-(hydroxymethyl)-7-(3-methyl-2-(trifluoromethyl)phenyl)quinolin-4-yl)amino)piperidine-1-carboxylate (0.90 g, 1.183 mmol) in DCM (11.8 ml) was added dess-martinperiodinane (0.602 g, 1.420 mmol). The resulting mixture was stirred for 1 h, to the reaction flask was added saturated NaHCO₃ and stirred for 10 min. The organic layer was separated and dried over Na₂SO₄, filtered and concentrated. The residue was purified with flash chromatography (eluting with 0%-25% ethyl acetate in hexanes) to give the desired product as foam (741 mg, 83 %). LCMS calculated for C₃₆H₄₆Cl₂F₄N₃O₄Si (M+H)⁺: m/z = 758.3; found: 758.3.

### Step 15. tert-butyl (2S,4S)-2-(2-((tert-butyldimethylsilyl)oxy)ethyl)-4-((2,6-dichloro-8-fluoro-3-((E)-(hydroxyimino)methyl)-7-(3-methyl-2-(trifluoromethyl)phenyl)quinolin-4-yl)amino)piperidine-1-carboxylate

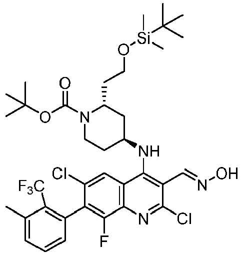

To a mixture of tert-butyl (2S,4S)-2-(2-((tert-butyldimethylsilyl)oxy)ethyl)-4-((2,6-dichloro-8-fluoro-3-formyl-7-(3-methyl-2-(trifluoromethyl)phenyl)quinolin-4-yl)amino)piperidine-1-carboxylate (741mg, 0.977 mmol) DCM (9.8 ml) and EtOH (9.8 ml) was added hydroxylamine hydrochloride (231 mg, 3.32 mmol) and pyridine (276 µl, 3.42 mmol). The resulting mixture was stirred at 40 °C for 16 hours. The solvent was evaporated *in vacuo.* The residue was dissolved in EtOAc and washed with water, brine. The organic layer was dried over MgSO₄, filtered and evaporated *in vacuo.* The crude mixture was purified by column chromatography on silica gel to give the desired product (0.46 g, 61%). LCMS calculated for C₃₆H₄₇Cl₂F₄N₄O₄Si (M+H)⁺: m/z = 773.3; found: 773.3.

### Step 16. tert-butyl (2S,4S)-2-(2-((tert-butyldimethylsilyl)oxy)ethyl)-4-(4,8-dichloro-6-fluoro-7-(3-methyl-2-(trifluoromethyl)phenyl)-1H-imidazo[4,5-c]quinolin-1-yl)piperidine-1-carboxylate

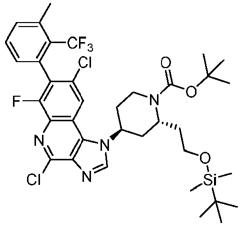

To a mixture of (tert-butyl (2S,4S)-2-(2-((tert-butyldimethylsilyl)oxy)ethyl)-4-((2,6-dichloro-8-fluoro-3-((E)-(hydroxyimino)methyl)-7-(3-methyl-2-(trifluoromethyl)phenyl)quinolin-4-yl)amino)piperidine-1-carboxylate (462 mg, 0.597 mmol), CH₂Cl₂ (1.5 mL) and 2-aminopyridine (112 mg, 1.194 mmol)) was added Ms-Cl (93 µl, 1.194 mmol) at 0 °C. After stirring at 0 °C for 2 h. The mixture was allowed to warm to room temperature overnight. The reaction mixture was diluted with water and DCM. The organic layer was washed with water, brine, dried over MgSO₄, filtered and concentrated. The crude was purifed by column chromatography on silica gel (90 mg, 20%). LCMS calculated for C₃₆H₄₅Cl₂F₄N₄O₃Si (M+H)⁺: m/z = 755.3; found: 755.3.

### Step 17. tert-butyl (2S,4S)-2-(2-((tert-butyldimethylsilyl)oxy)ethyl)-4-(8-chloro-6-fluoro-7-(3-methyl-2-(trifluoromethyl)phenyl)-4-(methylthio)-1H-imidazo[4,5-c]quinolin-1-yl)piperidine-1-carboxylate

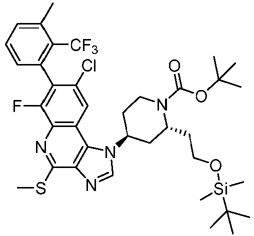

This compound was prepared according to the procedure described in **Example 80a and Example 80b, step 4,** replacing tert-butyl (2S,4S)-4-(7-bromo-4,8-dichloro-6-fluoro-1H-imidazo[4,5-c]quinolin-1-yl)-2-(2-(tert-butoxy)-2-oxoethyl)piperidine-1-carboxylate with tert-butyl (2S,4S)-2-(2-((tert-butyldimethylsilyl)oxy)ethyl)-4-(4,8-dichloro-6-fluoro-7-(3-methyl-2-(trifluoromethyl)phenyl)-1H-imidazo[4,5-c]quinolin-1-yl)piperidine-1-carboxylate. LCMS calculated for C₃₇H₄₈ClF₄N₄O₃SSi (M+H)⁺: m/z = 767.3; found: 767.4.

### Step 18. tert-butyl (2S,4S)-4-(8-chloro-6-fluoro-7-(3-methyl-2-(trifluoromethyl)phenyl)-4-(methylthio)-1H-imidazo[4,5-c]quinolin-1-yl)-2-(2-hydroxyethyl)piperidine-1-carboxylate

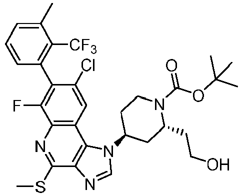

To a solution of tert-butyl (2S,4S)-2-(2-((tert-butyldimethylsilyl)oxy)ethyl)-4-(8-chloro-6-fluoro-7-(3-methyl-2-(trifluoromethyl)phenyl)-4-(methylthio)-1H-imidazo[4,5-c]quinolin-1-yl)piperidine-1-carboxylate (55 mg, 0.072 mmol) in THF (0.7 ml) was added 1.0 M TBAF in THF (108 µl, 0.108 mmol). The resulting mixture was stirred at 60 °C for 1 h. After cooling to rt, the reaction mixture was diluted with EtOAc and water, The organic layer was dried over Na₂SO₄, filtered and concentrated and used as is. LCMS calculated for C₃₁H₃₄ClF₄N₄O₃S (M+H)⁺: m/z = 653.2; found: 653.2.

### Step 19. tert-butyl (2S,4S)-4-(8-chloro-6-fluoro-7-(3-methyl-2-(trifluoromethyl)phenyl)-4-(methylthio)-1H-imidazo[4,5-c]quinolin-1-yl)-2-(cyanomethyl)piperidine-1-carboxylate

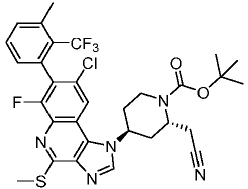

To a solution of tert-butyl (2S,4S)-4-(8-chloro-6-fluoro-7-(3-methyl-2-(trifluoromethyl)-phenyl)-4-(methylthio)-1H-imidazo[4,5-c]quinolin-1-yl)-2-(2-hydroxyethyl)piperidine-1-carboxylate (47 mg, 0.072 mmol) in DCM (0.7 ml) was added dess-martinperiodinane (33.6 mg, 0.079 mmol). The resulting mixture was stirred for 1 h, to the reaction flask was added saturated NaHCO₃ and stirred for 10 min. The organic layer was separated and dried over Na₂SO₄, filtered and concentrated. The crude was dissolved in THF (1 mL) and ammonium hydroxide (162 µl, 1.166 mmol) was added to reaction vial, followed by iodine (20.1 mg, 0.079 mmol). After stirring for 3 h, The reaction mixture was diluted with EtOAc and NaS₂O₃ solution. The organic layer was separated and concentrated. The residue was purified with flash chromatography to give the desired product (40 mg, 85%). LCMS calculated for C₃₁H₃₁ClF₄N₅O₂S (M+H)⁺: m/z = 648.2; found: 648.2.

### Step 20. tert-butyl (2S,4S)-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)-2-(cyanomethyl)piperidine-1-carboxylate

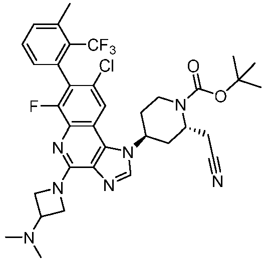

This compound was prepared according to the procedure described in **Example 80a and Example 80b, step 10,** replacing *N*,*N*,3-trimethylazetidin-3-amine hydrochloride with N,N*-*dimethylazetidin-3-amine dihydrochloride. LCMS calculated for C₃₅H₃₉ClF₄N₇O₂ (M+H)⁺: m/z = 700.3; found: 700.3.

### Step 21. 2-((2S,4S)-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)-1-((E)-4-(dimethylamino)but-2-enoyl) piperidin-2-yl)acetonitrile

A solution of tert-butyl (2S,4S)-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)-2-(cyanomethyl)piperidine-1-carboxylate (27.0 mg, 0.039 mmol) in CH₂Cl₂ (2.0 mL) and TFA (1.0 mL) was stirred at room temperature for 30 minutes before concentrating to dryness. The concentreate residue was redissolved in acetonitrile (1.0 mL) and DIPEA (100 µL) was added. The mixture was stirred for 5 minutes before HATU (5.1 mg, 0.014 mmol) and (E)-4-(dimethylamino)but-2-enoic acid hydrochloride (2.100 mg, 0.013 mmol) were added. Upon completion, the reaction was diluted with acetonitrile and purified using prep-LCMS (XBridge C18 column, eluting with a gradient of acetonitrile/water containing 0.1% TFA, at flow rate of 60 mL/min) afford the product as a mixture of diastereomers. LCMS calculated for C₃₆H₄₀ClF₄N₈O (M+H)⁺ m/z = 711.3; found 711.3.

### Example 265. 2-((2S,4S)-1-acryloyl-4-(8-chloro-7-(8-chloronaphthalen-1-yl)-4-(3-(dimethylamino)azetidin-1-yl)-6-fluoro-1H-imidazo[4,5-c]quinolin-1-yl)piperidin-2-yl)acetonitrile

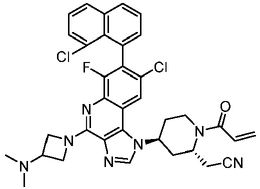

### Step 1. 2-(8-chloronaphthalen-1-yl)-4,4,5,5-tetramethyl-1,3,2-dioxaborolane

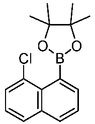

This compound was prepared according to the procedure described in **Example 89, Step 3,** replacing 3-bromo-5-chloro-4-methylpyridine with 1-bromo-8-chloronaphthalene. LCMS calculated for C₁₆H₁₉BClNO₂ (M+H)⁺: m/z = 289.1; found 289.1.

### Step 2. tert-butyl (2S,4S)-4-(8-chloro-7-(8-chloronaphthalen-1-yl)-4-(3-(dimethylamino)azetidin-1-yl)-6-fluoro-1H-imidazo[4,5-c]quinolin-1-yl)-2-(cyanomethyl)piperidine-1-carboxylate

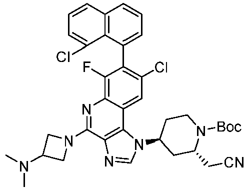

This compound was prepared according to the procedure described in **Example 89, Step 4,** replacing 3-chloro-4-methyl-5-(4,4,5,5-tetramethyl-1,3,2-dioxaborolan-2-yl)pyridine with 2-(8-chloronaphthalen-1-yl)-4,4,5,5-tetramethyl-1,3,2-dioxaborolane. LCMS calculated for C₃₇H₃₉Cl₂FN₇O₂ (M+H)⁺ m/z = 702.2; found 702.3.

### Step 3. 2-((2S,4S)-1-acryloyl-4-(8-chloro-7-(8-chloronaphthalen-1-yl)-4-(3-(dimethylamino)azetidin-1-yl)-6-fluoro-1H-imidazo[4,5-c]quinolin-1-yl)piperidin-2-yl)acetonitrile

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1*H*-[1,2,3]triazolo[4,5-*c*]quinolin-1-yl)-2-(cyanomethyl)-piperidine-1-carboxylate with tert-butyl (2S,4S)-4-(8-chloro-7-(8-chloronaphthalen-1-yl)-4-(3-(dimethylamino)azetidin-1-yl)-6-fluoro-1H-imidazo[4,5-c]quinolin-1-yl)-2-(cyanomethyl)-piperidine-1-carboxylate. The product was isolated as a mixture of diastereomers. LCMS calculated for C₃₅H₃₃Cl₂FN₇O (M+H)⁺ m/z = 656.2; found 656.2.

### Example 267a and Example 267b. 2-((2S,4S)-1-acryloyl-4-(8-chloro-7-(6-chloro-5-methyl-1H-indazol-4-yl)-4-(3-(dimethylamino)azetidin-1-yl)-6-fluoro-1H-imidazo[4,5-c]quinolin-1-yl)piperidin-2-yl)acetonitrile

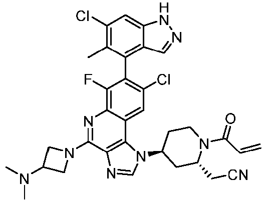

### Step 1. tert-butyl (2S,4S)-4-(8-chloro-7-(6-chloro-5-methyl-1-(tetrahydro-2H-pyran-2-yl)-1H-indazol-4-yl)-4-(3-(dimethylamino)azetidin-1-yl)-6-fluoro-1H-imidazo[4,5-c]quinolin-1-yl)-2-(cyanomethyl)piperidine-1-carboxylate

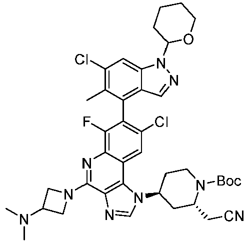

This compound was prepared according to the procedure described in **Example 89, Step 4,** replacing 3-chloro-4-methyl-5-(4,4,5,5-tetramethyl-1,3,2-dioxaborolan-2-yl)pyridine with 6-chloro-5-methyl-1-(tetrahydro-2H-pyran-2-yl)-4-(4,4,5,5-tetramethyl-1,3,2-dioxaborolan-2-yl)-1H-indazole. LCMS calculated for C₄₀H₄₇Cl₂FN₉O₃ (M+H)⁺ m/z = 790.3; found 790.3.

### Step 2. 2-((2S,4S)-1-acryloyl-4-(8-chloro-7-(6-chloro-5-methyl-1H-indazol-4-yl)-4-(3-(dimethylamino)azetidin-1-yl)-6-fluoro-1H-imidazo[4,5-c]quinolin-1-yl)piperidin-2-yl)acetonitrile

This compound was prepared according to the procedure described in **Example 85, Step 12,** replacing *tert*-butyl (2S,4S)-4-(8-chloro-7-(3-chloro-4-fluorophenyl)-6-fluoro-4-(((S)-1-methylpyrrolidin-2-yl)methoxy)-1*H*-[1,2,3]triazolo[4,5-*c*]quinolin-1-yl)-2-(cyanomethyl)piperidine-1-carboxylate with tert-butyl (2S,4S)-4-(8-chloro-7-(6-chloro-5-methyl-1-(tetrahydro-2H-pyran-2-yl)-1H-indazol-4-yl)-4-(3-(dimethylamino)azetidin-1-yl)-6-fluoro-1H-imidazo[4,5-c]quinolin-1-yl)-2-(cyanomethyl)piperidine-1-carboxylate.

**Example 267a:** Diastereomer 1. Peak 1. LCMS calculated for C₃₃H₃₃Cl₂FN₉O (M+H)⁺ m/z = 660.2; found 660.2.

**Example 267b:** Diastereomer 2. Peak 2. LCMS calculated for C₃₃H₃₃Cl₂FN₉O (M+H)⁺ m/z = 660.2; found 660.2.

### Example 270a and Example 270b. 2-((2S,4S)-1-(but-2-ynoyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile

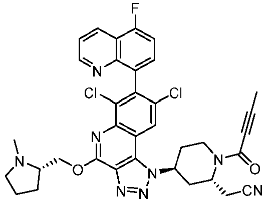

### Step 1. 2-amino-4-bromo-3,5-dichlorobenzoic acid

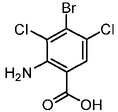

This compound was prepared according to the procedure described in **Example 1a and Example 1b, Step 1,** replacing 2-amino-4-bromo-3-fluorobenzoic acid with 2-amino-4-bromo-5-chlorobenzoic acid. LC-MS calculated for C₇H₅BrCl₂NO₂ (M+H)⁺: m/z = 283.9; found 284.0.

### Step 2. 7-bromo-6,8-dichloro-2H-benzo[d][1,3]oxazine-2,4(1H)-dione

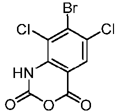

This compound was prepared according to the procedure described in **Example 1a and Example 1b, Step 2,** replacing 2-amino-4-bromo-5-chloro-3-fluorobenzoic acid with 2-amino-4-bromo-3,5-dichlorobenzoic acid. LC-MS calculated for C₈H₃BrCl₂NO₃ (M+H)⁺: m/z = 309.9; found 309.9.

### Step 3. 7-bromo-6,8-dichloro-3-nitroquinoline-2,4-diol

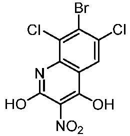

This compound was prepared according to the procedure described in **Example 1a and Example 1b, Step 3,** replacing 7-bromo-6-chloro-8-fluoro-2H-benzo[d][1,3]oxazine-2,4(1*H*)-dione with 7-bromo-6,8-dichloro-2H-benzo[d][1,3]oxazine-2,4(1H)-dione. LC-MS calculated for C₉H₄BrCl₂N₂O₄ (M+H)⁺: m/z = 352.9; found 352.9.

### Step 4. 7-bromo-2,4,6,8-tetrachloro-3-nitroquinoline

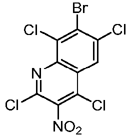

This compound was prepared according to the procedure described in **Example 1a and Example 1b, Step 4,** replacing 7-bromo-6-chloro-8-fluoro-3-nitroquinoline-2,4-diol with 7-bromo-6,8-dichloro-3-nitroquinoline-2,4-diol. LC-MS calculated for C₉H₂BrCl₄N₂O₂ (M+H)⁺: m/z = 388.8; found 388.8.

### Step 5. tert-butyl (2S,4S)-4-((7-bromo-2,6,8-trichloro-3-nitroquinolin-4-yl)amino)-2-(cyanomethyl)piperidine-1-carboxylate

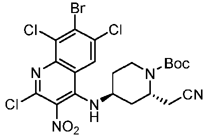

This compound was prepared according to the procedure described in **Example 1a and Example 1b, step 5,** replacing tert-butyl 4-aminopiperidine-1-carboxylate with tert-butyl (2S,4S)-4-amino-2-(cyanomethyl)piperidine-1-carboxylate **(Example 85, Step 6).** LCMS calculated for C₂₁H₂₂BrCl₃N₅O₄ (M+H)⁺: m/z = 592.0; found: 592.0.

### Step 6. tert-butyl (2S,4S)-4-((7-bromo-6,8-dichloro-2-(((S)-1-methylpyrrolidin-2-yl)methoxy)-3-nitroquinolin-4-yl)amino)-2-(cyanomethyl)piperidine-1-carboxylate

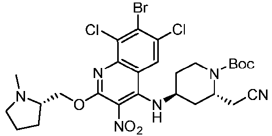

This compound was prepared according to the procedure described in **Example 1a and Example 1b, Step 6,** replacing *tert*-butyl 4-((7-bromo-2,6-dichloro-8-fluoro-3-nitroquinolin-4-yl)amino)piperidine-1-carboxylate with *tert*-butyl (2S,4S)-4-((7-bromo-2,6,8-trichloro-3-nitroquinolin-4-yl)amino)-2-(cyanomethyl)piperidine-1-carboxylate. LCMS calculated for C₂₇H₃₄BrCl₂N₆O₅ (M+H)⁺: m/z = 671.1; found: 671.1.

### Step 7. tert-butyl (2S,4S)-4-((3-amino-7-bromo-6,8-dichloro-2-(((S)-1-methylpyrrolidin-2-yl)methoxy)quinolin-4-yl)amino)-2-(cyanomethyl)piperidine-1-carboxylate

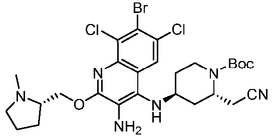

This compound was prepared according to the procedure described in **Example 1a and Example 1b, Step 7,** replacing *tert*-butyl (S)-4-((7-bromo-6-chloro-8-fluoro-2-((1-methylpyrrolidin-2-yl)-methoxy)-3-nitroquinolin-4-yl)amino)piperidine-1-carboxylate with *tert-*butyl (2S,4S)-4-((7-bromo-6,8-dichloro-2-(((S)-1-methylpyrrolidin-2-yl)methoxy)-3-nitroquinolin-4-yl)amino)-2-(cyanomethyl)piperidine-1-carboxylate. LCMS calculated for C₂₇H₃₆BrCl₂N₆O₃ (M+H)⁺: m/z = 641.1; found: 641.1.

### Step 8. tert-butyl (2S,4S)-4-(7-bromo-6,8-dichloro-4-(((S)-1-methylpyrrolidin-2-yl)methoxy)-1H-[1,2,3]triazolo[4,5-c]quinolin-1-yl)-2-(cyanomethyl)piperidine-1-carboxylate

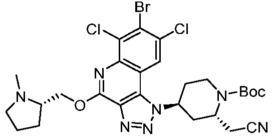

This compound was prepared according to the procedure described in **Example 14, Step 1,** replacing *tert*-butyl (S)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2S,4S)-4-((3-amino-7-bromo-6,8-dichloro-2-(((S)-1-methylpyrrolidin-2-yl)methoxy)quinolin-4-yl)amino)-2-(cyanomethyl)piperidine-1-carboxylate. LCMS calculated for C₂₇H₃₃BrCl₂N₇O₃ (M+H)⁺: m/z = 652.1; found: 652.2.

### Step 9. tert-butyl (2S,4S)-2-(cyanomethyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidine-1-carboxylate

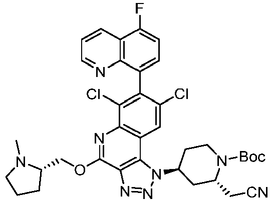

A screw-cap vial equipped with a magnetic stir bar was charged tert-butyl (2S,4S)-4-(7-bromo-6,8-dichloro-4-(((S)-1-methylpyrrolidin-2-yl)methoxy)-1H-[1,2,3]triazolo[4,5-c]quinolin-1-yl)-2-(cyanomethyl)piperidine-1-carboxylate (50.0 mg, 0.07 mmol) , (5-fluoroquinolin-8-yl)boronic acid (26 mg, 0.14 mmol), sodium carbonate (17.0 mg, 0.128 mmol), Pd(Ph₃P)₄ (8.0 mg, 7.0 µmol) and dioxane (0.8 ml)/water (0.2 ml). The vial was sealed with a Teflon-lined septum, evacuated and backfilled with nitrogen (this process was repeated a total of three times). Then the reaction was stirred at 95 °C for 2 h. The mixture was diluted with acetonitrile/water and purified using prep-LCMS (XBridge C18 column, eluting with a gradient of acetonitrile/water containing 0.1% TFA, at flow rate of 60 mL/min) to afford the desired products as TFA salt. LCMS calculated for C₃₆H₃₈Cl₂FN₈O₃ (M+H)⁺: m/z = 719.2; found: 719.2.

### Step 10. 2-((2S,4S)-1-(but-2-ynoyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile

*tert*-butyl (2S,4S)-2-(cyanomethyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidine-1-carboxylate (10 mg, 0.014 mmol) was redissolved in CH₂Cl₂ (2.0 mL) and TFA (1.0 mL). The mixture was stirred at room temperature for 30 minutes before concentrating to dryness. The concentreate residue was redissolved in MeCN (1.0 mL) and DIPEA (100 µL) was added. The mixture was stirred for 5 minutes before but-2-ynoic acid (3.5 mg, 0.042 mmol) and propylphosphonic anhydride solution (0.2 mL 50% in ethyl acetate) were added. Upon completion, the reaction was diluted with acetonitrile and purified using prep-LCMS (XBridge C18 column, eluting with a gradient of acetonitrile/water containing 0.15% NH₄OH, at flow rate of 60 mL/min) to afford the desired product.

**Example 270:** Diastereomer 1. Peak 1. LCMS calculated for C₃₅H₃₂Cl₂FN₈O₂ (M+H)⁺: m/z = 685.2; found: 685.2.

**Example 275:** Diastereomer 2. Peak 2. LCMS calculated for C₃₅H₃₂Cl₂FN₈O₂ (M+H)⁺: m/z = 685.2; found: 685.2.

### Example 271a and Example 271b. 2-((2S,4S)-1-acryloyl-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidin-2-yl)acetonitrile

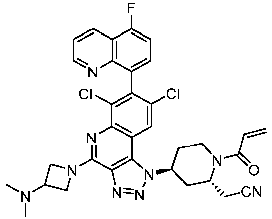

### Step 1. tert-butyl (2S,4S)-4-((7-bromo-6,8-dichloro-2-(3-(dimethylamino)azetidin-1-yl)-3-nitroquinolin-4-yl)amino)-2-(cyanomethyl)piperidine-1-carboxylate

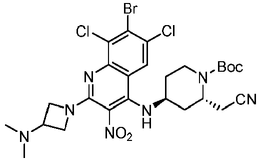

This compound was prepared according to the procedure described in **Example 9, Step 1,** replacing *tert*-butyl 4-((7-bromo-2,6-dichloro-8-fluoro-3-nitroquinolin-4-yl)amino)-piperidine-1-carboxylate with *tert*-butyl (2S,4S)-4-((7-bromo-2,6,8-trichloro-3-nitroquinolin-4-yl)amino)-2-(cyanomethyl)piperidine-1-carboxylate. LCMS calculated for C₂₆H₃₃BrCl₂N₇O₄ (M+H)⁺: m/z = 656.1; found: 656.1.

### Step 2. tert-butyl (2S,4S)-4-((3-amino-7-bromo-6,8-dichloro-2-(3-(dimethylamino)azetidin-1-yl)quinolin-4-yl)amino)-2-(cyanomethyl)piperidine-1-carboxylate

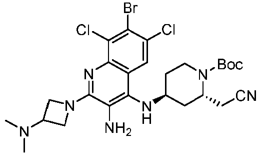

This compound was prepared according to the procedure described in **Example 1a and Example 1b, Step 7,** replacing tert-butyl (S)-4-((7-bromo-6-chloro-8-fluoro-2-((1-methylpyrrolidin-2-yl)-methoxy)-3-nitroquinolin-4-yl)amino)piperidine-1-carboxylate with *tert-butyl (2S,4S)-4-((7-bromo-6,8-dichloro-2-(3-(dimethylamino)azetidin-1-yl)-3-nitroquinolin-4-yl)amino)-2-(cyanomethyl)piperidine-1-carboxylate.* LCMS calculated for C₂₆H₃₅BrCl₂N₇O₂ (M+H)⁺: m/z = 626.1; found: 626.1.

### Step 3. tert-butyl (2S,4S)-4-(7-bromo-6,8-dichloro-4-(3-(dimethylamino)azetidin-1-yl)-1H-[1,2,3]triazolo[4,5-c]quinolin-1-yl)-2-(cyanomethyl)piperidine-1-carboxylate

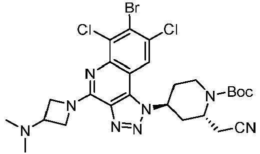

This compound was prepared according to the procedure described in **Example 14, Step 1,** replacing *tert*-butyl (S)-4-((3-amino-7-bromo-6-chloro-8-fluoro-2-((1-methylpyrrolidin-2-yl)methoxy)quinolin-4-yl)amino)piperidine-1-carboxylate with *tert*-butyl (2S,4S)-4-((3-amino-7-bromo-6,8-dichloro-2-(3-(dimethylamino)azetidin-1-yl)quinolin-4-yl)amino)-2-(cyanomethyl)piperidine-1-carboxylate. LCMS calculated for C₂₃H₃₂BrCl₂N₈O₂ (M+H)⁺: m/z = 637.1; found: 637.1.

### Step 4. tert-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate

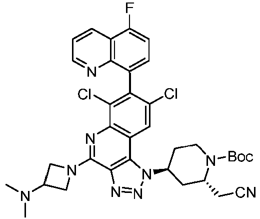

This compound was prepared according to the procedure described in **Example 270a and Example 270b, Step 9,** replacing tert-butyl (2S,4S)-4-(7-bromo-6,8-dichloro-4-(((S)-1-methylpyrrolidin-2-yl)methoxy)-1H-[1,2,3]triazolo[4,5-c]quinolin-1-yl)-2-(cyanomethyl)piperidine-1-carboxylate with *tert-butyl (2S,4S)-4-(7-bromo-6,8-dichloro-4-(3-(dimethylamino)azetidin-1-yl)-1H-[1,2,3]triazolo[4,5-c]quinolin-1-yl)-2-(cyanomethyl)piperidine-1-carboxylate.* LCMS calculated for C₃₅H₃₇Cl₂FN₉O₂ (M+H)⁺: m/z = 704.2; found: 704.2.

### Step 5. 2-((2S,4S)-1-acryloyl-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidin-2-yl)acetonitrile

tert-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate (20 mg, 0.028 mmol) was redissolved in CH₂Cl₂ (2.0 mL) and TFA (1.0 mL). The mixture was stirred at room temperature for 30 minutes before concentrating to dryness. The concentreate residue was redissolved in THF (1.0 mL) and triethylamine (100 µL) was added. The mixture was stirred for 5 minutes before acryloyl chloride (6.6 µL, 0.082 mmol) was added. The reaction was diluted with acetonitrile and purified using prep-LCMS (XBridge C18 column, eluting with a gradient of acetonitrile/water containing 0.15% NH₄OH, at flow rate of 60 mL/min) to afford the desired product.

**Example 271a:** Diastereomer 1. Peak 1. LCMS calculated for C₃₃H₃₁Cl₂FN₉O (M+H)⁺: m/z = 658.2; found: 658.2.

**Example 271b:** Diastereomer 2. Peak 2. LCMS calculated for C₃₃H₃₁Cl₂FN₉O (M+H)⁺: m/z = 658.2; found: 658.2.

### Example 276a and Example 276b. 2-((2S,4S)-1-acryloyl-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile

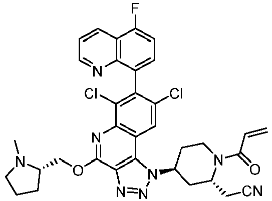

This compound was prepared according to the procedure described in **Example 271a and 271b, Step 5,** replacing tert-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with tert-butyl (2S,4S)-2-(cyanomethyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidine-1-carboxylate (**Example 270a and Example 270b, Step** 9).

**Example 276a:** Diastereomer 1. Peak 1. LCMS calculated for C₃₄H₃₂Cl₂FN₃O₂ (M+H)⁺ m/z = 673.2; found 673.2.

**Example 276b:** Diastereomer 2. Peak 2. LCMS calculated for C₃₄H₃₂Cl₂FN₃O₂ (M+H)⁺ m/z = 673.2; found 673.2.

### Example 277a and Example 277b. 2-((2S,4S)-1-(but-2-ynoyl)-4-(6,8-dichloro-4-(3-(dimethylamino)azetidin-1-yl)-7-(quinolin-8-yl)-1H-[1,2,3]triazolo[4,5-c]quinolin-1-yl)piperidin-2-yl)acetonitrile

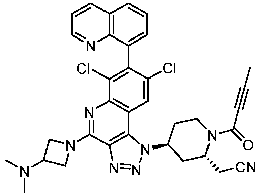

### Step 1. tert-butyl (2S,4S)-2-(cyanomethyl)-4-(6,8-dichloro-4-(3-(dimethylamino)azetidin-1-yl)-7-(quinolin-8-yl)-1H-[1,2,3]triazolo[4,5-c]quinolin-1-yl)piperidine-1-carboxylate

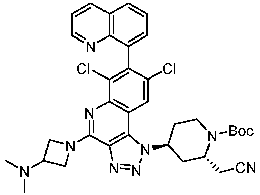

This compound was prepared according to the procedure described in **Example 271a and Example 271b, Step 4,** replacing (5-fluoroquinolin-8-yl)boronic acid with quinolin-8-ylboronic acid. The product was obtained as a mixture of diastereomers. LCMS calculated for C₃₅H₃₈Cl₂N₉O₂ (M+H)⁺: m/z = 686.2; found: 686.2.

### Step 2. 2-((2S,4S)-1-(but-2-ynoyl)-4-(6,8-dichloro-4-(3-(dimethylamino)azetidin-1-yl)-7-(quinolin-8-yl)-1H-[1,2,3]triazolo[4,5-c]quinolin-1-yl)piperidin-2-yl)acetonitrile

This compound was prepared according to the procedure described in **Example 270a and Example 270b, Step 10,** replacing tert-butyl (2S,4S)-2-(cyanomethyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidine-1-carboxylate with *tert*-butyl (2S,4S)-2-(cyanomethyl)-4-(6,8-dichloro-4-(3-(dimethylamino)azetidin-1-yl)-7-(quinolin-8-yl)-1H-[1,2,3]triazolo[4,5-c]quinolin-1-yl)piperidine-1-carboxylate.

**Example 277a:** Diastereomer 1. Peak 1. LCMS calculated for C₃₄H₃₂Cl₂N₉O (M+H)⁺ m/z = 652.2; found 652.2.

**Example 277b:** Diastereomer 2. Peak 2. LCMS calculated for C₃₄H₃₂Cl₂N₉O (M+H)⁺ m/z = 652.2; found 652.2.

### Example 278. 2-((2S,4S)-1-(but-2-ynoyl)-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidin-2-yl)acetonitrile

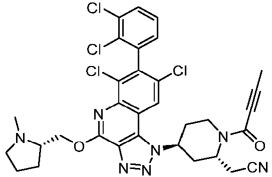

### Step 1. tert-butyl (2S,4S)-2-(cyanomethyl)-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidine-1-carboxylate

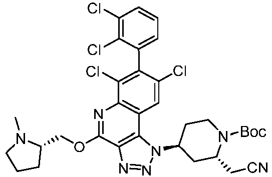

This compound was prepared according to the procedure described in **Example 270a and Example 270b, Step 9,** replacing (5-fluoroquinolin-8-yl)boronic acid with (2,3-dichlorophenyl)boronic acid. The product was isolated as a mixture of diastereomers. LCMS calculated for C₃₃H₃₃Cl₄N₇O₃ (M+H)⁺ m/z = 718.2; found 718.2.

### Step 2. 2-((2S,4S)-1-(but-2-ynoyl)-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 270a and Example 270b, Step 10,** replacing *tert*-butyl (2S,4S)-2-(cyanomethyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidine-1-carboxylate with *tert*-butyl (2S,4S)-2-(cyanomethyl)-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidine-1-carboxylate. The product was isolated as a mixture of diastereomers. LCMS calculated for C₃₂H₃₀Cl₄N₇O₂ (M+H)⁺ m/z = 684.1; found 684.1.

### Example 279a and Example 279b. 2-((2S,4S)-1-(but-2-ynoyl)-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidin-2-yl)acetonitrile

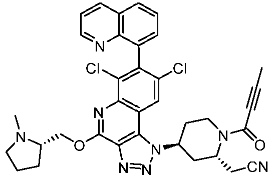

### Step 1. tert-butyl (2S,4S)-2-(cyanomethyl)-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidine-1-carboxylate

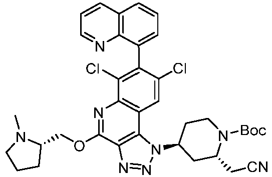

This compound was prepared according to the procedure described in **Example 270a and Example 270b, Step 9,** replacing (5-fluoroquinolin-8-yl)boronic acid with quinolin-8-ylboronic acid. The product was isolated as a mixture of diastereomers. LCMS calculated for C₃₃H₃₉Cl₂N₃O₃ (M+H)⁺ m/z = 701.2; found 701.2.

### Step 2. 2-((2S,4S)-1-(but-2-ynoyl)-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidin-2-yl)acetonitrile

This compound was prepared according to the procedure described in **Example 270a and Example 270b, Step 10,** replacing *tert*-butyl (2S,4S)-2-(cyanomethyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidine-1-carboxylate with tert-butyl (2S,4S)-2-(cyanomethyl)-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidine-1-carboxylate.

**Example 279a**: Diastereomer 1. Peak 1. LCMS calculated for C₃₅H₃₃Cl₂N₈O₂ (M+H)⁺ m/z = 667.2; found 667.2.

**Example 279b**: Diastereomer 2. Peak 2. LCMS calculated for C₃₅H₃₃Cl₂N₈O₂ (M+H)⁺ m/z = 667.2; found 667.2.

### Example 280a and Example 280b. 2-((2S,4S)-1-(but-2-ynoyl)-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile

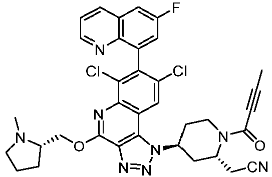

### Step 1. tert-butyl (2S,4S)-2-(cyanomethyl)-4-(6,8-dichloro-7-(6-fluoroquinolin-8-yl)-4-(((S)-1-methylpyrrolidin-2-yl)methoxy)-1H-[1, 2, 3]triazolo[4, 5-c]quinolin-1-yl)piperidine-1-carboxylate

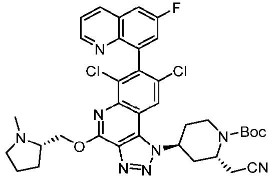

This compound was prepared according to the procedure described in **Example 270a and Example 270b, Step 9,** replacing (5-fluoroquinolin-8-yl)boronic acid with (6-fluoroquinolin-8-yl)boronic acid. The product was isolated as a mixture of diastereomers. LCMS calculated for C₃₆H₃₈Cl₂FN₈O₃ (M+H)⁺ m/z = 719.2; found 719.2.

### Step 2. 2-((2S,4S)-1-(but-2-ynoyl)-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 270a and Example 270b, Step 10,** replacing *tert*-butyl (2S,4S)-2-(cyanomethyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidine-1-carboxylate with *tert*-butyl (2S,4S)-2-(cyanomethyl)-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidine-1-carboxylate.

**Example 280a:** Diastereomer 1. Peak 1. LCMS calculated for C₃₅H₃₂Cl₂FN₈O₂ (M+H)⁺: m/z = 685.2; found: 685.2.

**Example 280b:** Diastereomer 2. Peak 2. LCMS calculated for C₃₅H₃₂Cl₂FN₈O₂ (M+H)⁺: m/z = 685.2; found: 685.2.

### Example 281. 2-((2S,4S)-1-acryloyl-4-(6,8-dichloro-7-(2,3-dichlorophenyl)-4-(3-(dimethylamino)azetidin-1-yl)-1H-[1,2,3]triazolo[4,5-c]quinolin-1-yl)piperidin-2-yl)acetonitrile

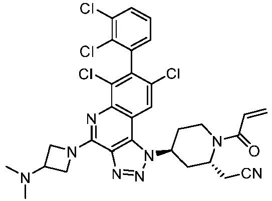

### Step 1. tert-butyl (2S,4S)-2-(cyanomethyl)-4-(6,8-dichloro-7-(2,3-dichlorophenyl)-4-(3-(dimethylamino)azetidin-1-yl)-1H-[1,2,3]triazolo[4,5-c]quinolin-1-yl)piperidine-1-carboxylate

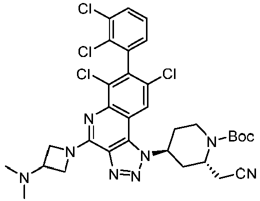

This compound was prepared according to the procedure described in **Example 271a and Example 271b, Step 4,** replacing (5-fluoroquinolin-8-yl)boronic acid with (2,3-dichlorophenyl)boronic acid. The product was isolated as a mixture of diastereomers. LCMS calculated for C₃₂H₃₅Cl₄N₈O₂ (M+H)⁺ m/z = 703.2; found 703.2.

### Step 2. 2-((2S,4S)-1-acryloyl-4-(6,8-dichloro-7-(2,3-dichlorophenyl)-4-(3-(dimethylamino)azetidin-1-yl)-1H-[1,2,3]triazolo[4,5-c]quinolin-1-yl)piperidin-2-yl)acetonitrile

This compound was prepared according to the procedure described in **Example 271a and Example 271b, Step 5,** replacing *tert*-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with *tert*-butyl (2S,4S)-2-(cyanomethyl)-4-(6,8-dichloro-7-(2,3-dichlorophenyl)-4-(3-(dimethylamino)azetidin-1-yl)-1H-[1,2,3]triazolo[4,5-c]quinolin-1-yl)piperidine-1-carboxylate. The product was isolated as a mixture of diastereomers. LCMS calculated for C₃₀H₂₉Cl₄N₈O (M+H)⁺ m/z = 657.1; found 657.2.

### Example 283a and example 283b. 2-((2S,4S)-1-acryloyl-4-(6,8-dichloro-4-(3-(dimethylamino)azetidin-1-yl)-7-(quinolin-8-yl)-1H-[1,2,3]triazolo[4,5-c]quinolin-1-yl)piperidin-2-yl)acetonitrile

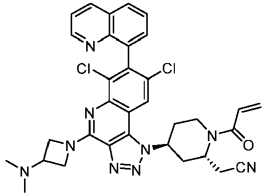

This compound was prepared according to the procedure described in **Example 271a and Example 271b, Step 5,** replacing *tert*-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with tert-butyl (2S,4S)-2-(cyanomethyl)-4-(6,8-dichloro-4-(3-(dimethylamino)azetidin-1-yl)-7-(quinolin-8-yl)-1H-[1,2,3]triazolo[4,5-c]quinolin-1-yl)piperidine-1-carboxylate **(Example 277a and Example 277b, Step 1).**

**Example 283a:** Diastereomer 1. Peak 1. LCMS calculated for C₃₃H₃₂Cl₂N₉O (M+H)⁺ m/z = 640.2; found 640.2.

**Example 283b:** Diastereomer 2. Peak 2. LCMS calculated for C₃₃H₃₂Cl₂N₉O (M+H)⁺ m/z = 640.2; found 640.2.

### Example 284a and example 284b. 2-((2S,4S)-1-acryloyl-4-(6,8-dichloro-4-(3-(dimethylamino)azetidin-1-yl)-7-(6-fluoroquinolin-8-yl)-1H-[1,2,3]triazolo[4,5-c]quinolin-1-yl)piperidin-2-yl)acetonitrile

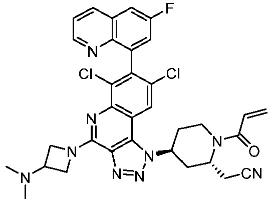

### Step 1. tert-butyl (2S,4S)-2-(cyanomethyl)-4-(6,8-dichloro-4-(3-(dimethylamino)azetidin-1-yl)-7-(6-fluoroquinolin-8-yl)-1H-[1,2,3]triazolo[4,5-c]quinolin-1-yl)piperidine-1-carboxylate

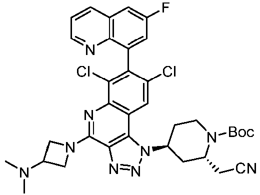

This compound was prepared according to the procedure described in **Example 271a and Example 271b, Step 4,** replacing (5-fluoroquinolin-8-yl)boronic acid with (6-fluoroquinolin-8-yl)boronic acid. The product was obtained as a mixture of diastereomers. LCMS calculated for C₃₅H₃₇Cl₂FN₉O₂ (M+H)⁺: m/z = 704.2; found: 704.2.

### Step 2. 2-((2S,4S)-1-acryloyl-4-(6,8-dichloro-4-(3-(dimethylamino)azetidin-1-yl)-7-(6-fluoroquinolin-8-yl)-1H-[1,2,3]triazolo[4,5-c]quinolin-1-yl)piperidin-2-yl)acetonitrile

This compound was prepared according to the procedure described in **Example 271a and Example 271b, Step 5,** replacing *tert*-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with tert-butyl (2S,4S)-2-(cyanomethyl)-4-(6,8-dichloro-4-(3-(dimethylamino)azetidin-1-yl)-7-(6-fluoroquinolin-8-yl)-1H-[1,2,3]triazolo[4,5-c]quinolin-1-yl)piperidine-1-carboxylate.

**Example 284a:** Diastereomer 1. Peak 1. LCMS calculated for C₃₃H₃₁Cl₂FN₉O (M+H)⁺ m/z = 658.2; found 658.2.

**Example 284b:** Diastereomer 2. Peak 2. LCMS calculated for C₃₃H₃₁Cl₂FN₉O (M+H)⁺ m/z = 658.2; found 658.2.

### Example 286. 2-((2S,4S)-1-acryloyl-4-(6,8-dichloro-7-(2-chloro-3-methylphenyl)-4-(((S)-1-methylpyrrolidin-2-yl)methoxy)-1H-[1,2,3]triazolo[4,5-c]quinolin-1-yl)piperidin-2-yl)acetonitrile

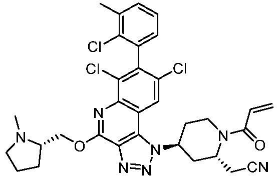

### Step 1. tert-butyl (2S,4S)-2-(cyanomethyl)-4-(6,8-dichloro-7-(2-chloro-3-methylphenyl)-4-(((S)-1-methylpyrrolidin-2-yl)methoxy)-1H-[1,2,3]triazolo[4,5-c]quinolin-1-yl)piperidine-1-carboxylate

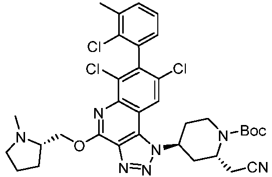

This compound was prepared according to the procedure described in **Example 270a and Example 270b, Step 9,** replacing (5-fluoroquinolin-8-yl)boronic acid with (2-chloro-3-methylphenyl)boronic acid. The product was isolated as a mixture of diastereomers. LCMS calculated for C₃₄H₃₉Cl₃N₇O₃ (M+H)⁺ m/z = 698.2; found 698.2.

### Step 2. 2-((2S,4S)-1-acryloyl-4-(6,8-dichloro-7-(2-chloro-3-methylphenyl)-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 271a and Example 271b, Step 5,** replacing *tert*-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with *tert-*butyl (2S,4S)-2-(cyanomethyl)-4-(6,8-dichloro-7-(2-chloro-3-methylphenyl)-4-(((S)-1-methylpyrrolidin-2-yl)methoxy)-1H-[1,2,3]triazolo[4,5-c]quinolin-1-yl)piperidine-1-carboxylate. The product was isolated as a mixture of diastereomers. LCMS calculated for C₃₂H₃₃Cl₃N₇O₂ (M+H)⁺ m/z = 652.2; found 652.2.

### Example 287. 2-((2S,4S)-1-acryloyl-4-(6,8-dichloro-7-(3-chloro-2-methylphenyl)-4-(((S)-1-methylpyrrolidin-2-yl)methoxy)-1H-[1,2,3]triazolo[4,5-c]quinolin-1-yl)piperidin-2-yl)acetonitrile

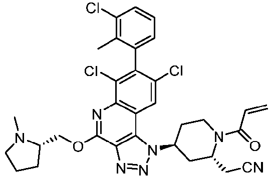

### Step 1. tert-butyl (2S,4S)-2-(cyanomethyl)-4-(6,8-dichloro-7-(3-chloro-2-methylphenyl)-4-(((S)-1-methylpyrrolidin-2-yl)methoxy)-1H-[1,2,3]triazolo[4,5-c]quinolin-1-yl)piperidine-1-carboxylate

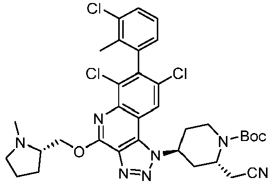

This compound was prepared according to the procedure described in **Example 270a and Example 270b, Step 9,** replacing (5-fluoroquinolin-8-yl)boronic acid with (3-chloro-2-methylphenyl)boronic acid. The product was isolated as a mixture of diastereomers. LCMS calculated for C₃₄H₃₉Cl₃N₇O₃ (M+H)⁺ m/z = 698.2; found 698.2.

### Step 2 2-((2S,4S)-1-acryloyl-4-(6,8-dichloro-7-(3-chloro-2-methylphenyl)-4-(((S)-1-methylpyrrolidin-2-yl)methoxy)-1H-[1,2,3]triazolo[4,5-c]quinolin-1-yl)piperidin-2-yl)acetonitrile

This compound was prepared according to the procedure described in **Example 271a and Example 271b, Step 5,** replacing *tert*-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with *tert*-butyl (2S,4S)-2-(cyanomethyl)-4-(6,8-dichloro-7-(3-chloro-2-methylphenyl)-4-(((S)-1-methylpyrrolidin-2-yl)methoxy)-1H-[1,2,3]triazolo[4,5-c]quinolin-1-yl)piperidine-1-carboxylate. The product was isolated as a mixture of diastereomers. LCMS calculated for C₃₂H₃₃Cl₃N₇O₂ (M+H)⁺ m/z = 652.2; found 652.2.

### Example 288a and example 288b. 2-((2S,4S)-1-acryloyl-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidin-2-yl)acetonitrile

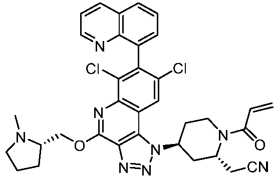

This compound was prepared according to the procedure described in **Example 271a and example 271b, Step 5,** replacing *tert*-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with *tert*-butyl (2S,4S)-2-(cyanomethyl)-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidine-1-carboxylate **(Example 279a and Example 279b, Step 1).**

**Example 288a:** Diastereomer 1. Peak 1. LCMS calculated for C₃₄H₃₃Cl₂N₈O₂ (M+H)⁺ m/z = 655.2; found 655.2.

**Example 288b:** Diastereomer 2. Peak 2. LCMS calculated for C₃₄H₃₃Cl₂N₃O₂ (M+H)⁺ m/z = 655.2; found 655.2.

### Example 289a and example 289b. 2-((2S,4S)-1-acryloyl-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile

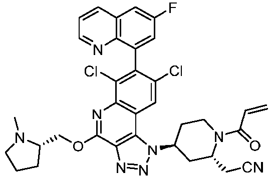

This compound was prepared according to the procedure described in **Example 271a and Example 271b, Step 5,** replacing *tert*-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with *tert*-butyl (2S,4S)-2-(cyanomethyl)-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidine-1-carboxylate **(Example 280a and Example 280b, Step 1).**

**Example 289a:** Diastereomer 1. Peak 1. LCMS calculated for C₃₄H₃₂Cl₂FN₃O₂ (M+H)⁺ m/z = 673.2; found 673.2.

**Example 289b:** Diastereomer 2. Peak 2. LCMS calculated for C₃₄H₃₂Cl₂FN₃O₂ (M+H)⁺ m/z = 673.2; found 673.2.

### Example 290. 2-((2S,4S)-1-acryloyl-4-(6,8-dichloro-7-(3-chloro-2-methoxyphenyl)-4-(3-(dimethylamino)azetidin-1-yl)-1H-[1,2,3]triazolo[4,5-c]quinolin-1-yl)piperidin-2-yl)acetonitrile

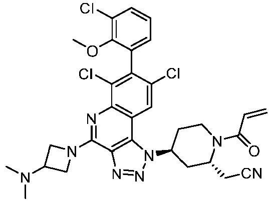

### Step 1. tert-butyl (2S,4S)-2-(cyanomethyl)-4-(6,8-dichloro-7-(3-chloro-2-methoxyphenyl)-4-(3-(dimethylamino)azetidin-1-yl)-1H-[1,2,3]triazolo[4,5-c]quinolin-1-yl)piperidine-1-carboxylate

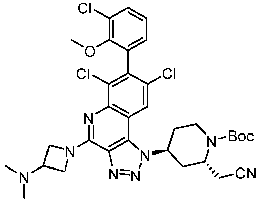

This compound was prepared according to the procedure described in **Example 271 and Example 272, Step 4,** replacing (5-fluoroquinolin-8-yl)boronic acid with (3-chloro-2-methoxyphenyl)boronic acid. The product was isolated as a mixture of diastereomers. LCMS calculated for C₃₃H₃₈Cl₃N₈O₃ (M+H)⁺ m/z = 699.2; found 699.2.

### Step 2. 2-((2S,4S)-1-acryloyl-4-(6,8-dichloro-7-(3-chloro-2-methoxyphenyl)-4-(3-(dimethylamino)azetidin-1-yl)-1H-[1,2,3]triazolo[4,5-c]quinolin-1-yl)piperidin-2-yl)acetonitrile

This compound was prepared according to the procedure described in **Example 271a and Example 271b, Step 5,** replacing *tert*-butyl (2S,4S)-2-(cyanomethyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidine-1-carboxylate with *tert*-butyl (2S,4S)-2-(cyanomethyl)-4-(6,8-dichloro-7-(3-chloro-2-methoxyphenyl)-4-(3-(dimethylamino)azetidin-1-yl)-1H-[1,2,3]triazolo[4,5-c]quinolin-1-yl)piperidine-1-carboxylate. The product was isolated as a mixture of diastereomers. LCMS calculated for C₃₁H₃₂Cl₃N₈O₂ (M+H)⁺ m/z = 653.2; found 653.2.

### Examples 291a and 291b. 3-(1-((endo)-2-azabicyclo[2.1.1]hexan-5-yl)-8-(2-cyanoethyl)-4-(3-(dimethylamino)azetidin-1-yl)-6-fluoro-7-(7-fluoronaphthalen-1-yl)-1H-imidazo[4,5-c]quinolin-2-yl)-N,N-dimethylpropanamide

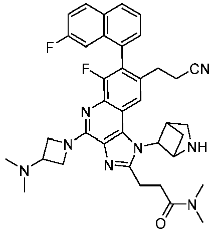

### Step 1. 7-bromo-2,4-dichloro-8-fluoro-6-iodo-3-nitroquinoline

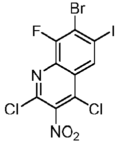

This compound was prepared according to protocols detailed in **Example 1a/1b** (Steps 1-4) using NIS instead of NCS in Step 1.

### Step 2. tert-butyl (endo)-5-((3-amino-7-bromo-2-(3-(dimethylamino)azetidin-1-yl)-8-fluoro-6-iodoquinolin-4-yl)amino)-2-azabicyclo[2.1.1]hexane-2-carboxylate

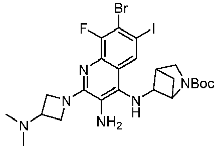

This compound was prepared according to the procedure described in **Example 15** (Steps 1 and 2), replacing 7-bromo-2,4,6-trichloro-8-fluoro-3-nitroquinoline with 7-bromo-2,4-dichloro-8-fluoro-6-iodo-3-nitroquinoline. LCMS calculated for C₂₄H₃₂BrFIN₃O₂ (M+H)⁺: m/z = 661.1; found 661.1.

### Step 3. tert-butyl (endo)-5-(7-bromo-2-(3-(dimethylamino)-3-oxopropyl)-4-(3-(dimethylamino)azetidin-1-yl)-6-fluoro-8-iodo-1H-imidazo[4,5-c]quinolin-1-yl)-2-azabicyclo[2.1.1]hexane-2-carboxylate

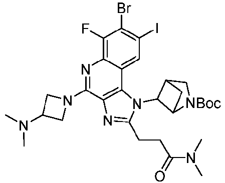

Tert-butyl (endo)-5-((3-amino-7-bromo-2-(3-(dimethylamino)azetidin-1-yl)-8-fluoro-6-iodoquinolin-4-yl)amino)-2-azabicyclo[2.1.1]hexane-2-carboxylate (2.38 g, 3.6 mmol) was dissolved in 10 mL EtOH, and N,N-dimethyl-4-oxobutanamide (558 mg, 4.3 mmol) was added at once. The reaction mixture was heated to 80 °C for 2 h in a sealed vial. Then the reaction was let to cool to 50 °C, and air was bubbled through for another 5 h. After reaction completion (monitored by LCMS), the volatiles were evaporated *in vacuo* and the crude product was purified by flash column chromatography (MeOH/DCM: 0-40%) to give the desired product as a brown solid (2.16 g, 78% yield). LCMS calculated for C₃₀H₃₉BrFIN₇O₃ (M+H)⁺: m/z = 770.1; found 770.2.

### Step 4. tert-butyl (endo)-5-(7-bromo-8-(2-cyanoethyl)-2-(3-(dimethylamino)-3-oxopropyl)-4-(3-(dimethylamino)azetidin-1-yl)-6-fluoro-1H-imidazo[4,5-c]quinolin-1-yl)-2-azabicyclo[2.1.1]hexane-2-carboxylate

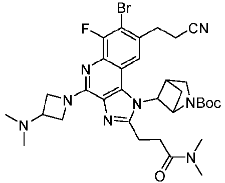

Part A: In a 40 mL vial tert-butyl (endo)-5-(7-bromo-2-(3-(dimethylamino)-3-oxopropyl)-4-(3-(dimethylamino)azetidin-1-yl)-6-fluoro-8-iodo-1H-imidazo[4,5-c]quinolin-1-yl)-2-azabicyclo[2.1.1]hexane-2-carboxylate (1000 mg, 1.298 mmol) and bis(tri-o-tolylphosphine)palladium(0) (93 mg, 0.130 mmol) were dissolved in DMF (5 ml). Triethylamine (362 µl, 2.60 mmol) and acrylonitrile (171 µl, 2.60 mmol) were added to the reaction mixture sequentially. The vial was purged with nitrogen and then capped and stirred at 80 °C for two hours. At this time LCMS indicated reaction completion. The reaction mixture was allowed to cool to RT and water was added (10 mL) followed by DCM (5 mL). The mixture was extracted (3 X 5 mL) with DCM. Organic extracts were combined, washed with water, and dried over MgSO₄. Volatiles were removed via rotary evaporation at 40 °C and 5 mbar (elevated temperature and low pressure to remove DMF). The crude was used directly in the Super-Hydride^{®} reduction reported below in Part B.

Part B: The crude residue from Part A was taken up in THF (10 mL) and cooled to 0 °C. Lithium triethylborohydride (Super-Hydride^{®} solution, 1M in THF) was added dropwise (1.3 mL, 1.0 equiv.) with LCMS monitoring. After 1 equivalent of Super-Hydride^{®} was added, ca. 30% conversion was observed. Another 0.6 equivalents (0.78 mL) of Super-Hydride^{®} was added at 0 °C followed by water (5mL) and DCM (10 mL). Brine was added to the mixture (10 mL) and the organic layer was isolated. The mixture was further extracted with DCM (3 x 10 ml) and organic extracts were then combined and dried over MgSO₄. Volatiles were removed *in vacuo* and the residue was dried via oil-pump high-vacuum overnight to yield the desired compound as a foamy, light-orange solid of suitable purity to be used in subsequent reactions without further purification (740 mg, 1.3 mmol, 82%). LCMS calculated for C₃₃H₄₃BrFN₃O₃ (M+H)⁺: m/z = 697.3; found 697.2.

### Step 5. 8-bromonaphthalene-2-diazonium tetrafluoroborate

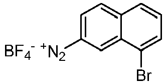

8-bromonaphthalen-2-amine (0.500 g, 2.25 mmol) was suspended in 1.5 mL AcOH open to air and set to stir at 21 °C. An HBF₄ solution (0.65 mL, 48 wt. % in H₂O) was added over 1 min and the suspension was then cooled on ice. 1.5 mL of a 2:1 acetic acid/isoamyl nitrite solution was added dropwise with stirring over 3 min. At this time, the mixture was diluted with diethyl ether (pre-cooled on dry-ice) to precipitate a light-orange solid. The mixture was filtered and washed with cold ether and dried on a fritted funnel to yield the title diazonium tetrafluoroborate as a light-orange solid (684 mg, 2.13 mmol, 95%). LCMS calculated for C₁₀H₆BrN₂ (M)⁺: m/z = 233.0; found 232.9.

### Step 6. 1-bromo-7-fluoronaphthalene

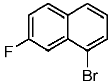

In a 40 mL vial 8-bromonaphthalene-2-diazonium tetrafluoroborate (255 mg, 0.795 mmol) was placed as a neat powder with a stir bar. The vial was set to stir and heated to 155 °C on a hot plate with an inlet needle of N₂ and an outlet needle vented to atmosphere. The vial was heated until white fumes evolved from the outlet needle and heating was continued until the white fumes ceased to evolve (~10 min). The vial was allowed to cool naturally to room temperature and then diluted with DCM (3 mL) and purified by automated flash-column chromatography (40 gram silica, 100% heptane as eluent) to yield the title compound as a white solid (55 mg, 31%).

### Step 7. 2-(7-fluoronaphthalen-1-yl)-4,4,5,5-tetramethyl-1,3,2-dioxaborolane

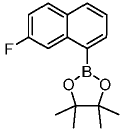

In a 1 dram vial 1-bromo-7-fluoronaphthalene (52 mg, 0.231 mmol), bis(pinacolato)diboron (70.4 mg, 0.277 mmol), potassium acetate (45.4 mg, 0.462 mmol), and dichloro[1,1'-bis(diphenylphosphino)ferrocene]palladium (II) dichloromethane adduct (189 mg, 0.231 mmol) were dissolved in dioxane (1.1 mL) under nitrogen. The mixture was heated to 80 °C and stirred for 2 h, at which time LCMS indicated completion of the reaction. The reaction solution was used directly in the subsequent Suzuki cross-coupling reaction as a ~0.2 M solution without further workup or purification.

### Step 8. 3-(1-((endo)-2-azabicyclo[2.1. 1]hexan-5-yl)-8-(2-cyanoethyl)-4-(3-(dimethylamino)azetidin-1-yl)-6-fluoro-7-(7-fluoronaphthalen-1-yl)-1H-imidazo[4,5-c]quinolin-2-yl)-N,N-dimethylpropanamide

In a 1 dram vial tert-butyl (*endo*)-5-(7-bromo-8-(2-cyanoethyl)-2-(3-(dimethylamino)-3-oxopropyl)-4-(3-(dimethylamino)azetidin-1-yl)-6-fluoro-1H-imidazo[4,5-c]quinolin-1-yl)-2-azabicyclo[2.1.1]hexane-2-carboxylate (100 mg, 0.143 mmol, 1.0 equiv.) and XPhos Pd G4 (12.3 mg, 0.0143 mmol, 0.1 equiv.) were combined. 2-(7-fluoronaphthalen-1-yl)-4,4,5,5-tetramethyl-1,3,2-dioxaborolane was added to the reaction vessel as the crude solution prepared above in Step 7 (0.235 mmol, 1.17 mL of a ~ 0.2 M solution) followed by 0.5 mL of 0.5 M K₃PO₄ solution. The reaction mixture was sparged with nitrogen for 2 minutes and the vial headspace was purged with nitrogen. The vial was sealed and heated to 80 °C for 1 hour. The mixture was diluted with acetonitrile, filtered, and purified via prep-LCMS (XBridge C18 column, eluting with a gradient of acetonitrile/water containing 0.1% TFA at flow rate of 60 mL/min) to afford the desired products as a TFA salt. The product was isolated as a pair of diastereomers (Peak 1 and Peak 2). Peak 1 and Peak 2 were combined separately and lyophilized to yield N-Boc-protected intermediates. The N-Boc-protected intermediates were treated with TFA (0.5 mL, 30 min, 21 °C), diluted with acetonitrile, and again purified via prep-LCMS (XBridge C18 column, eluting with a gradient of acetonitrile/water containing 0.1% TFA at flow rate of 60 mL/min) to afford the desired products as TFA their TFA salts after lyophilization.

**Example 291a.** Peak 1. LCMS calculated for C₃₈H₄₁F₂N₈O (M+H)⁺: m/z = 663.3; found: 663.4.

**Example 291b.** Peak 2. LCMS calculated for C₃₈H₄₁F₂N₈O (M+H)⁺: m/z = 663.3; found: 663.4. Peak 2 is more potent than Peak 1.

### Example 292a and 292b. 3-(1-((endo)-2-azabicyclo[2.1.1]hexan-5-yl)-4-(3-(dimethylamino)azetidin-1-yl)-2-ethyl-6-fluoro-7-(7-fluoro-3-hydroxynaphthalen-1-yl)-1H-imidazo[4,5-c]quinolin-8-yl)propanenitrile

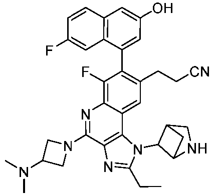

### Step 1. 6-fluoronaphthalen-1-amine

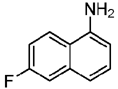

Part A: In a 40 mL vial 6-fluoro-1-naphthoic acid (0.500 g, 2.63 mmol, 1.0 equiv.) was dissolved in toluene (10 ml) open to air. Molecular sieves (3Å, 3 g) were added followed by tert-butanol (2.5 mL), DIPEA (2.3 mL, 13.15 mmol, 5.0 equiv.), and diphenylphosphoryl azide (DPPA, 0.85 mL, 3.94 mmol, 1.5 equiv.). The headspace was purged with nitrogen and the mixture was sealed and heated to 110 °C overnight. The mixture was filtered through Celite^{®} and volatiles were removed *in vacuo.* The residue was purified by automated FCC (0-30% EtOAc/heptane) to yield tert-butyl (6-fluoronaphthalen-1-yl)carbamate as a white solid (575 mg, 2.20 mmol, 84%).

Part B: tert-butyl (6-fluoronaphthalen-1-yl)carbamate (575 mg, 2.20 mmol, prepared in Part A) was dissolved in neat TFA (20 mL) and stirred at RT for 30 min. Volatiles were removed *in vacuo* and the residue was treated with saturated NaHCO₃ solution (30 mL) and extracted into DCM (3 X 15 mL). Organic extracts were combined, washed with brine (10 mL), dried over MgSO₄, and dried *in vacuo* to yield 6-fluoronaphthalen-1-amine as a white solid that was used in the subsequent step without further purification (339 mg, 2.10 mmol, 95%).

### Step 2. 6-fluoro-4-(4,4,5,5-tetramethyl-1,3,2-dioxaborolan-2-yl)naphthalen-2-ol

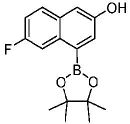

This compound was prepared according to protocols detailed in **Example 23a/23b** (Steps 2-4) using 6-fluoronaphthalen-1-amine in place of 6-chloronaphthalen-1-amine.

### Step 3. tert-butyl (endo)5-(7-bromo-4-(3-(dimethylamino)azetidin-1-yl)-2-ethyl-6-fluoro-8-iodo-1H-imidazo[4,5-c]quinolin-1-yl)-2-azabicyclo[2. 1. 1]hexane-2-carboxylate

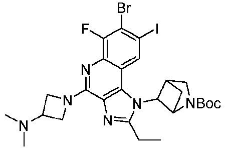

This compound was prepared according to protocols detailed in **Example 291a/291b** (Step 3) using propionaldehyde in place of *N*,*N*-dimethyl-4-oxobutanamide.

### Step 4. tert-butyl (endo)5-(7-bromo-8-(2-cyanoethyl)-4-(3-(dimethylamino)azetidin-1-yl)-2-ethyl-6-fluoro-1H-imidazo[4,5-c]quinolin-1-yl)-2-azabicyclo[2.1.1]hexane-2-carboxylate

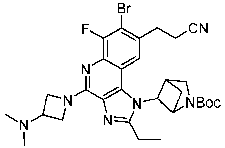

This compound was prepared according to protocols detailed in **Example 291a/291b** (Step 4) using tert-butyl 5-(7-bromo-4-(3-(dimethylamino)azetidin-1-yl)-2-ethyl-6-fluoro-8-iodo-1H-imidazo[4,5-c]quinolin-1-yl)-2-azabicyclo[2.1.1]hexane-2-carboxylate in place of tert-butyl 5-(7-bromo-2-(3-(dimethylamino)-3-oxopropyl)-4-(3-(dimethylamino)azetidin-1-yl)-6-fluoro-8-iodo-1H-imidazo[4,5-c]quinolin-1-yl)-2-azabicyclo[2.1.1]hexane-2-carboxylate.

### Step 5. 3-(1-((endo)-2-azabicyclo[2.1.1]hexan-5-yl)-4-(3-(dimethylamino)azetidin-1-yl)-2-ethyl-6-fluoro-7-(7-fluoro-3-hydroxynaphthalen-1-yl)-1H-imidazo[4,5-c]quinolin-8-yl)propanenitrile

This compound was prepared according to protocols detailed in **Example 291a/291b** (Step 8) using tert-butyl (*endo*)-5-(7-bromo-8-(2-cyanoethyl)-4-(3-(dimethylamino)azetidin-1-yl)-2-ethyl-6-fluoro-1H-imidazo[4,5-c]quinolin-1-yl)-2-azabicyclo[2.1.1]hexane-2-carboxylate instead of tert-butyl (*endo*)-5-(7-bromo-8-(2-cyanoethyl)-2-(3-(dimethylamino)-3-oxopropyl)-4-(3-(dimethylamino)azetidin-1-yl)-6-fluoro-1H-imidazo[4,5-c]quinolin-1-yl)-2-azabicyclo[2.1.1]hexane-2-carboxylate and 6-fluoro-4-(4,4,5,5-tetramethyl-1,3,2-dioxaborolan-2-yl)naphthalen-2-ol instead of 2-(7-fluoronaphthalen-1-yl)-4,4,5,5-tetramethyl-1,3,2-dioxaborolane.

**Example 292a.** Peak 1. LCMS calculated for C₃₅H₃₆F₂N₇O (M+H)⁺: m/z = 608.3; found: 608.3.

**Example 292b.** Peak 2. LCMS calculated for C₃₅H₃₆F₂N₇O (M+H)⁺: m/z = 608.3; found: 608.3. Peak 2 is more potent than Peak 1.

### Example 293. 3-(1-((endo)-2-azabicyclo[2.1.1]hexan-5-yl)-8-(2-cyanoethyl)-4-(3-(dimethylamino)azetidin-1-yl)-7-(3-hydroxynaphthalen-1-yl)-6-phenoxy-1H-imidazo[4,5-c]quinolin-2-yl)-N,N-dimethylpropanamide

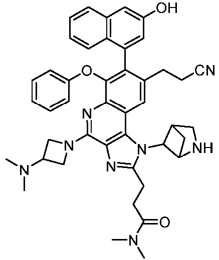

### Step 1. 2-amino-3-bromo-4-chloro-5-iodobenzoic acid

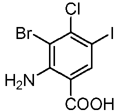

NIS (14.42 g, 64.1 mmol) was added to a solution of 2-amino-4-chlorobenzoic acid (10.0 g, 58.3 mmol) in DMF (200 ml) and then the reaction was stirred at 70 °C for overnight. After cooling to room temp, NBS (11.41 g, 64.1 mmol) was added and then the reaction was continued to stir at 70 °C for overnight. The resulting solution was added iced cold water (200 mL) and stirred for 20 min. The PPT was filtered and washed with water. The sticky solid was then dissolved with EtOAc and concentrated under reduced pressure. Water (200 mL) was added and the PPT was filtered, washed with water and hexanes, and then air-dried to provide the desired product as a solid. LC-MS calculated for C₇H₅BrCiINO₂⁺ (M+H)⁺: m/z = 375.8; found 375.9.

### Step 2. 8-bromo-7-chloro-6-iodo-2H-benzo[d][1,3]oxazine-2,4(1H)-dione

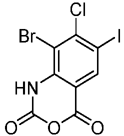

Triphosgene (10.32 g, 34.80 mmol) was added to a solution of 2-amino-3-bromo-4-chloro-5-iodobenzoic acid (18.7g, 49.7 mmol) in THF (166 ml) and then the reaction was stirred at room temp for overnight. The resulting residue was then filtered and washed with hexanes to afford the desired product as a solid. The filtrate was concentrated and then 1 N HCI (40 mL) was added and stirred for 1 h and then filtered. The solid was washed with hexanes to provide another batch of the product.

### Step 3. 8-bromo-7-chloro-6-iodo-3-nitroquinoline-2,4-diol

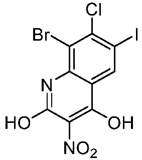

To a solution of 8-bromo-7-chloro-6-iodo-2H-benzo[*d*][1,3]oxazine-2,4(1*H*)-dione (9.30 g, 23.1 mmol) in toluene (77 mL) was added DIPEA (8.05 mL, 46.2 mmol) and ethyl 2-nitroacetate (5.13 mL, 46.2 mmol) dropwise at room temp. The resulting solution was stirred at 80 °C overnight. The reaction was cooled with ice water and then 1 N HCI (100 mL) was added and stirred 30 min. The PPT was filtered and washed with hexanes to afford the desired product as a solid.

### Step 4. 8-bromo-2,4,7-trichloro-6-iodo-3-nitroquinoline

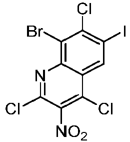

To a solution of 8-bromo-7-chloro-6-iodo-3-nitroquinoline-2,4-diol (3.60 g, 8.08 mmol) in POCl₃ (7.5 mL, 81 mmol) was added DIPEA (4.24 mL, 24.3 mmol) dropwise at room temp. The resulting solution was stirred at 110 °C for 3 h. The solvent was removed under vacuum and then azeotroped with toluene 3 times to provide the crude material which was purified by column chromatography (0-100% DCM:hexanes) to afford the desired product as a solid.

### Step 5. tert-butyl (endo)-5-((8-bromo-2,7-dichloro-6-iodo-3-nitroquinolin-4-yl)amino)-2-azabicyclo[2.1.1]hexane-2-carboxylate

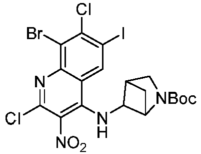

To a solution of 8-bromo-2,4,7-trichloro-6-iodo-3-nitroquinoline (1.59 g, 3.30 mmol) in THF (16.5 mL) was added DIPEA (2.59 mL, 14.8 mmol) dropwise at room temp. The resulting solution was stirred at room temp for overnight and used in the next step directly. LC-MS calculated for C₁₉H₁₉BrCl₂IN₄O₄⁺ (M+H)⁺: m/z = 642.9; found 643.0

### Step 6. tert-butyl (endo)-5-((8-bromo-7-chloro-2-(3-(dimethylamino)azetidin-1-yl)-6-iodo-3-nitroquinolin-4-yl)amino)-2-azabicyclo[2.1.1]hexane-2-carboxylate

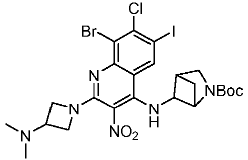

To the above solution (i.e. Step 5) was added *N*,*N*-dimethylazetidin-3-amine dihydrochloride (0.856 g, 4.95 mmol) and DIPEA (4.0 mL, 23 mmol) at room temp. The resulting solution was stirred at 50 °C for 1 h. The resultant mixture was concentrated and used in the next step directly without further purification. LC-MS calculated for C₂₄H₃₀BrClIN₆O₄⁺ (M+H)⁺: m/z = 707.0; found 707.1.

### Step 7. tert-butyl (endo)-5-((3-amino-8-bromo-7-chloro-2-(3-(dimethylamino)azetidin-1-yl)-6-iodoquinolin-4-yl)amino)-2-azabicyclo[2.1.1]hexane-2-carboxylate

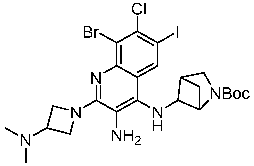

To the above residue (i.e. Step 6) in MeOH (20 mL) at 0 °C was added NH₄OH (30% aqueous solution, 4.28 mL, 33.0 mmol) in one portion. Sodium hydrosulfite (2.07 g, 11.9 mmol) (in water (10 mL)) was then added dropwise. The resultant mixture was stirred at 0 °C for 30 min. The reaction was quenched with water (30 mL) and extracted with DCM (x3). The organic layers were combined, dried, and concentrated under reduced pressure and used in the next step without further purification. LC-MS calculated for C₂₄H₃₂BrClIN₆O₂⁺ (M+H)⁺: m/z = 677.1; found 677.0.

### Step 8. tert-butyl (endo)-5-(6-bromo-7-chloro-2-(3-(dimethylamino)-3-oxopropyl)-4-(3-(dimethylamino)azetidin-1-yl)-8-iodo-1H-imidazo[4, 5-c]quinolin-1-yl)-2-azabicyclo[2.1.1]hexane-2-carboxylate

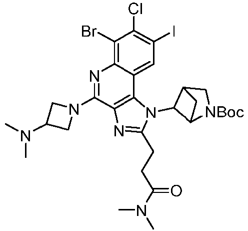

A solution of *tert*-butyl (*endo*)-5-((3-amino-8-bromo-7-chloro-2-(3-(dimethylamino)azetidin-1-yl)-6-iodoquinolin-4-yl)amino)-2-azabicyclo[2.1.1]hexane-2-carboxylate (600 mg, 0.885 mmol) and N,N-dimethyl-4-oxobutanamide (137 mg, 1.062 mmol) in EtOH:AcOH (5:1, 3.6 mL) was stirred at 80 °C for 3 h. The resultant mixture was concentrated and purified by column chromatography (0-20% MeOH:DCM) to afford the product as an oil. LC-MS calculated for C₃₀H₃₉BrClIN₇O₃⁺ (M+H)⁺: m/z = 786.1; found 786.2.

### Step 9. tert-butyl (endo)-5-(6-bromo-7-chloro-8-((E)-2-cyanovinyl)-2-(3-(dimethylamino)-3-oxopropyl)-4-(3-(dimethylamino)azetidin-1-yl)-1H-imidazo[4,5-c]quinolin-1-yl)-2-azabicyclo[2.1.1]hexane-2-carboxylate

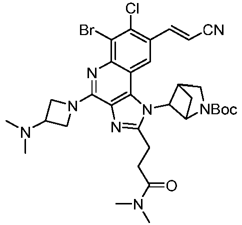

A solution of *tert*-butyl (endo)-5-(6-bromo-7-chloro-2-(3-(dimethylamino)-3-oxopropyl)-4-(3-(dimethylamino)azetidin-1-yl)-8-iodo-1H-imidazo[4,5-c]quinolin-1-yl)-2-azabicyclo[2.1.1]hexane-2-carboxylate (518 mg, 0.658 mmol), Pd(OAc)₂ (15 mg, 0.066 mmol), tri-o-tolylphosphine (40.1 mg, 0.132 mmol), acrylonitrile (87 µl, 1.316 mmol), TEA (183 µl, 1.316 mmol) in DMF (2.6 mL) was flushed with nitrogen for ca. 2 min. The resultant mixture was heated at 80 °C for 3 h then quenched by water at room temp. The mixture was extracted with DCM (x3). The combined organic extracts were dried, concentrated under reduced pressure and purified by column chromatography (0-20% MeOH:DCM) to afford the product as a foamy solid. LC-MS calculated for C₃₃H₄₁BrClN₈O₃⁺ (M+H)⁺: m/z = 711.2; found 711.3.

### Step 10. tert-butyl (endo)-5-(6-bromo-7-chloro-8-(2-cyanoethyl)-2-(3-(dimethylamino)-3-oxopropyl)-4-(3-(dimethylamino)azetidin-1-yl)-1H-imidazo[4,5-c]quinolin-1-yl)-2-azabicyclo[2.1.1]hexane-2-carboxylate

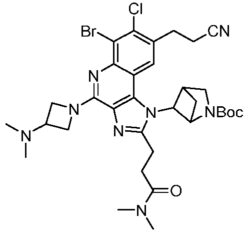

To a solution of tert-butyl (endo)-5-(6-bromo-7-chloro-8-((E)-2-cyanovinyl)-2-(3-(dimethylamino)-3-oxopropyl)-4-(3-(dimethylamino)azetidin-1-yl)-1*H*-imidazo[4,5-*c*]quinolin-1-yl)-2-azabicyclo[2.1.1]hexane-2-carboxylate (610 mg, 0.857 mmol) in THF (4.25 mL) was added super hydride (1M in THF, 1.3 mL, 1.3 mmol) at 0 °C. After a 10-min stir, the resultant mixture was quenched with water and extracted with DCM (x3). The combined organic extracts were dried, concentrated under reduced pressure and purified by column chromatography (0-20% MeOH:DCM) to afford the product as a foamy solid. LC-MS calculated for C₃₃H₄₃BrClN₈O₃⁺ (M+H)⁺: m/z = 713.2; found 713.3.

### Step 11. tert-butyl (endo)-5-(7-chloro-8-(2-cyanoethyl)-2-(3-(dimethylamino)-3-oxopropyl)-4-(3-(dimethylamino)azetidin-1-yl)-6-phenoxy-1H-imidazo[4,5-c]quinolin-1-yl)-2-azabicyclo[2.1.1]hexane-2-carboxylate

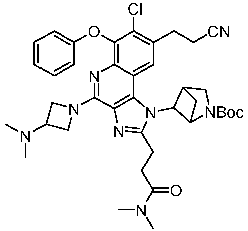

A solution of *tert*-butyl (*endo*)-5-(6-bromo-7-chloro-8-(2-cyanoethyl)-2-(3-(dimethylamino)-3-oxopropyl)-4-(3-(dimethylamino)azetidin-1-yl)-1H-imidazo[4,5-c]quinolin-1-yl)-2-azabicyclo[2.1.1]hexane-2-carboxylate (48 mg, 0.067 mmol), CuCl (3.3 mg, 0.034 mmol), 2,2,6,6-tetramethylheptane-3,5-dione (13 mg, 0.068 mmol), phenol (19 mg, 0.20 mmol), Cs₂CO₃ (43.8 mg, 0.134 mmol) in NMP (1 mL) was flushed with nitrogen for ca. 2 min. The resultant mixture was heated at 130 °C for overnight. After cooling to room temp, the mixture was quenched with water and extracted with EtOAc (x3). The combined organic extracts were dried, concentrated under reduced pressure and used without further purification. LC-MS calculated for C₃₉H₄₈ClN₈O₄⁺ (M+H)⁺: m/z = 727.4; found 727.5.

### Step 12. 3-(1-((endo)-2-azabicyclo[2.1.1]hexan-5-yl)-8-(2-cyanoethyl)-4-(3-(dimethylamino)azetidin-1-yl)-7-(3-hydroxynaphthalen-1-yl)-6-phenoxy-1H-imidazo[4,5-cjquinolin-2-yl)-N,N-dimethylpropanamide

A solution of tert-butyl (*endo*)-5-(7-chloro-8-(2-cyanoethyl)-2-(3-(dimethylamino)-3-oxopropyl)-4-(3-(dimethylamino)azetidin-1-yl)-6-phenoxy-1*H*-imidazo[4,5-c]quinolin-1-yl)-2-azabicyclo[2.1.1]hexane-2-carboxylate (45 mg, 0.067 mmol), XPhos Pd G2 (10.58 mg, 0.013 mmol), 4-(4,4,5,5-tetramethyl-1,3,2-dioxaborolan-2-yl)naphthalen-2-ol (27.2 mg, 0.101 mmol), phenol (19 mg, 0.20 mmol), K₃PO₄ (42.8 mg, 0.202 mmol) in dioxane:water (5:1, 1.2 mL) was flushed with nitrogen for ca. 2 min. The resultant mixture was heated at 100 °C for 1 h. After cooling to room temp, the mixture was quenched with water and extracted with EtOAc (x2). The combined organic extracts were dried and concentrated. To the resulting residue was added DCM (1 mL) and TFA (1 mL) and the mixture was stirred at room temp for 10 min. The mixture diluted with MeOH and purified using prep-LCMS (XBridge C18 column, eluting with a gradient of acetonitrile/water containing 0.1% TFA, at flow rate of 60 mL/min) to afford the desired product as a TFA salt. The product was isolated as a mixture of diastereomers. LC-MS calculated for C₄₄H₄₇N₈O₃⁺ (M+H)⁺: m/z = 735.4; found 735.5.

### Example 294. 3-(6-benzyl-1-((endo)-2-azabicyclo[2.1.1]hexan-5-yl)-8-(2-cyanoethyl)-4-(3-(dimethylamino)azetidin-1-yl)-7-(3-hydroxynaphthalen-1-yl)-1H-imidazo[4,5-c]quinolin-2-yl)-N,N-dimethylpropanamide

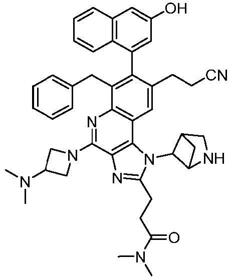

### Step 1. tert-butyl (endo)-5-(6-benzyl-7-chloro-8-(2-cyanoethyl)-2-(3-(dimethylamino)-3-oxopropyl)-4-(3-(dimethylamino)azetidin-1-yl)-1H-imidazo[4,5-c]quinolin-1-yl)-2-azabicyclo[2.1.1]hexane-2-carboxylate

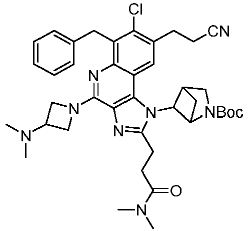

A solution of *tert*-butyl (endo)-5-(6-bromo-7-chloro-8-(2-cyanoethyl)-2-(3-(dimethylamino)-3-oxopropyl)-4-(3-(dimethylamino)azetidin-1-yl)-1H-imidazo[4,5-c]quinolin-1-yl)-2-azabicyclo[2.1.1]hexane-2-carboxylate **(Example 293, Step 10,** 20 mg, 0.028 mmol), bis(triphenylphosphine)palladium(II) chloride (3.93 mg, 5.60 µmol), 2,2,6,6-tetramethylheptane-3,5-dione (13 mg, 0.068 mmol), phenol (19 mg, 0.20 mmol) and benzylzinc(II) bromide (0.5 M in THF, 0.56 mL, 0.28 mmol) was flushed with nitrogen for ca. 2 min. The resultant mixture was heated at 80 °C for 1 h. After cooling to room temp, the mixture was quenched with water and extracted with EtOAc (x3). The combined organic extracts were dried, concentrated under reduced pressure and used without further purification. LC-MS calculated for C₄₀H₅₀ClN₈O₃⁺ (M+H)⁺: m/z = 725.4; found 725.5.

### Step 2. 3-(6-benzyl-1-((endo)-2-azabicyclo[2.1.1]hexan-5-yl)-8-(2-cyanoethyl)-4-(3-(dimethylamino)azetidin-1-yl)-7-(3-hydroxynaphthalen-1-yl)-1H-imidazo[4,5-c]quinolin-2-yl)-N,N-dimethylpropanamide

This compound was prepared according to the procedure described in **Example 293, Step 12,** replacing *tert*-butyl (*endo*)-5-(7-chloro-8-(2-cyanoethyl)-2-(3-(dimethylamino)-3-oxopropyl)-4-(3-(dimethylamino)azetidin-1-yl)-6-phenoxy-1*H*-imidazo[4,5-c]quinolin-1-yl)-2-azabicyclo[2.1.1]hexane-2-carboxylate with *tert*-butyl (*endo*)-5-(6-benzyl-7-chloro-8-(2-cyanoethyl)-2-(3-(dimethylamino)-3-oxopropyl)-4-(3-(dimethylamino)azetidin-1-yl)-1*H-*imidazo[4,5-*c*]quinolin-1-yl)-2-azabicyclo[2.1.1]hexane-2-carboxylate. The product was isolated as a mixture of diastereomers. LC-MS calculated for C₄₅H₄₉N₈O2⁺ (M+H)⁺: m/z = 733.4; found 733.5.

### Examples 295a, 295b, 295c, 295d. 4-(1-((endo)-2-azabicyclo[2.1.1]hexan-5-yl)-4-(3-(dimethylamino)azetidin-1-yl)-6-fluoro-2-(2-(methylamino)ethyl)-8-(1H-pyrazol-4-yl)-1H-imidazo[4,5-c]quinolin-7-yl)naphthalen-2-ol

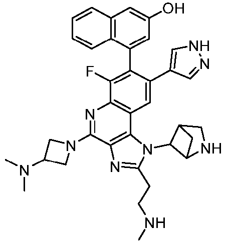

### Step 1. tert-butyl (endo)-5-(7-bromo-2-(2-((tert-butoxycarbonyl)(methyl)amino)ethyl)-8-chloro-4-(3-(dimethylamino)azetidin-1-yl)-6-fluoro-1H-imidazo[4,5-c]quinolin-1-yl)-2-azabicyclo[2.1.1]hexane-2-carboxylate

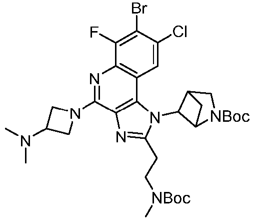

This compound was prepared according to the procedure described in **Example 293, Step 8,** replacing *tert-*butyl (*endo*)-5-((3-amino-8-bromo-7-chloro-2-(3-(dimethylamino)azetidin-1-yl)-6-iodoquinolin-4-yl)amino)-2-azabicyclo[2.1.1]hexane-2-carboxylate with *tert*-butyl (*endo*)-5-((3-amino-7-bromo-6-chloro-2-(3-(dimethylamino)azetidin-1-yl)-8-fluoroquinolin-4-yl)amino)-2-azabicyclo[2.1.1]hexane-2-carboxylate (**Example 15, Step 2)** and replacing *N,N*-dimethyl-4-oxobutanamide with *tert-*butyl methyl(3-oxopropyl)carbamate. LCMS calculated for C₃₃H₄₅BrClFN₇O₄⁺ (M+H)⁺: m/z = 736.2; found: 736.4.

### Step 2. tert-butyl (endo)-5-(2-(2-((tert-butoxycarbonyl)(methyl)amino)ethyl)-8-chloro-4-(3-(dimethylamino)azetidin-1-yl)-6-fluoro-7-(3-hydroxynaphthalen-1-yl)-1H-imidazo[4, 5-c]quinolin-1-yl)-2-azabicyclo[2.1.1]hexane-2-carboxylate

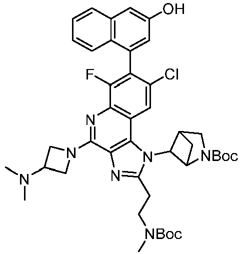

This compound was prepared according to the procedure described in **Example 293, Step 12,** replacing *tert*-butyl (*endo*)-5-(7-chloro-8-(2-cyanoethyl)-2-(3-(dimethylamino)-3-oxopropyl)-4-(3-(dimethylamino)azetidin-1-yl)-6-phenoxy-1*H*-imidazo[4,5-c]quinolin-1-yl)-2-azabicyclo[2.1.1]hexane-2-carboxylate with *tert*-butyl (endo)-5-(7-bromo-2-(2-((*tert-*butoxycarbonyl)(methyl)amino)ethyl)-8-chloro-4-(3-(dimethylamino)azetidin-1-yl)-6-fluoro-1*H*-imidazo[4,5-*c*]quinolin-1-yl)-2-azabicyclo[2.1.1]hexane-2-carboxylate and replacing XPhos Pd G2 with tetrakis(triphenylphosphine)palladium. LCMS calculated for C₄₃H₅₂ClFN₇O₅⁺ (M+H)⁺: m/z = 800.4; found: 800.4.

### Step 3. 4-(1-((endo)-2-azabicyclo[2.1.1]hexan-5-yl)-4-(3-(dimethylamino)azetidin-1-yl)-6-fluoro-2-(2-(methylamino)ethyl)-8-(1H-pyrazol-4-yl)-1H-imidazo[4,5-c]quinolin-7-yl)naphthalen-2-ol

This compound was prepared according to the procedure described in **Example 293, Step 12,** replacing *tert*-butyl (*endo*)-5-(7-chloro-8-(2-cyanoethyl)-2-(3-(dimethylamino)-3-oxopropyl)-4-(3-(dimethylamino)azetidin-1-yl)-6-phenoxy-1*H*-imidazo[4,5-c]quinolin-1-yl)-2-azabicyclo[2.1.1]hexane-2-carboxylate with *tert*-butyl (*endo*)-5-(2-(2-((*tert-*butoxycarbonyl)(methyl)amino)ethyl)-8-chloro-4-(3-(dimethylamino)azetidin-1-yl)-6-fluoro-7-(3-hydroxynaphthalen-1-yl)-1*H*-imidazo[4,5-c]quinolin-1-yl)-2-azabicyclo[2.1.1]hexane-2-carboxylate **(Step 2)** and replacing 4-(4,4,5,5-tetramethyl-1,3,2-dioxaborolan-2-yl)naphthalen-2-ol with 4-(4,4,5,5-tetramethyl-1,3,2-dioxaborolan-2-yl)-1H-pyrazole. The products were isolated as either a pair of enantiomers or a mixture of diastereomers.

**Example 295a.** Diastereomer 1. Peak 1. LCMS calculated for C₃₆H₃₉FN₉O⁺ (M+H)⁺: m/z = 632.3; found: 632.2.

**Example 295b.** Diastereomer 2. Peak 2. LCMS calculated for C₃₆H₃₉FN₉O⁺ (M+H)⁺: m/z = 632.3; found: 632.2. Peak 2 is the most potent peak out of these four isomers.

**Example 295c.** Diastereomer 3. Peak 3. LCMS calculated for C₃₆H₃₉FN₉O⁺ (M+H)⁺: m/z = 632.3; found: 632.2.

**Example 295d.** Diastereomer 4. Peak 4. LCMS calculated for C₃₆H₃₉FN₉O⁺ (M+H)⁺: m/z = 632.3; found: 632.2.

### Examples 296a and 296b. 3-(1-((endo)-2-((1H-pyrrol-2-yl)methyl)-2-azabicyclo[2.1.1]hexan-5-yl)-4-(3-(dimethylamino)azetidin-1-yl)-2-ethyl-6-fluoro-7-(3-hydroxynaphthalen-1-yl)-1H-imidazo[4,5-c]quinolin-8-yl)propanenitrile

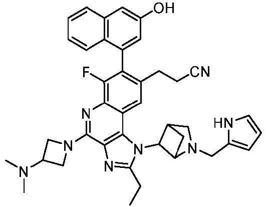

### Step 1. 3-(1-((endo)-2-azabicyclo[2.1.1]hexan-5-yl)-4-(3-(dimethylamino)azetidin-1-yl)-2-ethyl-6-fluoro-7-(3-hydroxynaphthalen-1-yl)-1H-imidazo[4,5-c]quinolin-8-yl)propanenitrile

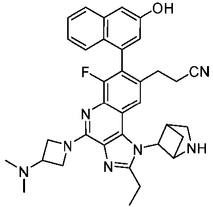

This compound was prepared according to the procedure described in **Example 292a/b,** replacing 6-fluoro-4-(4,4,5,5-tetramethyl-1,3,2-dioxaborolan-2-yl)naphthalen-2-ol with 4-(4,4,5,5-tetramethyl-1,3,2-dioxaborolan-2-yl)naphthalen-2-ol in **Step 5.** LCMS calculated for C₃₅H₃₇FN₇O (M+H)⁺: m/z = 590.3; found 590.3.

### Step 2. 3-(1-((endo)-2-((1H-pyrrol-2-yl)methyl)-2-azabicyclo[2.1.1]hexan-5-yl)-4-(3-(dimethylamino)azetidin-1-yl)-2-ethyl-6-fluoro-7-(3-hydroxynaphthalen-1-yl)-1H-imidazo[4,5-c]quinolin-8-yl)propanenitrile

To a solution of 3-(1-((*endo*)-2-azabicyclo[2.1.1]hexan-5-yl)-4-(3-(dimethylamino)azetidin-1-yl)-2-ethyl-6-fluoro-7-(3-hydroxynaphthalen-1-yl)-1H-imidazo[4,5-c]quinolin-8-yl)propanenitrile (15 mg, 0.025 mmol) and 1H-pyrrole-2-carbaldehyde (4.8 mg. 0.05 mmol) in 1,2-dichloroethane (1 mL) was added sodium triacetoxyborohydride (10.8 mg, 0.05 mmol), and the resulting mixture was stirring at room temperature overnight. The reaction mixture was then diluted with methanol and purified using prep-LCMS (XBridge C18 column, eluting with a gradient of acetonitrile/water containing 0.1% TFA, at flow rate of 60 mL/min) to afford the desired product as a TFA salt. The products were isolated as pairs of enantiomers.

**Example 296a.** Diastereomer 1. Peak 1. LCMS calculated for C₄₀H₄₂FN₈O (M+H)⁺: m/z = 669.4; found: 669.4.

**Example 296b.** Diastereomer 2. Peak 2. LCMS calculated for C₄₀H₄₂FN₈O (M+H)⁺: m/z = 669.4; found: 669.4. Peak 2 is the more potent isomer.

### Example 297. 5-(1-((endo)-2-azabicyclo[2.1.1]hexan-5-yl)-8-(2-cyanoethyl)-2-(3-(dimethylamino)-3-oxopropyl)-6-fluoro-7-(3-hydroxynaphthalen-1-yl)-1H-imidazo[4,5-c]quinolin-4-yl)-2-fluoro-N-methylbenzamide

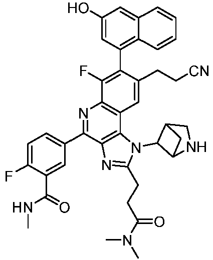

### Step 1: 1-bromo-3-(methoxymethoxy)naphthalene

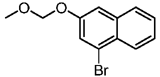

A sample of 4-bromonaphthalen-2-ol (1.57 g, 7.04 mmol) was dissolved in DCM (14 mL) and stirred at room temperature. The solution was treated with *N,N-*diisopropylethylamine (1.4 mL, 7.8 mmol) and chloromethyl methyl ether (0.6 mL, 7.8 mmol). After 20 mins, LCMS indicated complete conversion to the product. The reaction was quenched with satd. aq. NH₄Cl and diluted with DCM. The layers were separated, and the aqueous layer was extracted with additional DCM. The combined organic layers were dried over MgSO₄, filtered, and concentrated *in vacuo.* The crude material was dissolved in 50% DCM in hexanes and filtered through a silica plug. The filtrate was concentrated *in vacuo* to provide 1-bromo-3-(methoxymethoxy)naphthalene (1.76 g, 6.59 mmol, 94% yield). LCMS calculated for C₁₁H₈BrO₄ (M-MeOH)⁺: m/z = 235.0, 237.0; found: 235.0, 237.0.

### Step 2: 2-(3-(Methoxymethoxy)naphthalen-1-yl)-4,4,5,5-tetramethyl-1,3,2-dioxaborolane

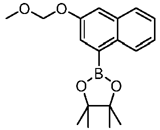

A sample of 1-bromo-3-(methoxymethoxy)naphthalene (1.76 g, 6.59 mmol) was dissolved in dioxane (19 mL) and stirred at room temperature. The solution was treated with potassium acetate (1.9 g, 19.8 mmol) and bis(pinacolato)diboron (2.5 g, 9.9 mmol). Finally, [1,1'-bis(diphenylphosphino)ferrocene]dichloropalladium(II), DCM complex (0.270 g, 0.329 mmol) was added to the solution, which was then stirred at 100 °C. After 16 hours, LCMS indicated complete conversion to the product. The reaction mixture was diluted with EtOAc, filtered to remove KOAc, and concentrated *in vacuo.* The crude material was purified by flash column chromatography (0-40% EtOAc/hexanes) to give 2-(3-(methoxymethoxy)naphthalen-1-yl)-4,4,5,5-tetramethyl-1,3,2-dioxaborolane (1.74 g, 5.53 mmol, 84% yield). LCMS calculated for C₁₇H₂₀BO₃ (M-MeOH)⁺: m/z = 283.2; found: 283.1.

### Step 3: tert-Butyl (endo)-5-((7-bromo-2-chloro-8-fluoro-6-iodo-3-nitroquinolin-4-yl)amino)-2-azabicyclo[2.1.1]hexane-2-carboxylate

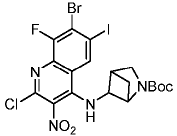

A sample of *tert*-butyl (*endo*)-5-amino-2-azabicyclo[2.1.1]hexane-2-carboxylate (10.64 g, 53.7 mmol) was dissolved in *N*-methyl-2-pyrrolidone (268 mL) and stirred at room temperature. The solution was treated with *N,N*-diisopropylethylamine (18.8 mL, 107 mmol) and 7-bromo-2,4-dichloro-8-fluoro-6-iodo-3-nitroquinoline (25.0 g, 53.7 mmol). The reaction mixture was heated to 60 °C and stirred. After 30 mins, LC/MS showed complete conversion to the desired product, with ~5% double addition.

The solution was poured into a mixture of water (300 mL) and saturated NH₄Cl (100 mL), and stirred at room temperature for 30 mins. The resultant suspension was vacuum filtered, and the solid was dried with continued air flow to provide *tert*-butyl (*endo*)-5-((7-bromo-2-chloro-8-fluoro-6-iodo-3-nitroquinolin-4-yl)amino)-2-azabicyclo[2.1.1]hexane-2-carboxylate (35.6 g, 56.7 mmol, quantitative yield) as a yellow powder. LCMS calculated for C₁₉H₁₉BrClFIN₄O₄ (M+H)⁺: m/z = 626.9, 628.9; found: 627.0, 629.0.

### Step 4: tert-Butyl (endo)-5-((3-amino-7-bromo-2-chloro-8-fluoro-6-iodoquinolin-4-yl)amino)-2-azabicyclo[2.1.1]hexane-2-carboxylate

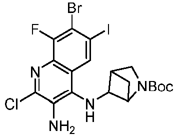

A sample of *tert*-butyl (*endo*)-5-((7-bromo-2-chloro-8-fluoro-6-iodo-3-nitroquinolin-4-yl)amino)-2-azabicyclo[2.1.1]hexane-2-carboxylate (35.6 g, 56.7 mmol) was dissolved in methanol (243 mL) and water (40 mL) and stirred at room temperature. The solution was treated with ammonium hydroxide (8.1 mL, 62 mmol). Sodium dithionate (52.6 g, 255 mmol) was then added as a powder to the solution in five portions, every 5 minutes. After 1 hour, LCMS showed complete reduction of the starting material, with two product peaks (reflecting different protonation states). The reaction was quenched with water and diluted with DCM. The layers were separated, and the aqueous layer was extracted with additional DCM. The combined organic layers were dried over MgSO₄, filtered, and concentrated *in vacuo.* The product tert-butyl (*endo*)-5-((3-amino-7-bromo-2-chloro-8-fluoro-6-iodoquinolin-4-yl)amino)-2-azabicyclo[2.1.1]hexane-2-carboxylate (36.3 g, 60.8 mmol, quantitative yield) was not purified further and was used as crude. LCMS calculated for C₁₉H₂₁BrClFIN₄O₂ (M+H)⁺: m/z = 597.0, 599.0; found: 596.9, 598.9.

### Step 5: tert-Butyl (endo)-5-(7-bromo-4-chloro-6-fluoro-8-iodo-2-(3-methoxy-3-oxopropyl)-1H-imidazo[4,5-c]quinolin-1-yl)-2-azabicyclo[2.1.1]hexane-2-carboxylate

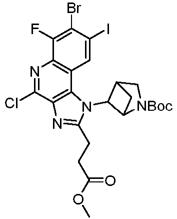

The crude sample of tert-butyl (endo)-5-((3-amino-7-bromo-2-chloro-8-fluoro-6-iodoquinolin-4-yl)amino)-2-azabicyclo[2.1.1]hexane-2-carboxylate (9 g, 15.1 mmol) was dissolved in DMF (75 mL) and AcOH (38 mL) in a round-bottomed flask and stirred at room temperature. The solution was treated with methyl 4-oxobutanoate (5.13 g, 40.7 mmol), warmed to 80 °C, and was stirred overnight, open to air. After 16 hours, LCMS indicated complete conversion to the product.

The reaction was cooled to RT, quenched with water and diluted with EtOAc. The layers were separated, and the aqueous layer was extracted with additional EtOAc. The combined organic layers were washed with sat. aq. NaHCO₃, dried over MgSO₄, filtered, and concentrated *in vacuo.* The crude material was purified by flash column chromatography (0-40% EtOAc/DCM) to give *tert-*butyl (*endo*)-5-(7-bromo-4-chloro-6-fluoro-8-iodo-2-(3-methoxy-3-oxopropyl)-1*H*-imidazo[4,5-*c*]quinolin-1-yl)-2-azabicyclo[2.1.1]hexane-2-carboxylate (4 g, 5.77 mmol, 38% yield). LCMS calculated for C₂₄H₂₅BrClFIN₄O₄ (M+H)⁺: m/z = 693.0, 695.0; found: 693.0, 695.0.

### Step 6: tert-Butyl (endo)-5-(7-bromo-4-chloro-8-(2-cyanoethyl)-6-fluoro-2-(3-methoxy-3-oxopropyl)-1H-imidazo[4,5-c]quinolin-1-yl)-2-azabicyclo[2.1.1]hexane-2-carboxylate

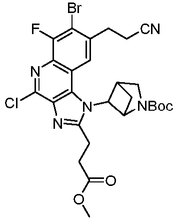

A sample of *tert*-butyl (*endo*)-5-(7-bromo-4-chloro-6-fluoro-8-iodo-2-(3-methoxy-3-oxopropyl)-1*H*-imidazo[4,5-*c*]quinolin-1-yl)-2-azabicyclo[2.1.1]hexane-2-carboxylate (4 g, 5.77 mmol) was dissolved in DMF (11.5 mL) and stirred at room temperature. The solution was treated with *N,N*-diisopropylethylamine (2.0 mL, 11.5 mmol) and acrylonitrile (0.44 mL, 11.5 mmol). Tetramethylammonium formate (4.12 mL, 8.65 mmol) was added as a 30% w/w solution in water. Lastly, the solution was treated with tetrakis(triphenylphosphine)-palladium(0) (0.333 g, 0.288 mmol) and stirred at 80 °C. After 90 minutes, LC/MS showed complete conversion to the products (~60% desired coupling to ~40% proto-dehalogenation).

The reaction was cooled to RT, quenched with satd. aq. NH₄Cl and diluted with EtOAc. The layers were separated, and the aqueous layer was extracted with additional EtOAc. The combined organic layers were dried over MgSO₄, filtered, and concentrated in *vacuo.* The crude material was purified by flash column chromatography (0-100% EtOAc/DCM) to give *tert*-butyl (*endo*)-5-(7-bromo-4-chloro-8-(2-cyanoethyl)-6-fluoro-2-(3-methoxy-3-oxopropyl)-1*H*-imidazo[4,5-*c*]quinolin-1-yl)-2-azabicyclo[2.1.1]hexane-2-carboxylate (1.47 g, 2.37 mmol, 41% yield). LCMS calculated for C₂₇H₂₉BrCIFN₅O₄ (M+H)⁺: m/z = 620.1, 622.1; found: 620.0, 622.0.

### Step 7: tert-Butyl (endo)-5-(7-bromo-8-(2-cyanoethyl)-6-fluoro-2-(3-methoxy-3-oxopropyl)-4-(methylthio)-1H-imidazo[4,5-c]quinolin-1-yl)-2-azabicyclo[2.1.1]hexane-2-carboxylate

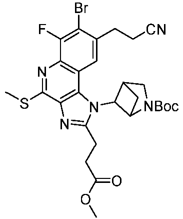

A sample of *tert*-butyl (*endo*)-5-(7-bromo-4-chloro-8-(2-cyanoethyl)-6-fluoro-2-(3-methoxy-3-oxopropyl)-1*H*-imidazo[4,5-*c*]quinolin-1-yl)-2-azabicyclo[2.1.1]hexane-2-carboxylate (1.47 g, 2.37 mmol) was dissolved in MeCN (30 mL) and stirred at room temperature. The solution was treated with sodium thiomethoxide (0.431 g, 6.16 mmol). After 45 minutes, LCMS indicated complete conversion to the product. The reaction was quenched by addition of acetic acid (1.4 mL, 24 mmol), and immediately adsorbed onto silica.

This material was purified by flash column chromatography (0-50% EtOAc/DCM) to give *tert*-butyl (*endo*)-5-(7-bromo-8-(2-cyanoethyl)-6-fluoro-2-(3-methoxy-3-oxopropyl)-4-(methylthio)-1*H*-imidazo[4,5-*c*]quinolin-1-yl)-2-azabicyclo[2.1.1]hexane-2-carboxylate (902 mg, 1.426 mmol, 60% yield). LCMS calculated for C₂₈H₃₂BrFN₅O₄S (M+H)⁺: m/z = 632.1, 634.1; found: 632.0, 634.1.

### Step 8: 3-(7-Bromo-1-((endo)-2-(tert-butoxycarbonyl)-2-azabicyclo[2.1.1]hexan-5-yl)-8-(2-cyanoethyl)-6-fluoro-4-(methylthio)-1H-imidazo[4,5-c]quinolin-2-yl)propanoic acid

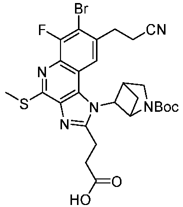

A sample of *tert*-butyl (*endo*)-5-(7-bromo-8-(2-cyanoethyl)-6-fluoro-2-(3-methoxy-3-oxopropyl)-4-(methylthio)-1*H*-imidazo[4,5-*c*]quinolin-1-yl)-2-azabicyclo[2.1.1]hexane-2-carboxylate (903 mg, 1.43 mmol) was dissolved in dioxane (9.5 mL) and water (4.8 mL) and stirred at room temperature. The solution was treated with lithium hydroxide (103 mg, 4.28 mmol). After 2 hours, LCMS indicated complete conversion to the product.

The reaction was quenched with satd. aq. NH₄Cl and diluted with EtOAc. The layers were separated, and the aqueous layer was extracted with additional EtOAc. The combined organic layers were dried over MgSO₄, filtered, and concentrated *in vacuo.* The crude product 3-(7-bromo-1-((*endo*)-2-(*tert*-butoxycarbonyl)-2-azabicyclo[2.1.1]hexan-5-yl)-8-(2-cyanoethyl)-6-fluoro-4-(methylthio)-1*H*-imidazo[4,5-*c*]quinolin-2-yl)propanoic acid (830 mg, 1.342 mmol, 94% yield) was used without further purification. LCMS calculated for C₂₇H₃₀BrFN₅O₄S (M+H)⁺: m/z = 618.1, 620.1; found: 618.2, 620.1.

### Step 9: tert-Butyl (endo)-5-(7-bromo-8-(2-cyanoethyl)-2-(3-(dimethylamino)-3-oxopropyl)-6-fluoro-4-(methylthio)-1H-imidazo[4,5-c]quinolin-1-yl)-2-azabicyclo[2.1.1]hexane-2-carboxylate

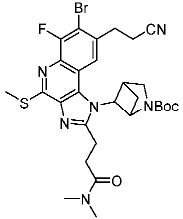

A sample of 3-(7-bromo-1-((*endo*)-2-(*tert*-butoxycarbonyl)-2-azabicyclo[2.1.1]hexan-5-yl)-8-(2-cyanoethyl)-6-fluoro-4-(methylthio)-1*H*-imidazo[4,5-*c*]quinolin-2-yl)propanoic acid (830 mg, 1.342 mmol) was dissolved in DMF (19 mL) and stirred at room temperature. The solution was treated with *N,N*-diisopropylethylamine (1.2 mL, 6.71 mmol) and HATU (2.0 g, 5.37 mmol), and warmed to 65 °C. After 15 minutes, the solution had turned red, and it was treated with 2M dimethylamine (3.4 mL, 6.8 mmol) as a solution in THF. The solution was stirred again at 65 °C. After 30 mins, LCMS indicated complete conversion to the product.

The reaction was cooled to RT, quenched with satd. aq. NH₄Cl and diluted with EtOAc. The layers were separated, and the aqueous layer was extracted with additional EtOAc, then once with DCM. The combined organic layers were dried over MgSO₄, filtered, and concentrated *in vacuo.* The crude material was purified by prep SFC to provide *tert*-butyl (*endo*)-5-(7-bromo-8-(2-cyanoethyl)-2-(3-(dimethylamino)-3-oxopropyl)-6-fluoro-4-(methylthio)-1*H*-imidazo[4,5-*c*]quinolin-1-yl)-2-azabicyclo[2.1.1]hexane-2-carboxylate (541 mg, 0.838 mmol, 62% yield). LCMS calculated for C₂₉H₃₅BrFNₑO₃S (M+H)⁺: m/z = 645.2, 647.2; found: 645.2, 647.2.

### Step 10: tert-Butyl (endo)-5-(8-(2-cyanoethyl)-2-(3-(dimethylamino)-3-oxopropyl)-6-fluoro-7-(3-(methoxymethoxy)naphthalen-1-yl)-4-(methylthio)-1H-imidazo[4,5-c]quinolin-1-yl)-2-azabicyclo[2.1.1]hexane-2-carboxylate

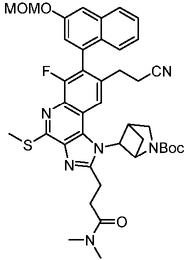

A sample of tert-butyl (endo)-5-(7-bromo-8-(2-cyanoethyl)-2-(3-(dimethylamino)-3-oxopropyl)-6-fluoro-4-(methylthio)-1*H*-imidazo[4,5-*c*]quinolin-1-yl)-2-azabicyclo[2.1.1]hexane-2-carboxylate (541 mg, 0.838 mmol) was dissolved in dioxane (6.7 mL) and water (1.7 mL) and stirred at room temperature. The solution was treated with potassium carbonate (347 mg, 2.51 mmol) and 2-(3-(methoxymethoxy)naphthalen-1-yl)-4,4,5,5-tetramethyl-1,3,2-dioxaborolane (922 mg, 2.93 mmol, see **Steps 1-2** of this example for preparation). The solution was de-gassed by bubbling with nitrogen and sonicating for 5 minutes. Finally, the solution was treated with chloro(2-dicyclohexylphosphino-2',4',6'-triisopropyl-1,1'-biphenyl)[2-(2'-amino-1,1'-biphenyl)]palladium(II) (99 mg, 0.126 mmol) and stirred at 65 °C. After 90 mins, LCMS indicated complete conversion to the product.

The reaction was cooled to RT, quenched with satd. aq. NH₄Cl and diluted with EtOAc several times. The layers were separated, and the aqueous layer was extracted with 25% IPA/CHCl₃. The combined organic layers were dried over MgSO₄, filtered, and concentrated *in vacuo.* The residue was purified by prep SFC to provide *tert*-butyl (*endo*)-5-(8-(2-cyanoethyl)-2-(3-(dimethylamino)-3-oxopropyl)-6-fluoro-7-(3-(methoxymethoxy)-naphthalen-1-yl)-4-(methylthio)-1*H*-imidazo[4,5-*c*]quinolin-1-yl)-2-azabicyclo[2.1.1]hexane-2-carboxylate (372 mg, 0.494 mmol, 59% yield). LCMS calculated for C₄₁H₄₆FN₆O₅S (M+H)⁺: m/z = 753.3; found: 753.4.

### Step 11: 5-(1-((endo)-2-azabicyclo[2. 1. 1]hexan-5-yl)-8-(2-cyanoethyl)-2-(3-(dimethylamino)-3-oxopropyl)-6-fluoro-7-(3-hydroxynaphthalen-1-yl)-1H-imidazo[4,5-c]quinolin-4-yl)-2-fluoro-N-methylbenzamide

A sample of *tert*-butyl 5-(8-(2-cyanoethyl)-2-(3-(dimethylamino)-3-oxopropyl)-6-fluoro-7-(3-(methoxymethoxy)naphthalen-1-yl)-4-(methylthio)-1*H*-imidazo[4,5-*c*]quinolin-1-yl)-2-azabicyclo[2.1.1]hexane-2-carboxylate (22 mg, 0.029 mmol) was dissolved in dioxane (0.15 mL) in a vial with a stir bar and stirred at room temperature. The solution was treated with (4-fluoro-3-(methylcarbamoyl)phenyl)boronic acid (17 mg, 0.088 mmol) and copper(I) thiophene-2-carboxylate (16 mg, 0.082 mmol). Lastly, chloro(2-dicyclohexylphosphino-2',4',6'-triisopropyl-1,1'-biphenyl)[2-(2'-amino-1,1'-biphenyl)]palladium(II) (2 mg, 3 µmol) was added, the vial was capped, and the solution was stirred at 100 °C. After 1 hour, LCMS indicated the reaction had stalled at ~50% conversion.

The reaction was cooled to RT, quenched with satd. aq. NH₄Cl and diluted with DCM. The layers were separated, and the aqueous layer was extracted with additional DCM. The aqueous phase was extracted once more with 25% IPA in CHCl3. The combined organic layers were dried over MgSO₄, filtered, and concentrated *in vacuo.*

The crude intermediate *tert*-butyl (*endo*)-5-(8-(2-cyanoethyl)-2-(3-(dimethylamino)-3-oxopropyl)-6-fluoro-4-(4-fluoro-3-(methylcarbamoyl)phenyl)-7-(3-(methoxymethoxy)-naphthalen-1-yl)-1*H*-imidazo[4,5-*c*]quinolin-1-yl)-2-azabicyclo[2.1.1]hexane-2-carboxylate was dissolved in 0.5 mL DCM and treated with 0.5 mL trifluoroacetic acid. The mixture was stirred for 1 hour, at which point LCMS indicated complete conversion to the desired product. The mixture was concentrated *in vacuo* without heating. The remaining solution was diluted with 4:1 acetonitrile/water, filtered through a SiliaPrep Thiol cartridge, and purified by HPLC (pH = 2 method) to give 5-(1-((endo)-2-azabicyclo[2.1.1]hexan-5-yl)-8-(2-cyanoethyl)-2-(3-(dimethylamino)-3-oxopropyl)-6-fluoro-7-(3-hydroxynaphthalen-1-yl)-1*H*-imidazo[4,5-*c*]quinolin-4-yl)-2-fluoro-N-methylbenzamide (0.7 mg, 1 µmol, 3% yield) as a mixture of diastereomers. LCMS calculated for intermediate C₄₈H₅₀F₂N₇O₆ (M+H)⁺: m/z = 858.4; found: 858.4. LCMS calculated for title compound C₄₁H₃₈F₂N₇O₃ (M+H)⁺: m/z = 714.3; found: 714.2.

### Example 298. 3-(1-((endo)-2-azabicyclo[2.1.1]hexan-5-yl)-8-(2-cyanoethyl)-6-fluoro-7-(3-hydroxynaphthalen-1-yl)-4-(1-methyl-6-oxo-1,6-dihydropyridin-3-yl)-1H-imidazo[4,5-c]quinolin-2-yl)-N,N-dimethylpropanamide

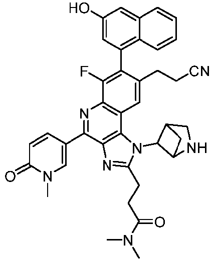

This compound was prepared according to the procedure described in **Example 297, Step 11,** replacing (4-fluoro-3-(methylcarbamoyl)phenyl)boronic acid with 1-methyl-5-(4,4,5,5-tetramethyl-1,3,2-dioxaborolan-2-yl)pyridin-2(1*H*)-one. The product 3-(1-((*endo*)-2-azabicyclo[2.1.1]hexan-5-yl)-8-(2-cyanoethyl)-6-fluoro-7-(3-hydroxynaphthalen-1-yl)-4-(1-methyl-6-oxo-1,6-dihydropyridin-3-yl)-1*H*-imidazo[4,5-*c*]quinolin-2-yl)-*N,N-*dimethylpropanamide (1.2 mg, 1.8 µmol, 7% yield) was isolated as a mixture of diastereomers. LCMS calculated for intermediate C₄₆H₄₉FN₇O₆ (M+H)⁺: m/z = 814.4; found: 814.4. LCMS calculated for title compound C₃₉H₃₇FN₇O₃ (M+H)⁺: m/z = 670.3; found: 670.2.

### Example 299. 3-(1-((endo)-2-azabicyclo[2.1.1]hexan-5-yl)-8-(2-cyanobenzyl)-4-(3-(dimethylamino)azetidin-1-yl)-6-fluoro-7-(3-hydroxynaphthalen-1-yl)-1H-imidazo[4,5-c]quinolin-2-yl)-N,N-dimethylpropanamide

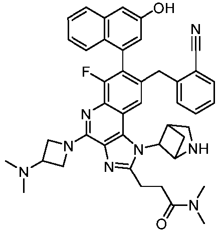

### Step 1. tert-butyl (endo)-5-(7-bromo-8-(2-cyanobenzyl)-2-(3-(dimethylamino)-3-oxopropyl)-4-(3-(dimethylamino)azetidin-1-yl)-6-fluoro-1H-imidazo[4, 5-c]quinolin-1-yl)-2-azabicyclo[2.1.1]hexane-2-carboxylate

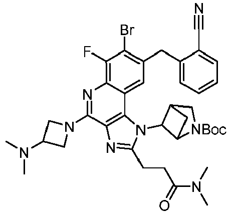

To a vial containing neat *tert*-butyl (*endo*)-5-(7-bromo-2-(3-(dimethylamino)-3-oxopropyl)-4-(3-(dimethylamino)azetidin-1-yl)-6-fluoro-8-iodo-1*H*-imidazo[4,5-*c*]quinolin-1-yl)-2-azabicyclo[2.1.1]hexane-2-carboxylate (**Examples 291a/b, Step 3,** 100 mg, 0.13 mmol) was added a THF solution of (2-cyanobenzyl)zinc(II) chloride (0.5 M solution in THF, 1.3 mL, 0.65 mmol). The mixture was stirred at room temperature until homogeneous, added bis(triphenylphosphine)palladium(II) chloride (9 mg, 0.013 mmol), degassed with N₂ for 5 min before heated at 50 °C for 1h. After completion, the reaction was quenched with the addition of saturated NH₄Cl solution (5 mL), and extracted with EtOAc (10 mL). The organic phase was concentrated, and the crude product was purified by flash column chromatography (MeOH/DCM: 0-40%) to provide a yellow solid (81 mg, 82%). LCMS calculated for C₃₃H₄₄BrFN₈O₃ (M+H)⁺: m/z = 759.2; found 759.2.

### Step 2. 3-(1-((endo)-2-azabicyclo[2.1.1]hexan-5-yl)-8-(2-cyanobenzyl)-4-(3-(dimethylamino)azetidin-1-yl)-6-fluoro-7-(3-hydroxynaphthalen-1-yl)-1H-imidazo[4, 5-c]quinolin-2-yl)-N,N-dimethylpropanamide

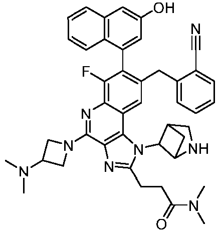

To a vial containing *tert*-butyl (endo)-5-(7-bromo-8-(2-cyanobenzyl)-2-(3-(dimethylamino)-3-oxopropyl)-4-(3-(dimethylamino)azetidin-1-yl)-6-fluoro-1*H*-imidazo[4,5-*c*]quinolin-1-yl)-2-azabicyclo[2.1.1]hexane-2-carboxylate (81 mg, 0.11 mmol) in dioxane (1 mL) and water (0.2 mL) was added XPhos Pd G2 (10.2 mg, 0.013 mmol) and K₃PO₄ (83 mg, 0.39 mmol). The reaction mixture was degassed with N₂ for 5 min before heated to 100 °C for 1h. After completion, the reaction was quenched with the addition of saturated NH₄Cl solution (2 mL), and extracted with EtOAc (5 mL). The organic phase was concentrated, and the crude product was added TFA (0.5 mL) and stirred at rt for 30 min. The mixture was purified using prep-LCMS (XBridge C18 column, eluting with a gradient of acetonitrile/water containing 0.1% TFA, at flow rate of 60 mL/min) to afford the desired product as a TFA salt. A pair of diastereomers were separated with Peak 1 being more potent. LCMS calculated for C₄₃H₄₄FN₈O₂ (M+H)⁺: m/z = 723.4; found: 723.4.

### Examples 300a/b. 3-(1-((endo)-2-azabicyclo[2.1.1]hexan-5-yl)-4-(3-(dimethylamino)azetidin-1-yl)-6-fluoro-7-(3-hydroxynaphthalen-1-yl)-2-((3-oxomorpholino)methyl)-1H-imidazo[4,5-c]quinolin-8-yl)propanenitrile

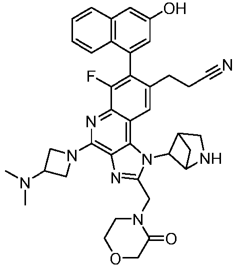

### Step 1. tert-butyl (1R,4R,5R)-5-((3-amino-7-bromo-6-(2-cyanoethyl)-2-(3-(dimethylamino)azetidin-1-yl)-8-fluoroquinolin-4-yl)amino)-2-azabicyclo[2.1.1]hexane-2-carboxylate

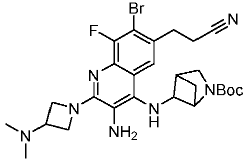

In a 1 dram vial tert-butyl (1R,4R,5R)-5-((3-amino-7-bromo-2-(3-(dimethylamino)-azetidin-1-yl)-8-fluoro-6-iodoquinolin-4-yl)amino)-2-azabicyclo[2.1.1]hexane-2-carboxylate **(Examples 291a/b, Step 2,** 1.17 g, 1.76 mmol), acrylonitrile (468 mg, 8.82 mmol) and aq. tetramethylammonium formate (25%, 1.24 mL, 2.65 mmol) in DMF (3.5 mL) was added DIPEA (616 µL, 3.53 mmol) and Pd(Ph₃P)₄ (204 mg, 0.176 mmol). The reaction mixture was heated at 80 °C for 2 h, then concentrated to dryness and added to a silica gel column and was eluted with methanol/dichloromethane from 0% to 5% to give tert-butyl (1R,4R,5R)-5-((3-amino-7-bromo-6-(2-cyanoethyl)-2-(3-(dimethylamino)azetidin-1-yl)-8-fluoroquinolin-4-yl)amino)-2-azabicyclo[2.1.1]hexane-2-carboxylate (782 mg, 70% yield). LCMS calculated for C₂₇H₃₆BrFN₇O₂ (M+H)⁺: m/z = 588.2/590.2; found 588.2/590.2.

### Step 2. tert-butyl (1R,4R,5R)-5-((3-amino-6-(2-cyanoethyl)-2-(3-(dimethylamino)azetidin-1-yl)-8-fluoro-7-(3-hydroxynaphthalen-1-yl)quinolin-4-yl)amino)-2-azabicyclo[2. 1. 1]hexane-2-carboxylate

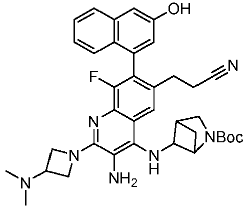

To a solution of tert-butyl (1R,4R,5R)-5-((3-amino-7-bromo-6-(2-cyanoethyl)-2-(3-(dimethylamino)azetidin-1-yl)-8-fluoroquinolin-4-yl)amino)-2-azabicyclo[2.1.1]hexane-2-carboxylate (1.04 g, 1.76 mmol) in water (5 mL) and dioxane (10 mL), (3-hydroxy-naphthalen-1-yl)boronic acid (763 mg, 4.06 mmol), Pd(Ph₃P)₄ (204 mg, 0.176 mmol) and Na₂CO₃ (374 mg, 3.53 mmol) were added. The reaction mixture was heated at 100 °C for 4 h. H₂O (20 mL) was added to the reaction mixture followed by extraction with DCM (20 mL x 3) and then the combined organic layers were washed with H₂O (20 mL), dried with Na₂SO₄, filtered and concentrated. The crude product was added to a silica gel column and was eluted with methanol/dichloromethane from 0% to 5% to give tert-butyl (1R,4R,5R)-5-((3-amino-6-(2-cyanoethyl)-2-(3-(dimethylamino)azetidin-1-yl)-8-fluoro-7-(3-hydroxynaphthalen-1-yl)quinolin-4-yl)amino)-2-azabicyclo[2.1.1]hexane-2-carboxylate (932 mg, 81.1 % yield). LCMS calculated for C₃₇H₄₃FN₇O₃ (M+H)⁺: m/z = 652.3; found 652.2.

### Step 3. tert-butyl (1R,4R,5S)-5-(2-(chloromethyl)-8-(2-cyanoethyl)-4-(3-(dimethylamino)azetidin-1-yl)-6-fluoro-7-(3-hydroxynaphthalen-1-yl)-1H-imidazo[4, 5-cjquinolin-1-yl)-2-azabicyclo[2.1.1]hexane-2-carboxylate

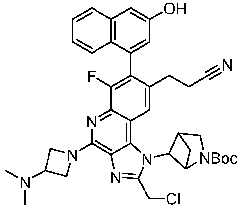

In a 1 dram vial tert-butyl (1R,4R,5R)-5-((3-amino-6-(2-cyanoethyl)-2-(3-(dimethylamino)azetidin-1-yl)-8-fluoro-7-(3-hydroxynaphthalen-1-yl)quinolin-4-yl)amino)-2-azabicyclo[2.1.1]hexane-2-carboxylate (774 mg, 0.891 mmol) and 2-chloro-1,1,1-triethoxyethane (525 mg, 2.67 mmol) in acetic acid (4.5 mL) was stirred at 100 °C for 0.5 h. The reaction mixture was concentrated and diluted with dichloromethane. Saturated NaHCO₃ (5 mL) was added to the reaction mixture followed by extraction with DCM (5 mL x 3). The combined organic layers were dried Na₂SO₄, filtered and concentrated. The crude product was added to a silica gel column and was eluted with methanol/dichloromethane from 0% to 5% to give tert-butyl (1R,4R,5S)-5-(2-(chloromethyl)-8-(2-cyanoethyl)-4-(3-(dimethylamino)azetidin-1-yl)-6-fluoro-7-(3-hydroxynaphthalen-1-yl)-1H-imidazo[4,5-c]quinolin-1-yl)-2-azabicyclo[2.1.1]hexane-2-carboxylate (440 mg, 69.6 % yield) as a yellow foam. LCMS calculated for C₃₉H₄₂ClFN₇O₃ (M+H)⁺: m/z = 710.3; found 710.3.

### Step 4. 3-(1-((1R,4R,5S)-2-azabicyclo[2.1.1]hexan-5-yl)-4-(3-(dimethylamino)azetidin-1-yl)-6-fluoro-7-(3-hydroxynaphthalen-1-yl)-2-((3-oxomorpholino)methyl)-1H-imidazo[4,5-c]quinolin-8-yl)propanenitrile

In a 1 dram vial 2.5 M ⁿBuLi in hexanes (56.3 µL, 0.141 mmol) was added to morpholin-3-one (16.5 mg, 0.141 mmol) in THF (0.5 mL) at 0 °C. After 15 min, tert-butyl (1R,4R,5S)-5-(2-(chloromethyl)-8-(2-cyanoethyl)-4-(3-(dimethylamino)azetidin-1-yl)-6-fluoro-7-(3-hydroxynaphthalen-1-yl)-1*H*-imidazo[4,5-*c*]quinolin-1-yl)-2-azabicyclo[2.1.1]hexane-2-carboxylate (20 mg, 0.028 mmol) in THF (0.5 mL) was added. The resulted mixture was stirred at 60 °C for another 5 h. Then conc. HCl (50 uL) was added. After 30 min, the mixture was diluted with acetonitrile/water and purified using prep-LCMS (XBridge C18 column, eluting with a gradient of acetonitrile/water containing 0.1% TFA, at flow rate of 60 mL/min) to afford the desired products as a TFA salt. The product was isolated as a pair of diastereomers.

**Example 300a.** Diastereomer 1. Peak 1. LCMS calculated for C₃₈H₄₀FN₈O₃ (M+H)⁺ m/z = 675.3; found 675.2. *Potent peak.

**Example 300b.** Diastereomer 2. Peak 2. LCMS calculated for C₃₈H₄₀FN₈O₃ (M+H)⁺ m/z = 675.3; found 675.2.

### Examples 301a/b. 3-(1-((endo)-2-azabicyclo[2.1.1]hexan-5-yl)-8-(2-cyanoethyl)-4-(3-(dimethylamino)azetidin-1-yl)-6-fluoro-7-(3-hydroxynaphthalen-1-yl)-1H-imidazo[4,5-c]quinolin-2-yl)-N-methyl-N-(pyridin-2-ylmethyl)propanamide

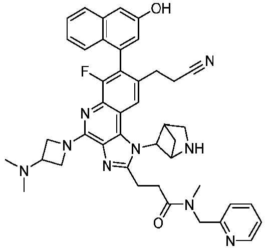

### Step 1. tert-butyl (1R,4R,5R)-5-((3-amino-6-((E)-2-cyanovinyl)-2-(3-(dimethylamino)azetidin-1-yl)-8-fluoro-7-(3-(methoxymethoxy)naphthalen-1-yl)quinolin-4-yl)amino)-2-azabicyclo[2.1.1]hexane-2-carboxylate

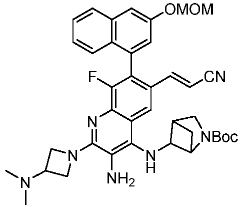

In a 1 dram vial a solution of tert-butyl (1R,4R,5R)-5-((3-amino-7-bromo-2-(3-(dimethylamino)azetidin-1-yl)-8-fluoro-6-iodoquinolin-4-yl)amino)-2-azabicyclo[2.1.1]hexane-2-carboxylate (**Examples 291a/b, Step 2,** 1.59 g, 2.40 mmol), acrylonitrile (230 mg, 4.33 mmol) and triethylamine (670 µL, 4.81 mmol) in DMF (12 mL) was added PdOAc₂ (54.0 mg, 0.240 mmol) and tri-o-tolylphosphine (146 mg, 0.481 mmol). The reaction mixture was heated at 80 °C for 2 h. Then the reaction was diluted with water (10 mL) and dioxane (10 mL). (3-(methoxymethoxy)naphthalen-1-yl)boronic acid (**Example 297, Step 2,** 1.12 g, 4.81 mmol), Pd(Ph₃P)₄ (278 mg, 0.240 mmol) and Na₂CO₃ (510 mg, 4.81 mmol) were added. The reaction mixture was heated at 105 °C for 15 h. Then H₂O (50 mL) was added to the reaction mixture followed by extraction with dichloromethane (50 mL x 3) and then the combined organic layers were washed with H₂O (50 mL), dried with Na₂SO₄, filtered and concentrated. The crude product was added to a silica gel column and was eluted with methanol/dichloromethane from 0% to 10% to give tert-butyl (1R,4R,5R)-5-((3-amino-6-((E)-2-cyanovinyl)-2-(3-(dimethylamino)azetidin-1-yl)-8-fluoro-7-(3-(methoxymethoxy)naphthalen-1-yl)quinolin-4-yl)amino)-2-azabicyclo[2.1.1]hexane-2-carboxylate (1.25 g, 75 % yield) as a yellow solid. LCMS calculated for C₃₉H₄₅FN₇O₄ (M+H)⁺: m/z = 694.4; found 694.3.

### Step 2. 3-(1-((1R,4R,5S)-2-(tert-butoxycarbonyl)-2-azabicyclo[2.1.1]hexan-5-yl)-8-(2-cyanoethyl)-4-(3-(dimethylamino)azetidin-1-yl)-6-fluoro-7-(3-(methoxymethoxy)naphthalen-1-yl)-1H-imidazo[4,5-c]quinolin-2-yl)propanoic acid

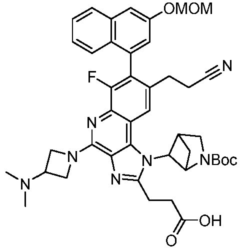

**Part A:** In a 1 dram vial tert-butyl (1R,4R,5R)-5-((3-amino-6-((E)-2-cyanovinyl)-2-(3-(dimethylamino)azetidin-1-yl)-8-fluoro-7-(3-(methoxymethoxy)naphthalen-1-yl)quinolin-4-yl)amino)-2-azabicyclo[2.1.1]hexane-2-carboxylate (2.0 g, 2.88 mmol) in THF (14 mL) was added L-selectride (5.77 ml, 1 M in THF, 5.77 mmol) dropwise at 0 °C for 1 h. Then H₂O (30 mL) was added to the reaction mixture followed by extraction with dichloromethane (30 mL x 3). The combined organic layers were dried Na₂SO₄, filtered and concentrated. The crude product was used directly without further purification.

**Part B:** To a solution of the above crude product in EtOH (20 mL) was added methyl 4-oxobutanoate (1.0 g, 8.65 mmol). The reaction mixture was stirred at 60 °C for 18 h. Then LiOH (0.414 g, 17.30 mmol) in water (2 mL) was added. After another 1 h, the reaction mixture was diluted with MeOH then purified by prep-HPLC (pH = 2, acetonitrile/water+TFA) to give the desired product 3-(1-((1R,4R,5S)-2-(tert-butoxycarbonyl)-2-azabicyclo[2.1.1]hexan-5-yl)-8-(2-cyanoethyl)-4-(3-(dimethylamino)azetidin-1-yl)-6-fluoro-7-(3-(methoxymethoxy)naphthalen-1-yl)-1H-imidazo[4,5-c]quinolin-2-yl)propanoic acid (270 mg, 12.0 % yield) as the TFA salt. LCMS calculated for C₄₃H₄₉FN₇O₆ (M+H)⁺: m/z = 778.4; found 778.3.

### Step 3. 3-(1-((1R,4R,5S)-2-azabicyclo[2.1.1]hexan-5-yl)-8-(2-cyanoethyl)-4-(3-(dimethylamino)azetidin-1-yl)-6-fluoro-7-(3-hydroxynaphthalen-1-yl)-1H-imidazo[4,5-c]quinolin-2-yl)-N-methyl-N-(pyridin-2-ylmethyl)propanamide

In a 1 dram vial 3-(1-((1R,4R,5S)-2-(tert-butoxycarbonyl)-2-azabicyclo[2.1.1]hexan-5-yl)-8-(2-cyanoethyl)-4-(3-(dimethylamino)azetidin-1-yl)-6-fluoro-7-(3-(methoxymethoxy)-naphthalen-1-yl)-1H-imidazo[4,5-c]quinolin-2-yl)propanoic acid (10 mg, 0.013 mmol), N-methyl-1-(pyridin-2-yl)methanamine (0.32 mg, 0.026 mmol) and DIPEA (6.74 µL, 0.039 mmol) in DMF (0.5 mL) was added BOP (7.4 mg, 0.017 mmol). The reaction mixture was stirred for 1 h. Then conc. HCl (50 uL) was added. After 30 min, the mixture was diluted with acetonitrile/water and purified using prep-LCMS (XBridge C18 column, eluting with a gradient of acetonitrile/water containing 0.1% TFA, at flow rate of 60 mL/min) to afford the desired products as a TFA salt. The product was isolated as a pair of diastereomers.

**Example 301a.** Diastereomer 1. Peak 1. LCMS calculated for C₄₃H₄₅FN₉O₂ (M+H)⁺ m/z = 738.4; found 738.3. This peak is the potent peak.

**Example 301b.** Diastereomer 2. Peak 2. LCMS calculated for C₄₃H₄₅FN₉O₂ (M+H)⁺ m/z = 738.4; found 738.3.

### Example 302a and Example 302b. 3-(1-((endo)-2-azabicyclo[2.1.1]hexan-5-yl)-4-(3-(dimethylamino)azetidin-1-yl)-6-fluoro-7-(3-hydroxynaphthalen-1-yl)-2-(2-(piperazin-1-yl)thiazol-4-yl)-1H-imidazo[4,5-c]quinolin-8-yl)propanenitrile

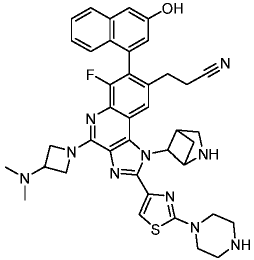

### Step 1: tert-Butyl (endo)-5-(2-(2-bromothiazol-4-yl)-8-(2-cyanoethyl)-4-(3-(dimethylamino)azetidin-1-yl)-6-fluoro-7-(3-hydroxynaphthalen-1-yl)-1H-imidazo[4, 5-c]quinolin-1-yl)-2-azabicyclo[2.1.1]hexane-2-carboxylate

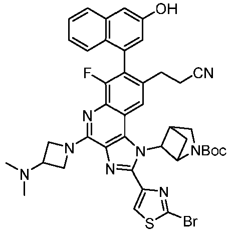

To a solution of *tert*-butyl (endo)-5-((3-amino-6-(2-cyanoethyl)-2-(3-(dimethylamino)-azetidin-1-yl)-8-fluoro-7-(3-hydroxynaphthalen-1-yl)quinolin-4-yl)amino)-2-azabicyclo[2.1.1]-hexane-2-carboxylate (**Examples 300a/b, Step 2,** 100 mg, 0.15 mmol) in EtOH (2 mL) was added 2-bromothiazole-4-carbaldehyde (30 mg, 0.15 mmol). The reaction mixture was stirred at room temperature for 3 days. The resultant mixture was concentrated and purified by flash column chromatography (Agela Flash Column Silica-CS (12 g), eluting with a gradient of 0 to 20% CH₂Cl₂/methanol) to afford tert-butyl (*endo*)-5-(2-(2-bromothiazol-4-yl)-8-(2-cyanoethyl)-4-(3-(dimethylamino)azetidin-1-yl)-6-fluoro-7-(3-hydroxynaphthalen-1-yl)-1*H*-imidazo[4,5-c]quinolin-1-yl)-2-azabicyclo[2.1.1]hexane-2-carboxylate (80 mg, 0.097 mmol, 63% yield). LCMS calculated for C₄₁H₄₁BrFN₈O₃S (M+H)⁺: m/z = 823.2; found 823.2.

### Step 2: 3-(1-((endo)-2-Azabicyclo[2.1.1]hexan-5-yl)-4-(3-(dimethylamino)azetidin-1-yl)-6-fluoro-7-(3-hydroxynaphthalen-1-yl)-2-(2-(piperazin-1-yl)thiazol-4-yl)-1H-imidazo[4,5-c]quinolin-8-yl)propanenitrile

To a solution of tert-butyl (*endo*)-5-(2-(2-bromothiazol-4-yl)-8-(2-cyanoethyl)-4-(3-(dimethylamino)azetidin-1-yl)-6-fluoro-7-(3-hydroxynaphthalen-1-yl)-1H-imidazo[4,5-c]quinolin-1-yl)-2-azabicyclo[2.1.1]hexane-2-carboxylate (20 mg, 0.024 mmol) and N,N-diisopropylethylamine (85 µL, 0.49 mmol) in EtOH (2 mL) was added 1-boc-piperazine (90 mg, 0.49 mmol). The reaction flask was sparged with nitrogen and the mixture was stirred at 100 °C for 48 hours. After concentrated under reduced pressure, the mixture was dissolved in EtOH (2 mL), followed by the addition of a 4M solution of HCl in dioxane (0.5 mL, 2 mmol). The solution was stirred at 40 °C for 3 hours. The material obtained was diluted with acetonitrile/water and purified using prep-LCMS (Sunfire C18 column, eluting with a gradient of acetonitrile/water containing 0.1% TFA, at flow rate of 60 mL/min) to afford the desired products as a TFA salt.

**Example 302a.** Diastereomer 1. Peak 1. LCMS calculated for C₄₀H₄₂FN₁₀OS (M+H)⁺: m/z = 729.3; found 729.3. Peak 1 is the potent compound.

**Example 302b.** Diastereomer 2. Peak 2. LCMS calculated for C₄₀H₄₂FN₁₀OS (M+H)⁺: m/z = 729.3; found 729.3.

Table A below lists additional compounds prepared in a similar manner to the procedures provided above.

**Table A**

| Example | Chemical name | Structure | Reference procedure | Mass found M+H |
|---|---|---|---|---|
| 81a and 81b | 2-((2*S*,4*S*)-1-(but-2-ynoyl)-4-(8-chloro-7-(6-chloro-5-methyl-1*H*-indazol-4-yl)-4-(3-(dimethyl-amino)-3-methylazetidin-1-yl)-6-fluoro-1*H*-imidazo[4,5-*c*]quinolin-1-yl)piperidin-2-yl)acetonitrile | 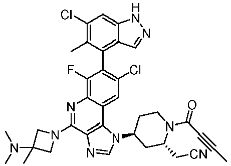 | Example 80a | 686.3 |
| 82a and 82b | 2-((2*S*,4*S*)-1-(but-2-ynoyl)-4-(8-chloro-7-(6-chloro-5-methyl-1*H*-indazol-4-yl)-4-(3-(dimethylamino)-3-methylazetidin-1-yl)-6-fluoro-1*H*-imidazo[4,5-*c*]quinolin-1-yl)piperidin-2-yl)acetonitrile | 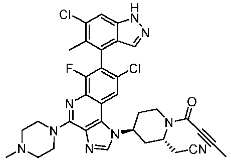 | Example 80a | 672.3 |
| 83a and 83b | 2-((2*S*,4*S*)-1-(but-2-ynoyl)-4-(8-chloro-7-(6-chloro-5-methyl-1*H*-indazol-4-yl)-6-fluoro-4-(5-methyl-2,5-diazaspiro[3.4]octan-2-yl)-1*H*-imidazo[4,5-*c*]quinolin-1-yl)piperidin-2-yl)acetonitrile | 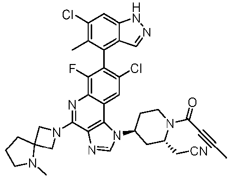 | Example 80a | 698.4 |
| 84a and 84b | 2-((2*S*,4*S*)-4-(8-chloro-7-(6-chloro-5-methyl-1*H*-indazol-4-yl)-6-fluoro-4-(5-methyl-2,5-diazaspiro[3.4]octan-2-yl)-1*H*-imidazo[4,5-*c*]quinolin-1-yl)-1-((*E*)-4-methoxybut-2-enoyl)piperidin-2-yl)acetonitrile | 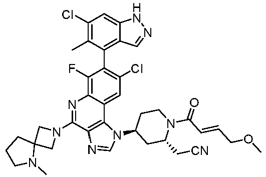 | Example 80a | 730.2 |
| 90 | 2-((2*S*,4*S*)-1-acryloyl-4-(8-chloro-7-(5-chloro-2-methoxy-4-methylpyridin-3-yl)-4-(3-(dimethylamino)azetidin-1-yl)-6-fluoro-1*H*-imidazo[4,5-*c*]quinolin-1-yl)piperidin-2-yl)acetonitrile | 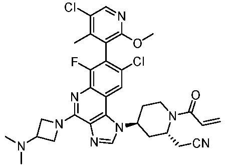 | Example 89 | 651.2 |
| | | | Example 85 | |
| 93 | 2-((2*S*,4*S*)-1-(but-2-ynoyl)-4-(8-chloro-7-(3-chloro-2-methylphenyl)-6-fluoro-4-(2-oxopiperidin-1-yl)-1*H-*[1,2,3]triazolo[4,5-*c*]quinolin-1-yl)piperidin-2-yl)acetonitrile | 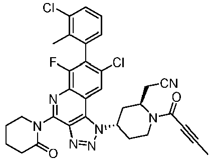 | Example 91 | 632.2 |
| 94 | 2-((2*S*,4*S*)-1-(but-2-ynoyl)-4-(8-chloro-7-(3-chloro-2-methylphenyl)-4-(((*S*)-1-(dimethylamino)propan-2-yl)oxy)-6-fluoro-1*H-*[1,2,3]triazolo[4,5-*c*]quinolin-1-yl)piperidin-2-yl)acetonitrile | 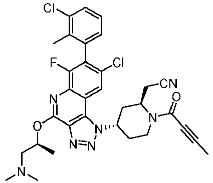 | Example 91 | 636.1 |
| 99 | 2-((2S,4S)-1-(but-2-ynoyl)-4-(7-(3-chloro-2-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile | 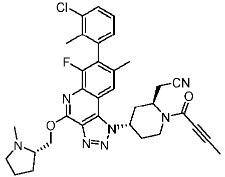 | Example 87a | 628.3 |
| 100 | 2-((2*S*,4*S*)-4-(7-(3-chloro-2-methylphenyl)-6-fluoro-8-methyl-4-(((*S*)-1-methylpyrrolidin-2-yl)methoxy)-1*H-*[1,2,3]triazolo[4,5-*c*]quinolin-1-yl)-1-((*E*)-4-methoxybut-2-enoyl)piperidin-2-yl)acetonitrile | 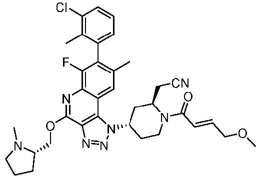 | Example 80a | 660.4 |
| 102 | 2-((2*S*,4*S*)-4-(7-(3-chloro-2-methylphenyl)-4-(3-(dimethylamino)-3-methylazetidin-1-yl)-6-fluoro-8-methyl-1*H*-[1 ,2,3]triazolo-[4,5-c]quinolin-1-yl)-1-((*E*)-4,4-difluorobut-2-enoyl)-piperidin-2-yl)acetonitrile | 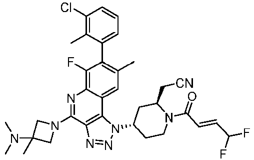 | Example 87a | 665.2 |
| 103 | 2-((2S*,*4*S*)-1-(but-2-ynoyl)-4-(7-(3-chloro-2-methylphenyl)-4-(3-(dimethylamino)-3-methylazetidin-1-yl)-6-fluoro-8-methyl-1*H-*[1,2,3]triazolo[4,5-*c*]quinolin-1-yl)piperidin-2-yl)acetonitrile | 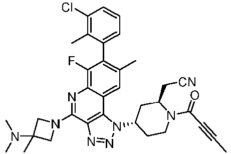 | Example 87a | 627.2 |
| 104 | 2-((2*S*,4*S*)-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)-1-((E)-4-fluorobut-2-enoyl)piperidin-2-yl)acetonitrile | 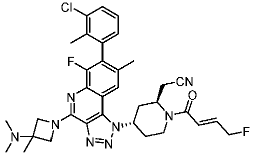 | Example 87a | 647.2 |
| 109 | 2-((2*S*,4*S*)-1-acryloyl-4-(8-chloro-4-(3-(dimethylamino)-azetidin-1-yl)-6-fluoro-7-(*m-*tolyl)-1*H*-imidazo[4,5-*c*]quinolin-1-yl)piperidin-2-yl)acetonitrile | 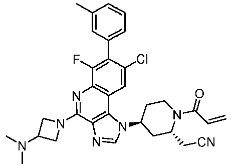 | Example 16 | 586.3 |
| 110a and 110b | 2-((2*S*,4*S*)-1-acryloyl-4-(8-chloro-4-(3-(dimethylamino)-azetidin-1-yl)-7-(4,5-dimethylpyridin-3-yl)-6-fluoro-1*H*-imidazo[4,5-*c*]quinolin-1-yl)piperidin-2-yl)acetonitrile | 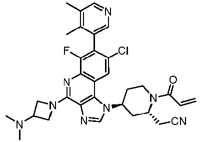 | Example 16 | 601.2 |
| 112 | 2-((2*S*,4*S*)-1-acryloyl-4-(8-chloro-7-(3-chloro-2-methylphenyl)-4-(3-(dimethylamino)azetidin-1-yl)-6-fluoro-1*H-*[1,2,3]triazolo[4,5-*c*]quinolin-1-yl)piperidin-2-yl)acetonitrile | 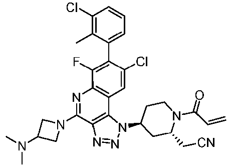 | Example 111 | 621.2 |
| 113 | 2-((2*S*,4*S*)-1-acryloyl-4-(8-chloro-7-(2,3-dichlorophenyl)-4-(3-(dimethylamino)azetidin-1-yl)-6-fluoro-1*H-*[1,2,3]triazolo[4,5-c]quinolin-1-yl)piperidin-2-yl)acetonitrile | 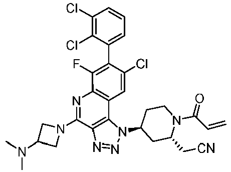 | Example 111 | 641.2 |
| 117 | 2-((2*S*,4*S*)-1-acryloyl-4-(8-chloro-7-(2-cyclopropylphenyl)-4-(3-(dimethylamino)azetidin-1-yl)-6-fluoro-1*H-*[1,2,3]triazolo[4,5-c]quinolin-1-yl)piperidin-2-yl)acetonitrile | 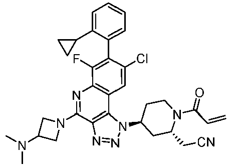 | Example 111 | 613.4 |
| 122 | 2-((2*S*,4*S*)-1-acryloyl-4-(8-chloro-4-(((*S*)-1-(dimethylamino)propan-2-yl)oxy)-6-fluoro-7-(4-fluorophenyl)-1*H-*imidazo[4,5-*c*]quinolin-1-yl)piperidin-2-yl)acetonitrile | 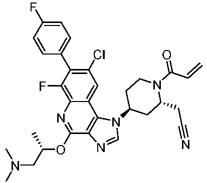 | Example 120 | 593.3 |
| 124 | 2-((2*S*,4*S*)-4-(8-chloro-6-fluoro-7-(4-fluorophenyl)-4-(2-methyl-1*H*-imidazol-1-yl)-1*H*-imidazo[4,5-*c*]quinolin-1-yl)-1-((*E*)-4-(dimethylamino)-but-2-enoyl)piperidin-2-yl)acetonitrile | 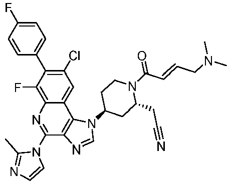 | Example 123 | 629.4 |
| 125 | 2-((2*S*,4*S*)-4-(8-chloro-6-fluoro-7-(4-fluorophenyl)-4-(2-methyl-1*H*-imidazol-1-yl)-1*H*-imidazo[4,5-*c*]quinolin-1-yl)-1-((*E*)-4-fluorobut-2-enoyl)piperidin-2-yl)acetonitrile | 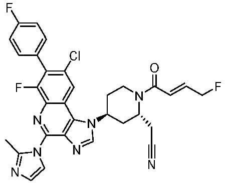 | Example 123 | 604.3 |
| 139 | 2-((2*S*,4*S*)-1-(but-2-ynoyl)-4-(7-(2,3-dimethylphenyl)-6-fluoro-8-methyl-4-(((*S*)-1-methylpyrrolidin-2-yl)methoxy)-1*H-*[1,2,3]triazolo[4,5-*c*]quinolin-1-yl)piperidin-2-yl)acetonitrile | 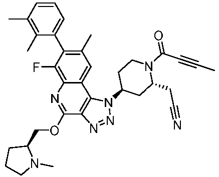 | Example 138 | 608.4 |
| 140 | 2-((2*S*,4*S*)-4-(7-(2,3-dimethylphenyl)-6-fluoro-8-methyl-4-(((*S*)-1-methylpyrrolidin-2-yl)methoxy)-1*H-*[1,2,3]triazolo[4,5-c]quinolin-1-yl)-1-((*E*)-4-methoxybut-2-enoyl)piperidin-2-yl)acetonitrile | 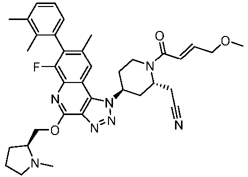 | Example 138 | 640.5 |
| 141 | 2-((2*S*,4*S*)-1-((*E*)-4,4-difluorobut-2-enoyl)-4-(7-(2,3-dimethylphenyl)-6-fluoro-8-methyl-4-(((*S*)-1-methylpyrrolidin-2-yl)methoxy)-1*H-*[1,2,3]triazolo[4,5-*c*]quinolin-1-yl)piperidin-2-yl)acetonitrile | 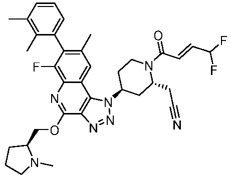 | Example 138 | 646.4 |
| 144 | 2-((2*S*,4*S*)-1-((E)-4,4-difluorobut-2-enoyl)-4-(4-(((*S*)-1-(dimethylamino)-propan-2-yl)oxy)-7-(2,3-dimethylphenyl)-6-fluoro-8-methyl-1*H*-[1 ,2,3]triazolo[4,5-*c*]quinolin-1-yl)piperidin-2-yl)acetonitrile | 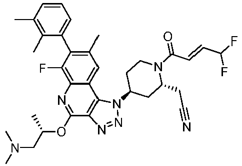 | Example 143 | 634.5 |
| 148 | 2-((2*S*,4*S*)-1-((E)-4,4-difluorobut-2-enoyl)-4-(4-(3-(dimethylamino)azetidin-1-yl)-7-(2,3-dimethylphenyl)-6-fluoro-8-methyl-1*H-*[1,2,3]triazolo[4,5-*c*]quinolin-1-yl)piperidin-2-yl)acetonitrile | 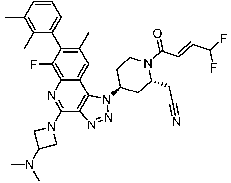 | Example 147 | 631.4 |
| 152 | 2-((2S,4S)-4-(8-chloro-6-fluoro-4-(2-methyl-1H-imidazol-1-yl)-7-phenyl-1 H-imidazo[4,5-c]quinolin-1-yl)-1-((E)-4-fluorobut-2-enoyl)piperidin-2-yl)acetonitrile | 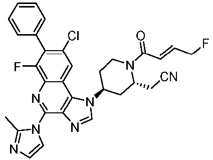 | Example 151 | 586.2 |
| 153 | 2-((2S,4S)-4-(8-chloro-6-fluoro-4-(2-methyl-1H-imidazol-1-yl)-7-phenyl-1H-[1,2,3]triazolo[4,5-c]quinolin-1-yl)-1-((E)-4-(dimethylamino)but-2-enoyl)piperidin-2-yl)acetonitrile | 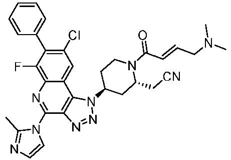 | Example 151 | 612.2 |
| 154 | 2-((2S,4S)-1-acryloyl-4-(8-chloro-4-(3-(dimethylamino)azetidin-1-yl)-6-fluoro-7-phenyl-1H-[1,2,3]triazolo[4,5-c]quinolin-1-yl)piperidin-2-yl)acetonitrile | 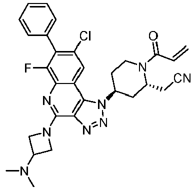 | Example 151 | 573.3 |
| 156 | 2-((2S,4S)-1-acryloyl-4-(6-fluoro-8-methyl-4-(2-methyl-1H-imidazol-1-yl)-7-phenyl-1H-[1,2,3]triazolo[4,5-c]quinolin-1-yl)piperidin-2-yl)acetonitrile | 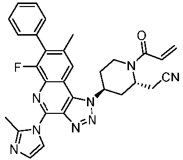 | Example 151 | 535.2 |
| 158a and 158b | 2-((2S,4S)-1-((E)-4-(dimethylamino)but-2-enoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile | 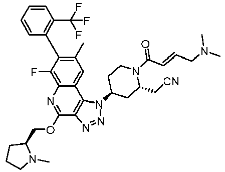 | Example 157 | 693.3 |
| 161a and 161b | 2-((2S,4S)-1-((E)-4,4-difluorobut-2-enoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile | 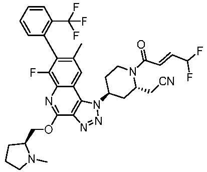 | Example 157 | 686.3 |
| 162a and 162b | 2-((2S,4S)-1-(but-2-ynoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile | 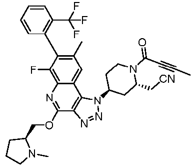 | Example 157 | 648.3 |
| 164a and 164b | 2-((2S,4S)-1-acryloyl-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)piperidin-2-yl)acetonitrile | 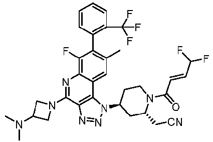 | Example 163 | 671.3 |
| 166a and 166b | 2-((2S,4S)-1-(but-2-ynoyl)-4-(4-(3-(dimethylamino)-azetidin-1-yl)-6-fluoro-8-methyl-7-(2-(trifluoromethyl)-phenyl)-1H-[1,2,3]triazolo-[4,5-c]quinolin-1-yl)piperidin-2-yl)acetonitrile | 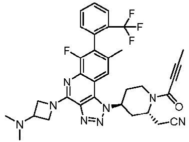 | Example 163 | 633.2 |
| 175 | 2-((2S,4S)-4-(8-chloro-4-(3-(dimethylamino)azetidin-1-yl)-6-fluoro-7-(isoquinolin-4-yl)-1H-imidazo[4,5-c]quinolin-1-yl)-1-((E)-4-(dimethylamino)but-2-enoyl)piperidin-2-yl)acetonitrile | 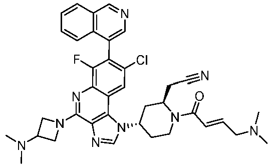 | Example 88 | 680.3 |
| | | | Example 89 | |
| 176 | 2-((2S,4S)-4-(8-chloro-7-(6-chloro-5-methyl-1H-indazol-4-yl)-4-(3-(dimethylamino)-azetidin-1-yl)-6-fluoro-1H-imidazo[4,5-c]quinolin-1-yl)-1-((E)-4-(dimethylamino)but-2-enoyl)piperidin-2-yl)acetonitrile | 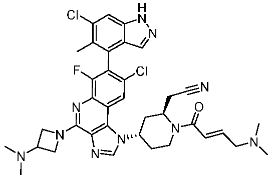 | Example 88 | 717.3 |
| | | | Example 89 | |
| 177a and 177b | 2-((2S,4S)-1-(but-2-ynoyl)-4-(8-chloro-7-(6-chloro-5-methyl-1H-indazol-4-yl)-4-(3-(dimethylamino)azetidin-1-yl)-6-fluoro-1H-imidazo[4,5-c]quinolin-1-yl)piperidin-2-yl)acetonitrile | 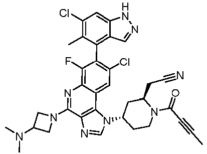 | Example 87a | 672.2 |
| 179a and 179b | 2-((2S,4S)-4-(8-chloro-7-(6-chloro-5-methyl-1H-indazol-4-yl)-4-(3-(dimethylamino)-azetidin-1-yl)-6-fluoro-1H-[1,2,3]triazolo[4,5-c]quinolin-1-yl)-1-((E)-4-(dimethylamino)but-2-enoyl)piperidin-2-yl)acetonitrile | 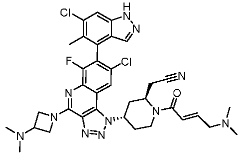 | Example 178 | 718.3 |
| 180a and 180b | 2-((2S,4S)-1-(but-2-ynoyl)-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)piperidin-2-yl)acetonitrile | 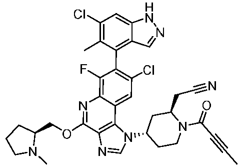 | Example 1a | 687.2 |
| | | | | 687.2 |
| | | | Example 80a | |
| | | | Example 87a | |
| 181a and 181b | 2-((2S,4S)-4-(8-chloro-7-(6-chloro-5-methyl-1H-indazol-4-yl)-6-fluoro-4-(((S)-1-methylpyrrolidin-2-yl)-methoxy)-1H-imidazo[4,5-c]quinolin-1-yl)-1-((E)-4-(dimethylamino)but-2-enoyl)piperidin-2-yl)acetonitrile | 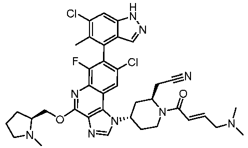 | Example 87a | 732.3 |
| 183a and 183b | 2-((2S,4S)-4-(8-chloro-7-(6-chloro-5-methyl-1H-indazol-4-yl)-6-fluoro-4-(((S)-1-methylpyrrolidin-2-yl)-methoxy)-1H-[1,2,3]triazolo-[4,5-c]quinolin-1-yl)-1-((E)-4-(dimethylamino)but-2-enoyl)piperidin-2-yl)acetonitrile | 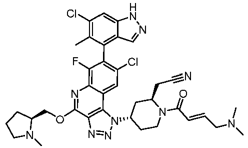 | Example 182 | 733.3 |
| 184a and 184b | 2-((2S,4S)-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)-1-((E)-4-methoxybut-2-enoyl)piperidin-2-yl)acetonitrile | 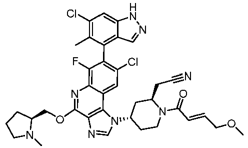 | Example 87a | 719.2 |
| 186a and 186b | 2-((2S,4S)-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)-1-((E)-4-methoxybut-2-enoyl)piperidin-2-yl)acetonitrile | 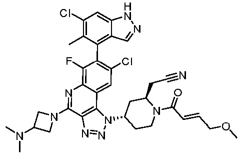 | Example 80a | 705.2 |
| | | | Example 178a | |
| 187 | 2-((2S,4S)-1-acryloyl-4-(8-chloro-6-fluoro-7-(4-fluoro-3-methylphenyl)-4-(((S)-1-methylpyrrolidin-2-yl)methoxy)-1H-[1,2,3]triazolo[4,5-c]quinolin-1-yl)piperidin-2-yl)acetonitrile | 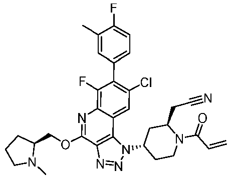 | Example 85 | 620.2 |
| 188 | 2-((2S,4S)-1-(but-2-ynoyl)-4-(8-chloro-7-(6-chloro-5-methyl-1H-indazol-4-yl)-6-fluoro-1H-[1,2,3]triazolo[4,5-c]quinolin-1-yl)piperidin-2-yl)acetonitrile | 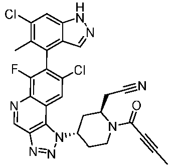 | Ex. 13 | 575.1 |
| | | | Ex. 14 | |
| | | | Ex. 80 | |
| | | | Ex. 87a | |
| 190a and 190b | 2-((2S,4S)-1-acryloyl-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)piperidin-2-yl)acetonitrile | 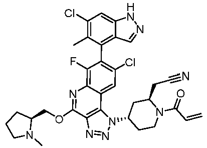 | Example 85 | 676.2 |
| | | | Example 182 | |
| 192 | 2-((2S,4S)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)-1-(2-fluoroacryloyl)piperidin-2-yl)acetonitrile | 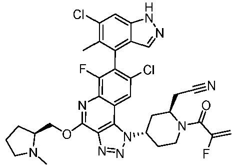 | Example 191 | 694.2 |
| 195a and 195b | 2-((2S,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4-methoxybut-2-enoyl)piperidin-2-yl)acetonitrile | 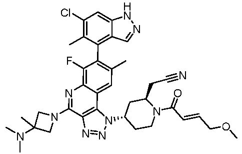 | Example 194a | 699.3 |
| 196a and 196b | 2-((2S,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile | 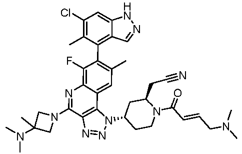 | Example 194a | 712.3 |
| 197a and 197b | 2-((2S,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4,4-difluorobut-2-enoyl)piperidin-2-yl)acetonitrile | 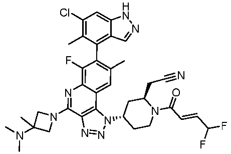 | Example 194a | 705.3 |
| 201a and 201b | 2-((2S,4S)-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)-1-((E)-4,4-difluorobut-2-enoyl)piperidin-2-yl)acetonitrile | 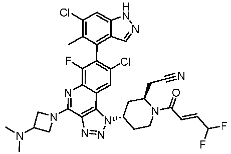 | Example 178a | 711.2 |
| 202a and 202b | 2-((2S,4S)-1-acryloyl-4-(4-(3-(dimethylamino)-3-methylazetidin-1-yl)-6-fluoro-7-(6-fluoro-5-methyl-1H-indazol-4-yl)-8-methyl-1H-[1,2,3]triazolo[4,5-c]quinolin-1-yl)piperidin-2-yl)acetonitrile | 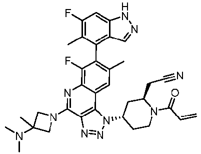 | Example 98 | 639.3 |
| | | | Example 80a | |
| | | | Example 85 | |
| 203a and 203b | 2-((2S,4S)-4-(4-(3-(dimethylamino)-3-methylazetidin-1-yl)-6-fluoro-7-(6-fluoro-5-methyl-1H-indazol-4-yl)-8-methyl-1H-[1,2,3]triazolo[4,5-c]quinolin-1-yl)-1-((E)-4-methoxybut-2-enoyl)piperidin-2-yl)acetonitrile | 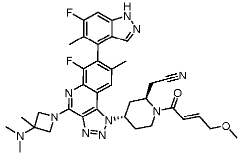 | Example 98 | 683.3 |
| | | | Example 80a | |
| 213a and 213b | 2-((2*S*)-1-(but-2-ynoyl)-4-(8-chloro-7-(8-chloronaphthalen-1-yl)-4-(3-(dimethylamino)-3-methylazetidin-1-yl)-6-fluoro-1H-imidazo[4,5-c]quinolin-1-yl)piperidin-2-yl)acetonitrile | 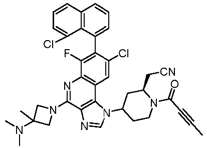 | Example 87a | 682.2 |
| | | | Example 212a | |
| 214a and 214b | 2-((2*S*)-1-acryloyl-4-(8-chloro-7-(8-chloronaphthalen-1-yl)-4-(3-(ethyl(methyl)amino)azetidin-1-yl)-6-fluoro-1H-imidazo[4,5-c]quinolin-1-yl)piperidin-2-yl)acetonitrile | 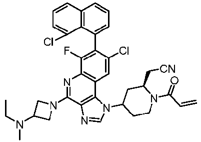 | Example 80a | 670.2 |
| | | | Example 212a | |
| 218a and 218b | 2-((2*S*)-1-(but-2-ynoyl)-4-(8-chloro-7-(8-chloronaphthalen-1-yl)-4-(3-(dimethylamino)-3-methylazetidin-1-yl)-6-fluoro-1*H*-[1,2,3]triazolo[4,5-c]quinolin-1-yl)piperidin-2-yl)acetonitrile | 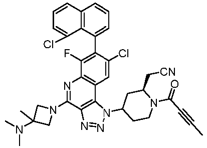 | Example 87a | 683.2 |
| | | | Example 216a | |
| 219a and 219b | 2-((2*S*)-1-acryloyl-4-(8-chloro-7-(8-chloronaphthalen-1-yl)-4-(3-(ethyl(methyl)amino)azetidin-1-yl)-6-fluoro-1*H-*[1,2,3]triazolo[4,5-c]quinolin-1-yl)piperidin-2-yl)acetonitrile | 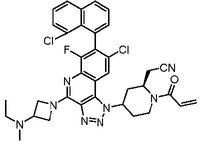 | Example 80a | 671.2dd |
| | | | Example 216a | |
| 222 | 2-((2*S*)-1-(but-2-ynoyl)-4-(7-(8-chloronaphthalen-1-yl)-4-(3-(dimethylamino)-3-methylazetidin-1-yl)-6-fluoro-8-methyl-1H-imidazo[4,5-c]quinolin-1-yl)piperidin-2-yl)acetonitrile | 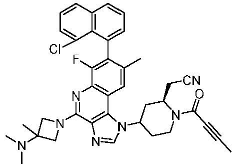 | Example 87a | 662.3 |
| | | | Example 221a | |
| 223a and 223b | 2-((2*S*)-1-acryloyl-4-(7-(8-chloronaphthalen-1-yl)-4-(3-(ethyl(methyl)amino)azetidin-1-yl)-6-fluoro-8-methyl-1*H-*imidazo[4,5-c]quinolin-1-yl)piperidin-2-yl)acetonitrile | 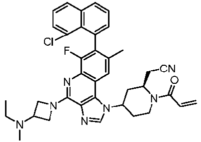 | Example 80a | 650.3 |
| | | | Example 221a | |
| 224a and 224b | 2-((2*S*)-1-(but-2-ynoyl)-4-(7-(8-chloronaphthalen-1-yl)-4-(3-(ethyl(methyl)amino)-azetidin-1-yl)-6-fluoro-8-methyl-1*H*-imidazo[4,5-c]quinolin-1-yl)piperidin-2-yl)acetonitrile | 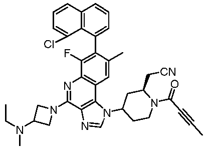 | Example 80a | 662.3 |
| | | | Example 87a | |
| | | | Example 221a | |
| 225 | 2-((2*S*)-1-acryloyl-4-(7-(8-chloronaphthalen-1-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile | 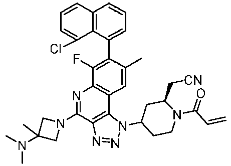 | Example 80a | 651.3 |
| 236 | 2-((2S,4S)-1-(but-2-ynoyl)-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile | 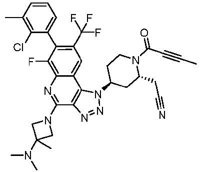 | Example 231a | 681.2 |
| 239a and 239b | 2-((2S,4S)-1-(but-2-ynoyl)-4-(7-(3-chloro-2-methylphenyl)-4-(3-(dimethyl-amino)-3-methylazetidin-1-yl)-6-fluoro-8-(trifluoromethyl)-1 H-[1,2,3]triazolo-[4,5-c]quinolin-1-yl)piperidin-2-yl)acetonitrile | 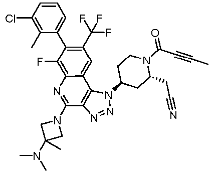 | Example 231a | 681.2 |
| 241a and 241b | 3-(1-((2S,4S)-1-(but-2-ynoyl)-2-(cyanomethyl)-piperidin-4-yl)-7-(3-chloro-2-methylphenyl)-6-fluoro-4-(((S)-1-methylpyrrolidin-2-yl)methoxy)-1H-[1,2,3]triazolo[4,5-c]quinolin-8-yl)propanenitrile | 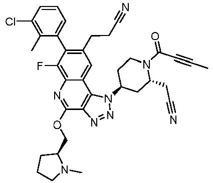 | Example 85 | 667.4 |
| | | | Example 231a | |
| | | | Example 238a | |
| 243a and 243b | 2-((2S,4S)-1-(but-2-ynoyl)-4-(7-(3-chloro-2-methoxyphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile | 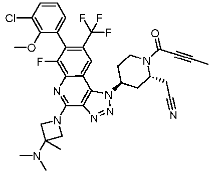 | Example 231a | 697.2 |
| | | | Example 233a | |
| 250 | 2-((2S,4S)-1-(but-2-ynoyl)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile | 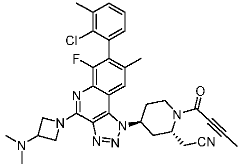 | Example 248 | 613.2 |
| 252 | 2-((2S,4S)-4-(7-(2-chloro-3-methylphenyl)-6-fluoro-8-methyl-4-(2-methyl-1H-imidazol-1-yl)-1H-[1,2,3]triazolo[4,5-c]quinolin-1-yl)-1-((E)-4-(dimethylamino)but-2-enoyl)piperidin-2-yl)acetonitrile | 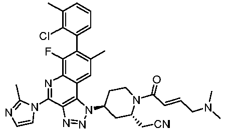 | Example 247 | 640.2 |
| | | | Example 248 | |
| | | | Example 249 | |
| 258 | 2-((2S,4S)-1-(but-2-ynoyl)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile | 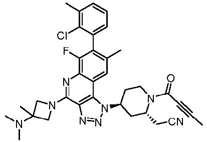 | Example 255 | 627.2 |
| | | | Example 254 | |
| 260 | 2-((2S,4S)-1-(but-2-ynoyl)-4-(7-(2-chloro-3-methylphenyl)-6-fluoro-8-methyl-4-(3-oxomorpholino)-1H-[1,2,3]triazolo[4,5-c]quinolin-1-yl)piperidin-2-yl)acetonitrile | 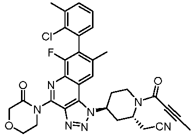 | Example 259 | 614.2 |
| | | | Example 254 | |
| 261 | 2-((2S,4S)-4-(7-(2-chloro-3-methylphenyl)-6-fluoro-8-methyl-4-(3-oxomorpholino)-1H-[1,2,3]triazolo[4,5-c]quinolin-1-yl)-1-((E)-4-fluorobut-2-enoyl)piperidin-2-yl)acetonitrile | 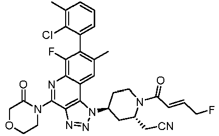 | Example 259 | 634.2 |
| | | | Example 248 | |
| 263 | 2-((2S,4S)-1-(but-2-ynoyl)-4-(8-chloro-4-(3-(dimethylamino)azetidin-1-yl)-6-fluoro-7-(3-methyl-2-(trifluoromethyl)phenyl)-1 H-imidazo[4,5-c]quinolin-1-yl)piperidin-2-yl)acetonitrile | 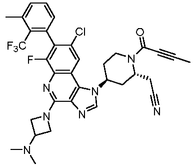 | Example 262 | 666.2 |
| 264 | 2-((2S,4S)-1-acryloyl-4-(8-chloro-4-(3-(dimethylamino)azetidin-1-yl)-6-fluoro-7-(3-methyl-2-(trifluoromethyl)phenyl)-1 H-imidazo[4,5-c]quinolin-1-yl)piperidin-2-yl)acetonitrile | 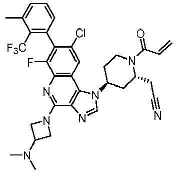 | Example 85 | 654.2 |
| 266a and 266b | 2-((2S,4S)-1-acryloyl-4-(8-chloro-4-(3-(dimethylamino)azetidin-1-yl)-6-fluoro-7-(6-fluoro-5-methyl-1H-indazol-4-yl)-1 H-imidazo[4,5-c]quinolin-1-yl)piperidin-2-yl)acetonitrile | 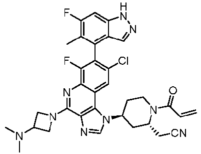 | Example 85 | 644.2 |
| | | | Example 89 | |
| 268a and 268b | 2-((2S,4S)-1-acryloyl-4-(8-chloro-4-(3-(dimethylamino)azetidin-1-yl)-6-fluoro-7-(1-methylisoquinolin-4-yl)-1H-imidazo[4,5-c]quinolin-1-yl)piperidin-2-yl)acetonitrile | 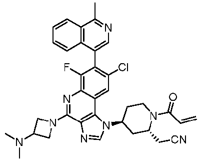 | Example 85 | 637.2 |
| | | | Example 89 | |
| 269a and 269b | 2-((2S,4S)-1-acryloyl-4-(8-chloro-4-(3-(dimethylamino)azetidin-1-yl)-6-fluoro-7-(3-methylisoquinolin-4-yl)-1H-imidazo[4,5-c]quinolin-1-yl)piperidin-2-yl)acetonitrile | 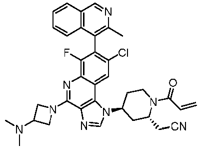 | Example 85 | 637.2 |
| | | | Example 89 | |
| 273 | 2-((2S,4S)-1-acryloyl-4-(6,8-dichloro-7-(3-chloro-2-methylphenyl)-4-(3-(dimethylamino)azetidin-1-yl)-1H-[1,2,3]triazolo[4,5-c]quinolin-1-yl)piperidin-2-yl)acetonitrile | 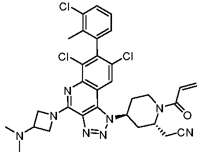 | Example 271a | 637.2 |
| 274a and 274b | 2-((2S,4S)-1-(but-2-ynoyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidin-2-yl)acetonitrile | 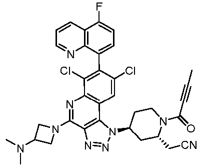 | Example 270a | 670.2 |
| | | | Example 271a | |
| 282 | 2-((2S,4S)-1-acryloyl-4-(6,8-dichloro-7-(2-chloro-3-methylphenyl)-4-(3-(dimethylamino)azetidin-1-yl)-1H-[1,2,3]triazolo[4,5-c]quinolin-1-yl)piperidin-2-yl)acetonitrile | 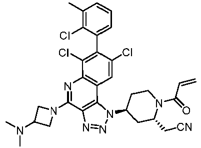 | Example 271a | 637.2 |
| 285 | 2-((2S,4S)-1-acryloyl-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidin-2-yl)acetonitrile | 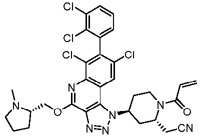 | Example 271a | 672.1 |
| | | | Example 278 | |

### Example A. GDP-GTP exchange assay.

The inhibitor potency of the exemplified compounds was determined in a fluorescence based guanine nucleotide exchange assay, which measures the exchange of bodipy-GDP (fluorescently labeled GDP) for GppNHp (Non-hydrolyzable GTP analog) to generate the active state of KRAS in the presence of SOS1 (guanine nucleotide exchange factor). Inhibitors were serially diluted in DMSO and a volume of 0.1 µL was transferred to the wells of a black low volume 384-well plate. 5 µL/well volume of bodipy-loaded KRAS G12C diluted to 5 nM in assay buffer (25 mM Hepes pH 7.5, 50 mM NaCl, 10 mM MgCl2 and 0.01% Brij-35) was added to the plate and pre-incubated with inhibitor for 2 hours at ambient temperature. Appropriate controls (enzyme with no inhibitor or with a G12C inhibitor (AMG-510)) were included on the plate. The exchange was initiated by the addition of a 5 µL/well volume containing 1 mM GppNHp and 300 nM SOS1 in assay buffer. The 10 µL/well reaction concentration of the bodipy-loaded KRAS G12C, GppNHp, and SOS1 were 2.5 nM, 500 uM, and 150 nM, respectively. The reaction plates were incubated at ambient temperature for 2 hours, a time estimated for complete GDP-GTP exchange in the absence of inhibitor. For the KRAS G12D and G12V mutants, similar guanine nucleotide exchange assays were used with 2.5nM as final concentration for the bodipy loaded KRAS proteins and with 4 hours and 3 hours incubation after adding GppNHp-SOS1 mixture for G12D and G12V respectively. A cyclic peptide described to selectively bind G12D mutant (Sakamoto et al., BBRC 484.3 (2017), 605-611) or internal compounds with confirmed binding were used as positive controls in the assay plates. Fluorescence intensities were measured on a PheraStar plate reader instrument (BMG Labtech) with excitation at 485 nm and emission at 520 nm.

Either GraphPad prism or XLfit was used to analyze the data. The IC₅₀ values were derived by fitting the data to a four parameter logistic equation producing a sigmoidal dose-response curve with a variable Hill coefficient. Prism equation: Y=Bottom + (Top-Bottom)/(1+10^((LogIC₅₀-X)*Hill slope)); XLfit equation: Y = (A+((B-A)/(1+((X/C)^D)))) where X is the logarithm of inhibitor concentration and Y is the response.

The KRAS_G12C exchange assay IC₅₀ data and KRAS_G12C pERK assay IC₅₀ data are provided in Table 1 below. The symbol "†" indicates IC₅₀ ≤ 100 nM, "††" indicates IC₅₀ > 100 nM but ≤ 1 µM; and "†††" indicates IC₅₀ is >1 µM but ≤ 5 µM, "††††" indictes IC₅₀ is >5 µM but ≤ 10 µM. "NA" indicates IC₅₀ not available.

**Table 1**

| **Ex. No.** | G12C_exchange | G12C_pERK |
|---|---|---|
| **1a** | † | †† |
| **2a** | †† | ††† |
| **3a** | ††† | NA |
| **4a** | † | †† |
| **5** | † | ††† |
| **6a** | † | †† |
| **7** | † | † |
| **8** | †† | NA |
| **9** | † | †† |
| **10** | † | †† |
| **11** | † | †† |
| **12** | †† | ††† |
| **13** | †† | ††† |
| **14a** | † | † |
| **16** | † | † |
| **17** | † | † |
| **18** | † | † |
| **19** | † | † |
| **20** | † | † |
| **21** | † | † |
| **22** | † | † |
| **26** | † | ††† |
| **27** | †† | ††† |
| **28** | †† | NA |
| **29b** | † | † |
| **30b** | † | † |
| **31a** | † | † |
| **32** | † | † |
| **33** | †† | ††† |
| **36a** | † | † |
| **37a** | † | † |
| **38** | † | † |
| **39a** | † | † |
| **40a** | † | † |
| **41** | † | † |
| **42** | † | † |
| **43** | † | † |
| **44** | † | † |
| **45** | † | † |
| **46** | † | † |
| **47a** | † | † |
| **52a** | † | † |
| **53a** | † | † |
| **54a** | † | † |
| **55** | † | † |
| **56** | † | † |
| **57** | † | † |
| **58** | † | † |
| **59** | † | † |
| **60** | † | † |
| **61** | † | † |
| **62** | † | † |
| **63** | † | † |
| **64a** | † | † |
| **65** | † | † |
| **66** | † | † |
| **67** | † | † |
| **68** | † | † |
| **69** | † | † |
| **70a** | † | † |
| **71a** | † | † |
| **71b** | † | † |
| **72a** | † | † |
| **73a** | † | † |
| **73b** | † | † |
| **74a** | † | † |
| **80a** | † | † |
| **81a** | † | † |
| **82a** | † | † |
| **83a** | † | † |
| **84a** | † | † |
| **85** | † | † |
| **86** | † | † |
| **87a** | † | † |
| **88** | † | † |
| **89** | † | † |
| **90** | † | † |
| **91** | † | † |
| **92** | † | † |
| **93** | † | † |
| **94** | † | † |
| **95** | † | † |
| **96** | † | † |
| **97** | † | † |
| **98** | † | † |
| **99** | † | † |
| **100** | † | † |
| **101** | † | † |
| **102** | † | † |
| **103** | † | † |
| **104** | † | † |
| **105** | † | † |
| **106** | † | † |
| **107** | † | † |
| **108** | † | † |
| **109** | † | † |
| **110a** | † | † |
| **111** | † | † |
| **112** | † | † |
| **113** | † | † |
| **114** | † | † |
| **115** | † | † |
| **116** | † | † |
| **117** | † | † |
| **118a** | † | † |
| **119a** | † | † |
| **120** | † | † |
| **121** | † | † |
| **122** | † | † |
| **123** | † | † |
| **124** | † | † |
| **125** | † | † |
| **126** | † | † |
| **127** | † | † |
| **128** | † | † |
| **129** | † | † |
| **130** | † | † |
| **131** | † | † |
| **132** | † | † |
| **133** | † | † |
| **134** | † | † |
| **135** | † | † |
| **136** | † | † |
| **137** | † | † |
| **138** | † | † |
| **139** | † | † |
| **140** | † | † |
| **141** | † | † |
| **142** | † | † |
| **143** | † | † |
| **144** | † | † |
| **145** | † | † |
| **146** | † | † |
| **147** | † | † |
| **148** | † | † |
| **149** | † | † |
| **150a** | † | † |
| **151** | † | † |
| **152** | † | † |
| **153** | † | † |
| **154** | † | † |
| **155** | † | † |
| **156** | † | † |
| **157a** | † | † |
| **158a** | † | † |
| **159a** | † | † |
| **160a** | † | † |
| **161a** | † | † |
| **162a** | † | † |
| **163a** | † | † |
| **164a** | † | † |
| **165a** | † | † |
| **166a** | † | † |
| **167a** | † | † |
| **168a** | † | † |
| **169** | † | † |
| **170** | † | † |
| **171** | † | † |
| **172** | † | † |
| **173** | † | † |
| **174** | † | † |
| **175** | † | † |
| **176** | † | † |
| **177a** | † | † |
| **178a** | † | † |
| **179a** | † | † |
| **180** | † | † |
| **181a** | † | † |
| **182** | † | † |
| **183a** | † | † |
| **184a** | † | † |
| **185a** | † | † |
| **186a** | † | † |
| **187** | † | † |
| **188** | † | † |
| **189** | † | † |
| **190a** | † | † |
| **191a** | † | † |
| **192** | † | † |
| **193a** | † | † |
| **194a** | † | † |
| **195a** | † | † |
| **196a** | † | † |
| **197a** | † | † |
| **198a** | † | † |
| **199** | † | † |
| **200a** | † | † |
| **201a** | † | † |
| **202a** | † | † |
| **203a** | † | † |
| **204** | † | † |
| **205a** | † | † |
| **206** | † | † |
| **207** | † | † |
| **208** | † | † |
| **209** | † | † |
| **210** | † | † |
| **211** | † | † |
| **212a** | † | † |
| **213b** | † | † |
| **214b** | † | † |
| **215b** | † | † |
| **216a** | † | † |
| **217** | † | † |
| **218a** | † | † |
| **219a** | † | † |
| **220a** | † | † |
| **221a** | † | † |
| **222** | † | † |
| **223a** | † | † |
| **224a** | † | † |
| **225** | † | † |
| **226** | † | † |
| **229** | † | † |
| **230a** | † | † |
| **231a** | † | † |
| **232** | † | † |
| **233a** | † | † |
| **234** | † | † |
| **235a** | † | † |
| **236** | † | † |
| **237** | † | † |
| **238a** | † | † |
| **239a** | † | † |
| **240** | † | † |
| **241a** | † | † |
| **242a** | † | † |
| **243b** | † | † |
| **244** | † | † |
| **245a** | † | † |
| **246** | † | † |
| **247** | † | † |
| **248** | † | † |
| **249** | † | † |
| **250** | † | † |
| **251** | † | † |
| **252** | † | † |
| **253** | † | † |
| **254** | † | † |
| **255** | † | † |
| **256** | † | † |
| **257** | † | † |
| **258** | † | † |
| **259** | † | † |
| **260** | † | † |
| **261** | † | † |
| **262** | † | † |
| **263** | † | † |
| **264** | † | † |
| **265** | † | † |
| **266a** | † | † |
| **267a** | † | † |
| **268a** | † | † |
| **269a** | † | † |
| **270a** | † | † |
| **271a** | † | † |
| **273** | † | † |
| **274a** | † | † |
| **276a** | † | † |
| **277a** | † | † |
| **278** | † | † |
| **279a** | † | † |
| **280a** | † | † |
| **281** | † | † |
| **282** | † | † |
| **283a** | † | † |
| **284a** | † | † |
| **285** | † | † |
| **286** | † | † |
| **287** | † | † |
| **288a** | † | † |
| **289a** | † | † |
| **290** | † | † |

The KRAS_G12D exchange assay IC₅₀ data are provided in Table 2 below. The symbol "†" indicates IC₅₀ ≤ 100 nM, "††" indicates IC₅₀ > 100 nM but ≤ 1 µM; and "†††" indicates IC₅₀ is >1 µM but ≤ 5 µM, "††††" indicates IC₅₀ is >5 µM but ≤ 10 µM. "NA" indicates IC₅₀ not available.

**Table 2**

| **Ex. No.** | G12D_exchange |
|---|---|
| **15** | ††† |
| **23a** | †† |
| **24** | ††† |
| **25a** | †††† |
| **34a** | †† |
| **35a** | ††† |
| **48a** | †† |
| **49** | †††† |
| **50** | †† |
| **75** | †† |
| **76** | †† |
| **77** | ††† |
| **78** | †† |
| **79** | †† |
| **291b** | † |
| **292b** | † |
| **293** | †† |
| **294** | †† |
| **295b** | †† |
| **296b** | † |
| **297** | † |
| **298** | † |
| **299** | † |
| **300a** | † |
| **301a** | † |
| **302a** | † |

### Example B: Luminescent Viability Assay

MIA PaCa-2 (KRAS G12C; ATCC^{®} CRL-1420), A427 (KRAS G12D; ATCC^{®} HTB53) and NCI-H838 (KRAS WT; ATCC^{®} CRL-5844) cells are cultured in RPMI 1640 media supplemented with 10% FBS (Gibco/Life Technologies). The cells are seeded (5×10³ cells/well/in 50 uL) into black, clear bottomed 96-well Greiner tissue culture plates and cultured overnight at 37°C, 5% CO₂. After overnight culture, 50 uL per well of serially diluted test compounds (2x final concentration) are added to the plates and incubated for 3 days. At the end of the assay, 100ul/well of CellTiter-Glo reagent (Promega) is added. Luminescence is read after 15 minutes with a TopCount (PerkinElmer). IC₅₀ determination is performed by fitting the curve of percent inhibition versus the log of the inhibitor concentration using the GraphPad Prism 7 software.

### Example C: Cellular pERK HTRF Assay

MIA PaCa-2 (KRAS G12C; ATCC^{®} CRL-1420), A427 (KRAS G12D; ATCC^{®} HTB53), HPAF-II (KRAS G12D; ATCC^{®} CRL-1997) and NCI-H838 (KRAS WT; ATCC^{®} CRL-5844) cells are purchased from ATCC and maintained in RPMI 1640 media supplemented with 10% FBS (Gibco/Life Technologies). The cells are plated at 5000 cells per well (8 uL) into Greiner 384-well low volume, flat-bottom, tissue culture treated white plates and incubated overnight at 37°C, 5% CO₂. The next morning, test compound stock solutions are diluted in media at 3x the final concentration, and 4 uL are added to the cells. The plate is mixed by gentle rotation for 30 seconds (250rpm) at room temperature. The cells are incubated with the KRAS G12C and G12D compounds for 4 hours or 2 hours respectively at 37°C, 5% CO2.

4 uL of 4x lysis buffer with blocking reagent (1:25) (Cisbio) are added to each well and plates are rotated gently (300 rpm) for 30 minutes at room temperature. 4 uL per well of Cisbio anti Phospho-ERK 1/2 d2 is mixed with anti Phospho-ERK 1/2 Cryptate (1:1) are added to each well, mixed by rotation and incubated overnight in the dark at room temperature. Plates are read on the Pherastar plate reader at 665 nm and 620 nm wavelengths. IC₅₀ determination is performed by fitting the curve of inhibitor percent inhibition versus the log of the inhibitor concentration using the GraphPad Prism 7 software.

### Example D: Whole Blood pERK1/2 HTRF Assay

MIA PaCa-2 cells (KRAS G12C; ATCC^{®} CRL-1420) and HPAF-II (KRAS G12D; ATCC^{®} CRL-1997) are maintained in RPMI 1640 with 10% FBS (Gibco/Life Technologies). The cells are seeded into 96 well tissue culture plates (Corning #3596) at 25000 cells per well in 100 uL media and cultured for 2 days at 37 °C, 5% CO₂ so that they are approximately 80% confluent at the start of the assay. Whole Blood are added to the 1uL dots of compounds (prepared in DMSO) in 96 well plates and mixed gently by pipetting up and down so that the concentration of the compound in blood is 1x of desired concentration. The media is aspirated from the cells and 50 uL per well of whole blood with G12C or G12D compound is added and incubated for 4 or 2 hours respectively at 37 °C, 5% CO₂. After dumping the blood, the plates are gently washed twice by adding PBS to the side of the wells and dumping the PBS from the plate onto a paper towel, tapping the plate to drain well. 50ul/well of 1x lysis buffer #1 (Cisbio) with blocking reagent (1:25) (Cisbio) is then added and incubated at room temperature for 30 minutes with shaking (250 rpm). Following lysis, 16 uL of lysate is transferred into 384-well Greiner small volume white plate using an Assist Plus (Integra Biosciences, NH). 4uL of 1:1 mixture of anti Phospho-ERK 1/2 d2 and anti Phospho-ERK 1/2 Cryptate (Cisbio) is added to the wells using the Assist Plus and incubated at room temperature overnight in the dark. Plates are read on the Pherastar plate reader at 665 nm and 620 nm wavelengths. IC₅₀ determination is performed by fitting the curve of inhibitor percent inhibition versus the log of the inhibitor concentration using the GraphPad Prism 7 software.

### Example E: Ras Activation Elisa

The 96-Well Ras Activation ELISA Kit (Cell Biolabs Inc; #STA441) uses the Raft RBD (Rho binding domain) bound to a 96-well plate to selectively pull down the active form of Ras from cell lysates. The captured GTP-Ras is then detected by a pan-Ras antibody and HRP-conjugated secondary antibody.

MIA PaCa-2 cells (KRAS G12C; ATCC^{®} CRL-1420) and HPAF-II (KRAS G12D; ATCC^{®} CRL-1997) are maintained in RPMI 1640 with 10% FBS (Gibco/Life Technologies). The cells are seeded into 96 well tissue culture plates (Corning #3596) at 25000 cells per well in 100 uL media and cultured for 2 days at 37 °C, 5% CO₂ so that they are approximately 80% confluent at the start of the assay. The cells are treated with compounds for either 2 hours or overnight at 37 °C, 5% CO₂. At the time of harvesting, the cells are washed with PBS, drained well and then lysed with 50 uL of the 1x Lysis buffer (provided by the kit) plus added Halt Protease and Phosphatase inhibitors (1:100) for 1 hour on ice.

The Raf-1 RBD is diluted 1:500 in Assay Diluent (provided in kit) and 100 µL of the diluted Raf-1 RBD is added to each well of the Raf-1 RBD Capture Plate. The plate is covered with a plate sealing film and incubated at room temperature for 1 hour on an orbital shaker. The plate is washed 3 times with 250 µL 1X Wash Buffer per well with thorough aspiration between each wash. 50 µL of Ras lysate sample (10-100 µg) is added per well in duplicate. A "no cell lysate" control is added in a couple of wells for background determination. 50 µL of Assay Diluent is added to all wells immediately to each well and the plate is incubated at room temperature for 1 hour on an orbital shaker. The plate is washed 5 times with 250 µL 1X Wash Buffer per well with thorough aspiration between each wash. 100 µL of the diluted Anti-pan-Ras Antibody is added to each well and the plate is incubated at room temperature for 1 hour on an orbital shaker. The plate is washed 5 times as previously. 100 µL of the diluted Secondary Antibody, HRP Conjugate is added to each well and the plate is incubated at room temperature for 1 hour on an orbital shaker. The plate is washed 5 times as previously and drained well. 100 µL of Chemiluminescent Reagent (provided in the kit) is added to each well, including the blank wells. The plate is incubated at room temperature for 5 minutes on an orbital shaker before the luminescence of each microwell is read on a plate luminometer. The % inhibition is calculated relative to the DMSO control wells after a background level of the "no lysate control" is subtracted from all the values. IC₅₀ determination is performed by fitting the curve of inhibitor percent inhibition versus the log of the inhibitor concentration using the GraphPad Prism 7 software.

### Example F: Inhibition of RAS-RAF and PI3K-AKT Pathways

The cellular potency of compounds was determined by measuring phosphorylation of KRAS downstream effectors extracellular-signal-regulated kinase (ERK), ribosomal S6 kinase (RSK), AKT (also known as protein kinase B, PKB) and downstream substrate S6 ribosomal protein.

To measure phosphorylated extracellular-signal-regulated kinase (ERK), ribosomal S6 kinase (RSK), AKT and S6 ribosomal protein, cells (details regarding the cell lines and types of data produced are further detailed in Table 4) were seeded overnight in Corning 96-well tissue culture treated plates in RPMI medium with 10% FBS at 4×10⁴ cells/well. The following day, cells were incubated in the presence or absence of a concentration range of test compounds for 4 hours at 37 °C, 5% CO₂. Cells were washed with PBS and lysed with 1x lysis buffer (Cisbio) with protease and phosphatase inhibitors. 10 µg of total protein lysates was subjected to SDS-PAGE and immunoblot analysis using following antibodies: phospho-ERK1/2-Thr202/Tyr204 (#9101L), total-ERK1/2 (#9102L), phosphor-AKT-Ser473 (#4060L), phospho-p90RSK-Ser380 (#11989S) and phospho-S6 ribosomal protein-Ser235/Ser236 (#2211S) are from Cell Signaling Technologies (Danvers, MA).

**Table 3**

| **Cell Line** | **Histology** | **KRAS alteration** | **Readout** |
|---|---|---|---|
| H358 | Lung | G12C | pERK, pAKT |
| MIA PaCa-2 | Pancreas | G12C | pERK, pAKT |
| HPAF II | Pancreas | G12D | pERK, pAKT |
| SU.86.86 | Pancreas | G12D | pERK, pAKT |
| PaTu 8988s | Pancreas | G12V | pERK, pAKT |
| H441 | Lung | G12V | pERK, pAKT |

### Example G: In vivo efficacy studies

Mia-Paca-2 human pancreatic cancer cells were obtained from the American Type Culture Collection and maintained in RPMI media supplemented with 10% FBS. For efficacy studies experiments, 5 × 10⁶ Mia-Paca-2 cells were inoculated subcutaneously into the right hind flank of 6- to 8-week-old BALB/c nude mice (Charles River Laboratories, Wilmington, MA, USA). When tumor volumes were approximately 150-250 mm3, mice were randomized by tumor volume and compounds were orally administered. Tumor volume was calculated using the formula (L × W²)/2, where L and W refer to the length and width dimensions, respectively. Tumor growth inhibition was calculated using the formula (1 - (V_{T}/V_{C})) × 100, where V_{T} is the tumor volume of the treatment group on the last day of treatment, and V_{C} is the tumor volume of the control group on the last day of treatment. Two-way analysis of variance with Dunnett's multiple comparisons test was used to determine statistical differences between treatment groups (GraphPad Prism). Mice were housed at 10-12 animals per cage, and were provided enrichment and exposed to 12-hour light/dark cycles. Mice whose tumor volumes exceeded limits (10% of body weight) were humanely euthanized by CO₂ inhalation. Animals were maintained in a barrier facility fully accredited by the Association for Assessment and Accreditation of Laboratory Animal Care, International. All of the procedures were conducted in accordance with the US Public Service Policy on Human Care and Use of Laboratory Animals and with Incyte Animal Care and Use Committee Guidelines.

Various modifications of the invention, in addition to those described herein, will be apparent to those skilled in the art from the foregoing description. Such modifications are also intended to fall within the scope of the appended clauses. Each reference, including without limitation all patent, patent applications, and publications, cited in the present application is incorporated herein by reference in its entirety.

The invention will now be described in relation to the following non-limiting clauses:
1. A compound of Formula I: 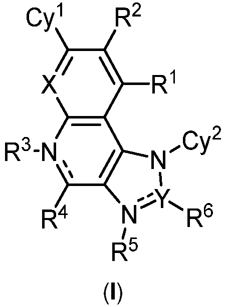 or a pharmaceutically acceptable salt thereof,
wherein:
each --- represents a single bond or a double bond;
X is N or CR⁷;
Y is N or C;
R¹ is selected from **H,** D, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, CN, OR^{a1}, SR^{a1}, C(O)R^{b1}, C(O)NW^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)OR^{a1}, NR^{c1}C(O)NR^{c1}R^{d1}, NR^{c1}S(O)R^{b1}, NR^{c1}S(O)₂R^{b1}, NR^{c1}S(O)₂NR^{c1}R^{d1}, S(O)R^{b1}, S(O)NR^{c1}R^{d1}, S(O)₂R^{b1}, S(O)₂NR^{c1}R^{d1}, and BR^{h1}Rⁱ¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R^{g};
R² is selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, C(=NR^{e2})R^{b2}, C(=NOR^{a2})R^{b2}, C(=NR^{e2})NR^{c2}R^{d2}, NR^{c2}C(=NR^{e2})NR^{c2}R^{d2}, NR^{c2}C(=NR^{e2})R^{b2}, NR^{c2}S(O)R^{b2}, NR^{c2}S(O)₂R^{b2}, NR^{c2}S(O)₂NR^{c2}R^{d2}, S(O)R^{b2}, S(O)NR^{c2}R^{d2}, S(O)₂R^{b2}, S(O)₂NR^{c2}R^{d2}, and BR^{h2}Rⁱ²; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²²;
Cy¹ is selected from C₃₋₁₀ cycloalkyl, 4-10 membered heterocycloalkyl, C₆₋₁₀ aryl and 5-10 membered heteroaryl; wherein the 4-10 membered heterocycloalkyl and 5-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 5-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 5-10 membered heteroaryl are each optionally substituted with 1, 2, 3 or 4 substituents independently selected from R¹⁰;
when R³N---CR⁴ is a single bond, then R⁴ is selected from =O and =S; and
R³ is selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R³N---CR⁴ is a double bond, then R³ is absent; and
R⁴ is selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, NR^{c3}C(O)NR^{c3}R^{d3}, C(=NR^{e3})R^{b3}, C(=NOR^{a3})R^{b3}, C(=NR^{e3})NR^{c3}R^{d3}, NR^{c3}C(=NR^{e3})NR^{c3}R^{d3}, NR^{c3}C(=NR^{e3})R^{b3}, NR^{c3}S(O)R^{b3}, NR^{c3}S(O)₂R^{b3}, NR^{c3}S(O)₂NR^{c3}R^{d3}, S(O)R^{b3}, S(O)NR^{c3}R^{d3}, S(O)₂R^{b3}, S(O)₂NR^{c3}R^{d3}, and BR^{h3}Rⁱ³; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R⁵N---YR⁶ is a single bond and Y is C, then YR⁶ is selected from C=O and C=S; and
R⁵ is selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁵⁰;
when R⁵N---YR⁶ is a double bond and Y is N, then R⁵ and R⁶ are absent;
when R⁵N---YR⁶ is a double bond and Y is C, then R⁵ is absent; and
R⁶ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃alkylene, halo, D, CN, NO₂, OR^{a6}, SR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, C(=NR^{e6})R^{b6}, C(=NOR^{a6})R^{b6}, C(=NR^{e6})NR^{c6}R^{d6}, NR^{c6}C(=NR^{e6})NR^{c6}R^{d6}, NR^{c6}C(=NR^{e6})R^{b6}, NR^{c6}S(O)R^{b6}, NR^{c6}S(O)₂R^{b6}, NR^{c6}S(O)₂NR^{c6}R^{d6}, S(O)R^{b6}, S(O)NR^{c6}R^{d6}, S(O)₂R^{b6}, S(O)₂NR^{c6}R^{d6}, and BR^{h6}Rⁱ⁶; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶⁰;
R⁷ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a7}, SR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7}, C(=NR^{e7})R^{b7}, C(=NOR^{a7})R^{b7}, C(=NR^{e7})NR^{c7}R^{d7}, NR^{c7}C(=NR^{e7})NR^{c7}R^{d7}, NR^{c7}C(=NR^{e7})R^{b7}, NR^{c7}S(O)R^{b7}, NR^{c7}S(O)₂R^{b7}, NR^{c7}S(O)₂NR^{c7}R^{d7}, S(O)R^{b7}, S(O)NR^{c7}R^{d7}, S(O)₂R^{b7}, S(O)₂NR^{c7}R^{d7}, and BR^{h7}Rⁱ⁷; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷⁰;
Cy² is selected from C₃₋₁₀ cycloalkyl, 4-14 membered heterocycloalkyl, C₆₋₁₀ aryl and 5-10 membered heteroaryl; wherein the 4-14 membered heterocycloalkyl and 5-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 5-10 membered heteroaryl and 4-14 membered heterocycloalkyl is optionally substituted by oxo to form a carbonyl group; and wherein the C₃₋₁₀ cycloalkyl, 4-14 membered heterocycloalkyl, C₆₋₁₀ aryl and 5-10 membered heteroaryl are each optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a10}, SR^{a10}, C(O)R^{b10}, C(O)NR^{c10}R^{d10}, C(O)OR^{a10}, OC(O)R^{b10}, OC(O)NR^{c10}Rd¹⁰, NR^{c10}R^{d10}, NR^{c10}C(O)R^{b10}, NR^{c10}C(O)OR^{a10}, NR^{c10}C(O)NR^{c10}R^{d10}, C(=NR^{e10})R^{b10}, C(=NOR^{a10})R^{b10}, C(=NR^{e10})NR^{c10}R^{d10}, NR^{c10}C(=NR^{e10})NR^{c10}R^{d10}, NR^{c10}S(O)R^{b10}, NR^{c10}S(O)₂R^{b10}, NR^{c10}S(O)₂NR^{c10}R^{d10}, S(O)R^{b10}, S(O)NR^{c10}R^{d10}, S(O)₂R^{b10}, S(O)₂NR^{c10}R^{d10}, and BR^{h10}Rⁱ¹⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R¹¹;
each R¹¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, C_{N}, OR^{a11}**,** SR^{a11}, C(O)R^{b11}, C(O)NR^{c11}R^{d11}, C(O)OR^{a11}, OC(O)R^{b11}, OC(O)NR^{c11}R^{d11}, NR^{c11}R^{d11}, NR^{c11}C(O)R^{b11}, NR^{c11}C(O)OR^{a11}, NR^{c11}C(O)NR^{c11}R^{d11}, NR^{c11}S(O)R^{b11}, NR^{c11}S(O)₂R^{b11}, NR^{c11}S(O)₂NR^{c11}R^{d11}, S(O)R^{b11}, R^{d11}, S(O)₂R^{b11}, R^{d11}, and BR^{h11}Rⁱ¹¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R¹²;
each R¹² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a12}, SR^{a12}, C(O)R^{b12}, C(O)NR^{c12}R^{d12}, C(O)OR^{a12}, OC(O)R^{b12}, OC(O)NR^{c12}R^{d12}, NR^{c12}R^{d12}, NR^{c12}C(O)R^{b12}, NR^{c12}C(O)OR^{a12}, NR^{c12}C(O)NRC¹²R^{d12}, NR^{c12}S(O)R^{b12}, NR^{c12}S(O)₂R^{b12}, NR^{c12}S(O)₂NR^{c12}R^{d12}, S(O)R^{b12}, S(O)NR^{c12}R^{d12}, S(O)₂R^{b12}, S(O)₂NR^{c12}R^{d12}, and BR^{h12}Rⁱ¹²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R²⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a20}, SR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, NR^{c20}C(O)NR^{c20}R^{d20}, C(=NR^{e20})R^{b20}, C(=NOR^{a20})R^{b20}, C(=NR^{e20})NR^{c20}R^{d20}, NR^{c20}C(=NR^{e20})NR^{c20}R^{d20}, NR^{c20}S(O)R^{b20}, NR^{c20}S(O)₂R^{b20}, NR^{c20}S(O)₂NR^{c20}R^{d20}, S(O)R^{b20}, S(O)NR^{c20}R^{d20}, S(O)₂R^{b20}, S(O)₂NR^{c20}R^{d20}, and BR^{h20}Rⁱ²⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²¹;
each R²¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a21}, SR^{a21}, C(O)R^{b21}, C(O)NR^{c21}R^{d21}, C(O)OR^{a21}, OC(O)R^{b21}, OC(O)NR²¹R^{d21}, NR^{c21}R^{d21}, NR^{c21}C(O)R^{b21}, NR^{c21}C(O)OR^{a21}, NR^{c21}C(O)NR^{c21}R^{d21}, NR^{c21}S(O)R^{b21}, NR^{c21}S(O)₂R^{b21}, NR^{c21}S(O)₂NR^{c21}R^{d21}, S(O)R^{b21}, S(O)NR^{c21}R^{d21}, S(O)₂R^{b21}, S(O)₂NR^{c21}R^{d21}, and BR^{h21}Rⁱ²¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R^{g};
each R²² is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a22}, SR^{a22}, C(O)R^{b22}, C(O)NR^{c22}R^{d22}, C(O)OR^{a22}, OC(O)R^{b22}, OC(O)NR^{c22}R^{d22}, NR^{c22}R^{d22}, NR^{c22}C(O)R^{b22}, NR^{c22}C(O)OR^{a22}, NR^{c22}C(O)NR^{c22}R^{d22}, NR^{c22}S(O)R^{b22}, NR^{c22}S(O)₂R^{b22}, NR^{c22}S(O)₂NR^{c22}R^{d22}, S(O)R^{b22}, S(O)NR^{c22}R^{d22}, S(O)₂R^{b22}, S(O)₂NR^{c22}R^{d22}, and BR^{h22}Rⁱ²²; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²³;
each R²³ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a23}, SR^{a23}, C(O)R^{b23}, C(O)NR^{c23}R^{d23}, C(O)OR^{a23}, OC(O)R^{b23}, OC(O)NR^{c23}R^{d23}, NR^{c23}R^{d23}, NR^{c23}C(O)R^{b23}, NR^{c23}C(O)OR^{a23}, NR^{c23}C(O)NR^{c23}R^{d23}, NR^{c23}S(O)R^{b23}, NR^{c23}S(O)₂R^{b23}, NR^{c23}S(O)₂NR^{c23}R^{d23}, S(O)R^{b23}, S(O)NR^{c23}R^{d23}, S(O)₂NR^{c23}R^{d23}, and BR^{h23}Rⁱ²³; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²⁴;
each R²⁴ is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a24}, SR^{a24}, C(O)R^{b24}, C(O)NR^{c24}R^{d24}, C(O)OR^{a24}, OC(O)R^{b24}, OC(O)NR^{c24}R^{d24}, NR^{c24}R^{d24}, NR^{c24}C(O)R^{b24}, NR^{c24}C(O)OR^{a24}, NR^{c24}C(O)NR^{c24}R^{d24}, NR^{c24}S(O)R^{b24}, NR^{c24}S(O)₂R^{b24}, NR^{c24}S(O)₂NR^{c24}R^{d24}, S(O)R^{b24}, S(O)NR^{c24}R^{d24}, S(O)₂R^{b24}, S(O)₂NR^{c24}R^{d24}, and BR^{h24}Rⁱ²⁴; wherein said C₁₋₆ alkyl, C₂₋₆ alkenyl, C₂₋₆ alkynyl, C₃₋₆ cycloalkyl, phenyl, 5-6 membered heteroaryl and 4-7 membered heterocycloalkyl, are each optionally substituted with 1, 2, 3, or 4 substituents independently selected from R^{g};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a30}, SR^{a30}, C(O)R^{b30}, C(O)NR^{c30}R^{d30}, C(O)OR^{a30}, OC(O)R^{b30}, OC(O)NR^{c30}R^{d30},NR^{c30}R^{d30}, NR^{c30}C(O)R^{b30}, NR^{c30}C(O)OR^{a30}, NR^{c30}C(O)NR^{c30}R^{d30}, NR^{c30}S(O)R^{b30}, NR^{c30}S(O)₂R^{b30}, NR^{c30}S(O)₂NR^{c30}R^{d30}, S(O)R^{b30}, S(O)NR^{c30}R^{d30}, S(O)₂R^{b30}, S(O)₂NR^{c30}R^{d30}, and BR^{h30}Rⁱ³⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a31}, SR^{a31}, C(O)R^{b31}, C(O)NR^{c31}R^{d31}, C(O)OR^{a31}, OC(O)R^{b31}, OC(O)NR^{c31}R^{d31}, NR^{c31}R^{d31}, NR^{c31}C(O)R^{b31}, NR^{c31}C(O)OR^{a31}, NR^{c31}C(O)NR^{c31}R^{d31}, NR^{c31}S(O)R^{b31}, NR^{c31}S(O)₂R^{b31}, NR^{c31}S(O)₂NR^{c31}R^{d31}, S(O)R^{b31}, S(O)NR^{c31}R^{d31}, S(O)₂R^{b31}, S(O)₂NR^{c31}R^{d31}, and BR^{h31}Rⁱ³¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³²;
each R³² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a32}, SRa32, C(O)R^{b32}, C(O)NR^{c32}R^{d32}, C(O)OR^{a32}, OC(O)R^{b32}, OC(O)NR^{c32}R^{d32}, NR^{c32}R^{d32}, NR^{c32}C(O)R^{b32}, NR^{c32}C(O)OR^{a32}, NR^{c32}C(O)NR^{c32}R^{d32}, NR^{c32}S(O)R^{b32}, NR^{c32}S(O)₂R^{b32}, NR^{c32}S(O)₂NR^{c32}R^{d32}, S(O)R^{b32}, S(O)NR^{c32}R^{d32}, S(O)₂R^{b32}, S(O)₂NR^{c32}R^{d32}, and BR^{h32}Rⁱ³²; wherein said C₁₋₆ alkyl, C₂₋₆ alkenyl, C₂₋₆ alkynyl, C₃₋₆ cycloalkyl, phenyl, 5-6 membered heteroaryl and 4-7 membered heterocycloalkyl, are each optionally substituted with 1, 2, 3, or 4 substituents independently selected from R^{g};
each R⁵⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a50}, SR^{a50}, C(O)R^{b50}, C(O)NR^{c50}R^{d50}, C(O)OR^{a50}, OC(O)R^{b50}, OC(O)NR^{c50}R^{d50}, NR^{c50}R^{d50}, NR^{c50}C(O)R^{b50}, NR^{c50}C(O)OR^{a50}, NR^{c50}C(O)NR^{c50}R^{d50}, NR^{c50}S(O)R^{b50}, NR^{c50}S(O)₂R^{b50}, NR^{c50}S(O)₂NR^{c50}R^{d50}, S(O)R^{b50}, S(O)NR^{c50}R^{d50}, S(O)₂R^{b50}, S(O)₂NR^{c50}R^{d50}, and BR^{h50}Rⁱ⁵⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁵¹;
each R⁵¹ is independently selected from C₁₋₆ alkyl, C₂₋₆ alkenyl, C₂₋₆ alkynyl, C₁₋₆ haloalkyl, C₃₋₆ cycloalkyl, C₆₋₁₀ aryl, 5-6 membered heteroaryl, 4-7 membered heterocycloalkyl, halo, D, CN, OR^{a51}, SR^{a51}, C(O)R^{b51}, C(O)NR^{c51}R^{d51}, C(O)OR^{a51}, OC(O)R^{b51}, OC(O)NR^{c51}R^{d51}, NR^{c51}R^{d51}, NR^{c51}C(O)R^{b51}, NR^{c51}C(O)OR^{a51}, NR^{c51}C(O)NR^{c51}R^{d51}, NR^{c51}S(O)R^{b51}, NR^{c51}S(O)₂R^{b51}, NR^{c51}S(O)₂NR^{c51}R^{d51}, S(O)R^{b51}, S(O)NR^{c51}R^{d51}, S(O)₂R^{b51}, S(O)₂NR^{c51}R^{d51}, and BR^{h51}Rⁱ⁵¹; wherein said C₁₋₆ alkyl, C₂₋₆ alkenyl, C₂₋₆ alkynyl, C₃₋₆ cycloalkyl, C₆₋₁₀ aryl, 5-6 membered heteroaryl and 4-7 membered heterocycloalkyl, are each optionally substituted with 1, 2, 3, or 4 substituents independently selected from R⁵²;
each R⁵² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a52}, SR^{a52}, C(O)R^{b52}, C(O)NR^{c52}R^{d52}, C(O)OR^{a52}, OC(O)R^{b52}, OC(O)NR^{c52}R^{d52}, NR^{c52}R^{d52}, NR^{c52}C(O)R^{b52}, NR^{c52}C(O)OR^{a52}, NR^{c52}C(O)NR^{c52}R^{d52}, NR^{c52}S(O)R^{b52}, NR^{c52}S(O)₂R^{b52}, NR^{c52}S(O)₂NR^{c52}R^{d52}, S(O)R^{b52}, S(O)NR^{c52}R^{d52}, S(O)₂R^{b52}, S(O)₂NR^{c52}R^{d52}, and BR^{h52}Rⁱ⁵²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R⁶⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a60}, SR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, NR^{c60}C(O)NR^{c60}R^{d60}, NR^{c60}S(O)R^{b60}, NR^{c60}S(O)₂R^{b60}, NR^{c60}S(O)₂NR^{c60}R^{d60}, S(O)R^{b60}, S(O)NR^{c60}R^{d60}, S(O)₂R^{b60}, S(O)₂NR^{c60}R^{d60}, and BR^{h60}Rⁱ⁶⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶¹;
each R⁶¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a61}, SR^{a61}, C(O)R^{b61}, C(O)NR^{c61}R^{d61}, C(O)OR^{a61}, OC(O)R^{b61}, OC(O)NR^{c61}R^{d61}, NR^{c61}R^{d61}, NR^{c61}C(O)R^{b61}, NR^{c61}C(O)OR^{a61}, NR^{c61}C(O)NR^{c61}R^{d61}, NR^{c61}S(O)R^{b61} NR^{c61}S(O)₂R^{b61}, NR^{c61}S(O)₂NR^{c61}R^{d61}, S(O)R^{b61}, S(O)NR^{c61}R^{d61}, S(O)₂R^{b61}, S(O)₂NR^{c61}R^{d61}, and BR^{h61}Rⁱ⁶¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶²;
each R⁶² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a62}, SR^{a62}, C(O)R^{b62}, C(O)NR^{c62}R^{d62}, C(O)OR^{a62}, OC(O)R^{b62}, OC(O)NR^{c62}R^{d62}, NR^{c62}R^{d62}, NR^{c62}C(O)R^{b62}, NR^{c62}C(O)OR^{a62}, NR^{c62}C(O)NR^{c62}R^{d62}, NR^{c62}S(O)R^{b62}, NR^{c62}S(O)₂R^{b62}, NR^{c62}S(O)₂NR^{c62}R^{d62}, S(O)R^{b62}, S(O)NR^{c62}R^{d62}, S(O)₂R^{b62}, S(O)₂NR^{c62}R^{d62}, and BR^{h62}Rⁱ⁶²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R⁷⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a70}, SR^{a70}, C(O)R^{b70}, C(O)NR^{c70}R^{d70}, C(O)OR^{a70}, OC(O)R^{b70}, OC(O)NR^{c70}R^{d70}, NR^{c70}R^{d70}, NR^{c70}C(O)R^{b70}, NR^{c70}C(O)OR^{a70}, NR^{c70}C(O)NR^{c70}R^{d70}, NR^{c70}S(O)R^{b70}, NR^{c70}S(O)₂R^{b70}, NR^{c70}S(O)₂NR^{c70}R^{d70}, S(O)R^{b70}, S(O)NR^{c70}R^{d70}, S(O)₂R^{b70}, S(O)₂NR^{c70}R^{d70}, and BR^{h70}Rⁱ⁷⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷¹;
each R⁷¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a71}, SR^{a71}, C(O)R^{b71}, C(O)NR^{c71}R^{d71}, C(O)OR^{a71}, OC(O)R^{b71}, OC(O)NR^{c71}R^{d71}, NR^{c71}R^{d71}, NR^{c71}C(O)R^{b71}, NR^{c71}C(O)OR^{a71}, NR^{c71}C(O)NR^{c71}R^{d71}, NR^{c71}S(O)R^{b71}, NR^{c71}S(O)₂R^{b71}, NR^{c71}S(O)₂NR^{c71}R^{d71}, S(O)R^{b71}, S(O)NR^{c71}R^{d71}, S(O)₂R^{b71}, S(O)₂NR^{c71}R^{d71}, and BR^{h71}Rⁱ⁷¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷²;
each R⁷² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a72}, SR^{a72}, C(O)R^{b72}, C(O)NR^{c72}R^{d72}, C(O)OR^{a72}, OC(O)R^{b72}, OC(O)NR^{c72}R^{d72}, NR^{c72}R^{d72}, NR^{c72}C(O)R^{b72}, NR^{c72}C(O)OR^{a72}, NR^{c72}C(O)NR^{c72}R^{d72}, NR^{c72}S(O)R^{b72}, NR^{c72}S(O)₂R^{b72}, NR^{c72}S(O)₂NR^{c72}R^{d72}, S(O)R^{b72}, S(O)NR^{c72}R^{d72}, S(O)₂R^{b72}, S(O)₂NR^{c72}R^{d72}, and BR^{h72}Rⁱ⁷²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R^{a1}, R^{b1}, R^{c1}, and R^{d1} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R^{g};
or any R^{c1} and R^{d1} attached to the same N atom, together with the N atom to which they are attached, form a 4-, 5-, 6- or 7-membered heterocycloalkyl group optionally substituted with 1, 2 or 3 substituents independently selected from R^{g};
each R^{h1} and Rⁱ¹ is independently selected from OH, C₁₋₆ alkoxy, and C₁₋₆ haloalkoxy; or any R^{h1} and Rⁱ¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a2}, R^{b2}, R^{c2} and R^{d2} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²²;
or any R^{c2} and R^{d2} attached to the same N atom, together with the N atom to which they are attached, form a 4-, 5-, 6- or 7-membered heterocycloalkyl group optionally substituted with 1, 2, 3, or 4 substituents independently selected from R²²;
each R^{e2} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h2} and Rⁱ² is independently selected from OH, C₁₋₆ alkoxy, and C₁₋₆ haloalkoxy; or any R^{h2} and Rⁱ² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{e3} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h3} and Rⁱ³ is independently selected from OH, C₁₋₆ alkoxy, and C₁₋₆ haloalkoxy; or any R^{h3} and R³ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{j3} is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶⁰;
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R⁶⁰;
each R^{e6} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h6} and Rⁱ⁶ is independently selected from OH, C₁₋₆ alkoxy, and C₁₋₆ haloalkoxy; or any R^{h6} and Rⁱ⁶ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a7}, R^{b7}, R^{c7} and R^{d7} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷⁰;
or any R^{c7} and R^{d7} attached to the same N atom, together with the N atom to which they are attached, form a 4-, 5-, 6- or 7-membered heterocycloalkyl group optionally substituted with 1, 2, 3, or 4 substituents independently selected from R⁷⁰;
each R^{e7} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h7} and Rⁱ⁷ is independently selected from OH, C₁₋₆ alkoxy, and C₁₋₆ haloalkoxy; or any R^{h7} and Rⁱ⁷ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R¹¹;
or any R^{c10} and R^{d10} attached to the same N atom, together with the N atom to which they are attached, form a 4-, 5-, 6- or 7-membered heterocycloalkyl group optionally substituted with 1, 2, 3, or 4 substituents independently selected from R¹¹;
each R^{e10} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h10} and Rⁱ¹⁰ is independently selected from OH, C₁₋₆ alkoxy, and C₁₋₆ haloalkoxy; or any R^{h10} and Rⁱ¹⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a11}, R^{b11}, R^{c11} and R^{d11}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R¹²;
or any R^{c11} and R^{d11} attached to the same N atom, together with the N atom to which they are attached, form a 4-, 5-, 6- or 7-membered heterocycloalkyl group optionally substituted with 1, 2 or 3 substituents independently selected from R¹²;
each R^{h11} and Rⁱ¹¹ is independently selected from OH, C₁₋₆ alkoxy, and C₁₋₆ haloalkoxy; or any R^{h11} and Rⁱ¹¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a12}, R^{b12}, R^{c12} and R^{d12}, is independently selected from **H,** C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
each R^{h12} and Rⁱ¹² is independently selected from OH, C₁₋₆ alkoxy, and C₁₋₆ haloalkoxy; or any R^{h12} and Rⁱ¹² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a20}, R^{b20}, R^{c20} and R^{d20} is independently selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²¹;
or any R^{c20} and R^{d20} attached to the same N atom, together with the N atom to which they are attached, form a 4-, 5-, 6- or 7-membered heterocycloalkyl group optionally substituted with 1, 2, 3, or 4 substituents independently selected from R²¹;
each R^{e20} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h20} and Rⁱ²⁰ is independently selected from OH, C₁₋₆ alkoxy, and C₁₋₆ haloalkoxy; or any R^{h20} and Rⁱ²⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a21}, R^{b21}, R^{c21} and R^{d21}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R^{g};
or any R^{c21} and R^{d21} attached to the same N atom, together with the N atom to which they are attached, form a 4-, 5-, 6- or 7-membered heterocycloalkyl group optionally substituted with 1, 2 or 3 substituents independently selected from R^{g};
each R^{h21} and Rⁱ²¹ is independently selected from OH, C₁₋₆ alkoxy, and C₁₋₆ haloalkoxy; or any R^{h21} and Rⁱ²¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a22}, R^{b22}, R^{c22} and R^{d22} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²³;
or any R^{c22} and R^{d22} attached to the same N atom, together with the N atom to which they are attached, form a 4-, 5-, 6- or 7-membered heterocycloalkyl group optionally substituted with 1, 2, 3, or 4 substituents independently selected from R²³;
each R^{h22} and Rⁱ²² is independently selected from OH, C₁₋₆ alkoxy, and C₁₋₆ haloalkoxy; or any R^{h22} and Rⁱ²² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a23}, R^{b23}, R^{c23} and R^{d23}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R²⁴;
or any R^{c23} and R^{d23} attached to the same N atom, together with the N atom to which they are attached, form a 4-, 5-, 6- or 7-membered heterocycloalkyl group optionally substituted with 1, 2 or 3 substituents independently selected from R²⁴;
each R^{h23} and Rⁱ²³ is independently selected from OH, C₁₋₆ alkoxy, and C₁₋₆ haloalkoxy; or any R^{h23} and Rⁱ²³ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a24}, R^{b24}, R^{c24} and R^{d24}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
each R^{h24} and Rⁱ²⁴ is independently selected from OH, C₁₋₆ alkoxy, and C₁₋₆ haloalkoxy; or any R^{h24} and Rⁱ²⁴ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{h30} and Rⁱ³⁶ is independently selected from OH, C₁₋₆ alkoxy, and C₁₋₆ haloalkoxy; or any R^{h30} and Rⁱ³⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a31}, R^{b31}, R^{c31} and R^{d31}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R³²;
or any R^{c31} and R^{d31} attached to the same N atom, together with the N atom to which they are attached, form a 4-, 5-, 6- or 7-membered heterocycloalkyl group optionally substituted with 1, 2 or 3 substituents independently selected from R³²;
each R^{h31} and Rⁱ³¹ is independently selected from OH, C₁₋₆ alkoxy, and C₁₋₆ haloalkoxy; or any R^{h31} and Rⁱ³¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a32}, R^{b32}, R^{c32} and R^{d32}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c32} and R^{d32} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h32} and Rⁱ³² is independently selected from OH, C₁₋₆ alkoxy, and C₁₋₆ haloalkoxy; or any R^{h32} and Rⁱ³² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a50}, R^{b50}, R^{c50} and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁵¹;
or any R^{c50} and R^{d50} attached to the same N atom, together with the N atom to which they are attached, form a 4-, 5-, 6- or 7-membered heterocycloalkyl group optionally substituted with 1, 2 or 3 substituents independently selected from R⁵¹;
each R^{h50} and Rⁱ⁵⁰ is independently selected from OH, C₁₋₆ alkoxy, and C₁₋₆ haloalkoxy; or any R^{h50} and Rⁱ⁵⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a51}, R^{b51}, R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁵²;
or any R^{c51} and R^{d51} attached to the same N atom, together with the N atom to which they are attached, form a 4-, 5-, 6- or 7-membered heterocycloalkyl group optionally substituted with 1, 2, 3, or 4 substituents independently selected from R⁵²;
each R^{h51} and Rⁱ⁵¹ is independently selected from OH, C₁₋₆ alkoxy, and C₁₋₆ haloalkoxy; or any R^{h51} and Rⁱ⁵¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a52}, R^{b52}, R^{c52} and R^{d52}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c52} and R^{d52} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h52} and Rⁱ⁵² is independently selected from OH, C₁₋₆ alkoxy, and C₁₋₆ haloalkoxy; or any R^{h52} and Rⁱ⁵² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a60}, R^{b60}, R^{c60} and R^{d60} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶¹;
or any R^{c60} and R^{d60} attached to the same N atom, together with the N atom to which they are attached, form a 4-, 5-, 6- or 7-membered heterocycloalkyl group optionally substituted with 1, 2, 3, or 4 substituents independently selected from R⁶¹;
each R^{h60} and Rⁱ⁶⁰ is independently selected from OH, C₁₋₆ alkoxy, and C₁₋₆ haloalkoxy; or any R^{h60} and Rⁱ⁶⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a61}, R^{b61}, R^{c61} and R^{d61}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁶²;
or any R^{c61} and R^{d61} attached to the same N atom, together with the N atom to which they are attached, form a 4-, 5-, 6- or 7-membered heterocycloalkyl group optionally substituted with 1, 2 or 3 substituents independently selected from R⁶²;
each R^{h61} and Rⁱ⁶¹ is independently selected from OH, C₁₋₆ alkoxy, and C₁₋₆ haloalkoxy; or any R^{h61} and Rⁱ⁶¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a62}, R^{b62}, R^{c62} and R^{d62}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c62} and R^{d62} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h62} and Rⁱ⁶² is independently selected from OH, C₁₋₆ alkoxy, and C₁₋₆ haloalkoxy; or any R^{h62} and Rⁱ⁶² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a70}, R^{b70}, R^{c70} and R^{d70} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷¹;
or any R^{c70} and R^{d70} attached to the same N atom, together with the N atom to which they are attached, form a 4-, 5-, 6- or 7-membered heterocycloalkyl group optionally substituted with 1, 2, 3, or 4 substituents independently selected from R⁷¹;
each R^{h70} and Rⁱ⁷⁰ is independently selected from OH, C₁₋₆ alkoxy, and C₁₋₆ haloalkoxy; or any R^{h70} and Rⁱ⁷⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a71}, R^{b71}, R^{c71} and R^{d71}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁷²;
or any R^{c71} and R^{d71} attached to the same N atom, together with the N atom to which they are attached, form a 4-, 5-, 6- or 7-membered heterocycloalkyl group optionally substituted with 1, 2 or 3 substituents independently selected from R⁷²;
each R^{h71} and Rⁱ⁷¹ is independently selected from OH, C₁₋₆ alkoxy, and C₁₋₆ haloalkoxy; or any R^{h71} and Rⁱ⁷¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a72}, R^{b72}, R^{c72} and R^{d72}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c72} and R^{d72} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h72} and Rⁱ⁷² is independently selected from OH, C₁₋₆ alkoxy, and C₁₋₆ haloalkoxy; or any R^{h72} and Rⁱ⁷² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl; and
each R^{g} is independently selected from D, OH, NO₂, CN, halo, C₁₋₆ alkyl, C₂₋₆ alkenyl, C₂₋₆ alkynyl, C₁₋₆ haloalkyl, C₃₋₆ cycloalkyl, C₃₋₆ cycloalkyl-C₁₋₂ alkylene, C₁₋₆ alkoxy, C₁₋₆ haloalkoxy, C₁₋₃ alkoxy-C₁₋₃ alkyl, C₁₋₃ alkoxy-C₁₋₃ alkoxy, HO-C₁₋₃ alkoxy, HO-C₁₋₃ alkyl, cyano-C₁₋₃ alkyl, H₂N-C₁₋₃ alkyl, amino, C₁₋₆ alkylamino, di(C₁₋₆ alkyl)amino, thio, C₁₋₆ alkylthio, C₁₋₆ alkylsulfinyl, C₁₋₆ alkylsulfonyl, carbamyl, C₁₋₆ alkylcarbamyl, di(C₁₋₆ alkyl)carbamyl, carboxy, C₁₋₆ alkylcarbonyl, C₁₋₆ alkoxycarbonyl, C₁₋₆ alkylcarbonylamino, C₁₋₆ alkoxycarbonylamino, C₁₋₆ alkylcarbonyloxy, aminocarbonyloxy, C₁₋₆ alkylaminocarbonyloxy, di(C₁₋₆ alkyl)aminocarbonyloxy, C₁₋₆ alkylsulfonylamino, aminosulfonyl, C₁₋₆ alkylaminosulfonyl, di(C₁₋₆ alkyl)aminosulfonyl, aminosulfonylamino, C₁₋₆ alkylaminosulfonylamino, di(C₁₋₆ alkyl)aminosulfonylamino, aminocarbonylamino, C₁₋₆ alkylaminocarbonylamino, and di(C₁₋₆ alkyl)aminocarbonylamino;
provided that, Cy¹ is other than 3,5-dimethylisoxazol-4-yl, 3,5-dimethyl-1H-pyrazol-4-yl or 4-(1-oxo-2-propen-1-yl)phenyl.
2. The compound of clause 1, wherein
each - - - represents a single bond or a double bond;
X is N or CR⁷;
Y is N or C;
R¹ is selected from H, D, C₁₋₆ alkyl, C₁₋₆ haloalkyl, halo, CN, OR^{a1}, C(O)R^{b1}, C(O)NR^{c1}R^{d1}, C(O)OR^{a1}, OC(O)R^{b1}, OC(O)NR^{c1}R^{d1}, NR^{c1}R^{d1}, NR^{c1}C(O)R^{b1}, NR^{c1}C(O)OR^{a1}, S(O)₂R^{b1}, and S(O)₂NR^{c1}R^{d1}; wherein said C₁₋₆ alkyl, is optionally substituted with 1, 2, 3, or 4 substituents independently selected from R^{g};
R² is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a2}, SR^{a2}, C(O)R^{b2}, C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)₂R^{b2}, NR^{c2}S(O)₂NR^{c2}R^{d2}, S(O)₂R^{b2}, and S(O)₂NR^{c2}R^{d2}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, 3 or 4 substituents independently selected from R¹⁰;
when R³N- - -CR⁴ is a single bond, then R⁴ is =O; and
R³ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R³N- - -CR⁴ is a double bond, then R³ is absent; and
R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃alkylene, halo, D, CN, OR^{a3}, SR^{a3}, C(O)R^{b3}, C(0)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, NR^{c3}C(O)NR^{c3}R^{d3}, NR^{c3}S(O)₂R^{b3}, NR^{c3}S(O)₂NR^{c3}R^{d3}, S(O)₂R^{b3}, and S(O)₂NR^{c3}R^{d3}; wherein said C₁₋₆ alkyl, C₂₋₆ alkenyl, C₂₋₆ alkynyl, C₃₋₁₀cycloalkyl, 4-10 membered heterocycloalkyl, C₆₋₁₀ aryl, 5-10 membered heteroaryl, C₃₋₁₀cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when R⁵N- - -YR⁶ is a single bond and Y is C, then YR⁶ is C=O; and
R⁵ is selected from H, C₁₋₆ alkyl, C₁₋₆ haloalkyl, C₃₋₁₀ cycloalkyl, and 4-10 membered heterocycloalkyl; wherein said C₁₋₆ alkyl, C₃₋₁₀cycloalkyl, and 4-10 membered heterocycloalkyl, are each optionally substituted with 1, 2, 3, or 4 substituents independently selected from R⁵⁰;
when R⁵N- - -YR⁶ is a double bond and Y is N, then R⁵ and R⁶ are absent;
when R⁵N- - -YR⁶ is a double bond and Y is C, then R⁵ is absent; and
R⁶ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃alkylene, halo, D, CN, OR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, S(O)₂R^{b6}, and S(O)₂NR^{c6}R^{d6}; wherein said C₁₋₆ alkyl, C₂₋₆ alkenyl, C₂₋₆ alkynyl, C₃₋₁₀cycloalkyl, 4-10 membered heterocycloalkyl, C₆₋₁₀ aryl, 5-10 membered heteroaryl, C₃₋₁₀cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶⁰;
R⁷ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl, halo, D, CN, OR^{a7}, SR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7}, NR^{c7}S(O)₂R^{b7}, NR^{c7}S(O)₂NR^{c7}R^{d7}, S(O)₂R^{b7}, and S(O)₂NR^{c7}R^{d7}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁷⁰;
Cy² is selected from 4-14 membered heterocycloalkyl, each has at least one ring-forming carbon atom and 1, 2, 3, or 4 ring-forming heteroatoms independently selected from N, O, and S; wherein the N and S are optionally oxidized; wherein a ring-forming carbon atom of 4-14 membered heterocycloalkyl is optionally substituted by oxo to form a carbonyl group; and wherein the 4-14 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, halo, D, CN, OR^{a10}, SR^{a10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, NR^{c10}C(O)NR^{c10}R^{d10}, NR^{c10}S(O)₂R^{b10}, NR^{c10}S(O)₂NR^{c10}R^{d10}, S(O)₂R^{b10}, and S(O)₂NR^{c10}R^{d10}; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R¹¹;
each R¹¹ is independently selected from C₁₋₆ alkyl, C₁₋₆ haloalkyl, halo, D, CN, OR^{a11}, C(O)R^{b11}, C(O)NR^{c11}R^{d11}, C(O)OR^{a11}, OC(O)R^{b11}, OC(O)NR^{c11}R^{d11}, NR^{c11}R^{d11}, NR^{c11}C(O)R^{b11}, NR^{c11}C(O)OR^{a11}, S(O)₂R^{b11}, and S(O)₂NR^{c11}R^{d11};
each R²⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, halo, D, CN, OR^{a20}, SR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, NR^{c20}C(O)NR^{c20}R^{d20}, NR^{c20}S(O)₂R^{b20}, NR^{c20}S(O)₂NR^{c20}R^{d20}, S(O)₂R^{b20}, and S(O)₂NR^{c20}R^{d20}; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R²¹;
each R²¹ is independently selected from C₁₋₆ alkyl, C₂₋₆ alkenyl, C₂₋₆ alkynyl, C₁₋₆ haloalkyl, halo, D, CN, OR^{a21}, C(O)R^{b21}, C(O)NR^{c21}R^{d21}, C(O)OR^{a21}, OC(O)R^{b21}, OC(O)NW^{c21}R^{d21}, NR^{c21}R^{d21}, NR^{c21}C(O)R^{b21}, NR^{c21}C(O)OR^{a21}, S(O)₂R^{b21}, and S(O)₂NR^{c21}R^{d21}; wherein said C₁₋₆ alkyl, C₂₋₆ alkenyl, and C₂₋₆ alkynyl, are each optionally substituted with 1, 2, 3, or 4 substituents independently selected from R^{g};
each R²² is independently selected from C₁₋₆ alkyl, C₁₋₆ haloalkyl, halo, D, CN, OR^{a22}, C(O)R^{b22}, C(O)NR^{c22}R^{d22}, C(O)OR^{a22}, OC(O)R^{b22}, OC(O)NR^{c22}R^{d22}, NR^{c22}R^{d22}, NR^{c22}C(O)R^{b22}, NR^{c22}C(O)OR^{a22}, S(O)₂R^{b22}, and S(O)₂NR^{c22}R^{d22};
each R³⁰ is independently selected from C₁₋₆ alkyl, C₂₋₆ alkenyl, C₂₋₆ alkynyl, C₁₋₆ haloalkyl, C₃₋₁₀cycloalkyl, 4-10 membered heterocycloalkyl, C₆₋₁₀ aryl, 5-10 membered heteroaryl, C₃₋₁₀cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a30}, SR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, S(O)₂R^{b30}, and S(O)₂NR^{c30}R^{d30}; wherein said C₁₋₆ alkyl, C₂₋₆ alkenyl, C₂₋₆ alkynyl, C₃₋₁₀cycloalkyl, 4-10 membered heterocycloalkyl, C₆₋₁₀aryl, 5-10 membered heteroaryl, C₃₋₁₀cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl, halo, D, CN, OR^{a31}, C(O)R^{b31}, C(O)NR^{c31}R^{d31}, C(O)OR^{a31}, OC(O)R^{b31}, OC(O)NR^{c31}R^{d31}, NR^{c31}R^{d31}, NR^{c31}C(O)R^{b31}, NR^{c31}C(O)OR^{a31}, S(O)₂R^{b31}, and S(O)₂NR^{c31}R^{d31}; wherein said C₁₋₆ alkyl, C₂₋₆ alkenyl, C₂₋₆ alkynyl, C₃₋₁₀cycloalkyl, and 4-10 membered heterocycloalkyl, are each optionally substituted with 1, 2, 3, or 4 substituents independently selected from R³²;
each R³² is independently selected from C₁₋₆ alkyl, C₁₋₆ haloalkyl, halo, D, CN, OR^{a32}, C(O)R^{b32}, C(O)NR^{c32}R^{d32}, C(O)OR^{a32}, OC(O)R^{b32}, OC(O)NR^{c32}R^{d32}, NR^{c32}R^{d32}, NR^{c32}C(O)R^{b32}, NR^{c32}C(O)OR^{a32}, S(O)₂R^{b32}, and S(O)₂NR^{c32}R^{d32};
each R⁵⁰ is independently selected from C₁₋₆ alkyl, C₁₋₆ haloalkyl, C₃₋₁₀cycloalkyl, 4-10 membered heterocycloalkyl, C₆₋₁₀ aryl, 5-10 membered heteroaryl, halo, D, CN, OR^{a50}, C(O)R^{b50}, C(O)NR^{c50}R^{d50}, C(O)OR^{a50}, OC(O)R^{b50}, OC(O)NR^{c50}R^{d50}, NR^{c50}R^{d50}, NR^{c50}C(O)R^{b50}, NR^{c50}C(O)OR^{a50}, S(O)₂R^{b50}, and S(O)₂NR^{c50}R^{d50}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁵¹;
each R⁵¹ is independently selected from C₁₋₆ alkyl, C₁₋₆ haloalkyl, halo, D, CN, OR^{a51}, C(O)R^{b51}, C(O)NR^{c51}R^{d51}, C(O)OR^{a51}, OC(O)R^{b51}, OC(O)NR^{c51}R^{d51}, NR^{c51}R^{d51}, NR^{c51}C(O)R^{b51}, NR^{c51}C(O)OR^{a51}, S(O)₂R^{b51}, and S(O)₂NR^{c51}R^{d51}; wherein said C₁₋₆ alkyl, are each optionally substituted with 1, 2, 3, or 4 substituents independently selected from R⁵²;
each R⁵² is independently selected from C₁₋₆ alkyl, C₁₋₆ haloalkyl, halo, D, CN, OR^{a52}, C(O)R^{b52}, C(O)NR^{c52}R^{d52}, C(O)OR^{a52}, OC(O)R^{b52}, OC(O)NR^{c52}R^{d52}, NR^{c52}R^{d52}, NR^{c52}C(O)R^{b52}, NR^{c52}C(O)OR^{a52}, S(O)₂R^{b52}, and S(O)₂NR^{c52}R^{d52};
each R⁶⁰ is independently selected from C₁₋₆ alkyl, C₂₋₆ alkenyl, C₂₋₆ alkynyl, C₁₋₆ haloalkyl, C₃₋₁₀cycloalkyl, 4-10 membered heterocycloalkyl, C₆₋₁₀ aryl, 5-10 membered heteroaryl, halo, D, CN, OR^{a60}, SR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, S(O)₂R^{b60}, and S(O)₂NR^{c60}R^{d60}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁶¹;
each R⁶¹ is independently selected from C₁₋₆ alkyl, C₁₋₆ haloalkyl, halo, D, CN, OR^{a61}, C(O)R^{b61}, C(O)NR^{c61}R^{d61}, C(O)OR^{a61}, OC(O)R^{b61}, OC(O)NR^{c61}R^{d61}, NR^{c61}R^{d61}, NR^{c61}C(O)R^{b61}, NR^{c61}C(O)OR^{a61}, S(O)₂R^{b61}, and S(O)₂NR^{c61}R^{d61};
each R⁷⁰ is independently selected from C₁₋₆ alkyl, C₁₋₆ haloalkyl, halo, D, CN, OR^{a70}, C(O)R^{b70}, C(O)NR^{c70}R^{d70}, C(O)OR^{a70}, OC(O)R^{b70}, OC(O)NR^{c70}R^{d70}, NR^{c70}R^{d70}, NR^{c70}C(O)R^{b70}, NR^{c70}C(O)OR^{a70}, S(O)₂R^{b70}, and S(O)₂NR^{c70}R^{d70};
each R^{a1}, R^{b1}, R^{c1}, and R^{d1} is independently selected from H, C₁₋₆ alkyl, C₁₋₆ haloalkyl; wherein said C₁₋₆ alkyl, are each optionally substituted with 1, 2, 3, or 4 substituents independently selected from R⁹;
each R^{a2}, R^{b2}, R^{c2} and R^{d2} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²²;
or any R^{c2} and R^{d2} attached to the same N atom, together with the N atom to which they are attached, form a 4-, 5-, 6- or 7-membered heterocycloalkyl group optionally substituted with 1, 2, 3, or 4 substituents independently selected from R²²;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{j3} is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶⁰;
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R⁶⁰;
each R^{a7}, R^{b7}, R^{c7} and R^{d7} is independently selected from H, C₁₋₆ alkyl, and C₁₋₆ haloalkyl; wherein said C₁₋₆ alkyl, are each optionally substituted with 1, 2, 3, or 4 substituents independently selected from R⁷⁰;
each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl; wherein said C₁₋₆ alkyl, C₂₋₆ alkenyl, C₂₋₆ alkynyl, C₃₋₁₀ cycloalkyl, and 4-10 membered heterocycloalkyl are each optionally substituted with 1, 2, 3, or 4 substituents independently selected from R¹¹;
or any R^{c10} and R^{d10} attached to the same N atom, together with the N atom to which they are attached, form a 4-, 5-, 6- or 7-membered heterocycloalkyl group optionally substituted with 1, 2, 3, or 4 substituents independently selected from R¹¹;
each R^{a11}, R^{b11}, R^{c11} and R^{d11}, is independently selected from H, C₁₋₆ alkyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R²¹;
or any R^{c20} and R^{d20} attached to the same N atom, together with the N atom to which they are attached, form a 4-, 5-, 6- or 7-membered heterocycloalkyl group optionally substituted with 1, 2, 3, or 4 substituents independently selected from R²¹;
each R^{a21}, R^{b21}, R^{c21} and R^{d21}, is independently selected from H, C₁₋₆ alkyl, C₂₋₆ alkenyl, C₂₋₆ alkynyl, C₁₋₆ haloalkyl; wherein said C₁₋₆ alkyl C₂₋₆ alkenyl, and C₂₋₆ alkynyl, are each optionally substituted with 1, 2, 3, or 4 substituents independently selected from R^{g};
each R^{a22}, R^{b22}, R^{c22} and R^{d22} is independently selected from H, C₁₋₆ alkyl, and C₁₋₆ haloalkyl;
each R^{a30}, R^{b30}, R^{c30} and R^{d30} is independently selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{a31}, R^{b31}, R^{c31} and R^{d31}, is independently selected from **H,** C₁₋₆ alkyl, C₂₋₆ alkenyl, C₂₋₆ alkynyl, C₁₋₆ haloalkyl, C₃₋₆ cycloalkyl, and 4-7 membered heterocycloalkyl; wherein said C₁₋₆ alkyl C₂₋₆ alkenyl, C₂₋₆ alkynyl, C₃₋₆ cycloalkyl, and 4-7 membered heterocycloalkyl are each optionally substituted with 1, 2, 3, or 4 substituents independently selected from R³²;
or any R^{c31} and R^{d31} attached to the same N atom, together with the N atom to which they are attached, form a 4-, 5-, 6- or 7-membered heterocycloalkyl group optionally substituted with 1, 2 or 3 substituents independently selected from R³²;
each R^{a32}, R^{b32}, R^{c32} and R^{d32}, is independently selected from **H,** C₁₋₆ alkyl, and C₁₋₆ haloalkyl;
each R^{a50}, R^{b50} R^{c50} and R^{d50}, is independently selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁵¹;
or any R^{c50} and R^{d50} attached to the same N atom, together with the N atom to which they are attached, form a 4-, 5-, 6- or 7-membered heterocycloalkyl group optionally substituted with 1, 2 or 3 substituents independently selected from R⁵¹;
each R^{a51}, R^{b51}, R^{c51} and R^{d51}, is independently selected from **H,** C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁵²;
each R^{a52}, R^{b52}, R^{c52} and R^{d52}, is independently selected from **H,** C₁₋₆ alkyl, and C₁₋₆ haloalkyl;
each R^{a60}, R^{b60}, R^{c60} and R^{d60} is independently selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶¹;
or any R^{c60} and R^{d60} attached to the same N atom, together with the N atom to which they are attached, form a 4-, 5-, 6- or 7-membered heterocycloalkyl group optionally substituted with 1, 2, 3, or 4 substituents independently selected from R⁶¹;
each R^{a61}, R^{b61}, R^{c61} and R^{d61}, is independently selected from **H,** C₁₋₆ alkyl, and C₁₋₆ haloalkyl;
each R^{a70}, R^{b70}, R^{c70} and R^{d70} is independently selected from H, C₁₋₆ alkyl, and C₁₋₆ haloalkyl; and
each R⁹ is independently selected from D, OH, CN, halo, C₁₋₆ alkyl, C₂₋₆ alkenyl, C₂₋₆ alkynyl, C₁₋₆ haloalkyl, C₃₋₆ cycloalkyl, C₃₋₆ cycloalkyl-C₁₋₂ alkylene, C₁₋₆ alkoxy, C₁₋₆ haloalkoxy, C₁₋₃ alkoxy-C₁₋₃ alkyl, C₁₋₃ alkoxy-C₁₋₃ alkoxy, HO-C₁₋₃ alkoxy, HO-C₁₋₃ alkyl, cyano-C₁₋₃ alkyl, H₂N-C₁₋₃ alkyl, amino, C₁₋₆ alkylamino, di(C₁₋₆ alkyl)amino, C₁₋₆ alkylthio, C₁₋₆ alkylsulfonyl, C₁₋₆ alkylcarbamyl, di(C₁₋₆ alkyl)carbamyl, carboxy, C₁₋₆ alkylcarbonyl, C₁₋₆ alkoxycarbonyl, C₁₋₆ alkylcarbonylamino, C₁₋₆ alkoxycarbonylamino, C₁₋₆ alkylcarbonyloxy, and di(C₁₋₆ alkyl)aminosulfonyl.
3. The compound of clause 1 or 2, or pharmaceutically acceptable salt thereof, wherein
each - - - represents a single bond or a double bond;
X is Nor CR⁷;
Y is N or C;
R' is selected from **H,** D, C₁₋₆ alkyl, C₁₋₆ haloalkyl, halo, and CN;
R² is selected from H, C₁₋₆ alkyl, C₁₋₆ haloalkyl, halo, D, CN, OR^{a2}, and NR^{c2}R^{d2}; wherein said C₁₋₆ alkyl, is optionally substituted with 1 or 2, substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, 3 or 4 substituents independently selected from R¹⁰;
when R³N- - -CR⁴ is a single bond, then R⁴ is =O; and
R³ is selected from **H,** C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R³N- - -CR⁴ is a double bond, then R³ is absent; and
R⁴ is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, and S(O)₂R^{b3}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰;
when R⁵N- - -YR⁶ is a single bond and Y is C, then YR⁶ is C=O; and
R⁵ is selected from H, C₁₋₆ alkyl, and C₁₋₆ haloalkyl; wherein said C₁₋₆ alkyl, are each optionally substituted with 1, 2, 3, or 4 substituents independently selected from R⁵⁰;
when R⁵N- - -YR⁶ is a double bond and Y is N, then R⁵ and R⁶ are absent;
when R⁵N- - -YR⁶ is a double bond and Y is C, then R⁵ is absent; and
R⁶ is selected from H, C₁₋₆ alkyl, and C₁₋₆ haloalkyl;
R⁷ is selected from H, C₁₋₆ alkyl, C₁₋₆ haloalkyl, halo, D, CN, OR^{a7}, and NR^{c7}R^{d7};
Cy² is selected from 4-10 membered heterocycloalkyl, each has at least one ring-forming carbon atom and 1, 2, 3, or 4 ring-forming heteroatoms independently selected from N, O, and S; wherein a ring-forming carbon atom of 4-10 membered heterocycloalkyl is optionally substituted by oxo to form a carbonyl group; and wherein the 4-10 membered heterocycloalkyl, is optionally substituted with 1, 2, 3 or 4 substituents independently selected from R²⁰;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, halo, D, CN, OR^{a10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, and S(O)₂R^{b10};
each R²⁰ is independently selected from C₁₋₆ alkyl, C₂₋₆ alkenyl, C₂₋₆ alkynyl, C₁₋₆ haloalkyl, halo, D, CN, OR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, and S(O)₂R^{b20}; wherein said C₁₋₆ alkyl, C₂₋₆ alkenyl, and C₂₋₆ alkynyl, are each optionally substituted with 1, 2, 3, or 4 substituents independently selected from R²¹;
each R²¹ is independently selected from C₁₋₆ alkyl, C₁₋₆ haloalkyl, halo, D, CN, OR^{a21}, and NR^{c21}R^{d21};
each R²² is independently selected from C₁₋₆ alkyl, C₁₋₆ haloalkyl, halo, D, and CN;
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, and S(O)₂R^{b30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³¹;
each R³¹ is independently selected from C₁₋₆ alkyl, C₁₋₆ haloalkyl, halo, D, CN, OR^{a31}, and NR^{c31}R^{d31};
each R⁵⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a50}, and NR^{c50}R^{d50}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁵¹;
each R⁵¹ is independently selected from C₁₋₆ alkyl, C₁₋₆ haloalkyl, halo, D, CN, OR^{a51,} and NR^{c51}R^{d51};
each R^{a2}, R^{c2} and R^{d2} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl;
or any R^{c2} and R^{d2} attached to the same N atom, together with the N atom to which they are attached, form a 4-, 5-, 6- or 7-membered heterocycloalkyl group;
each R^{a3}, R^{b3}, R^{c3} and R^{d3} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{j3} is independently selected from C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a7}, R^{c7} and R^{d7} is independently selected from H, C₁₋₆ alkyl, and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, and C₁₋₆ haloalkyl; wherein said C₁₋₆ alkyl, C₂₋₆ alkenyl, and C₂₋₆ alkynyl, are each optionally substituted with 1, 2, 3, or 4 substituents independently selected from R²¹;
each R^{a21}, R^{c21} and R^{d21}, is independently selected from H, C₁₋₆ alkyl, and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{a31}, R^{c31} and R^{d31}, is independently selected from H, C₁₋₆ alkyl, and C₁₋₆ haloalkyl;
each R^{a50}, R^{c50} and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁵¹;
or any R^{c50} and R^{d50} attached to the same N atom, together with the N atom to which they are attached, form a 4-, 5-, or 6-membered heterocycloalkyl group optionally substituted with 1, 2 or 3 substituents independently selected from R⁵¹; and
each R^{a51}, R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl.
4. The compound of clause 1, wherein the compound of Formula I is a compound of Formula II: 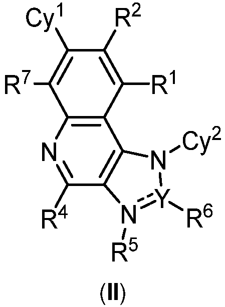 or a pharmaceutically acceptable salt thereof.
5. The compound of clause 4, or a pharmaceutically acceptable salt thereof, wherein
each - - - represents a single bond or a double bond;
X is N or CR⁷;
Y is N or C;
R¹ is selected from H, D, C₁₋₃ alkyl, C₁₋₃ haloalkyl, halo, and CN;
R² is selected from H, C₁₋₃ alkyl, C₁₋₃ haloalkyl, halo, D, and CN; wherein said C₁₋₃ alkyl, is optionally substituted with 1 or 2, substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, or 3 ring-forming heteroatoms independently selected from N and O; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2 or 3 substituents independently selected from R¹⁰;
when R³N- - -CR⁴ is a single bond, then R⁴ is =O; and
R³ is selected from H, C₁₋₃ alkyl, C₁₋₃ haloalkyl, phenyl, and 5-6 membered heteroaryl; wherein said C₁₋₃ alkyl, phenyl, and 5-6 membered heteroaryl, are each optionally substituted with 1 or 2 substituents independently selected from R³⁰;
when R³N- - -CR⁴ is a double bond, then R³ is absent; and
R⁴ is selected from H, C₁₋₃ alkyl, C₁₋₃ haloalkyl, C₃₋₆ cycloalkyl, 4-6 membered heterocycloalkyl, C₆₋₁₀ aryl, 5-6 membered heteroaryl, halo, D, CN, OR^{a3}, and NR^{c3}R^{j3}; wherein said C₁₋₃ alkyl, C₃₋₆ cycloalkyl, 4-6 membered heterocycloalkyl, C₆₋₁₀ aryl, and 5-6 membered heteroaryl, are each optionally substituted with 1 or 2 substituents independently selected from R³⁰;
when R⁵N- - -YR⁶ is a single bond and Y is C, then YR⁶ is C=O; and
R⁵ is selected from H, C₁₋₃ alkyl, and C₁₋₃ haloalkyl; wherein said C₁₋₃ alkyl, is optionally substituted with 1 or 2 substituents independently selected from R⁵⁰;
when R⁵N- - -YR⁶ is a double bond and Y is N, then R⁵ and R ⁶ are absent;
when R⁵N- - -YR⁶ is a double bond and Y is C, then R⁵ is absent; and R⁶ is selected from H, C₁₋₃ alkyl, and C₁₋₃ haloalkyl;
R⁷ is selected from H, C₁₋₃ alkyl, C₁₋₃ haloalkyl, halo, D, and CN;
Cy² is selected from 4-6 membered heterocycloalkyl, each has at least one ring-forming carbon atom and 1, 2, 3, or 4 ring-forming heteroatoms independently selected from N, O, and S; wherein a ring-forming carbon atom of 4-6 membered heterocycloalkyl is optionally substituted by oxo to form a carbonyl group; and wherein the 4-6 membered heterocycloalkyl, is optionally substituted with 1 or 2 substituents independently selected from R²⁰;
each R¹⁰ is independently selected from C₁₋₃ alkyl, C₁₋₃ haloalkyl, C₃₋₅ cycloalkyl, halo, D, CN, OR^{a10}, and NR^{c10}R^{d10};
each R²⁰ is independently selected from C₁₋₃ alkyl, C₂₋₃ alkenyl, C₂₋₃ alkynyl, C₁₋₃ haloalkyl, halo, D, CN, OR^{a20}, C(O)R^{b20}, C(O)NR^{c20}R^{d20}, C(O)OR^{a20}, and NR^{c20}R^{d20}; wherein said C₁₋₃ alkyl, C₂₋₃ alkenyl, and C₂₋₃ alkynyl, are each optionally substituted with 1 or 2 substituents independently selected from R²¹;
each R²¹ is independently selected from C₁₋₃ alkyl, C₁₋₃ haloalkyl, halo, D, CN OR^{a21}, and NR^{c21}R^{d21};
each R²² is independently selected from C₁₋₆ alkyl, C₁₋₆ haloalkyl, halo, D, and CN;
each R³⁰ is independently selected from C₁₋₃ alkyl, C₁₋₃ haloalkyl, C₃₋₆ cycloalkyl, 4-6 membered heterocycloalkyl, phenyl, 5-6 membered heteroaryl, halo, D, CN, OR^{a30}, and NR^{c30}R^{d30}; wherein said C₁₋₃ alkyl, C₃₋₆ cycloalkyl, 4-6 membered heterocycloalkyl, phenyl, and 5-6 membered heteroaryl, are each optionally substituted with 1 or 2 substituents independently selected from R³¹;
each R³¹ is independently selected from C₁₋₃ alkyl, C₁₋₃ haloalkyl, halo, D, and CN;
each R⁵⁰ is independently selected from C₁₋₃ alkyl, C₁₋₃ haloalkyl, C₃₋₆ cycloalkyl, 4-6 membered heterocycloalkyl, halo, D, and CN; wherein said C₁₋₃ alkyl, C₃₋₆ cycloalkyl, and 4-6 membered heterocycloalkyl, are each optionally substituted with 1 or 2 substituents independently selected from R⁵¹;
each R⁵¹ is independently selected from C₁₋₃ alkyl, C₁₋₃ haloalkyl, halo, D, and CN;
each R^{a3} and R^{c3} is independently selected from **H,** C₁₋₃ alkyl, and C₁₋₃ haloalkyl; wherein said C₁₋₃ alkyl, is optionally substituted with 1 or 2 substituents independently selected from R³⁰;
each R^{j3} is independently selected from C₁₋₃ alkyl, and C₁₋₃ haloalkyl; wherein said C₁₋₃ alkyl, is optionally substituted with 1 or 2 substituents independently selected from R³⁰;
each R^{a10}, R^{b10}, R^{c10} and R^{d10} is independently selected from **H,** C₁₋₃ alkyl, and C₁₋₃ haloalkyl;
each R^{a20}, R^{b20}, R^{c20} and R^{d20} is independently selected from **H,** C₁₋₃ alkyl, C₂₋₃ alkenyl, C₂₋₃ alkynyl, and C₁₋₃ haloalkyl; wherein said C₁₋₃ alkyl, C₂₋₃ alkenyl, and C₂₋₃ alkynyl, are each optionally substituted with 1 or 2 substituents independently selected from R²¹;
each R^{a21}, R^{c21} and R^{d21}, is independently selected from **H,** C₁₋₆ alkyl, and C₁₋₆ haloalkyl; and
each R^{a30}, R^{c30} and R^{d30} is independently selected from H, C₁₋₃ alkyl, and C₁₋₃ haloalkyl; wherein said C₁₋₃ alkyl, are each optionally substituted with 1 or 2 substituents independently selected from R³¹.
6. The compound of clause 1 or 2, or a pharmaceutically acceptable salt thereof, wherein
each - - - represents a single bond or a double bond;
X is N or CR⁷;
Y is N or C;
R¹ is H;
R² is selected from H, C₁₋₆ alkyl, C₁₋₆ haloalkyl, halo, and CN; wherein said C₁₋₆ alkyl, is optionally substituted with 1 or 2, substituents independently selected from R²²;
Cy¹ is selected from C₃₋₁₀ cycloalkyl, 4-10 membered heterocycloalkyl, C₆₋₁₀ aryl and 6-10 membered heteroaryl; each optionally substituted with 1, 2, 3 or 4 substituents independently selected from R¹⁰;
when R³N- - -CR⁴ is a single bond, then R⁴ is =O; and
R³ is selected from C₁₋₆ alkyl, C₆₋₁₀ aryl, and 5-10 membered heteroaryl; each optionally substituted with 1, 2, 3, or 4 substituents independently selected from R³⁰;
when R³N- - -CR⁴ is a double bond, then R³ is absent; and
R⁴ is selected from H, 4-10 membered heterocycloalkyl, C₆₋₁₀ aryl, 5-10 membered heteroaryl, and OR^{a3}; wherein said C₁₋₆ alkyl, 4-10 membered heterocycloalkyl, and C₆₋₁₀ aryl, are each optionally substituted with 1, 2, 3, or 4 substituents independently selected from R³⁰;
when R^{S}N- - -YR⁶ is a single bond and Y is C, then YR⁶ is C=O; and
R⁵ is selected from H, and C₁₋₆ alkyl; wherein said C₁₋₆ alkyl, is optionally substituted with 1, 2, 3, or 4 substituents independently selected from R⁵⁰;
when R⁵N- - -YR⁶ is a double bond and Y is N, then R⁵ and R⁶ are absent;
when R⁵N- - -YR⁶ is a double bond and Y is C, then R⁵ is absent; and R⁶ is selected from H;
R⁷ is selected from H and halo;
Cy² is selected from 4-10 membered heterocycloalkyl, optionally substituted with 1, 2, 3 or 4 substituents independently selected from R²⁰;
each R¹⁰ is independently selected from C₁₋₆ alkyl, C₁₋₆ haloalkyl, C₃₋₁₀ cycloalkyl, halo, CN, and OR^{a10};
each R²⁰ is independently selected from C₁₋₆ alkyl, CN, OR^{a20}, C(O)R^{b20}, and C(O)NR^{c20}R^{d20}; wherein said C₁₋₆ alkyl, is optionally substituted with 1, 2, 3, or 4 substituents independently selected from R²¹;
each R²¹ is independently selected from C₁₋₆ haloalkyl, halo, CN, OR^{a21}, and NR^{c21}R^{d21};
each R²² is CN;
each R³⁰ is independently selected from C₁₋₆ alkyl, 4-10 membered heterocycloalkyl, 5-10 membered heteroaryl, halo, OR^{a30}, and NR^{c30}R^{d30}; wherein said C₁₋₆ alkyl, 4-10 membered heterocycloalkyl, and 5-10 membered heteroaryl, are each optionally substituted with 1, 2, 3, or 4 substituents independently selected from R³¹;
each R³¹ is C₁₋₆ alkyl;
each R⁵⁶ is 4-10 membered heterocycloalkyl, optionally substituted with 1, 2, 3, or 4 substituents independently selected from R⁵¹;
each R⁵¹ is C₁₋₆ alkyl;
each R^{a3}, is C₁₋₆ alkyl, optionally substituted with 1, 2, 3, or 4 substituents independently selected from R³⁰;
each R^{a16}, is independently selected from H, and C₁₋₆ alkyl;
each R^{a20}, R^{b20}, R^{c20} and R^{d20} is independently selected from H, C₁₋₆ alkyl, C₂₋₆ alkenyl and C₂₋₆ alkynyl; wherein said C₁₋₆ alkyl, C₂₋₆ alkenyl, and C₂₋₆ alkynyl are each optionally substituted with 1, 2, 3, or 4 substituents independently selected from R²¹;
each R^{a21}, R^{c21} and R^{d21}, is independently selected from H and C₁₋₆ alkyl; and
each R^{a30}, R^{c30} and R^{d30} is independently selected from H, and C₁₋₆ alkyl.
7. The compound of any one of clauses 4-6, wherein the compound of Formula II is a compound of Formula Ila: 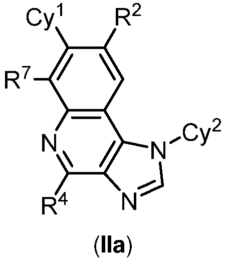 or a pharmaceutically acceptable salt thereof.
8. The compound of any one of clauses 4-6, wherein the compound of Formula II is a compound of Formula IIb: 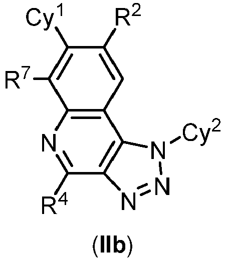 or a pharmaceutically acceptable salt thereof.
9. The compound of any one of clauses 4-6, wherein the compound of Formula II is a compound of Formula llc: 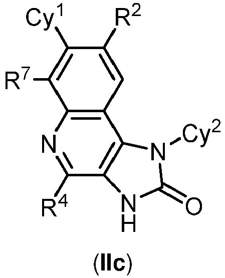 or a pharmaceutically acceptable salt thereof.
10. The compound of any one of clauses 4-7, wherein the compound of Formula I is a compound of Formula Ig: 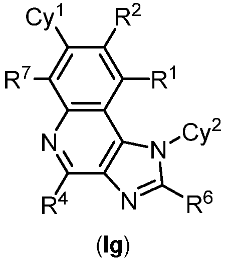 or a pharmaceutically acceptable salt thereof.
11. The compound of any one of clauses 4-7, wherein the compound of Formula I is a compound of Formula le: 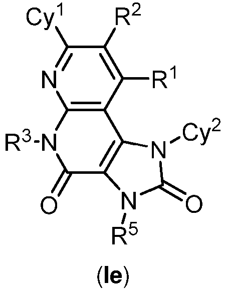 or a pharmaceutically acceptable salt thereof.
12. The compound of any one of clauses 1-5, wherein R¹ is selected from H, D, and C₁₋₃ alkyl.
13. The compound of any one of clauses 1-5, wherein R¹ is H.
14. The compound of clause 1 or 4, wherein R² is selected from H, C₁₋₃ alkyl, C₁₋₃ haloalkyl, phenyl, 5-6 membered heteroaryl, halo, D, and CN.
15. The compound of clause 14, wherein R² is selected from C₁₋₆ alkyl, C₁₋₆ haloalkyl, and halo.
16. The compound of clause 1 or 4, wherein Cy¹ is selected from C₆₋₁₀ aryl and 6-10 membered heteroaryl; wherein the 6-10 membered heteroaryl each has at least one ring-forming carbon atom and 1, 2, or 3 ring-forming heteroatoms independently selected from N, O, and S; wherein the N and S are optionally oxidized; wherein a ring-forming carbon atom of 6-10 membered heteroaryl is optionally substituted by oxo to form a carbonyl group; and wherein the C₆₋₁₀ aryl and 6-10 membered heteroaryl are each optionally substituted with 1, 2, or 3 substituents independently selected from R¹⁰.
17. The compound of clause 16, wherein Cy¹ is selected from phenyl, naphthalenyl, quinolinyl, isoquinolinyl, indazolyl, chromanyl, 2,3-dihydro-1H-indenyl, 2,3-dihydrobenzo[b][1,4]dioxinyl, and pyridinyl; wherein the phenyl, naphthalenyl, quinolinyl, isoquinolinyl, indazolyl, chromanyl, 2,3-dihydro-1H-indenyl, 2,3-dihydrobenzo[b][1,4]dioxinyl, and pyridinyl are each optionally substituted with 1, 2, or 3 substituents independently selected from R¹⁰.
18. The compound of any one of clauses 1-3, wherein X is N.
19. The compound of any one of clauses 1-3, wherein X is CR⁷.
20. The compound of clause 19, wherein R⁷ is halo.
21. The compound of 19 or 20, wherein R⁷ is fluoro.
22. The compound of any one of clauses 1-3, wherein when R³N- - -CR⁴ is a single bond, then R⁴ is =O.
23. The compound of any one of clauses 1-3, wherein when R³N- - -CR⁴ is a double bond, then R³ is absent.
24. The compound of any one of clauses 1-3, wherein R³ is selected from H, C₁₋₆ alkyl, C₁₋₆ haloalkyl, phenyl, and 5-6 membered heteroaryl; wherein said C₁₋₆ alkyl, phenyl, and 5-6 membered heteroaryl, are each optionally substituted with 1 or 2 substituents independently selected from R³⁰.
25. The compound of clause 24, wherein R³ is selected from C₁₋₃ alkyl, phenyl, and 5-6 membered heteroaryl; wherein said C₁₋₃ alkyl, phenyl, and 5-6 membered heteroaryl, are each optionally substituted with 1 or 2 substituents independently selected from R³⁰.
26. The compound of clause 1 or 4, wherein R⁴ is selected from H, C₁₋₆ alkyl, C₁₋₆ haloalkyl, 4-10 membered heterocycloalkyl, C₆₋₁₀ aryl, 5-10 membered heteroaryl, halo, D, CN, OR^{a3}, and NR^{c3}R^{j3}; wherein said C₁₋₆ alkyl, 4-10 membered heterocycloalkyl, C₆₋₁₀ aryl, and 5-10 membered heteroaryl, are each optionally substituted with 1, 2, or 3 substituents independently selected from R³⁰.
27. The compound of clause 26, wherein R⁴ is selected from H, 4-10 membered heterocycloalkyl, C₆₋₁₀ aryl, 5-10 membered heteroaryl, and OR^{a3}; wherein said 4-10 membered heterocycloalkyl, C₆₋₁₀ aryl, and 5-10 membered heteroaryl are each optionally substituted with 1 or 2 substituents independently selected from R³⁰.
28. The compound of any one of clauses 1-6, wherein Y is N.
29. The compound of any one of clauses 1-6, wherein Y is C.
30. The compound of any one of clauses 1-6, wherein when R⁵N- - -YR⁶ is a single bond, Y is C, and YR⁶ is C=O.
31. The compound of any one of clauses 1-6, wherein when R⁵N- - -YR⁶ is a double bond and Y is C, then R⁵ is absent.
32. The compound of any one of clauses 1-6, wherein when R⁵N- - -YR⁶ is a double bond, Y is N, and R⁵ and R⁶ are absent.
33. The compound of any one of clauses 1-6, wherein R⁵ is selected from H, C₁₋₆ alkyl, and C₁₋₆ haloalkyl; wherein said C₁₋₆ alkyl is optionally substituted by 1 or 2 substituents independently selected from R⁵⁰.
34. The compound of clause 33, wherein R⁵ is H.
35. The compound of any one of clauses 1-6, wherein R⁶ is selected from H, C₁₋₃ alkyl, 4-6 membered heterocycloalkyl, and 5-6 membered heteroaryl; wherein said C₁₋₃ alkyl, 4-6 membered heterocycloalkyl, and 5-6 membered heteroaryl, are each optionally substituted with 1 or 2 substituents independently selected from R⁶⁰.
36. The compound of clause 35, wherein R⁶ is H.
37. The compound of clause 1 or 4, wherein Cy² is 4-6 membered heterocycloalkyl; wherein the 4-6 membered heterocycloalkyl has at least one ring-forming carbon atom and 1 or 2 ring-forming heteroatoms independently selected from N, O, and S; wherein a ring-forming carbon atom of 4-6 membered heterocycloalkyl is optionally substituted by oxo to form a carbonyl group; and wherein the 4-6 membered heterocycloalkyl, is optionally substituted with 1, 2, 3 or 4 substituents independently selected from R²⁰.
38. The compound of clause 37, wherein Cy² is selected from:

| | | | |
|---|---|---|---|
| 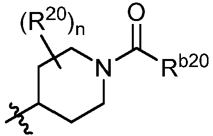 | 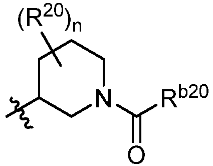 | 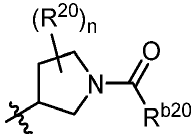 | 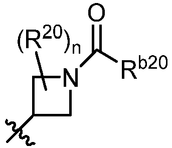 |
| Cy²-a1, | Cy²-b1, | Cy²-c1, and | Cy²-d1, |

wherein n is 0, 1 or 2.
39. The compound of any one of clauses 1-11, wherein the compound of Formula I is selected from:
1-(4-(8-chloro-6-fluoro-7-(3-hydroxynaphthalen-1-yl)-4-((1-methylpyrrolidin-2-yl)-methoxy)-1H-imidazo[4,5-c]quinolin-1-yl)piperidin-1-yl)prop-2-en-1-one;
1-(4-(8-chloro-6-fluoro-7-(2-fluoro-6-hydroxyphenyl)-4-((1-methylpyrrolidin-2-yl)-methoxy)-1H-imidazo[4,5-c]quinolin-1-yl)piperidin-1-yl)prop-2-en-1-one;
1-(4-(8-chloro-6-fluoro-7-(2-fluoro-3-hydroxyphenyl)-4-((1-methylpyrrolidin-2-yl)-methoxy)-1H-imidazo[4,5-c]quinolin-1-yl)piperidin-1-yl)prop-2-en-1-one;
1-(4-(8-chloro-6-fluoro-7-(5-methyl-1H-indazol-4-yl)-4-((1-methylpyrrolidin-2-yl)-methoxy)-1H-imidazo[4,5-c]quinolin-1-yl)piperidin-1-yl)prop-2-en-1-one;
1-(3-(8-chloro-6-fluoro-7-(3-hydroxynaphthalen-1-yl)-4-((1-methylpyrrolidin-2-yl)-methoxy)-1H-imidazo[4,5-c]quinolin-1-yl)piperidin-1-yl)prop-2-en-1-one;
1-(3-(8-chloro-6-fluoro-7-(3-hydroxynaphthalen-1-yl)-4-((1-methylpyrrolidin-2-yl)-methoxy)-1H-imidazo[4,5-c]quinolin-1-yl)azetidin-1-yl)prop-2-en-1-one;
1-(3-(8-chloro-6-fluoro-7-(3-hydroxynaphthalen-1-yl)-4-((1-methylpyrrolidin-2-yl)-methoxy)-1H-imidazo[4,5-c]quinolin-1-yl)pyrrolidin-1-yl)prop-2-en-1-one;
1-(4-(8-chloro-6-fluoro-7-(3-hydroxynaphthalen-1-yl)-4-((1-methyl-1H-pyrazol-5-yl)-methoxy)-1H-imidazo[4,5-c]quinolin-1-yl)piperidin-1-yl)prop-2-en-1-one;
1-(4-(8-chloro-4-(3-(dimethylamino)azetidin-1-yl)-6-fluoro-7-(3-hydroxynaphthalen-1-yl)-1H-imidazo[4,5-c]quinolin-1-yl)piperidin-1-yl)prop-2-en-1-one;
1-(1-acryloylpiperidin-4-yl)-8-chloro-6-fluoro-7-(3-hydroxynaphthalen-1-yl)-4-((1-methyl-pyrrolidin-2-yl)methoxy)-1,3-dihydro-2H-imidazo[4,5-c]quinolin-2-one;
1-(4-(8-chloro-6-fluoro-4,7-bis(3-hydroxynaphthalen-1-yl)-1H-imidazo[4,5-c]quinolin-1-yl)piperidin-1-yl)prop-2-en-1-one;
1-(4-(8-chloro-6-fluoro-4,7-bis(2-fluoro-6-hydroxyphenyl)-1H-imidazo[4,5-c]quinolin-1-yl)piperidin-1-yl)prop-2-en-1-one;
1-(4-(8-chloro-6-fluoro-7-(2-fluoro-6-hydroxyphenyl)-1H-imidazo[4,5-c]quinolin-1-yl)-piperidin-1-yl)prop-2-en-1-one; and
1-(4-(8-chloro-6-fluoro-7-(3-hydroxynaphthalen-1-yl)-4-((1-methylpyrrolidin-2-yl)-methoxy)-1H-[1,2,3]triazolo[4,5-c]quinolin-1-yl)piperidin-1-yl)prop-2-en-1-one.
40. The compound of any one of clauses 1-11, wherein the compound of Formula I is selected from:
4-(1-((*endo*)-2-azabicyclo[2.1.1]hexan-5-yl)-8-chloro-4-(3-(dimethylamino)azetidin-1-yl)-6-fluoro-2-methyl-1*H*-imidazo[4,5-*c*]quinolin-7-yl)naphthalen-2-ol;
2-(1-acryloyl-4-(8-chloro-4-(3-(dimethylamino)azetidin-1-yl)-7-(2,3-dimethylphenyl)-6-fluoro-1*H*-imidazo[4,5-*c*]quinolin-1-yl)piperidin-2-yl)acetonitrile;
3-(1-(1-acryloyl-2-(cyanomethyl)piperidin-4-yl)-8-chloro-4-(3-(dimethylamino)azetidin-1-yl)-6-fluoro-1*H*-imidazo[4,5-*c*]quinolin-7-yl)-2-methylbenzonitrile;
2-(1-acryloyl-4-(8-chloro-4-(3-(dimethylamino)azetidin-1-yl)-6-fluoro-7-(3-fluoro-2-methyl-phenyl)-1*H*-imidazo[4,5-*c*]quinolin-1-yl)piperidin-2-yl)acetonitrile;
2-(1-acryloyl-4-(8-chloro-7-(2-chloro-3-methylphenyl)-4-(3-(dimethylamino)-azetidin-1-yl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(*o*-tolyl)-1H-imidazo[4,5-*c*]quinolin-1-yl)piperidin-2-yl)acetonitrile;
1-acryloyl-4-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)piperidine-2-carboxamide;
1-acryloyl-4-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)piperidine-2-carbonitrile;
4-(1-((*endo*)-2-azabicyclo[2.1.1]hexan-5-yl)-8-chloro-4-(3-(dimethylamino)azetidin-1-yl)-6-fluoro-1*H*-imidazo[4,5-*c*]quinolin-7-yl)-6-chloronaphthalen-2-ol;
4-(1-((*endo*)-2-azabicyclo[2.1.1]hexan-5-yl)-8-chloro-4-(3-(dimethylamino)-azetidin-1-yl)-6-fluoro-1*H*-imidazo[4,5-*c*]quinolin-7-yl)-6-methylnaphthalen-2-ol;
4-(1-((*endo*)-2-azabicyclo[2.1.1]hexan-5-yl)-4-(3-(dimethylamino)azetidin-1-yl)-6-fluoro-8-(1*H*-pyrazol-4-yl)-1*H*-imidazo[4,5-*c*]quinolin-7-yl)naphthalen-2-ol;
1-(1-acryloylazetidin-3-yl)-8-fluoro-7-(3-hydroxynaphthalen-1-yl)-5-(2-isopropyl-4-methyl-pyridin-3-yl)-3,5-dihydro-1*H*-imidazo[4,5-*c*][1,8]naphthyridine-2,4-dione;
1-(1-acryloylazetidin-3-yl)-8-fluoro-7-(3-hydroxynaphthalen-1-yl)-5-(2-isopropyl-4-methyl-pyridin-3-yl)-3-((1-methylpyrrolidin-2-yl)methyl)-3,5-dihydro-1*H*-imidazo[4,5-*c*][1,8]naphthyridine-2,4-dione;
1-(1-acryloylazetidin-3-yl)-8-fluoro-7-(3-hydroxynaphthalen-1-yl)-5-phenyl-3,5-dihydro-1*H*-imidazo[4,5-*c*][1,8]naphthyridine-2,4-dione;
1-(3-(8-chloro-6-fluoro-7-(3-hydroxynaphthalen-1-yl)-4-((1-methylpyrrolidin-2-yl)-methoxy)-1*H*-imidazo[4,5-*c*]quinolin-1-yl)-4-methylpyrrolidin-1-yl)prop-2-en-1-one;
1-((*trans*)-3-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)-4-methoxypyrrolidin-1-yl)prop-2-en-1-one;
1-((*trans*)-3-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)-4-methylpyrrolidin-1-yl)prop-2-en-1-one;
2-(1-acryloyl-4-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)pyrrolidin-2-yl)acetonitrile;
1-(1-acryloylpyrrolidin-3-yl)-8-chloro-6-fluoro-7-(3-hydroxynaphthalen-1-yl)-5-methyl-1,5-dihydro-4*H*-imidazo[4,5-*c*]quinolin-4-one;
4-(1-((*endo*)-2-azabicyclo[2.1.1]hexan-5-yl)-4-(3-(dimethyl-amino)azetidin-1-yl)-6-fluoro-8-methyl-1*H*-imidazo[4,5-*c*]quinolin-7-yl)naphthalen-2-ol;
4-(1-((*endo*)-2-azabicyclo[2.1.1]hexan-5-yl)-4-(3,8-diazabicyclo[3.2.1]octan-8-yl)-8-chloro-6-fluoro-1*H*-imidazo[4,5-*c*]quinolin-7-yl)naphthalen-2-ol;
2-(1-acryloyl-4-(8-chloro-6-fluoro-7-(3-hydroxynaphthalen-1-yl)-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-6-fluoro-7-(5-methyl-1*H*-indazol-4-yl)-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-6-fluoro-4-(((*S*)-1-methylpyrrolidin-2-yl)methoxy)-7-(naphthalen-1-yl)-1*H*-imidazo[4,5-*c*]quinolin-1-yl)piperidin-2-yl)acetonitrile;
2-(1-acryloyl-4-(8-chloro-6-fluoro-7-(5-hydroxy-2-methylphenyl)-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-7-(6-chloro-5-methyl-1*H*-indazol-4-yl)-6-fluoro-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-7-(3-chloro-2-methylphenyl)-6-fluoro-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-6-fluoro-7-(3-fluoro-2-methylphenyl)-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-7-(2,3-dimethylphenyl)-6-fluoro-4-((1-methyl-pyrrolidin-2-yl)methoxy)-1*H*-imidazo[4,5-*c*]quinolin-1-yl)piperidin-2-yl)acetonitrile;
2-(1-acryloyl-4-(8-chloro-7-(2,3-dihydro-1*H*-inden-4-yl)-6-fluoro-4-((1-methylpyrrolidin-2-yl)methoxy)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-phenyl-1*H-*imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(naphthalen-1-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(3-hydroxy-naphthalen-1-yl)-1*H*-imidazo[4,5-*c*]quinolin-1-yl)piperidin-2-yl)acetonitrile;
4-(1-((*endo*)-2-azabicyclo[2.1.1]hexan-5-yl)-8-chloro-4-(3-(dimethylamino)azetidin-1-yl)-6-fluoro-1*H*-imidazo[4,5-*c*]quinolin-7-yl)naphthalen-2-ol;
4-(1-((*endo*)-2-azabicyclo[2.1.1]hexan-5-yl)-8-chloro-4-(3-(dimethylamino)-azetidin-1-yl)-2-((dimethylamino)methyl)-6-fluoro-1*H*-imidazo[4,5-*c*]quinolin-7-yl)naphthalen-2-ol;
1-((*endo*)-2-azabicyclo[2.1.1]hexan-5-yl)-4-(3-(dimethylamino)azetidin-1-yl)-6-fluoro-7-(3-hydroxynaphthalen-1-yl)-1*H*-imidazo[4,5-*c*]quinoline-8-carbonitrile;
4-(1-((*endo*)-2-azabicyclo[2.1.1]hexan-5-yl)-4-(3-(dimethylamino)azetidin-1-yl)-6-fluoro-1*H*-imidazo[4,5-*c*]quinolin-7-yl)naphthalen-2-ol;
2-(1-acryloyl-4-(8-chloro-4-(3-(dimethylamino)azetidin-1-yl)-6-fluoro-7-(5-hydroxy-2,3-dimethylphenyl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(5-hydroxy-2-methylphenyl)-1*H*-imidazo[4,5-*c*]quinolin-1-yl)piperidin-2-yl)acetonitrile;
2-(1-acryloyl-4-(8-chloro-7-(2-chloro-5-hydroxyphenyl)-4-(3-(dimethylamino)azetidin-1-yl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(2-fluoro-5-hydroxyphenyl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(2-fluorophenyl)-1*H*-imidazo[4,5-*c*]quinolin-1-yl)piperidin-2-yl)acetonitrile;
2-(1-acryloyl-4-(8-chloro-7-(2-chlorophenyl)-4-(3-(dimethylamino)-azetidin-1-yl)-6-fluoro-1*H*-imidazo[4,5-*c*]quinolin-1-yl)piperidin-2-yl)acetonitrile;
2-(1-acryloyl-4-(8-chloro-7-(2-chloro-3-fluorophenyl)-4-(3-(dimethyl-amino)azetidin-1-yl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-7-(2,4-dimethylphenyl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-7-(2,5-dimethylphenyl)-6-fluoro-1*H*-imidazo[4,5-*c*]quinolin-1-yl)piperidin-2-yl)acetonitrile;
2-(1-acryloyl-4-(8-chloro-7-(3-chloro-2-methylphenyl)-4-(3-(dimethyl-amino)azetidin-1-yl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-7-(2,3-dimethylphenyl)-6-fluoro-1*H*-imidazo[4,5-*c*]quinolin-1-yl)pyrrolidin-2-yl)acetonitrile;
2-(1-acryloyl-4-(8-chloro-4-(3-(dimethylamino)azetidin-1-yl)-6-fluoro-7-(naphthalen-1-yl)-1*H*-imidazo[4,5-*c*]quinolin-1-yl)pyrrolidin-2-yl)acetonitrile;
2-(1-acryloyl-4-(8-chloro-4-(3-(dimethylamino)azetidin-1-yl)-6-fluoro-7-(5-fluoroquinolin-8-yl)-1*H*-imidazo[4,5-*c*]quinolin-1-yl)piperidin-2-yl)acetonitrile;
2-(1-acryloyl-4-(8-chloro-7-(chroman-8-yl)-4-(3-(dimethylamino)-azetidin-1-yl)-6-fluoro-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(2-methoxy-naphthalen-1-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(4-fluoro-naphthalen-1-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(8-methyl-naphthalen-1-yl)-1*H*-imidazo[4,5-*c*]quinolin-1-yl)piperidin-2-yl)acetonitrile;
2-(1-acryloyl-4-(8-chloro-7-(2,3-dihydrobenzo[*b*][1,4]dioxin-5-yl)-4-(3-(dimethylamino)-azetidin-1-yl)-6-fluoro-1H-imidazo[4,5-c]quinolin-1-yl)piperidin-2-yl)acetonitrile;
2-(1-acryloyl-4-(8-chloro-4-(3-(dimethylamino)azetidin-1-yl)-6-fluoro-7-(isoquinolin-5-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(isoquinolin-8-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(quinolin-8-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(isoquinolin-4-yl)-1*H*-imidazo[4,5-*c*]quinolin-1-yl)piperidin-2-yl)acetonitrile;
2-(1-acryloyl-4-(8-chloro-4-(3-(dimethylamino)azetidin-1-yl)-6-fluoro-7-(quinolin-4-yl)-1*H*-imidazo[4,5-*c*]quinolin-1-yl)piperidin-2-yl)acetonitrile;
4-(1-((*endo*)-2-azabicyclo[2.1.1]hexan-5-yl)-8-chloro-4-(3-(dimethylamino)-azetidin-1-yl)-6-fluoro-2-(hydroxymethyl)-1*H*-imidazo[4,5-*c*]quinolin-7-yl)naphthalen-2-ol;
4-(2-(2-aminoethyl)-1-((*endo*)-2-azabicyclo[2.1.1]hexan-5-yl)-8-chloro-4-(3-(dimethyl-amino)azetidin-1-yl)-6-fluoro-1*H*-imidazo[4,5-*c*]quinolin-7-yl)naphthalen-2-ol;
4-(1-((*endo*)-2-azabicyclo[2.1.1]hexan-5-yl)-8-chloro-4-(3-(dimethylamino)azetidin-1-yl)-6-fluoro-2-(piperidin-4-ylmethyl)-1*H*-imidazo[4,5-*c*]quinolin-7-yl)naphthalen-2-ol;
4-(1-((*endo*)-2-azabicyclo[2.1.1]hexan-5-yl)-8-chloro-4-(3-(dimethylamino)-azetidin-1-yl)-6-fluoro-2-(1-methyl-1*H*-imidazol-4-yl)-1*H*-imidazo[4,5-*c*]quinolin-7-yl)naphthalen-2-ol; and
4-(1-((*endo*)-2-azabicyclo[2.1.1]hexan-5-yl)-8-chloro-4-(3-(dimethylamino)azetidin-1-yl)-6-fluoro-7-(3-hydroxynaphthalen-1-yl)-1*H*-imidazo[4,5-c]quinolin-2-yl)tetrahydro-2*H-*thiopyran 1,1-dioxide.
41. The compound of any one of clauses 1-11, wherein the compound of Formula I is selected from:
2-((2*S*,4*S*)-4-(8-chloro-7-(6-chloro-5-methyl-1*H*-indazol-4-yl)-4-(3-(dimethylamino)-3-methylazetidin-1-yl)-6-fluoro-1*H*-imidazo[4,5-*c*]quinolin-1-yl)-1-((*E*)-4-methoxybut-2-enoyl)piperidin-2-yl)acetonitrile;
2-((2*S*,4*S*)-1-(but-2-ynoyl)-4-(8-chloro-7-(6-chloro-5-methyl-1*H*-indazol-4-yl)-4-(3-(dimethylamino)-3-methylazetidin-1-yl)-6-fluoro-1*H*-imidazo[4,5-*c*]quinolin-1-yl)piperidin-2-yl)acetonitrile;
2-((2*S*,4*S*)-1-(but-2-ynoyl)-4-(8-chloro-7-(6-chloro-5-methyl-1*H*-indazol-4-yl)-4-(3-(dimethylamino)-3-methylazetidin-1-yl)-6-fluoro-1*H*-imidazo[4,5-*c*]quinolin-1-yl)piperidin-2-yl)acetonitrile;
2-((2*S*,4*S*)-1-(but-2-ynoyl)-4-(8-chloro-7-(6-chloro-5-methyl-1*H*-indazol-4-yl)-6-fluoro-4-(5-methyl-2,5-diazaspiro[3.4]octan-2-yl)-1*H*-imidazo[4,5-*c*]quinolin-1-yl)piperidin-2-yl)acetonitrile;
2-((2*S*,4*S*)-4-(8-chloro-7-(6-chloro-5-methyl-1*H*-indazol-4-yl)-6-fluoro-4-(5-methyl-2,5-diazaspiro[3.4]octan-2-yl)-1*H*-imidazo[4,5-*c*]quinolin-1-yl)-1-((*E*)-4-methoxybut-2-enoyl)piperidin-2-yl)acetonitrile;
2-((2*S*,4*S*)-1-acryloyl-4-(8-chloro-7-(3-chloro-4-fluorophenyl)-6-fluoro-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-1-acryloyl-4-(8-chloro-7-(3-chloro-2-methylphenyl)-6-fluoro-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-1-(but-2-ynoyl)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-4-(8-chloro-7-(3-chloro-2-methylphenyl)-6-fluoro-4-(((*S*)-1-methylpyrrolidin-2-yl)methoxy)-1*H*-[1,2,3]triazolo[4,5-*c*]quinolin-1-yl)-1-((*E*)-4-(dimethylamino)but-2-enoyl)piperidin-2-yl)acetonitrile;
2-((2*S*,4*S*)-1-acryloyl-4-(8-chloro-7-(5-chloro-4-methylpyridin-3-yl)-4-(3-(dimethylamino)azetidin-1-yl)-6-fluoro-1*H*-imidazo[4,5-*c*]quinolin-1-yl)piperidin-2-yl)acetonitrile;
2-((2*S*,4*S*)-1-acryloyl-4-(8-chloro-7-(5-chloro-2-methoxy-4-methylpyridin-3-yl)-4-(3-(dimethylamino)azetidin-1-yl)-6-fluoro-1*H*-imidazo[4,5-*c*]quinolin-1-yl)piperidin-2-yl)acetonitrile;
2-((2*S*,4*S*)-1-(but-2-ynoyl)-4-(8-chloro-7-(3-chloro-2-methylphenyl)-6-fluoro-4-(3-oxomorpholino)-1*H*-[1,2,3]triazolo[4,5-*c*]quinolin-1-yl)piperidin-2-yl)acetonitrile;
2-((2*S*,4*S*)-1-(but-2-ynoyl)-4-(8-chloro-7-(3-chloro-2-methylphenyl)-6-fluoro-4-(4-methyl-2-oxopiperazin-1-yl)-1*H*-[1,2,3]triazolo[4,5-*c*]quinolin-1-yl)piperidin-2-yl)acetonitrile;
2-((2*S*,4*S*)-1-(but-2-ynoyl)-4-(8-chloro-7-(3-chloro-2-methylphenyl)-6-fluoro-4-(2-oxopiperidin-1-yl)-1*H*-[1,2,3]triazolo[4,5-*c*]quinolin-1-yl)piperidin-2-yl)acetonitrile;
2-((2*S*,4*S*)-1-(but-2-ynoyl)-4-(8-chloro-7-(3-chloro-2-methylphenyl)-4-(((*S*)-1-(dimethylamino)propan-2-yl)oxy)-6-fluoro-1*H*-[1,2,3]triazolo[4,5-*c*]quinolin-1-yl)piperidin-2-yl)acetonitrile;
3-(1-((2*S*,4*S*)-1-acryloyl-2-(cyanomethyl)piperidin-4-yl)-8-chloro-6-fluoro-4-(((*S*)-1-methylpyrrolidin-2-yl)methoxy)-1*H*-[1,2,3]triazolo[4,5-*c*]quinolin-7-yl)-2-methylbenzonitrile;
3-(1-((2*S*,4*S*)-1-(but-2-ynoyl)-2-(cyanomethyl)piperidin-4-yl)-8-chloro-6-fluoro-4-(((*S*)-1-methylpyrrolidin-2-yl)methoxy)-1*H*-[1,2,3]triazolo[4,5-*c*]quinolin-7-yl)-2-methylbenzonitrile;
3-(8-chloro-1-((2*S*,4*S*)-2-(cyanomethyl)-1-((*E*)-4-(dimethylamino)but-2-enoyl)piperidin-4-yl)-6-fluoro-4-(((*S*)-1-methylpyrrolidin-2-yl)methoxy)-1*H*-[1,2,3]triazolo[4,5-*c*]quinolin-7-yl)-2-methylbenzonitrile;
2-((2*S*,4*S*)-1-acryloyl-4-(7-(3-chloro-2-methylphenyl)-6-fluoro-8-methyl-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-1-(but-2-ynoyl)-4-(7-(3-chloro-2-methylphenyl)-6-fluoro-8-methyl-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-4-(7-(3-chloro-2-methylphenyl)-6-fluoro-8-methyl-4-(((*S*)-1-methylpyrrolidin-2-yl)methoxy)-1*H*-[1,2,3]triazolo[4,5-*c*]quinolin-1-yl)-1-((*E*)-4-methoxybut-2-enoyl)piperidin-2-yl)acetonitrile;
2-((2*S*,4*S*)-1-acryloyl-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)piperidin-2-yl)acetonitrile;
2-((2*S*,4*S*)-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)-1-((*E*)-4,4-difluorobut-2-enoyl)piperidin-2-yl)acetonitrile;
2-((2*S*,4*S*)-1-(but-2-ynoyl)-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)piperidin-2-yl)acetonitrile;
2-((2*S*,4*S*)-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)-1-((*E*)-4-fluorobut-2-enoyl)piperidin-2-yl)acetonitrile;
2-((2*S*,4*S*)-4-(7-(3-chloro-2-methylphenyl)-4-(3-(dimethylamino)-3-methylazetidin-1-yl)-6-fluoro-8-methyl-1*H*-[1,2,3]triazolo[4,5-*c*]quinolin-1-yl)-1-((*E*)-4-(dimethylamino)but-2-enoyl)piperidin-2-yl)acetonitrile;
2-((2*S*,4*S*)-1-acryloyl-4-(8-chloro-4-(3-(dimethylamino)azetidin-1-yl)-6-fluoro-7-(2-(trifluoromethyl)phenyl)-11*H*-imidazo[4,5-*c*]quinolin-1-yl)piperidin-2-yl)acetonitrile;
2-((2*S*,4*S*)-1-acryloyl-4-(8-chloro-7-(2,3-dichlorophenyl)-4-(3-(dimethylamino)azetidin-1-yl)-6-fluoro-1*H*-imidazo[4,5-*c*]quinolin-1-yl)piperidin-2-yl)acetonitrile;
2-((2*S*,4*S*)-1-acryloyl-4-(8-chloro-4-(3-(dimethylamino)azetidin-1-yl)-6-fluoro-7-(4-methylpyridin-3-yl)-1*H*-imidazo[4,5-*c*]quinolin-1-yl)piperidin-2-yl)acetonitrile;
2-((2*S*,4*S*)-1-acryloyl-4-(8-chloro-4-(3-(dimethylamino)azetidin-1-yl)-6-fluoro-7-(m-tolyl)-1*H-*imidazo[4,5-*c*]quinolin-1-yl)piperidin-2-yl)acetonitrile;
2-((2*S*,4*S*)-1-acryloyl-4-(8-chloro-4-(3-(dimethylamino)azetidin-1-yl)-7-(4,5-dimethylpyridin-3-yl)-6-fluoro-1*H*-imidazo[4,5-*c*]quinolin-1-yl)piperidin-2-yl)acetonitrile;
2-((2*S*,4*S*)-1-acryloyl-4-(8-chloro-4-(3-(dimethylamino)azetidin-1-yl)-7-(2,3-dimethylphenyl)-6-fluoro-1*H*-[1,2,3]triazolo[4,5-*c*]quinolin-1-yl)piperidin-2-yl)acetonitrile;
2-((2*S*,4*S*)-1-acryloyl-4-(8-chloro-7-(3-chloro-2-methylphenyl)-4-(3-(dimethylamino)azetidin-1-yl)-6-fluoro-1*H*-[1,2,3]triazolo[4,5-*c*]quinolin-1-yl)piperidin-2-yl)acetonitrile;
2-((2*S*,4*S*)-1-acryloyl-4-(8-chloro-7-(2,3-dichlorophenyl)-4-(3-(dimethylamino)azetidin-1-yl)-6-fluoro-1*H*-[1,2,3]triazolo[4,5-*c*]quinolin-1-yl)piperidin-2-yl)acetonitrile;
2-((2*S*,4*S*)-1-acryloyl-4-(8-chloro-4-(3-(dimethylamino)azetidin-1-yl)-6-fluoro-7-(m-tolyl)-1*H*-[1,2,3]triazolo[4,5-*c*]quinolin-1-yl)piperidin-2-yl)acetonitrile;
3-(1-((2*S*,4*S*)-1-acryloyl-2-(cyanomethyl)piperidin-4-yl)-8-chloro-4-(3-(dimethylamino)-azetidin-1-yl)-6-fluoro-1*H*-[1,2,3]triazolo[4,5-*c*]quinolin-7-yl)-2-methylbenzonitrile;
2-((2*S*,4*S*)-1-acryloyl-4-(8-chloro-4-(3-(dimethylamino)azetidin-1-yl)-6-fluoro-7-(4-fluoro-2,3-dimethylphenyl)-1*H*-[1,2,3]triazolo[4,5-*c*]quinolin-1-yl)piperidin-2-yl)acetonitrile;
2-((2*S*,4*S*)-1-acryloyl-4-(8-chloro-7-(2-cyclopropylphenyl)-4-(3-(dimethylamino)azetidin-1-yl)-6-fluoro-1*H*-[1,2,3]triazolo[4,5-*c*]quinolin-1-yl)piperidin-2-yl)acetonitrile;
2-((2*S*,4*S*)-1-acryloyl-4-(8-chloro-7-(3-chloro-2-methylphenyl)-6-fluoro-4-(((*S*)-1-methylpyrrolidin-2-yl)methoxy)-11*H*-imidazo[4,5-*c*]quinolin-1-yl)piperidin-2-yl)acetonitrile;
2-((2*S*,4*S*)-1-acryloyl-4-(8-chloro-7-(3-chloro-2-methylphenyl)-6-fluoro-4-(((*R*)-1-methylpyrrolidin-2-yl)methoxy)-11*H*-imidazo[4,5-*c*]quinolin-1-yl)piperidin-2-yl)acetonitrile;
2-((2*S*,4*S*)-1-acryloyl-4-(8-chloro-4-(3-(dimethylamino)-3-methylazetidin-1-yl)-6-fluoro-7-(4-fluorophenyl)-1*H*-imidazo[4,5-*c*]quinolin-1-yl)piperidin-2-yl)acetonitrile;
2-((2*S*,4*S*)-1-acryloyl-4-(4-(3-(dimethylamino)-3-methylazetidin-1-yl)-6-fluoro-7-(4-fluorophenyl)-8-methyl-11*H*-imidazo[4,5-*c*]quinolin-1-yl)piperidin-2-yl)acetonitrile;
2-((2*S*,4*S*)-1-acryloyl-4-(8-chloro-4-(((*S*)-1-(dimethylamino)propan-2-yl)oxy)-6-fluoro-7-(4-fluorophenyl)-1*H*-imidazo[4,5-*c*]quinolin-1-yl)piperidin-2-yl)acetonitrile;
2-((2*S*,4*S*)-1-acryloyl-4-(8-chloro-6-fluoro-7-(4-fluorophenyl)-4-(2-methyl-1*H*-imidazol-1-yl)-1*H*-imidazo[4,5-*c*]quinolin-1-yl)piperidin-2-yl)acetonitrile;
2-((2*S*,4*S*)-4-(8-chloro-6-fluoro-7-(4-fluorophenyl)-4-(2-methyl-1*H*-imidazol-1-yl)-1*H-*imidazo[4,5-c]quinolin-1-yl)-1-((*E*)-4-(dimethylamino)but-2-enoyl)piperidin-2-yl)acetonitrile;
2-((2*S*,4*S*)-4-(8-chloro-6-fluoro-7-(4-fluorophenyl)-4-(2-methyl-1*H*-imidazol-1-yl)-1*H-*imidazo[4,5-*c*]quinolin-1-yl)-1-((*E*)-4-fluorobut-2-enoyl)piperidin-2-yl)acetonitrile;
2-((2*S*,4*S*)-1-acryloyl-4-(8-chloro-4-(3-(dimethylamino)-3-methylazetidin-1-yl)-6-fluoro-7-(4-fluorophenyl)-1*H*-[1,2,3]triazolo[4,5-*c*]quinolin-1-yl)piperidin-2-yl)acetonitrile;
2-((2*S*,4*S*)-1-acryloyl-4-(4-(3-(dimethylamino)-3-methylazetidin-1-yl)-6-fluoro-7-(4-fluorophenyl)-8-methyl-*H*-[1,2,3]triazolo[4,5-*c*]quinolin-1-yl)piperidin-2-yl)acetonitrile;
2-((2*S*,4*S*)-1-acryloyl-4-(8-chloro-4-(((*S*)-1-(dimethylamino)propan-2-yl)oxy)-6-fluoro-7-(4-fluorophenyl)-1*H*-[1,2,3]triazolo[4,5-*c*]quinolin-1-yl)piperidin-2-yl)acetonitrile;
2-((2*S*,4*S*)-1-(but-2-ynoyl)-4-(8-chloro-4-(((*S*)-1-(dimethylamino)propan-2-yl)oxy)-6-fluoro-7-(4-fluorophenyl)-1*H*-[1,2,3]triazolo[4,5-*c*]quinolin-1-yl)piperidin-2-yl)acetonitrile;
2-((2*S*,4*S*)-4-(8-chloro-4-(((*S*)-1-(dimethylamino)propan-2-yl)oxy)-6-fluoro-7-(4-fluorophenyl)-1*H*-[1,2,3]triazolo[4,5-*c*]quinolin-1-yl)-1-((*E*)-4-fluorobut-2-enoyl)piperidin-2-yl)acetonitrile;
2-((2*S*,4*S*)-1-acryloyl-4-(8-chloro-6-fluoro-7-(4-fluorophenyl)-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-1-acryloyl-4-(4-(((*S*)-1-(dimethylamino)propan-2-yl)oxy)-6-fluoro-7-(4-fluorophenyl)-8-methyl-1*H*-[1,2,3]triazolo[4,5-*c*]quinolin-1-yl)piperidin-2-yl)acetonitrile;
2-((2*S*,4*S*)-1-(but-2-ynoyl)-4-(4-(((*S*)-1-(dimethylamino)propan-2-yl)oxy)-6-fluoro-7-(4-fluorophenyl)-8-methyl-1*H*-[1,2,3]triazolo[4,5-*c*]quinolin-1-yl)piperidin-2-yl)acetonitrile;
2-((2*S*,4*S*)-4-(4-(((*S*)-1-(dimethylamino)propan-2-yl)oxy)-6-fluoro-7-(4-fluorophenyl)-8-methyl-1*H*-[1,2,3]triazolo[4,5-*c*]quinolin-1-yl)-1-((*E*)-4-fluorobut-2-enoyl)piperidin-2-yl)acetonitrile;
2-((2*S*,4*S*)-1-acryloyl-4-(6-fluoro-7-(4-fluorophenyl)-8-methyl-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-4-(6-fluoro-7-(4-fluorophenyl)-8-methyl-4-(((*S*)-1-methylpyrrolidin-2-yl)methoxy)-1*H*-[1,2,3]triazolo[4,5-*c*]quinolin-1-yl)-1-((*E*)-4-fluorobut-2-enoyl)piperidin-2-yl)acetonitrile;
2-((2*S*,4*S*)-1-acryloyl-4-(7-(2,3-dimethylphenyl)-6-fluoro-8-methyl-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-4-(7-(2,3-dimethylphenyl)-6-fluoro-8-methyl-4-(((*S*)-1-methylpyrrolidin-2-yl)methoxy)-1*H*-[1,2,3]triazolo[4,5-*c*]quinolin-1-yl)-1-((*E*)-4-fluorobut-2-enoyl)piperidin-2-yl)acetonitrile;
2-((2*S*,4*S*)-1-(but-2-ynoyl)-4-(7-(2,3-dimethylphenyl)-6-fluoro-8-methyl-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-4-(7-(2,3-dimethylphenyl)-6-fluoro-8-methyl-4-(((*S*)-1-methylpyrrolidin-2-yl)methoxy)-1*H*-[1,2,3]triazolo[4,5-*c*]quinolin-1-yl)-1-((*E*)-4-methoxybut-2-enoyl)piperidin-2-yl)acetonitrile;
2-((2*S*,4*S*)-1-((*E*)-4,4-difluorobut-2-enoyl)-4-(7-(2,3-dimethylphenyl)-6-fluoro-8-methyl-4-(((*S*)-1-methylpyrrolidin-2-yl)methoxy)-1*H*-[1,2,3]triazolo[4,5-*c*]quinolin-1-yl)piperidin-2-yl)acetonitrile;
2-((2*S*,4*S*)-1-acryloyl-4-(4-(((*S*)-1-(dimethylamino)propan-2-yl)oxy)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4S*)*-4-(4-(((*S*)-1-(dimethylamino)propan-2-yl)oxy)-7-(2,3-dimethylphenyl)-6-fluoro-8-methyl-1*H*-[1,2,3]triazolo[4,5-*c*]quinolin-1-yl)-1-((*E*)-4-fluorobut-2-enoyl)piperidin-2-yl)acetonitrile;
2-((2*S*,4*S*)-1-((*E*)-4,4-difluorobut-2-enoyl)-4-(4-(((*S*)-1-(dimethylamino)-propan-2-yl)oxy)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4*S*)-4-(4-(((*S*)-1-(dimethylamino)propan-2-yl)oxy)-7-(2,3-dimethylphenyl)-6-fluoro-8-methyl-1*H*-[1,2,3]triazolo[4,5-*c*]quinolin-1-yl)-1-((*E*)-4-methoxybut-2-enoyl)piperidin-2-yl)acetonitrile;
2-((2*S*,4*S*)-1-(but-2-ynoyl)-4-(4-(((*S*)-1-(dimethylamino)propan-2-yl)oxy)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4*S*)-1-acryloyl-4-(4-(3-(dimethylamino)azetidin-1-yl)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4*S*)-1-((*E*)-4,4-difluorobut-2-enoyl)-4-(4-(3-(dimethylamino)-azetidin-1-yl)-7-(2,3-dimethylphenyl)-6-fluoro-8-methyl-1*H*-[1,2,3]triazolo[4,5-*c*]quinolin-1-yl)piperidin-2-yl)acetonitrile;
2-((2*S*,4*S*)-4-(8-chloro-4-(3-(dimethylamino)azetidin-1-yl)-7-(2,3-dimethylphenyl)-6-fluoro-1*H*-imidazo[4,5-c]quinolin-1-yl)-1-((*E*)-4-fluorobut-2-enoyl)piperidin-2-yl)acetonitrile;
2-((2*S*,4*S*)-1-acryloyl-4-(8-chloro-7-(6-chloro-1,5-dimethyl-1*H*-indazol-4-yl)-4-(3-(dimethylamino)azetidin-1-yl)-6-fluoro-1*H*-[1,2,3]triazolo[4,5-*c*]quinolin-1-yl)piperidin-2-yl)acetonitrile;
2-((2S,4S)-4-(8-chloro-6-fluoro-4-(2-methyl-1H-imidazol-1-yl)-7-phenyl-1H-imidazo[4,5-c]quinolin-1-yl)-1-((E)-4-(dimethylamino)but-2-enoyl)piperidin-2-yl)acetonitrile;
2-((2S,4S)-4-(8-chloro-6-fluoro-4-(2-methyl-1H-imidazol-1-yl)-7-phenyl-1H-imidazo[4,5-c]quinolin-1-yl)-1-((E)-4-fluorobut-2-enoyl)piperidin-2-yl)acetonitrile;
2-((2S,4S)-4-(8-chloro-6-fluoro-4-(2-methyl-1H-imidazol-1-yl)-7-phenyl-1H-[1,2,3]triazolo[4,5-c]quinolin-1-yl)-1-((E)-4-(dimethylamino)but-2-enoyl)piperidin-2-yl)acetonitrile;
2-((2S,4S)-1-acryloyl-4-(8-chloro-4-(3-(dimethylamino)azetidin-1-yl)-6-fluoro-7-phenyl-1H-[1,2,3]triazolo[4,5-c]quinolin-1-yl)piperidin-2-yl)acetonitrile;
2-((2S,4S)-1-acryloyl-4-(8-chloro-6-fluoro-4-(((S)-1-methylpyrrolidin-2-yl)methoxy)-7-phenyl-1H-[1,2,3]triazolo[4,5-c]quinolin-1-yl)piperidin-2-yl)acetonitrile;
2-((2S,4S)-1-acryloyl-4-(6-fluoro-8-methyl-4-(2-methyl-1H-imidazol-1-yl)-7-phenyl-1H-[1,2,3]triazolo[4,5-c]quinolin-1-yl)piperidin-2-yl)acetonitrile;
2-((2S,4S)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)-1-((E)-4-methoxybut-2-enoyl)piperidin-2-yl)acetonitrile;
2-((2S,4S)-1-((E)-4-(dimethylamino)but-2-enoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile;
2-((2S,4S)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)-1-((E)-4-fluorobut-2-enoyl)piperidin-2-yl)acetonitrile;
2-((2S,4S)-1-acryloyl-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile;
2-((2S,4S)-1-((E)-4,4-difluorobut-2-enoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile;
2-((2S,4S)-1-(but-2-ynoyl)-4-(6-fluoro-8-methyl-4-(((S)-1-methylpyrrolidin-2-yl)methoxy)-7-(2-(trifluoromethyl)phenyl)-1H-[1,2,3]triazolo[4,5-c]quinolin-1-yl)piperidin-2-yl)acetonitrile;
2-((2S,4S)-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)-1-((E)-4-(dimethylamino)but-2-enoyl)piperidin-2-yl)acetonitrile;
2-((2S,4S)-1-((E)-4,4-difluorobut-2-enoyl)-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)piperidin-2-yl)acetonitrile;
2-((2S,4S)-1-acryloyl-4-(4-(3-(dimethylamino)azetidin-1-yl)-6-fluoro-8-methyl-7-(2-(trifluoromethyl)phenyl)-1H-[1,2,3]triazolo[4,5-c]quinolin-1-yl)piperidin-2-yl)acetonitrile;
2-((2S,4S)-1-(but-2-ynoyl)-4-(4-(3-(dimethylamino)a-zetidin-1-yl)-6-fluoro-8-methyl-7-(2-(trifluoromethyl)phenyl)-1H-[1,2,3]triazolo[4,5-c]quinolin-1-yl)piperidin-2-yl)acetonitrile;
2-((2S,4S)-1-((E)-4-(dimethylamino)but-2-enoyl)-4-(6-fluoro-8-methyl-4-(2-methyl-1H-imidazol-1-yl)-7-(2-(trifluoromethyl)phenyl)-1H-[1,2,3]triazolo[4,5-c]quinolin-1-yl)piperidin-2-yl)acetonitrile;
2-((2S,4S)-1-((E)-4,4-difluorobut-2-enoyl)-4-(6-fluoro-8-methyl-4-(((S)-1-methylpyrrolidin-2-yl)methoxy)-7-(2-(trifluoromethyl)pyridin-3-yl)-1H-[1,2,3]triazolo[4,5-c]quinolin-1-yl)piperidin-2-yl)acetonitrile;
2-((2S,4S)-1-(but-2-ynoyl)-4-(6-fluoro-8-methyl-4-(((S)-1-methylpyrrolidin-2-yl)methoxy)-7-(2-(trifluoromethyl)pyridin-3-yl)-1H-[1,2,3]triazolo[4,5-c]quinolin-1-yl)piperidin-2-yl)acetonitrile;
2-((2S,4S)-4-(4-(3-(dimethylamino)azetidin-1-yl)-7-(2,3-dimethylphenyl)-6-fluoro-8-methyl-1H-[1,2,3]triazolo[4,5-c]quinolin-1-yl)-1-((E)-4-fluorobut-2-enoyl)piperidin-2-yl)acetonitrile;
2-((2S,4S)-1-(but-2-ynoyl)-4-(4-(3-(dimethylamino)azetidin-1-yl)-7-(2,3-dimethylphenyl)-6-fluoro-8-methyl-1H-[1,2,3]triazolo[4,5-c]quinolin-1-yl)piperidin-2-yl)acetonitrile;
2-((2S,4S)-1-acryloyl-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile;
2-((2S,4S)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)-1-((E)-4-fluorobut-2-enoyl)piperidin-2-yl)acetonitrile;
2-((2S,4S)-1-(but-2-ynoyl)-4-(4-(3-(dimethylamino)-3-methylazetidin-1-yl)-7-(2,3-dimethylphenyl)-6-fluoro-8-methyl-1H-[1,2,3]triazolo[4,5-c]quinolin-1-yl)piperidin-2-yl)acetonitrile;
1-((2*S*,4*S*)-1-acryloyl-2-(cyanomethyl)piperidin-4-yl)-7 -(6-chloro-5-methyl-1*H*-indazol-4-yl)-4-(3-(dimethylamino)azetidin-1-yl)-6-fluoro-1*H*-[1,2,3]triazolo[4,5-*c*]quinoline-8-carbonitrile;
1-((2*S*,4*S*)-1-(but-2-ynoyl)-2-(cyanomethyl)piperidin-4-yl)-7-(6-chloro-5-methyl-1*H-*indazol-4-yl)-4-(3-(dimethylamino)azetidin-1-yl)-6-fluoro-1*H*-[1,2,3]triazolo[4,5-*c*]quinoline-8-carbonitrile;
2-((2S,4S)-1-acryloyl-4-(8-chloro-7-(3-chloro-2-methylphenyl)-4-(3-(dimethylamino)azetidin-1-yl)-1H-imidazo[4,5-c]quinolin-1-yl)piperidin-2-yl)acetonitrile;
2-((2S,4S)-1-acryloyl-4-(7-(3-chloro-2-methylphenyl)-4-(3-(dimethylamino)azetidin-1-yl)-8-methyl-1H-[1,2,3]triazolo[4,5-c]quinolin-1-yl)piperidin-2-yl)acetonitrile;
2-((2S,4S)-1-acryloyl-4-(8-chloro-7-(3-chloro-2-methylphenyl)-6-fluoro-4-(2-methylpyridin-3-yl)-1H-imidazo[4,5-c]quinolin-1-yl)piperidin-2-yl)acetonitrile;
2-((2S,4S)-1-acryloyl-4-(8-chloro-7-(3-chloro-2-methylphenyl)-6-fluoro-4-(2-methylpyridin-3-yl)-1H-[1,2,3]triazolo[4,5-c]quinolin-1-yl)piperidin-2-yl)acetonitrile;
2-((2S,4S)-4-(8-chloro-4-(3-(dimethylamino)azetidin-1-yl)-6-fluoro-7-(isoquinolin-4-yl)-1H-imidazo[4,5-c]quinolin-1-yl)-1-((E)-4-(dimethylamino)but-2-enoyl)piperidin-2-yl)acetonitrile;
2-((2S,4S)-4-(8-chloro-7-(6-chloro-5-methyl-1 H-indazol-4-yl)-4-(3-(dimethylamino)-azetidin-1-yl)-6-fluoro-1H-imidazo[4,5-c]quinolin-1-yl)-1-((E)-4-(dimethylamino)but-2-enoyl)piperidin-2-yl)acetonitrile;
2-((2S,4S)-1-(but-2-ynoyl)-4-(8-chloro-7-(6-chloro-5-methyl-1H-indazol-4-yl)-4-(3-(dimethylamino)azetidin-1-yl)-6-fluoro-1H-imidazo[4,5-c]quinolin-1-yl)piperidin-2-yl)acetonitrile;
2-((2S,4S)-1-(but-2-ynoyl)-4-(8-chloro-7-(6-chloro-5-methyl-1H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile;
2-((2S,4S)-4-(8-chloro-7-(6-chloro-5-methyl-1 H-indazol-4-yl)-4-(3-(dimethylamino)-azetidin-1-yl)-6-fluoro-1H-[1,2,3]triazolo[4,5-c]quinolin-1-yl)-1-((E)-4-(dimethylamino)but-2-enoyl)piperidin-2-yl)acetonitrile;
2-((2S,4S)-1-(but-2-ynoyl)-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)piperidin-2-yl)acetonitrile;
2-((2S,4S)-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)-1-((E)-4-(dimethylamino)but-2-enoyl)piperidin-2-yl)acetonitrile;
2-((2S,4S)-1-(but-2-ynoyl)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)-1-((E)-4-(dimethylamino)but-2-enoyl)piperidin-2-yl)acetonitrile;
2-((2S,4S)-4-(8-chloro-7-(6-chloro-5-methyl-1H-indazol-4-yl)-6-fluoro-4-(((S)-1-methylpyrrolidin-2-yl)methoxy)-1H-imidazo[4,5-c]quinolin-1-yl)-1-((E)-4-methoxybut-2-enoyl)piperidin-2-yl)acetonitrile;
2-((2S,4S)-1-acryloyl-4-(8-chloro-7-(6-chloro-5-methyl-1 H-indazol-4-yl)-4-(3-(dimethylamino)azetidin-1-yl)-6-fluoro-1H-[1,2,3]triazolo[4,5-c]quinolin-1-yl)piperidin-2-yl)acetonitrile;
2-((2S,4S)-4-(8-chloro-7-(6-chloro-5-methyl-1 H-indazol-4-yl)-4-(3-(dimethylamino)azetidin-1-yl)-6-fluoro-1H-[1,2,3]triazolo[4,5-c]quinolin-1-yl)-1-((E)-4-methoxybut-2-enoyl)piperidin-2-yl)acetonitrile;
2-((2S,4S)-1-acryloyl-4-(8-chloro-6-fluoro-7-(4-fluoro-3-methylphenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-(but-2-ynoyl)-4-(8-chloro-7-(6-chloro-5-methyl-1H-indazol-4-yl)-6-fluoro-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-4-(3-(dimethylamino)-azetidin-1-yl)-6-fluoro-1H-[1,2,3]triazolo[4,5-c]quinolin-1-yl)-1-(2-fluoroacryloyl)piperidin-2-yl)acetonitrile;
2-((2S,4S)-1-acryloyl-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)-1-((E)-4-methoxybut-2-enoyl)piperidin-2-yl)acetonitrile;
2-((2S,4S)-4-(8-chloro-7-(6-chloro-5-methyl-1H-indazol-4-yl)-6-fluoro-4-(((S)-1-methylpyrrolidin-2-yl)methoxy)-1H-[1,2,3]triazolo[4,5-c]quinolin-1-yl)-1-(2-fluoroacryloyl)piperidin-2-yl)acetonitrile;
2-((2S,4S)-1-acryloyl-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-1-(but-2-ynoyl)-4-(7-(6-chloro-5-methyl-1 H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((25,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4-methoxybut-2-enoyl)piperidin-2-yl)acetonitrile;
2-((2S,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile;
\2-((2S,4S)-4-(7-(6-chloro-5-methyl-1H-indazol-4-yl)-4-(3-(dimethylamino)-3-methylazetidin-1-yl)-6-fluoro-8-methyl-1H-[1,2,3]triazolo[4,5-c]quinolin-1-yl)-1-((E)-4,4-difluorobut-2-enoyl)piperidin-2-yl)acetonitrile;
2-((2S,4S)-1-acryloyl-4-(7-(5,6-dimethyl-1H-indazol-4-yl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-1-acryloyl-4-(6-fluoro-7-(4-fluoro-3-methylphenyl)-8-methyl-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(4-(3-(dimethylamino)-3-methylazetidin-1-yl)-6-fluoro-8-methyl-7-(5-methyl-1H-indazol-4-yl)-1H-[1,2,3]triazolo[4,5-c]quinolin-1-yl)piperidin-2-yl)acetonitrile;
2-((2S,4S)-4-(8-chloro-7-(6-chloro-5-methyl-1H-indazol-4-yl)-4-(3-(dimethylamino)-azetidin-1-yl)-6-fluoro-1H-[1,2,3]triazolo[4,5-c]quinolin-1-yl)-1-((E)-4,4-difluorobut-2-enoyl)piperidin-2-yl)acetonitrile;
2-((2S,4S)-1-acryloyl-4-(4-(3-(dimethylamino)-3-methylazetidin-1-yl)-6-fluoro-7-(6-fluoro-5-methyl-1H-indazol-4-yl)-8-methyl-1H-[1,2,3]triazolo[4,5-c]quinolin-1-yl)piperidin-2-yl)acetonitrile;
2-((2S,4S)-4-(4-(3-(dimethylamino)-3-methylazetidin-1-yl)-6-fluoro-7-(6-fluoro-5-methyl-1H-indazol-4-yl)-8-methyl-1H-[1,2,3]triazolo[4,5-c]quinolin-1-yl)-1-((E)-4-methoxybut-2-enoyl)piperidin-2-yl)acetonitrile;
2-((2*S*)-1-acryloyl-4-(8-chloro-6-fluoro-4-(((*S*)-1-methylpyrrolidin-2-yl)methoxy)-7-(*m-*tolyl)-1*H*-[1,2,3]triazolo[4,5-*c*]quinolin-1-yl)piperidin-2-yl)acetonitrile;
2-((2*S*)-1-acryloyl-4-(8-chloro-4-(3-(dimethylamino)-3-methylazetidin-1-yl)-6-fluoro-7-(*m*-tolyl)-1*H*-[1,2,3]triazolo[4,5-c]quinolin-1-yl)piperidin-2-yl)acetonitrile;
2-((2*S*)-1-acryloyl-4-(8-chloro-7-(8-chloronaphthalen-1-yl)-4-(3-(dimethylamino)-3-methylazetidin-1-yl)-6-fluoro-1*H*-imidazo[4,5-*c*]quinolin-1-yl)piperidin-2-yl)acetonitrile;
2-((2*S*)-1-(but-2-ynoyl)-4-(8-chloro-7-(8-chloronaphthalen-1-yl)-4-(3-(dimethylamino)-3-methylazetidin-1-yl)-6-fluoro-1*H*-imidazo[4,5-*c*]quinolin-1-yl)piperidin-2-yl)acetonitrile;
2-((2*S*)-1-acryloyl-4-(8-chloro-7-(8-chloronaphthalen-1-yl)-4-(3-(ethyl(methyl)amino)azetidin-1-yl)-6-fluoro-1*H*-imidazo[4,5-*c*]quinolin-1-yl)piperidin-2-yl)acetonitrile;
2-((2*S*)-1-acryloyl-4-(8-chloro-7-(8-chloronaphthalen-1-yl)-6-fluoro-4-(((*S*)-1-methylpyrrolidin-2-yl)methoxy)-1*H*-imidazo[4,5-*c*]quinolin-1-yl)piperidin-2-yl)acetonitrile;
2-((2*S*)-1-acryloyl-4-(8-chloro-7-(8-chloronaphthalen-1-yl)-4-(3-(dimethylamino)-3-methylazetidin-1-yl)-6-fluoro-1*H*-[1,2,3]triazolo[4,5-*c*]quinolin-1-yl)piperidin-2-yl)acetonitrile;
2-((2*S*)-4-(8-chloro-7-(8-chloronaphthalen-1-yl)-4-(3-(dimethylamino)-3-methylazetidin-1-yl)-6-fluoro-1*H*-[1,2,3]triazolo[4,5-*c*]quinolin-1-yl)-1-((*E*)-4-(dimethylamino)but-2-enoyl)piperidin-2-yl)acetonitrile;
2-((2*S*)-1-(but-2-ynoyl)-4-(8-chloro-7-(8-chloronaphthalen-1-yl)-4-(3-(dimethylamino)-3-methylazetidin-1-yl)-6-fluoro-1*H*-[1,2,3]triazolo[4,5-*c*]quinolin-1-yl)piperidin-2-yl)acetonitrile;
2-((2*S*)-1-acryloyl-4-(8-chloro-7-(8-chloronaphthalen-1-yl)-4-(3-(ethyl(methyl)amino)-azetidin-1-yl)-6-fluoro-1*H*-[1,2,3]triazolo[4,5-*c*]quinolin-1-yl)piperidin-2-yl)acetonitrile;
8-(1-((2*S*)-1-acryloyl-2-(cyanomethyl)piperidin-4-yl)-8-chloro-4-(3-(dimethylamino)-3-methylazetidin-1-yl)-6-fluoro-1*H*-[1,2,3]triazolo[4,5-*c*]quinolin-7-yl)-1-naphthonitrile;
2-((2*S*)-1-acryloyl-4-(7-(8-chloronaphthalen-1-yl)-4-(3-(dimethylamino)-3-methylazetidin-1-yl)-6-fluoro-8-methyl-1*H*-imidazo[4,5-*c*]quinolin-1-yl)piperidin-2-yl)acetonitrile;
2-((2*S*)-1-(but-2-ynoyl)-4-(7-(8-chloronaphthalen-1-yl)-4-(3-(dimethylamino)-3-methylazetidin-1-yl)-6-fluoro-8-methyl-1*H*-imidazo[4,5-*c*]quinolin-1-yl)piperidin-2-yl)acetonitrile;
2-((2*S*)-1-acryloyl-4-(7-(8-chloronaphthalen-1-yl)-4-(3-(ethyl(methyl)amino)azetidin-1-yl)-6-fluoro-8-methyl-1*H*-imidazo[4,5-*c*]quinolin-1-yl)piperidin-2-yl)acetonitrile;
2-((2*S*)-1-(but-2-ynoyl)-4-(7-(8-chloronaphthalen-1-yl)-4-(3-(ethyl(methyl)amino)azetidin-1-yl)-6-fluoro-8-methyl-1*H*-imidazo[4,5-*c*]quinolin-1-yl)piperidin-2-yl)acetonitrile;
2-((2*S*)-1-acryloyl-4-(7-(8-chloronaphthalen-1-yl)-4-(3-(dimethylamino)-3-methylazetidin-1-yl)-6-fluoro-8-methyl-1*H*-[1,2,3]triazolo[4,5-*c*]quinolin-1-yl)piperidin-2-yl)acetonitrile;
2-((2*S*,4*S*)-1-acryloyl-4-(4-(3-(dimethylamino)azetidin-1-yl)-6-fluoro-7-(5-fluoroquinolin-8-yl)-8-(trifluoromethyl)-1*H*-imidazo[4,5-*c*]quinolin-1-yl)piperidin-2-yl)acetonitrile;
2-((2*S*,4*S*)-4-(4-(3-(dimethylamino)azetidin-1-yl)-6-fluoro-7-(5-fluoroquinolin-8-yl)-8-(trifluoromethyl)-1*H*-imidazo[4,5-*c*]quinolin-1-yl)-1-((*E*)-4-(dimethylamino)but-2-enoyl)piperidin-2-yl)acetonitrile;
3-(1-((2*S*,4*S*)-1-(but-2-ynoyl)-2-(cyanomethyl)-piperidin-4-yl)-7-(6-chloro-5-methyl-1*H*-indazol-4-yl)-4-(3-(dimethylamino)azetidin-1-yl)-6-fluoro-1*H*-[1,2,3]triazolo[4,5-c]quinolin-8-yl)propanenitrile;
3-(7-(6-chloro-5-methyl-1H-indazol-4-yl)-1-((2S,4S)-2-(cyanomethyl)-1-((E)-4-(dimethylamino)but-2-enoyl)piperidin-4-yl)-4-(3-(dimethylamino)azetidin-1-yl)-6-fluoro-1H-[1,2,3]triazolo[4,5-c]quinolin-8-yl)propanenitrile;
2-((2S,4S)-1-acryloyl-4-(4-(3-(dimethylamino)-3-methylazetidin-1-yl)-6-fluoro-7-(5-fluoroquinolin-8-yl)-8-(trifluoromethyl)-1H-[1,2,3]triazolo[4,5-c]quinolin-1-yl)piperidin-2-yl)acetonitrile;
2-((2S,4S)-1-(but-2-ynoyl)-4-(4-(3-(dimethylamino)-3-methylazetidin-1-yl)-6-fluoro-7-(5-fluoroquinolin-8-yl)-8-(trifluoromethyl)-1H-[1,2,3]triazolo[4,5-c]quinolin-1-yl)piperidin-2-yl)acetonitrile;
2-((2S,4S)-1-acryloyl-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-1-(but-2-ynoyl)-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-4-(7-(2-chloro-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile;
2-((2S,4S)-1-acryloyl-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-1-(but-2-ynoyl)-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-4-(7-(3-chloro-2-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile;
3-(1-((2S,4S)-1-(but-2-ynoyl)-2-(cyanomethyl)piperidin-4-yl)-7-(3-chloro-2-methylphenyl)-6-fluoro-4-(((S)-1-methylpyrrolidin-2-yl)methoxy)-1H-[1,2,3]triazolo[4,5-c]quinolin-8-yl)propanenitrile;
3-(1-((2S,4S)-1-(but-2-ynoyl)-2-(cyanomethyl)piperidin-4-yl)-7-(2-chloro-3-methylphenyl)-6-fluoro-4-(((S)-1-methylpyrrolidin-2-yl)methoxy)-1H-[1,2,3]triazolo[4,5-c]quinolin-8-yl)propanenitrile;
2-((2S,4S)-1-(but-2-ynoyl)-4-(7-(3-chloro-2-methoxyphenyl)-4-(3-(dimethylamino)-3-methylazetidin-1-yl)-6-fluoro-8-(trifluoromethyl)-1H-[1,2,3]triazolo[4,5-c]quinolin-1-yl)piperidin-2-yl)acetonitrile;
2-((2S,4S)-4-(7-(3-chloro-2-methoxyphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile;
2-((2S,4S)-1-(but-2-ynoyl)-4-(4-(3-(dimethylamino)-3-methylazetidin-1-yl)-6-fluoro-7-(2-methoxy-3-methylphenyl)-8-(trifluoromethyl)-1H-[1,2,3]triazolo[4,5-c]quinolin-1-yl)piperidin-2-yl)acetonitrile;
2-((2S,4S)-4-(7-(2-methoxy-3-methylphenyl)-4-(3-(dimethylamino)-3-methylazetidin-1-yl)-6-fluoro-8-(trifluoromethyl)-1H-[1,2,3]triazolo[4,5-c]quinolin-1-yl)-1-((E)-4-(dimethylamino)but-2-enoyl)piperidin-2-yl)acetonitrile;
3-(1-((2S,4S)-1-(but-2-ynoyl)-2-(cyanomethyl)piperidin-4-yl)-7-(3-chloro-2-methoxyphenyl)-6-fluoro-4-(((S)-1-methylpyrrolidin-2-yl)methoxy)-1H-[1,2,3]triazolo[4,5-c]quinolin-8-yl)propanenitrile;
2-((2S,4S)-1-acryloyl-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile;
2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-1-((E)-4-fluorobut-2-enoyl)piperidin-2-yl)acetonitrile;
2-((2S,4S)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile;
2-((2S,4S)-1-(but-2-ynoyl)-4-(7-(2-chloro-3-methylphenyl)-4-(3-(dimethylamino)azetidin-1-yl)-6-fluoro-8-methyl-1H-[1,2,3]triazolo[4,5-c]quinolin-1-yl)piperidin-2-yl)acetonitrile;
2-((2S,4S)-4-(7-(2-chloro-3-methylphenyl)-6-fluoro-8-methyl-4-(2-methyl-1H-imidazol-1-yl)-1H-[1,2,3]triazolo[4,5-c]quinolin-1-yl)-1-((E)-4-(dimethylamino)but-2-enoyl)piperidin-2-yl)acetonitrile;
2-((2S,4S)-1-acryloyl-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)-1-((E)-4-fluorobut-2-enoyl)piperidin-2-yl)acetonitrile;
2-((2S,4S)-1-(but-2-ynoyl)-4-(7-(2-chloro-3-methylphenyl)-6-fluoro-8-methyl-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-fluorobut-2-enoyl)piperidin-2-yl)acetonitrile;
2-((2S,4S)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)-1-((E)-4-(dimethylamino)but-2-enoyl)piperidin-2-yl)acetonitrile;
2-((2S,4S)-1-(but-2-ynoyl)-4-(7-(2-chloro-3-methylphenyl)-4-(3-(dimethylamino)-3-methylazetidin-1-yl)-6-fluoro-8-methyl-1H-[1,2,3]triazolo[4,5-c]quinolin-1-yl)piperidin-2-yl)acetonitrile;
2-((2S,4S)-1-acryloyl-4-(7-(2-chloro-3-methylphenyl)-6-fluoro-8-methyl-4-(3-oxomorpholino)-1H-[1,2,3]triazolo[4,5-c]quinolin-1-yl)piperidin-2-yl)acetonitrile;
2-((2S,4S)-1-(but-2-ynoyl)-4-(7-(2-chloro-3-methylphenyl)-6-fluoro-8-methyl-4-(3-oxomorpholino)-1H-[1,2,3]triazolo[4,5-c]quinolin-1-yl)piperidin-2-yl)acetonitrile;
2-((2S,4S)-4-(7-(2-chloro-3-methylphenyl)-6-fluoro-8-methyl-4-(3-oxomorpholino)-1H-[1,2,3]triazolo[4,5-c]quinolin-1-yl)-1-((E)-4-fluorobut-2-enoyl)piperidin-2-yl)acetonitrile;
2-((2S,4S)-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)-1-((E)-4-(dimethylamino)but-2-enoyl)piperidin-2-yl)acetonitrile;
2-((2S,4S)-1-(but-2-ynoyl)-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)piperidin-2-yl)acetonitrile;
2-((2S,4S)-1-acryloyl-4-(8-chloro-4-(3-(dimethylamino)azetidin-1-yl)-6-fluoro-7-(3-methyl-2-(trifluoromethyl)phenyl)-1H-imidazo[4,5-c]quinolin-1-yl)piperidin-2-yl)acetonitrile;
2-((2S,4S)-1-acryloyl-4-(8-chloro-7-(8-chloronaphthalen-1-yl)-4-(3-(dimethylamino)azetidin-1-yl)-6-fluoro-1H-imidazo[4,5-c]quinolin-1-yl)piperidin-2-yl)acetonitrile;
2-((2S,4S)-1-acryloyl-4-(8-chloro-4-(3-(dimethylamino)azetidin-1-yl)-6-fluoro-7-(6-fluoro-5-methyl-1H-indazol-4-yl)-1H-imidazo[4,5-c]quinolin-1-yl)piperidin-2-yl)acetonitrile;
2-((2S,4S)-1-acryloyl-4-(8-chloro-7-(6-chloro-5-methyl-1 H-indazol-4-yl)-4-(3-(dimethylamino)azetidin-1-yl)-6-fluoro-1H-imidazo[4,5-c]quinolin-1-yl)piperidin-2-yl)acetonitrile;
2-((2S,4S)-1-acryloyl-4-(8-chloro-4-(3-(dimethylamino)azetidin-1-yl)-6-fluoro-7-(1-methylisoquinolin-4-yl)-1H-imidazo[4,5-c]quinolin-1-yl)piperidin-2-yl)acetonitrile;
2-((2S,4S)-1-acryloyl-4-(8-chloro-4-(3-(dimethylamino)azetidin-1-yl)-6-fluoro-7-(3-methylisoquinolin-4-yl)-1H-imidazo[4,5-c]quinolin-1-yl)piperidin-2-yl)acetonitrile;
2-((2S,4S)-1-(but-2-ynoyl)-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidin-2-yl)acetonitrile;
2-((2S,4S)-1-acryloyl-4-(6,8-dichloro-7-(3-chloro-2-methylphenyl)-4-(3-(dimethylamino)azetidin-1-yl)-1H-[1,2,3]triazolo[4,5-c]quinolin-1-yl)piperidin-2-yl)acetonitrile;
2-((2S,4S)-1-(but-2-ynoyl)-4-(6,8-dichloro-4-(3-(dimethylamino)azetidin-1-yl)-7-(5-fluoroquinolin-8-yl)-1H-[1,2,3]triazolo[4,5-c]quinolin-1-yl)piperidin-2-yl)acetonitrile;
2-((2S,4S)-1-acryloyl-4-(6,8-dichloro-7-(5-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-(but-2-ynoyl)-4-(6,8-dichloro-4-(3-(dimethylamino)azetidin-1-yl)-7-(quinolin-8-yl)-1H-[1,2,3]triazolo[4,5-c]quinolin-1-yl)piperidin-2-yl)acetonitrile;
2-((2S,4S)-1-(but-2-ynoyl)-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-(but-2-ynoyl)-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidin-2-yl)acetonitrile;
2-((2S,4S)-1-(but-2-ynoyl)-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-7-(2,3-dichlorophenyl)-4-(3-(dimethylamino)azetidin-1-yl)-1H-[1,2,3]triazolo[4,5-c]quinolin-1-yl)piperidin-2-yl)acetonitrile;
2-((2S,4S)-1-acryloyl-4-(6,8-dichloro-7-(2-chloro-3-methylphenyl)-4-(3-(dimethylamino)azetidin-1-yl)-1H-[1,2,3]triazolo[4,5-c]quinolin-1-yl)piperidin-2-yl)acetonitrile;
2-((2S,4S)-1-acryloyl-4-(6,8-dichloro-4-(3-(dimethylamino)azetidin-1-yl)-7-(quinolin-8-yl)-1H-[1,2,3]triazolo[4,5-c]quinolin-1-yl)piperidin-2-yl)acetonitrile;
2-((2S,4S)-1-acryloyl-4-(6,8-dichloro-4-(3-(dimethylamino)azetidin-1-yl)-7-(6-fluoroquinolin-8-yl)-1H-[1,2,3]triazolo[4,5-c]quinolin-1-yl)piperidin-2-yl)acetonitrile;
2-((2S,4S)-1-acryloyl-4-(6,8-dichloro-7-(2,3-dichlorophenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-7-(2-chloro-3-methylphenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-7-(3-chloro-2-methylphenyl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-4-(((S)-1-methylpyrrolidin-2-yl)methoxy)-7-(quinolin-8-yl)-1H-[1,2,3]triazolo[4,5-c]quinolin-1-yl)piperidin-2-yl)acetonitrile;
2-((2S,4S)-1-acryloyl-4-(6,8-dichloro-7-(6-fluoroquinolin-8-yl)-4-(((S)-1-methylpyrrolidin-2-yl)methoxy)-1H-[1,2,3]triazolo[4,5-c]quinolin-1-yl)piperidin-2-yl)acetonitrile;
2-((2S,4S)-1-acryloyl-4-(6,8-dichloro-7-(3-chloro-2-methoxyphenyl)-4-(3-(dimethylamino)azetidin-1-yl)-1H-[1,2,3]triazolo[4,5-c]quinolin-1-yl)piperidin-2-yl)acetonitrile;
3-(1-((*endo*)-2-azabicyclo[2.1.1]hexan-5-yl)-8-(2-cyanoethyl)-4-(3-(dimethylamino)azetidin-1-yl)-6-fluoro-7-(7-fluoronaphthalen-1-yl)-1H-imidazo[4,5-c]quinolin-2-yl)-N,N-dimethylpropanamide;
3-(1-((*endo*)-2-azabicyclo[2.1.1]hexan-5-yl)-4-(3-(dimethylamino)azetidin-1-yl)-2-ethyl-6-fluoro-7-(7-fluoro-3-hydroxynaphthalen-1-yl)-1H-imidazo[4,5-c]quinolin-8-yl)propanenitrile;
3-(1-((*endo*)-2-azabicyclo[2.1.1]hexan-5-yl)-8-(2-cyanoethyl)-4-(3-(dimethylamino)azetidin-1-yl)-7-(3-hydroxynaphthalen-1-yl)-6-phenoxy-1*H*-imidazo[4,5-c]quinolin-2-yl)-*N*,*N*-dimethylpropanamide;
3-(6-benzyl-1-((*endo*)-2-azabicyclo[2.1.1]hexan-5-yl)-8-(2-cyanoethyl)-4-(3-(dimethylamino)azetidin-1-yl)-7-(3-hydroxynaphthalen-1-yl)-1*H*-imidazo[4,5-*c*]quinolin-2-yl)-*N*,*N*-dimethylpropanamide;
4-(1-((*endo*)-2-azabicyclo[2.1.1]hexan-5-yl)-4-(3-(dimethylamino)azetidin-1-yl)-6-fluoro-2-(2-(methylamino)ethyl)-8-(1*H*-pyrazol-4-yl)-1H-imidazo[4,5-*c*]quinolin-7-yl)naphthalen-2-ol;
3-(1-((*endo*)-2-((1H-pyrrol-2-yl)methyl)-2-azabicyclo[2.1.1]hexan-5-yl)-4-(3-(dimethylamino)azetidin-1-yl)-2-ethyl-6-fluoro-7-(3-hydroxynaphthalen-1-yl)-1H-imidazo[4,5-c]quinolin-8-yl)propanenitrile;
5-(1-((*endo*)-2-azabicyclo[2.1.1]hexan-5-yl)-8-(2-cyanoethyl)-2-(3-(dimethylamino)-3-oxopropyl)-6-fluoro-7-(3-hydroxynaphthalen-1-yl)-1*H*-imidazo[4,5-*c*]quinolin-4-yl)-2-fluoro-*N-*methylbenzamide;
3-(1-((*endo*)-2-azabicyclo[2.1.1]hexan-5-yl)-8-(2-cyanoethyl)-6-fluoro-7-(3-hydroxynaphthalen-1-yl)-4-(1-methyl-6-oxo-1,6-dihydropyridin-3-yl)-1*H*-imidazo[4,5-c]quinolin-2-yl)-*N,N*-dimethylpropanamide;
3-(1-((*endo*)-2-azabicyclo[2.1.1]hexan-5-yl)-8-(2-cyanobenzyl)-4-(3-(dimethylamino)azetidin-1-yl)-6-fluoro-7-(3-hydroxynaphthalen-1-yl)-1*H*-imidazo[4,5-c]quinolin-2-yl)-*N*,*N-*dimethylpropanamide;
3-(1-((*endo*)-2-azabicyclo[2.1.1]hexan-5-yl)-4-(3-(dimethylamino)azetidin-1-yl)-6-fluoro-7-(3-hydroxynaphthalen-1-yl)-2-((3-oxomorpholino)methyl)-1H-imidazo[4,5-c]quinolin-8-yl)propanenitrile;
3-(1-((endo)-2-azabicyclo[2.1.1]hexan-5-yl)-8-(2-cyanoethyl)-4-(3-(dimethylamino)azetidin-1-yl)-6-fluoro-7-(3-hydroxynaphthalen-1-yl)-1H-imidazo[4,5-c]quinolin-2-yl)-N-methyl-N-(pyridin-2-ylmethyl)propanamide; and
3-(1-((*endo*)-2-azabicyclo[2.1.1]hexan-5-yl)-4-(3-(dimethylamino)azetidin-1-yl)-6-fluoro-7-(3-hydroxynaphthalen-1-yl)-2-(2-(piperazin-1-yl)thiazol-4-yl)-1*H*-imidazo[4,5-*c*]quinolin-8-yl)propanenitrile;
or a pharmaceutically acceptable salt thereof.
42. A pharmaceutical composition comprising a compound of any one of clauses 1-41, or a pharmaceutically acceptable salt thereof, and at least one pharmaceutically acceptable carrier or excipient.
43. A method of inhibiting KRAS activity, said method comprising contacting a compound of any one of clauses 1-41, or the composition of clause 42, with KRAS.
44. The method of clause 43, wherein the contacting comprises administering the compound to a patient.
45. A method of treating a disease or disorder associated with inhibition of KRAS interaction, said method comprising administering to a patient in need thereof a therapeutically effective amount of a compound of any one of clauses 1-41, or the composition of clause 42.
46. A method of treating a disease or disorder associated with inhibiting a KRAS protein harboring a G12C mutation, said method comprising administering to a patient in need thereof a therapeutically effective amount of a compound of any one of clauses 1-41, or the composition of clause 42.
47. A method for treating a cancer in a patient, said method comprising administering to the patient a therapeutically effective amount of the compound of any one of clauses 1-41, or the composition of clause 42.
48. The method of clause 47, wherein the cancer is selected from carcinomas, hematological cancers, sarcomas, and glioblastoma.
49. The method of clause 48, wherein the hematological cancer is selected from myeloproliferative neoplasms, myelodysplastic syndrome, chronic and juvenile myelomonocytic leukemia, acute myeloid leukemia, acute lymphocytic leukemia, and multiple myeloma.
50. The method of clause 48, wherein the carcinomas is selected from pancreatic, colorectal, lung, bladder, gastric, esophageal, breast, head and neck, cervical, skin, and thyroid.
51. The method of clause 45, wherein the disease or disorder is an immunological or inflammatory disorder.
52. The method of clause 51, wherein the immunological or inflammatory disorder is Ras-associated lymphoproliferative disorder and juvenile myelomonocytic leukemia caused by somatic mutations of KRAS.

## Claims

1. A compound of Formula I: 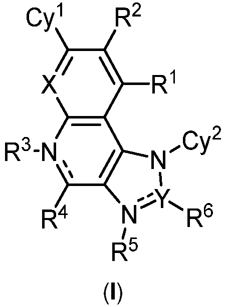 or a pharmaceutically acceptable salt thereof,
wherein:
each - - - independently represents a single bond or a double bond;
X is N or CR⁷;
Y is N or C;
R¹ is H;
R² is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a2}, SR^{a2}, C(O)R^{b2}, C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, C(=NR^{e2})R^{b2}, C(=NOR^{a2})R^{b2}, C(=NR^{e2})NR^{c2}R^{d2}, NR^{c2}C(=NR^{e2})NR^{c2}R^{d2}, NR^{c2}C(=NR^{e2})R^{b2}, NR^{c2}S(O)R^{b2}, NR^{c2}S(O)₂R^{b2}, NR^{c2}S(O)₂NR^{c2}R^{d2}, S(O)R^{b2}, S(O)NR^{c2}R^{d2}, S(O)₂R^{b2}, S(O)₂NR^{c2}R^{d2}, and BR^{h2}Rⁱ²; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²²;
Cy¹ is selected from C₃₋₁₀ cycloalkyl, 4-10 membered heterocycloalkyl, C₆₋₁₀ aryl and 5-10 membered heteroaryl; wherein the 4-10 membered heterocycloalkyl and 5-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 5-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 5-10 membered heteroaryl are each optionally substituted with 1, 2, 3 or 4 substituents independently selected from R¹⁰;
when R³N- - -CR⁴ is a single bond, then R⁴ is selected from =O and =S; and
R³ is selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰;
when - - - of R³N- - -CR⁴ represents a double bond:
R³ is absent; and
R⁴ is selected from H**,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, NR^{c3}C(O)NR^{c3}R^{d3}, C(=NR^{e3})R^{b3}, C(=NOR^{a3})R^{b3}, C(=NR^{e3})NR^{c3}R^{d3}, NR^{c3}C(=NR^{e3})NR^{c3}R^{d3}, NR^{c3}C(=NR^{e3})R^{b3}, NR^{c3}S(O)R^{b3}, NR^{c3}S(O)₂R^{b3}, NR^{c3}S(O)₂NR^{c3}R^{d3}, S(O)R^{b3}, S(O)NR^{c3}R^{d3}, S(O)₂R^{b3}, S(O)₂NR^{c3}R^{d3}, and BR^{h3}Rⁱ³; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰; or
when - - - of R⁵N- - -YR⁶ represents a single bond and Y is C;
YR⁶ is selected from C=O and C=S; and
R⁵ is selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, and 5-10 membered heteroaryl-C₁₋₃ alkylene; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁵⁰; or
when - - - of R⁵N- - -YR⁶ represents a double bond and Y is N;
then R⁵ and R⁶ are absent; or
when - - - of R⁵N- - -YR⁶ represents a double bond and Y is C;
then R⁵ is absent; and
R⁶ is selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃alkylene, halo, D, CN, NO₂, OR^{a6}, SR^{a6}, C(O)R^{b6}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, C(=NR^{e6})R^{b6}, C(=NOR^{a6})R^{b6}, C(=NR^{e6})NR^{c6}R^{d6}, NR^{c6}C(=NR^{e6})NR^{c6}R^{d6}, NR^{c6}C(=NR^{e6})R^{b6}, NR^{c6}S(O)R^{b6}, NR^{c6}S(O)₂R^{b6}, NR^{c6}S(O)₂NR^{c6}R^{d6}, S(O)R^{b6}, S(O)NR^{c6}R^{d6}, S(O)₂R^{b6}, S(O)₂NR^{c6}R^{d6}, and BR^{h6}Rⁱ⁶; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶⁰;
R⁷ is selected from H**,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a7}, SR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7}, C(=NR^{e7})R^{b7}, C(=NOR^{a7})R^{b7}, C(=NR^{e7})NR^{c7}R^{d7}, NR^{c7}C(=NR^{e7})NR^{c7}R^{d7}, NR^{c7}C(=NR^{e7})R^{b7}, NR^{c7}S(O)R^{b7}, NR^{c7}S(O)₂R^{b7}, NR^{c7}S(O)₂NR^{c7}R^{d7}, S(O)R^{b7}, S(O)NR^{c7}R^{d7}, S(O)₂R^{b7}, S(O)₂NR^{c7}R^{d7}, and BR^{h7}Rⁱ⁷; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷⁰;
Cy² is selected from
| | | | |
|---|---|---|---|
| 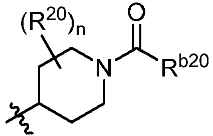 | 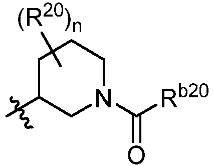 | 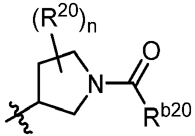 | 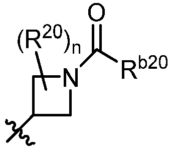 |
| Cy²-a1, | Cy²-b1, | Cy²-c1, and | Cy²-d1, |
n is 0, 1, or 2;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a10}, SR^{a10}, C(O)R^{b10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10}, NR^{c10}C(O)NR^{c10}R^{d10}, C(=NR^{e10})R^{b10}, C(=NOR^{a10})R^{b10}, C(=NR^{e10})NR^{c10}R^{d10}, NR^{c10}C(=NR^{e10})NR^{c10}R^{d10}, NR^{c10}S(O)R^{b10}, NR^{c10}S(O)₂R^{b10}, NR^{c10}S(O)₂NR^{c10}R^{d10}, S(O)R^{b10}, S(O)NR^{c10}R^{d10}, S(O)₂R^{b10}, S(O)₂NR^{c10}R^{d10}, and BR^{h10}Rⁱ¹⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R¹¹;
each R¹¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a11}, SR^{a11}, C(O)R^{b11}, C(O)NR^{c11}R^{d11}, C(O)OR^{a11}, OC(O)R^{b11}, OC(O)NR^{c11}R^{d11}, NR^{c11}R^{d11}, NR^{c11}C(O)R^{b11}, NR^{c11}C(O)OR^{a11}, NR^{c11}C(O)NR^{c11}R^{d11}, NR^{c11}S(O)R^{b11}, NR^{c11}S(O)₂R^{b11}, NR^{c11}S(O)₂NR^{c11}R^{d11}, S(O)R^{b11}, S(O)NR^{c11}R^{d11}, S(O)₂R^{b11}, R^{d11}, and BR^{h11}Rⁱ¹¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R¹²;
each R¹² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a12}, SR^{a12}, C(O)R^{b12}, C(O)NR^{c12}R^{d12}, C(O)OR^{a12}, OC(O)R^{b12}, OC(O)NR^{c12}R^{d12}, NR^{c12}R^{d12}, NR^{c12}C(O)R^{b12}, NR^{c12}C(O)OR^{a12} , NR^{c12}C(O)NR^{c12}R^{d12}, NR^{c12}S(O)R^{b12}, NR^{c12}S(O)₂R^{b12}, NR^{c12}S(O)₂NR^{c12}R^{d12}, S(O)R^{b12}, S(O)NR^{c12}R^{d12}, S(O)₂R^{b12}, S(O)₂NR^{c12}R^{d12}, and BR^{h12}Rⁱ¹²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R²⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a20}, SR^{a20}, C(O)R^{b20}, C(O)NR^{c20}R^{d20}, C(O)OR^{a20}, OC(O)R^{b20}, OC(O)NR^{c20}R^{d20}, NR^{c20}R^{d20}, NR^{c20}C(O)R^{b20}, NR^{c20}C(O)OR^{a20}, NR^{c20}C(O)NR^{c20}R^{d20}, C(=NR^{e20})R^{b20}, C(=NOR^{a20})R^{b20}, C(=NR^{e20})NR^{c20}R^{d20}, NR^{c20}C(=NR^{e20})NR^{c20}R^{d20}, NR^{c20}S(O)R^{b20}, NR^{c20}S(O)₂R^{b20}, NR^{c20}S(O)₂NR^{c20}R^{d20}, S(O)R^{b20}, S(O)NR^{c20}R^{d20}, S(O)₂R^{b20}, S(O)₂NR^{c20}R^{d20}, and BR^{h20}Rⁱ²⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²¹;
each R²¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a21}, SR^{a21}, C(O)R^{b21}, C(O)NR^{c21}R^{d21}, C(O)OR^{a21}, OC(O)R^{b21}, OC(O)NR^{c21}R^{d21}, NR^{c21}R^{d21}, NR^{c21}C(O)R^{b21}, NR^{cC21}C(O)OR^{a21}, NR^{c21}C(O)NR^{c21}R^{d21}, NR^{c21}S(O)R^{b21}, NR^{c21}S(O)₂R^{b21}, NR^{c21}S(O)₂NR^{c21}R^{d21}, S(O)R^{b21}, S(O)NR^{c21}R^{d21}, S(O)₂R^{b21}, S(O)₂NR^{c21}R^{d21}, and BR^{h21}Rⁱ²¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R^{g};
each R²² is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a22}, SR^{a22},
C(O)R^{b22}, C(O)NR^{c22}R^{d22}, C(O)OR^{a22}, OC(O)R^{b22}, OC(O)NR^{c22}R^{d22}, NR^{c22}R^{d22}, NR^{c22}C(O)R^{b22}, NR^{c22}C(_{O)}OR^{a22}, NR^{c22}C(O)NR^{c22}R^{d22}, NR^{c22}S(O)R^{b22}, NR^{c22}S(O)₂R^{b22}, NR^{c22}S(O)₂NR^{c22}R^{d22}, S(O)R^{b22}, S(O)NR^{c22}R^{d22}, S(O)₂R^{b22}, S(O)₂NR^{c22}R^{d22}, and BR^{h22}Rⁱ²²; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²³;
each R²³ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a23}, SR^{a23}, C(O)R^{b23}, C(O)NR^{c23}R^{d23}, C(O)OR^{a23}, OC(O)R^{b23}, OC(O)NR^{c23}R^{d23}, NR^{c23}R^{d23}, NR^{c23}C(O)R^{b23}, NR^{c23}C(O)OR^{a23}, NR^{c23}C(O)NR^{c23}R^{d23}, NR^{c23}S(O)R^{b23}, NR^{c23}S(O)₂R^{b23}, NR^{c23}S(O)₂NR^{c23}R^{d23}, S(O)R^{b23}, S(O)NR^{c23}R^{d23}, S(O)₂R^{b23}, S(O)₂NR^{c23}R^{d23}, and BR^{h23}Rⁱ²³; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R²⁴;
each R²⁴ is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a24}, SR^{a24}, C(O)R^{b24}, C(O)NR^{c24}R^{d24}, C(O)OR^{a24}, OC(O)R^{b24}, OC(O)NR^{c24}R^{d24}, NR^{c24}R^{d24}, NR^{c24}C(O)R^{b24}, NR^{c24}C(O)OR^{a24}, NR^{c24}C(O)NR^{c24}R^{d24}, NR^{c24}S(O)R^{b24}, NR^{c24}S(O)₂R^{b24}, NR^{c24}S(O)₂NR^{c24}R^{d24}, S(O)R^{b24}, S(O)NR^{c24}R^{d24}, S(O)₂R^{b24}, S(O)2NR^{c24}R^{d24}, and BR^{h24}Rⁱ²⁴; wherein said C₁₋₆ alkyl, C₂₋₆ alkenyl, C₂₋₆ alkynyl, C₃₋₆ cycloalkyl, phenyl, 5-6 membered heteroaryl and 4-7 membered heterocycloalkyl, are each optionally substituted with 1, 2, 3, or 4 substituents independently selected from R^{g};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a30}, SR^{a30}, C(O)R^{b30}, C(O)NR^{c30}R^{d30}, C(O)OR^{a30}, OC(O)R^{b30}, OC(O)NR^{c30}R^{d30}, NR^{c30}R^{d30}, NR^{c30}C(O)R^{b30}, NR^{c30}C(O)OR^{a30}, NR^{c30}C(O)NR^{c30}R^{d30}, NR^{c30}S(O)R^{b30}, NR^{c30}S(O)₂R^{b30}, NR^{c30}S(O)₂NR^{c30}R^{d30}, S(O)R^{b30}, S(O)NR^{c30}R^{d30}, S(O)₂R^{b30}, S(O)₂NR^{c30}R^{d30}, and BR^{h30}Rⁱ³⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹;
each R³¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a31}, SR^{a31}, C(O)R^{b31}, C(O)NR^{c31}R^{d31}, C(O)OR^{a31}, OC(O)R^{b31}, OC(O)NR^{c31}R^{d31}, NR^{c31}R^{d31}, NR^{c31}C(O)R^{b31}, NR^{c31}C(O)OR^{a31}, NR^{c31}C(O)NR^{c31}R^{d31}, NR^{c31}S(O)R^{b31}, NR^{c31}S(O)₂R^{b31}, NR^{c31}S(O)₂NR^{c31}R^{d31}, S(O)R^{b31}, S(O)NR^{c31}R^{d31}, S(O)₂R^{b31}, S(O)₂NR^{c31}R^{d31}, and BR^{h31}Rⁱ³¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³²;
each R³² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a32}, SR^{a32}, C(O)R^{b32}, C(O)NR^{c32}R^{d32}, C(O)OR^{a32}, OC(O)R^{b32}, OC(O)NR^{c32}R^{d32}, NR^{c32}R^{d32}, NR^{c32}C(O)R^{b32}, NR^{c32}C(O)OR^{a32}, NR^{c32}C(O)NR^{c32}R^{d32}, NR^{c32}S(O)R^{b32}, NR^{c32}S(O)₂R^{b32}, NR^{c32}S(O)₂NR^{c32}R^{d32}, S(O)R^{b32}, S(O)NR^{c32}R^{d32}, S(O)₂R^{b32}, S(O)₂NR^{c32}R^{d32}, and BR^{h32}Rⁱ³²; wherein said C₁₋₆ alkyl, C₂₋₆ alkenyl, C₂₋₆ alkynyl, C₃₋₆ cycloalkyl, phenyl, 5-6 membered heteroaryl and 4-7 membered heterocycloalkyl, are each optionally substituted with 1, 2, 3, or 4 substituents independently selected from R^{g};
each R⁵⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a50}, SR^{a50}, C(O)R^{b50}, C(O)NR^{c50}R^{d50}, C(O)OR^{a50}, OC(O)R^{b50}, OC(O)NR^{c50}R^{d50}, NR^{c50}R^{d50}, NR^{c50}C(O)R^{b50}, NR^{c50}C(O)OR^{a50}, NR^{c50}C(O)NR^{c50}R^{d50}, NR^{c50}S(O)R^{b50}, NR^{c50}S(O)₂R^{b50}, NR^{c50}S(O)₂NR^{c50}R^{d50}, S(O)R^{b50}, S(O)NR^{c50}R^{d50}, S(O)₂R^{b50}, S(O)₂NR^{c50}R^{d50}, and BR^{h50}Rⁱ⁵⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁵¹;
each R⁵¹ is independently selected from C₁₋₆ alkyl, C₂₋₆ alkenyl, C₂₋₆ alkynyl, C₁₋₆ haloalkyl, C₃₋₆ cycloalkyl, C₆₋₁₀ aryl, 5-6 membered heteroaryl, 4-7 membered heterocycloalkyl, halo, D, CN, OR^{a51}, SR^{a51}, C(O)R^{b51}, C(O)NR^{c51}R^{d51}, C(O)OR^{a51}, OC(O)R^{b51}, OC(O)NR^{c51}R^{d51}, NR^{c51}R^{d51}, NR^{c51}C(O)R^{b51}, NR^{c51}C(O)OR^{a51}, NR^{c51}C(O)NR^{c51}R^{d51}, NR^{c51}S(O)R^{b51}, NR^{c51}S(O)₂R^{b51}, NR^{c51}S(O)₂NR^{c51}R^{d51}, S(O)R^{b51}, S(O)NR^{c51}R^{d51}, S(O)₂R^{b51}, S(O)₂NR^{c51}R^{d51}, and BR^{h51}Rⁱ⁵¹; wherein said C₁₋₆ alkyl, C₂₋₆ alkenyl, C₂₋₆ alkynyl, C₃₋₆ cycloalkyl, C₆₋₁₀ aryl, 5-6 membered heteroaryl and 4-7 membered heterocycloalkyl, are each optionally substituted with 1, 2, 3, or 4 substituents independently selected from R⁵²;
each R⁵² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a52}, SR^{a52}, C(O)R^{b52}, C(O)NR^{c52}R^{d52}, C(O)OR^{a52}, OC(O)R^{b52}, OC(O)NR^{c52}R^{d52}, NR^{c52}R^{d52}, NR^{c52}C(O)R^{b52}, NR^{c52}C(O)OR^{a52}, NR^{c52}C(O)NR^{c52}R^{d52}, NR^{c52}S(O)R^{b52}, NR^{c52}S(O)₂R^{b52}, NR^{c52}S(O)₂NR^{c52}R^{d52}, S(O)R^{b52}, S(O)NR^{c52}R^{d52}, S(O)₂R^{b52}, S(O)₂NR^{c52}R^{d52}, and BR^{h52}Rⁱ⁵²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R⁶⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a60}, SR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, NR^{c60}C(O)NR^{c60}R^{d60}, NR^{c60}S(O)R^{b60}, NR^{c60}S(O)₂R^{b60}, NR^{c60}S(O)₂NR^{c60}R^{d60}, S(O)R^{b60}, S(O)NR^{c60}R^{d60}, S(O)₂R^{b60}, S(O)₂NR^{c60}R^{d60}, and BR^{h60}Rⁱ⁶⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶¹;
each R⁶¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a61}, SR^{a61}, C(O)R^{b61}, C(O)NR^{c61}R^{d61}, C(O)OR^{a61}, OC(O)R^{b61}, OC(O)NR^{c61}R^{d61}, NR^{c61}R^{d61}, NR^{c61}C(O)R^{b61}, NR^{c61}C(O)OR^{a61}, NR^{c61}C(O)NR^{c61}R^{d61}, NR^{c61}S(O)R^{b61}, NR^{c61}S(O)₂R^{b61}, NR^{c61}S(O)₂NR^{c61}R^{d61}, S(O)R^{b61}, S(O)NR^{c61}R^{d61}, S(O)₂R^{b61}, S(O)₂NR^{c61}R^{d61}, and BR^{h61}Rⁱ⁶¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶²;
each R⁶² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a62}, SR^{a62}, C(O)R^{b62}, C(O)NR^{c62}R^{d62}, C(O)OR^{a62}, OC(O)R^{b62}, OC(O)NR^{c62}R^{d62}, NR^{c62}R^{d62}, NR^{c62}C(O)R^{b62}, NR^{c62}C(O)OR^{a62}, NR^{c62}C(O)NR^{c62}R^{d62}, NR^{c62}S(O)R^{b62}, NR^{c62}S(O)R^{b62}, NR^{c62}S(O)₂NR^{c62}R^{d62}, S(O)R^{b62}, S(O)NR^{c62}R^{d62}, S(O)R^{b62}, S(O)₂NR^{c62}R^{d62}, and BR^{h62}Rⁱ⁶²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R⁷⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, NO₂, OR^{a70}, SR^{a70}, C(O)R^{b70}, C(O)NR^{c70}R^{d70}, C(O)OR^{a70}, OC(O)R^{b70}, OC(O)NR^{c70}R^{d70}, NR^{c70}R^{d70}, NR^{c70}C(O)R^{b70}, NR^{c70}C(O)OR^{a70}, NR^{c70}C(O)NR^{c70}R^{d70}, NR^{c70}S(O)R^{b70}, NR^{c70}S(O)₂R^{b70}, NR^{c70}S(O)₂NR^{c70}R^{d70}, S(O)R^{b70}, S(O)NR^{c70}R^{d70}, S(O)₂R^{b70}, S(O)₂NR^{c70}R^{d70}, and BR^{h70}Rⁱ⁷⁰; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁷¹;
each R⁷¹ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a71}, SR^{a71}, C(O)R^{b71}, C(O)NR^{c71}R^{d71}, C(O)OR^{a71}, OC(O)R^{b71}, OC(O)NR^{c71}R^{d71}, NR^{c71}R^{d71}, NR^{c71}C(O)R^{b71}, NR^{c71}C(O)OR^{a71}, NR^{c71}C(O)NR^{c71}R^{d71}, NR^{c71}S(O)R^{b71}, NR^{c71}S(O)₂R^{b71}, NR^{c71}S(O)₂NR^{c71}R^{d71}, S(O)R^{b71}, S(O)NR^{c71}R^{d71}, S(O)₂R^{b71}, S(O)₂NR^{c71}R^{d71}, and BR^{h71}Rⁱ⁷¹; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃alkylene are each optionally substituted with 1, 2, 3, or 4 substituents independently selected from R⁷²;
each R⁷² is independently selected from C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl, 4-7 membered heterocycloalkyl, halo, D, CN, OR^{a72}, SR^{a72}, C(O)R^{b72}, C(O)NR^{c72}R^{d72}, C(O)OR^{a72}, OC(O)R^{b72}, OC(O)NR^{c72}R^{d72}, NR^{c72}R^{d72}, NR^{c72}C(O)R^{c72}, NR^{c72}C(O)OR^{a72}, NR^{c72}C(O)NR^{c72}R^{d72}, NR^{c72}S(O)R^{b72}, NR^{c72}S(O)₂R^{b72}, NR^{c72}S(O)₂NR^{c72}R^{d72}, S(O)R^{b72}, S(O)NR^{c72}R^{d72}, S(O)₂R^{b72}, S(O)₂NR^{c72}R^{d72}, and BR^{h72}Rⁱ⁷²; wherein said C₁₋₆ alkyl, C₂₋₆ alkenyl, C₂₋₆ alkynyl, C₃₋₆ cycloalkyl, phenyl, 5-6 membered heteroaryl, and 4-7 membered heterocycloalkyl, are each optionally substituted with 1, 2, 3, or 4 substituents independently selected from R^{g};
each R^{a2}, R^{b2}, R^{c2} and R^{d2} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²²;
or any R^{c2} and R^{d2} attached to the same N atom, together with the N atom to which they are attached, form a 4-, 5-, 6- or 7-membered heterocycloalkyl group optionally substituted with 1, 2, 3, or 4 substituents independently selected from R²²;
each R^{e2} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h2} and Rⁱ² is independently selected from OH, C₁₋₆ alkoxy, and C₁₋₆ haloalkoxy; or any R^{h2} and Rⁱ² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{e3} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h3} and Rⁱ³ is independently selected from OH, C₁₋₆ alkoxy, and C₁₋₆ haloalkoxy; or any R^{h3} and Rⁱ³ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{j3} is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶⁰;
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R⁶⁰;
each R^{e6} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h6} and Rⁱ⁶ is independently selected from OH, C₁₋₆ alkoxy, and C₁₋₆ haloalkoxy; or any R^{h6} and Rⁱ⁶ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a7}, R^{b7}, R^{c7} and R^{d7} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷⁰;
or any R^{c7} and R^{d7} attached to the same N atom, together with the N atom to which they are attached, form a 4-, 5-, 6- or 7-membered heterocycloalkyl group optionally substituted with 1, 2, 3, or 4 substituents independently selected from R⁷⁰;
each R^{e7} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h7} and Rⁱ⁷ is independently selected from OH, C₁₋₆ alkoxy, and C₁₋₆ haloalkoxy; or any R^{h7} and Rⁱ⁷ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R¹¹;
or any R^{c10} and R^{d10} attached to the same N atom, together with the N atom to which they are attached, form a 4-, 5-, 6- or 7-membered heterocycloalkyl group optionally substituted with 1, 2, 3, or 4 substituents independently selected from R¹¹;
each R^{e10} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h10} and Rⁱ¹⁰ is independently selected from OH, C₁₋₆ alkoxy, and C₁₋₆ haloalkoxy; or any R^{h10} and Rⁱ¹⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a11}, R^{b11}, R^{c11} and R^{d11}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R¹²;
or any R^{c11} and R^{d11} attached to the same N atom, together with the N atom to which they are attached, form a 4-, 5-, 6- or 7-membered heterocycloalkyl group optionally substituted with 1, 2 or 3 substituents independently selected from R¹²;
each R^{h11} and Rⁱ¹¹ is independently selected from OH, C₁₋₆ alkoxy, and C₁₋₆ haloalkoxy; or any R^{h11} and Rⁱ¹¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a12}, R^{b12}, R^{c12} and R^{d12}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
each R^{h12} and Rⁱ¹² is independently selected from OH, C₁₋₆ alkoxy, and C₁₋₆ haloalkoxy; or any R^{h12} and Rⁱ¹² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a20}, R^{b20}, R^{c20} and R^{d20} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²¹;
or any R^{c20} and R^{d20} attached to the same N atom, together with the N atom to which they are attached, form a 4-, 5-, 6- or 7-membered heterocycloalkyl group optionally substituted with 1, 2, 3, or 4 substituents independently selected from R²¹;
each R^{e20} is independently selected from H, CN, C₁₋₆ alkyl, C₂₋₆ alkenyl, C₂₋₆ alkynyl, C₁₋₆ haloalkyl, C₁₋₆ alkylthio, C₁₋₆ alkylsulfonyl, C₁₋₆ alkylcarbonyl, C₁₋₆ alkylaminosulfonyl, carbamyl, C₁₋₆ alkylcarbamyl, di(C₁₋₆ alkyl)carbamyl, aminosulfonyl, C₁₋₆ alkylaminosulfonyl and di(C₁₋₆ alkyl)aminosulfonyl;
each R^{h20} and Rⁱ²⁰ is independently selected from OH, C₁₋₆ alkoxy, and C₁₋₆ haloalkoxy; or any R^{h20} and Rⁱ²⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a21}, R^{b21}, R^{c21} and R^{d21}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R^{g};
or any R^{c21} and R^{d21} attached to the same N atom, together with the N atom to which they are attached, form a 4-, 5-, 6- or 7-membered heterocycloalkyl group optionally substituted with 1, 2 or 3 substituents independently selected from R^{g};
each R^{h21} and Rⁱ²¹ is independently selected from OH, C₁₋₆ alkoxy, and C₁₋₆ haloalkoxy; or any R^{h21} and Rⁱ²¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a22}, R^{b22}, R^{c22} and R^{d22} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²³;
or any R^{c22} and R^{d22} attached to the same N atom, together with the N atom to which they are attached, form a 4-, 5-, 6- or 7-membered heterocycloalkyl group optionally substituted with 1, 2, 3, or 4 substituents independently selected from R²³;
each R^{h22} and Rⁱ²² is independently selected from OH, C₁₋₆ alkoxy, and C₁₋₆ haloalkoxy; or any R^{h22} and Rⁱ²² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a23}, R^{b23}, R^{c23} and R^{d23}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R²⁴;
or any R^{c23} and R^{d23} attached to the same N atom, together with the N atom to which they are attached, form a 4-, 5-, 6- or 7-membered heterocycloalkyl group optionally substituted with 1, 2 or 3 substituents independently selected from R²⁴;
each R^{h23} and Rⁱ²³ is independently selected from OH, C₁₋₆ alkoxy, and C₁₋₆ haloalkoxy; or any R^{h23} and Rⁱ²³ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a24}, R^{b24}, R^{c24} and R^{d24}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
each R^{h24} and Rⁱ²⁴ is independently selected from OH, C₁₋₆ alkoxy, and C₁₋₆ haloalkoxy; or any R^{h24} and Rⁱ²⁴ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a30}, R^{b30}, R^{c30} and R^{d30} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{h30} and Rⁱ³⁰ is independently selected from OH, C₁₋₆ alkoxy, and C₁₋₆ haloalkoxy; or any R^{h30} and Rⁱ³⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a31}, R^{b31}, R^{c31} and R^{d31}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R³²;
or any R^{c31} and R^{d31} attached to the same N atom, together with the N atom to which they are attached, form a 4-, 5-, 6- or 7-membered heterocycloalkyl group optionally substituted with 1, 2 or 3 substituents independently selected from R³²;
each R^{h31} and Rⁱ³¹ is independently selected from OH, C₁₋₆ alkoxy, and C₁₋₆ haloalkoxy; or any R^{h31} and Rⁱ³¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a32}, R^{b32}, R^{c32} and R^{d32}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c32} and R^{d32} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h32} and Rⁱ³² is independently selected from OH, C₁₋₆ alkoxy, and C₁₋₆ haloalkoxy; or any R^{h32} and Rⁱ³² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a50}, R^{b50}, R^{c50} and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R^{51;}
or any R^{c50} and R^{d50} attached to the same N atom, together with the N atom to which they are attached, form a 4-, 5-, 6- or 7-membered heterocycloalkyl group optionally substituted with 1, 2 or 3 substituents independently selected from R^{51;}
each R^{h50} and Rⁱ⁵⁰ is independently selected from OH, C₁₋₆ alkoxy, and C₁₋₆ haloalkoxy; or any R^{h50} and Rⁱ⁵⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a51} , R^{b51} R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁵²;
or any R^{c51} and R^{d51} attached to the same N atom, together with the N atom to which they are attached, form a 4-, 5-, 6- or 7-membered heterocycloalkyl group optionally substituted with 1, 2, 3, or 4 substituents independently selected from R⁵²;
each R^{h51} and Rⁱ⁵¹ is independently selected from OH, C₁₋₆ alkoxy, and C₁₋₆ haloalkoxy; or any R^{h51} and Rⁱ⁵¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a52}, R^{b52}, R^{c52} and R^{d52}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c52} and R^{d52} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h52} and Rⁱ⁵² is independently selected from OH, C₁₋₆ alkoxy, and C₁₋₆ haloalkoxy; or any R^{h52} and Rⁱ⁵² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a60}, R^{b60}, R^{c60} and R^{d60} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶¹;
or any R^{c60} and R^{d60} attached to the same N atom, together with the N atom to which they are attached, form a 4-, 5-, 6- or 7-membered heterocycloalkyl group optionally substituted with 1, 2, 3, or 4 substituents independently selected from R⁶¹;
each R^{h60} and Rⁱ⁶⁰ is independently selected from OH, C₁₋₆ alkoxy, and C₁₋₆ haloalkoxy; or any R^{h60} and Rⁱ⁶⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a61}, R^{b61}, R^{c61} and R^{d61}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁶²;
or any R^{c61} and R^{d61} attached to the same N atom, together with the N atom to which they are attached, form a 4-, 5-, 6- or 7-membered heterocycloalkyl group optionally substituted with 1, 2 or 3 substituents independently selected from R⁶²;
each R^{h61} and Rⁱ⁶¹ is independently selected from OH, C₁₋₆ alkoxy, and C₁₋₆ haloalkoxy; or any R^{h61} and Rⁱ⁶¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a62}, R^{b62}, R^{c62} and R^{d62}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c62} and R^{d62} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h62} and Rⁱ⁶² is independently selected from OH, C₁₋₆ alkoxy, and C₁₋₆ haloalkoxy; or any R^{h62} and Rⁱ⁶² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a70}, R^{b70}, R^{c70} and R^{d70} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁷¹;
or any R^{c70} and R^{d70} attached to the same N atom, together with the N atom to which they are attached, form a 4-, 5-, 6- or 7-membered heterocycloalkyl group optionally substituted with 1, 2, 3, or 4 substituents independently selected from R⁷¹;
each R^{h70} and Rⁱ⁷⁰ is independently selected from OH, C₁₋₆ alkoxy, and C₁₋₆ haloalkoxy; or any R^{h70} and Rⁱ⁷⁰ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a71}, R^{b71}, R^{c71} and R^{d71}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁷²;
or any R^{c71} and R^{d71} attached to the same N atom, together with the N atom to which they are attached, form a 4-, 5-, 6- or 7-membered heterocycloalkyl group optionally substituted with 1, 2 or 3 substituents independently selected from R⁷²;
each R^{h71} and Rⁱ⁷¹ is independently selected from OH, C₁₋₆ alkoxy, and C₁₋₆ haloalkoxy; or any R^{h71} and Rⁱ⁷¹ attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl;
each R^{a72}, R^{b72}, R^{c72} and R^{d72}, is independently selected from H, C₁₋₆ alkyl, C₂₋₆ alkenyl, C₂₋₆ alkynyl and C₁₋₆ haloalkyl; wherein said C₁₋₆ alkyl, C₂₋₆ alkenyl and C₂₋₆ alkynyl are each optionally substituted with 1, 2, 3, or 4 substituents independently selected from R^{g};
or any R^{c72} and R^{d72} attached to the same N atom, together with the N atom to which they are attached, form a 4-, 5-, 6- or 7-membered heterocycloalkyl group optionally substituted with 1, 2, 3, or 4 substituents independently selected from R^{g};
each R^{h72} and R⁷² is independently selected from OH, C₁₋₆ alkoxy, and C₁₋₆ haloalkoxy; or any R^{h72} and Rⁱ⁷² attached to the same B atom, together with the B atom to which they are attached, form a 5- or 6-membered heterocycloalkyl group optionally substituted with 1, 2, 3, or 4 substituents independently selected from C₁₋₆ alkyl and C₁₋₆ haloalkyl; and
each R⁹ is independently selected from D, OH, NO₂, CN, halo, C₁₋₆ alkyl, C₂₋₆ alkenyl, C₂₋₆ alkynyl, C₁₋₆ haloalkyl, C₃₋₆ cycloalkyl, C₃₋₆ cycloalkyl-C₁₋₂ alkylene, C₁₋₆ alkoxy, C₁₋₆ haloalkoxy, C₁₋₃alkoxy-C₁₋₃ alkyl, C₁₋₃ alkoxy-C₁₋₃ alkoxy, HO-C₁₋₃ alkoxy, HO-C₁₋₃ alkyl, cyano-C₁₋₃ alkyl, H₂N-C₁₋₃ alkyl, amino, C₁₋₆ alkylamino, di(C₁₋₆ alkyl)amino, thio, C₁₋₆ alkylthio, C₁₋₆ alkylsulfinyl, C₁₋₆ alkylsulfonyl, carbamyl, C₁₋₆ alkylcarbamyl, di(C₁₋₆ alkyl)carbamyl, carboxy, C₁₋₆ alkylcarbonyl, C₁₋₆ alkoxycarbonyl, C₁₋₆ alkylcarbonylamino, C₁₋₆ alkoxycarbonylamino, C₁₋₆ alkylcarbonyloxy, aminocarbonyloxy, C₁₋₆ alkylaminocarbonyloxy, di(C₁₋₆ alkyl)aminocarbonyloxy, C₁₋₆ alkylsulfonylamino, aminosulfonyl, C₁₋₆ alkylaminosulfonyl, di(C₁₋₆ alkyl)aminosulfonyl, aminosulfonylamino, C₁₋₆ alkylaminosulfonylamino, di(C₁₋₆ alkyl)aminosulfonylamino, aminocarbonylamino, C₁₋₆ alkylaminocarbonylamino, and di(C₁₋₆ alkyl)aminocarbonylamino;
provided that, Cy¹ is other than 3,5-dimethylisoxazol-4-yl, 3,5-dimethyl-1H-pyrazol-4-yl or 4-(1-oxo-2-propen-1-yl)phenyl.

2. The compound of claim 1, wherein
each - - - independently represents a single bond or a double bond;
X is N or CR⁷;
Y is N or C;
R¹ is H;
R² is selected from H, C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a2}, SR^{a2}, C(O)R^{b2}, C(O)NR^{c2}R^{d2}, C(O)OR^{a2}, OC(O)R^{b2}, OC(O)NR^{c2}R^{d2}, NR^{c2}R^{d2}, NR^{c2}C(O)R^{b2}, NR^{c2}C(O)OR^{a2}, NR^{c2}C(O)NR^{c2}R^{d2}, NR^{c2}S(O)₂R^{b2}, NR^{c2}S(O)₂NR^{c2}R^{d2}, S(O)₂R^{b2}, and S(O)₂NR^{c2}R^{d2}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, 3, or 4 ring-forming heteroatoms independently selected from N, O, and S; wherein the N and S are optionally oxidized; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2, 3 or 4 substituents independently selected from R¹⁰;
when - - - of R³N- - -CR⁴ represents a single bond;
then R⁴ is =O; and
R³ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R³⁰; or
when - - - of R³N- - -CR⁴ represents a double bond, then R³ is absent; and
R⁴ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a3}, SR^{a3}, C(O)R^{b3}, C(O)NR^{c3}R^{d3}, C(O)OR^{a3}, OC(O)R^{b3}, OC(O)NR^{c3}R^{d3}, NR^{c3}R^{j3}, NR^{c3}C(O)R^{b3}, NR^{c3}C(O)OR^{a3}, NR^{c3}C(0)NR^{c3}R^{d3}, NR^{c3}S(O)₂R^{b3}, NR^{c3}S(O)₂NR^{c3}R^{d3}, S(O)₂R^{b3}, and S(O)₂NR^{c3}R^{d3}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³⁰; or
when - - - of R⁵N- - -YR⁶ represents a single bond and Y is C;
then YR⁶ is C=O; and
R⁵ is selected from H, C₁₋₆ alkyl, C₁₋₆ haloalkyl, C₃₋₁₀ cycloalkyl, and 4-10 membered heterocycloalkyl; wherein said C₁₋₆ alkyl, C₃₋₁₀ cycloalkyl, and 4-10 membered heterocycloalkyl, are each optionally substituted with 1, 2, 3, or 4 substituents independently selected from R⁵⁰; or
when - - - of R⁵N- - -YR⁶ represents a double bond and Y is N;
then R⁵ and R⁶ are absent; or
When - - - of R⁵N- - -YR⁶ represents a double bond and Y is C;
then R⁵ is absent; and
R⁶ is selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a6}, C(O)R^{b5}, C(O)NR^{c6}R^{d6}, C(O)OR^{a6}, OC(O)R^{b6}, OC(O)NR^{c6}R^{d6}, NR^{c6}R^{d6}, NR^{c6}C(O)R^{b6}, NR^{c6}C(O)OR^{a6}, NR^{c6}C(O)NR^{c6}R^{d6}, S(O)₂R^{b6}, and S(O)₂NR^{c6}R^{d6}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R⁶⁰;
R⁷ is selected from H, C₁₋₆ alkyl, C₁₋₆ haloalkyl, C₃₋₁₀ cycloalkyl, 4-10 membered heterocycloalkyl, C₆₋₁₀ aryl, and 5-10 membered heteroaryl, halo, D, CN, OR^{a7}, SR^{a7}, C(O)R^{b7}, C(O)NR^{c7}R^{d7}, C(O)OR^{a7}, OC(O)R^{b7}, OC(O)NR^{c7}R^{d7}, NR^{c7}R^{d7}, NR^{c7}C(O)R^{b7}, NR^{c7}C(O)OR^{a7}, NR^{c7}C(O)NR^{c7}R^{d7} NR^{c7}S(O)₂R^{b7} NR^{c7}S(O)₂NR^{c7}R^{d7}, S(O)₂R^{b7}, and S(O)₂NR^{c7}R^{d7}; wherein said C₁₋₆ alkyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁷⁰;
Cy² is selected from
| | | | |
|---|---|---|---|
| 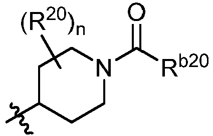 | 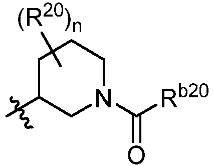 | 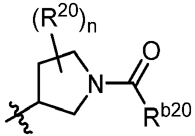 | 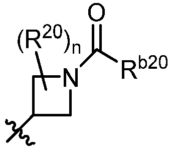 |
| Cy²-a1, | Cy²-b1, | Cy²-c1, and | Cy²-d1, |
n is 0, 1, or 2;
each R¹⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, halo, D, CN, OR^{a10}, SR^{a10}, C(O)NR^{c10}R^{d10}, C(O)OR^{a10}, OC(O)R^{b10}, OC(O)NR^{c10}R^{d10}, NR^{c10}R^{d10}, NR^{c10}C(O)R^{b10}, NR^{c10}C(O)OR^{a10,} NR^{c10}C(O)NR^{c10}R^{d10}, NR^{c10}S(O)₂R^{b10}, NR^{c10}S(O)₂NR^{c10}R^{d10}, S(O)_{2R}^{b10}, and S(O)_{2NR}^{c10}R^{d10}; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R¹¹;
each R¹¹ is independently selected from C₁₋₆ alkyl, C₁₋₆ haloalkyl, halo, D, CN, OR^{a11}, C(O)R^{b11} C(O)NR^{c11}R^{d11}, C(O)OR^{a11}, OC(O)R^{b11}, OC(O)NR^{c11}R^{d11}, NR^{c11}R^{d11}, NR^{c11}C(O)R^{b11}, NR^{c11} C(O)OR^{a11}, S(O)₂R^{b11}, and S(O)₂NR^{c11}R^{d11};
each R²⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, halo, D, CN, OR^{a20}, SR^{a20}, C(O)R^{b20}, C(O)NR^{c20}R^{d20}, C(O)OR^{a32}, OC(O)R^{b20}, OC(O)NR^{c20}R^{d20}, NR^{c20}R^{d20,} NR^{c20}C(O)R^{b20}, NR^{c20}C(O)OR^{a20,} NR^{c20}C(O)NR^{c20}R^{d20}, NR^{c20}S(O)₂R^{b20}, NR^{c20}S(O)₂NR^{c20}R^{d20}, S(O)₂R^{b20}, and S(O)₂NR^{c20}R^{d20}; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R²¹;
each R²¹ is independently selected from C₁₋₆ alkyl, C₂₋₆ alkenyl, C₂₋₆ alkynyl, C₁₋₆ haloalkyl, halo, D, CN, OR^{a21}, C(O)R^{b21}, C(O)NR^{c21}R^{d21}, C(O)OR^{a21}, OC(O)R^{b21}, OC(O)NR^{c21}R^{d21}, NR^{c21}R^{d21}, NR^{c21}C(O)R^{b21}, NR^{c21}C(O)OR^{a21}, S(O)₂R^{b21}, and S(O)₂NR^{c21}R^{d21}; wherein said C₁₋₆ alkyl, C₂₋₆ alkenyl, and C₂₋₆ alkynyl, are each optionally substituted with 1, 2, 3, or 4 substituents independently selected from R^{g};
each R²² is independently selected from C₁₋₆ alkyl, C₁₋₆ haloalkyl, halo, D, CN, OR^{a22}, C(O)R^{b22}, C(O)NR^{c22}R^{d22}, C(O)OR^{a22}, OC(O)R^{b22}, OC(O)NR^{c22}R^{d22}, NR^{c22}R^{d22}, NR^{c22}C(O)R^{b22}, NR^{c22}C(O)OR^{a22}, S(O)₂R^{b22}, and S(O)₂NR^{c22}R^{d22};
each R³⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene, 5-10 membered heteroaryl-C₁₋₃ alkylene, halo, D, CN, OR^{a30}, SR^{a30}, C(O)R^{b30}, C(O)NR^{c30}R^{d30}, C(O)OR^{a30}, OC(O)R^{b30}, OC(O)NR^{c30}R^{d30} NR^{c30}R^{d30}, NR^{c30}C(O)R^{b30}, NR^{c30}C(O)OR^{a30}, S(O)₂R^{b30}, and S(O)₂NR^{c30}R^{d30}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, 5-10 membered heteroaryl, C₃₋₁₀ cycloalkyl-C₁₋₃ alkylene, 4-10 membered heterocycloalkyl-C₁₋₃ alkylene, C₆₋₁₀ aryl-C₁₋₃ alkylene and 5-10 membered heteroaryl-C₁₋₃ alkylene are each optionally substituted with 1, 2, 3, or 4 substituents independently selected from R³¹;
each Rⁱ³¹ is independently selected from C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl, halo, D, CN, OR^{a31}, C(O)R^{b31}, C(O)NR^{c31}R^{d31}, C(O)OR^{a31,} OC(O)R^{b31} , OC(O)NW³¹R^{d31}, NR^{c31}R^{d31}, NR^{c31}C(O)R^{b31}, NR^{c31}C(O)OR^{a31}, S(O)₂R^{b31}, and S(O)₂NR^{c31}R^{d31}; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R³²;
each R³² is independently selected from C₁₋₆ alkyl, C₁₋₆ haloalkyl, halo, D, CN, OR^{a32}, C(O)R^{b32}, C(O)NR^{c32}R^{d32}, C(O)OR^{a32}, OC(O)R^{b32}, OC(O)NR^{c32}R^{d32}, NR^{c32}R^{d32}, NR^{c32}C(O)R^{b32}, NR^{c32}C(O)OR^{a32} , S(O)₂R³², and S(O)₂NR^{c32}R^{d32};
each R⁵⁰ is independently selected from C₁₋₆ alkyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a50}, C(O)R^{b50}, C(O)NR^{c50}R^{d50}, C(O)OR^{a50}, OC(O)R^{b50}, OC(O)NR^{c50}R^{d50}, NR^{c50}R^{d50}, NR^{c50}C(O)R^{b50}, NR^{c50}C(O)OR^{a50}, S(O)₂R^{b50}, and S(O)₂NR^{c50}R^{d50}; wherein said C₁₋₆ alkyl, C₃₋₁₀cycloalkyl, 4-10 membered heterocycloalkyl, C₆₋₁₀ aryl, and 5-10 membered heteroaryl, are each optionally substituted with 1, 2, 3, or 4 substituents independently selected from R^{51;}
each R⁵¹ is independently selected from C₁₋₆ alkyl, C₁₋₆ haloalkyl, halo, D, CN, OR^{a51,} C(O)R^{b51}, C(O)NR^{c51}R^{d51}, C(O)OR^{a51}, OC(O)R^{b51}, OC(O)NR^{c51}R^{d51} NR^{c51}R^{d51}, NR^{c51}C(O)R^{b51} , NR^{c51}C(O)OR^{a51}, S(O)₂R^{b51}, and S(O)₂NR^{c51}R^{d51}; wherein said C₁₋₆ alkyl, are each optionally substituted with 1, 2, 3, or 4 substituents independently selected from R⁵²;
each R⁵² is independently selected from C₁₋₆ alkyl, C₁₋₆ haloalkyl, halo, D, CN, OR^{a52}, C(O)R^{bS2}, C(O)NR^{c52}R^{d52}, C(O)OR^{a52}, OC(O)R^{b52}, OC(O)NR^{c52}R^{d52}, NR^{c52}R^{d52}, NR^{c52}C(O)R^{b52}, NR^{c52}C(O)OR^{a52}, S(O)2R^{b52}, and S(O)₂NR^{c52}R^{d52};
each R⁶⁰ is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl, 5-10 membered heteroaryl, halo, D, CN, OR^{a60}, SR^{a60}, C(O)R^{b60}, C(O)NR^{c60}R^{d60}, C(O)OR^{a60}, OC(O)R^{b60}, OC(O)NR^{c60}R^{d60}, NR^{c60}R^{d60}, NR^{c60}C(O)R^{b60}, NR^{c60}C(O)OR^{a60}, S(O)₂R^{b60}, and S(O)₂NR^{c60}R^{d60}; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl, and 5-10 membered heteroaryl, are each optionally substituted with 1, 2, 3, or 4 substituents independently selected from R⁶¹;
each R⁶¹ is independently selected from C₁₋₆ alkyl, C₁₋₆ haloalkyl, halo, D, CN, OR^{a61}, C(O)R^{b61}, C(O)NR^{c61}R^{d61}, C(O)OR^{a61}, OC(O)R^{b61}, OC(O)NR^{c61}R^{d61}, NR^{c61}R^{d61}, NR^{c61}C(O)R^{b61}, NR^{c61}C(O)OR^{a61}, S(O)₂R^{b61}, and S(O)₂NR^{c61}R^{d61};
each R⁷⁰ is independently selected from C₁₋₆ alkyl, C₁₋₆ haloalkyl, halo, D, CN, OR^{a70}, C(O)R^{b70}, C(O)NR^{c70}R^{d70}, C(O)OR^{a70}, OC(O)R^{b70}, OC(O)NR^{c70}R^{d70}, NR^{c70}R^{d70}, NR^{c70}C(O)R^{b70}, NR^{c70}C(O)OR^{a70}, S(O)₂R^{b70}, and S(O)₂NR^{c70}R^{d70};
each R^{a2}, R^{b2}, R^{c2} and R^{d2} is independently selected from H, C₁₋₆ alkyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R²²;
or any R^{c2} and R^{d2} attached to the same N atom, together with the N atom to which they are attached, form a 4-, 5-, 6- or 7-membered heterocycloalkyl group optionally substituted with 1, 2, 3, or 4 substituents independently selected from R²²;
each R^{a3}, R^{b3}, R^{c3} and R^{d3} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{d3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{j3} is independently selected from C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³⁰;
or any R^{c3} and R^{j3} attached to the same N atom, together with the N atom to which they are attached, form a 4-, 5-, 6- or 7-membered heterocycloalkyl group optionally substituted with 1, 2, 3, or 4 substituents independently selected from R³⁰;
each R^{a6}, R^{b6}, R^{c6}, and R^{d6} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶⁰;
or any R^{c6} and R^{d6} attached to the same N atom, together with the N atom to which they are attached, form a 4-, 5-, 6- or 7-membered heterocycloalkyl group optionally substituted with 1, 2 or 3 substituents independently selected from R⁶⁰;
each R^{a7}, R^{b7}, R^{c7} and R^{d7} is independently selected from H, C₁₋₆ alkyl, and C₁₋₆ haloalkyl; wherein said C₁₋₆ alkyl, are each optionally substituted with 1, 2, 3, or 4 substituents independently selected from R⁷⁰;
each R^{a10}, R^{b10}, R^{c10} and R^{d10} is independently selected from H, C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl; wherein said C₁₋₆ alkyl, C₂₋₆ alkenyl, C₂₋₆ alkynyl, C₃₋₁₀ cycloalkyl, and 4-10 membered heterocycloalkyl are each optionally substituted with 1, 2, 3, or 4 substituents independently selected from R¹¹;
or any R^{c10} and R^{d10} attached to the same N atom, together with the N atom to which they are attached, form a 4-, 5-, 6- or 7-membered heterocycloalkyl group optionally substituted with 1, 2, 3, or 4 substituents independently selected from R¹¹;
each R^{a11}, R^{b11}, R^{c11} and R^{d11}, is independently selected from H, C₁₋₆ alkyl, and C₁₋₆ haloalkyl;
each R^{a20}, R^{b20}, R^{c20} and R^{d20} is independently selected from **H,** C₁₋₆ alkyl, C₂₋₆ alkenyl, C₂₋₆ alkynyl, C₁₋₆ haloalkyl, C₃₋₁₀ cycloalkyl, and 4-10 membered heterocycloalkyl; wherein said C₁₋₆ alkyl, C₂₋₆ alkenyl, C₂₋₆ alkynyl, C₃₋₁₀ cycloalkyl, and 4-10 membered heterocycloalkyl, are each optionally substituted with 1, 2, 3, or 4 substituents independently selected from R²¹;
or any R^{c20} and R^{d20} attached to the same N atom, together with the N atom to which they are attached, form a 4-, 5-, 6- or 7-membered heterocycloalkyl group optionally substituted with 1, 2, 3, or 4 substituents independently selected from R²¹;
each R^{a21}, R^{b21}, R^{c21} and R^{d21}, is independently selected from **H,** C₁₋₆ alkyl, C₂₋₆ alkenyl, C₂₋₆ alkynyl, C₁₋₆ haloalkyl; wherein said C₁₋₆ alkyl C₂₋₆ alkenyl, and C₂₋₆ alkynyl, are each optionally substituted with 1, 2, 3, or 4 substituents independently selected from R^{g};
each R^{a22}, R^{b22}, R^{c22} and R^{d22} is independently selected from **H,** C₁₋₆ alkyl, and C₁₋₆ haloalkyl;
each R^{a30}, R^{b30}, R^{c30} and R^{d30} is independently selected from **H,** C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R³¹;
or any R^{c30} and R^{d30} attached to the same N atom, together with the N atom to which they are attached, form a 4-, 5-, 6- or 7-membered heterocycloalkyl group optionally substituted with 1, 2, 3, or 4 substituents independently selected from R³¹;
each R^{a31}, R^{b31}, R^{c31} and R^{d31}, is independently selected from **H,** C₁₋₆ alkyl, C₂₋₆ alkenyl, C₂₋₆ alkynyl, C₁₋₆ haloalkyl, C₃₋₆ cycloalkyl, and 4-7 membered heterocycloalkyl; wherein said C₁₋₆ alkyl C₂₋₆ alkenyl, C₂₋₆ alkynyl, C₃₋₆ cycloalkyl, and 4-7 membered heterocycloalkyl are each optionally substituted with 1, 2, 3, or 4 substituents independently selected from R³²;
or any R^{c31} and R^{d31} attached to the same N atom, together with the N atom to which they are attached, form a 4-, 5-, 6- or 7-membered heterocycloalkyl group optionally substituted with 1, 2 or 3 substituents independently selected from R³²;
each R^{a32}, R^{b32}, R^{c32} and R^{d32}, is independently selected from **H,** C₁₋₆ alkyl, and C₁₋₆ haloalkyl;
each R^{a50}, R^{b50} R^{c50} and R^{d50}, is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁵¹;
or any R^{c50} and R^{d50} attached to the same N atom, together with the N atom to which they are attached, form a 4-, 5-, 6- or 7-membered heterocycloalkyl group optionally substituted with 1, 2 or 3 substituents independently selected from R⁵¹;
each R^{a51}, R^{b51}, R^{c51} and R^{d51}, is independently selected from H, C₁₋₆ alkyl, C₂₋₆ alkenyl, C₂₋₆ alkynyl, C₁₋₆ haloalkyl, C₃₋₆ cycloalkyl, phenyl, 5-6 membered heteroaryl and 4-7 membered heterocycloalkyl; wherein said C₁₋₆ alkyl C₂₋₆ alkenyl, C₂₋₆ alkynyl, C₃₋₆ cycloalkyl, phenyl, 5-6 membered heteroaryl and 4-7 membered heterocycloalkyl are each optionally substituted with 1, 2, 3, or 4 substituents independently selected from R⁵²;
each R^{a52}, R^{b52}, R^{c52} and R^{d52}, is independently selected from H, C₁₋₆ alkyl, and C₁₋₆ haloalkyl;
each R^{a60}, R^{b60}, R^{c60} and R^{d60} is independently selected from H, C₁₋₆ alkyl, C₂₋₆ alkenyl, C₂₋₆ alkynyl, C₁₋₆ haloalkyl, C₃₋₁₀ cycloalkyl, 4-10 membered heterocycloalkyl, C₆₋₁₀ aryl and 5-10 membered heteroaryl; wherein said C₁₋₆ alkyl, C₂₋₆ alkenyl, C₂₋₆ alkynyl, C₃₋₁₀ cycloalkyl, 4-10 membered heterocycloalkyl, C₆₋₁₀ aryl and 5-10 membered heteroaryl, are each optionally substituted with 1, 2, 3, or 4 substituents independently selected from R⁶¹;
or any R^{c60} and R^{d60} attached to the same N atom, together with the N atom to which they are attached, form a 4-, 5-, 6- or 7-membered heterocycloalkyl group optionally substituted with 1, 2, 3, or 4 substituents independently selected from R⁶¹;
each R^{a61}, R^{b61}, R^{c61} and R^{d61}, is independently selected from H, C₁₋₆ alkyl, and C₁₋₆ haloalkyl;
each R^{a70}, R^{b70}, R^{c70} and R^{d70} is independently selected from H, C₁₋₆ alkyl, and C₁₋₆ haloalkyl; and
each R⁹ is independently selected from D, OH, CN, halo, C₁₋₆ alkyl, C₂₋₆ alkenyl, C₂₋₆ alkynyl, C₁₋₆ haloalkyl, C₃₋₆ cycloalkyl, C₃₋₆ cycloalkyl-C₁₋₂ alkylene, C₁₋₆ alkoxy, C₁₋₆ haloalkoxy, C₁₋₃ alkoxy-C₁₋₃ alkyl, C₁₋₃ alkoxy-C₁₋₃ alkoxy, HO-C₁₋₃ alkoxy, HO-C₁₋₃ alkyl, cyano-C₁₋₃ alkyl, H₂N-C₁₋₃ alkyl, amino, C₁₋₆ alkylamino, di(C₁₋₆ alkyl)amino, C₁₋₆ alkylthio, C₁₋₆ alkylsulfonyl, C₁₋₆ alkylcarbamyl, di(C₁₋₆ alkyl)carbamyl, carboxy, C₁₋₆ alkylcarbonyl, C₁₋₆ alkoxycarbonyl, C₁₋₆ alkylcarbonylamino, C₁₋₆ alkoxycarbonylamino, C₁₋₆ alkylcarbonyloxy, and di(C₁₋₆ alkyl)aminosulfonyl.

3. The compound of claim 1, wherein the compound of Formula I is a compound of Formula II: 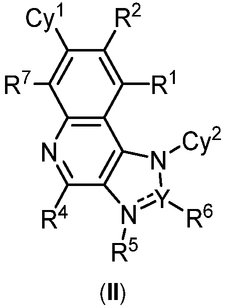 or a pharmaceutically acceptable salt thereof; wherein
each --- independently represents a single bond or a double bond;
Y is N or C;
R¹ is H;
R² is selected from H, C₁₋₃ alkyl, C₁₋₃ haloalkyl, halo, D, and CN; wherein said C₁₋₃ alkyl, is optionally substituted with 1 or 2, substituents independently selected from R²²;
Cy¹ is selected from C₃₋₁₀ cycloalkyl, 4-10 membered heterocycloalkyl, C₆₋₁₀ aryl and 6-10 membered heteroaryl; wherein the 4-10 membered heterocycloalkyl and 6-10 membered heteroaryl each has at least one ring-forming carbon atom and 1, 2, or 3 ring-forming heteroatoms independently selected from N and O; wherein a ring-forming carbon atom of 6-10 membered heteroaryl and 4-10 membered heterocycloalkyl is optionally substituted by oxo to form a carbonyl group; and wherein the C₃₋₁₀ cycloalkyl, 4-10 membered heterocycloalkyl, C₆₋₁₀ aryl and 6-10 membered heteroaryl are each optionally substituted with 1, 2 or 3 substituents independently selected from R¹⁰;
when --- of R³N---CR⁴ represents a single bond;
then R⁴ is =O; and
R³ is selected from H, C₁₋₃ alkyl, C₁₋₃ haloalkyl, phenyl, and 5-6 membered heteroaryl; wherein said C₁₋₃ alkyl, phenyl, and 5-6 membered heteroaryl, are each optionally substituted with 1 or 2 substituents independently selected from R³⁰; or
when --- of R³N---CR⁴ represents a double bond;
then R³ is absent; and
R⁴ is selected from H, C₁₋₃ alkyl, C₁₋₃ haloalkyl, C₃₋₆ cycloalkyl, 4-6 membered heterocycloalkyl, C₆₋₁₀ aryl, 5-6 membered heteroaryl, halo, D, CN, OR^{a3}, and NR^{c3}R^{j3}; wherein said C₁₋₃ alkyl, C₃₋₆ cycloalkyl, 4-6 membered heterocycloalkyl, C₆₋₁₀ aryl, and 5-6 membered heteroaryl, are each optionally substituted with 1 or 2 substituents independently selected from R³⁰; or
when --- of R⁵N---YR⁶ represents a single bond and Y is C;
then YR⁶ is C=O; and
R⁵ is selected from H, C₁₋₃ alkyl, and C₁₋₃ haloalkyl; wherein said C₁₋₃ alkyl, is optionally substituted with 1 or 2 substituents independently selected from R⁵⁰; or
when --- of R⁵N---YR⁶ represents a double bond and Y is N;
then R⁵ and R⁶ are absent; or
when --- of R⁵N---YR⁶ represents a double bond and Y is C;
then R⁵ is absent; and
R⁶ is selected from H, C₁₋₃ alkyl, and C₁₋₃ haloalkyl;
R⁷ is selected from H, C₁₋₃ alkyl, C₁₋₃ haloalkyl, halo, D, and CN;
Cy² is selected from
| | | | |
|---|---|---|---|
| 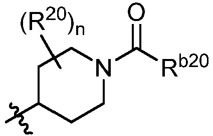 | 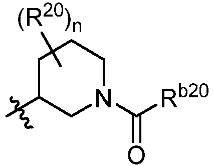 | 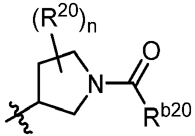 | 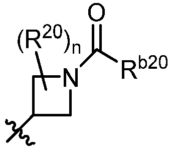 |
| Cy²-a1, | Cy²-b1, | Cy²-c1, and | Cy²-d1, |
n is 0, 1, or 2;
each R¹⁰ is independently selected from C₁₋₃ alkyl, C₁₋₃ haloalkyl, C₃₋₅ cycloalkyl, halo, D, CN, OR^{a10}, and NR^{c10}Rd¹⁰;
each R²⁰ is independently selected from C₁₋₃ alkyl, C₂₋₃ alkenyl, C₂₋₃ alkynyl, C₁₋₃ haloalkyl, halo, D, CN, OR^{a20}, C(O)R^{b20}, C(O)NR^{c20}R^{d20}, C(O)OR^{a20}, and NR^{c20}R^{d20}; wherein said C₁₋₃ alkyl, C₂₋₃ alkenyl, and C₂₋₃ alkynyl, are each optionally substituted with 1 or 2 substituents independently selected from R²¹;
each R²¹ is independently selected from C₁₋₃ alkyl, C₁₋₃ haloalkyl, halo, D, CN OR^{a21}, and NR^{c21}R^{d21};
each R²² is independently selected from C₁₋₆ alkyl, C₁₋₆ haloalkyl, halo, D, and CN;
each R³⁰ is independently selected from C₁₋₃ alkyl, C₁₋₃ haloalkyl, C₃₋₆ cycloalkyl, 4-6 membered heterocycloalkyl, phenyl, 5-6 membered heteroaryl, halo, D, CN, OR^{a30}, and NR^{c30}R^{d30}; wherein said C₁₋₃ alkyl, C₃₋₆ cycloalkyl, 4-6 membered heterocycloalkyl, phenyl, and 5-6 membered heteroaryl, are each optionally substituted with 1 or 2 substituents independently selected from R³¹;
each R³¹ is independently selected from C₁₋₃ alkyl, C₁₋₃ haloalkyl, halo, D, and CN;
each R⁵⁰ is independently selected from C₁₋₃ alkyl, C₁₋₃ haloalkyl, C₃₋₆ cycloalkyl, 4-6 membered heterocycloalkyl, halo, D, and CN; wherein said C₁₋₃ alkyl, C₃₋₆ cycloalkyl, and 4-6 membered heterocycloalkyl, are each optionally substituted with 1 or 2 substituents independently selected from R⁵¹;
each R⁵¹ is independently selected from C₁₋₃ alkyl, C₁₋₃ haloalkyl, halo, D, and CN;
each R^{a3} and R^{c3} is independently selected from H, C₁₋₃ alkyl, and C₁₋₃ haloalkyl; wherein said C₁₋₃ alkyl, is optionally substituted with 1 or 2 substituents independently selected from R³⁰;
each R^{j3} is independently selected from C₁₋₃ alkyl, and C₁₋₃ haloalkyl; wherein said C₁₋₃ alkyl, is optionally substituted with 1 or 2 substituents independently selected from R³⁰;
each R^{a10}, R^{b10}, R^{c10} and R^{d10} is independently selected from H, C₁₋₃ alkyl, and C1-3 haloalkyl;
each R^{a20}, R^{b20}, R^{c20} and R^{d20} is independently selected from H, C₁₋₃ alkyl, C₂₋₃ alkenyl, C₂₋₃ alkynyl, and C₁₋₃ haloalkyl; wherein said C₁₋₃ alkyl, C₂₋₃ alkenyl, and C₂₋₃ alkynyl, are each optionally substituted with 1 or 2 substituents independently selected from R²¹;
each R^{a21}, R^{c21} and R^{d21}, is independently selected from H, C₁₋₆ alkyl, and C₁₋₆ haloalkyl; and
each R^{a30}, R^{c30} and R^{d30} is independently selected from H, C₁₋₃ alkyl, and C₁₋₃ haloalkyl; wherein said C₁₋₃ alkyl, are each optionally substituted with 1 or 2 substituents independently selected from R³¹.

4. The compound of claim 3, wherein the compound of Formula II is a compound of Formula IIa: 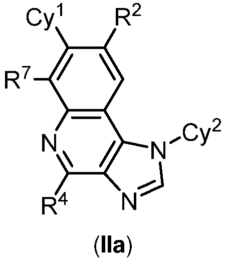 or a pharmaceutically acceptable salt thereof; or
the compound of Formula II is a compound of Formula IIb: 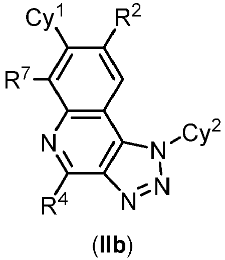 or a pharmaceutically acceptable salt thereof; or the compound of Formula II is a compound of Formula llc: 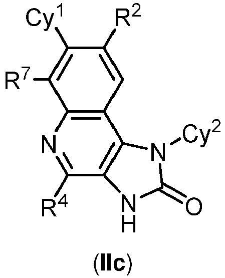 or a pharmaceutically acceptable salt thereof; or the compound of Formula I is a compound of Formula Ig: 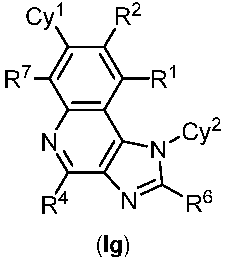 or a pharmaceutically acceptable salt thereof; or the compound of Formula I is a compound of Formula le: 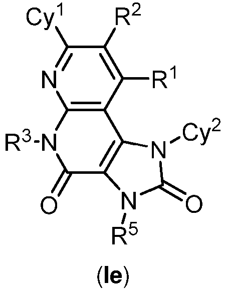 or a pharmaceutically acceptable salt thereof.

5. The compound of claim 1 or 3, or a pharmaceutically acceptable salt thereof, wherein Cy¹ is selected from C₆₋₁₀ aryl and 6-10 membered heteroaryl; wherein the 6-10 membered heteroaryl each has at least one ring-forming carbon atom and 1, 2, or 3 ring-forming heteroatoms independently selected from N, O, and S; wherein the N and S are optionally oxidized; wherein a ring-forming carbon atom of 6-10 membered heteroaryl is optionally substituted by oxo to form a carbonyl group; and wherein the C₆₋₁₀ aryl and 6-10 membered heteroaryl are each optionally substituted with 1, 2, or 3 substituents independently selected from R¹⁰.

6. The compound of claim 1 or 2, or a pharmaceutically acceptable salt thereof, wherein X is N.

7. The compound of claim 1 or 2, or a pharmaceutically acceptable salt thereof, wherein X is CR⁷.

8. The compound of claim 1 or 2, or a pharmaceutically acceptable salt thereof, wherein R³ is selected from H, C₁₋₆ alkyl, C₁₋₆ haloalkyl, phenyl, and 5-6 membered heteroaryl; wherein said C₁₋₆ alkyl, phenyl, and 5-6 membered heteroaryl, are each optionally substituted with 1 or 2 substituents independently selected from R³⁰.

9. The compound of claim 1 or 2, or a pharmaceutically acceptable salt thereof, wherein R⁴ is selected from H, C₁₋₆ alkyl, C₁₋₆ haloalkyl, 4-10 membered heterocycloalkyl, C₆₋₁₀ aryl, 5-10 membered heteroaryl, halo, D, CN, OR^{a3}, and NR^{c3}R^{j3}; wherein said C₁₋₆ alkyl, 4-10 membered heterocycloalkyl, C₆₋₁₀ aryl, and 5-10 membered heteroaryl, are each optionally substituted with 1, 2, or 3 substituents independently selected from R³⁰.

10. The compound of any one of claims 1-3, or a pharmaceutically acceptable salt thereof, wherein Y is N.

11. The compound of any one of claims 1-3, or a pharmaceutically acceptable salt thereof, wherein Y is C.

12. The compound of claim 1 or 2, or a pharmaceutically acceptable salt thereof, wherein R⁵ is selected from H, C₁₋₆ alkyl, and C₁₋₆ haloalkyl; wherein said C₁₋₆ alkyl is optionally substituted by 1 or 2 substituents independently selected from R⁵⁰.

13. A pharmaceutical composition comprising a compound of any one of claims 1-12, or a pharmaceutically acceptable salt thereof, and at least one pharmaceutically acceptable carrier or excipient.

14. A compound according to any one of claims 1-12, or a pharmaceutically acceptable salt thereof, or a composition according to claim 13, for use in medicine.

15. A compound of any one of claims 1-12, or a pharmaceutically acceptable salt thereof, or the composition of claim 13, for use in treating a disease or disorder associated with inhibiting a KRAS protein harboring a G12C mutation

16. A compound of any one of claims 1-12, or a pharmaceutically acceptable salt thereof, or the composition of claim 13, for use in treating a cancer

17. The compound, or a pharmaceutically acceptable salt thereof, or the composition according to claim 16, wherein the cancer is selected from carcinomas, hematological cancers, sarcomas, and glioblastoma; preferably wherein the cancer is a hematological cancer is selected from myeloproliferative neoplasms, myelodysplastic syndrome, chronic and juvenile myelomonocytic leukemia, acute myeloid leukemia, acute lymphocytic leukemia, and multiple myeloma; or the cancer is a carcinoma selected from pancreatic, colorectal, lung, bladder, gastric, esophageal, breast, head and neck, cervical, skin, and thyroid carcinomas.

18. A compound of any one of claims 1-12, or a pharmaceutically acceptable salt thereof, or the composition of claim 13, for use in treating an immunological or inflammatory disorder, preferably wherein the immunological or inflammatory disorder is Ras-associated lymphoproliferative disorder or juvenile myelomonocytic leukemia caused by somatic mutations of KRAS.
